# EUROPEAN PATENT APPLICATION

(11) **EP 1 510 210 A1**
(43) Date of publication of application: **02.03.2005**
(21) Application number: 03730840.0
(22) Date of filing: 05.06.2003
(51) Int. Cl.: A61K 31/167, A61K 31/381, A61K 31/402, A61K 31/426, A61K 31/496, A61K 31/5377, A61P 19/02, A61P 19/10, A61P 29/00, A61P 37/02, A61P 43/00

(54) **IMMUNITY-RELATED PROTEIN KINASE INHIBITORS**

(30) Priority: 05.06.2002 JP 2002164525
(71) Applicant: Institute of Medicinal Molecular Design, Inc., Tokyo 113-0033 (JP)
(72) Inventor: MUTO, Susumu, Koganei-shi, Tokyo 184-0003 (JP); ITAI, A. INST. OF MEDICINAL MOLECULAR DESIGN INC., Bunkyo-ku, Tokyo 113-033 (JP)
(74) Representative: Sternagel, Fleischer, Godemeyer & Partner
(86) International application number: PCT/JP2003/007130
(87) International publication number: WO 2003/103658

(57) **Abstract**

A medicament having an inhibitory activity against IKK-β and/or MEKK-1 or other protein kinases structurally similar thereto, which comprises as an active ingredient a substance selected from the group consisting of a compound represented by the following general formula (I) and a pharmacologically acceptable salt thereof, and a hydrate thereof and a solvate thereof: 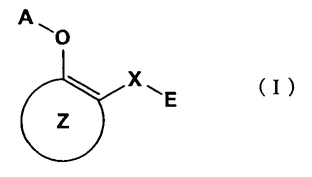 wherein X represents a connecting group whose number of atoms in the main chain is 2 to 5 (said connecting group may be substituted), A represents hydrogen atom or acetyl group, E represents an aryl group which may be substituted or a hetero aryl group which may be substituted, ring Z represents an arene which may have one or more substituents in addition to the group represented by formula -O-A wherein A has the same meaning as that defined above and the group represented by formula -X-E wherein each of X and E has the same meaning as that defined above, or a heteroarene which may have one or more substituents in addition to the group represented by formula -O-A wherein A has the same meaning as that defined above and the group represented by formula -X-E wherein each of X and E has the same meaning as that defined above.

## Description

### Field of Invention

The present invention relates to pharmaceutical compositions having an inhibitory activity against IKK-β and/or MEKK-1 or other protein kinases structurally similar thereto.

### Background Art

Inflammation is a basic defense mechanism to various infestations, where inflammatory cytokine such as interleukin (IL)-1 and TNF-α (tumor necrosis factor) are known to play important roles. Due to the progress of gene analysis of inflammatory cytokines and inflammatory cell adhesion factors, it has been revealed that these cytokines are controlled by a common transcription factor (also called as "transcription regulatory factor"). This transcription factor is a protein called as NF-κB (also described as NF κ B, Nucleic Acids Research, (England), 1986, Vol.14, No.20, p.7897-1914; Cold Spring Harbor Symposia on Quantitative Biology, (USA), 1986, Vol.51, No.1, p.611-624).

The NF-κB is a hetero dimer(also called as "complex") of p65(also called as "Rel A") and p50(also called as "NF-κB-1"), usually binds to I-κB when external stimulation does not exist, and exists in cytoplasm as an inactive type. I-κB is phosphorated by various external stimulations such as oxidative stress, cytokine, lipopolysaccharide, virus, UV, free radical, and protein kinase C to become ubiquitin, and then decomposed by proteasome (Genes & Development, (USA), 1995, Vol.9, No.22, p.2723-2735). NF-κB separated from I-*κ*B immediately move into nucleus, and plays a role as a transcription factor by binding to promoter region which has recognition sequence of NF-κ B.

In 1997, phosphoenzyme (called as I κB kinase abbreviated as "IKK") which participates in phosphorylation of I-κB was identified (Nature, (England), 1997, Vol.388, p.548-554; Cell, (USA), 1997, Vol.90, No.2, p.373-383). IKK-α (also called as "IKK1") and IKK-β (also called as "IKK2") which are similar to each other exist among a class of IKK, and they are known to form a complex to bind directly to I-κB and phosphorize I-κB (Science, (USA), 1997, Vol.278, p.866-869; Cell, (USA), 1997, Vol.91, No.2, p.243-252).

Recently, a mechanism except cyclooxygenase inhibition is suggested for aspirin, which is a widely used anti-inflammatory agent, and the mechanism is known to be based on the inhibition of NF-κB activation (Science, (USA), 1994, Vol.265, p.956-959). Moreover, it was revealed that aspirin regulates the release and activation of NF-κB by binding reversibly to IKK-β, as being an I-κB kinase, under competition with ATP and by inhibiting phosphorylation of I-κB (Nature, (England), 1998, Vol.396, p.77-80). However, a huge amount of aspirin needs to be administered to sufficiently suppress NF-κB activation, and as a result, side effects such as gastrointestinal disorders by prostaglandin synthesis inhibition and increase of bleeding tendency by anticoagulation action are expected to be caused with high probability. Accordingly, aspirin is not suitable for long term application.

Besides aspirin, some pharmaceuticals are known to have inhibitory action against NF-κB activation. Glucocorticoids (steroid hormones) such as dexamethasone suppress NF-κB activation by binding to their receptors (called as "glucocorticoid receptor," Science, (USA), 1995, Vol.270, p.283-286). However, long term use is not suitable, because they have serious side effects such as aggravation of an infectious disease, generation of peptic ulcer, degradation of bone density, and central action. Leflunomide as an immunosuppressive agent, an isoxazole-type agent, also has NF-κ inhibitory action (Journal of Immunology, (USA), 1999, Vol.162, No.4, p.2095-2102). However, this drug is also not suitable for long term use due to serious side effects. Furthermore, substituted pyrimidine derivatives (Japanese Patent Publication of International Application (KOHYO) No.(Hei)11-512399, and Journal of Medicinal Chemistry, (USA), 1998, Vol.41, No.4, p.413-419), xanthine derivatives (Japanese Patent Unexamined Publication (KOKAI) No.(Hei)9-227561), isoquinoline derivatives (Japanese Patent Unexamined Publication (KOKAI) No.(Hei)10-87491), indan derivatives (International Patent Publication WO00/05234 pamphlet), N-phenylsalicylamide derivatives (International Publication WO99/65499 pamphlet, International Publication W002/49632 pamphlet, and International Publication WO02/076918 pamphlet), epoxyquinomycin C, D, and their derivatives (Japanese Patent Unexamined Publication (KOKAI) No.(Hei)10-45738, and Bioorganic & Medicinal Chemistry Letters, (England), 2000, Vol.10, No.9, p.865-869) are known as inhibitors against NF-κB activation. However, mechanism of inhibition against NF- κB activation and participating receptors or proteins have not been revealed. β -Carboline derivatives (International Publication WO01/68648 pamphlet) are known as IKK-β inhibitors, however, any data which show usefulness as a medicament are not disclosed. Moreover, in the pamphlet of International Patent Publication W002/051397, N-phenylsalicylamide derivatives are disclosed as inhibitors against the production of cytokines.

Compounds having specific inhibitory action against IKK-β, found by using IKK-β as a target which directly induces phosphorylation of IKK-β, are expected to have inhibitory action against production and release of the target inflammatory cytokine and inhibitory action against production of inflammatory cell adhesion molecules, without affecting other signal transfer pathway, that is, without causing serious side effects. NF-κB activation is induced by the aforementioned external stimulation, and as a result, proteins such as inflammatory cytokine are expressed. Among the inflammatory cytokines, TNF-α and interleukin (IL)-1 whose gene expression itself is considered to be regulated positively by NF-κB to form positive feedback loop (TNF-α →NF-κB→TNF-α ) and is considered to participate in chronicity of inflammation (18th Meeting of The Japanese Inflammatory Society, Symposium "Mechanism of Antirheumatic Pharmaceutical composition and New Development" Tokyo, 2000). Accordingly, the compounds which specifically inhibit IKK-β as a target are expected to be useful drugs for inflammatory diseases advanced in a chronic stage and diseases caused by TNF-α and IL-1.

### Disclosure of the Invention

An object of the present invention is to provide medicaments useful for preventive and/or therapeutic treatment of inflammatory disorders, autoimmune disease such as chronic arthrorheumatism, and bone disease such as osteoporosis, in which inflammatory cytokine is participated. Another object of the present invention is to provide an inhibitor against release of an inflammatory cytokine which avoids side effects by specifically inhibiting IKK-β, and has inhibitory activity against NF- κB activation.

The inventors of the present invention carried out search for compounds having inhibitory action against NF-κB activation by selective inhibition of IKK-β by using computerized molecular design technology to solve the aforementioned object. Appropriate protein kinases with high homology with IKK-β were selected from the kinases whose structures are registered in PDB (Protein Data Bank), and three-dimensional structure model of IKK-β was constructed by applying the homology modeling technique employing the chosen kinase as a template, and then binding mode of aspirin to the ATP binding region of IKK-β and characteristic intermolecular interactions were analyzed by using automatic search program for binding modes of a drug molecule to a protein. On the basis of the results obtained, an automatic search program of a ligand from a three-dimensional compound database based on the tree-dimensional structure of the protein was carried out, and compounds potentially be specific inhibitors against IKK-β were selected by a virtual screening out of compounds registered in databases of compounds commercially available from suppliers such as Sigma-Aldrich, Aldrich, Maybridge, Specs, Bionet, Labotest, Lancaster, Tocris, Tokyo Kasei Kogyo Co., Wako Pure Chemical Industries and the like. Inhibitory activity of those compounds against NF-κB activation was confirmed by a reporter assay method by a forced expression of Mitogen-activated protein kinase kinase 1 (MEKK-1) which is serine-threonine kinase. Further, inhibitory activity against phosphorylation of I κB (I κB α) was confirmed by the Western blot method under TNF-α stimulation.

It is suggested that MEKK-1 directly phosphorylates and activates IKK-β, when NF-κB is activated under TNF-α stimulation, MEKK-1 is known to be involved in IKK-β activation (Cellular Signaling, (England), 2001, Vol.13, No.5, p.389-400; Trends in Cell Biology, (England), 2001, Vol.11, No.9, p.372-377; Proceedings of The National Academy of Sciences of The United States of America, (USA), 1998, Vol.95, No. 16, p.9319-9324; Proceedings of The National Academy of Sciences of The United States of America, (USA), 1998, Vol.95, No.16, p.9067-9069; Cell, (USA), 1998, Vol.93, No.5, p.875-884). As already mentioned above, it is known that IKK-β directly phosphorylates I κB α and induces decomposition of I κ B. Therefore, it is obvious that the compounds that are recognized to have activities by the above two methods are inhibitors agaist either of MEKK-1 or IKK-β or both. Furthermore, since the compounds of the present invention are designed to be inhibitors targeting ATP binding regions that commonly exist in protein kinase, they may be inhibitors to other protein kinases structurally similar thereto. The inventors synthesized analogous compounds to those compounds whose activities were confirmed by the above two methods, and the present invention was achieved.

The present invention thus provides:
(1) A medicament having an inhibitory activity against IKK-β and/or MEKK-1 or other protein kinases structurally similar thereto which comprises as an active ingredient a substance selected from the group consisting of a compound represented by the following general formula (I) and a pharmacologically acceptable salt thereof, and a hydrate thereof and a solvate thereof: 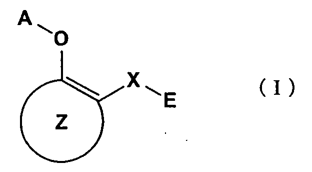 wherein X represents a connecting group whose number of atoms in a main chain is 2 to 5 (said connecting group may be substituted),
   A represents hydrogen atom or acetyl group,
   E represents an aryl group which may be substituted or a heteroaryl group which may be substituted,
   ring Z represents an arene which may have one or more substituents in addition to the group represented by formula -O-A wherein A has the same meaning as that defined above and the group represented by formula -X-E wherein each of X and E has the same meaning as that defined above, or a heteroarene which may have one or more substituents in addition to the group represented by formula -O-A wherein A has the same meaning as that defined above and the group represented by formula -X-E wherein each of X and E has the same meaning as that defined above.
   Examples of preferred medicaments include:
(2) the aforementioned medicament which comprises as an active ingredient a substance selected from the group consisting of the compound and a pharmacologically acceptable salt thereof, and a hydrate thereof and a solvate thereof, wherein X is a group selected from the following connecting group α (said group may be substituted):
   [Connecting group α] The groups of the following formulas: 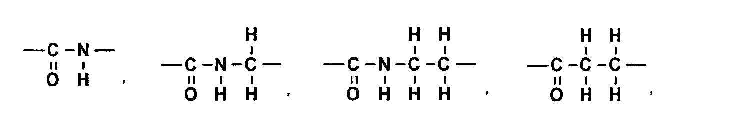 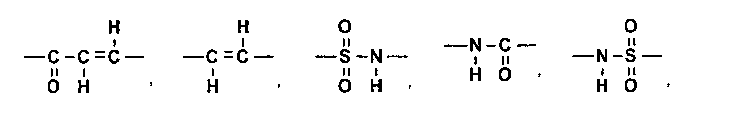 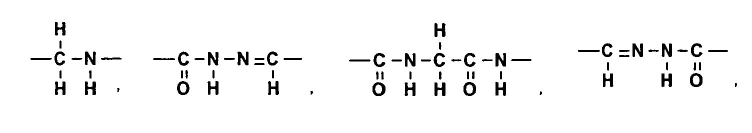 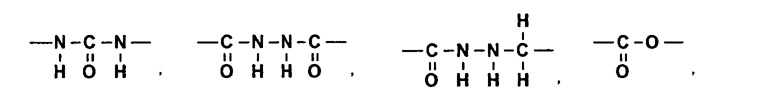 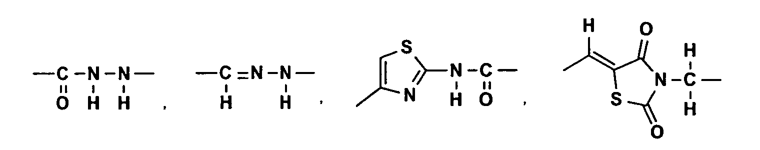 wherein a bond at the left end binds to ring Z and a bond at the right end binds to E;
(3) the aforementioned medicament which comprises as an active ingredient a substance selected from the group consisting of the compound and a pharmacologically acceptable salt thereof, and a hydrate thereof and a solvate thereof, wherein X is a group represented by the following formula (said group may be substituted): 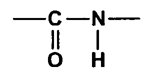 wherein a bond at the left end binds to ring Z and a bond at the right end binds to E;
(4) the aforementioned medicament which comprises as an active ingredient a substance selected from the group consisting of the compound and a pharmacologically acceptable salt thereof, and a hydrate thereof and a solvate thereof, wherein A is a hydrogen atom;
(5) the aforementioned medicament which comprises as an active ingredient a substance selected from the group consisting of the compound and a pharmacologically acceptable salt thereof, and a hydrate thereof and a solvate thereof, wherein ring Z is a C₆ to C₁₀ arene which may have one or more substituents in addition to the group represented by formula -O-A wherein A has the same meaning as that defined in the general formula (I) and the group represented by formula -X-E wherein each of X and E has the same meaning as that defined in the general formula (I), or a 5 to 13-membered heteroarene which may have one or more substituents in addition to the group represented by formula -O-A wherein A has the same meaning as that defined in the general formula (I) and the group represented by formula -X-E wherein each of X and E has the same meaning as that defined in the general formula (I);
(6) the aforementioned medicament which comprises as an active ingredient a substance selected from the group consisting of the compound and a pharmacologically acceptable salt thereof, and a hydrate thereof and a solvate thereof, wherein ring Z is a ring selected from the following ring group β:
   [Ring Group β] benzene ring, naphthalene ring, thiophene ring, pyridine ring, indole ring, quinoxaline ring, and carbazole ring
   wherein said ring may have one or more substituents in addition to the group represented by formula -O-A wherein A has the same meaning as that defined in the general formula(I) and the group represented by formula -X-E wherein each of X and E has the same meaning as that defined in the general formula (I);
(7) the aforementioned medicament which comprises as an active ingredient a substance selected from the group consisting of the compound and a pharmacologically acceptable salt thereof, and a hydrate thereof and a solvate thereof, wherein ring Z is a benzene ring which may have one or more substituents in addition to the group represented by formula -O-A wherein A has the same meaning as that defined in the general formula (I) and the group represented by formula -X-E wherein each of X and E has the same meaning as that defined in the general formula (I);
(8) the aforementioned medicament which comprises as an active ingredient a substance selected from the group consisting of the compound and a pharmacologically acceptable salt thereof, and a hydrate thereof and a solvate thereof, wherein ring Z is a benzene ring which is substituted with halogen atom(s) in addition to the group represented by formula -O-A wherein A has the same meaning as that defined in the general formula (I) and the group represented by formula -X-E wherein each of X and E has the same meaning as that defined in the general formula (I);
(9) the aforementioned medicament which comprises as an active ingredient a substance selected from the group consisting of the compound and a pharmacologically acceptable salt thereof, and a hydrate thereof and a solvate thereof, wherein ring Z is a naphthalene ring which may have one or more substituents in addition to the group represented by formula -O-A wherein A has the same meaning as that defined in the general formula (I) and the group represented by formula -X-E wherein each of X and E has the same meaning as that defined in the general formula (I);
(10) the aforementioned medicament which comprises as an active ingredient a substance selected from the group consisting of the compound and a pharmacologically acceptable salt thereof, and a hydrate thereof and a solvate thereof, wherein E is a C₆ to C₁₀ aryl group which may be substituted or a 5 to 13-membered heteroaryl group which may be substituted;
(11) the aforementioned medicament which comprises as an active ingredient a substance selected from the group consisting of the compound and a pharmacologically acceptable salt thereof, and a hydrate thereof and a solvate thereof, wherein E is a phenyl group which may be substituted;
(12) the aforementioned medicament which comprises as an active ingredient a substance selected from the group consisting of the compound and a pharmacologically acceptable salt thereof, and a hydrate thereof and a solvate thereof, wherein E is 3,5-bis(trifluoromethyl)phenyl group;
(13) the aforementioned medicament which comprises as an active ingredient a substance selected from the group consisting of the compound and a pharmacologically acceptable salt thereof, and a hydrate thereof and a solvate thereof, wherein E is a 5-membered heteroaryl group which may be substituted.

From another aspect, the present invention provides use of each of the aforementioned substances for manufacture of the medicament according to the aforementioned (1) to (13). Moreover, the present invention provides an inhibitor which comprises each of the aforementioned substances against IKK-β and/or MEKK-1 or other protein kinases structurally similar thereto.

The present invention further provides a method for inhibiting IKK-β and/or MEKK-1 or other protein kinases structurally similar thereto in a mammal including a human, which comprises the step of administering the medicament according to the aforementioned (1) to (13) to a mammal including a human.

### Best Mode for Carrying out the Invention

Reference to the disclosure of the pamphlet of International Publication WO02/49632 is useful for better understanding of the present invention. The entire disclosure of the aforementioned pamphlet of International Publication W002/49632 is incorporated by reference in the disclosures of the present specification.

The terms used in the present specification have the following meanings.

As the halogen atom, any of fluorine atom, chlorine atom, bromine atom, or iodine atom may be used unless otherwise specifically referred to.

Examples of the hydrocarbon group include, for example, an aliphatic hydrocarbon group, an aryl group, an arylene group, an aralkyl group, a bridged cyclic hydrocarbon group, a spiro cyclic hydrocarbon group, and a terpene hydrocarbon.

Examples of the aliphatic hydrocarbon group include, for example, alkyl group, alkenyl group, alkynyl group, alkylene group, alkenylene group, alkylidene group and the like which are straight chain or branched chain monovalent or bivalent acyclic hydrocarbon groups; cycloalkyl group, cycloalkenyl group, cycloalkanedienyl group, cycloalkyl-alkyl group, cycloalkylene group, and cycloalkenylene group, which are saturated or unsaturated monovalent or bivalent alicyclic hydrocarbon groups.

Examples of the alkyl group include, for example, methyl, ethyl, n-propyl, isopropyl, n-butyl, isobutyl, sec-butyl, tert-butyl, n-pentyl, isopentyl, 2-methylbutyl, 1-methylbutyl, neopentyl, 1,2-dimethylpropyl, 1-ethylpropyl, n-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-ethylbutyl, 1-ethyl-1-methylpropyl, n-heptyl, n-octyl, n-nonyl, n-decyl, n-undecyl, n-dodecyl, n-tridecyl, n-tetradecyl, and n-pentadecyl, which are C₁ to C₁₅ straight chain or branched chain alkyl groups.

Examples of the alkenyl group include, for example, vinyl, prop-1-en-1-yl, allyl, isopropenyl, but-1-en-1-yl, but-2-en-1-yl, but-3-en-1-yl, 2-methylprop-2-en-1-yl, 1-methylprop-2-en-1-yl, pent-1-en-1-yl, pent-2-en-1-yl, pent-3-en-1-yl, pent-4-en-1-yl, 3-methylbut-2-en-1-yl, 3-methylbut-3-en-1-yl, hex-1-en-1-yl, hex-2-en-1-yl, hex-3-en-1-yl, hex-4-en-1-yl, hex-5-en-1-yl, 4-methylpent-3-en-1-yl, 4-methylpent-3-en-1-yl, hept-1-en-1-yl, hept-6-en-1-yl, oct-1-en-1-yl, oct-7-en-1-yl, non-1-en-1-yl, non-8-en-1-yl, dec-1-en-1-yl, dec-9-en-1-yl, undec-1-en-1-yl, undec-10-en-1-yl, dodec-1-en-1-yl, dodec-11-en-1-yl, tridec-1-en-1-yl, tridec-12-en-1-yl, tetradec-1-en-1-yl, tetradec-13-en-1-yl, pentadec-1-en-1-yl, and pentadec-14-en-1-yl, which are C₂ to C₁₅ straight chain or branched chain alkenyl groups.

Examples of the alkynyl group include, for example, ethynyl, prop-1-yn-1-yl, prop-2-yn-1-yl, but-1-yn-1-yl, but-3-yn-1-yl, 1-methylprop-2-yn-1-yl, pent-1-yn-1-yl, pent-4-yn-1-yl, hex-1-yn-1-yl, hex-5-yn-1-yl, hept-1-yn-1-yl, hept-6-yn-1-yl, oct-1-yn-1-yl, oct-7-yn-1-yl, non-1-yn-1-yl, non-8-yn-1-yl, dec-1-yn-1-yl, dec-9-yn-1-yl, undec-1-yn-1-yl, undec-10-yn-1-yl, dodec-1-yn-1-yl, dodec-11-yn-1-yl, tridec-1-yn-1-yl, tridec-12-yn-1-yl, tetradec-1-yn-1-yl, tetradec-13-yn-1-yl, pentadec-1-yn-1-yl, and pentadec-14-yn-1-yl, which are C₂ to C₁₅ straight chain or branched chain alkynyl groups.

Examples of the alkylene group include, for example, methylene, ethylene, ethane-1,1-diyl, propane-1,3-diyl, propane-1,2-diyl, propane-2,2-diyl, butane-1,4-diyl, pentane-1,5-diyl, hexane-1,6-diyl, and 1,1,4,4-tetramethylbutane-1,4-diyl group, which are C₁ to C₈ straight chain or branched chain alkylene groups.

Examples of the alkenylene group include, for example, ethene-1,2-diyl, propene-1,3-diyl, but-1-ene-1,4-diyl, but-2-ene-1,4-diyl, 2-methylpropene-1,3-diyl, pent-2-ene-1,5-diyl, and hex-3-ene-1,6-diyl, which are C₁ to C₆ straight chain or branched chain alkylene groups.

Examples of the alkylidene group include, for example, methylidene, ethylidene, propylidene, isopropylidene, butylidene, pentylidene, and hexylidene, which are C₁ to C₆ straight chain or branched chain alkylidene groups.

Examples of the cycloalkyl group include, for example, cyclopropyl, cyclobutyl, cyclopentyl, cyclohexyl, cycloheptyl, and cyclooctyl, which are C₃ to C₈ cycloalkyl groups.

The aforementioned cycloalkyl group may be fused with benzene ring, naphthalene ring and the like, and examples include, for example, 1-indanyl, 2-indanyl, 1,2,3,4-tetrahydronaphthalen-1-yl, and 1,2,3,4-tetrahydronaphthalen-2-yl.

Examples of the cycloalkenyl group include, for example, 2-cyclopropen-1-yl, 2-cyclobuten-1-yl, 2-cyclopenten-1-yl, 3-cyclopenten-1-yl, 2-cyclohexen-1-yl, 3-cyclohexen-1-yl, 1-cyclobuten-1-yl, and 1-cyclopenten-1-yl, which are C₃ to C₆ cycloalkenyl groups.

The aforementioned cycloalkenyl group may be fused with benzene ring, naphthalene ring and the like, and examples include, for example, 1-indanyl, 2-indanyl, 1,2,3,4-tetrahydronaphthalen-1-yl, 1,2,3,4-tetrahydronaphthalen-2-yl, 1-indenyl, and 2-indenyl.

Examples of the cycloalkanedienyl group include, for example, 2,4-cyclopentadien-1-yl, 2,4-cyclohexanedien-1-yl, and 2,5-cyclohexanedien-1-yl, which are C₅ to C₆ cycloalkanedienyl groups.

The aforementioned cycloalkanedienyl group may be fused with benzene ring, naphthalene ring and the like, and examples include, for example, 1-indenyl and 2-indenyl.

Examples of the cycloalkyl-alkyl group include the groups in which one hydrogen atom of the alkyl group is substituted with a cycloalkyl group, and include, for example, cyclopropylmethyl, 1-cyclopropylethyl, 2-cyclopropylethyl, 3-cyclopropylpropyl, 4-cyclopropylbutyl, 5-cyclopropylpentyl, 6-cyclopropylhexyl, cyclobutylmethyl, cyclopentylmethyl, cyclobutylmethyl, cyclopentylmethyl, cyclohexylmethyl, cyclohexylpropyl, cyclohexylbutyl, cycloheptylmethyl, cyclooctylmethyl, and 6-cyclooctylhexyl, which are C₄ to C₁₄ cycloalkyl-alkyl groups.

Examples of the cycloalkylene group include, for example, cyclopropane-1,1-diyl, cyclopropane-1,2-diyl, cyclobutane-1,1-diyl, cyclobutane-1,2-diyl, cyclobutane-1,3-diyl, cyclopentane-1,1-diyl, cyclopentane-1,2-diyl, cyclopentane-1,3-diyl, cyclohexane-1,1-diyl, cyclohexane-1,2-diyl, cyclohexane-1,3-diyl, cyclohexane-1,4-diyl, cycloheptane-1,1-diyl, cycloheptane-1,2-diyl, cyclooctane-1,1-diyl, and cyclooctane-1,2-diyl, which are C₃ to C₈ cycloalkylene groups.

Examples of the cycloalkenylene group include, for example, 2-cyclopropene-1,1-diyl, 2-cyclobutene-1,1-diyl, 2-cyclopentene-1,1-diyl, 3-cyclopentene-1,1-diyl, 2-cyclohexene-1,1-diyl, 2-cyclohexene-1,2-diyl, 2-cyclohexene-1,4-diyl, 3-cyclohexene-1,1-diyl, 1-cyclobutene-1,2-diyl, 1-cyclopentene-1,2-diyl, and 1-cyclohexene-1,2-diyl, which are C₃ to C₆ cycloalkenylene groups.

Examples of the aryl group include a monocyclic or a fused polycyclic aromatic hydrocarbon group, and include, for example, phenyl, 1-naphthyl, 2-naphthyl, anthryl, phenanthryl, and acenaphthylenyl, which are C₆ to C₁₄ aryl groups.

The aforementioned aryl group may be fused with the aforementioned C₃ to C₈ cycloalkyl group, C₃ to C₆ cycloalkenyl group, C₅ to C₆ cycloalkanedienyl group or the like, and examples include, for example, 4-indanyl, 5-indanyl, 1,2,3,4-tetrahydronaphthalen-5-yl, 1,2,3,4-tetrahydronaphthalen-6-yl, 3-acenaphthenyl, 4-acenaphthenyl, inden-4-yl, inden-5-yl, inden-6-yl, inden-7-yl, 4-phenalenyl, 5-phenalenyl, 6-phenalenyl, 7-phenalenyl, 8-phenalenyl, and 9-phenalenyl.

Examples of the arylene group include, for example, 1,2-phenylene, 1,3-phenylene, 1,4-phenylene, naphthalene-1,2-diyl, naphthalene-1,3-diyl, naphthalene-1,4-diyl, naphthalene-1,5-diyl, naphthalene-1,6-diyl, , naphthalene-1,7-diyl, naphthalene-1,8-diyl, naphthalene-2,3-diyl, naphthalene-2,4-diyl, naphthalene-2,5-diyl, naphthalene-2,6-diyl, naphthalene-2,7-diyl, naphthalene-2,8-diyl, and anthracene-1,4-diyl, which are C₆ to C₁₄ arylene groups.

Examples of the aralkyl group include the groups in which one hydrogen atom of the alkyl group is substituted with an aryl group, and include, for example, benzyl, 1-naphthylmethyl, 2-naphthylmethyl, anthracenylmethyl, phenanthrenylmethyl, acenaphthylenylmethyl, diphenylmethyl, 1-phenethyl, 2-phenethyl, 1-(1-naphthyl)ethyl, 1-(2-naphthyl)ethyl, 2-(1-naphthyl)ethyl, 2-(2-naphthyl)ethyl, 3-phenylpropyl, 3-(1-naphthyl)propyl, 3-(2-naphthyl)propyl, 4-phenylbutyl, 4-(1-naphthyl)butyl, 4-(2-naphthyl)butyl, 5-phenylpentyl, 5-(1-naphthyl)pentyl, 5-(2-naphthyl)pentyl, 6-phenylhexyl, 6-(1-naphthyl)hexyl, and 6-(2-naphthyl)hexyl, which are C₇ to C₁₆ aralkyl groups.

Examples of the bridged cyclic hydrocarbon group include, for example, bicyclo[2.1.0]pentyl, bicyclo[2.2.1]heptyl, bicyclo[2.2.1]octyl, and adamantyl.

Examples of the spiro cyclic hydrocarbon group include, for example, spiro[3.4]octyl, and spiro[4.5]deca-1,6-dienyl.

Examples of the terpene hydrocarbon include, for example, geranyl, neryl, linalyl, phytyl, menthyl, and bornyl.

Examples of the halogenated alkyl group include the groups in which one hydrogen atom of the alkyl group is substituted with a halogen atom, and include, for example, fluoromethyl, difluoromethyl, trifluoromethyl, chloromethyl, dichloromethyl, trichloromethyl, bromomethyl, dibromomethyl, tribromomethyl, iodomethyl, diiodomethyl, triiodomethyl, 2,2,2-trifluoroethyl, pentafluoroethyl, 3,3,3-trifluoropropyl, heptafluoropropyl, heptafluoroisopropyl, nonafluorobutyl, and perfluorohexyl, which are C₁ to C₆ straight chain or branched chain halogenated alkyl groups substituted with 1 to 13 halogen atoms.

Examples of the heterocyclic group include, for example, a monocyclic or a fused polycyclic hetero aryl group which comprises at least one atom of 1 to 3 kinds of hetero atoms selected from oxygen atom, sulfur atom, nitrogen atom and the like as ring-constituting atoms (ring forming atoms), and a monocyclic or a fused polycyclic non-aromatic heterocyclic group which comprises at least one atom of 1 to 3 kinds of hetero atoms selected from oxygen atom, sulfur atom, nitrogen atom and the like as ring-constituting atoms (ring forming atoms).

Examples of the monocyclic heteroaryl group include, for example, 2-furyl, 3-furyl, 2-thienyl, 3-thienyl, 1-pyrrolyl, 2-pyrrolyl, 3-pyrrolyl, 2-oxazolyl, 4-oxazolyl, 5-oxazolyl, 3-isoxazolyl, 4-isoxazolyl, 5-isoxazolyl, 2-thiazolyl, 4-thiazolyl, 5-thiazolyl, 3-isothiazolyl, 4-isothiazolyl, 5-isothiazolyl, 1-imidazolyl, 2-imidazolyl, 4-imidazolyl, 5-imidazolyl, 1-pyrazolyl, 3-pyrazolyl, 4-pyrazolyl, 5-pyrazolyl, (1,2,3-oxadiazol)-4-yl, (1,2,3-oxadiazol)-5-yl, (1,2,4-oxadiazol)-3-yl, (1,2,4-oxadiazol)-5-yl, (1,2,5-oxadiazol)-3-yl, (1,2,5-oxadiazol)-4-yl, (1,3,4-oxadiazol)-2-yl, (1,3,4-oxadiazol)-5-yl, furazanyl, (1,2,3-thiadiazol)-4-yl, (1,2,3-thiadiazol)-5-yl, (1,2,4-thiadiazol)-3-yl, (1,2,4-thiadiazol)-5-yl, (1,2,5-thiadiazol)-3-yl, (1,2,5-thiadiazol)-4-yl, (1,3,4-thiadiazolyl)-2-yl, (1,3,4-thiadiazolyl)-5-yl, (1H-1,2,3-triazol)-1-yl, (1H-1,2,3-triazol)-4-yl, (1H-1,2,3-triazol)-5-yl, (2H-1,2,3-triazol)-2-yl, (2H-1,2,3-triazol)-4-yl, (1H-1,2,4-triazol)-1-yl, (1H-1,2,4-triazol)-3-yl, (1H-1,2,4-triazol)-5-yl, (4H-1,2,4-triazol)-3-yl, (4H-1,2,4-triazol)-4-yl, (1H-tetrazol)-1-yl, (1H-tetrazol)-5-yl, (2H-tetrazol)-2-yl, (2H-tetrazol)-5-yl, 2-pyridyl, 3-pyridyl, 4-pyridyl, 3-pyridazinyl, 4-pyridazinyl, 2-pyrimidinyl, 4-pyrimidinyl, 5-pyrimidinyl, 2-pyrazinyl, (1,2,3-triazin)-4-yl, (1,2,3-triazin)-5-yl, (1,2,4-triazin)-3-yl, (1,2,4-triazin)-5-yl, (1,2,4-triazin)-6-yl, (1,3,5-triazin)-2-yl, 1-azepinyl, 2-azepinyl, 3-azepinyl, 4-azepinyl, (1,4-oxazepin)-2-yl, (1,4-oxazepin)-3-yl, (1,4-oxazepin)-5-yl, (1,4-oxazepin)-6-yl, (1,4-oxazepin)-7-yl, (1,4-thiazepin)-2-yl, (1,4-thiazepin)-3-yl, (1,4-thiazepin)-5-yl, (1,4-thiazepin)-6-yl, and (1,4-thiazepin)-7-yl, which are 5 to 7-membered monocyclic heteroaryl groups.

Examples of the fused polycyclic heteroaryl group include, for example, 2-benzofuranyl, 3-benzofuranyl, 4-benzofuranyl, 5-benzofuranyl, 6-benzofuranyl, 7-benzofuranyl, 1-isobenzofuranyl, 4-isobenzofuranyl, 5-isobenzofuranyl, 2-benzo[b]thienyl, 3-benzo[b]thienyl, 4-benzo[b]thienyl, 5-benzo[b]thienyl, 6-benzo[b]thienyl, 7-benzo[b]thienyl, 1-benzo[c]thienyl, 4-benzo[c]thienyl, 5-benzo[c]thienyl, 1-indolyl, 1-indolyl, 2-indolyl, 3-indolyl, 4-indolyl, 5-indolyl, 6-indolyl, 7-indolyl, (2H-isoindol)-1-yl, (2H-isoindol)-2-yl, (2H-isoindol)-4-yl, (2H-isoindol)-5-yl, (1H-indazol)-1-yl, (1H-indazol)-3-yl, (1H-indazol)-4-yl, (1H-indazol)-5-yl, (1H-indazol)-6-yl, (1H-indazol)-7-yl, (2H-indazol)-1-yl, (2H-indazol)-2-yl, (2H-indazol)-4-yl, (2H-indazol)-5-yl, 2-benzoxazolyl, 2-benzoxazolyl, 4-benzoxazolyl, 5-benzoxazolyl, 6-benzoxazolyl, 7-benzoxazolyl, (1,2-benzisoxazol)-3-yl, (1,2-benzisoxazol)-4-yl, (1,2-benzisoxazol)-5-yl, (1,2-benzisoxazol)-6-yl, (1,2-benzisoxazol)-7-yl, (2,1-benzisoxazol)-3-yl, (2,1-benzisoxazol)-4-yl, (2,1-benzisoxazol)-5-yl, (2,1-benzisoxazol)-6-yl, (2,1-benzisoxazol)-7-yl, 2-benzothiazolyl, 4-benzothiazolyl, 5-benzothiazolyl, 6-benzothiazolyl, 7-benzothiazolyl, (1,2-benzisothiazol)-3-yl, (1,2-benzisothiazol)-4-yl, (1,2-benzisothiazol)-5-yl, (1,2-benzisothiazol)-6-yl, (1,2-benzisothiazol)-7-yl, (2,1-benzisothiazol)-3-yl, (2,1-benzisothiazol)-4-yl, (2,1-benzisothiazol)-5-yl, (2,1-benzisothiazol)-6-yl, (2,1-benzisothiazol)-7-yl, (1,2,3-benzoxadiazol)-4-yl, (1,2,3-benzoxadiazol)-5-yl, (1,2,3-benzoxadiazol)-6-yl, (1,2,3-benzoxadiazol)-7-yl, (2,1,3-benzoxadiazol)-4-yl, (2,1,3-benzoxadiazol)-5-yl, (1,2,3-benzothiadiazol)-4-yl, (1,2,3-benzothiadiazol)-5-yl, (1,2,3-benzothiadiazol)-6-yl, (1,2,3-benzothiadiazol)-7-yl, (2,1,3-benzothiadiazol)-4-yl, (2,1,3-benzothiadiazol)-5-yl, (1H-benzotriazol)-1-yl, (1H-benzotriazol)-4-yl, (1H-benzotriazol)-5-yl, (1H-benzotriazol)-6-yl, (1H-benzotriazol)-7-yl, (2H-benzotriazol)-2-yl, (2H-benzotriazol)-4-yl, (2H-benzotriazol)-5-yl, 2-quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 3-cinnolinyl, 4-cinnolinyl, 5-cinnolinyl, 6-cinnolinyl, 7-cinnolinyl, 8-cinnolinyl, 2-quinazolinyl, 4-quinazolinyl, 5-quinazolinyl, 6-quinazolinyl, 7-quinazolinyl, 8-quinazolinyl, 2-quinoxalinyl, 5-quinoxalinyl, 6-quinoxalinyl, 1-phthalazinyl, 5-phthalazinyl, 6-phthalazinyl, 2-naphthyridinyl, 3-naphthyridinyl, 4-naphthyridinyl, 2-purinyl, 6-purinyl, 7-purinyl, 8-purinyl, 2-pteridinyl, 4-pteridinyl, 6-pteridinyl, 7-pteridinyl, 1-carbazolyl, 2-carbazolyl, 3-carbazolyl, 4-carbazolyl, 9-carbazolyl, 2-(α-carbolinyl), 3-(α-carbolinyl), 4-(α -carbolinyl), 5-(α-carbolinyl), 6-(α-carbolinyl), 7-(α-carbolinyl), 8-(α-carbolinyl), 9-(α-carbolinyl), 1-(β-carbolinyl), 3-(β-carbolinyl), 4-(β-carbolinyl), 5-(β-carbolinyl), 6-(β-carbolinyl), 7-(β-carbolinyl), 8-(β-carbolinyl), 9-(β-carbolinyl), 1-(γ-carbolinyl), 2-(γ-carbolinyl), 4-(γ-carbolinyl), 5-(γ-carbolinyl), 6-(γ-carbolinyl), 7-(γ-carbolinyl), 8-(γ-carbolinyl), 9-(γ-carbolinyl), 1-acridinyl, 2-acridinyl, 3-acridinyl, 4-acridinyl, 9-acridinyl, 1-phenoxazinyl, 2-phenoxazinyl, 3-phenoxazinyl, 4-phenoxazinyl, 10-phenoxazinyl, 1-phenothiazinyl, 2-phenothiazinyl, 3-phenothiazinyl, 4-phenothiazinyl, 10-phenothiazinyl, 1-phenazinyl, 2-phenazinyl, 1-phenanthridinyl, 2-phenanthridinyl, 3-phenanthridinyl, 4-phenanthridinyl, 6-phenanthridinyl, 7-phenanthridinyl, 8-phenanthridinyl, 9-phenanthridinyl, 10-phenanthridinyl, 2-phenanthrolinyl, 3-phenanthrolinyl, 4-phenanthrolinyl, 5-phenanthrolinyl, 6-phenanthrolinyl, 7-phenanthrolinyl, 8-phenanthrolinyl, 9-phenanthrolinyl, 10-phenanthrolinyl, 1-thianthrenyl, 2-thianthrenyl, 1-indolizinyl, 2-indolizinyl, 3-indolizinyl, 5-indolizinyl, 6-indolizinyl, 7-indolizinyl, 8-indolizinyl, 1-phenoxathiinyl, 2-phenoxathiinyl, 3-phenoxathiinyl, 4-phenoxathiinyl, thieno[2,3-b]furyl, pyrrolo[1,2-b]pyridazinyl, pyrazolo[1,5-a]pyridyl, imidazo[11,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, and 1,2,4-triazolo[4,3-a]pyridazinyl, which are 8 to 14-membered fused polycyclic heteroaryl groups.

Examples of the monocyclic non-aromatic heterocyclic group include, for example, 1-aziridinyl, 1-azetidinyl, 1-pyrrolidinyl, 2-pyrrolidinyl, 3-pyrrolidinyl, 2-tetrahydrofuryl, 3-tetrahydrofuryl, thiolanyl, 1-imidazolidinyl, 2-imidazolidinyl, 4-imidazolidinyl, 1-pyrazolidinyl, 3-pyrazolidinyl, 4-pyrazolidinyl, 1-(2-pyrrolinyl), 1-(2-imidazolinyl), 2-(2-imidazolinyl), 1-(2-pyrazolinyl), 3-(2-pyrazolinyl), piperidino, 2-piperidinyl, 3-piperidinyl, 4-piperidinyl, 1-homopiperidinyl, 2-tetrahydropyranyl, morpholino, (thiomorpholin)-4-yl, 1-piperazinyl, and 1-homopiperazinyl, which are 3 to 7-membered saturated or unsaturated monocyclic non-aromatic heterocyclic groups.

Examples of the fused polycyclic non-aromatic heterocyclic group include, for example, 2-quinuclidinyl, 2-chromanyl, 3-chromanyl, 4-chromanyl, 5-chromanyl, 6-chromanyl, 7-chromanyl, 8-chromanyl, 1-isochromanyl, 3-isochromanyl, 4-isochromanyl, 5-isochromanyl, 6-isochromanyl, 7-isochromanyl, 8-isochromanyl, 2-thiochromanyl, 3-thiochromanyl, 4-thiochromanyl, 5-thiochromanyl, 6-thiochromanyl, 7-thiochromanyl, 8-thiochromanyl, 1-isothiochromanyl, 3-isothiochromanyl, 4-isothiochromanyl, 5-isothiochromanyl, 6-isothiochromanyl, 7-isothiochromanyl, 8-isothiochromanyl, 1-indolinyl, 2-indolinyl, 3-indolinyl, 4-indolinyl, 5-indolinyl, 6-indolinyl, 7-indolinyl, 1-isoindolinyl, 2-isoindolinyl, 4-isoindolinyl, 5-isoindolinyl, 2-(4H-chromenyl), 3-(4H-chromenyl), 4-(4H-chromenyl), 5-(4H-chromenyl), 6-(4H-chromenyl), 7-(4H-chromenyl), 8-(4H-chromenyl), 1-isochromenyl, 3-isochromenyl, 4-isochromenyl, 5-isochromenyl, 6-isochromenyl, 7-isochromenyl, 8-isochromenyl, 1-(1H-pyrrolidinyl), 2-(1H-pyrrolidinyl), 3-(1H-pyrrolidinyl), 5-(1H-pyrrolidinyl), 6-(1H-pyrrolidinyl), and 7-(1H-pyrrolidinyl), which are 8 to 10-membered saturated or unsaturated fused polycyclic non-aromatic heterocyclic groups.

Among the aforementioned heterocyclic groups, a monocyclic or a fused polycyclic hetero aryl groups which may have 1 to 3 kinds of hetero atoms selected from oxygen atom, sulfur atom, nitrogen atom and the like, in addition to the nitrogen atom that has the bond, as ring-constituting atoms (ring forming atoms), and a monocyclic or a fused polycyclic non-aromatic heterocyclic groups which may have 1 to 3 kinds of hetero atoms selected from oxygen atom, sulfur atom, nitrogen atom and the like, in addition to the nitrogen atom that has the bond, as ring-constituting atoms (ring forming atoms) are referred to as "cyclic amino group." Examples include, for example, 1-pyrrolidinyl, 1-imidazolidinyl, 1-pyrazolidinyl, 1-oxazolidinyl, 1-thiazolidinyl, piperidino, morpholino, 1-piperazinyl, thiomorpholin-4-yl, 1-homopiperidinyl, 1-homopiperazinyl, 2-pyrolin-1-yl, 2-imidazolin-1-yl, 2-pyrazolin-1-yl, 1-indolinyl, 2-isoindolinyl, 1,2,3,4-tetrahydroquinolin-1-yl, 1,2,3,4-tetrahydroisoquinolin-2-yl, 1-pyrrolyl, 1-imidazolyl, 1-pyrazolyl, 1-indolyl, 1-indazolyl, and 2-isoindolyl.

The aforementioned cycloalkyl group, cycloalkenyl group, cycloalkanedienyl group, aryl group, cycloalkylene group, cycloalkenylene group, arylene group, bridged cyclic hydrocarbon group, spiro cyclic hydrocarbon group, and heterocyclic group are generically referred to as "cyclic group." Furthermore, among the said cyclic groups, particularly, aryl group, arylene group, monocyclic heteroaryl group, and fused polycyclic heteroaryl group are generically referred to as "aromatic ring group."

Examples of the hydrocarbon-oxy group include the groups in which a hydrogen atom of the hydroxy group is substituted with a hydrocarbon group, and examples of the hydrocarbon include similar groups to the aforementioned hydrocarbon groups. Examples of the hydrocarbon-oxy group include, for example, alkoxy group (alkyl-oxy group), alkenyl-oxy group, alkynyl-oxy group, cycloalkyl-oxy group, cycloalkyl-alkyl-oxy group and the like, which are aliphatic hydrocarbon-oxy groups; aryl-oxy group; aralkyl-oxy group; and alkylene-dioxy group.

Examples of the alkoxy (alkyl-oxy group) include, for example, methoxy, ethoxy, n-propoxy, isopropoxy, n-butoxy, isobutoxy, sec-butoxy, tert-butoxy, n-pentyloxy, isopentyloxy, 2-methylbutoxy, 1-methylbutoxy, neopentyloxy, 1,2-dimethylpropoxy, 1-ethylpropoxy, n-hexyloxy, 4-methylpentyloxy, 3-methylpentyloxy, 2-methylpentyloxy, 1-methylpentyloxy, 3,3-dimethylbutoxy, 2,2-dimethybutoxy, 1,1-dimethylbutoxy, 1,2-dimethylbutoxy, 1,3-dimethylbutoxy, 2,3-dimethylbutoxy, 2-ethylbutoxy, 1-ethylbutoxy, 1-ethyl-1-methylpropoxy, n-heptyloxy, n-octyloxy, n-nonyloxy, n-decyloxy, n-undecyloxy, n-dodecyloxy, n-tridecyloxy, n-tetradecyloxy, and n-pentadecyloxy, which are C₁ to C₁₅ straight chain or branched chain alkoxy groups.

Examples of the alkenyl-oxy group include, for example, vinyloxy, (prop-1-en-1-yl)oxy, allyloxy, isopropenyloxy, (but-1-en-1-yl)oxy, (but-2-en-1-yl)oxy, (but-3-en-1-yl)oxy, (2-methylprop-2-en-1-yl)oxy, (1-methylprop-2-en-1-yl)oxy, (pent-1-en-1-yl)oxy, (pent-2-en-1-yl)oxy, (pent-3-en-1-yl)oxy, (pent-4-en-1-yl)oxy, (3-methylbut-2-en-1-yl)oxy, (3-methylbut-3-en-1-yl)oxy, (hex-1-en-1-yl)oxy, (hex-2-en-1-yl)oxy, (hex-3-en-1-yl)oxy, (hex-4-en-1-yl)oxy, (hex-5-en-1-yl)oxy, (4-methylpent-3-en-1-yl)oxy, (4-methylpent-3-en-1-yl)oxy, (hept-1-en-1-yl)oxy, (hept-6-en-1-yl)oxy, (oct-1-en-1-yl)oxy, (oct-7-en-1-yl)oxy, (non-1-en-1-yl)oxy, (non-8-en-1-yl)oxy, (dec-1-en-1-yl)oxy, (dec-9-en-1-yl)oxy, (undec-1-en-1-yl)oxy, (undec-10-en-1-yl)oxy, (dodec-1-en-1-yl)oxy, (dodec-11-en-1-yl)oxy, (tridec-1-en-1-yl)oxy, (tridec-12-en-1-yl)oxy, (tetradec-1-en-1-yl)oxy, (tetradec-13-en-1-yl)oxy, (pentadec-1-en-1-yl)oxy, and (pentadec-14-en-1-yl)oxy, which are C₂ to C₁₅ straight chain or branched chain alkenyl-oxy groups.

Examples of the alkynyl-oxy group include, for example, ethynyloxy, (prop-1-yn-1-yl)oxy, (prop-2-yn-1-yl)oxy, (but-1-yn-1-yl)oxy, (but-3-yn-1-yl)oxy, (1-methylprop-2-yn-1-yl)oxy, (pent-1-yn-1-yl)oxy, (pent-4-yn-1-yl)oxy, (hex-1-yn-1-yl)oxy, (hex-5-yn-1-yl)oxy, (hept-1-yn-1-yl)oxy, (hept-6-yn-1-yl)oxy, (oct-1-yn-1-yl)oxy, (oct-7-yn-1-yl)oxy, (non-1-yn-1-yl)oxy, (non-8-yn-1-yl)oxy, (dec-1-yn-1-yl)oxy, (dec-9-yn-1-yl)oxy, (undec-1-yn-1-yl)oxy, (undec-10-yn-1-yl)oxy, (dodec-1-yn-1-yl)oxy, (dodec-11-yn-1-yl)oxy, (tridec-1-yn-1-yl)oxy, (tridec-12-yn-1-yl)oxy, (tetradec-1-yn-1-yl)oxy, (tetradec-13-yn-1-yl)oxy, (pentadec-1-yn-1-yl)oxy, and (pentadec-14-yn-1-yl)oxy, which are C₂ to C₁₅ straight chain or branched chain alkynyl-oxy groups.

Examples of the cycloalkyl-oxy group include, for example, cyclopropoxy, cyclobutoxy, cyclopentyloxy, cyclohexyloxy, cycloheptyloxy, and cyclooctyloxy, which are C₃ to C₈ cycloalkyl-oxy groups.

Examples of the cycloalkyl-alkyl-oxy group include, for example, cyclopropylmethoxy, 1-cyclopropylethoxy, 2-cyclopropylethoxy, 3-cyclopropylpropoxy, 4-cyclopropylbutoxy, 5-cyclopropylpentyloxy, 6-cyclopropylhexyloxy, cyclobutylmethoxy, cyclopentylmethoxy, cyclobutylmethoxy, cyclopentylmethoxy, cyclohexylmethoxy, 2-cyclohexylethoxy, 3-cyclohexylpropoxy, 4-cyclohexylbutoxy, cycloheptylmethoxy, cyclooctylmethoxy, and 6-cyclooctylhexyloxy, which are C₄ to C₁₄ cycloalkyl-alkyl-oxy groups.

Examples of the aryl-oxy group include, for example, phenoxy, 1-naphthyloxy, 2-naphthyloxy, anthryloxy, phenanthryloxy, and acenaphthylenyloxy, which are C₆ to C₁₄ aryl-oxy groups.

Examples of the aralkyl-oxy group include, for example, benzyloxy, 1-naphthylmethoxy, 2-naphthylmethoxy, anthracenylmethoxy, phenanthrenylmethoxy, acenaphthylenylmethoxy, diphenylmethoxy, 1-phenethyloxy, 2-phenethyloxy, 1-(1-naphthyl)ethoxy, 1-(2-naphthyl)ethoxy, 2-(1-naphthyl)ethoxy, 2-(2-naphthyl)ethoxy, 3-phenylpropoxy, 3-(1-naphthyl)propoxy, 3-(2-naphthyl)propoxy, 4-phenylbutoxy, 4-(1-naphthyl)butoxy, 4-(2-naphthyl)butoxy, 5-phenylpentyloxy, 5-(1-naphthyl)pentyloxy, 5-(2-naphthyl)pentyloxy, 6-phenylhexyloxy, 6-(1-naphthyl)hexyloxy, and 6-(2-naphthyl)hexyloxy, which are C₇ to C₁₆ aralkyl-oxy groups.

Examples of the alkylenedioxy group include, for example, methylenedioxy, ethylenedioxy, 1-methylmethylenedioxy, and 1,1-dimethylmethylenedioxy.

Examples of the halogenated alkoxy group (halogenated alkyl-oxy group) include the groups in which a hydrogen atom of the hydroxy group is substituted with a halogenated alkyl group, and include, for example, fluoromethoxy, difluoromethoxy, chloromethoxy, bromomethoxy, iodomethoxy, trifluoromethoxy, trichloromethoxy, 2,2,2-trifluoroethoxy, pentafluoroethoxy, 3,3,3-trifluoropropoxy, heptafluoropropoxy, heptafluoroisopropoxy, nonafluorobutoxy, and perfluorohexyloxy, which are C₁ to C₆ straight chain or branched chain halogenated alkoxy groups substituted with 1 to 13 halogen atoms.

Examples of the heterocyclic-oxy group include the groups in which a hydrogen atom of the hydroxy group is substituted with a heterocyclic group, and examples of the heterocyclic ring include similar groups to the aforementioned heterocyclic groups. Examples of the heterocyclic-oxy group include, for example, a monocyclic heteroaryl-oxy group, a fused polycyclic heteroaryl-oxy group, a monocyclic non-aromatic heterocyclic-oxy group, and a fused polycyclic non-aromatic heterocyclic-oxy group.

Examples of the monocyclic heteroaryl-oxy group include, for example, 3-thienyloxy, (isoxazol-3-yl)oxy, (thiazol-4-yl)oxy, 2-pyridyloxy, 3-pyridyloxy, 4-pyridyloxy, and (pyrimidin-4-yl)oxy.

Examples of the fused polycyclic heteroaryl-oxy group include, for example, 5-indolyloxy, (benzimidazol-2-yl)oxy, 2-quinolyloxy, 3-quinolyloxy, and 4-quinolyloxy.

Examples of the monocyclic non-aromatic heterocyclic-oxy group include, for example, 3-pyrrolidinyloxy, and 4-piperidinyloxy.

Examples of the fused polycyclic non-aromatic heterocyclic-oxy group include, for example, 3-indolynyloxy, and 4-chromanyloxy.

Examples of the hydrocarbon-sulfanyl group include the groups in which a hydrogen atom of the sulfanyl group is substituted with a hydrocarbon group, and examples of the hydrocarbon include similar groups to the aforementioned hydrocarbon groups. Examples of the hydrocarbon-sulfanyl groups include, for example, alkyl-sulfanyl group, alkenyl-sulfanyl group, alkynyl-sulfanyl group, cycloalkyl-sulfanyl group, cycloalkyl-alkyl-sulfanyl group and the like, which are aliphatic hydrocarbon-sulfanyl groups; aryl-sulfanyl group, and aralkyl-sulfanyl group.

Examples of the alkyl-sulfanyl group include, for example, methylsulfanyl, ethylsulfanyl, n-propylsulfanyl, isopropylsulfanyl, n-butylsulfanyl, isobutylsulfanyl, sec-butylsulfanyl, tert-butylsulfanyl, n-pentylsulfanyl, isopentylsulfanyl, (2-methylbutyl)sulfanyl, (1-methylbutyl)sulfanyl, neopentylsulfanyl, (1,2-dimethylpropyl)sulfanyl, (1-ethylpropyl)sulfanyl, n-hexylsulfanyl, (4-methylpentyl)sulfanyl, (3-methylpentyl)sulfanyl, (2-methylpentyl)sulfanyl, (1-methylpentyl)sulfanyl, (3,3-dimethylbutyl)sulfanyl, (2,2-dimethylbutyl)sulfanyl, (1,1-dimethylbutyl)sulfanyl, (1,2-dimethylbutyl)sulfanyl, (1,3-dimethylbutyl)sulfanyl, (2,3-dimethylbutyl)sulfanyl, (2-ethylbutyl)sulfanyl, (1-ethylbutyl)sulfanyl, (1-ethyl-1-methylpropyl)sulfanyl, n-heptylsulfanyl, n-octylsulfanyl, n-nonylsulfanyl, n-decylsulfanyl, n-undecylsulfanyl, n-dodecylsulfanyl, n-tridecylsulfanyl, n-tetradecylsulfanyl, and n-pentadecylsulfanyl, which are C₁ to C₁₅ straight chain or branched chain alkyl-sulfanyl groups.

Examples of the alkenyl-sulfanyl group include, for example, vinylsulfanyl, (prop-1-en-1-yl)sulfanyl, allylsulfanyl, isopropenylsulfanyl, (but-1-en-1-yl)sulfanyl, (but-2-en-1-yl)sulfanyl, (but-3-en-1-yl)sulfanyl, (2-methylprop-2-en-1-yl)sulfanyl, (1-methylprop-2-en-1-yl)sulfanyl, (pent-1-en-1-yl)sulfanyl, (pent-2-en-1-yl)sulfanyl, (pent-3-en-1-yl)sulfanyl, (pent-4-en-1-yl)sulfanyl, (3-methylbut-2-en-1-yl)sulfanyl, (3-methylbut-3-en-1-yl)sulfanyl, (hex-1-en-1-yl)sulfanyl, (hex-2-en-1-yl)sulfanyl, (hex-3-en-1-yl)sulfanyl, (hex-4-en-1-yl)sulfanyl, (hex-5-en-1-yl)sulfanyl, (4-methylpent-3-en-1-yl)sulfanyl, (4-methylpent-3-en-1-yl)sulfanyl, (hept-1-en-1-yl)sulfanyl, (hept-6-en-1-yl)sulfanyl, (oct-1-en-1-yl)sulfanyl, (oct-7-en-1-yl)sulfanyl, (non-1-en-1-yl)sulfanyl, (non-8-en-1-yl)sulfanyl, (dec-1-en-1-yl)sulfanyl, (dec-9-en-1-yl)sulfanyl, (undec-1-en-1-yl)sulfanyl, (undec-10-en-1-yl)sulfanyl, (dodec-1-en-1-yl)sulfanyl, (dodec-11-en-1-yl)sulfanyl, (tridec-1-en-1-yl)sulfanyl, (tridec-12-en-1-yl)sulfanyl, (tetradec-1-en-1-yl)sulfanyl, (tetradec-13-en-1-yl)sulfanyl, (pentadec-1-en-1-yl)sulfanyl, and (pentadec-14-en-1-yl)sulfanyl, which are C₂ to C₁₅ straight chain or branched chain alkenyl-sulfanyl groups.

Examples of the alkynyl-sulfanyl group include, for example, ethynylsulfanyl, (prop-1-yn-1-yl)sulfanyl, (prop-2-yn-1-yl)sulfanyl, (but-1-yn-1-yl)sulfanyl, (but-3-yn-1-yl)sulfanyl, (1-methylprop-2-yn-1-yl)sulfanyl, (pent-1-yn-1-yl)sulfanyl, (pent-4-yn-1-yl)sulfanyl, (hex-1-yn-1-yl)sulfanyl, (hex-5-yn-1-yl)sulfanyl, (hept-1-yn-1-yl)sulfanyl, (hept-6-yn-1-yl)sulfanyl, (oct-1-yn-1-yl)sulfanyl, (oct-7-yn-1-yl)sulfanyl, (non-1-yn-1-yl)sulfanyl, (non-8-yn-1-yl)sulfanyl, (dec-1-yn-1-yl)sulfanyl, (dec-9-yn-1-yl)sulfanyl, (undec-1-yn-1-yl)sulfanyl, (undec-10-yn-1-yl)sulfanyl, (dodec-1-yn-1-yl)sulfanyl, (dodec-11-yn-1-yl)sulfanyl, (tridec-1-yn-1-yl)sulfanyl, (tridec-12-yn-1-yl)sulfanyl, (tetradec-1-yn-1-yl)sulfanyl, (tetradec-13-yn-1-yl)sulfanyl, (pentadec-1-yn-1-yl)sulfanyl, and (pentadec-14-yn-1-yl)sulfanyl, which are C₂ to C₁₅ straight chain or branched chain alkynyl-sulfanyl groups.

Examples of the cycloalkyl-sulfanyl group include, for example, cyclopropylsulfanyl, cyclobutylsulfanyl, cyclopentylsulfanyl, cyclohexylsulfanyl, cycloheptylsulfanyl, and cyclooctylsulfanyl, which are C₃ to C₈ cycloalkyl-sulfanyl groups.

Examples of the cycloalkyl-alkyl-sulfanyl group include, for example, (cyclopropylmethyl)sulfanyl, (1-cyclopropylethyl)sulfanyl, (2-cyclopropylethyl)sulfanyl, (3-cyclopropylpropyl)sulfanyl, (4-cyclopropylbutyl)sulfanyl, (5-cyclopropylpentyl)sulfanyl, (6-cyclopropylhexyl)sulfanyl, (cyclobutylmethyl)sulfanyl, (cyclopentylmethyl)sulfanyl, (cyclobutylmethyl)sulfanyl, (cyclopentylmethyl)sulfanyl, (cyclohexylmethyl)sulfanyl, (2-cyclohexylethyl)sulfanyl, (3-cyclohexylpropyl)sulfanyl, (4-cyclohexylbutyl)sulfanyl, (cycloheptylmethyl)sulfanyl, (cyclooctylmethyl)sulfanyl, and (6-cyclooctylhexyl)sulfanyl, which are C₄ to C₁₄ cycloalkyl-alkyl-sulfanyl groups.

Examples of the aryl-sulfanyl group include, for example, phenylsulfanyl, 1-naphthylsulfanyl, 2-naphthylsulfanyl, anthrylsulfanyl, fenanthrylsulfanyl, and acenaphthylenylsulfanyl, which are C₆ to C₁₄ aryl-sulfanyl groups.

Examples of the aralkyl-sulfanyl group include, for example, benzylsulfanyl, (1-naphthylmethyl)sulfanyl, (2-naphthylmethyl)sulfanyl, (anthracenylmethyl)sulfanyl, (phenanthrenylmethyl)sulfanyl, (acenaphthylenylmethyl)sulfanyl, (diphenylmethyl)sulfanyl, (1-phenethyl)sulfanyl, (2-phenethyl)sulfanyl, (1-(1-naphthyl)ethyl)sulfanyl, (1-(2-naphthyl)ethyl)sulfanyl, (2-(1-naphthyl)ehyl)sulfanyl, (2-(2-naphthyl)ethyl)sulfanyl, (3-phenylpropyl)sulfanyl, (3-(1-naphthyl)propyl)sulfanyl, (3-(2-naphthyl)propyl)sulfanyl, (4-phenylbutyl)sulfanyl, (4-(1-naphthyl)butyl)sulfanyl, (4-(2-naphthyl)butyl)sulfanyl, (5-phenylpentyl)sulfanyl, (5-(1-naphthyl)pentyl)sulfanyl, (5-(2-naphthyl)pentyl)sulfanyl, (6-phenylhexyl)sulfanyl, (6-(1-naphthyl)hexyl)sulfanyl, and (6-(2-naphthyl)hexyl)sulfanyl, which are C₇ to C₁₆ aralkyl-sulfanyl groups.

Examples of the halogenated alkyl-sulfanyl group include the groups in which a hydrogen atom of the sulfanyl group is substituted with a halogenated alkyl group, and include, for example, (fluoromethyl)sulfanyl, (chloromethyl)sulfanyl, (bromomethyl)sulfanyl, (iodomethyl)sulfanyl, (difluoromethyl)sulfanyl, (trifluoromethyl)sulfanyl, (trichloromethyl)sulfanyl, (2,2,2-trifluoroethyl)sulfanyl, (pentafluoroethyl)sulfanyl, (3,3,3-trifluoropropyl)sulfanyl, (heptafluoropropyl)sulfanyl, (heptafluoroisopropyl)sulfanyl, (nonafluorobutyl)sulfanyl, and (perfluorohexyl)sulfanyl, which are C₁ to C₆ straight chain or branched chain halogenated alkyl-sulfanyl groups substituted with 1 to 13 halogen atoms.

Examples of the heterocyclic-sulfanyl group include the groups in which a hydrogen atom of the sulfanyl group is substituted with a heterocyclic group, and examples of the heterocyclic ring include similar groups to the aforementioned heterocyclic groups. Examples of the heterocyclic-sulfanyl group include, for example, a monocyclic heteroaryl-sulfanyl group, a fused polycyclic heteroaryl-sulfanyl group, a monocyclic non-aromatic heterocyclic-sulfanyl group, and a fused polycyclic non-aromatic heterocyclic-sulfanyl group.

Examples of the monocyclic heteroaryl-sulfanyl group include, for example, (imidazol-2-yl)sulfanyl, (1,2,4-triazol-2-yl)sulfanyl, (pyridin-2-yl)sulfanyl, (pyridin-4-yl)sulfanyl, and (pyrimidin-2-yl)sulfanyl.

Examples of the fused polycyclic heteroaryl-sulfanyl group include, for example, (benzimidazol-2-yl)sulfanyl, (quinolin-2-yl)sulfanyl, and (quinolin-4-yl)sulfanyl.

Examples of the monocyclic non-aromatic heterocyclic-sulfanyl groups include, for example, (3-pyrrolidinyl)sulfanyl, and (4-piperidinyl)sulfanyl.

Examples of the fused polycyclic non-aromatic heterocyclic-sulfanyl group include, for example, (3-indolinyl)sulfanyl, and (4-chromanyl)sulfanyl.

Examples of the acyl group include, for example, formyl group, glyoxyloyl group, thioformyl group, carbamoyl group, thiocarbamoyl group, sulfamoyl group, sulfinamoyl group, carboxy group, sulfo group, phosphono group, and groups represented by the following formulas: 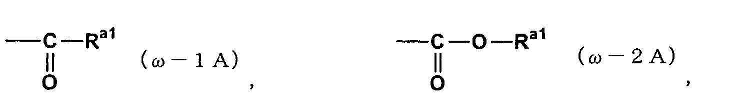 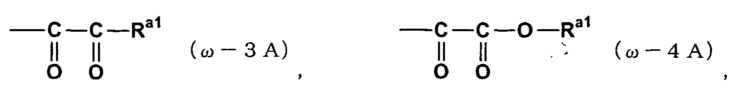 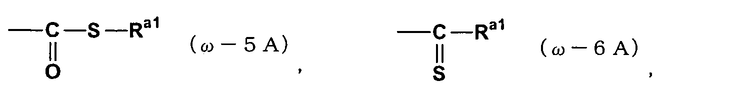 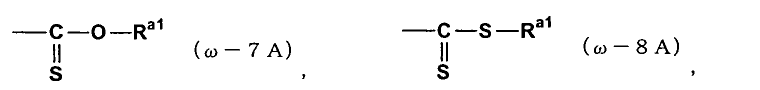 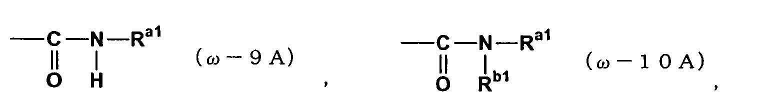 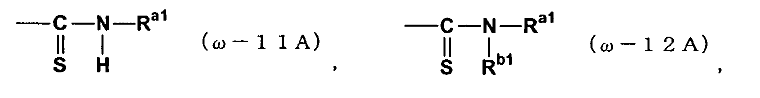 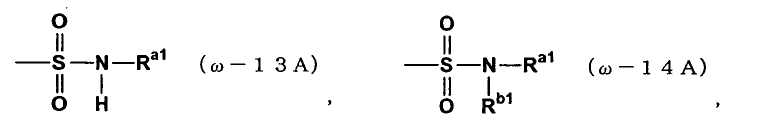 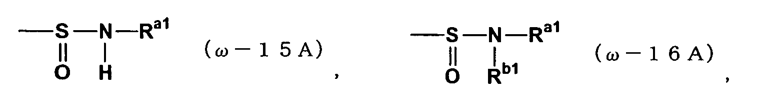 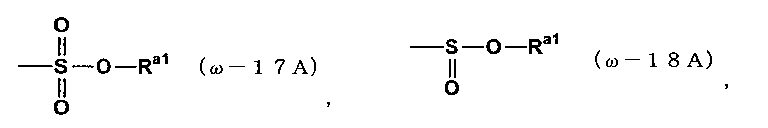 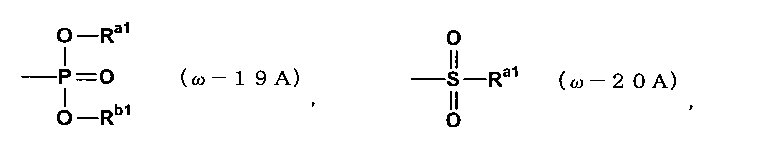 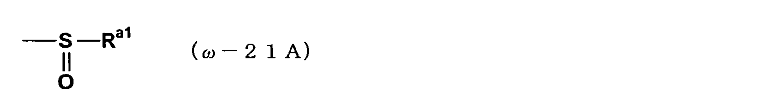 wherein R^{a1} and R^{b1} may be the same or different and represent a hydrocarbon group or a heterocyclic group, or R^{a1} and R^{b1} combine to each other, together with the nitrogen atom to which they bind, to form a cyclic amino group.

In the definition of the aforementioned acyl group, among the groups represented by the formula (ω-1A), those groups in which R^{a1} is a hydrocarbon group are referred to as "hydrocarbon-carbonyl group" whose examples include, for example, acetyl, propionyl, butyryl, isobutyryl, valeryl, isovaleryl, pivaloyl, lauroyl, myristoryl, palmitoyl, acryloyl, propioloyl, methacryloyl, crotonoyl, isocrotonoyl, cyclohexylcarbonyl, cyclohexylmethylcarbonyl, benzoyl, 1-naphthoyl, 2-naphthoyl, and phenylacetyl, and those groups in which R^{a1} is a heterocyclic group are referred to as "heterocyclic ring-carbonyl group" whose examples include, for example, 2-thenoyl, 3-furoyl, nicotinoyl, and isonicotinoyl.

Among the groups represented by the formula (ω-2A), those groups in which R^{a1} is a hydrocarbon group are referred to as "hydrocarbon-oxy-carbonyl group" whose examples include, for example, methoxycarbonyl, ethoxycarbonyl, phenoxycarbonyl, and benzyloxycarbonyl, and those groups in which R^{a1} is a heterocyclic group are referred to as "heterocyclic ring-oxy-carbonyl group" whose examples include, for example, 3-pyridyloxycarbonyl.

Among the groups represented by the formula (ω-3A), those groups in which R^{a1} is a hydrocarbon group are referred to as "hydrocarbon-carbonyl-carbonyl group" whose examples include, for example, pyruvoyl, and those groups in which R^{a1} is a heterocyclic group are referred to as "heterocyclic ring-carbonyl-carbonyl group."

Among the groups represented by the formula (ω-4A), those groups in which R^{a1} is a hydrocarbon group are referred to as "hydrocarbon-oxy-carbonyl-carbonyl group" whose examples include, for example, methoxalyl and ethoxalyl groups, and those groups in which R^{a1} is a heterocyclic group are referred to as "heterocyclic ring-oxy-carbonyl-carbonyl group."

Among the groups represented by the formula (ω-5A), those groups in which R^{a1} is a hydrocarbon group are referred to as "hydrocarbon-sulfanyl-carbonyl group," and those groups in which R^{a1} is a heterocyclic group are referred to as "heterocyclic ring-sulfanyl-carbonyl group."

Among the groups represented by the formula (ω-6A), those groups in which R^{a1} is a hydrocarbon group are referred to as "hydrocarbon-thiocarbonyl group," and those groups in which R^{a1} is a heterocyclic group are referred to as "heterocyclic ring-thiocarbonyl group."

Among the groups represented by the formula (ω-7A), those groups in which R^{a1} is a hydrocarbon group are referred to as "hydrocarbon-oxy-thiocarbonyl group," and those groups in which R^{a1} is a heterocyclic group are referred to as "heterocyclic ring-oxy-thiocarbonyl group."

Among the groups represented by the formula (ω -8A), those groups in which R^{a1} is a hydrocarbon group are referred to as "hydrocarbon-sulfanyl-thiocarbonyl group," and those groups in which R^{a1} is a heterocyclic group are referred to as "heterocyclic ring-sulfanyl-thiocarbonyl group."

Among the groups represented by the formula (ω -9A), those groups in which R^{a1} is a hydrocarbon group are referred to as referred to as "N-hydrocarbon-carbamoyl group" whose examples include, for example, N-methylcarbamoyl group, and those groups in which R^{a1} is a heterocyclic group are referred to as "N-heterocyclic ring-carbamoyl group."

Among the groups represented by the formula (ω-10A), those groups in which both R^{a1} and R^{b1} are hydrocarbon groups are referred to as "N,N-di(hydrocarbon)-carbamoyl group" whose examples include, for example, N,N-dimethylcarbamoyl group, those groups in which both R^{a1} and R^{b1} are heterocyclic groups are referred to as "N,N-di(heterocyclic ring)-carbamoyl group," those groups in which R^{a1} is a hydrocarbon group and R^{b1} is a heterocyclic group are referred to as "N-hydrocarbon-N-heterocyclic ring-substituted carbamoyl group," and those groups in which R^{a1} and R^{b1} combine to each other, together with the nitrogen atom to which they bind, to form a cyclic amino group are referred to as "cyclic amino-carbonyl group" whose examples include, for example, morpholino-carbonyl.

Among the groups represented by the formula (ω-11A), those groups in which R^{a1} is a hydrocarbon group are referred to as "N-hydrocarbon-thiocarbamoyl group," and those groups in which R^{a1} is a heterocyclic group are referred to as "N-heterocyclic ring-thiocarbamoyl group."

Among the groups represented by the formula (ω-12A), those groups in which both R^{a1} and R^{b1} are hydrocarbon groups are referred to as "N,N-di(hydrocarbon)-thiocarbamoyl group," those groups in which both R^{a1} and R^{b1} are heterocyclic groups are referred to as "N,N-di(heterocyclic ring)-thiocarbamoyl group," those groups in which R^{a1} is a hydrocarbon group and R^{b1} is a heterocyclic group are referred to as "N-hydrocarbon-N-heterocyclic ring-thiocarbamoyl group," and those groups in which R^{a1} and R^{b1} combine to each other, together with the nitrogen atom to which they bind, to form a cyclic amino group are referred to as "cyclic amino-thiocarbonyl group."

Among the groups represented by the formula (ω-13A), those groups in which R^{a1} is a hydrocarbon group are referred to as "N-hydrocarbon-sulfamoyl group," and those groups in which R^{a1} is a heterocyclic group are referred to as "N-heterocyclic ring-sulfamoyl group."

Among the groups represented by the formula (ω-14A), those groups in which both R^{a1} and R^{b1} are hydrocarbon groups are referred to as "N,N-di(hydrocarbon)-sulfamoyl group" whose examples include, for example, N,N-dimethylsulfamoyl group, those groups in which both R^{a1} and R^{b1} are heterocyclic groups are referred to as "N,N-di(heterocyclic ring)-sulfamoyl group," those groups in which R^{a1} is a hydrocarbon group and R^{b1} is a heterocyclic group are referred to as "N-hydrocarbon-N-heterocyclic ring-sulfamoyl group," and those groups in which R^{a1} and R^{b1} combine to each other, together with the nitrogen atom to which they bind, to form a cyclic amino group are referred to as "cyclic amino-sulfonyl group" whose examples include, for example 1-pyrrolylsulfonyl.

Among the groups represented by the formula (ω-15A), those groups in which R^{a1} is a hydrocarbon group are referred to as "N-hydrocarbon-sulfinamoyl group," and those groups in which R^{a1} is a heterocyclic group are referred to as "N-heterocyclic ring-sulfinamoyl group."

Among the groups represented by the formula (ω-16A), those groups in which both R^{a1} and R^{b1} are hydrocarbon groups are referred to as "N,N-di(hydrocarbon)-sulfinamoyl group," those groups in which both R^{a1} and R^{b1} are heterocyclic groups are referred to as "N,N-di(heterocyclic ring)-sulfinamoyl group," those groups in which R^{a1} is a hydrocarbon group and R^{b1} is a heterocyclic group are referred to as "N-hydrocarbon-N-heterocyclic ring-sulfinamoyl group," and those groups in which R^{a1} and R^{b1} combine to each other, together with the nitrogen atom to which they bind, to form a cyclic amino group are referred to as "cyclic amino-sulfinyl group."

Among the groups represented by the formula (ω-17A), those groups in which R^{a1} is a hydrocarbon group are referred to as "hydrocarbon-oxy-sulfonyl group," and those groups in which R^{a1} is a heterocyclic group are referred to as "heterocyclic ring-oxy-sulfonyl group."

Among the groups represented by the formula (ω-18A), those groups in which R^{a1} is a hydrocarbon group are referred to as "hydrocarbon-oxy-sulfinyl group," and those groups in which R^{a1} is a heterocyclic group are referred to as "heterocyclic ring-oxy-sulfinyl group."

Among the groups represented by the formula (ω-19A), those groups in which both R^{a1} and R^{b1} are hydrocarbon groups are referred to as "O,O'-di(hydrocarbon)-phosphono group," those groups in which both R^{a1} and R^{b1} are heterocyclic groups are referred to as "O,O'-di(heterocyclic ring)-phosphono group," and those groups in which R^{a1} is a hydrocarbon group and R^{b1} is a heterocyclic group are referred to as "O-hydrocarbon-O'-heterocyclic ring-phosphono group."

Among the groups represented by the formula (ω-20A), those groups in which R^{a1} is a hydrocarbon group are referred to as "hydrocarbon-sulfonyl group" whose examples include, for example, methanesulfonyl and benzenesulfonyl, and those groups in which R^{a1} is a heterocyclic group are referred to as "heterocyclic ring-sulfonyl group. "

Among the groups represented by the formula (ω-21A), those groups in which R^{a1} is a hydrocarbon group are referred to as "hydrocarbon-sulfinyl group" whose examples include, for example, methylsulfinyl and benzenesulfinyl, and those groups in which R^{a1} is a heterocyclic group are referred to as "heterocyclic ring-sulfinyl group."

Examples of the hydrocarbon in the groups represented by the aforementioned formulas (ω-1A) through (ω-21A) include the similar groups to the aforementioned hydrocarbon group. Examples of the hydrocarbon-carbonyl group represented by the formula (ω-1A) include, for example, an alkyl-carbonyl group, an alkenyl-carbonyl group, an alkynyl-carbonyl group, a cycloalkyl-carbonyl group, a cycloalkenyl-carbonyl group, a cycloalkanedienyl-carbonyl group, a cycloalkyl-alkyl-carbonyl group, which are aliphatic hydrocarbon-carbonyl groups; an aryl-carbonyl group; an aralkyl-carbonyl group; a bridged cyclic hydrocarbon-carbonyl group; a spirocyclic hydrocarbon-carbonyl group; and a terpene family hydrocarbon-carbonyl group. In the following, groups represented by the formulas (ω-2A) through (ω-21A) are similar to those explained above.

Examples of the heterocyclic ring in the groups represented by the aforementioned formulas (ω-1A) through (ω-21A) include similar groups to the aforementioned heterocyclic group. Examples of the heterocyclic ring-carbonyl group represented by the formula (ω-1A) include, for example, a monocyclic . heteroaryl-carbonyl group, a fused polycyclic heteroaryl-carbonyl group, a monocyclic non-aromatic heterocyclic ring-carbonyl group, and a fused polycyclic non-aromatic heterocyclic ring-carbonyl group. In the following, groups represented by the formulas (ω-2A) through (ω-21A) are similar to those explained above.

Examples of the cyclic amino in the groups represented by the aforementioned formulas (ω-10A) through (ω-16A) include similar groups to the aforementioned cyclic amino group.

In the present specification, when a certain functional group is defined as "which may be substituted," the definition means that the functional group may sometimes have one or more substituents at chemically substitutable positions, unless otherwise specifically mentioned. Kind of substituents, number of substituents, and the position of substituents existing in the functional groups are not particularly limited, and when two or more substituents exist, they may be the same or different. Examples of the substituent existing in the functional group include, for example, halogen atoms, oxo group, thioxo group, nitro group, nitroso group, cyano group, isocyano group, cyanato group, thiocyanato group, isocyanato group, isothiocyanato group, hydroxy group, sulfanyl group, carboxy group, sulfanylcarbonyl group, oxalo group, methooxalo group, thiocarboxy group, dithiocarboxy group, carbamoyl group, thiocarbamoyl group, sulfo group, sulfamoyl group, sulfino group, sulfinamoyl group, sulfeno group, sulfenamoyl group, phosphono group, hydroxyphosphonyl group, hydrocarbon group, heterocyclic group, hydrocarbon-oxy group, heterocyclic ring-oxy group, hydrocarbon-sulfanyl group, heterocyclic ring-sulfanyl group, acyl group, amino group, hydrazino group, hydrazono group, diazenyl group, ureido group, thioureido group, guanidino group, carbamoimidoyl group (amidino group), azido group, imino group, hydroxyamino group, hydroxyimino group, aminooxy group, diazo group, semicarbazino group, semicarbazono group, allophanyl group, hydantoyl group, phosphano group, phosphoroso group, phospho group, boryl group, silyl group, stannyl group, selanyl group, oxido group and the like.

When two or more substituents exist according to the aforementioned definition of "which may be substituted," said two or more substituents may combine to each other, together with atom(s) to which they bind, to form a ring. For these cyclic groups, as ring-constituting atoms (ring forming atoms), one to three kinds of one or more hetero atoms selected from oxygen atom, sulfur atom, nitrogen atom and the like may be included, and one or more substituents may exist on the ring. The ring may be monocyclic or fused polycyclic, and aromatic or non-aromatic.

The above substituents according to the aforementioned definition of "which may be substituted" may further be substituted with the aforementioned substituents at the chemically substitutable positions on the substituent. Kind of substituents, number of substituents, and positions of substituents are not particularly limited, and when the substituents are substituted with two or more substituents, they may be the same or different. Examples of the substituent include, for example, a halogenated alkyl-carbonyl group whose examples include, for example, trifluoroacetyl, a halogenated alkyl-sulfonyl group whose examples include, for example, trifluoromethanesulfonyl, an acyl-oxy group, an acyl-sulfanyl group, an N-hydrocarbon-amino group, an N,N-di(hydrocarbon)-amino group, an N-heterocyclic ring-amino group, an N-hydrocarbon-N-heterocyclic ring-amino group, an acyl-amino group, and a di(acyl)-amino group. Moreover, substitution on the aforementioned substituents may be repeated multiple orders.

Examples of the acyl-oxy group include the groups in which hydrogen atom of hydroxy group is substituted with acyl group, and include, for example, formyloxy group, glyoxyloyloxy group, thioformyloxy group, carbamoloxy group, thiocarbamoyloxy group, sulfamoyloxy group, sulfinamoloxy group, carboxyoxy group, sulphooxy group, phosphonooxy group, and groups represented by the following formulas: 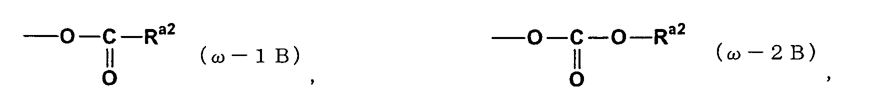 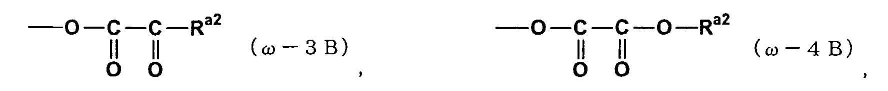 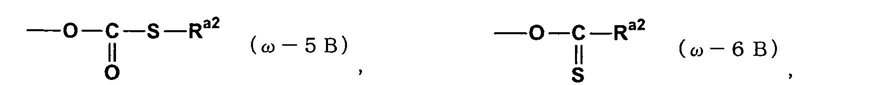 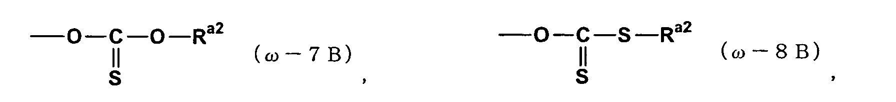 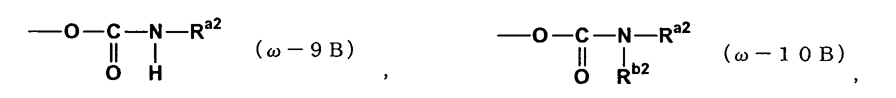 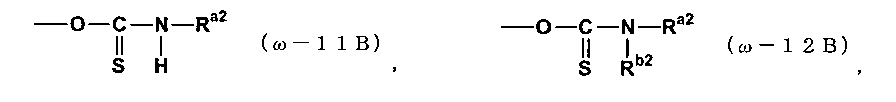 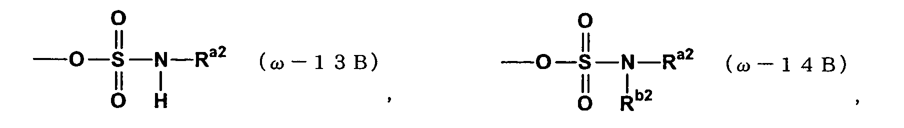 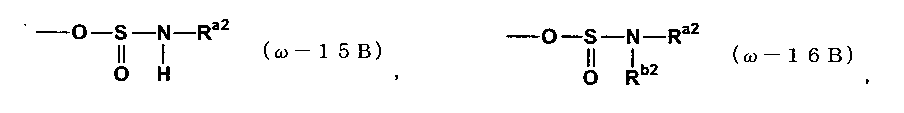 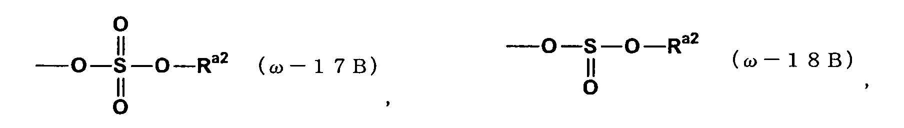 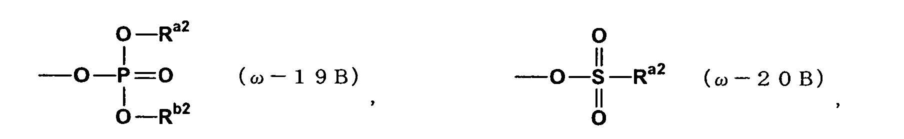 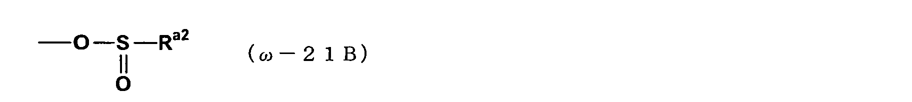 wherein R^{a2} and R^{b2} may be the same or different and represent a hydrocarbon group or a heterocyclic group, or R^{a2} and R^{b2} combine to each other, together with the nitrogen atom to which they bind, to form a cyclic amino group.

In the definition of the aforementioned acyl-oxy group, among the groups represented by the formula (ω-1B), those groups in which R^{a2} is a hydrocarbon group are referred to as "hydrocarbon-carbonyl-oxy group" whose examples include, for example, acetoxy and benzoyloxy, and those groups in which R^{a2} is a heterocyclic group are referred to as "heterocyclic ring-carbonyl-oxy group."

Among the groups represented by the formula (ω-2B), those groups in which R^{a2} is a hydrocarbon group are referred to as "hydrocarbon-oxy-carbonyl-oxy group," and those groups in which R^{a2} is a heterocyclic group are referred to as "heterocyclic ring-oxy-carbonyl-oxy group."

Among the groups represented by the formula (ω-3B), those groups in which R^{a2} is a hydrocarbon group are referred to as "hydrocarbon-carbonyl-carbonyl-oxy group," and those groups in which R^{a2} is a heterocyclic group are referred to as "heterocyclic ring-carbonyl-carbonyl-oxy group."

Among the groups represented by the formula (ω-4B), those groups in which R^{a2} is a hydrocarbon group are referred to as "hydrocarbon-oxy-carbonyl-carbonyl-oxy group," and those groups in which R^{a2} is a heterocyclic group are referred to as "heterocyclic ring-oxy-carbonyl-carbonyl-oxy group."

Among the groups represented by the formula (ω-5B), those groups in which R^{a2} is a hydrocarbon group are referred to as "hydrocarbon-sulfanyl-carbonyl-oxy group," and those groups where R^{a2} is a heterocyclic group are referred to as "heterocyclic ring-sulfanyl-carbonyl-oxy group."

Among the groups represented by the formula (ω-6B), those groups in which R^{a2} is a hydrocarbon group are referred to as "hydrocarbon-thiocarbonyl-oxy group," and those groups where R^{a2} is a heterocyclic group are referred to as "heterocyclic ring-thiocarbonyl-oxy group."

Among the groups represented by the formula (ω-7B), those groups in which R^{a2} is a hydrocarbon group are referred to as "hydrocarbon-oxy-thiocarbonyl-oxy group," and those groups in which R^{a2} is a heterocyclic group are referred to as "heterocyclic ring-oxy-thiocarbonyl-oxy group."

Among the groups represented by the formula (ω-8B), those groups in which R^{a2} is a hydrocarbon group are referred to as "hydrocarbon-sulfanyl-thiocarbonyl-oxy group," and those groups wherein R^{a2} is a heterocyclic group are referred to as "heterocyclic ring-sulfanyl-thiocarbonyl-oxy group."

Among the groups represented by the formula (ω-9B), those groups in which R^{a2} is a hydrocarbon group are referred to as "N-hydrocarbon-carbamoyl-oxy group," and those groups in which R^{a2} is a heterocyclic group are referred to as "N-heterocyclic ring-carbamoyl-oxy group."

Among the groups represented by the formula (ω-10B), those groups in which both R^{a2} and R^{b2} are hydrocarbon groups are referred to as "N,N-di(hydrocarbon)-carbamoyl-oxy group," those groups in which both R^{a2} and R^{b2} are heterocyclic groups are referred to as "N,N-di(heterocyclic ring)-carbamoyl-oxy group," those groups in which R^{a2} is a hydrocarbon group and R^{b2} is a heterocyclic group are referred to as "N-hydrocarbon-N-heterocyclic ring-carbamoyl-oxy group," and those groups in which R^{a2} and R^{b2} combine to each other, together with the nitrogen atom to which they bind, to form a cyclicic amino group are referred to as "cyclicamino-carbonyl-oxy group."

Among the groups represented by the formula (ω-11B), those groups in which R^{a2} is a hydrocarbon group are referred to as "N-hydrocarbon-thiocarbamoyl-oxy group," and those groups in which R^{a2} is a heterocyclic group are referred to as "N-heterocyclic ring-thiocarbamoyl-oxy group."

Among the groups represented by the formula (ω-12B), those groups in which both R^{a2} and R^{b2} are hydrocarbon groups are referred to as "N,N-di(hydrocarbon)-thiocarbamoyl-oxy group," those groups in which both R^{a2} and R^{b2} are heterocyclic groups are referred to as "N,N-di(heterocyclic ring)-thiocarbamoyl-oxy group," those groups in which R^{a2} is a hydrocarbon group and R^{b2} is a heterocyclic group are referred to as "N-hydrocarbon-N-heterocyclic ring-thiocarbamoyl-oxy group," and those groups in which R^{a2} and R^{b2} combine to each other, together with the nitrogen atom to which they bind, to form a cyclic amino group are referred to as "cyclicamino-thiocarbonyl-oxy group."

Among the groups represented by the formula (ω-13B), those groups in which R^{a2} is a hydrocarbon group are referred to as "N-hydrocarbon-sulfamoyl-oxy group," and those groups in which R^{a2} is a heterocyclic group are referred to as "N-heterocyclic ring-sulfamoyl-oxy group."

Among the groups represented by the formula (ω-14B), those groups in which both R^{a2} and R^{b2} are hydrocarbon groups are referred to as "N,N-di(hydrocarbon)-sulfamoyl-oxy group," those groups in which both R^{a2} and R^{b2} are heterocyclic groups are referred to as "N,N-di(heterocyclic ring)-sulfamoyl-oxy group," those groups in which R^{a2} is a hydrocarbon group and R^{b2} is a heterocyclic group are referred to as "N-hydrocarbon-N-heterocyclic ring-sulfamoyl-oxy group," and those groups in which R^{a2} and R^{b2} combine to each other, together with the nitrogen atom to which they bind, to form a cyclic amino group are referred to as "cyclic amino-sulfonyl-oxy group."

Among the groups represented by the formula (ω-15B), those groups in which R^{a2} is a hydrocarbon group are referred to as "N-hydrocarbon-sulfinamoyl-oxy group," and those groups where R^{a2} is a heterocyclic group are referred to as "N-heterocyclic ring-sulfinamoyl-oxy group."

Among the groups represented by the formula (ω-16B), those groups in which both R^{a2} and R^{b2} are hydrocarbon groups are referred to as "N,N-di(hydrocarbon)-sulfinamoyl-oxy group," those groups in which both R^{a2} and R^{b2} are heterocyclic groups are referred to as "N,N-di(heterocyclic ring)-sulfinamoyl-oxy group," those groups in which R^{a2} is a hydrocarbon group and R^{b2} is a heterocyclic group are referred to as "N-hydrocarbon-N-heterocyclic ring-sulfinamoyl-oxy group," and those groups in which R^{a2} and R^{b2} combine to each other, together with the nitrogen atom to which they bind, to form a cyclic amino group are referred to as "cyclic amino-sulfinyl-oxy group."

Among the groups represented by the formula (ω-17B), those groups in which R^{a2} is a hydrocarbon group are referred to as "hydrocarbon-oxy-sulfonyl-oxy group," and those groups in which R^{a2} is a heterocyclic group are referred to as "heterocyclic ring-oxy-sulfonyl-oxy group."

Among the groups represented by the formula (ω-18B), those groups in which R^{a2} is a hydrocarbon group are referred to as "hydrocarbon-oxy-sulfinyl-oxy group," those groups in which R^{a2} is a heterocyclic group are referred to as "heterocyclic ring-oxy-sulfinyl-oxy group."

Among the groups represented by the formula (ω-19B), those groups in which both R^{a2} and R^{b2} are hydrocarbon groups are referred to as "O,O'-di(hydrocarbon)-phosphono-oxy group," those groups in which both R^{a2} and R^{b2} are heterocyclic groups are referred to as "O,O'-di(heterocyclic ring)-phosphono-oxy group," and those groups in which R^{a2} is a hydrocarbon group and R^{b2} is a heterocyclic group are referred to as "O-hydrocarbon substituted-O'-heterocyclic ring substituted phophono-oxy group."

Among the groups represented by the formula (ω-20B), those groups in which R^{a2} is a hydrocarbon group are referred to as "hydrocarbon-sulfonyl-oxy group," and those groups in which R^{a2} is a heterocyclic group referred to as "heterocyclic ring-sulfonyl-oxy group."

Among the groups represented by the formula (ω-21B), those groups in which R^{a2} is a hydrocarbon group are referred to as "hydrocarbon-sulfinyl-oxy group," and those groups in which R^{a2} is a heterocyclic group are referred to as "heterocyclic ring-sulfinyl-oxy group."

Examples of the hydrocarbon in the groups represented by the aforementioned formulas (ω-1B) through (ω-21B) include the similar groups to the aforementioned hydrocarbon group. Examples of the hydrocarbon-carbonyl-oxy group represented by the formula (ω-1B) include, for example, an alkyl-carbonyl-oxy group, an alkenyl-carbonyl-oxy group, an alkynyl-carbonyl-oxy group, a cycloalkyl-carbonyl-oxy group, a cycloalkenyl-carbonyl-oxy group, a cycloalkanedienyl-carbonyl-oxy group, and a cycloalkyl-alkyl-carbonyl-oxy group, which are aliphatic hydrocarbon-carbonyl-oxy groups; an aryl-carbonyl-oxy group; an aralkyl-carbonyl-oxy group; a bridged cyclic hydrocarbon-carbonyl-oxy group; a spirocyclic hydrocarbon-carbonyl-oxy group; and a terpene family hydrocarbon-carbonyl-oxy group. In the following, groups represented by the formulas (ω-2B) through (ω-21B) are similar to those explained above.

Examples of the heterocyclic ring in the groups represented by the aforementioned formulas (ω-1B) through (ω-21B) include similar groups to the aforementioned heterocyclic group. Examples of the heterocyclic ring-carbonyl group represented by the formula (ω-1B) include, for example, a monocyclic heteroaryl-carbonyl group, a fused polycyclic heteroaryl-carbonyl group, a monocyclic non-aromatic heterocyclic ring-carbonyl group, and a fused polycyclic non-aromatic heterocyclic ring-carbonyl group. In the following, groups represented by the formulas (ω-2B) through (ω-21B) are similar to those groups explained above.

Examples of the cyclic amino in the groups represented by the aforementioned formulas (ω-10B) through (ω-16B) include similar groups to the aforementioned cyclic amino group.

The aforementioned acyl-oxy group, hydrocarbon-oxy group, and heterocyclic-oxy group are generically referred to as "substituted oxy group." Moreover, these substituted oxy group and hydroxy group are generically referred to as "hydroxy group which may be substituted."

Examples of the acyl-sulfanyl group include the groups in which hydrogen atom of sulfanyl group is substituted with acyl group, and include, for example, formylsulfanyl group, glyoxyloylsulfanyl group, thioformylsulfanyl group, carbamoyloxy group, thicarbamoyloxy group, sulfamoyloxy group, sulfinamoyloxy group, carboxyoxy group, sulphooxy group, phosphonooxy group, and groups represented by the following formulas: 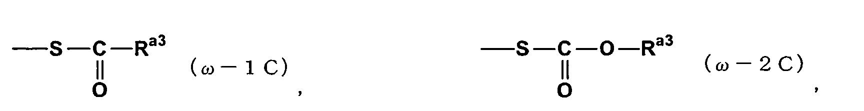 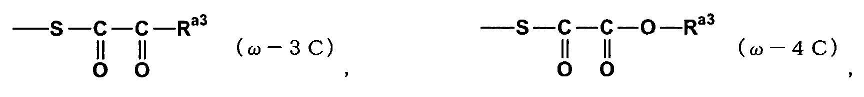 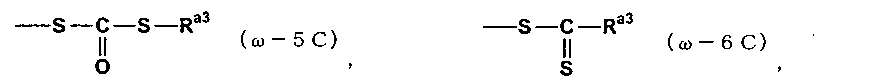 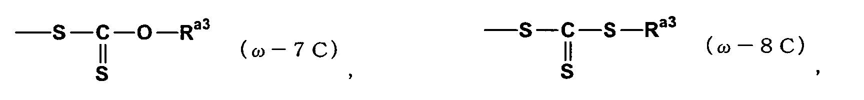 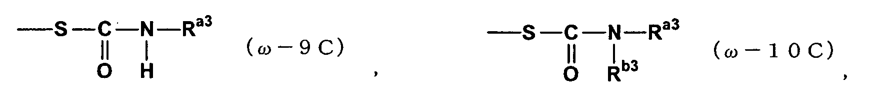 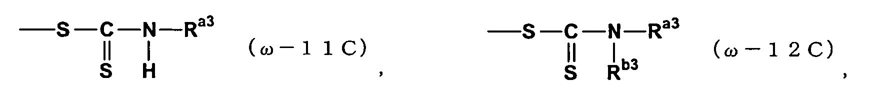 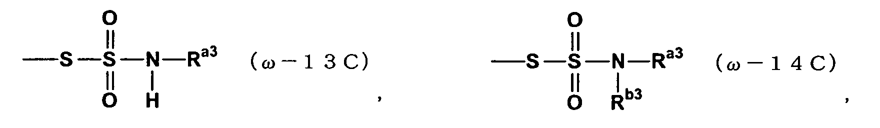 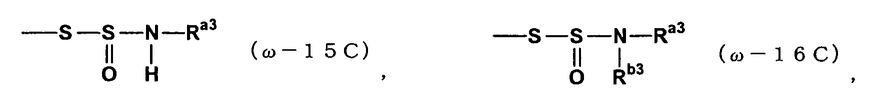 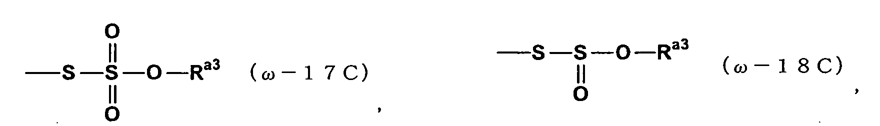 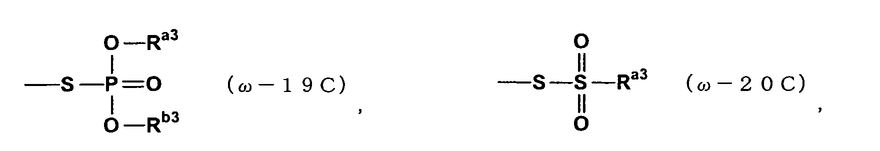 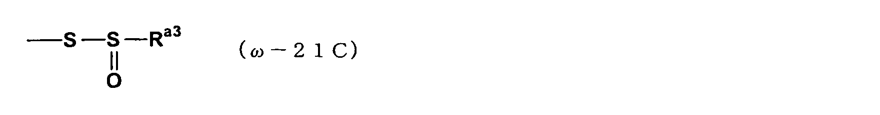 wherein R^{a3} and R^{b3} may be the same or different and represent a hydrocarbon group which may be substituted or a heterocyclic group which may be substituted, or R^{a3} and R^{b3} combine to each other, together with the nitrogen atom to which they bind, to form a cyclic amino group which may be substituted.

In the definition of the aforementioned acyl-sulfanyl group, among the groups represented by the formula (ω-1C), those groups in which R^{a3} is a hydrocarbon group are referred to as "hydrocarbon-carbonyl-sulfanyl group," and those groups in which R^{a3} is a heterocyclic group are referred to as "heterocyclic ring-carbonyl-sulfanyl group."

Among the groups represented by the formula (ω-2C), those groups in which R^{a3} is a hydrocarbon group are referred to as "hydrocarbon-oxy-carbonyl-sulfanyl group," and those groups in which R^{a3} is a heterocyclic group are referred to as "heterocyclic ring-oxy-carbonyl-sulfanyl group."

Among the groups represented by the formula (ω-3C), those groups in which R^{a3} is a hydrocarbon group are referred to as "hydrocarbon-carbonyl-carbonyl-sulfanyl group," and those groups in which R^{a3} is a heterocyclic group are referred to as "heterocyclic ring-carbonyl-carbonyl-sulfanyl group."

Among the groups represented by the formula (ω -4C), those groups in which R^{a3} is a hydrocarbon group are referred to as "hydrocarbon-oxy-carbonyl-carbonyl-sulfanyl group," and those groups in which R^{a3} is a heterocyclic group are referred to as "heterocyclic ring-oxy-carbonyl-carbonyl-sulfanyl group."

Among the groups represented by the formula (ω-5C), those groups in which R^{a3} is a hydrocarbon group are referred to as "hydrocarbon-sulfanyl-carbonyl-sulfanyl group," and those groups in which R^{a3} is a heterocyclic group are referred to as "heterocyclic ring-sulfanyl-carbonyl-sulfanyl group."

Among the groups represented by the formula (ω-6C), those groups in which R^{a3} is a hydrocarbon group are referred to as "hydrocarbon-thiocarbonyl-sulfanyl group," and those groups in which R^{a3} is a heterocyclic group are referred to as "heterocyclic ring-thiocarbonyl-sulfanyl group."

Among the groups represented by the formula (ω-7C), those groups in which R^{a3} is a hydrocarbon group are referred to as "hydrocarbon-oxy-thiocarbonyl-sulfanyl group," and those groups in which R^{a3} is a heterocyclic group are referred to as "heterocyclic ring-oxy-thiocarbonyl-sulfanyl group."

Among the groups represented by the formula (ω-8C), those groups in which R^{a3} is a hydrocarbon group are referred to as "hydrocarbon-sulfanyl-thiocarbonyl-sulfanyl group," and those groups in which R^{a3} is a heterocyclic group are referred to as "heterocyclic ring-sulfanyl-thiocarbonyl-sulfanyl group."

Among the groups represented by the formula (ω-9C), those groups in which R^{a3} is a hydrocarbon group are referred to as "N-hydrocarbon-carbamoyl-sulfanyl group," and those groups in which R^{a3} is a heterocyclic group are referred to as "N-heterocyclic ring-carbamoyl-sulfanyl group."

Among the groups represented by the formula (ω-10C), those groups in which both R^{a3} and R^{b3} are a hydrocarbon groups are referred to as "N,N-di(hydrocarbon)-carbamoyl-sulfanyl group," those groups in which both R^{a3} and R^{b3} are heterocyclic groups are referred to as "N,N-di(heterocyclic ring)-carbamoyl-sulfanyl group," those groups in which R^{a3} is a hydrocarbon group and R^{b3} is a heterocyclic group are referred to as "N-hydrocarbon-N-heterocyclic ring-carbamoyl-sulfanyl group," and those groups in which R^{a3} and R^{b3} combine to each other, together with the nitrogen atom to which they bind, to form a cyclic amino group are referred to as "cyclicamino-carbonyl-sulfamoyl group."

Among the groups represented by the formula (ω-11C), those groups in which R^{a3} is a hydrocarbon group are referred to as "N-hydrocarbon-thiocarbamoyl-sulfanyl group," and those groups in which R^{a3} is a heterocyclic group are referred to as "N-heterocyclic ring-thiocarbamoyl-sulfanyl group."

Among the groups represented by the formula (ω-12C), those groups in which both R^{a3} and R^{b3} are hydrocarbon groups are referred to as "N,N-di(hydrocarbon)-thiocarbamoyl-sulfanyl group," those groups in which and R^{a3} and R^{b3} are heterocyclic groups are referred to as "N,N-di(heterocyclic ring)-thiocarbamoyl-sulfanyl group," those groups in which R^{a3} is a hydrocarbon group and R^{b3} is a heterocyclic group are referred to as "N-hydrocarbon-N-heterocyclic ring-thiocarbamoyl-sulfanyl group," and those groups in which R^{a3} and R^{b3} combine to each other, together with the nitrogen atom to which they bind, to form a cyclic amino group are referred to as "cyclicamino-thiocarbonyl-sulfamoyl group."

Among the groups represented by the formula (ω-13C), those groups in which R^{a3} is a hydrocarbon group are referred to as "N-hydrocarbon-sulfamoyl-sulfanyl group," and those groups in which R^{a3} is a heterocyclic group are referred to as "N-heterocyclic ring-sulfamoyl-sulfanyl group."

Among the groups represented by the formula (ω-14C), those groups in which both R^{a3} and R^{b3} are hydrocarbon groups are referred to as "N,N-di(hydrocarbon)-sulfamoyl-sulfanyl group," those groups in which both R^{a3} and R^{b3} are heterocyclic groups are referred to as "N,N-di(heterocyclic ring)-sulfamoyl-sulfinyl group," those groups in which R^{a3} is a hydrocarbon group and R^{b3} is a heterocyclic group are referred to as "N-hydrocarbon-N-heterocyclic ring-sulfamoyl-sulfanyl group," and those groups in which R^{a3} and R^{b3} combine to each other, together with the nitrogen atom to which they bind, to form a cyclic amino group are referred to as "cyclicamino-sulfonyl-sulfanyl group."

Among the groups represented by the formula (ω-15C), those groups in which R^{a3} is a hydrocarbon group are referred to as "N-hydrocarbon-sulfinamoyl-sulfanyl group," and those groups in which R^{a3} is a heterocyclic group are referred to as "N-heterocyclic ring-sulfinamoyl-sulfanyl group."

Among the groups represented by the formula (ω-16C), those groups in which both R^{a3} and R^{b3} are hydrocarbon groups are referred to as "N,N-di(hydrocarbon)-sulfinamoyl-sulfanyl group," those groups in which both R^{a3} and R^{b3} are heterocyclic groups are referred to as "N,N-di(heterocyclic ring)-sulfinamoyl-sulfanyl group," those groups in which R^{a3} is a hydrocarbon group and R^{b3} is a heterocyclic group are referred to as "N-hydrocarbon-N-heterocyclic ring-sulfinamoyl-sulfanyl group," and those groups in which R^{a3} and R^{b3} combine to each other, together with the nitrogen atom to which they bind, to form a cyclic amino group are referred to as "cyclicamino-sulfanyl-sulfanyl group."

Among the groups represented by the formula (ω-17C), those groups in which R^{a3} is a hydrocarbon group are referred to as "hydrocarbon-oxy-sulfonyl-sulfanyl group," and those groups in which R^{a3} is a heterocyclic group are referred to as "heterocyclic ring-oxy-sulfonyl-sulfanyl group."

Among the groups represented by the formula (ω-18C), those groups in which R^{a3} is a hydrocarbon group are referred to as "hydrocarbon-oxy-sulfinyl-sulfanyl group," and those groups in which R^{a3} is a heterocyclic group are referred to as "heterocyclic ring-oxy-sulfinyl-sulfanyl group."

Among the groups represented by the formula (ω-19C), those groups in which both R^{a3} and R^{b3} are hydrocarbon groups are referred to as "O,O'-di(hydrocarbon)-phosphono-sulfanyl group," those groups in which both R^{a3} and R^{b3} are heterocyclic groups are referred to as "O,O'-di(heterocyclic ring)-phosphono-sulfanyl group," and those groups in which R^{a3} is a hydrocarbon group and R^{b3} is a heterocyclic group are referred to as "O-hydrocarbon-O'-heterocyclic ring-phosphono-sulfanyl group."

Among the groups represented by the formula (ω-20C), those groups in which R^{a3} is a hydrocarbon group are referred to as "hydrocarbon-sulfonyl-sulfanyl group," and those groups in which R^{a3} is a heterocyclic group are referred to as "heterocyclic ring-sulfonyl-sulfanyl group."

Among the groups represented by the formula (ω-21C), those groups in which R^{a3} is a hydrocarbon group are referred to as "hydrocarbon-sulfinyl-sulfanyl group," and those groups in which R^{a3} is a heterocyclic group are referred to as "heterocyclic ring-sulfinyl-sulfanyl group."

Examples of the hydrocarbon in the groups represented by the aforementioned formulas (ω-1C) through (ω-21C) include similar groups to the aforementioned hydrocarbon group. Examples of the hydrocarbon-carbonyl-sulfanyl group represented by the formula (ω-1C) include, for example, an alkyl-carbonyl-sulfanyl group, an alkenyl-carbonyl-sulfanyl group, an alkynyl-carbonyl-sulfanyl group, a cycloalkyl-carbonyl-sulfanyl group, a cycloalkenyl-carbonyl-sulfanyl group, a cycloalkanedienyl-carbonyl-sulfanyl group, a cycloalkyl-alkyl-carbonyl-sulfanyl group which are aliphatic hydrocarbon-carbonyl-sulfanyl groups; an aryl-carbonyl-sulfanyl group; an aralkyl-carbonyl-sulfanyl group; a bridged cyclic hydrocarbon-carbonyl-sulfanyl group; a spiro cyclic hydrocarbon-carbonyl-sulfanyl group; and a terpene family hydrocarbon-carbonyl-sulfanyl group. In the following, groups represented by the formulas (ω-2C) through (ω-21C) are similar to those explained above.

Examples of the heterocyclic ring in the groups represented by the aforementioned formulas (ω-1C) through (ω-21C) include similar groups to the aforementioned heterocyclic group. Examples of the heterocyclic ring-carbonyl-sulfanyl group represented by the formula (ω-1C) include, for example, a monocyclic heteroaryl-carbonyl-sulfanyl group, a fused polycyclic heteroaryl-carbonyl-sulfanyl group, a monocyclic non-aromatic heterocyclic ring-carbonyl-sulfanyl group, and a fused polycyclic non-aromatic heterocyclic ring-carbonyl-sulfanyl group. In the following, groups represented by the formula (ω-2C) through (ω-21C) are similar to those groups explained above.

Examples of the cyclic amino in the groups represented by the aforementioned formulas (ω-10C) through (ω-16C) include similar groups to the aforementioned cyclic amino group.

The aforementioned acyl-sulfanyl group, hydrocarbon-sulfanyl group, and heterocyclic-sulfanyl group are generically referred to as "substituted sulfanyl group." Moreover, these substituted sulfanyl group and sulfanyl group are generically referred to as "sulfanyl group which may be substituted."

Examples of the N-hydrocarbon-amino group include the groups in which one hydrogen atom of amino group is substituted with a hydrocarbon group, and include, for example, an N-alkyl-amino group, an N-alkenyl-amino group, an N-alkynyl-amino group, an N-cycloalkyl-amino group, an N-cycloalkyl-alkyl-amino group, an N-aryl-amino group, and an N-aralkyl-amino group.

Examples of the N-alkyl-amino group include, for example, methylamino, ethylamino, n-propylamino, isopropylamino, n-butylamino, isobutylamino, sec-butylamino, tert-butylamino, n-pentylamino, isopentylamino, (2-methylbutyl)amino, (1-methylbutyl)amino, neopentylamino, (1,2-dimethylpropyl)amino, (1-ethylpropyl)amino, n-hexylamino, (4-methylpentyl)amino, (3-methylpentyl)amino, (2-methylpentyl)amino, (1-methylpentyl)amino, (3,3-dimethylbutyl)amino, (2,2-dimethylbutyl)amino, (1,1-dimethylbutyl)amino, (1,2-dimethylbutyl)amino, (1,3-dimethylbutyl)amino, (2,3-dimethylbutyl)amino, (2-ethylbutyl)amino, (1-ethylbutyl)amino, (1-ethyl-1-methylpropyl)amino, n-heptylamino, n-octylamino, n-nonylamino, n-decylamino, n-undecylamino, n-dodecylamino, n-tridecylamino, n-tetradecylamino, and n-pentadecylamino, which are C₁ to C₁₅ straight chain or branched chain N-alkyl amino groups.

Examples of the N-alkenyl-amino group include, for example, vinyl amino, (prop-1-en-1-yl)amino, allylamino, isopropenylamino, (but-1-en-1-yl)amino, (but-2-en-1-yl)amino, (but-3-en-1-yl)amino, (2-methylprop-2-en-1-yl)amino, (1-methylprop-2-en-1-yl)amino, (pent-1-en-1-yl)amino, (pent-2-en-1-yl)amino, (pent-3-en-1-yl)amino, (pent-4-en-1-yl)amino, (3-methylbut-2-en-1-yl)amino, (3-methylbut-3-en-1-yl)amino, (hex-1-en-1-yl)amino, (hex-2-en-1-yl)amino, (hex-3-en-1-yl)amino, (hex-4-en-1-yl)amino, (hex-5-en-1-yl)amino, (4-methylpent-3-en-1-yl)amino, (4-methylpent-3-en-1-yl)amino, (hept-1-en-1-yl)amino, (hept-6-en-1-yl)amino, (oct-1-en-1-yl)amino, (oct-7-en-1-yl)amino, (non-1-en-1-yl)amino, (non-8-en-1-yl)amino, (dec-1-en-1-yl)amino, (dec-9-en-1-yl)amino, (undec-1-en-1-yl)amino, (undec-10-en-1-yl)amino, (dodec-1-en-1-yl)amino, (dodec-11-en-1-yl)amino, (tridec-1-en-1-yl)amino, (tridec-12-en-1-yl)amino, (tetradec-1-en-1-yl)amino, (tetradec-13-en-1-yl)amino, (pentadec-1-en-1-yl)amino, and (pentadec-14-en-1-yl)amino, which are C₂ to C₁₅ straight chain or branched chain N-alkenyl amino groups.

Examples of the N-alkynyl-amino group include, for example, ethynylamino, (prop-1-yn-1-yl)amino, (prop-2-yn-1-yl)amino, (but-1-yn-1-yl)amino, (but-3-yn-1-yl)amino, (1-methylprop-2-yn-1-yl)amino, (pent-1-yn-1-yl)amino, (pent-4-yn-1-yl)amino, (hex-1-yn-1-yl)amino, (hex-5-yn-1-yl)amino, (hept-1-yn-1-yl)amino, (hept-6-yn-1-yl)amino, (oct-1-yn-1-yl)amino, (oct-7-yn-1-yl)amino, (non-1-yn-1-yl)amino, (non-8-yn-1-yl)amino, (dec-1-yn-1-yl)amino, (dec-9-yn-1-yl)amino, (undec-1-yn-1-yl)amino, (undec-10-yn-1-yl)amino, (dodec-1-yn-1-yl)amino, (dodec-11-yn-1-yl)amino, (tridec-1-yn-1-yl)amino, (tridec-12-yn-1-yl)amino, (tetradec-1-yn-1-yl)amino, (tetradec-13-yn-1-yl)amino, (pentadec-1-yn-1-yl)amino, and (pentadec-14-yn-1-yl)amino, which are C₂ to C₁₅ straight chain or branched chain N-alkynyl-amino groups.

Examples of the N-cycloalkyl-amino group include, for example, cyclopropylamino, cyclobutylamino, cyclopentylamino, cyclohexylamino, cycloheptylamino, and cyclooctylamino, which are C₃ to C₈ N-cycloalkyl-amino groups.

Examples of the N-cycloalkyl-alkyl-amino group include, for example, (cyclopropylmethyl)amino, (1-cyclopropylethyl)amino, (2-cyclopropylethyl)amino, (3-cyclopropylpropyl)amino, (4-cyclopropylbutyl)amino, (5-cyclopropylpentyl)amino, (6-cyclopropylhexyl)amino, (cyclobutylmethyl)amino, (cyclopentylmethyl)amino, (cyclobutylmethyl)amino, (cyclopentylmethyl)amino, (cyclohexylmethyl)amino, (2-cyclohexylethyl)amino, (3-cyclohexylpropyl)amino, (4-cyclohexylbutyl)amino, (cycloheptylmethyl)amino, (cyclooctylmethyl)amino, and (6-cyclooctylhexyl)amino, which are C₄ to C₁₄ N-cycloalkyl-alkyl-amino groups.

Examples of the N-aryl-amino group include, for example, phenylamino, 1-naphthylamino, 2-naphtylamino, anthrylamino, phenanthrylamino, and acenaphthylenylamino, which are C₆ to C₁₄ N-mono-arylamino groups.

Examples of the N-aralkyl-amino group include, for example, benzylamino, (1-naphthylmethyl)amino, (2-naphthylmethyl)amino, (anthracenylmethyl)amino, (phenanthrenylmethyl)amino, (acenaphthylenylmethyl)amino, (diphenylmethyl)amino, (1-phenethyl)amino, (2-phenethyl)amino, (1-(1-naphthyl)ethyl)amino, (1-(2-naphthyl)ethyl)amino, (2-(1-naphthyl)ethyl)amino, (2-(2-naphthyl)ethyl)amino, (3-phenylpropyl)amino, (3-(1-naphthyl)propyl)amino, (3-(2-naphthyl)propyl)amino, (4-phenylbutyl)amino, (4-(1-naphthyl)butyl)amino, (4-(2-naphthyl)butyl)amino, (5-phenylpentyl)amino, (5-(1-naphthyl)pentyl)amino, (5-(2-naphthyl)pentyl)amino, (6-phenylhexyl)amino, (6-(1-naphthyl)hexyl)amino, and (6-(2-naphthyl)hexyl)amino, which are C₇ to C₁₆ N-aralkyl-amino groups.

Examples of the N,N-di(hydrocarbon)-amino group include the groups in which two hydrogen atoms of amino group are substituted with hydrocarbon groups, and include, for example, N,N-dimethylamino, N,N-diethylamino, N-ethyl-N-methylamino, N,N-di-n-propylamino, N,N-diisopropylamino, N-allyl-N-methylamino, N-(prop-2-yn-1-yl)-N-methylamino, N,N-dicyclohexylamino, N-cyclohexyl-N-methylamino, N-cyclohexylmethylamino-N-methylamino, N,N-diphenylamino, N-methyl-N-phenylamino, N,N-dibenzylamino, and N-benzyl-N-methylamino.

Examples of the N-heterocyclic ring-amino group include the groups in which one hydrogen atom of amino group is substituted with a heterocyclic group, and include, for example, (3-pyrrolizinyl)amino, (4-piperidinyl)amino, (2-tetrahydropyranyl)amino, (3-indolinyl)amino, (4-chromanyl)amino, (3-thienyl)amino, (3-pyridyl)amino, (3-quinolyl)amino, and (5-indolyl)amino.

Examples of the N-hydrocarbon-N-heterocyclic ring-amino group include the groups in which two hydrogen atoms of amino group are substituted with hydrocarbon group and heterocyclic group respectively, and include, for example, N-methyl-N-(4-piperidinyl)amino, N-(4-chromanyl)-N-methylamino, N-methyl-N-(3-thienyl)amino, N-methyl-N-(3-pyridyl)amino, N-methyl-N-(3-quinolyl)amino.

Examples of the acyl-amino group include the groups in which one hydrogen atom of the amino group is substituted with an acyl group, and include, for example, formylamino group, glyoxyloylamino group, thioformylamino group, carbamoylamino group, thiocarbamoylamino group, sulfamoylamino group, sulfinamoylamino group, carboxyamino group, sulphoamino group, phosphonoamino group, and groups represented by the following formulas : 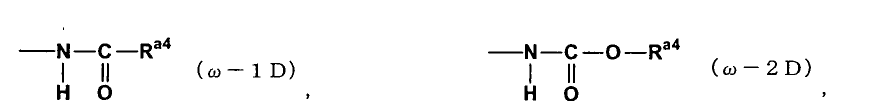 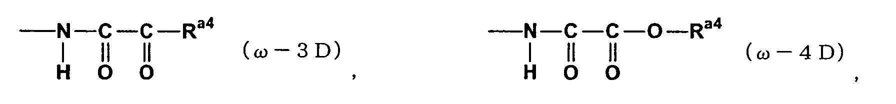 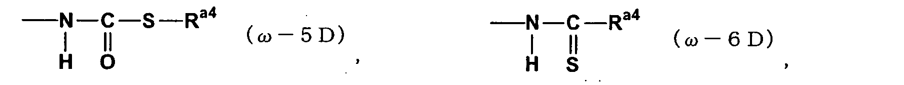 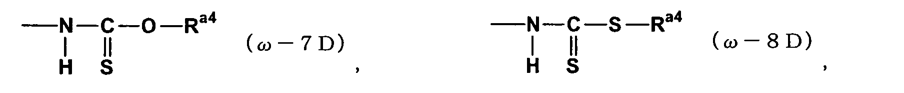 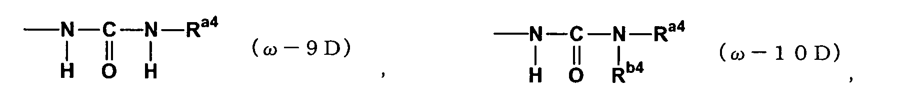 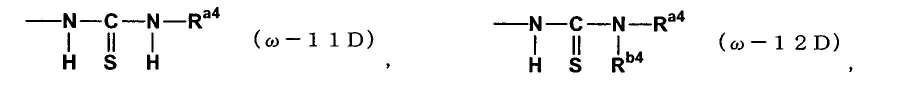 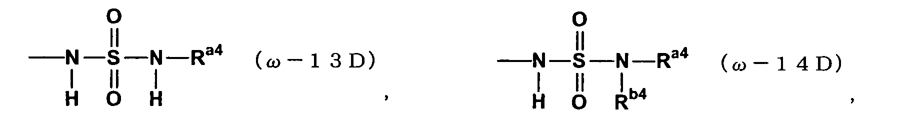 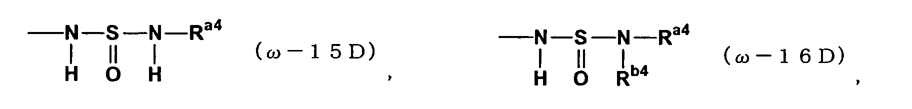 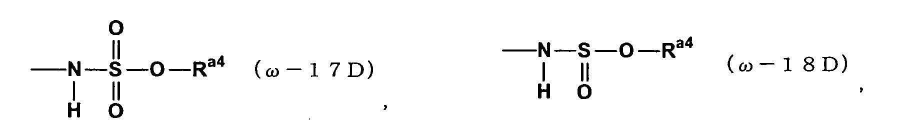 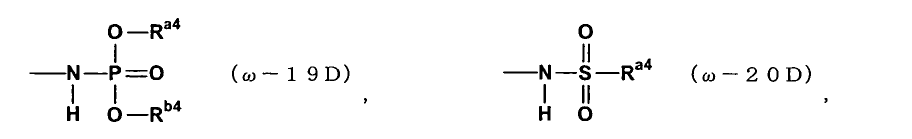 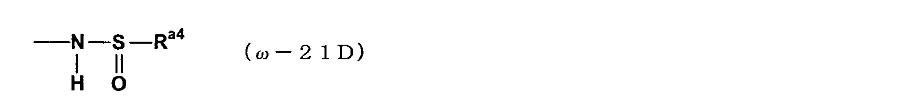 wherein R^{a4} and R^{b4} may be the same or different and represent a hydrocarbon group which may be substituted or a heterocyclic group which may be substituted, or R^{a4} and R^{b4} combine to each other, together with the nitrogen atom to which they bind, to form a cyclic amino group which may be substituted.

In the definition of the aforementioned acyl-amino group, among the groups represented by the formula (ω-1D), those groups in which R^{a4} is a hydrocarbon group are referred to as "hydrocarbon-carbonyl-amino group," and those groups in which R^{a4} is a heterocyclic group are referred to as "heterocyclic ring-carbonyl-amino group."

Among the groups represented by the formula (ω-2D), those groups in which R^{a4} is a hydrocarbon group are referred to as "hydrocarbon-oxy-carbonyl-amino group," and those groups in which R^{a4} is a heterocyclic group are referred to as "heterocyclic ring-oxy-carbonyl-amino group."

Among the groups represented by the formula (ω-3D), those groups in which R^{a4} is a hydrocarbon group are referred to as "hydrocarbon-carbonyl-carbonyl-amino group," and those groups in which R^{a4} is a heterocyclic group are referred to as "heterocyclic ring-carbonyl-carbonyl-amino group."

Among the groups represented by the formula (ω-4D), those groups in which R^{a4} is a hydrocarbon group are referred to as "hydrocarbon-oxy-carbonyl-carbonyl-amino group," and those groups in which R^{a4} is a heterocyclic group are referred to as "heterocyclic ring-oxy-carbonyl-carbonyl-amino group."

Among the groups represented by the formula (ω-5D), those groups in which R^{a4} is a hydrocarbon group are referred to as "hydrocarbon-sulfanyl-carbonyl-amino group," and those groups in which R^{a4} is a heterocyclic group are referred to as "heterocyclic ring-sulfanyl-carbonyl-amino group."

Among the groups represented by the formula (ω-6D), those groups in which R^{a4} is a hydrocarbon group are referred to as "hydrocarbon-thiocarbonyl-amino group," and those groups in which R^{a4} is a heterocyclic group are referred to as "heterocyclic ring-thiocarbonyl-amino group."

Among the groups represented by the formula (ω-7D), those groups in which R^{a4} is a hydrocarbon group are referred to as "hydrocarbon-oxy-thiocarbonyl-amino group," and those groups in which R^{a4} is a heterocyclic group are referred to as "heterocyclic ring-oxy-thiocarbonyl-amino group."

Among the groups represented by the formula (ω-8D), those groups in which R^{a4} is a hydrocarbon group are referred to as "hydrocarbon-sulfanyl-thiocarbonyl-amino group," and those groups in which R^{a4} is a heterocyclic group are referred to as "heterocyclic ring-sulfanyl-thiocarbonyl-amino group."

Among the groups represented by the formula (ω-9D), those groups in which R^{a4} is a hydrocarbon group are referred to as "N-hydrocarbon-carbamoyl group," and those groups in which R^{a4} is a heterocyclic group are referred to as "N-heterocyclic ring-carbamoyl-amino group."

Among the groups represented by the formula (ω-10D), those groups in which both R^{a4} and R^{b4} are hydrocarbon groups are referred to as "N,N-di(hydrocarbon)-carbamoyl-amino group," those groups in which both R^{a4} and R^{b4} are heterocyclic groups are referred to as "N,N-di(heterocyclic ring)-carbamoyl-amino group," those groups in which R^{a4} is a hydrocarbon group and R^{b4} is a heterocyclic group are referred to as "N-hydrocarbon-N-heterocyclic ring-carbamoyl-amino group," and those groups in which R^{a4} and R^{b4} combine to each other, together with the nitrogen atom to which they bind, to form a cyclic amino group are referred to as "cyclic amino-carbonyl-amino group."

Among the groups represented by the formula (ω-11D), those groups in which R^{a4} is a hydrocarbon group are referred to as "N-hydrocarbon-thiocarbamoyl-amino group," and those groups in which R^{a4} is a heterocyclic ring group are referred to as "N-heterocyclic-thiocarbamoyl-amino group."

Among the groups represented by the formula (ω-12D), those groups in which both R^{a4} and R^{b4} are hydrocarbon groups are referred to as "N,N-di(hydrocarbon)-thiocarbamoyl-amino group," those groups in which both R^{a4} and R^{b4} are heterocyclic groups are referred to as "N,N-di(heterocyclic ring)-thiocarbamoyl-amino group," those groups in which R^{a4} is a hydrocarbon group and R^{b4} is a heterocyclic group are referred to as "N-hydrocarbon-N-heterocyclic ring-thiocarbamoyl-amino group," and those groups in which R^{a4} and R^{b4} combine to each other, together with the nitrogen atom to which they bind, to form a cyclic amino group are referred to as "cyclic amino-thiocarbonyl-amino group."

Among the groups represented by the formula (ω-13D), those groups in which R^{a4} is a hydrocarbon group are referred to as "N-hydrocarbon-sulfamoyl-amino group," and those groups in which R^{a4} is a heterocyclic group are referred to as "N-heterocyclic ring-sulfamoyl-amino group."

Among the groups represented by the formula (ω-14D), those groups in which both R^{a4} and R^{b4} are hydrocarbon groups are referred to as "di(hydrocarbon)-sulfamoyl-amino group," those groups in which both R^{a4} and R^{b4} are heterocyclic groups are referred to as "N,N-di(heterocyclic ring)-sulfamoyl-amino group," those groups in which R^{a4} is a hydrocarbon group and R^{b4} is a heterocyclic group are referred to as "N-hydrocarbon-N-heterocyclic ring-sulfamoyl-amino group," and those groups in which R^{a4} and R^{b4} combine to each other, together with the nitrogen atom to which they bind, to form a cyclic amino group are referred to as "cyclic amino-sulfonyl-amino group."

Among the groups represented by the formula (ω-15D), those groups in which R^{a4} is a hydrocarbon group are referred to as "N-hydrocarbon-sulfinamoyl-amino group," and those groups in which R^{a4} is a heterocyclic group are referred to as "N-heterocyclic ring-sulfinamoyl-amino group."

Among the groups represented by the formula (ω-16D), those groups in which both R^{a4} and R^{b4} are hydrocarbon groups are referred to as "N,N-di(hydrocarbon)-sulfinamoyl-amino group," those groups in which both R^{a4} and R^{b4} are heterocyclic groups are referred to as "N,N-di(heterocyclic ring)-sulfinamoyl-amino group," groups in which R^{a4} is a hydrocarbon group and R^{b4} is a heterocyclic group are referred to as "N-hydrocarbon-N-heterocyclic ring-sulfinamoyl-amino group," and those groups in which R^{a4} and R^{b4} combine to each other, together with the nitrogen atom to which they bind, to form a cyclic amino group are referred to as "cyclic amino-sulfinyl-amino group."

Among the groups represented by the formula (ω-17D), those groups in which R^{a4} is a hydrocarbon group are referred to as "hydrocarbon-oxy-sulfonyl-amino group," and those groups in which R^{a4} is a heterocyclic group are referred to as "heterocyclic ring-oxy-sulfoyl-amino group."

Among the groups represented by the formula (ω-18D), those groups in which R^{a4} is a hydrocarbon group are referred to as "hydrocarbon-oxy-sulfinyl-amino group," and those groups in which R^{a4} is a heterocyclic group are referred to as "heterocyclic ring-oxy-sulfinyl-amino group."

Among the groups represented by the formula (ω-19D), those groups in which both R^{a4} and R^{b4} are hydrocarbon groups are referred to as "O,O'-di(hydrocarbon)-phosphono-amino group," those groups in which both R^{a4} and R^{b4} are heterocyclic groups are referred to as "O,O'-di(heterocyclic ring)-phosphono-amino group," and those groups in which R^{a4} is a hydrocarbon group and R^{b4} is a heterocyclic group are referred to as "O-hydrocarbon-O'-heterocyclic ring-phosphono-amino group."

Among the groups represented by the formula (ω-20D), those groups in which R^{a4} is a hydrocarbon group are referred to as "hydrocarbon-sulfonyl-amino group," and those groups in which R^{a4} is a heterocyclic group are referred to as "heterocyclic ring-sulfonyl-amino group."

Among the groups represented by the formula (ω-21D), those groups in which R^{a4} is a hydrocarbon group are referred to as "hydrocarbon-sulfinyl-amino group," and those groups in which R^{a4} is a heterocyclic group are referred to as "heterocyclic ring-sulfinyl-amino group."

Examples of the hydrocarbon in the groups represented by the aforementioned formulas (ω-1D) through (ω-21D) include the similar groups to the aforementioned hydrocarbon group. Examples of the hydrocarbon-carbonyl-amino groups represented by the formula (ω-1D) include, for example, an alkyl-carbonyl-amino group, an alkenyl-carbonyl-amino group, an alkynyl-carbonyl-amino group, a cycloalkyl-carbonyl-amino group, a cycloalkenyl-carbonyl-amino group, a cycloalkanedienyl-carbonyl-amino group, a cycloalkyl-alkyl-carbonyl-amino group which are aliphatic hydrocarbon-carbonyl-amino groups; an aryl-carbonyl-amino group; an aralkyl-carbonyl-amino group; a bridged cyclic hydrocarbon-carbonyl-amino group; a spiro cyclic hydrocarbon-carbonyl-amino group; and a terpene family hydrocarbon-carbonyl-amino group. In the following, groups represented by the formulas (ω-2D) through (ω-21D) are similar to those explained above.

Examples of the heterocyclic ring in the groups represented by the aforementioned formulas (ω-1D) through (ω-21D) include similar groups to the aforementioned heterocyclic group. Examples of the heterocyclic ring-carbonyl-amino group represented by the formula (ω-1D) include, for example, a monocyclic heteroaryl-carbonyl-amino group, a fused polycyclic heteroaryl-carbonyl-amino group, a monocyclic non-aromatic heterocyclic-carbonyl-amino group, and a fused polycyclic non-aromatic heterocyclic-carbonyl-amino group. In the following, groups represented by the formulas (ω-2D) through (ω-21D) are similar to those groups explained above.

Examples of the cyclic amino in the groups represented by the aforementioned formulas (ω-10D) through (ω-16D) include similar groups to the aforementioned cyclic amino group.

Examples of the di(acyl)-amino group include the groups in which two hydrogen atoms of amino group are substituted with acyl groups in the definitions of the aforementioned substituents according to "which may be substituted." Examples include, for example, di(formyl)-amino group, di(glyoxyloyl)-amino group, di(thioformyl)-amino group, di(carbamoyl)-amino group, di(thiocarbamoyl)-amino group, di(sulfamoyl)-amino group, di(sulfinamoyl)-amino group, di(carboxy)-amino group, di(sulfo)-amino group, di(phosphono)-amino group, and groups represented by the following formulas : 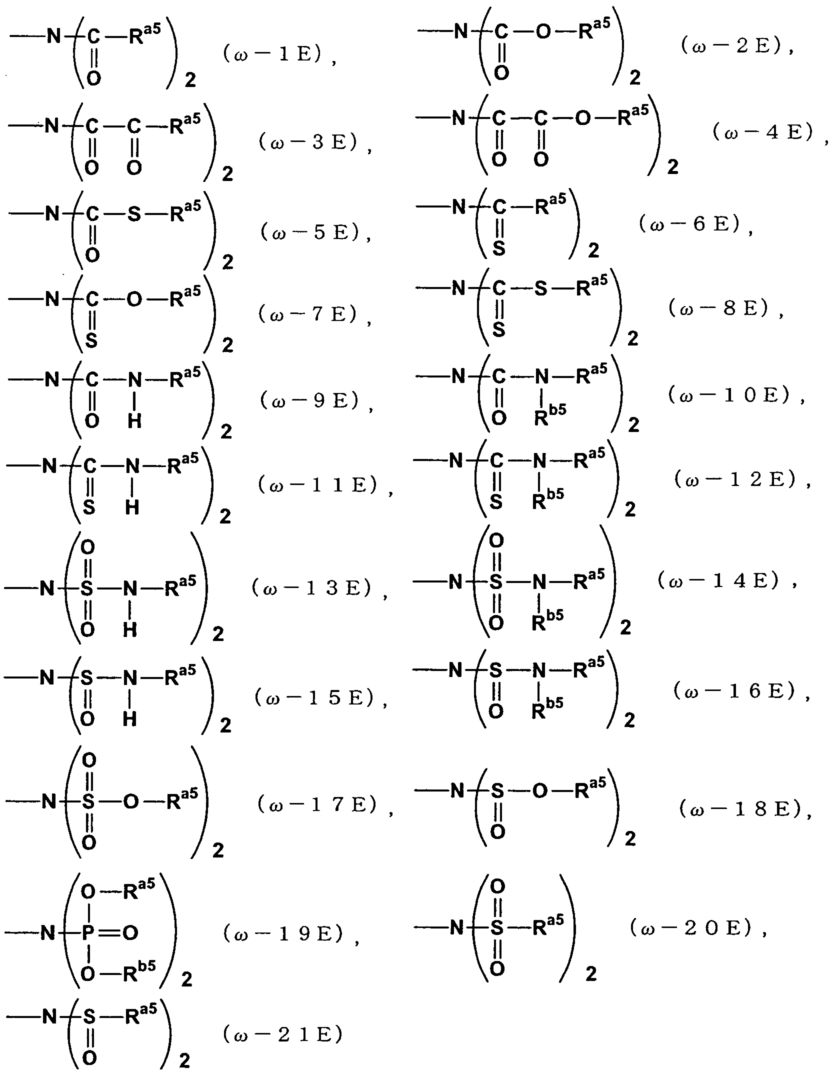 wherein R^{a5} and R^{b5} may be the same or different and represent hydrogen atom, a hydrocarbon group which may be substituted or a heterocyclic group which may be substituted, or R^{a5} and R^{b5} combine to each other, together with the nitrogen atom to which they bind, to form a cyclic amino group which may be substituted.

In the definition of aforementioned di(acyl)-amino group, among the groups represented by the formula (ω-1E), those groups in which R^{a5} is a hydrocarbon group are referred to as "bis(hydrocarbon-carbonyl)-amino group," and those groups in which R^{a5} is a heterocyclic group are referred to as "bis(heterocyclic ring-carbonyl)-amino group."

Among the groups represented by the formula (ω-2E), those groups in which R^{a5} is a hydrocarbon group are referred to as "bis(hydrocarbon-oxy-carbonyl)-amino group," and those groups in which R^{a5} is a heterocyclic group are referred to as "bis(heterocyclic ring-oxy-carbonyl)-amino group."

Among the groups represented by the formula (ω-3E), those groups in which R^{a5} is a hydrocarbon group are referred to as "bis(hydrocarbon-carbonyl-carbonyl)-amino group," and those groups in which R^{a5} is a heterocyclic group are referred to as "bis(heterocyclic ring-carbonyl-carbonyl)-amino group."

Among the groups represented by the formula (ω-4E), those groups in which R^{a5} is a hydrocarbon group are referred to as "bis(hydrocarbon-oxy-carbonyl-carbonyl)-amino group," and those groups in which R^{a5} is a heterocyclic group are referred to as "bis(heterocyclic ring-oxy-carbonyl-carbonyl)-amino group."

Among the groups represented by the formula (ω-5E), those groups in which R^{a5} is a hydrocarbon group are referred to as "bis(hydrocarbon-sulfanyl-carbonyl)-amino group," and those groups in which R^{a5} is a heterocyclic group are referred to as "bis(heterocyclic ring-sulfanyl-carbonyl)-amino group. "

Among the groups represented by the formula (ω-6E), those groups in which R^{a5} is a hydrocarbon group are referred to as "bis(hydrocarbon-thiocarbonyl)-amino group," and those groups in which R^{a5} is a heterocyclic group are referred to as "bis(heterocyclic ring-thiocarbonyl)-amino group."

Among the groups represented by the formula (ω-7E), those groups in which R^{a5} is a hydrocarbon group are referred to as "bis(hydrocarbon-oxy-thiocarbonyl)-amino group," and those groups in which R^{a5} is a heterocyclic group are referred to as "bis(heterocyclic ring-oxy-thiocarbonyl)-amino group."

Among the groups represented by the formula (ω-8E), those groups in which R^{a5} is a hydrocarbon group are referred to as "bis(hydrocarbon-sulfanyl-thiocarbonyl)-amino group," and those groups in which R^{a5} is a heterocyclic group are referred to as "bis(heterocyclic ring-sulfanyl-thiocarbonyl)-amino group."

Among the groups represented by the formula (ω-9E), those groups in which R^{a5} is a hydrocarbon group are referred to as "bis(N-hydrocarbon-carbamoyl)-amino group," and those groups in which R^{a5} is a heterocyclic group are referred to as "bis(N-heterocyclic ring-carbamoyl)-amino group."

Among the groups represented by the formula (ω-10E), those groups in which both R^{a5} and R^{b5} are hydrocarbon groups are referred to as "bis[N,N-di(hydrocarbon)-carbamoyl]-amino group," those groups in which both R^{a5} and R^{b5} are heterocyclic groups are referred to as "bis[N,N-di(heterocyclic ring)-carbamoyl]-amino group," groups in which R^{a5} is a hydrocarbon group and R^{b5} is a heterocyclic group are referred to as "bis(N-hydrocarbon-N-heterocyclic ring-carbamoyl)-amino group," and those groups in which R^{a5} and R^{b5} combine to each other, together with the nitrogen atom to which they bind, to form a cyclic amino groups are referred to as "bis(cyclic amino-carbonyl)amino group."

Among the groups represented by the formula (ω-11E), those groups in which R^{a5} is a hydrocarbon group are referred to as "bis(N-hydrocarbon-thiocarbamoyl)-amino group," and those groups in which R^{a5} is a heterocyclic group are referred to as "bis(N-heterocyclic ring-thiocarbamoyl)-amino group."

Among the groups represented by the formula (ω-12E), those groups in which both R^{a5} and R^{b5} are hydrocarbon groups are referred to as "bis[N,N-di(hydrocarbon)-thiocarbamoyl]-amino group," those groups in which both R^{a5} and R^{b5} are heterocyclic groups are referred to as "bis[N,N-di(heterocyclic ring)-thiocarbamoyl]-amino group," those groups in which R^{a5} is a hydrocarbon group and R^{b5} is a heterocyclic group are referred to as "bis(N-hydrocarbon-N-heterocyclic ring-thiocarbamoyl)-amino group," and those groups in which R^{a5} and R^{b5} combine to each other, together with the nitrogen atom to which they bind, to form a cyclic amino group are referred to as "bis(cyclic amino-thiocarbonyl)-amino group."

Among the groups represented by the formula (ω-13E), those groups in which R^{a5} is a hydrocarbon group are referred to as "bis(N-hydrocarbon-sulfamoyl)-amino group," and those groups in which R^{a5} is a heterocyclic group are referred to as "bis(N-heterocyclic ring-sulfamoyl)-amino group."

Among the groups represented by the formula (ω-14E), those groups in which both R^{a5} and R^{b5} are hydrocarbon groups are referred to as "bis[N,N-di(hydrocarbon)-sulfamoyl]-amino group," those groups in which both R^{a5} and R^{b5} are heterocyclic groups are referred to as "bis[N,N-di(heterocyclic ring)-sulfamoyl]-amino group," those groups in which R^{a5} is a hydrocarbon group and R^{b5} is a heterocyclic group are referred to as "bis(N-hydrocarbon-N-heterocyclic ring-sulfamoyl)-amino group," and those groups in which R^{a5} and R^{b5} combine to each other, together with the nitrogen atom to which they bind, to form a cyclic amino group are referred to as "bis(cyclic amino-sulfonyl)amino group."

Among the groups represented by the formula (ω-15E), those groups in which R^{a5} is a hydrocarbon group are referred to as "bis(N-hydrocarbon-sulfinamoyl)-amino group," and those groups in which R^{a5} is a heterocyclic group are referred to as "bis(N-heterocyclic ring-sulfinamoyl)-amino group."

Among the groups represented by the formula (ω-16E), those groups in which R^{a5} and R^{b5} are hydrocarbon groups are referred to as "bis[N,N-di(hydrocarbon)-sulfinamoyl]-amino group," those groups in which R^{a5} and R^{b5} are heterocyclic groups are referred to as "bis[N,N-di(heterocyclic ring)-sulfinamoyl]-amino group," those groups in which R^{a5} is a hydrocarbon group and R^{b5} is a heterocyclic group are referred to as "bis(N-hydrocarbon-N-heterocyclic ring-sulfinamoyl)-amino group," and those groups in which R^{a5} and R^{b5} combine to each other, together with the nitrogen atom to which they bind, to form a cyclic amino group are referred to as "bis(cyclic amino-sulfinyl)amino group."

Among the groups represented by the formula (ω-17E), those groups in which R^{a5} is a hydrocarbon group are referred to as "bis(hydrocarbon-oxy-sulfonyl)-amino group," and those groups in which R^{a5} is a heterocyclic group are referred to as "bis(heterocyclic ring-oxy-sulfonyl)-amino group."

Among the groups represented by the formula (ω-18E), those groups in which R^{a5} is a hydrocarbon group are referred to as "bis(hydrocarbon-oxy-sulfinyl)-amino group," and those groups in which R^{a5} is a heterocyclic group are referred to as "bis(heterocyclic ring-oxy-sulfinyl)-amino group."

Among the groups represented by the formula (ω-19E), those groups in which both R^{a5} and R^{b5} are hydrocarbon groups are referred to as "bis[O,O'-di(hydrocarbon)-phosphono]-amino group," those groups in which both R^{a5} and R^{b5} are heterocyclic groups are referred to as "bis[O,O'-di(heterocyclic ring)-phosphono]-amino group," and those groups in which R^{a5} is a hydrocarbon group and R^{b5} is a heterocyclic group are referred to as "bis(O-hydrocarbon-O'-heterocyclic ring-phosphono)-amino group."

Among the groups represented by the formula (ω-20E), those groups in which R^{a5} is a hydrocarbon group are referred to as "bis(hydrocarbon-sulfonyl)-amino group," and those groups in which R^{a5} is a heterocyclic group are referred to as "bis(heterocyclic ring-sulfonyl)-amino group."

Among the groups represented by the formula (ω-21E), those groups in which R^{a5} is a hydrocarbon group are referred to as "bis(hydrocarbon-sulfinyl)-amino group," and those groups in which R^{a5} is a heterocyclic group are referred to as "bis(heterocyclic ring-sulfinyl)-amino group."

Examples of the hydrocarbon in the groups represented by the aforementioned formulas (ω-1E) through (ω-21E) include the similar groups to the aforementioned hydrocarbon group. Examples of the bis(hydrocarbon-carbonyl)-amino groups represented by the formula (ω-1E) include, for example, a bis(alkyl-carbonyl)-amino group, a bis(alkenyl-carbonyl)-amino group, a bis(alkynyl-carbonyl)-amino group, a bis(cycloalkyl-carbonyl)-amino group, a bis(cycloalkenyl-carbonyl)-amino group, a bis(cycloalkanedienyl-carbonyl)-amino group, a bis(cycloalkyl-alkyl-carbonyl)-amino group which are bis(aliphatic hydrocarbon-carbonyl)-amino groups; a bis(aryl-carbonyl)-amino group; a bis(aralkyl-carbonyl)-amino group; a bis(bridged cyclic hydrocarbon-carbonyl)-amino group; a bis(spiro cyclic hydrocarbon-carbonyl)-amino group; and a bis(terpene family hydrocarbon-carbonyl)-amino group. In the following, groups represented by the formulas (ω-2E) through (ω-21E) are similar to those explained above.

Examples of the heterocyclic ring in the groups represented by the aforementioned formulas (ω-1E) through (ω-21E) include similar groups to the aforementioned heterocyclic group. Examples of the bis(heterocyclic ring-carbonyl)-amino group represented by the formula (ω-1E) include, for example, a bis(monocyclic heteroaryl-carbonyl)-amino group, a bis(fused polycyclic heteroaryl-carbonyl)-amino group, a bis(monocyclic non-aromatic heterocyclic-carbonyl)-amino group, and a bis(fused polycyclic non-aromatic heterocyclic-carbonyl)-amino group. In the following, groups represented by the formulas (ω-2E) through (ω-21E) are similar to those groups explained above.

Examples of the cyclic amino in the groups represented by the aforementioned formulas (ω-10E) through (ω-16E) include similar groups to the aforementioned cyclic amino group.

The aforementioned acyl-amino group and di(acyl)-amino group are generically referred to as "acyl substituted amino group." Furthermore, the aforementioned N-hydrocarbon-amino group, N,N-di(hydrocarbon)-amino group, N-heterocyclic-amino group, N-hydrocarbon-N-heterocyclic-amino group, cyclic amino group, acyl-amino group, and di(acyl)-amino group are generically referred to as "substituted amino group."

In the following, compounds represented by the aforementioned general formula (I) are explained in details.

"Connecting group whose number of atoms of main chain is 2 to 5" in the definition of X means connecting groups wherein 2 to 5 atoms in a main chain link together between rings Z and E. The aforementioned "number of atoms of the main chain" is counted so as to minimize the number of connecting atoms existing between the rings Z and E, regardless of the presence or absence of hetero atom(s). For example, the number of atoms of 1,2-cyclopentylene is counted as 2, the number of atoms of 1,3-cyclopentylene is counted as 3, the number of atoms of 1,4-phenylene is counted as 4, and the number of atoms of 2,6-pyridine-diyl is counted as 3.

The aforementioned "connecting group whose number of atoms of main chain is 2 to 5" is formed by one functional group selected from the following group of divalent group ζ-1, or formed by combining 2 to 4 functional groups of 1 to 4 kinds selected from the following divalent group ζ-2.
[Divalent group ζ-1] the following formulas: 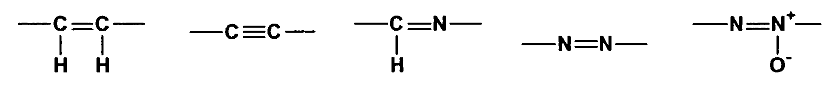 [Divalent group ζ -2] the following formulas: 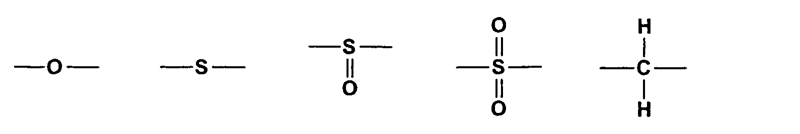 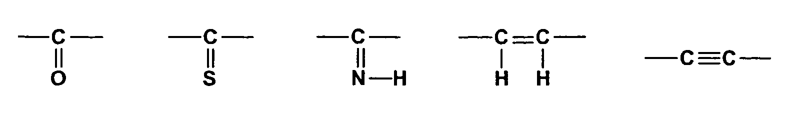 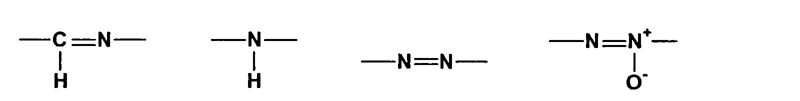 When 2 or more divalent groups combine, each group may be the same or different.

The aforementioned "connecting group wherein the number of atoms of the main chain is 2 to 5," is preferably a group selected from the following "connecting group α."
[Connecting group α] the following formulas: 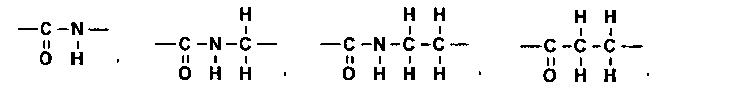 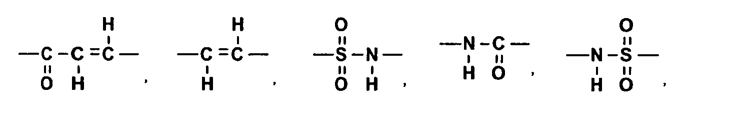 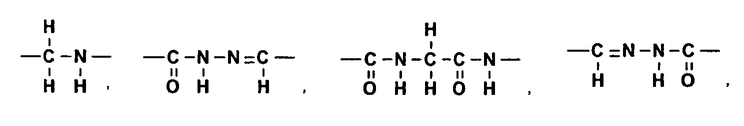 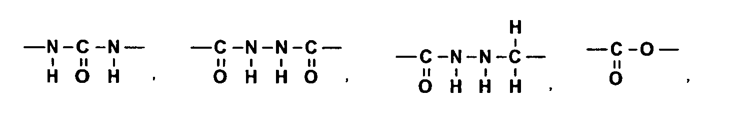 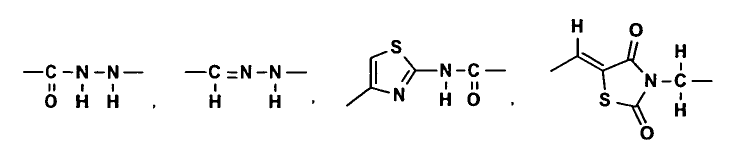 wherein a bond at the left end binds to ring Z and a bond at the right end binds to E.

The group represented by the following formula is most preferred: 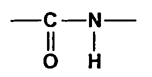 wherein the bond at the left end binds to ring Z and the bond at the right end binds to E.

Examples of the substituent, according to "connecting group which may be substituted" in the definition of "a connecting group whose number of atoms of the main chain is 2 to 5," include similar groups to the substituents in the definition of the aforementioned "which may be substituted." A C₁ to C₆ alkyl group is preferred, and a methyl group is more preferred. The substituent may combine with a substituent of the ring E or Z, together with atoms to which they bind, to form a cyclic group which may be substituted. Examples include the compounds represented by the general formula (I) being those represented by the following formulas:

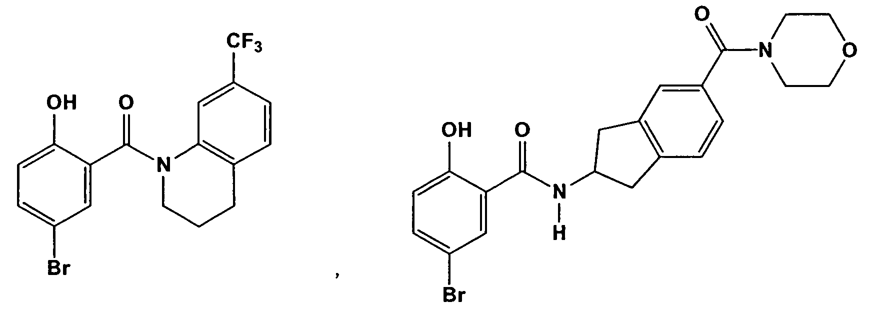

In the aforementioned general formula (I), examples of A include hydrogen atom or acetyl group, and hydrogen atom is preferred.

Examples of the "arene" in "an arene which may have one or more substituents in addition to the group represented by formula -O-A wherein A has the same meaning as that defined above and the group represented by formula -X-E wherein each of X and E has the same meaning as that defined above" in the definition of ring Z include a monocyclic or fused heterocyclic aromatic hydrocarbon, and include, for example, benzene ring, naphthalene ring, anthracene ring, phenanthrene ring, and acenaphylene ring. C₆ to C₁₀ arenes such as benzene ring, naphthalene ring and the like are preferred, benzene ring and naphthalene ring are more preferred, and benzene ring is most preferred.

Examples of the substituent in the definition of "an arene which may have one or more substituents in addition to the group represented by formula -O-A wherein A has the same meaning as that defined above and the group represented by formula -X-E wherein each of X and E has the same meaning as that defined above" in the aforementioned definition of ring Z include similar groups to the substituent explained for the definition "which may be substituted." The position of substituents existing on the arene is not particularly limited, and when two or more substituents exist, they may be the same or different.

When "an arene which may have one or more substituents in addition to the group represented by formula -O-A wherein A has the same meaning as that defined above and the group represented by formula -X-E wherein each of X and E has the same meaning as that defined above" in the aforementioned definition of ring Z is "a benzene ring which may have one or more substituents in addition to the group represented by formula -O-A wherein A has the same meaning as that defined above and the group represented by formula -X-E wherein each of X and E has the same meaning as that defined above," "a benzene ring which has one to three substituents in addition to the group represented by formula -O-A wherein A has the same meaning as that defined above and the group represented by formula -X-E wherein each of X and E has the same meaning as that defined above" is preferred, and "a benzene ring which has one substituent in addition to the group represented by formula -O-A wherein A has the same meaning as that defined above and the group represented by formula -X-E wherein each of X and E has the same meaning as that defined above" is more preferred. Preferred examples of the said substituents include groups selected from the following Substituent Group γ-1z. Halogen atom and tert-butyl group [(1,1-dimethyl)ethyl group] are more preferred, and halogen atom is most preferred.
[Substituent Group γ-1z] halogen atom, nitro group, cyano group, hydroxy group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group [thiophen-2-yl group], 3-thienyl group [thiophen-3-yl group], 1-pyrrolyl group [pyrrol-1-yl group], 2-methylthiazol-4-yl group, imidazo[1,2-a]pyridin-2-yl group, 2-pyridyl group [pyridin-2-yl group], acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, and {[4-(pyridin-2-yl)sulfamoyl]phenyl}diazenyl group

When "an arene which may have one or more substituents in addition to the group represented by formula -O-A wherein A has the same meaning as that defined above and the group represented by formula -X-E wherein each of X and E has the same meaning as that defined above" in the aforementioned definition of ring Z is "a benzene ring which may have one or more substituents in addition to the group represented by formula -O-A wherein A has the same meaning as that defined above and the group represented by formula -X-E wherein each of X and E has the same meaning as that defined above," it is most preferable that one substituent exists and locates on the position of R^{z} when the following partial formula (Iz-1) in the general formula containing ring Z 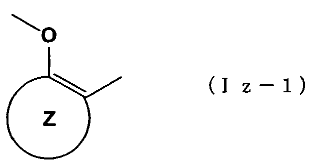 is represented by the following formula (Iz-2). 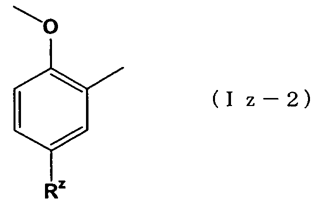 At this time, the said substituents can be defined as R^{z}. Preferred examples of R^{z} include a group selected from the following Substituent Group γ-2z. Halogen atom and tert-butyl group are more preferred, and halogen atom is most preferred.
[Substituent Group γ-2z] halogen atom, nitro group, cyano group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group, 3-thienyl group, 1-pyrrolyl group, 2-methylthiazol-4-yl group, imidazo[1,2-a]pyridin-2-yl group, 2-pyridyl group, acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, and {[4-(pyridin-2-yl)sulfamoyl]phenyl}diazenyl group

When "an arene which may have one or more substituents in addition to the group represented by formula -O-A wherein A has the same meaning as that defined above and the group represented by formula -X-E wherein each of X and E has the same meaning as that defined above" in the aforementioned definition of ring Z is "a naphthalene ring which may have one or more substituents in addition to the group represented by formula -O-A wherein A has the same meaning as that defined above and the group represented by formula -X-E wherein each of X and E has the same meaning as that defined above," naphthalene ring is preferred.

Examples of the "hetero arene" in "a hetero arene which may have one or more substituents in addition to the group represented by formula -O-A wherein A has the same meaning as that defined above and the group represented by formula -X-E wherein each of X and E has the same meaning as that defined above" in the aforementioned definition of ring Z include a monocyclic or a fused polycyclic aromatic heterocyclic rings containing at least one of 1 to 3 kinds of heteroatoms selected from oxygen atom, sulfur atom and nitrogen atom and the like as ring-constituting atoms (ring forming atoms), and include, for example, furan ring, thiophene ring, pyrrole ring, oxazole ring, isoxazole ring, thiazole ring, isothiazole ring, imidazole ring, pyrazole ring, 1,2,3-oxadiazole ring, 1,2,3-thiadiazole ring, 1,2,3-triazole ring, pyridine ring, pyridazine ring, pyrimidine ring, pyrazine ring, 1,2,3-triazine ring, 1,2,4-triazine ring, 1H-azepine ring, 1,4-oxepine ring, 1,4-thiazepine ring, benzofuran ring, isobenzofuran ring, benzo[b]thiophene ring, benzo[c]thiophene ring, indole ring, 2H-isoindole ring, 1H-indazole ring, 2H-indazole ring, benzoxazole ring, 1,2-benzisoxazole ring, 2,1-benzisoxazole ring, benzothiazole ring, 1,2-benzisothiazole ring, 2,1-benzisothiazole ring, 1,2,3-benzoxadiazol ring, 2,1,3-benzoxadiazol ring, 1,2,3-benzothiadiazole ring, 2,1,3-benzothiadiazole ring, 1H-benzotriazole ring, 2H-benzotriazole ring, quinoline ring, isoquinoline ring, cinnoline ring, quinazoline ring, quinoxaline ring, phthalazine ring, naphthyridine ring, 1H-1,5-benzodiazepine ring, carbazole ring, α-carboline ring, β-carboline ring, γ-carboline ring, acridine ring, phenoxazine ring, phenothiazine ring, phenazine ring, phenanthridine ring, phenanthroline ring, thianthrene ring, indolizine ring, and phenoxathiine ring, which are 5 to 14-membered monocyclic or fused polycyclic aromatic heterocyclic rings. 5 to 13-membered monocyclic or fused polycyclic aromatic heterocyclic rings are preferred, and thiophene ring, pyridine ring, indole ring, quinoxaline ring, and carbazole ring are more preferred.

Examples of the substituent in the definition of "a hetero arene which may have one or more substituents in addition to the group represented by formula -O-A wherein A has the same meaning as that defined above and the group represented by formula -X-E wherein each of X and E has the same meaning as that defined above" in the aforementioned definition of ring Z include similar groups to the substituent explained for the aforementioned definition "which may be substituted." The position of substituents existing on the hetero arene is not particularly limited, and when two or more substituents exist, they may be the same or different.

Halogen atoms are preferred as the substituent in the definition of "a hetero arene which may have one or more substituents in addition to the group represented by formula -O-A wherein A has the same meaning as that defined above and the group represented by formula -X-E wherein each of X and E has the same meaning as that defined above" in the aforementioned definition of ring Z.

Examples of the aryl group of "an aryl group which may be substituted" in the definition of E include similar groups to the aryl group in the definition of the aforementioned "hydrocarbon group," and C₆ to C₁₀ aryl groups such as phenyl group, 1-naphthyl group, 2-naphthyl group and the like are preferred, and phenyl group is most preferred.

Examples of the substituent in the definition of "an aryl group which may be substituted" in the definition of E include similar groups to the substituent explained for the definition "which may be substituted." The position of substituents existing on the aryl group is not particularly limited, and when two or more substituents exist, they may be the same or different.

When "an aryl group which may be substituted" in the aforementioned definition of E is "a phenyl group which may be substituted," "a mono-substituted phenyl group," "a di-substituted phenyl group," and "a phenyl group which has three or more substituents" are preferred, and "a di-substituted phenyl group" is more preferred.

When "an aryl group which may be substituted" in the aforementioned definition of E is "a di-substituted phenyl group," preferred examples of the group include groups represented by the following Substituent Group δ-1e.
[Substituent Group δ-1e] 3,5-bis(trifluoromethyl)phenyl group, 3,4-propylenedioxyphenyl group, 3,5-dichlorophenyl group, 2,4-dihydroxyphenyl group, 2,5-dimethoxyphenyl group, 2-chloro-5-(trifluoromethyl)phenyl group, 3,5-bis[(1,1-dimethyl)ethyl]phenyl group, 2,5-bis(trifluoromethyl)phenyl group, 4-chloro-2-(trifluoromethyl)phenyl group, 2-fluoro-3-(trifluoromethyl)phenyl group, 4-fluoro-3-(trifluoromethyl)phenyl group, 4-chloro-3-(trifluoromethyl)phenyl group, 3-fluoro-5-(trifluoromethyl)phenyl group, 3-bromo-5-(trifluoromethyl)phenyl group, 2-fluoro-5-(trifluoromethyl)phenyl group, 4-nitro-3-(trifluoromethyl)phenyl group, 2-nitro-5-(trifluoromethyl)phenyl group, 4-cyano-3-(trifluoromethyl)phenyl group, 2-methyl-3-(trifluoromethyl)phenyl group, 4-methyl-3-(trifluoromethyl)phenyl group, 2-methyl-5-(trifluoromethyl)phenyl group, 4-methoxy-3-(trifluoromethyl)phenyl group, 3-methoxy-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2-chloro-4-(trifluoromethyl)phenyl group, 2,5-dichlorophenyl group, 3,4-dichlorophenyl group, 3,5-difluorophenyl group, 3,5-dinitrophenyl group, 2,5-bis[(1,1-dimethyl)ethyl]phenyl group, 5-[(1,1-dimethyl)ethyl]-2-methoxyphenyl group, 3,5-dimethylphenyl group, 4-methoxybiphenyl-3-yl group, 3,5-dimethoxyphenyl group, 3,5-bis(methoxycarbonyl)phenyl group, 2-bromo-5-(trifluoromethyl)phenyl group, 3-methoxycarbonyl-5-(trifluoromethyl)phenyl group, 3-carboxy-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 3,5-dicarboxyphenyl group, 5-isopropyl-2-methylphenyl group, 2,5-diethoxyphenyl group, 2,5-dimethylphenyl group, 5-chloro-2-cyano group, 5-diethylsulfamoyl-2-methoxyphenyl group, 2-chloro-5-nitrophenyl group, 2-methoxy-5-(phenylcarbamoyl)phenyl group, 5-acetylamino-2-methoxyphenyl group, 5-methoxy-2-methylphenyl group, 2,5-dibutoxyphenyl group, 2,5-diisopentyloxy group, 5-carbamoyl-2-methoxyphenyl group, 5-[(1,1-dimethyl)propyl]-2-phenoxyphenyl group, 2-hexyloxy-5-methanesulfonyl group, 5-(2,2-dimethylpropionyl)-2-methylphenyl group, 5-methoxy-2-(1-pyrrolyl)phenyl group, 5-chloro-2-(p-toluenesulfonyl)phenyl group, 2-chloro-5-(p-toluenesulfonyl)phenyl group, 2-fluoro-5-methanesulfonyl group, 2-methoxy-5-phenoxy group, 4-methylbiphenyl-3-yl group, 2-methoxy-5-(1-methyl-1-phenylethyl)phenyl group, 5-morpholino-2-nitrophenyl group, 5-fluoro-2-(1-imidazolyl)phenyl group, 2-butyl-5-nitrophenyl group, 5-[(1,1-dimethyl)]propyl-2-hydroxyphenyl group, 2-methoxy-5-methylphenyl group, 2,5-difluorophenyl group, 4-isopropyl-2-(trifluoromethyl)phenyl group, 2-nitro-4-(trifluoromethyl)phenyl group, 4-bromo-3-(trifluoromethyl)phenyl group, 4-bromo-2-(trifluoromethyl)phenyl group, 2-bromo-4-(trifluoromethyl)phenyl group, 4-fluoro-2-(trifluoromethyl)phenyl group, 4-isopropoxy-2-(trifluoromethyl)phenyl group, 4-cyano-2-(trifluoromethyl)phenyl group, 2,6-diisopropylphenyl group, 2,6-dimethylphenyl group, 3,4-dimethylphenyl group, 2,4-dichlorophenyl group, 2,3-dimethylphenyl group, indan-5-yl group, 2,4-dimethylphenyl group, 2,6-dichlorophenyl group, 4-bromo-2-(trifluoromethoxy)phenyl group, 3,4-ethylenedioxyphenyl group, 3-chloro-4-cyanophenyl group, 3-chloro-4-(trifluoromethoxy)phenyl group, 2-chloro-4-cyanophenyl group, 2,3-dichlorophenyl group, 4-isopropyl-3-methylphenyl group, 4-[(1,1-dimethyl)propyl]-2-hydroxyphenyl group, 3-chloro-2-cyanophenyl group, 2-cyano-4-methylphenyl group, 2,2-difluoro-1,3-benzodioxol-4-yl group, 2,2,3,3-tetrafluoro-1,4-benzodioxen-5-yl group, 3-chloro-4-(trifluoromethylsulfanyl)phenyl group, 2-nitro-4-(trifluoromethoxy)phenyl group, 2,2-difluoro-1,3-benzodioxol-5-yl group, 2-methyl-4-(trifluoromethoxy)phenyl group, 4-bromo-2-fluorophenyl group, 2,4-bis(methanesulfonyl)phenyl group, 2,2,3,3-tetrafluoro-1,4-benzodioxen-6-yl group, 2-benzoyl-4-chlorophenyl group, 2-bromo-4-fluorophenyl group, 3,4-dimethoxyphenyl group, 3,4-difluorophenyl group, 3-chloro-4-methoxyphenyl group, 2-chloro-4-nitrophenyl group, 2,4-difluorophenyl group, 2-benzoyl-5-methylphenyl group, 2-bromo-4-(trifluoromethoxy)phenyl group, 3,4-dihexyloxyphenyl group, 2,4-bis(trifluoromethyl)phenyl group, 4-cyano-2-(trifluoromethoxy)phenyl group, 2-(4-cyanophenoxy)-5-(trifluoromethyl)phenyl group, and 2-(4-methoxyphenoxy)-5-(trifluoromethyl)phenyl group

When "an aryl group which may be substituted" in the aforementioned definition of E is "a di-substituted phenyl group," "a 2,5-di-substituted phenyl group," and "a 3,5-di-substituted phenyl group" are preferred.

When "an aryl group which may be substituted" in the aforementioned definition of E is "a 2,5-di-substituted phenyl group," preferred examples of the group include groups represented by the following Substituent Group δ-2e.
[Substituent Group δ-2e] 2,5-dimethoxyphenyl group, 2-chloro-5-(trifluoromethyl)phenyl group, 2,5-bis(trifluoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2,5-dichlorophenyl group, 2,5-bis[(1,1-dimethyl)ethyl]phenyl group, 5-[(1,1-dimethyl)ethyl]-2-methoxyphenyl group, 4-methoxybiphenyl-3-yl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 5-isopropyl-2-methylphenyl group, 2,5-diethoxyphenyl group, 2,5-dimethylphenyl group, 5-chloro-2-cyano group, 5-diethylsulfamoyl-2-methoxyphenyl group, 2-chloro-5-nitrophenyl group, 2-methoxy-5-(phenylcarbamoyl)phenyl group, 5-acetylamino-2-methoxyphenyl group, 5-methoxy-2-methylphenyl group, 2,5-dibutoxyphenyl group, 2,5-diisopentyloxy group, 5-carbamoyl-2-methoxyphenyl group, 5-[(1,1-dimethyl)propyl]-2-phenoxyphenyl group, 2-hexyloxy-5-methanesulfonyl group, 5-(2,2-dimethylpropionyl)-2-methylphenyl group, 5-methoxy-2-(1-pyrrolyl)phenyl group, 5-chloro-2-(p-toluenesulfonyl)phenyl group, 2-chloro-5-(p-toluenesulfonyl)phenyl group, 2-fluoro-5-methanesulfonyl group, 2-methoxy-5-phenoxy group, 2-methoxy-5-(1-methyl-1-phenylethyl)phenyl group, 5-morpholino-2-nitrophenyl group, 5-fluoro-2-(1-imidazolyl)phenyl group, 2-butyl-5-nitrophenyl group, 5-[(1,1-dimethyl)propyl]-2-hydroxyphenyl group, 2-methoxy-5-methylphenyl group, 2,5-difluorophenyl group, 2-benzoyl-5-methylphenyl group, 2-(4-cyanophenoxy)-5-(trifluoromethyl)phenyl group, and 2-(4-methoxyphenoxy)-5-(trifluoromethyl)phenyl group

When "an aryl group which may be substituted" in the aforementioned definition of E is "a 2,5-di-substituted phenyl group," "a 2,5-di-substituted phenyl group wherein at least one of the said substituents is trifluoromethyl group" is more preferred, a group selected from the following Substituent Group δ-3e is further preferred, and 2,5-bis(trifluoromethyl)phenyl group is most preferred.
[Substituent Group δ-3e] 2-chloro-5-(trifluoromethyl)phenyl group, 2,5-bis(trifluoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 2-(4-cyanophenoxy)-5-(trifluoromethyl)phenyl group, and 2-(4-methoxyphenoxy)-5-(trifluoromethyl)phenyl group

When "an aryl group which may be substituted" in the aforementioned definition of E is "a 3,5-di-substituted phenyl group," preferred examples of the group include groups represented by the following Substituent Group δ-4e.
[Substituent Group δ-4e] 3,5-bis(trifluoromethyl)phenyl group, 3,5-dichlorophenyl group, 3,5-bis[(1,1-dimethyl)ethyl]phenyl group, 3-fluoro-5-(trifluoromethyl)phenyl group, 3-bromo-5-(trifluoromethyl)phenyl group, 3-methoxy-5-(trifluoromethyl)phenyl group, 3,5-difluorophenyl group, 3,5-dinitrophenyl group, 3,5-dimethylphenyl group, 3,5-dimethoxyphenyl group, 3,5-bis(methoxycarbonyl)phenyl group, 3-methoxycarbonyl-5-(trifluoromethyl)phenyl group, 3-carboxy-5-(trifluoromethyl)phenyl group, and 3,5-dicarboxyphenyl group

When "an aryl group which may be substituted" in the aforementioned definition of E is "a 3,5-di-substituted phenyl group," "a 3,5-di-substituted phenyl group wherein at least one of the said substituents is trifluoromethyl group" is more preferred, a group selected from the following Substituent Group δ-5e is further preferred, and 3,5-bis(trifluoromethyl)phenyl group is most preferred.
[Substituent Group δ-5e] 3,5-bis(trifluoromethyl)phenyl group, 3-fluoro-5-(trifluoromethyl)phenyl group, 3-bromo-5-(trifluoromethyl)phenyl group, 3-methoxy-5-(trifluoromethyl)phenyl group, 3-methoxycarbonyl-5-(trifluoromethyl)phenyl group, and 3-carboxy-5-(trifluoromethyl)phenyl group

When "an aryl group which may be substituted" in the aforementioned definition of E is "a mono-substituted phenyl group," preferred examples of the group include groups represented by the following Substituent Group δ-6e.
[Substituent Group δ-6e] 4-methoxyphenyl group, 4-chlorophenyl group, 2-methoxyphenyl group, 2-(trifluoromethyl)phenyl group, 3-(trifluoromethyl)phenyl group, 4-(trifluoromethyl)phenyl group, 3-chlorophenyl group, biphenyl-3-yl group, 3-acetylphenyl group, 3-(acetylamino)phenyl group, 3-carbamoylphenyl group, 3-methylcarbomoylphenyl group, 4-methylphenyl group, 3-(trifluoromethoxy)phenyl group, 2-benzylphenyl group, 4-(trifluoromethoxy)phenyl group, 4-[(1,1-dimethyl)ethyl]phenyl group, 3-isopropoxyphenyl group, 4-isopropoxyphenyl group, 4-hexylphenyl group, 3-methylphenyl group, 4-cyclohexylphenyl group, 4-benzylphenyl group, 2-chlorophenyl group, 2-methylphenyl group, 4-butylphenyl group, 4-benzyloxyphenyl group, 3-benzylphenyl group, 4-hexyloxyphenyl group, 3-isopropylphenyl group, 4-cyanophenyl group, 3-cyanophenyl group, 4-(ethoxycarbonylmethyl)phenyl group, 3-(trifluoromethylsulfanyl)phenyl group, 4-(trifluoromethylsulfanyl)phenyl group, 4-(trifluoromethanesulfonyl)phenyl group, 3-ethynylphenyl group, 4-(1-methylpropyl)phenyl group, 3-benzoylphenyl group, 3-methoxyphenyl group, 4-(acetylamino)phenyl group, 4-sulfamoylphenyl group, 4-difluoromethoxy)phenyl group, 3-methylsulfanylphenyl group, 4-methanesulfonylphenyl group, 3-(butylsulfamoyl)phenyl group, 3-benzyloxyphenyl group, 4-(p-toluenesulfonylamino)phenyl group, 4-morpholinophenyl group, 3-[(1,1-dimethyl)ethyl]phenyl group, 3-(5-methylfuran-2-yl)phenyl group, 3-sulfamoylphenyl group, 3-(trifluoromethanesulfonyl)phenyl group, 3-hexyloxyphenyl group, 4-acetylphenyl group, biphenyl-2-yl group, biphenyl-4-yl group, 3-[5-phenyl-3-(trifluoromethyl)pyrazol-1-yl]phenyl group, 3-{5-[(1,1-dimethyl)ethyl]-3-(trifluoromethyl)pyrazol-1-yl}phenyl group, 4-[3,5-bis(trifluoromethyl)pyrazol-1-yl]phenyl group, 3-[3,5-bis(trifluoromethyl)pyrazol-1-yl]phenyl group, and 4-[5-phenyl-3-(trifluoromethyl)pyrazol-1-yl]phenyl group

When "an aryl group which may be substituted" in the aforementioned definition of E is "a phenyl group which has three or more substituents," preferred examples of the group include groups represented by the following Substituent Group δ-7e.
[Substituent Group δ-7e] 3,5-bis(trifluoromethyl)-2-bromophenyl group, 3,4,5-trichlorophenyl group, 3,5-dichloro-4-hydroxyphenyl group, pentafluorophenyl group, 3,5,5,8,8-pentamethyl-5,6,7,8-tetrahydronaphthalen-2-yl group, 3,5-bis(trifluoromethyl)-2-methylphenyl group, 2,6-dichloro-4-(trifluoromethyl)phenyl group, 2,4-dimethoxy-5-(trifluoromethyl)phenyl group, 2,4-difluoro-5-(trifluoromethyl)phenyl group, 4-chloro-2-(4-chlorobenzenesulfonyl)-5-(trifluoromethyl)phenyl group, 5-chloro-2-nitro-4-(trifluoromethyl)phenyl group, 2,3-difluoro-4-(trifluoromethyl)phenyl group, 2,3,5,6-tetrafluoro-4-(trifluoromethyl)phenyl group, 2,4,6-trimethylphenyl group, 2-cyano-4,5-dimethoxyphenyl group, 2,4-dichloro-5-isopropoxyphenyl group, 2,3,5-trifluorophenyl group, 2,4,5-trichlorophenyl group, and 5-ethoxy-4-fluoro-2-nitrophenyl group

When "an aryl group which may be substituted" in the aforementioned definition of E is "a naphthyl group which may be substituted," preferred examples of the group include 1-naphthyl group, 4-methoxynaphthalen-2-yl group, and 4-hydroxy-3-methylnaphthalen-1-yl group.

Examples of the "heteroaryl group" in "a heteroaryl group which may be substituted" in the definition of E include similar groups to the "monocyclic heteroaryl group" and "fused polycyclic heteroaryl group" in the definition of the aforementioned "heterocyclic group." A 5 to 13-membered heteroaryl group is preferred, and preferred examples of the group include thienyl group, pyrazolyl group, oxazolyl group, 1,3,4-thiadiazolyl group, pyridyl group, pyrimidinyl group, indolyl group, quinolyl group, carbazolyl group, thiazolyl group, and pyrazinyl group.

A 5-membered heteroaryl group is more preferred as the "heteroaryl group" in "a heteroaryl group which may be substituted" in the definition of E. Thienyl group, pyrazolyl group, oxazolyl group, 1,3,4-thiadiazolyl group, and thiazolyl group are further preferred, and thiazolyl group is most preferred.

Examples of the substituent in the definition of "a heteroaryl group which may be substituted" in the aforementioned definition of E include similar groups to the substituent explained for the definition "which may be substituted." The position of substituents existing on the heteroaryl group is not particularly limited, and when two or more substituents exist, they may be the same or different.

When "a heteroaryl group which may be substituted" in the aforementioned definition of E is "a thiazolyl group which may be substituted," "a thiazol-2-yl group which may be substituted." "A mono-substituted thiazol-2-yl group" and "a di-substituted thiazol-2-yl group" are more preferred, and "a di-substituted thiazol-2-yl group" is further preferred.

When "a heteroaryl group which may be substituted" in the aforementioned definition of E is "a di-substituted thiazol-2-yl group," a group selected from the following Substituent Group δ-8e is preferred, and 4-[(1,1-dimethyl)ethyl]-5-[(2,2-dimethyl)propionyl]thiazol-2-yl group is most preferred.
[Substituent Group δ-8e] 5-bromo-4-[(1,1-dimethyl)ethyl]thiazol-2-yl group, 5-bromo-4-(trifluoromethyl)thiazol-2-yl group, 5-cyano-4-[(1,1-dimethyl)ethyl]thiazol-2-yl group, 5-methylthiazol-2-yl group, 4,5-dimethylthiazol-2-yl group, 5-methyl-4-phenylthiazol-2-yl group, 5-(4-fluorophenyl)-4-methylthiazol-2-yl group, 4-methyl-5-[3-(trifluoromethyl)phenyl]thiazol-2-yl group, 4-[(1,1-dimethyl)ethyl]-5-ethylthiazol-2-yl group, 4-ethyl-5-phenylthiazol-2-yl group, 4-isopropyl-5-phenylthiazol-2-yl group, 4-butyl-5-phenylthiazol-2-yl group, 4-[(1,1-dimethyl)ethyl]-5-[(2,2-dimethyl)propionyl]thiazol-2-yl group, 4-[(1,1-dimethyl)ethyl]-5-(ethoxycarbonyl)thiazol-2-yl group, 4-[(1,1-dimethyl)ethyl]-5-piperidinothiazol-2-yl group, 4-[(1,1-dimethyl)ethyl]-5-morpholinothiazol-2-yl group, 4-[(1,1-dimethyl)ethyl]-5-(4-methylpiperazin-1-yl)thiazol-2-yl group, 4-[(1,1-dimethyl)ethyl]-5-(4-phenylpiperazin-1-yl)thiazol-2-yl group, 5-carboxymethyl-4-phenylthiazol-2-yl group, 4,5-diphenylthiazol-2-yl group, 4-benzyl-5-phenylthiazol-2-yl group, 5-phenyl-4-(trifluoromethyl)thiazol-2-yl group, 5-acetyl-4-phenylthiazol-2-yl group, 5-benzoyl-4-phenylthiazol-2-yl group, 5-ethoxycarbonyl-4-phenylthiazol-2-yl group, 5-ethoxycarbonyl-4-(pentafluorophenyl)thiazol-2-yl group, 5-methylcarbamoyl-4-phenylthiazol-2-yl group, 5-ethylcarbamoyl-4-phenylthiazol-2-yl group, 5-isopropylcarbamoyl-4-phenylthiazol-2-yl group, 5-(2-phenylethyl)carbamoyl-4-phenylthiazol-2-yl group, 5-ethoxycarbonyl-4-(trifluoromethyl)thiazol-2-yl group, 5-carboxy-4-[(1,1-dimethyl)ethyl]thiazol-2-yl group, 5-(ethoxycarbonyl)methyl-4-phenylthiazol-2-yl group, 5-carboxy-4-phenylthiazol-2-yl group, and 5-propylcarbamoyl-4-phenylthiazol-2-yl group.

When "a heteroaryl group which may be substituted" in the aforementioned definition of E is "a mono-substituted thiazol-2-yl group," preferred examples of the group include groups represented by the following Substituent Group δ-9e.
[Substituent Group δ-9e] 4-[(1,1-dimethyl)ethyl]thiazol-2-yl group, 4-phenylthiazol-2-yl group, 4-[3,5-bis(trifluoromethyl)phenyl]thiazol-2-yl group, 4-(2,4-dichlorophenyl)thiazol-2-yl group, 4-(3,4-dichlorophenyl)thiazol-2-yl group, 4-[4-(trifluoromethyl)phenyl]thiazol-2-yl group, 4-(2,5-difluorophenyl)thiazol-2-yl group, 4-(4-methoxyphenyl)thiazol-2-yl group, 4-[3-(trifluoromethyl)phenyl]thiazol-2-yl group, and 4-(pentafluorophenyl)thiazol-2-yl group

The compounds represented by the aforementioned general formula (I) may form salts. Examples of pharmacologically acceptable salts include, when acidic groups exist, metal salts such as lithium salt, sodium salt, potassium salt, magnesium salt, calcium salts, or ammonium salts such as ammonium salt, methylammonium salt, dimethylammonium salt, trimethylammonium salt, dicyclohexylammonium salt, and when basic groups exist, mineral acid salts such as hydrochloride, oxalate, hydrosulfate, nitrate, phosphate, or organic acid salts such as methane sulfonate, benzene sulfonate, para-toluene sulfonate, acetate, propionate, tartrate, fumarate, maleate, malate, oxalate, succinate, citrate, benzoate, mandelate, cinnamate, lactate. Salts may sometimes be formed with amino acids such as glycine. As active ingredients of the medicament of the present invention, pharmacologically acceptable salts may also be suitably used.

The compounds or salts thereof represented by the aforementioned general formula (I) may exist as hydrates or solvates. As active ingredients of the medicament of the present invention, any of the aforementioned substances may be used. Furthermore, the compounds represented by the aforementioned general formula (I) may sometimes have one or more asymmetric carbons, and may exist as steric isomers such as optically active substance and diastereomer. As active ingredients of the medicament of the present invention, pure forms of stereoisomers, arbitrary mixture of enantiomers or diastereomers, and racemates may be used.

Furthermore, when the compounds represented by the general formula (I) has, for example, 2-hydroxypyridine form, the compounds may exist as 2-pyridone form which is a tautomer. As active ingredients of the medicament of the present invention, pure forms of tautomers or a mixture thereof may be used. When the compounds represented by the general formula (I) have olefinic double bonds, the configuration may be in either E or Z, and as active ingredients of the medicament of the present invention, geometrical isomer in either of the configurations or a mixture thereof may be used.

Examples of the compounds included in the general formula (I) as active ingredients of the medicaments of the present invention are shown below. However, the active ingredients of the medicaments of the present invention are not limited to the compound set out below.

The abbreviations used in the following tables have the following meanings.
Me: methyl group, Et: ethyl group.

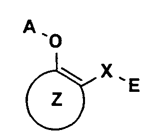

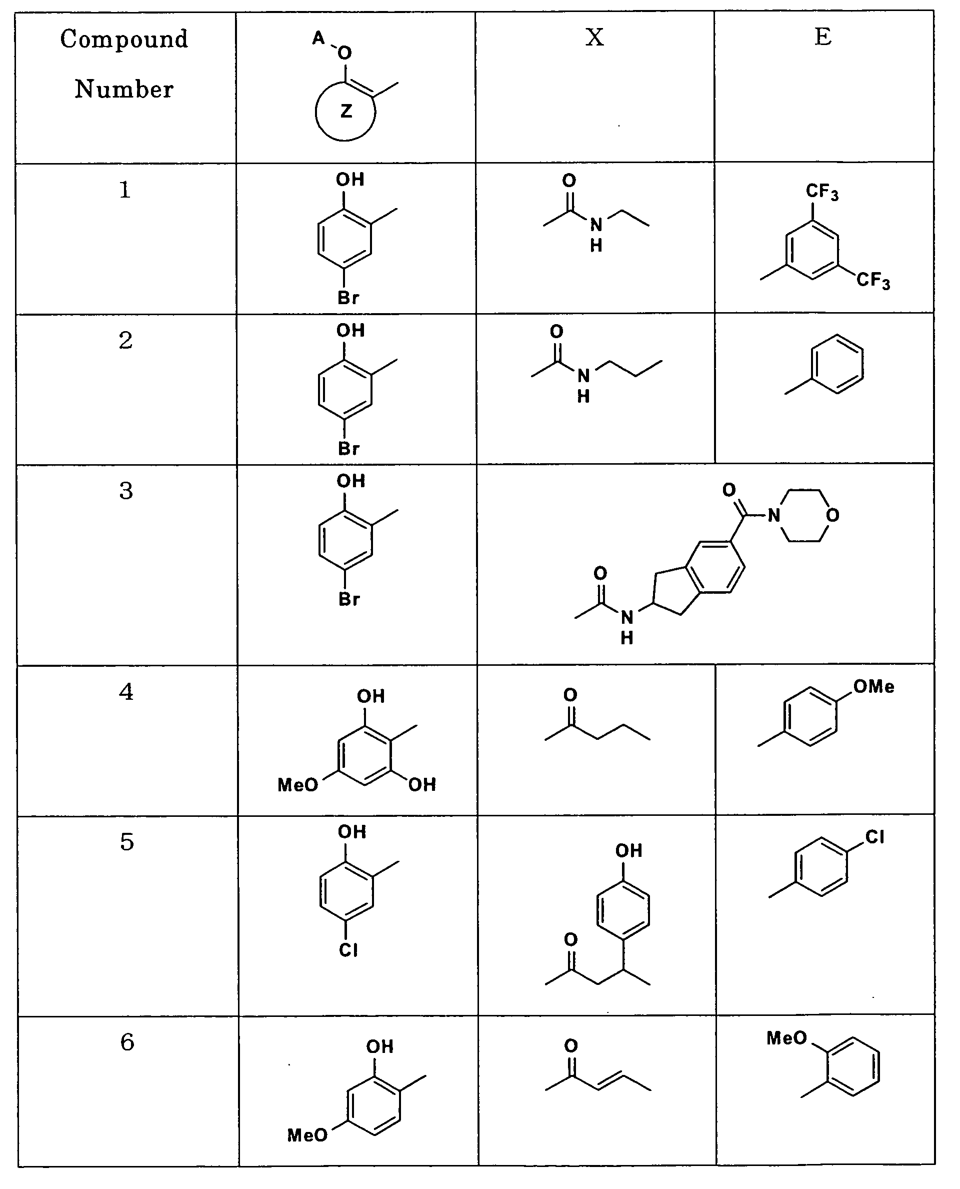

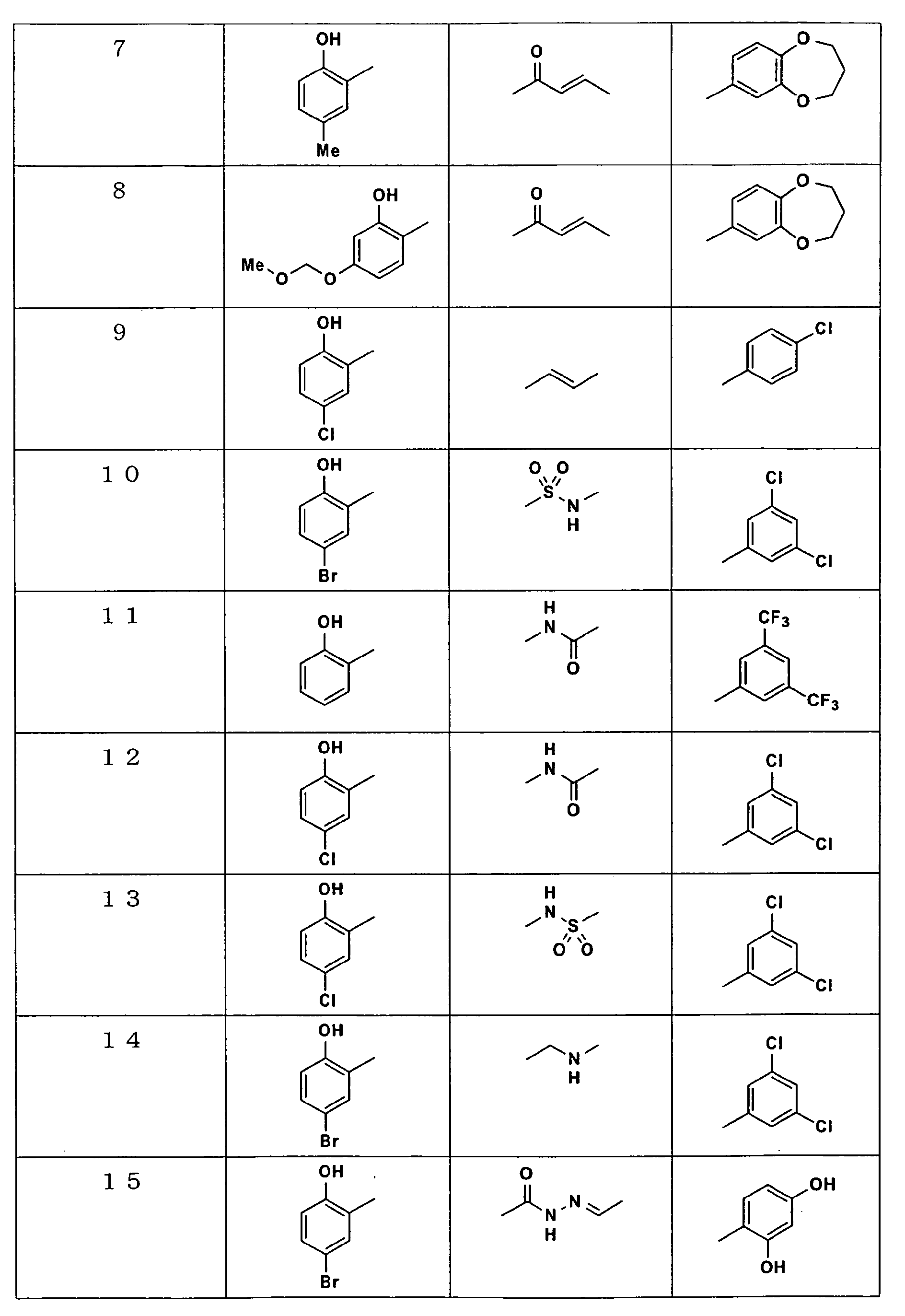

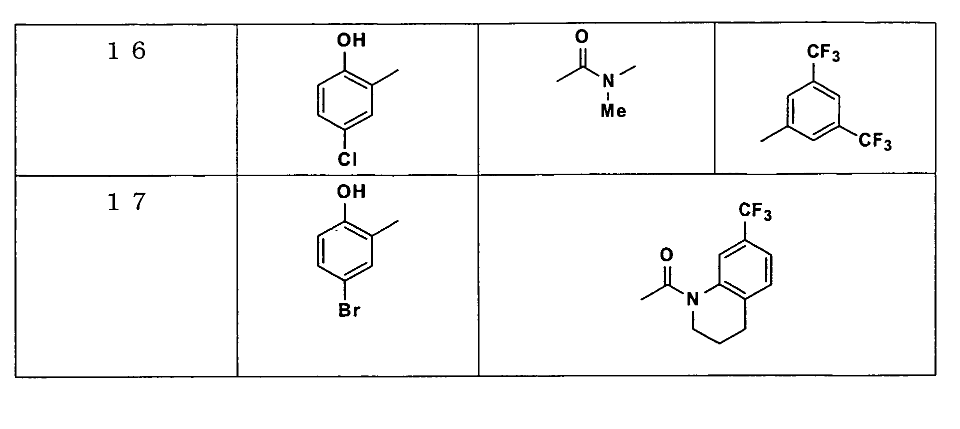

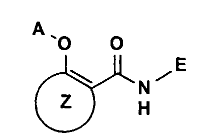

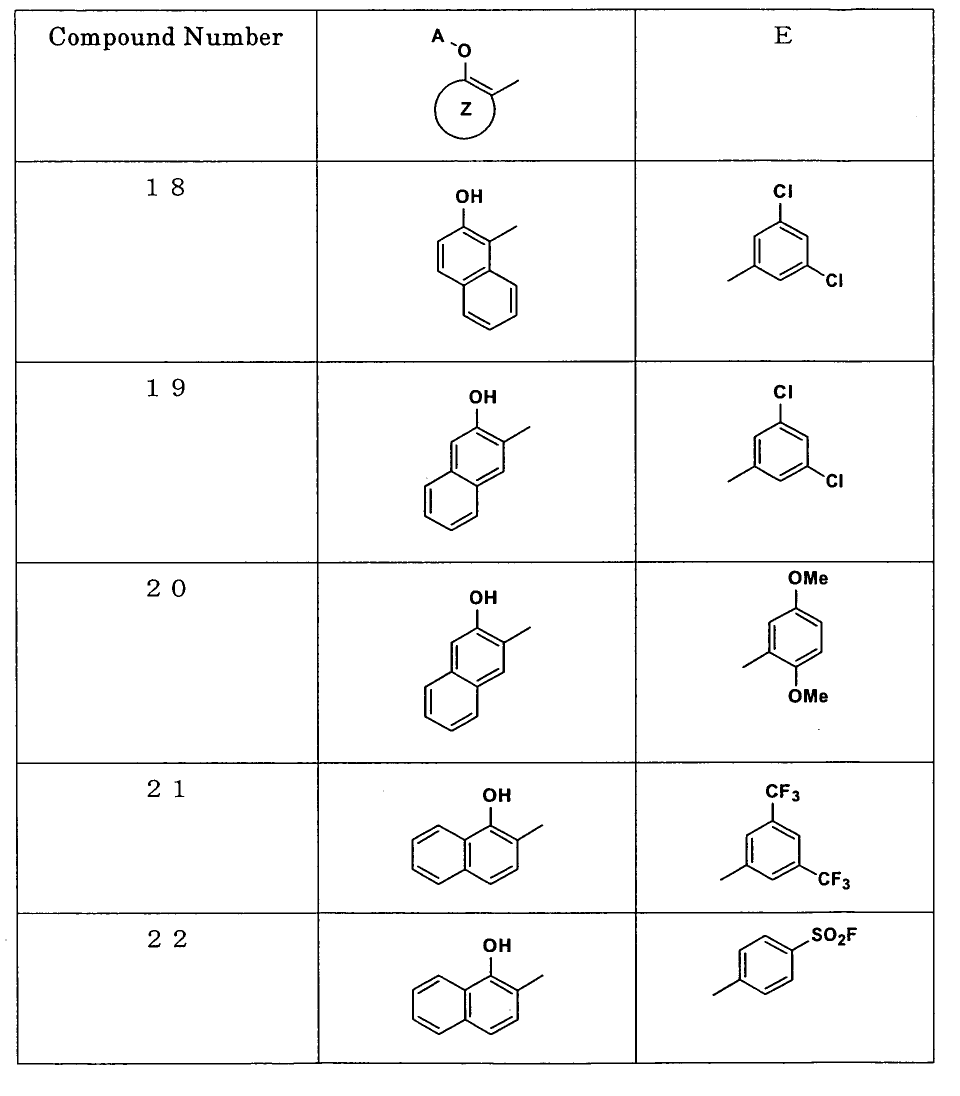

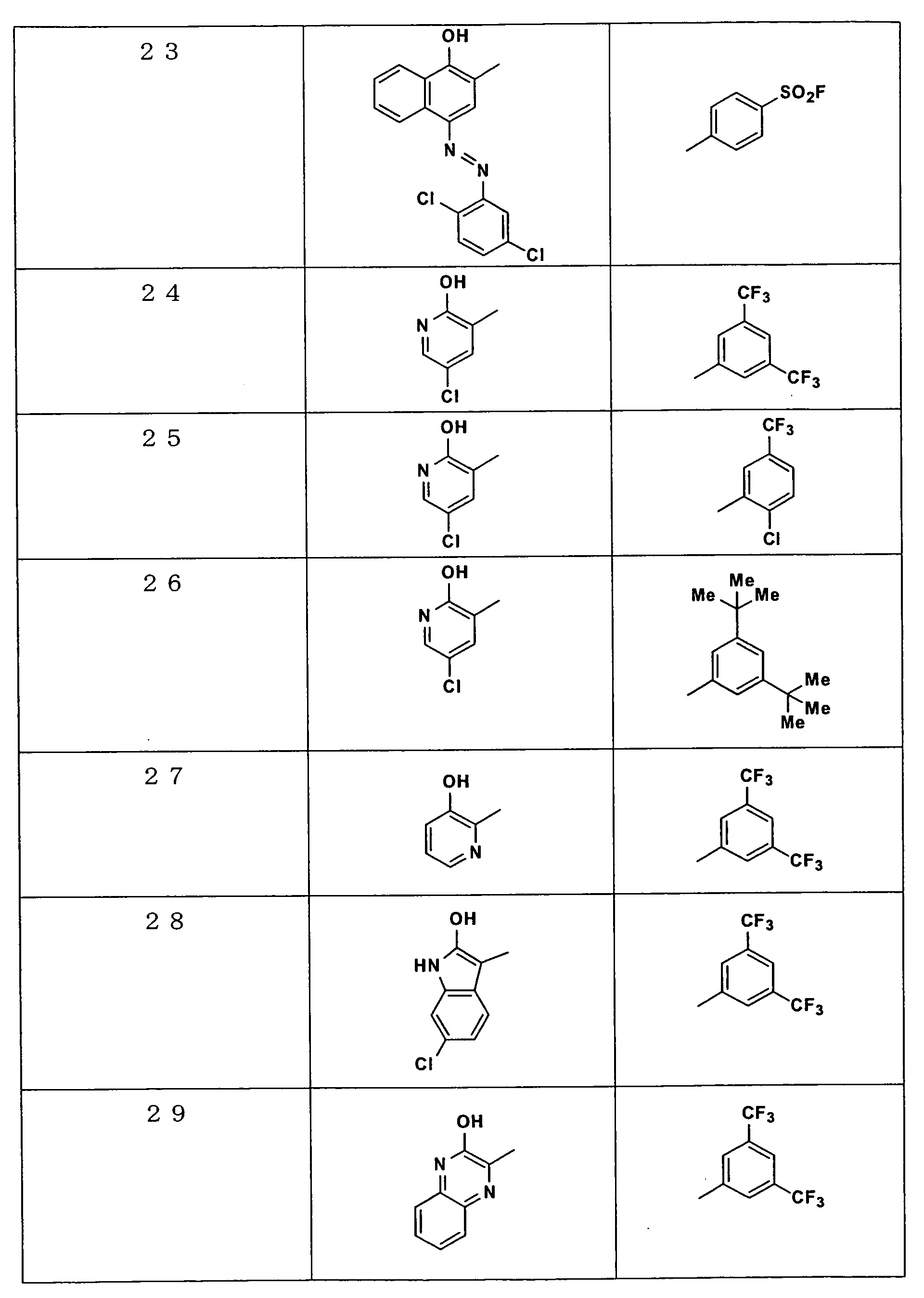

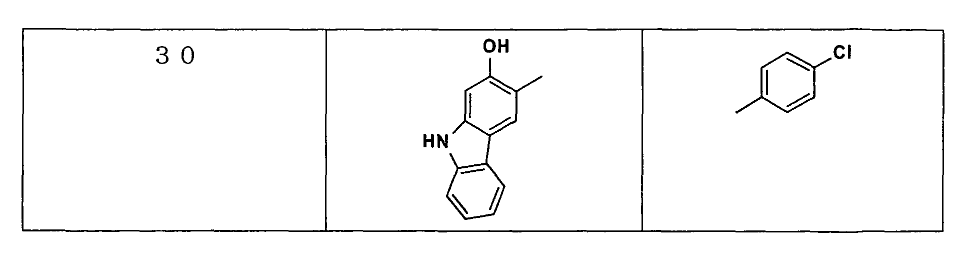

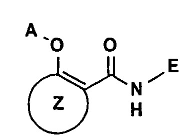

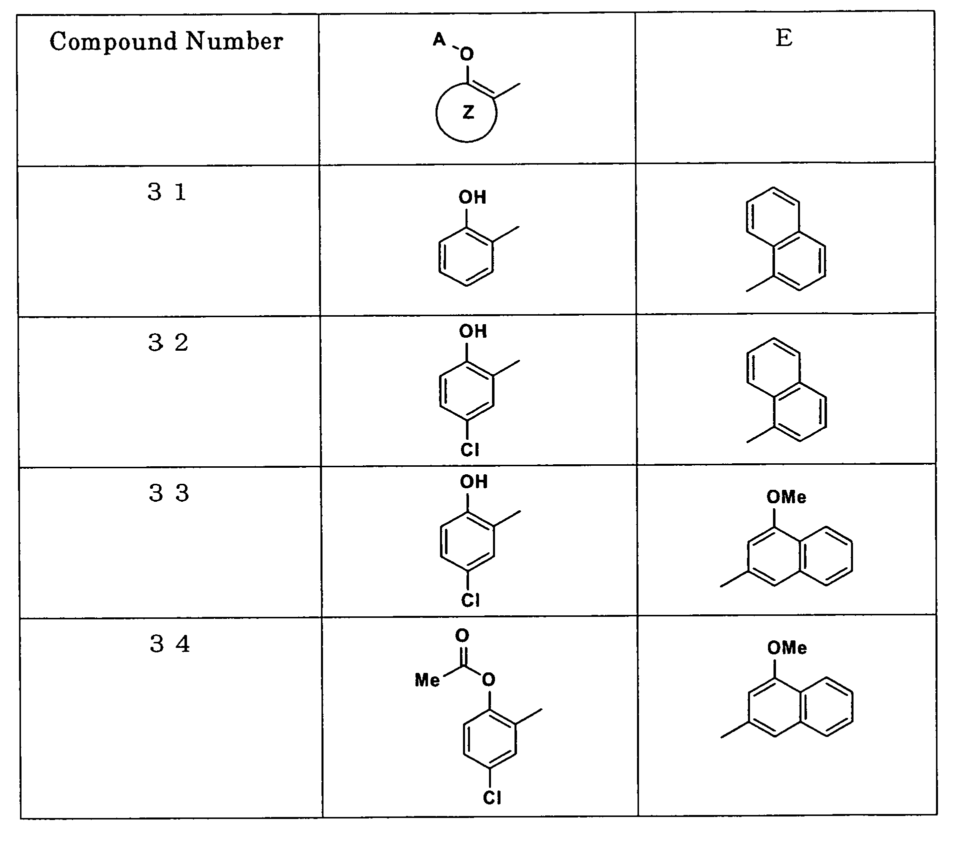

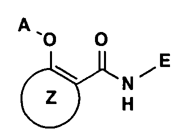

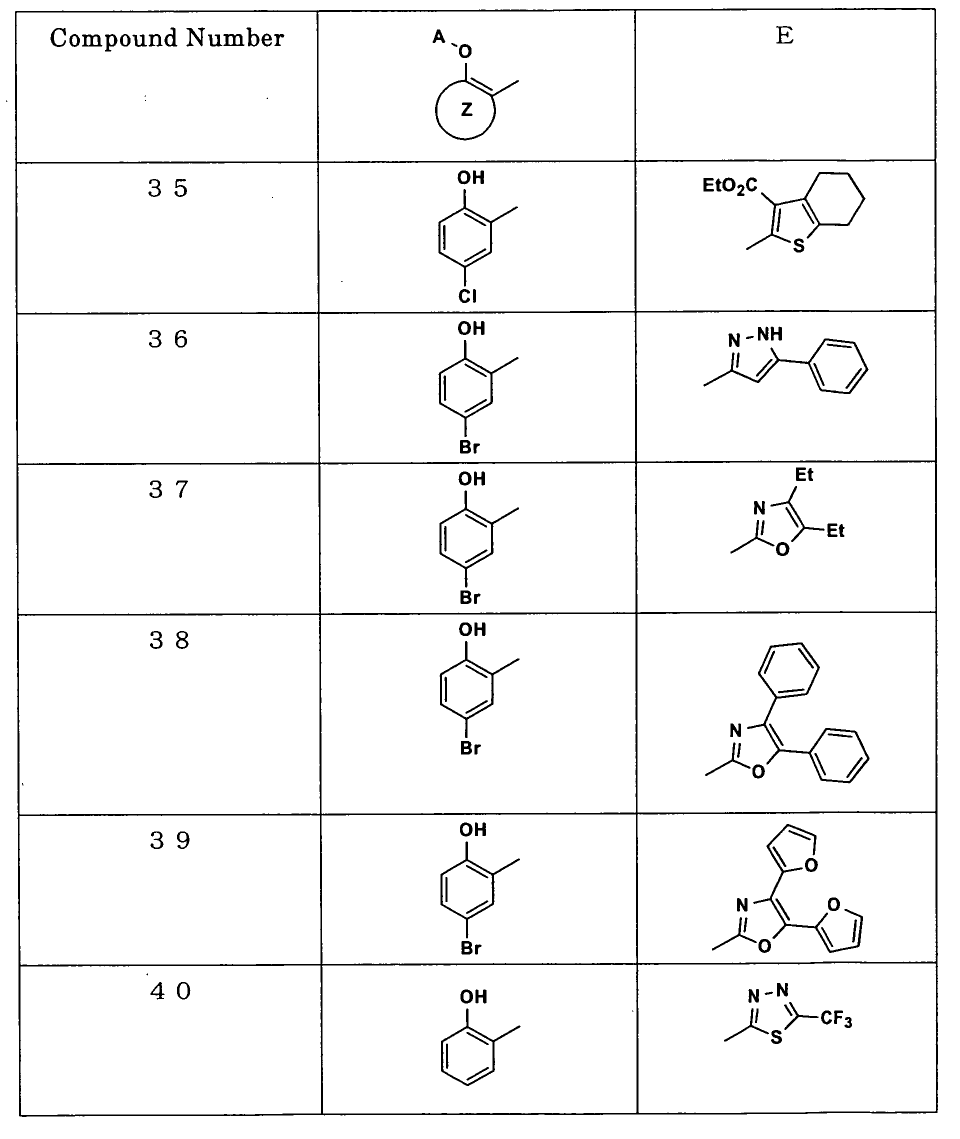

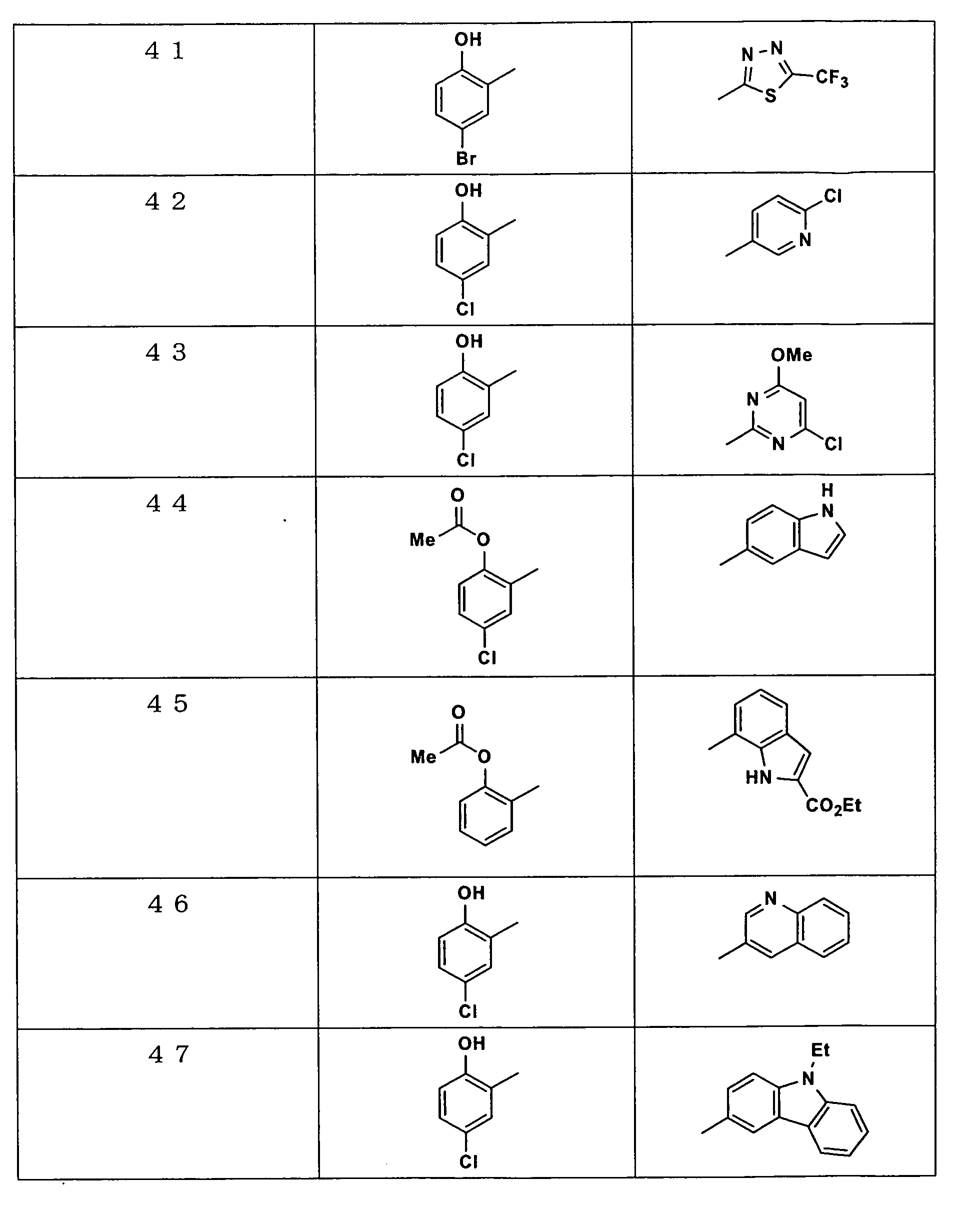

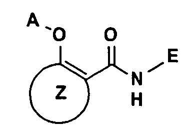

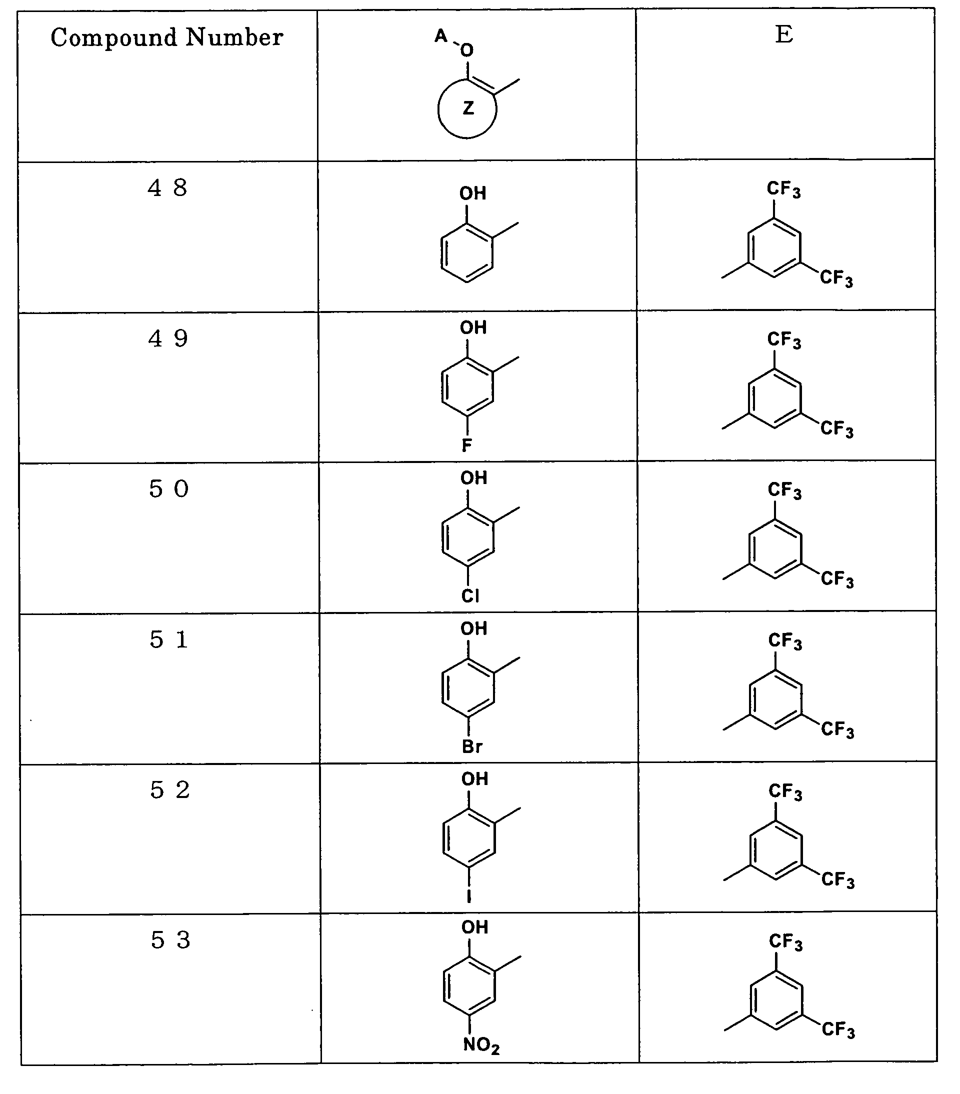

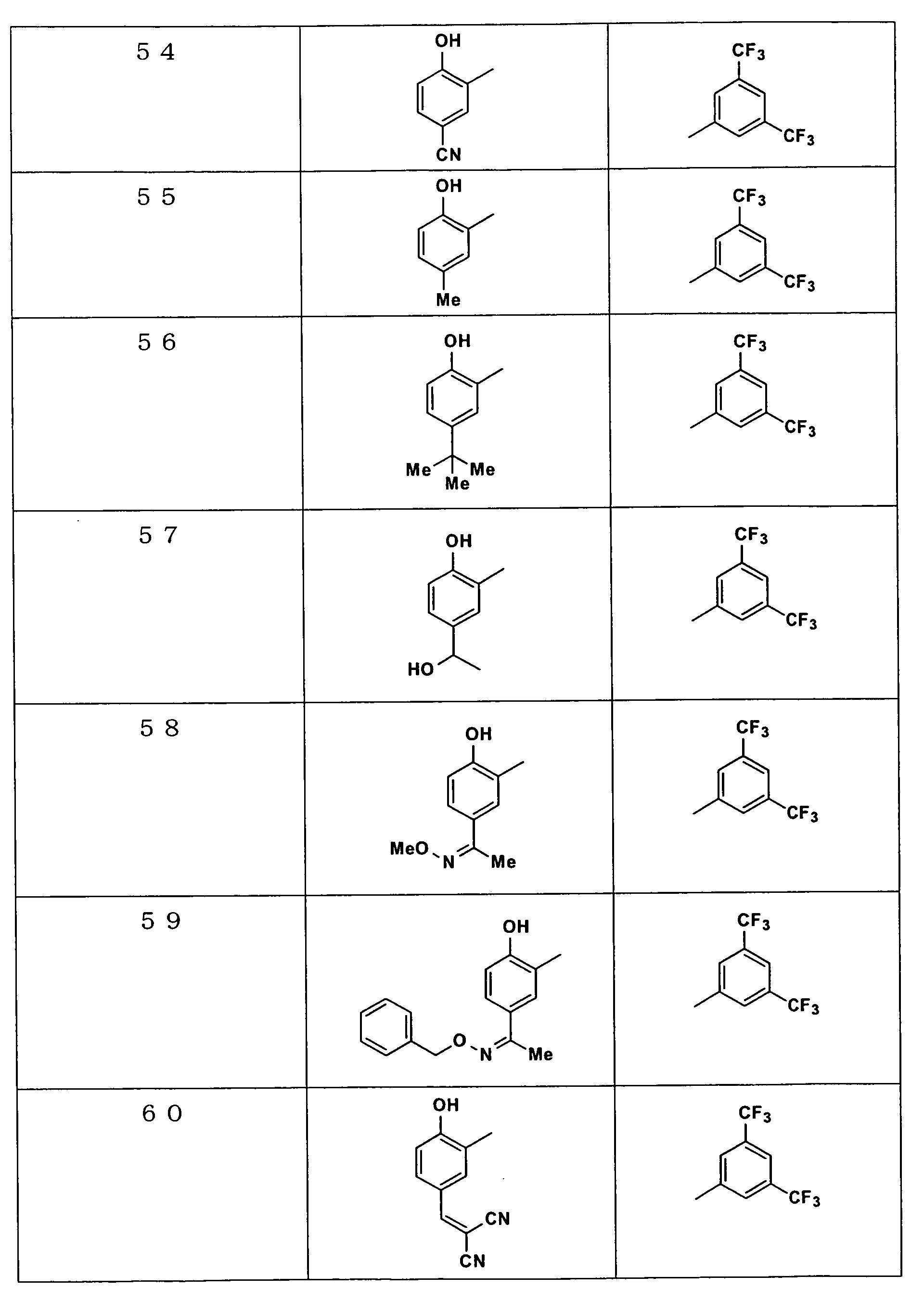

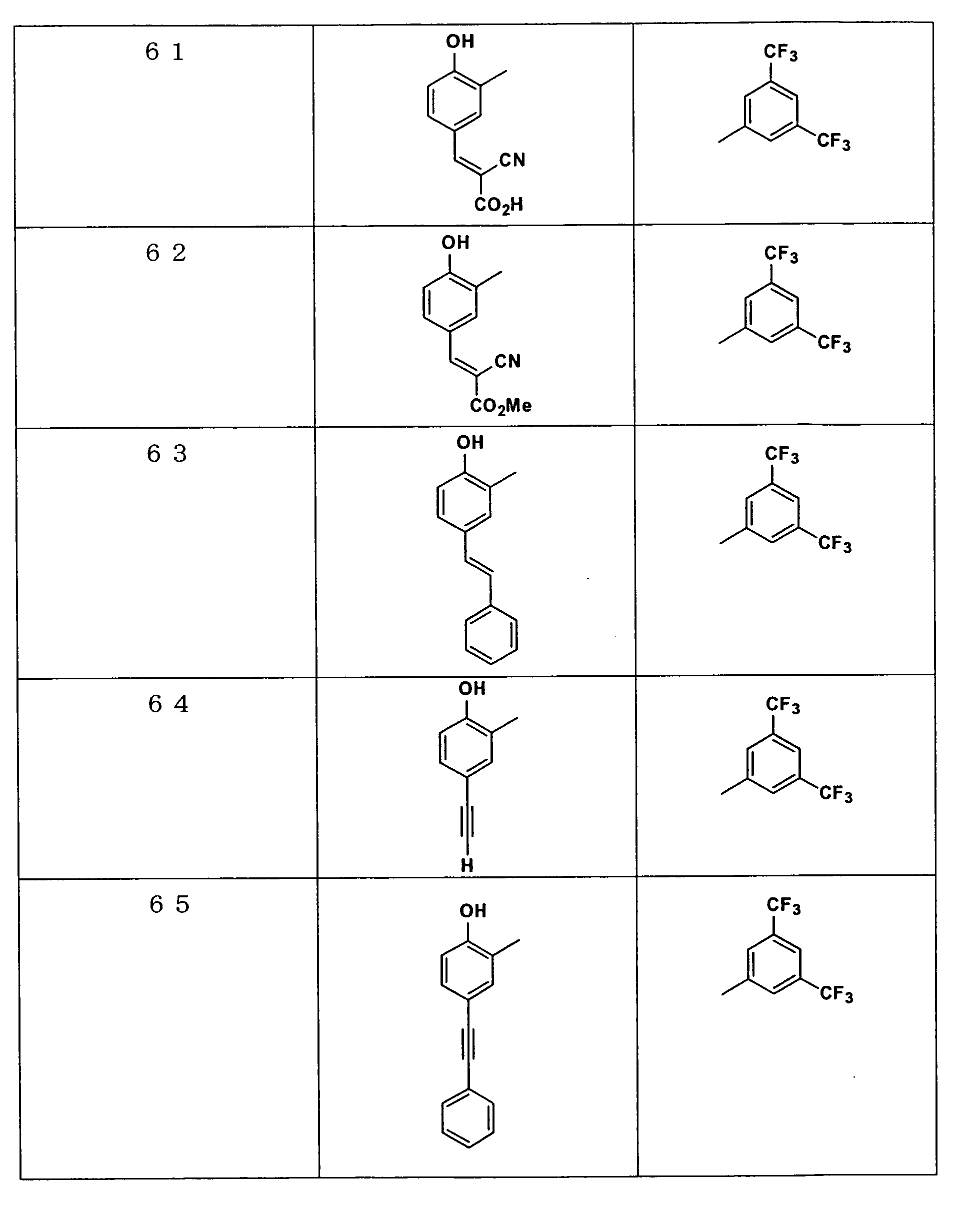

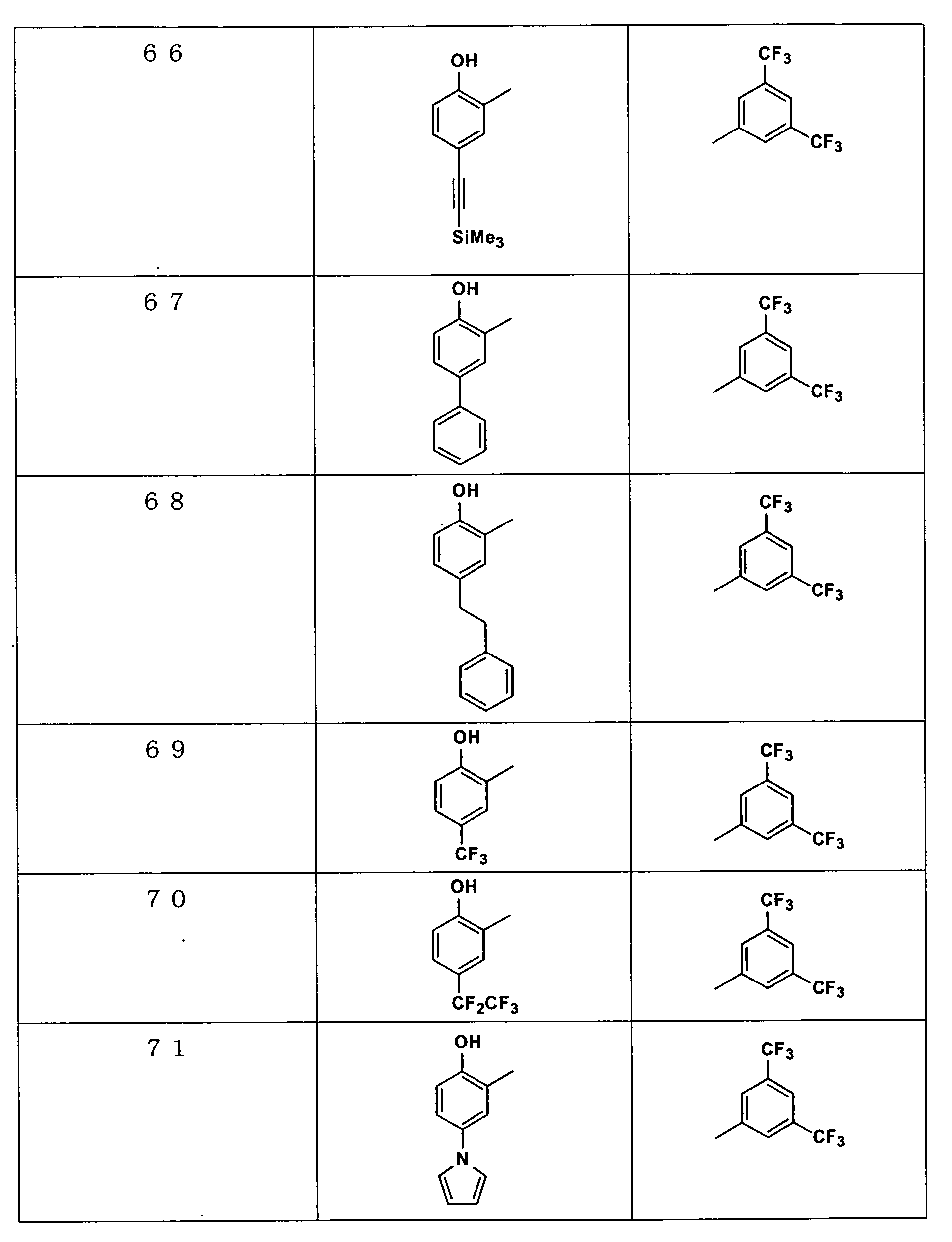

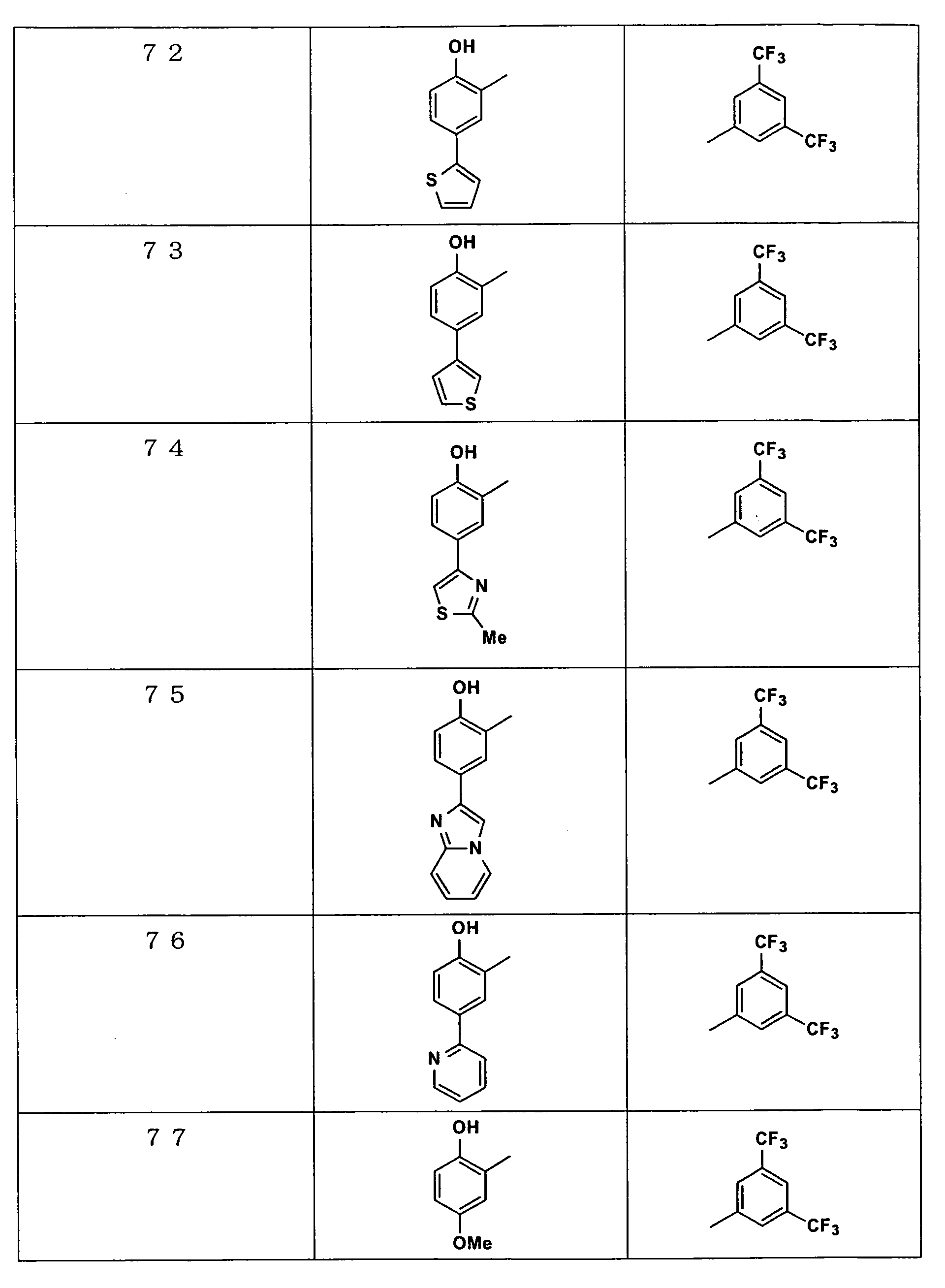

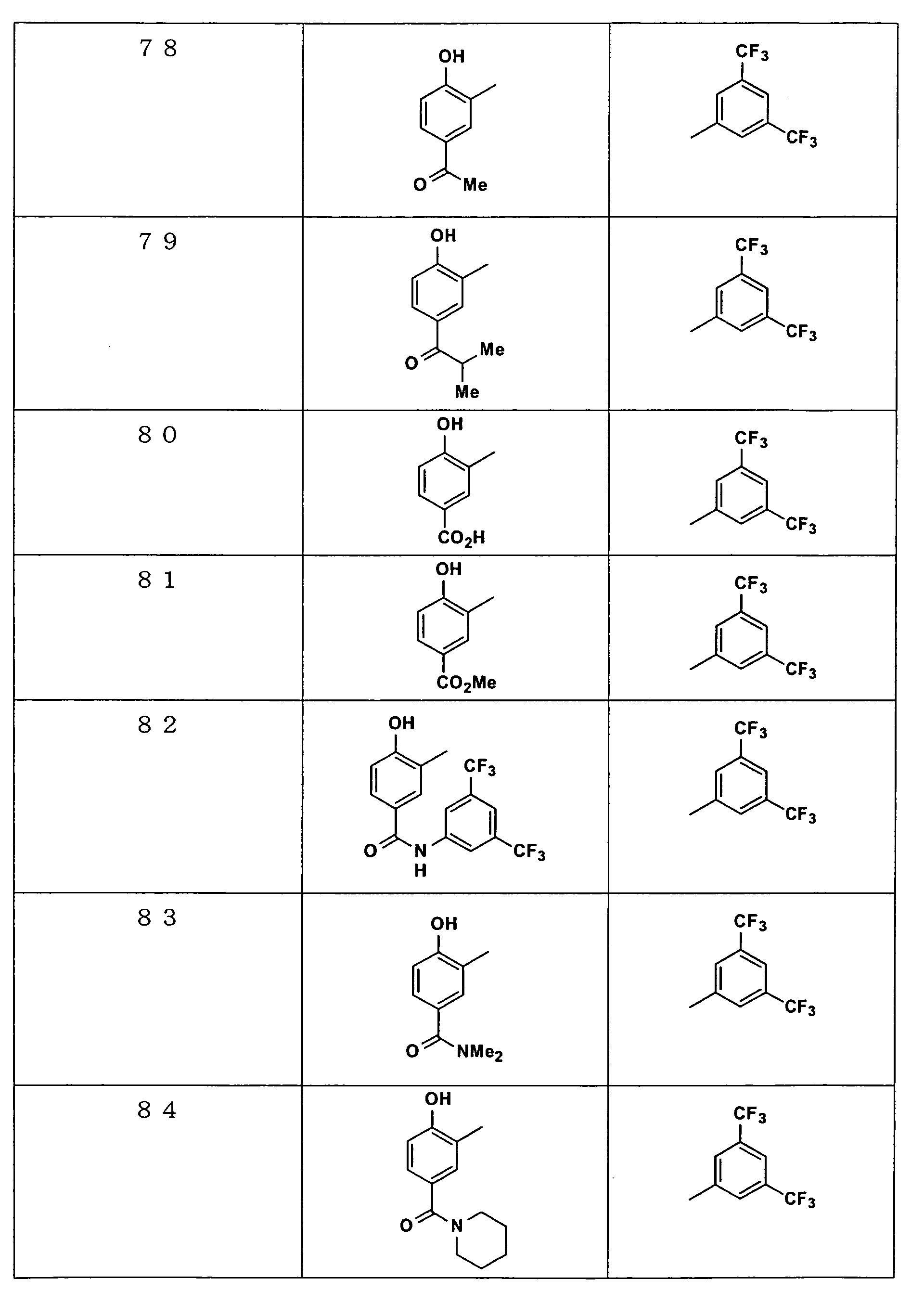

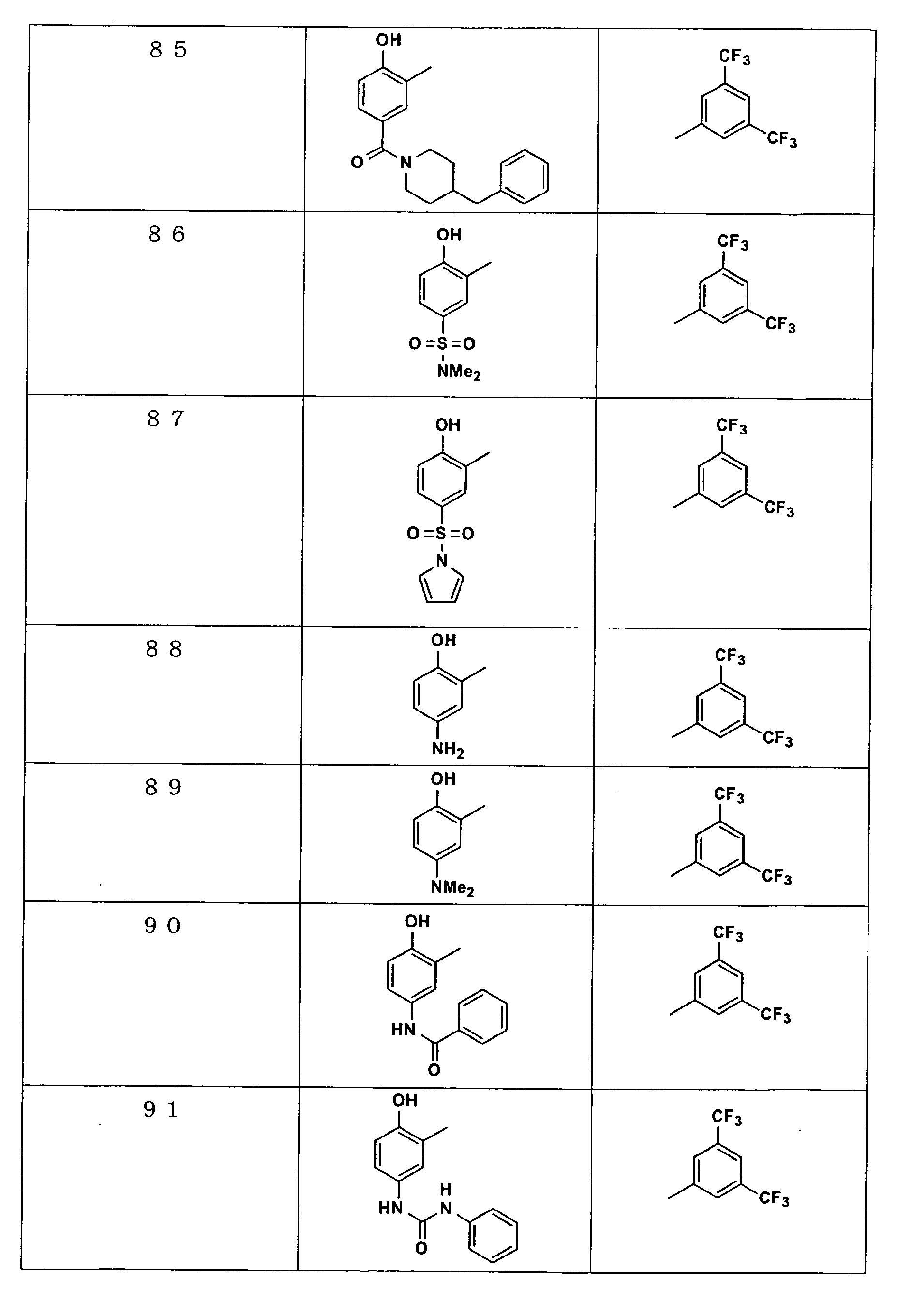

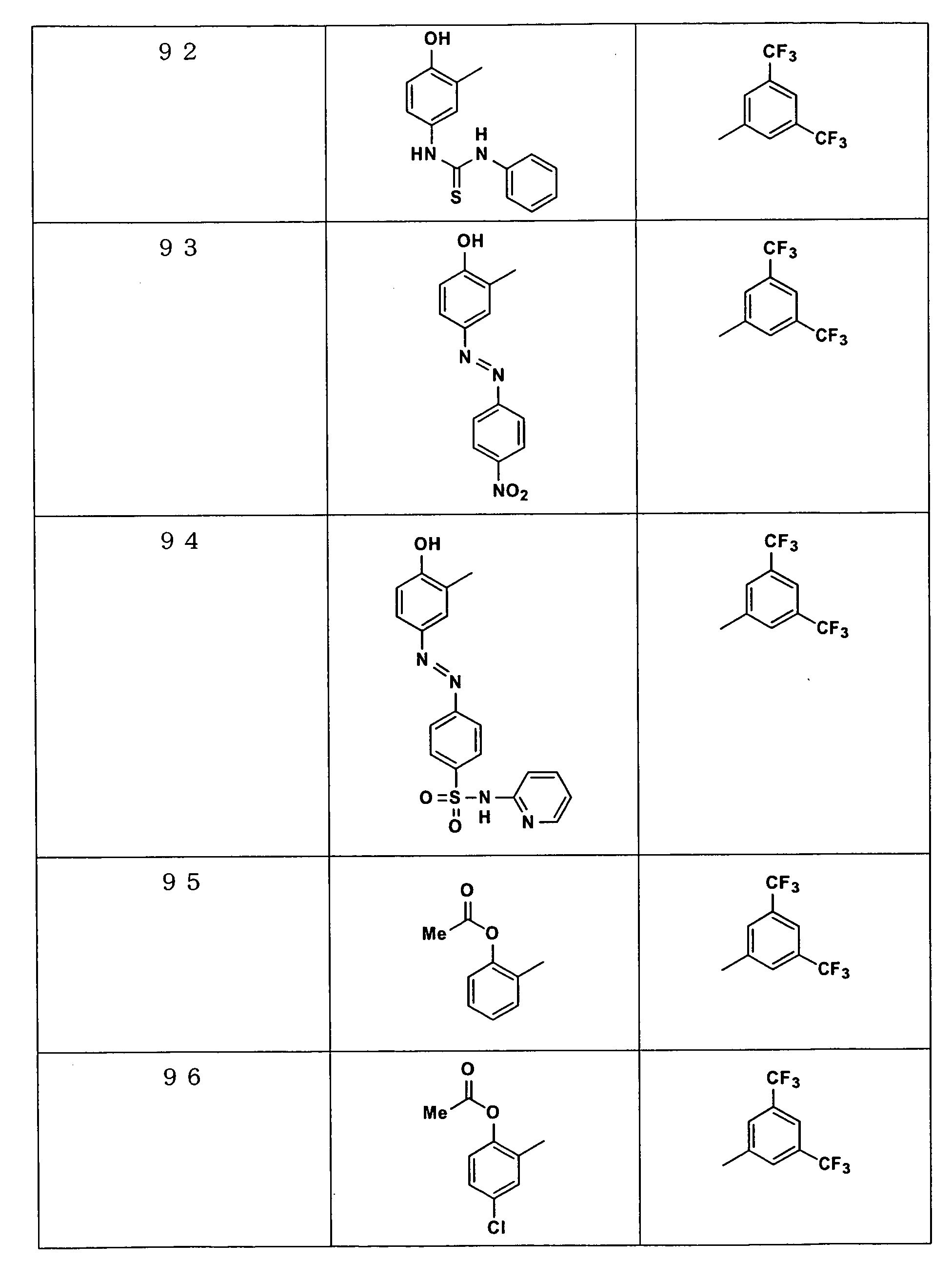

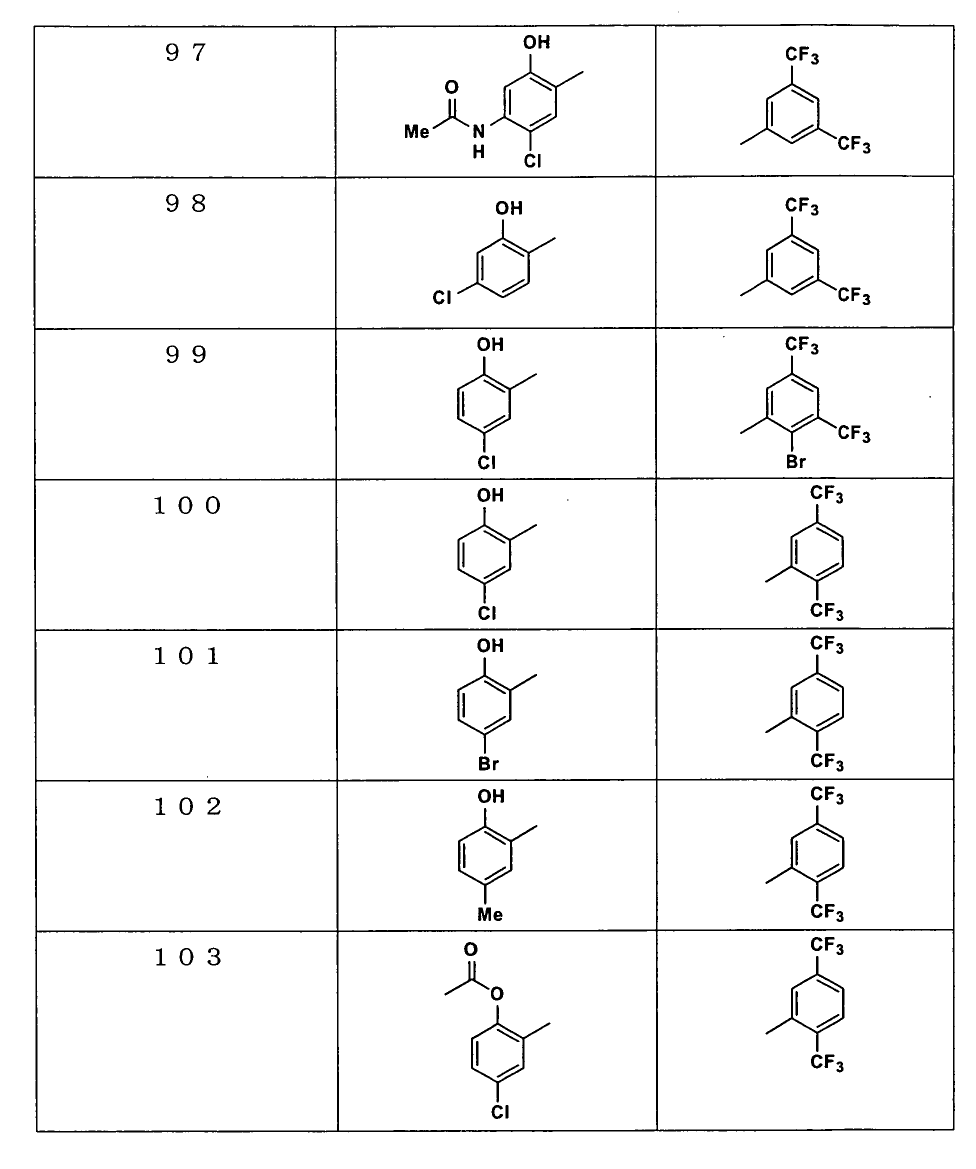

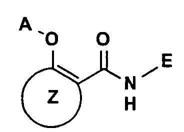

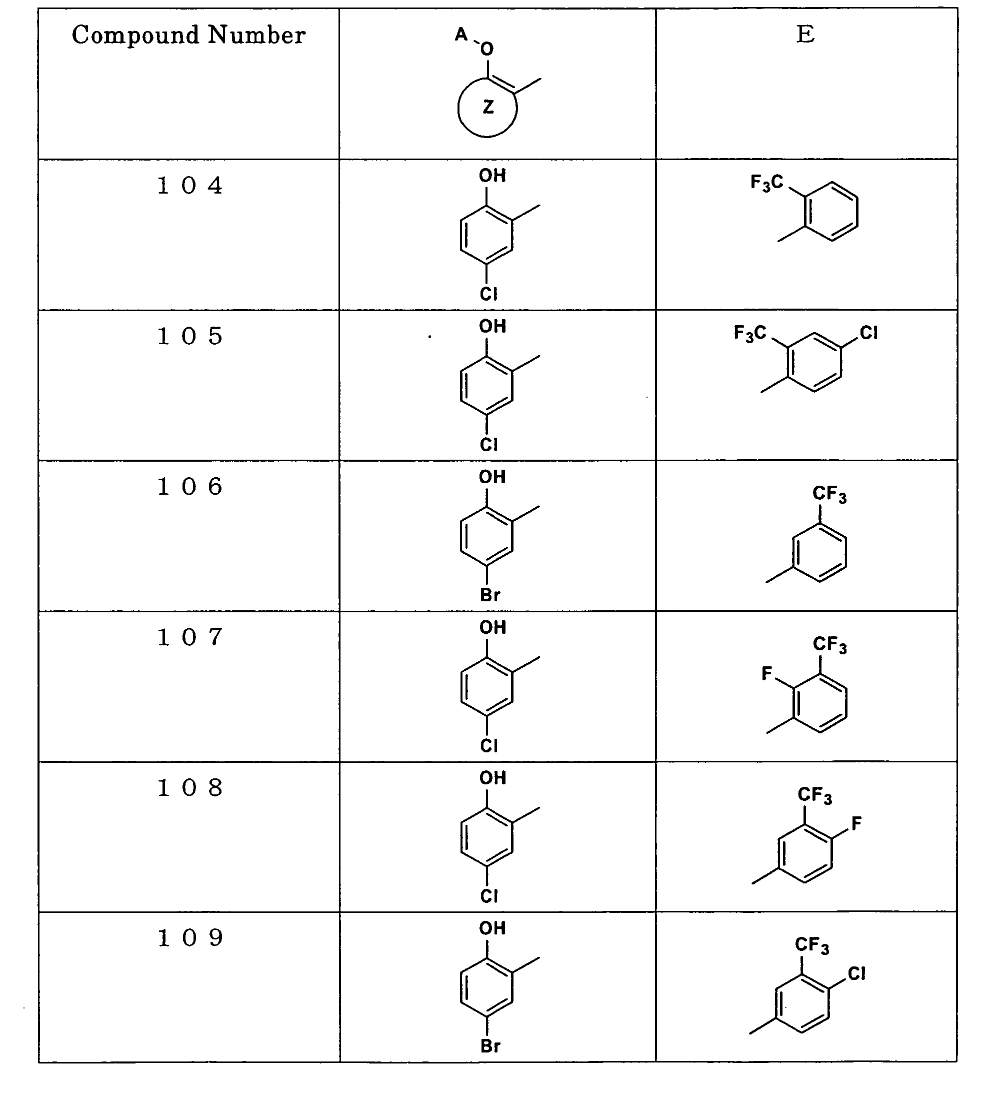

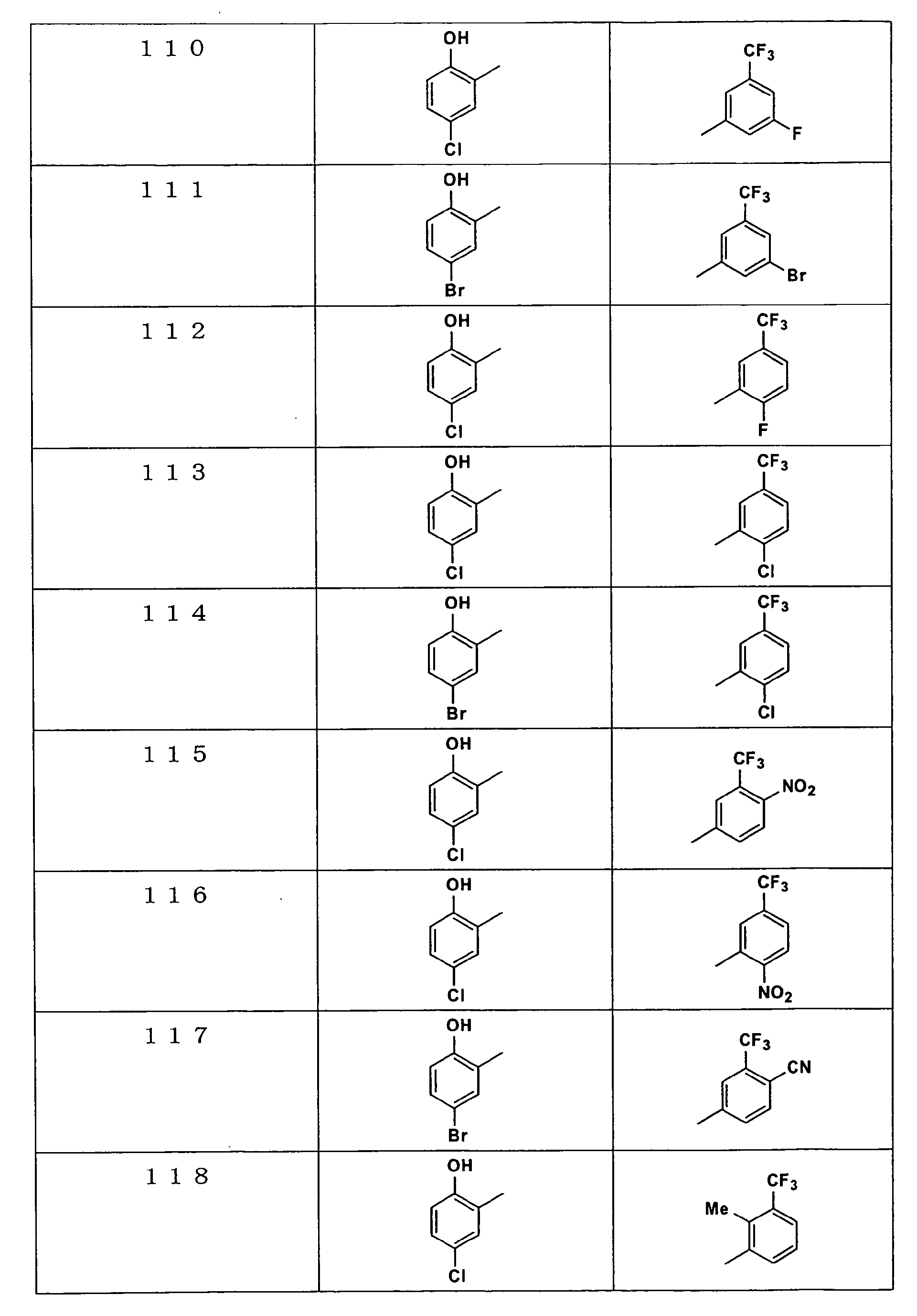

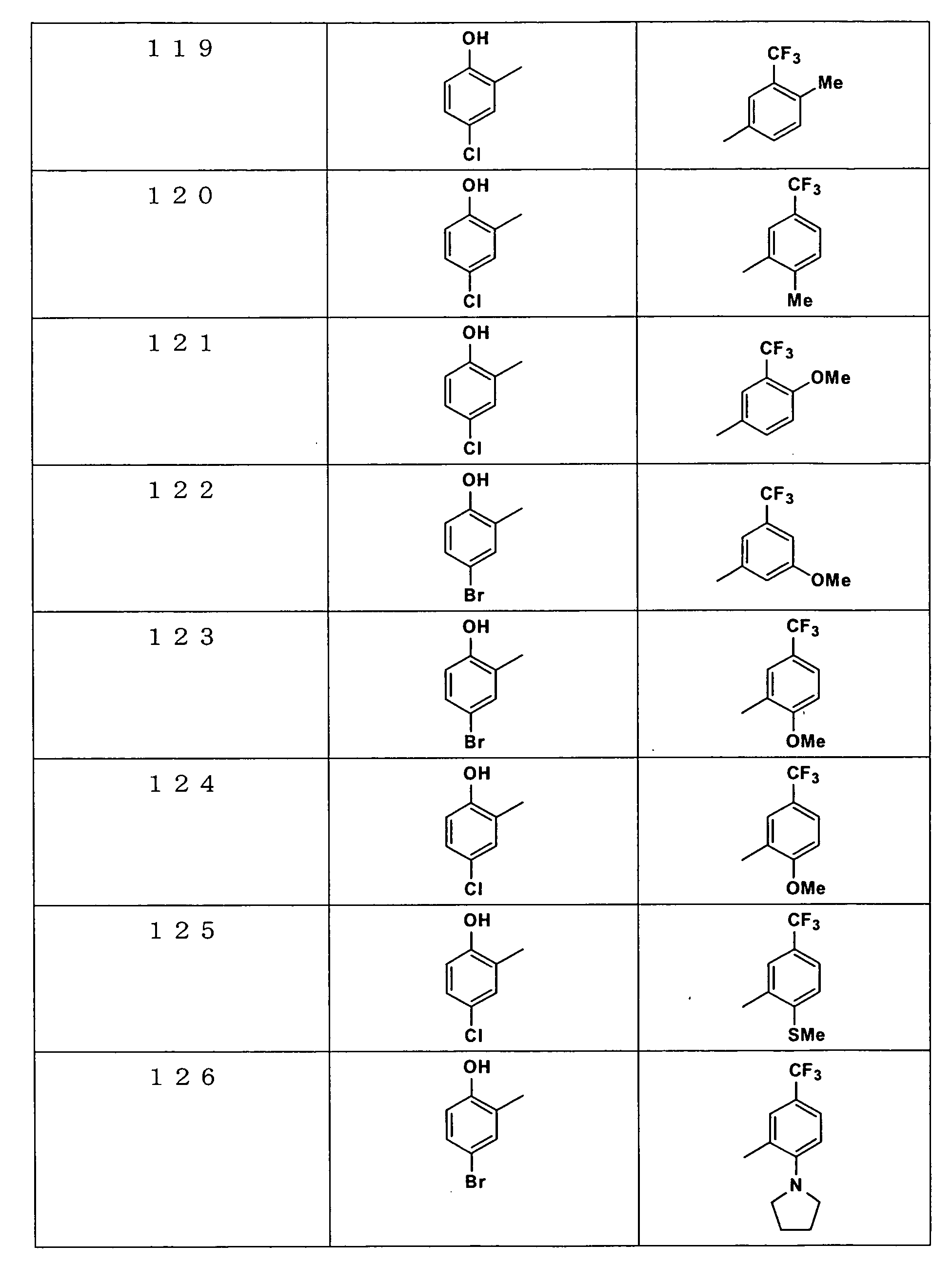

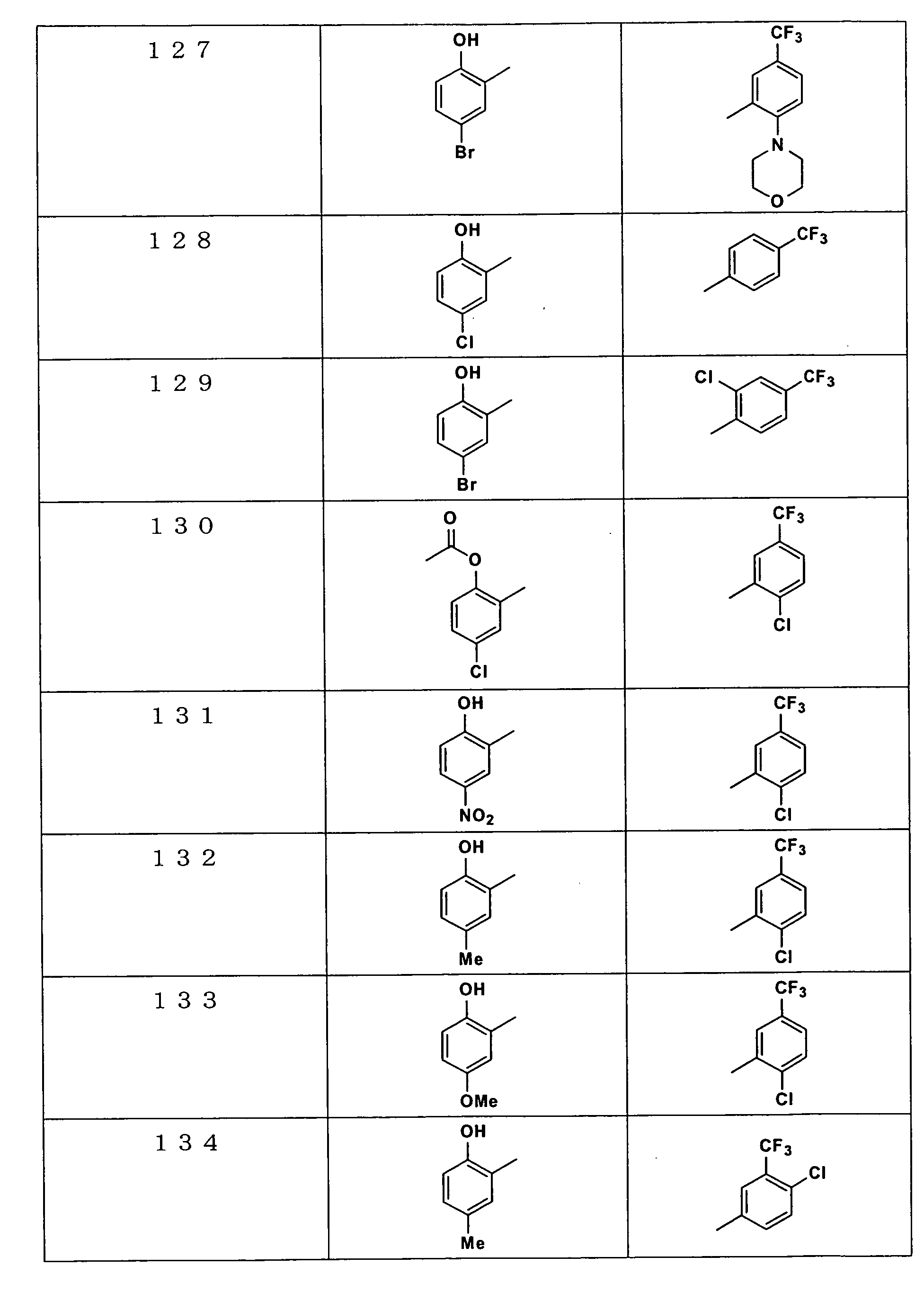

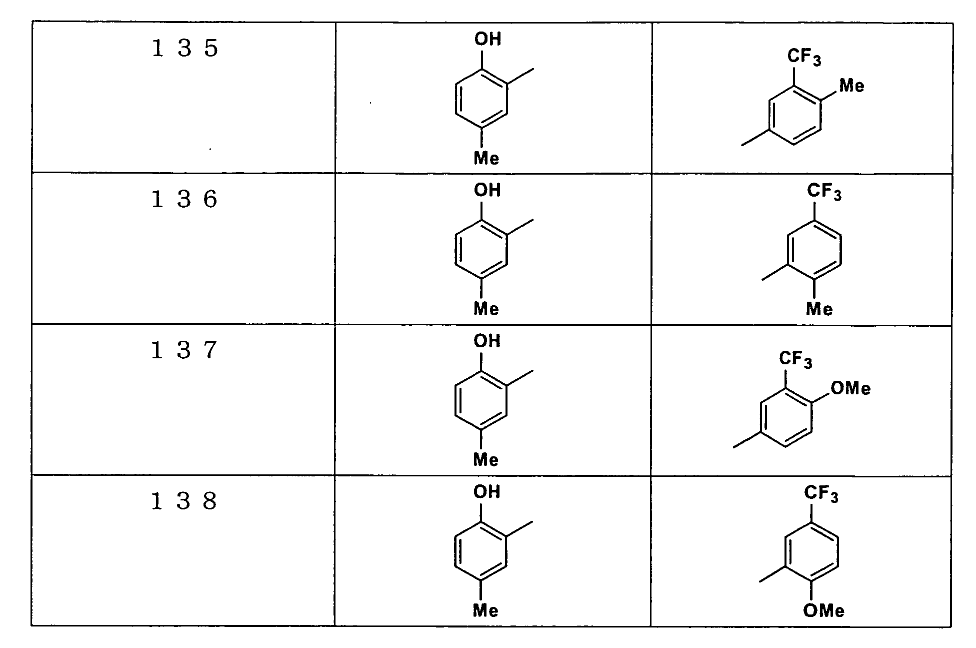

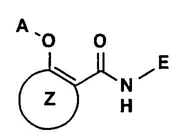

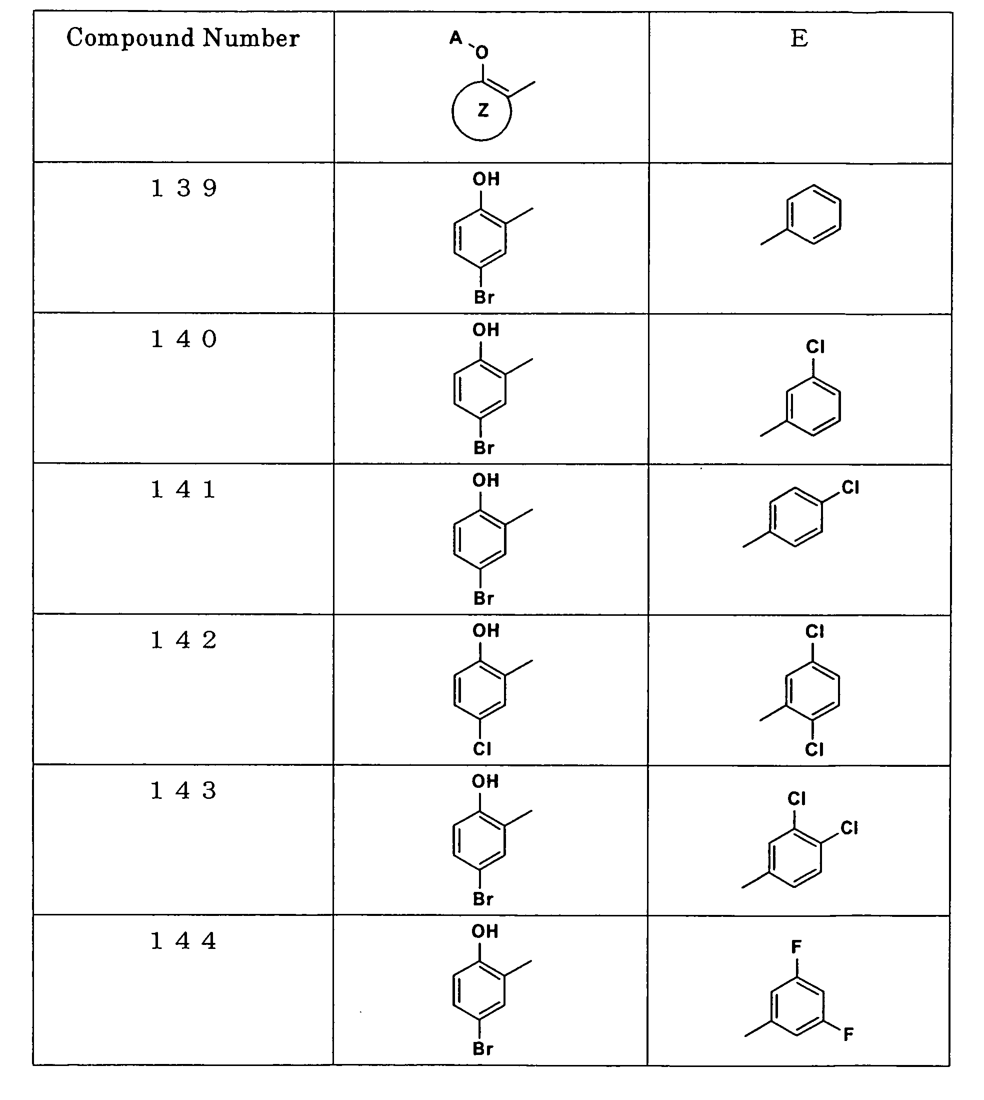

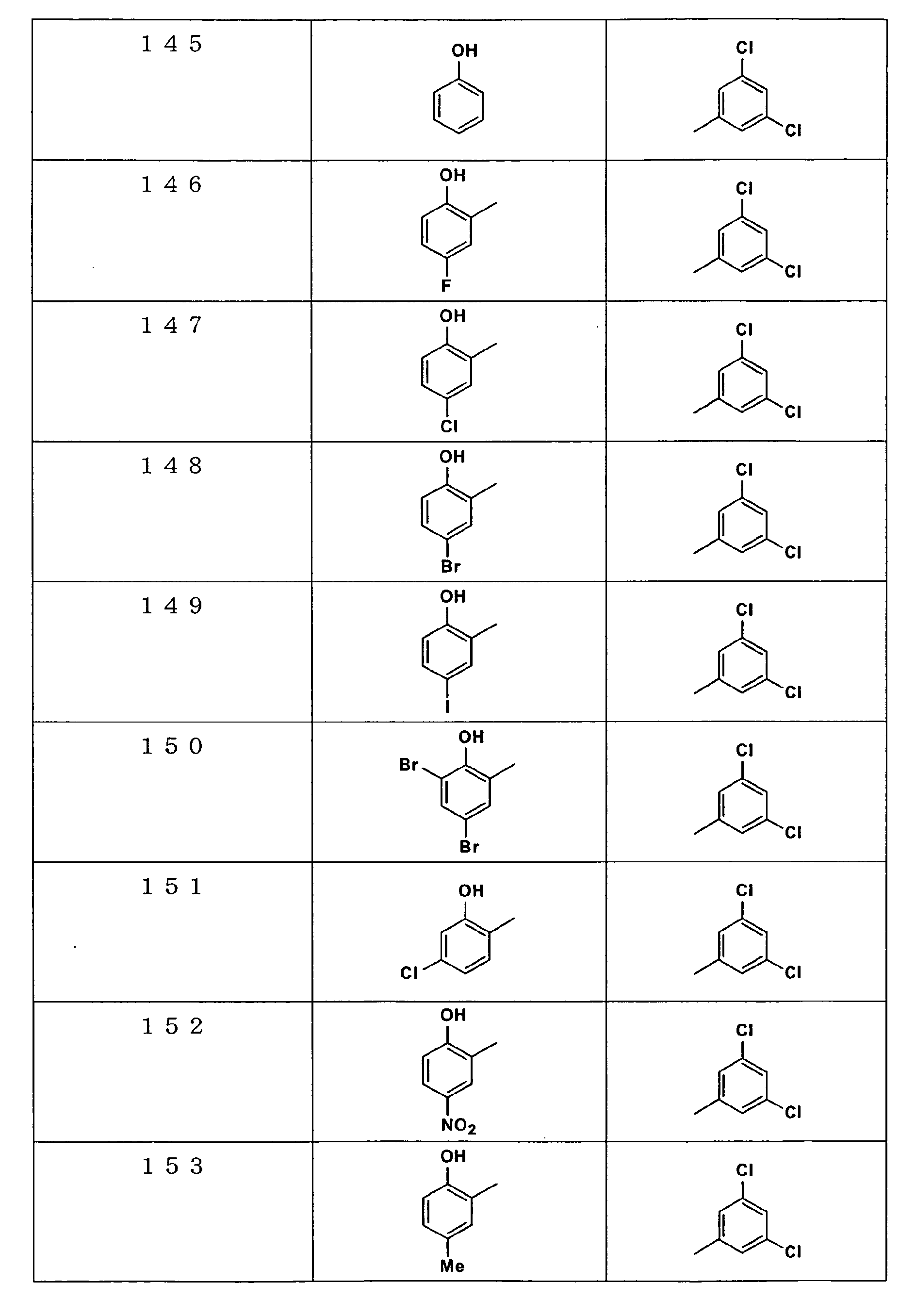

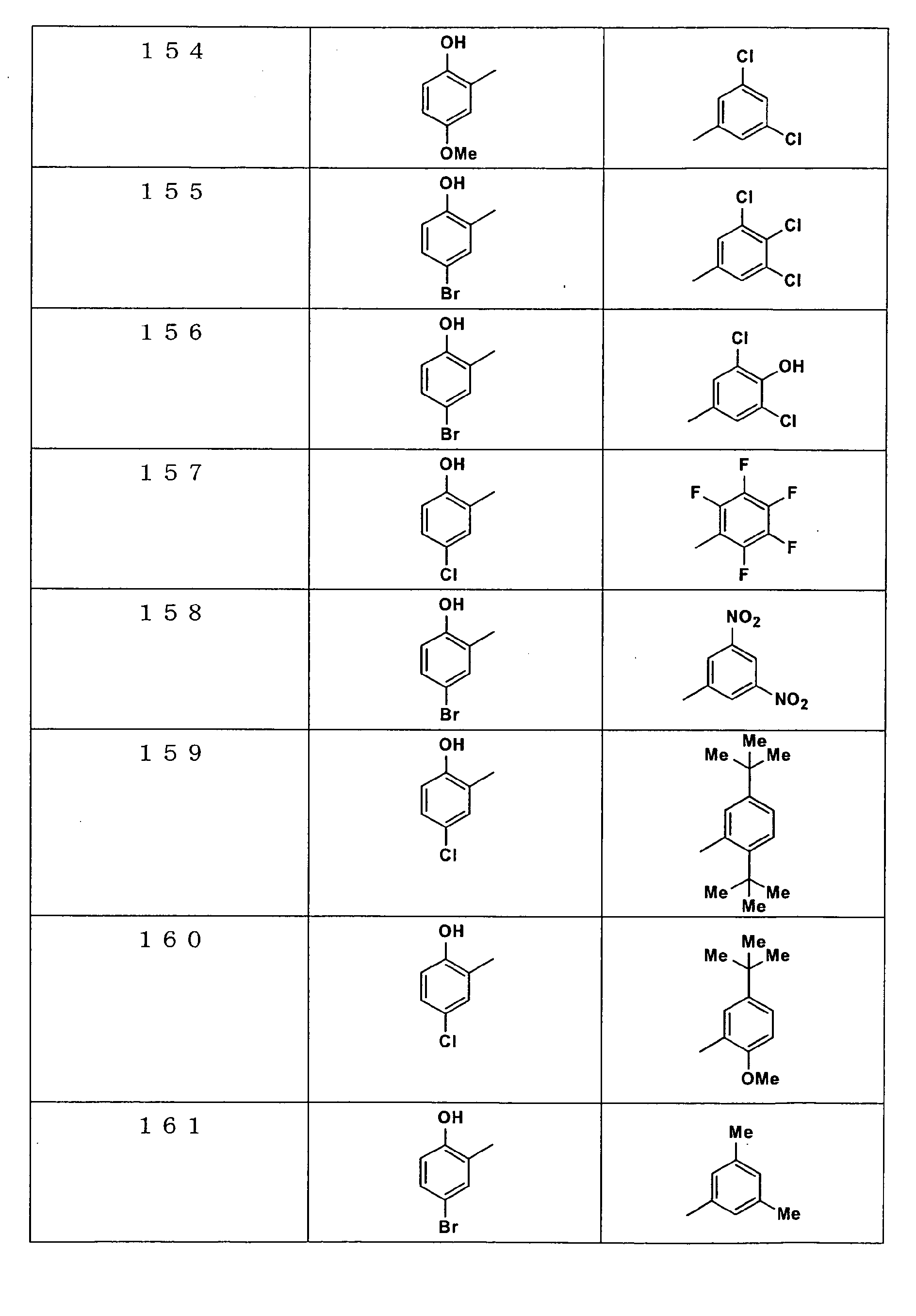

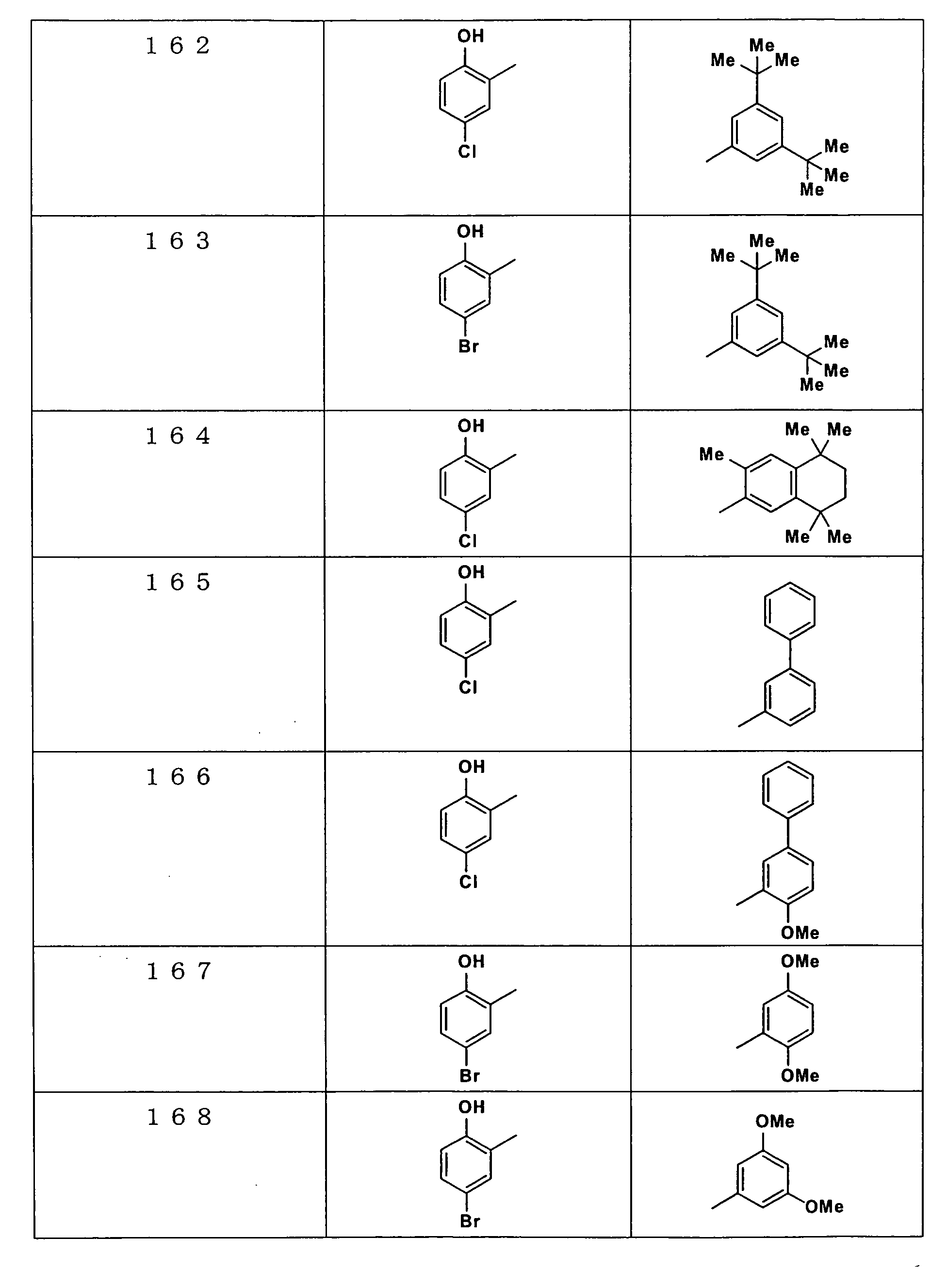

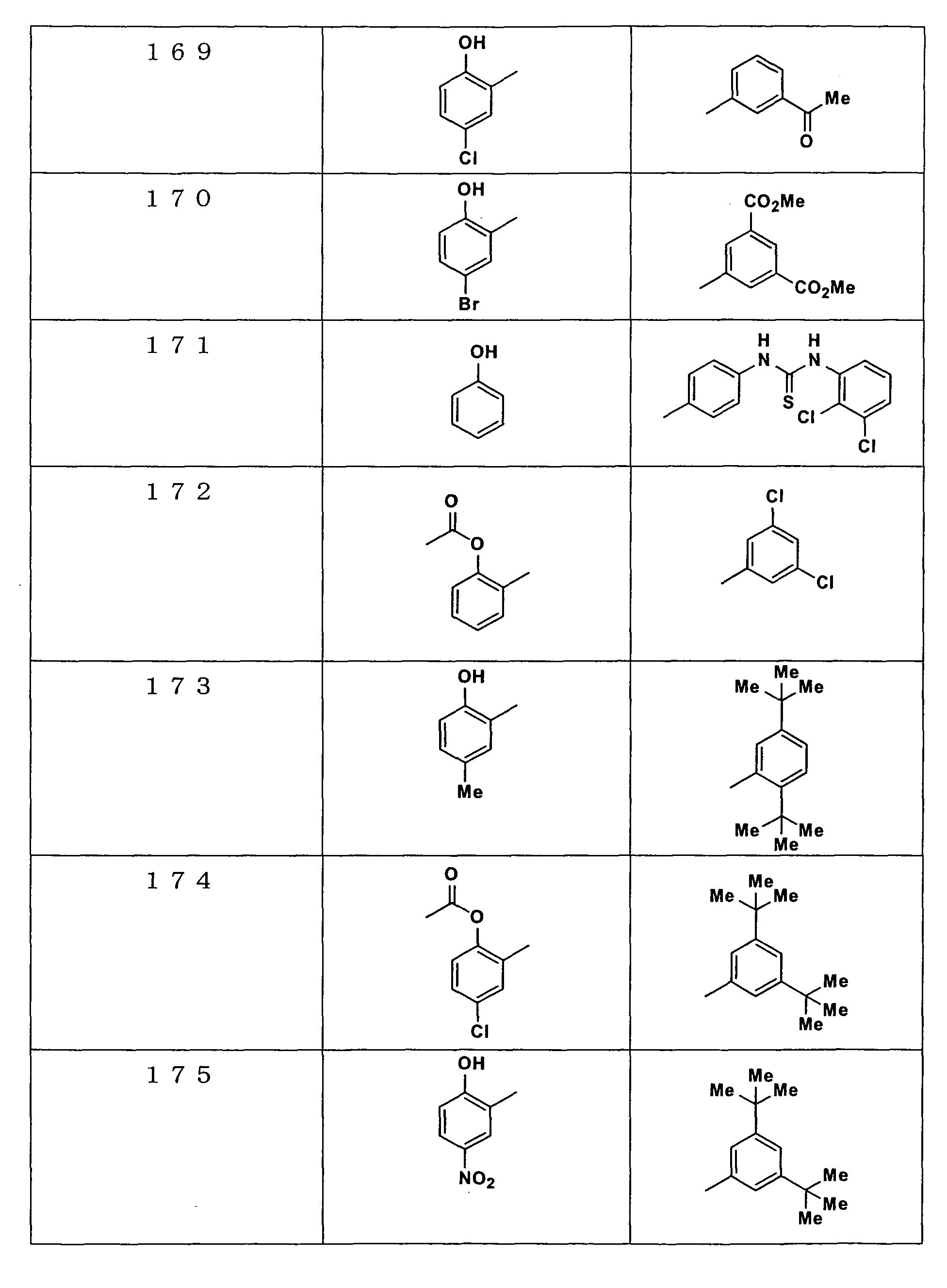

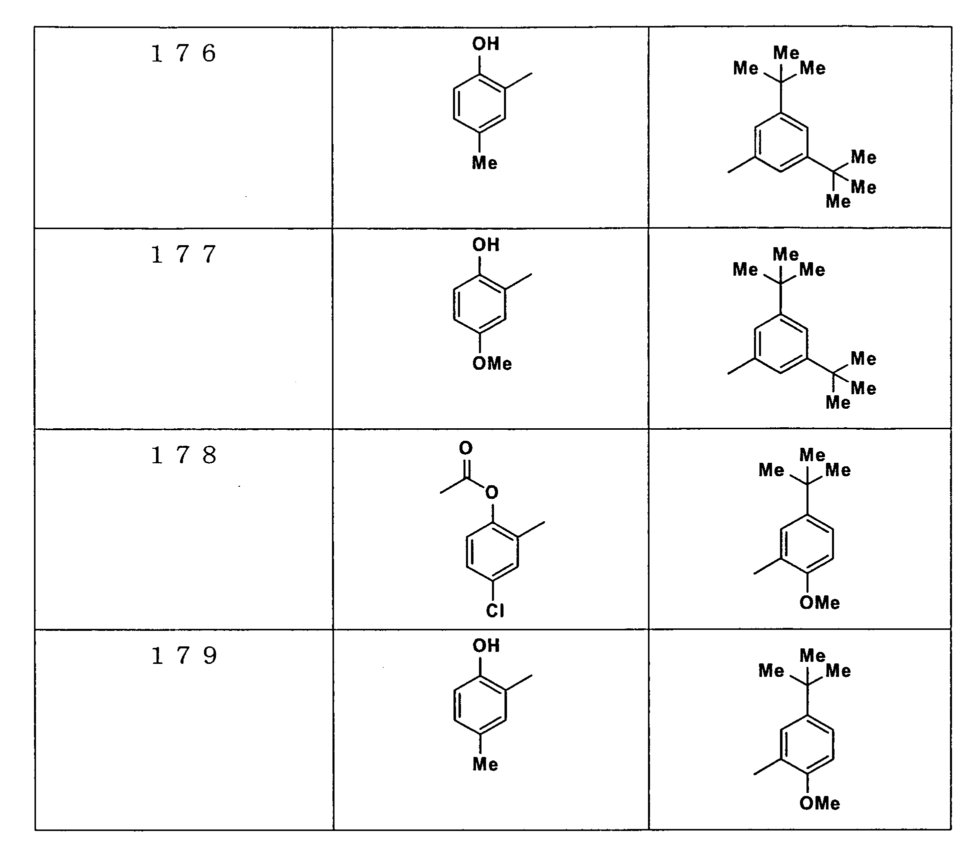

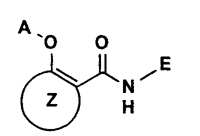

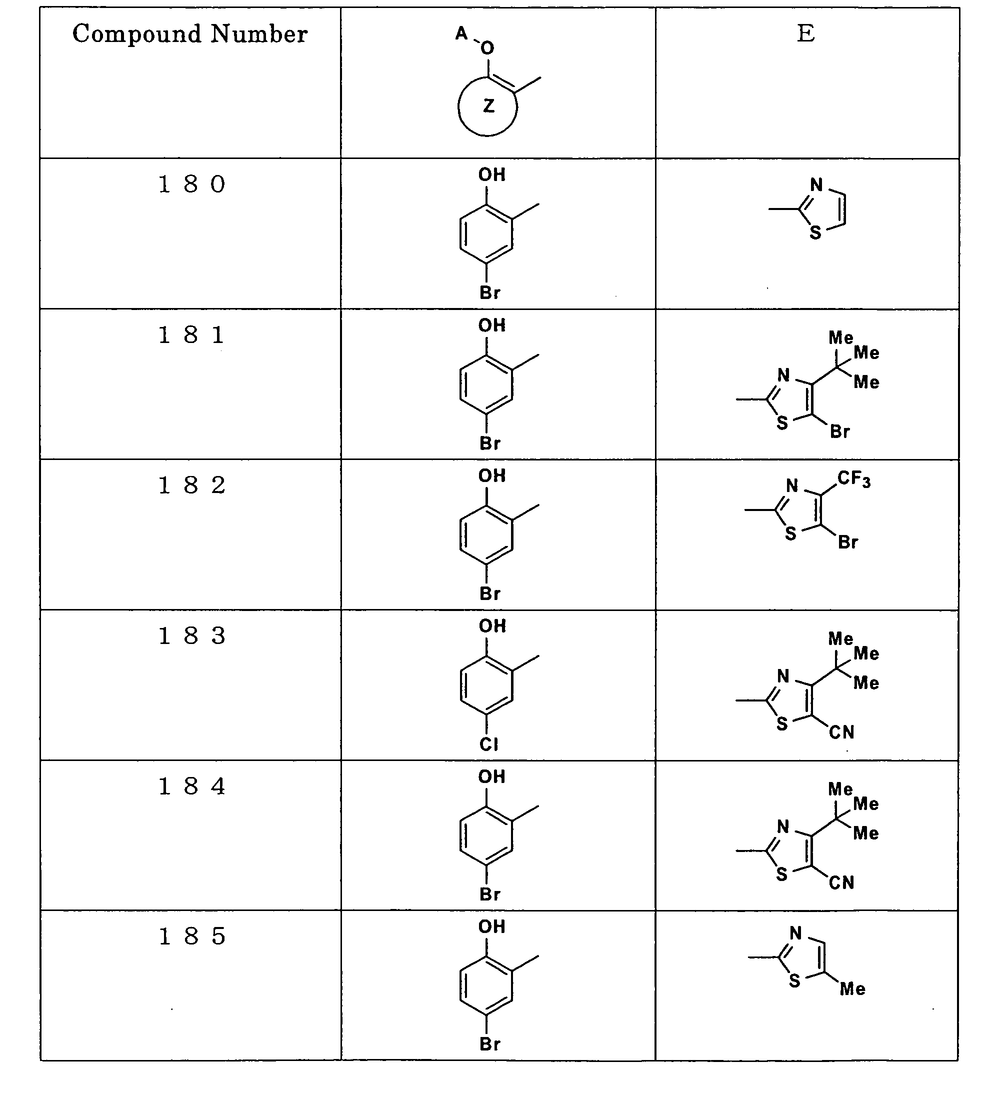

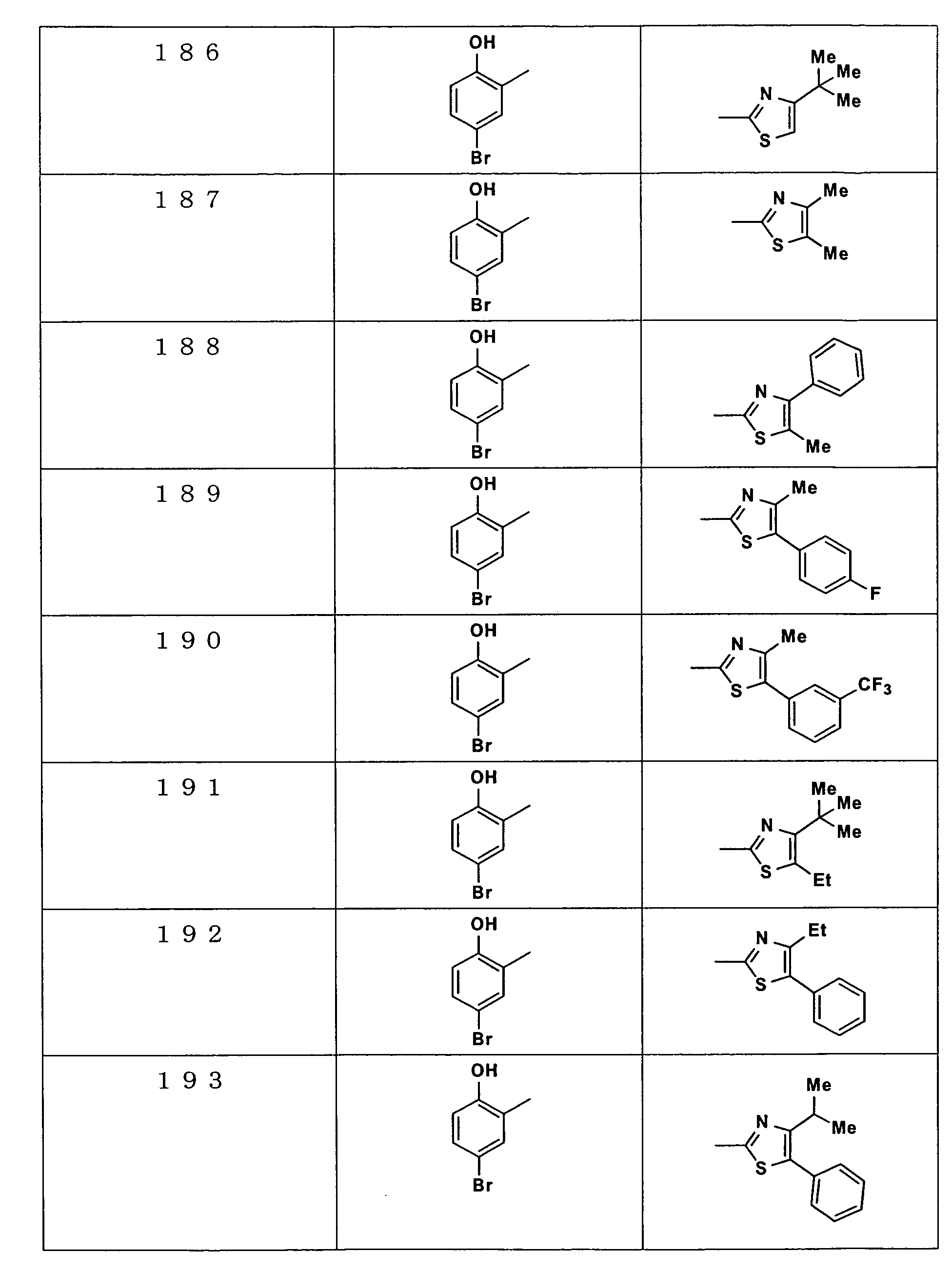

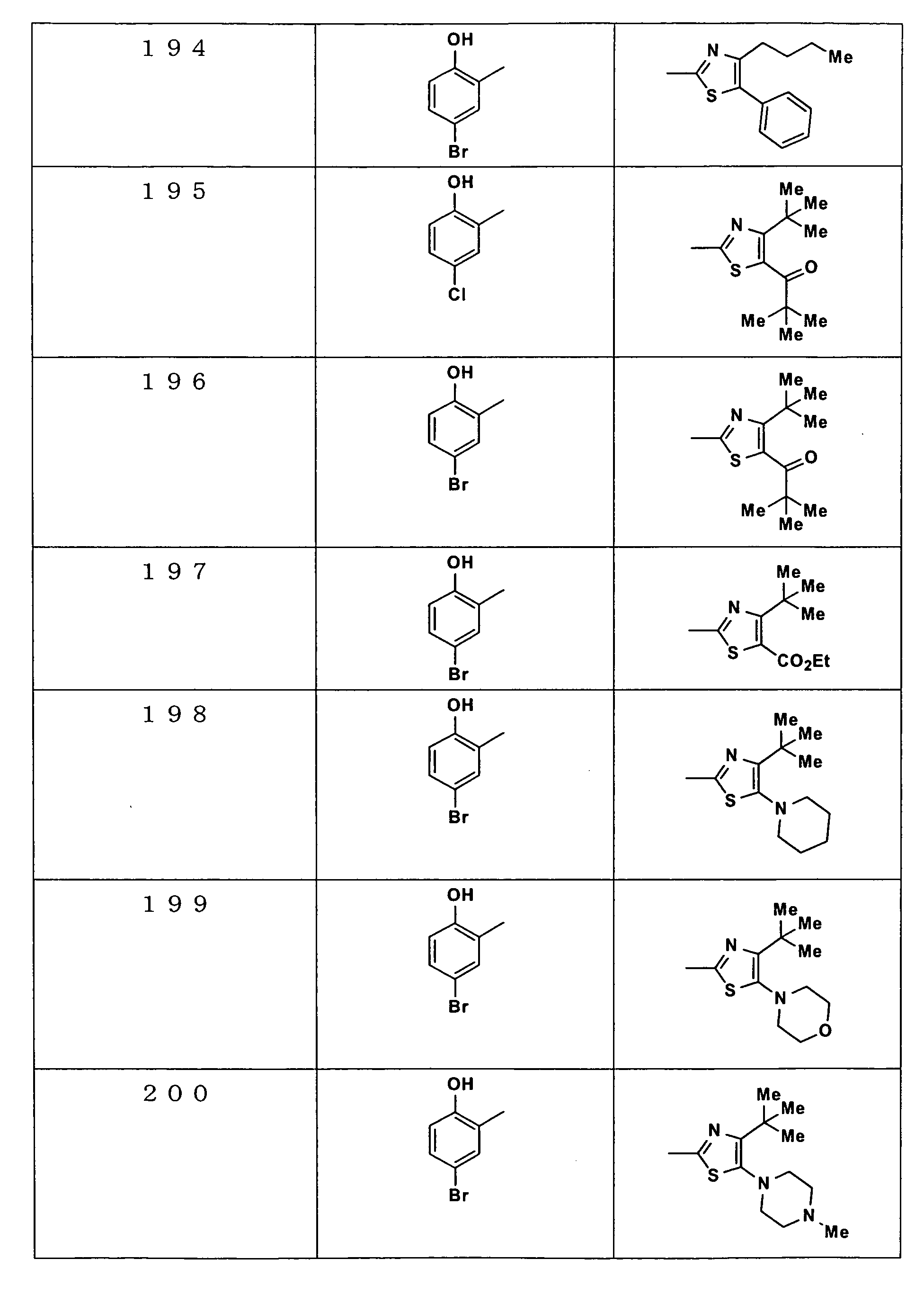

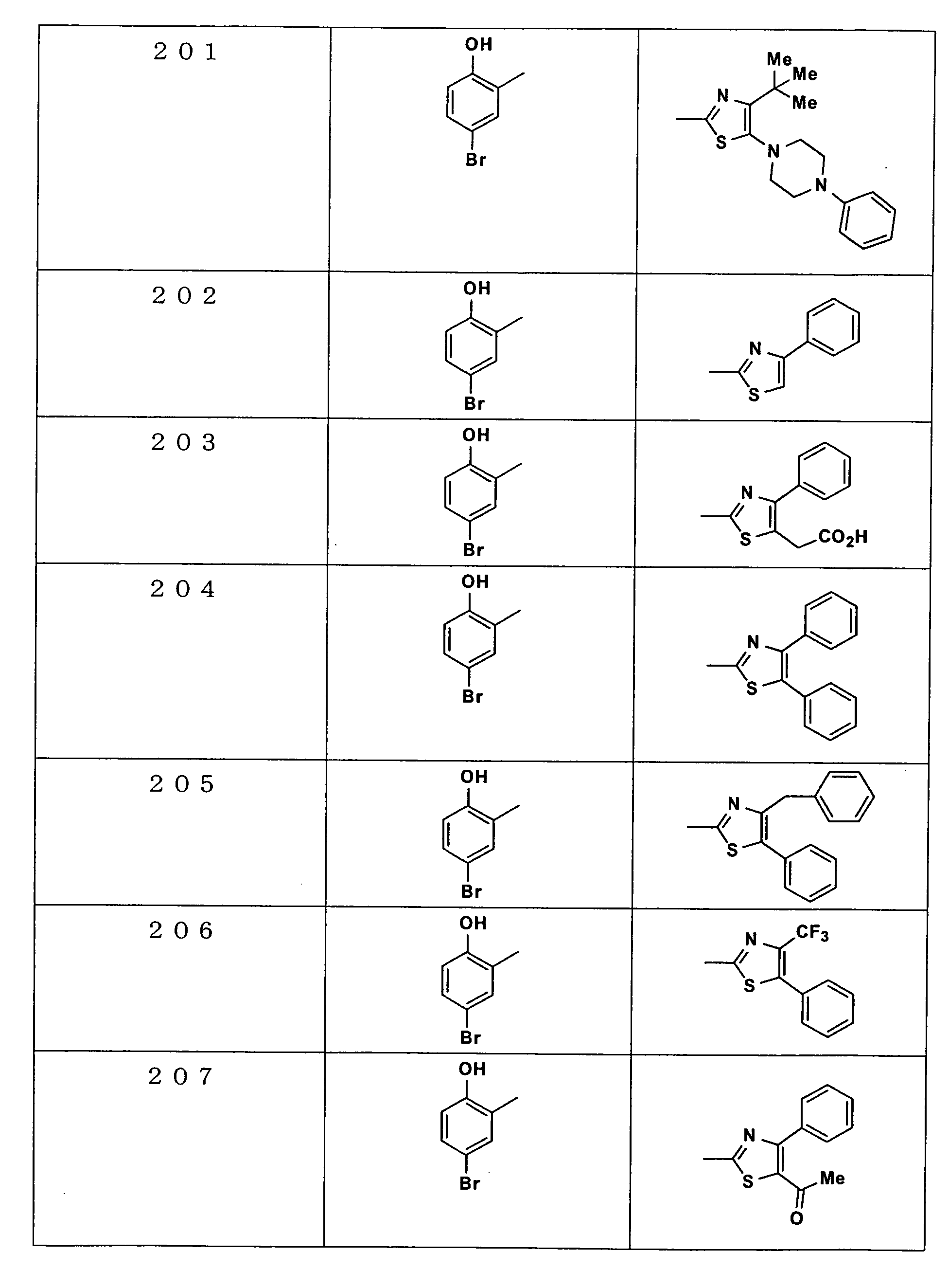

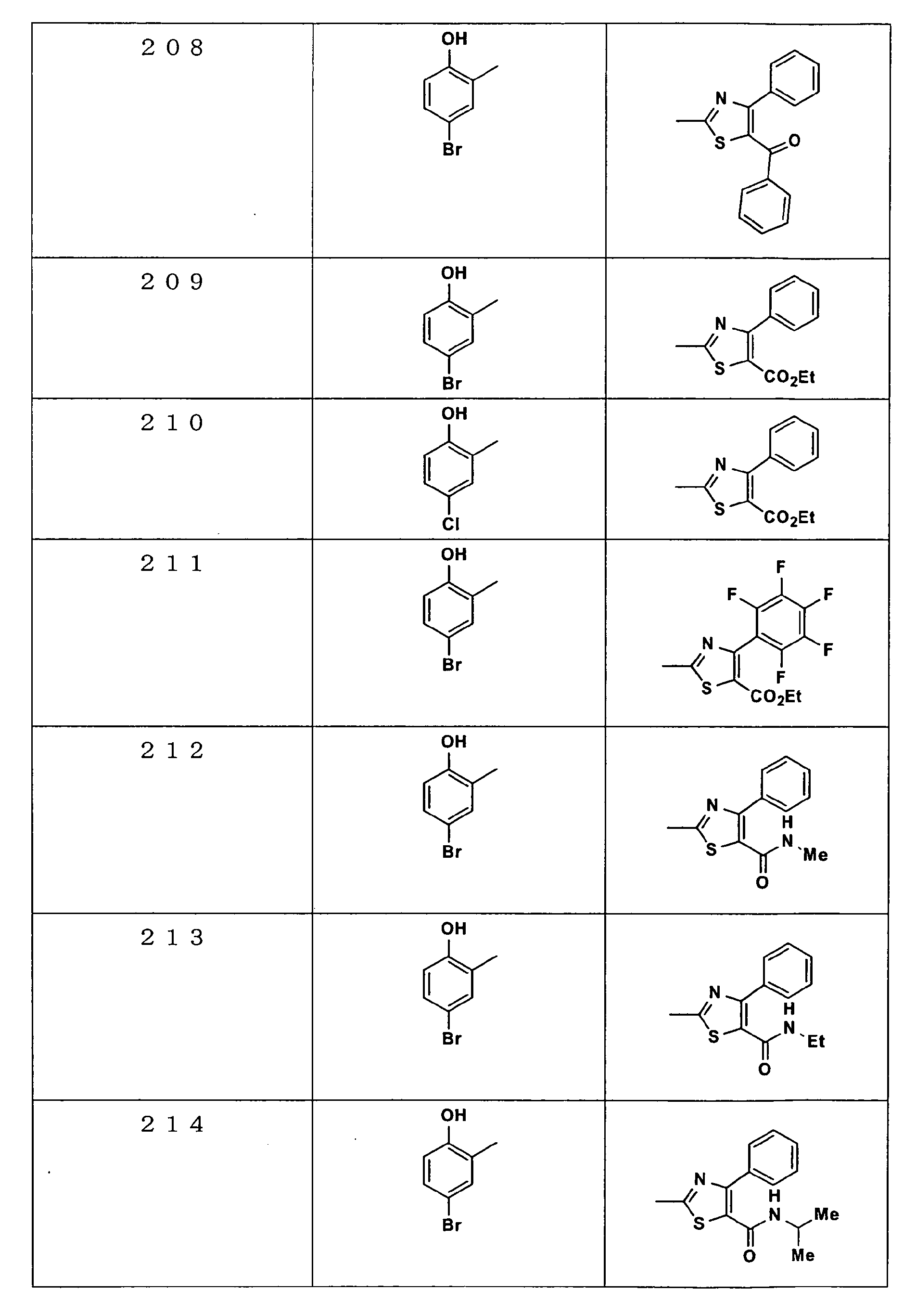

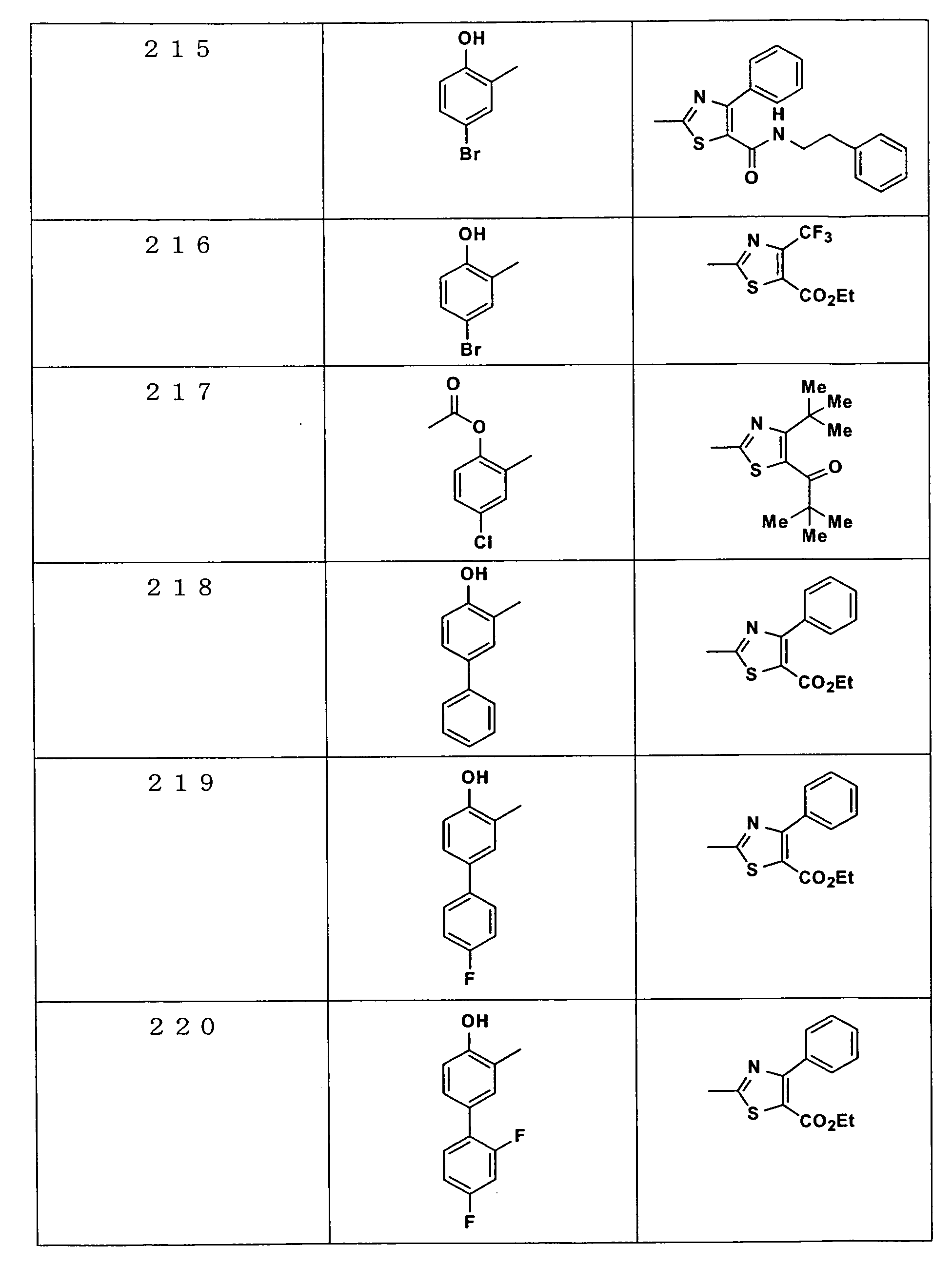

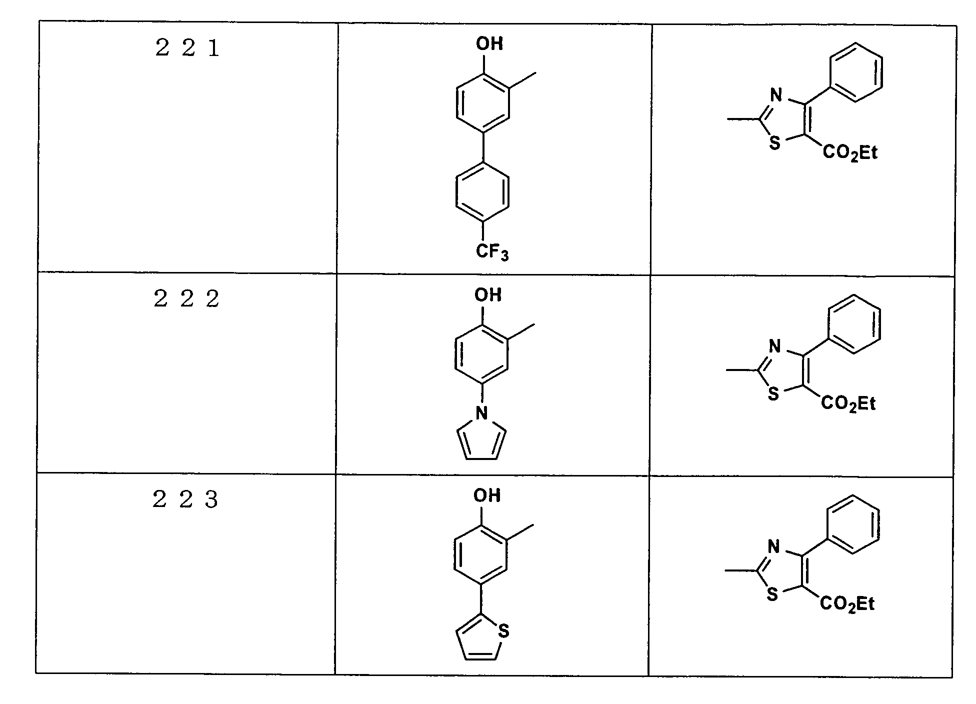

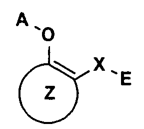

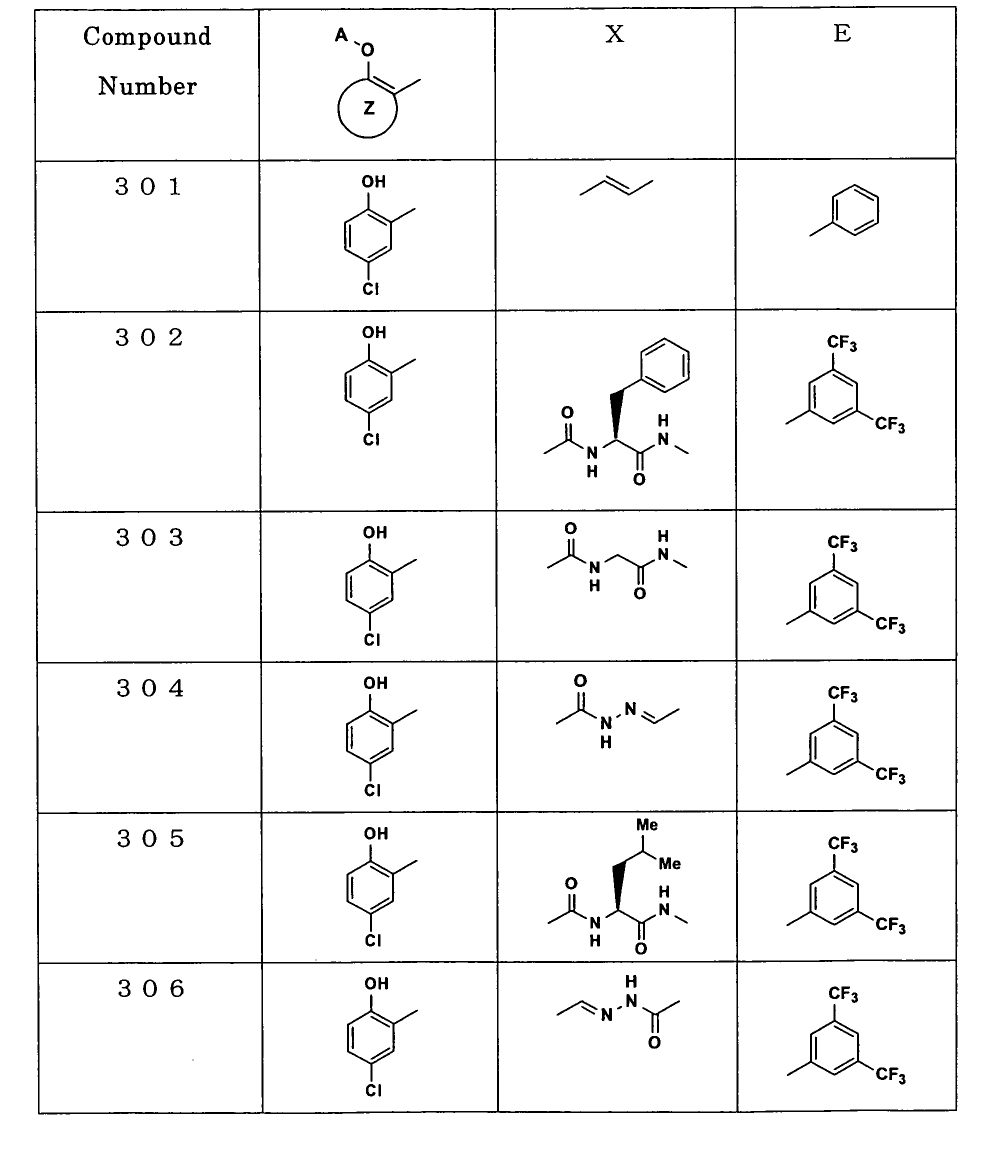

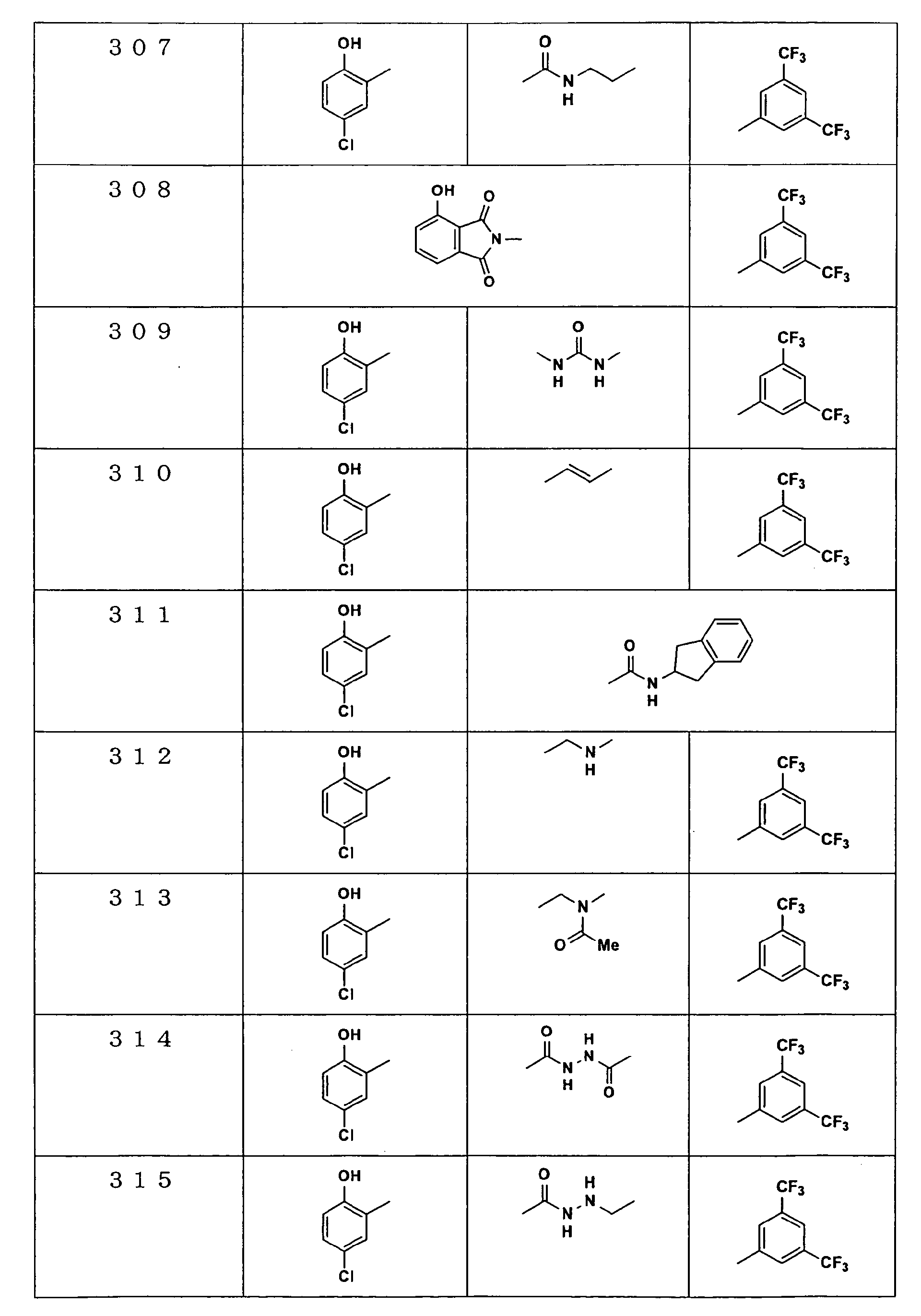

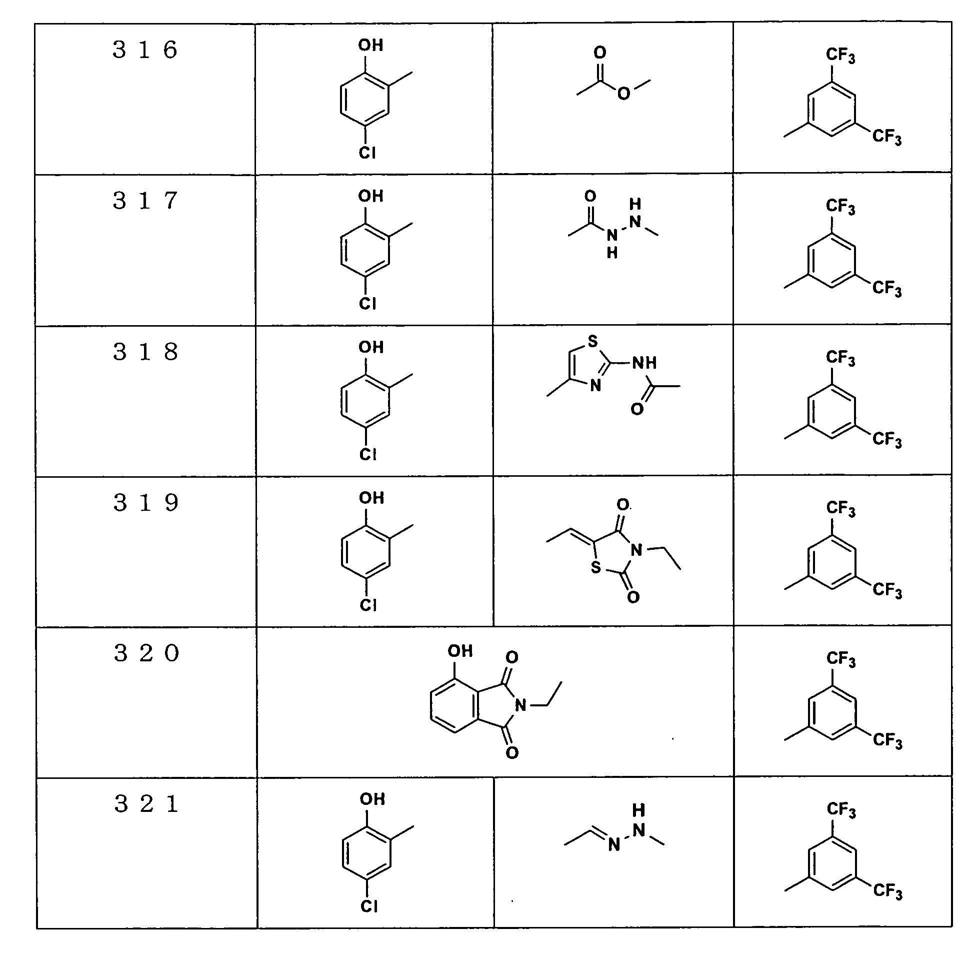

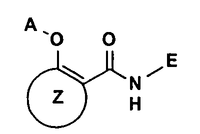

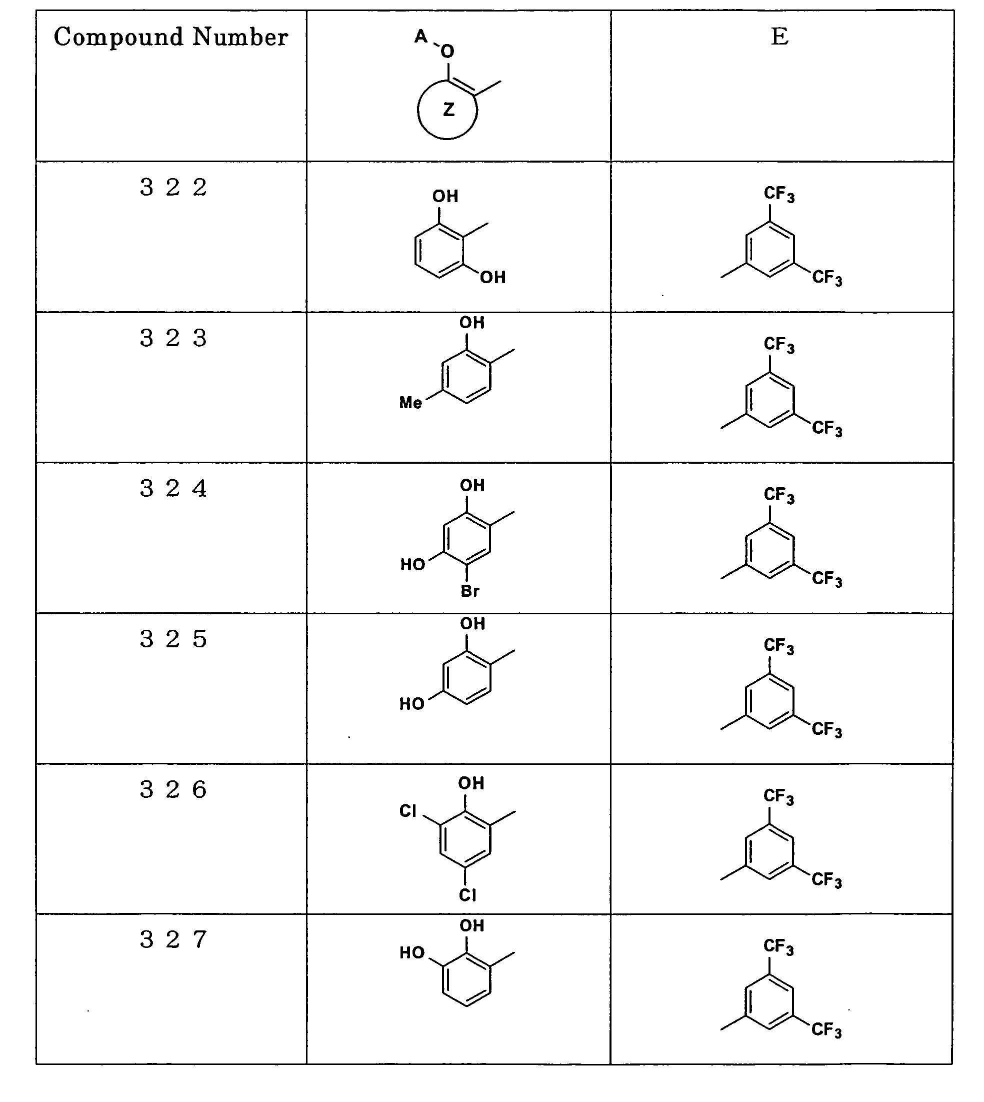

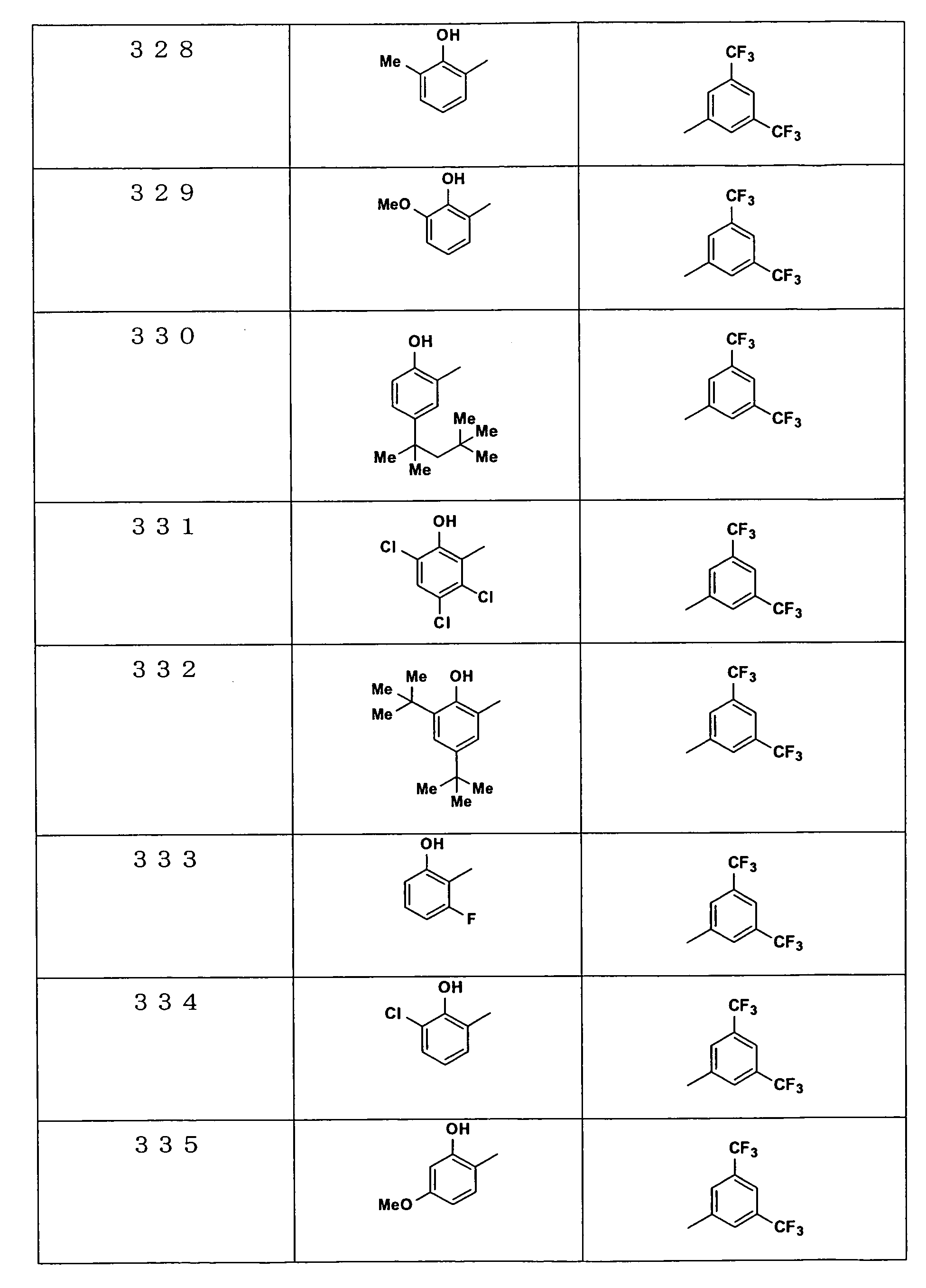

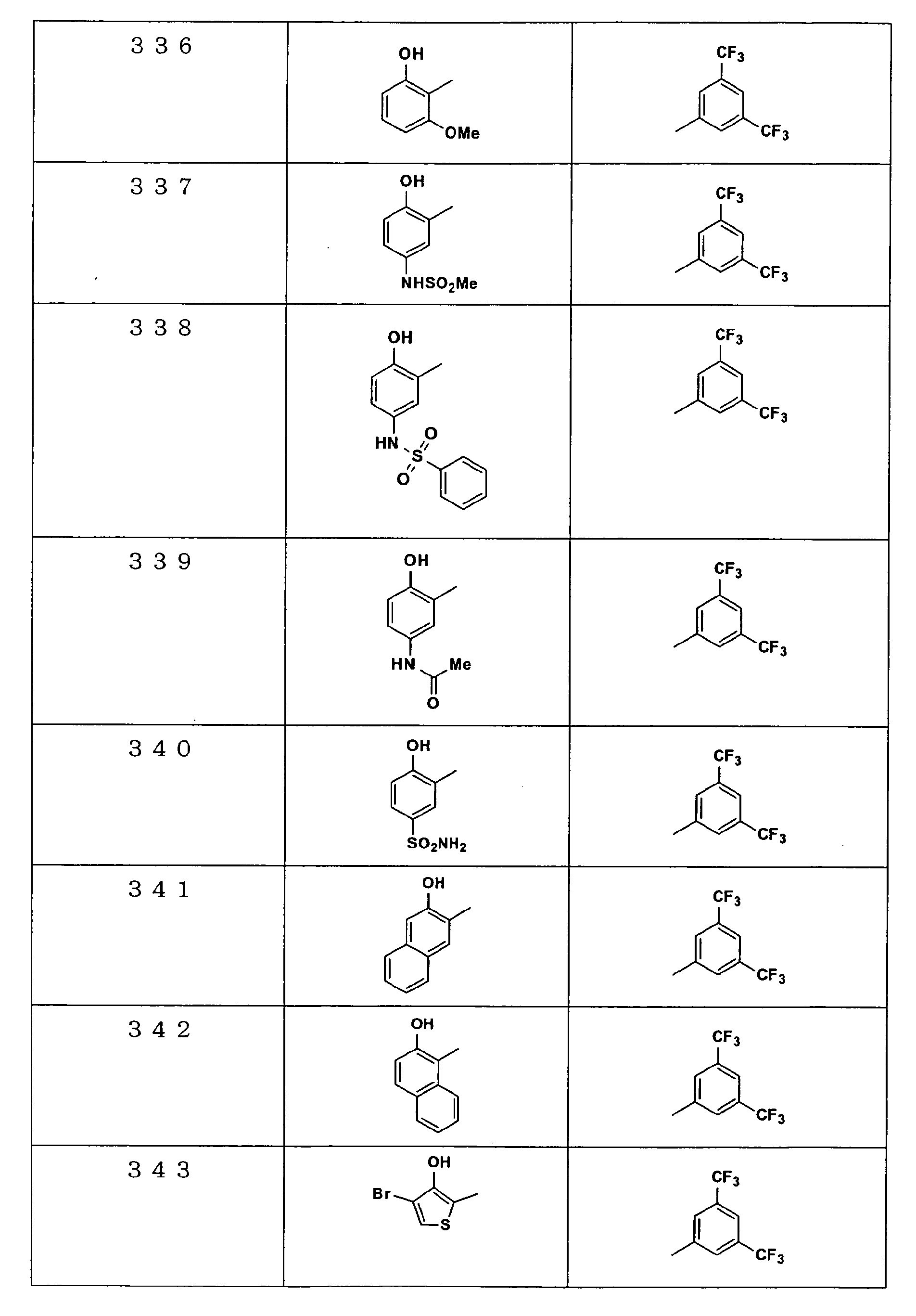

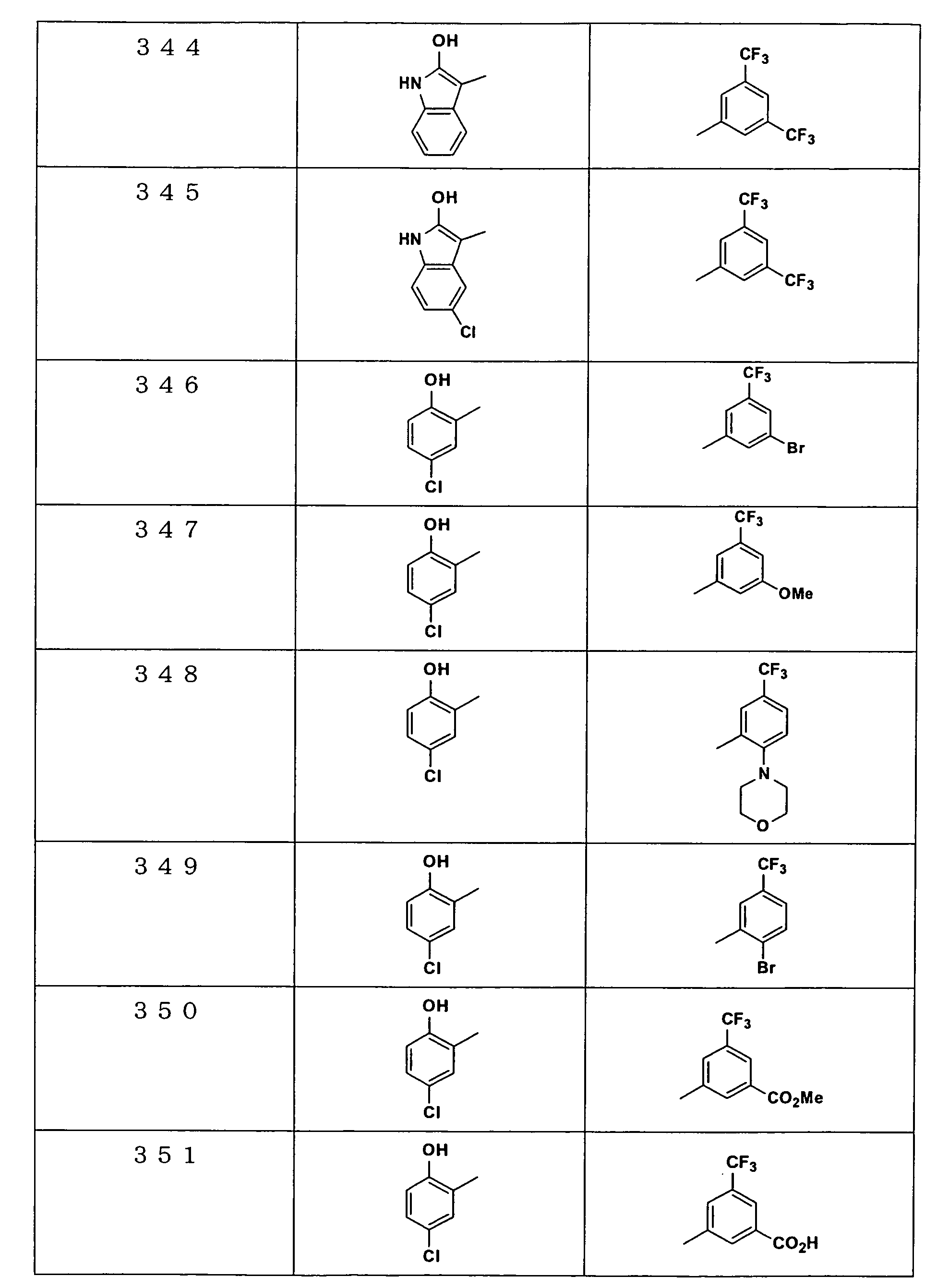

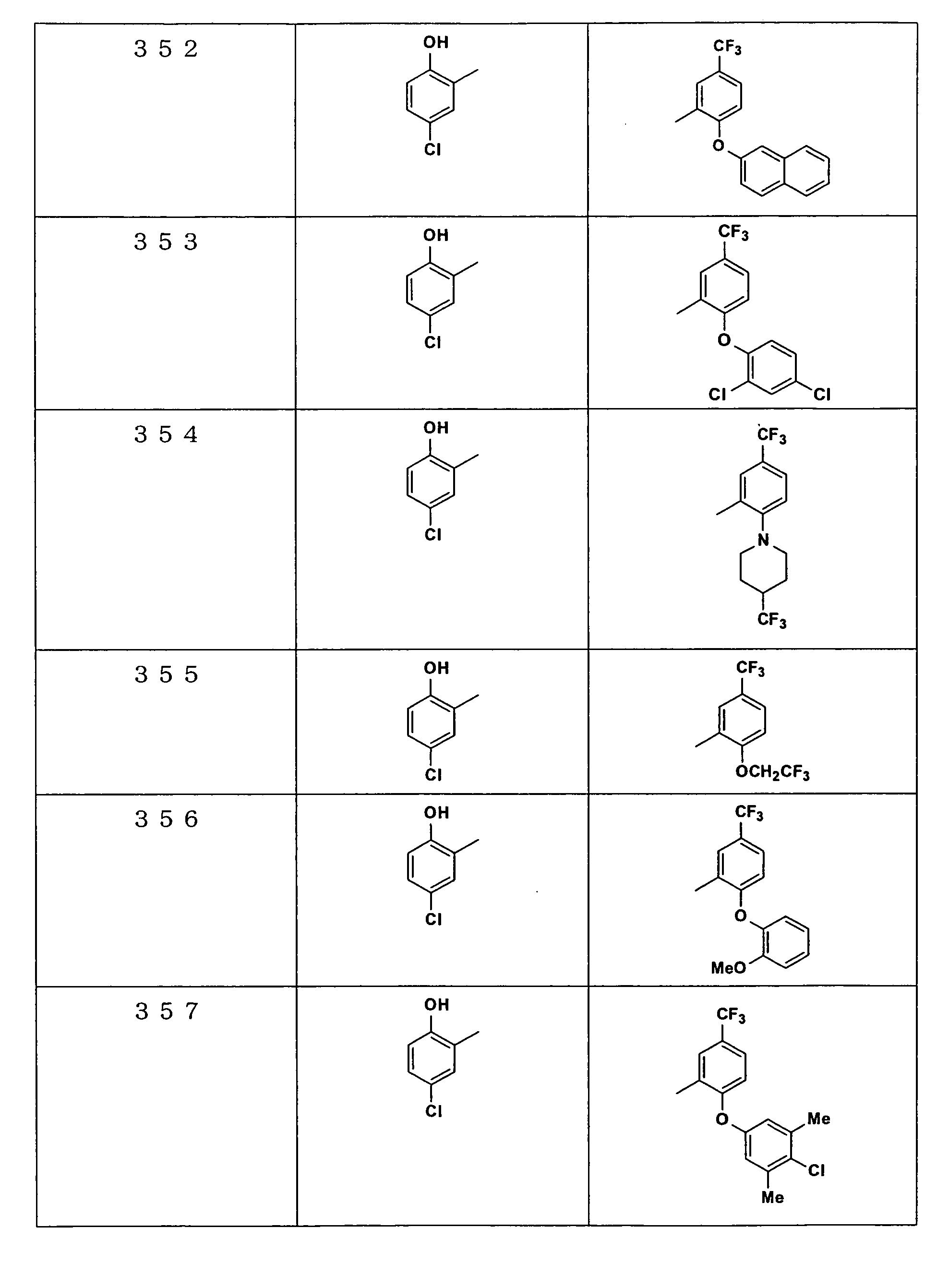

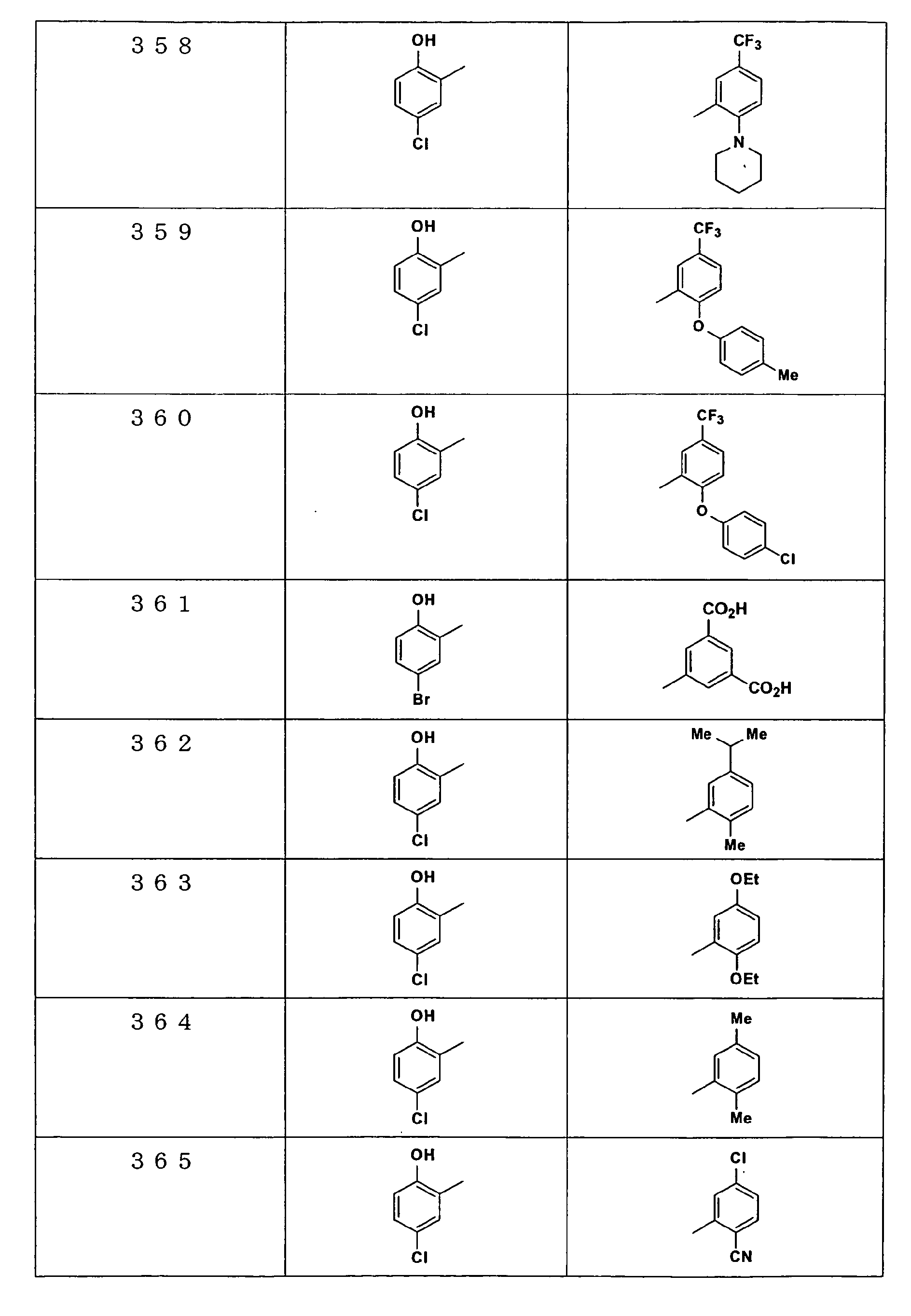

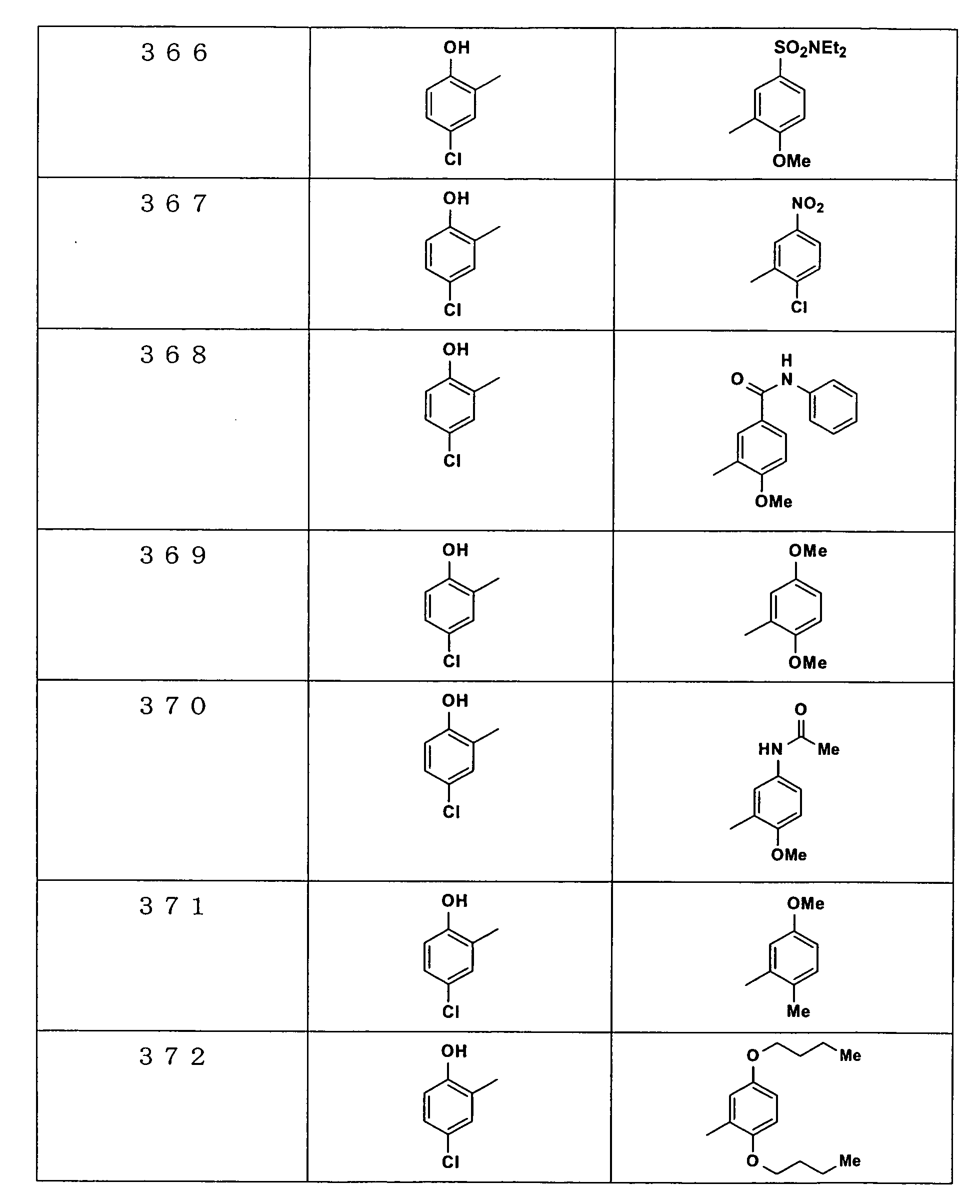

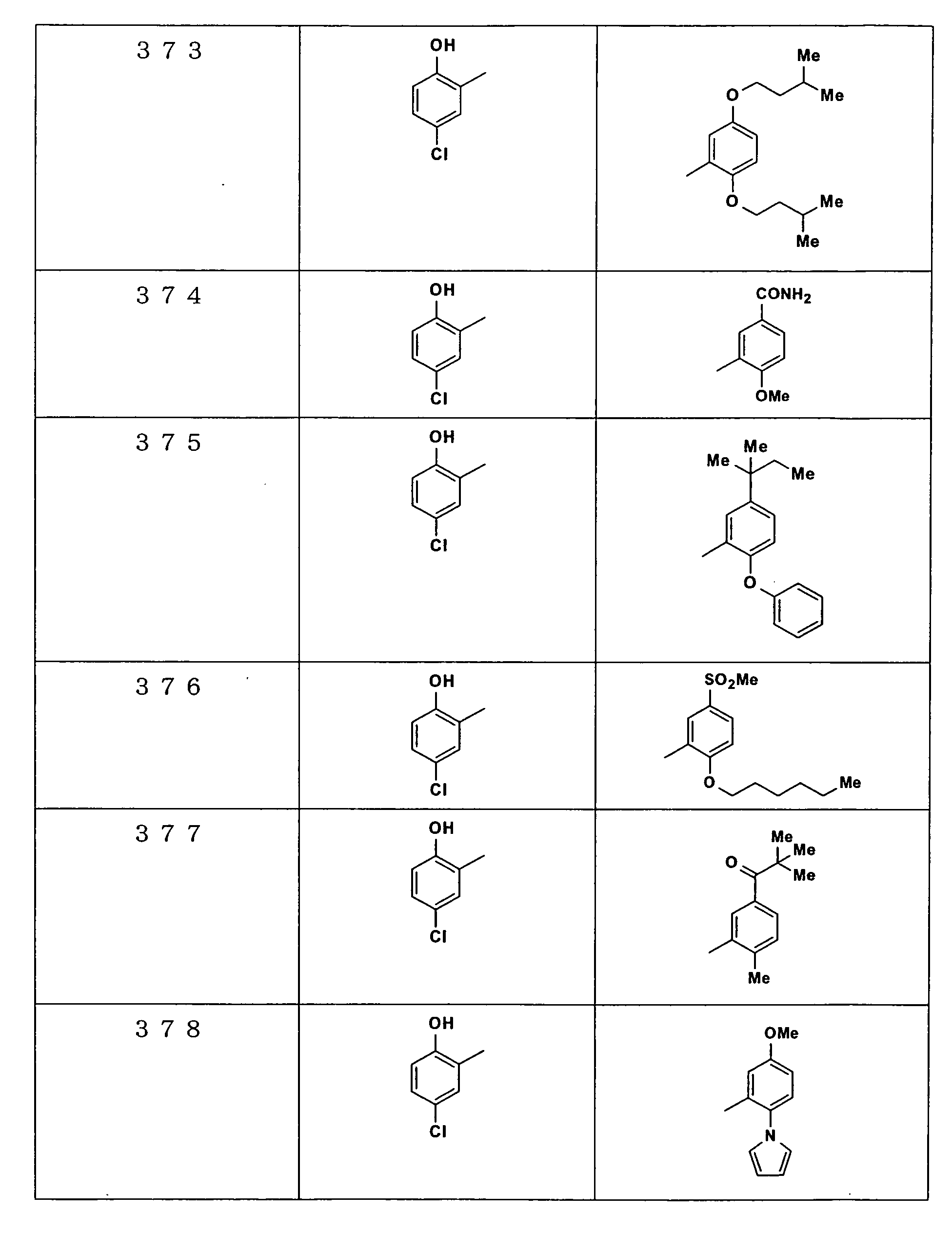

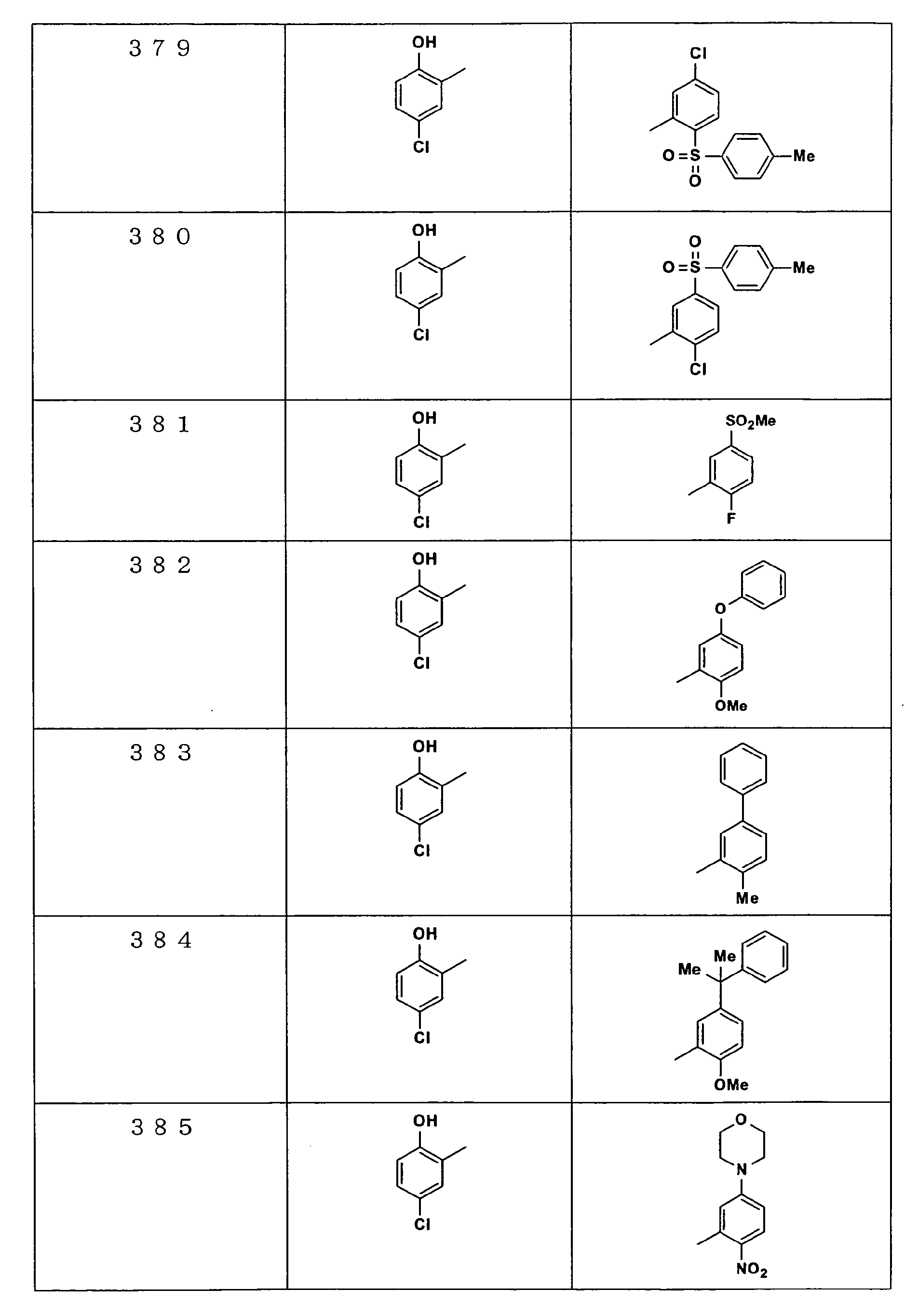

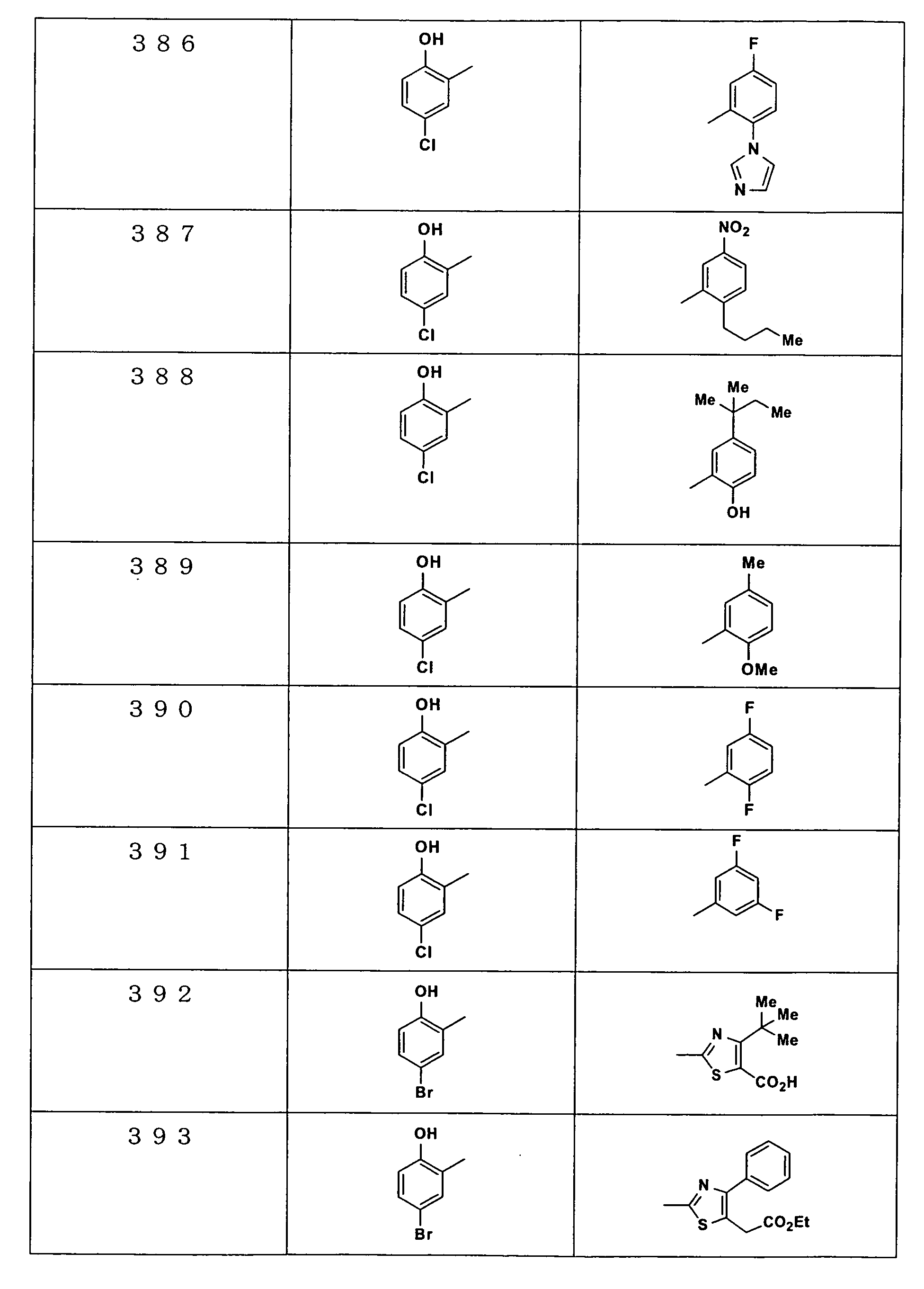

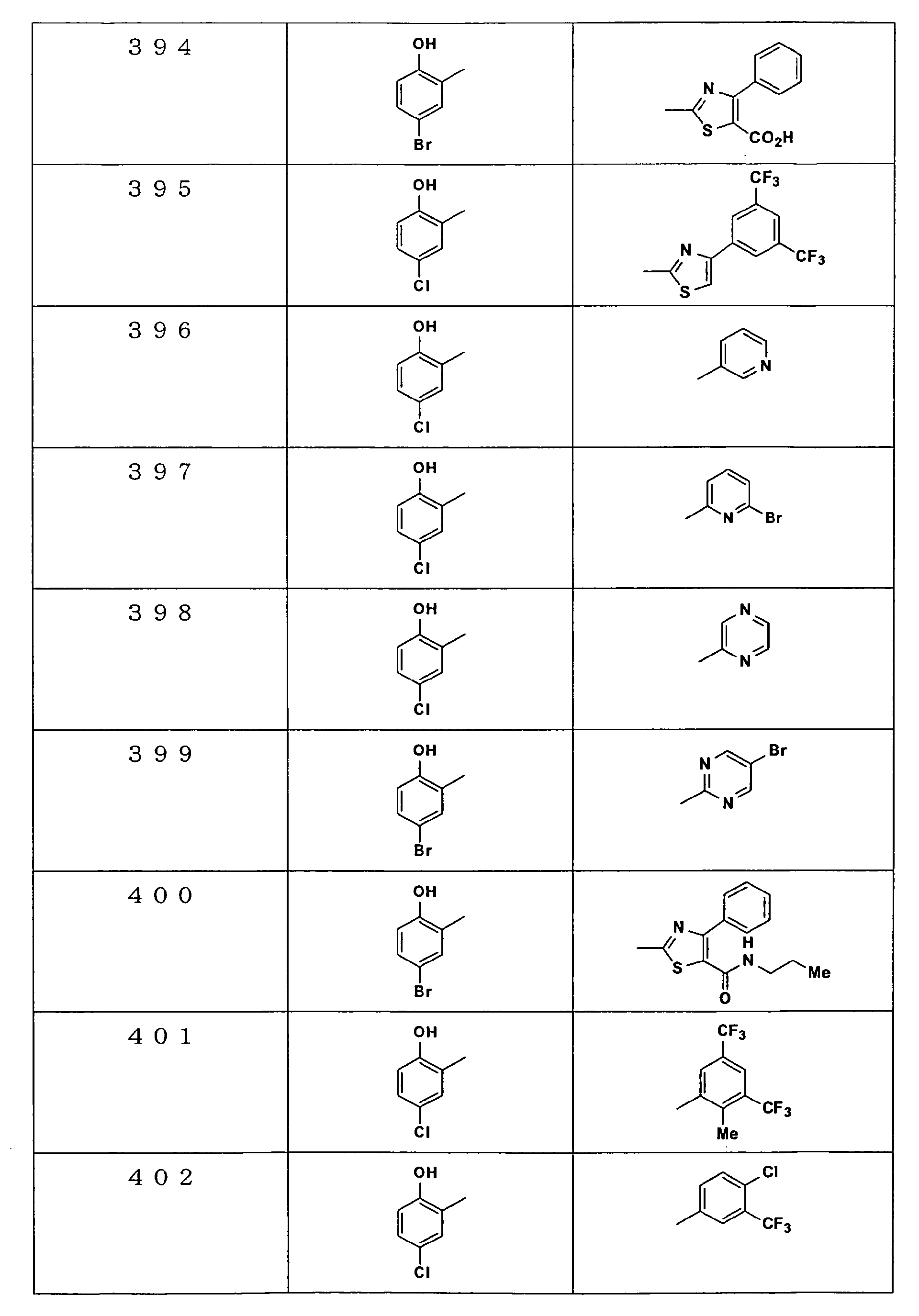

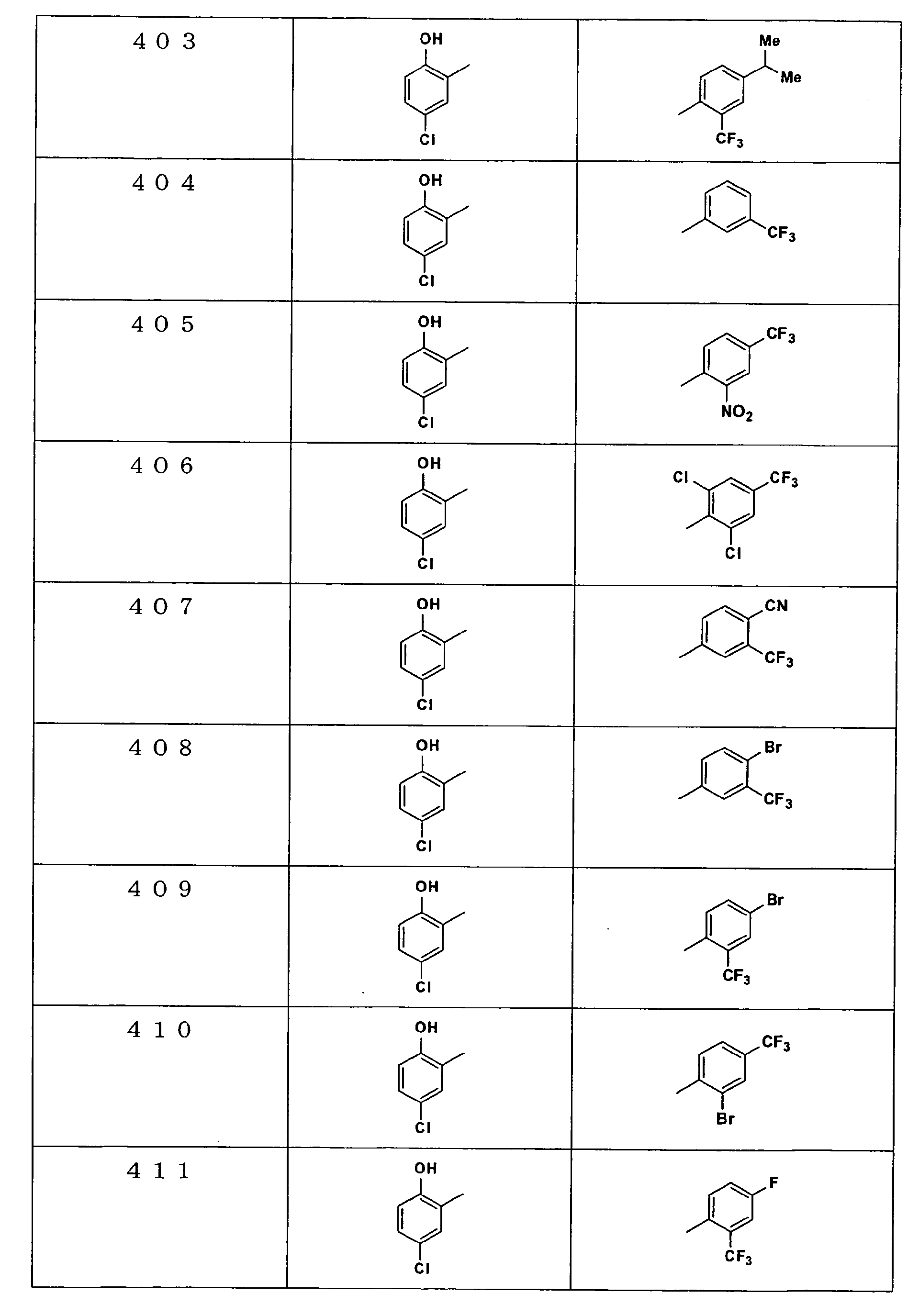

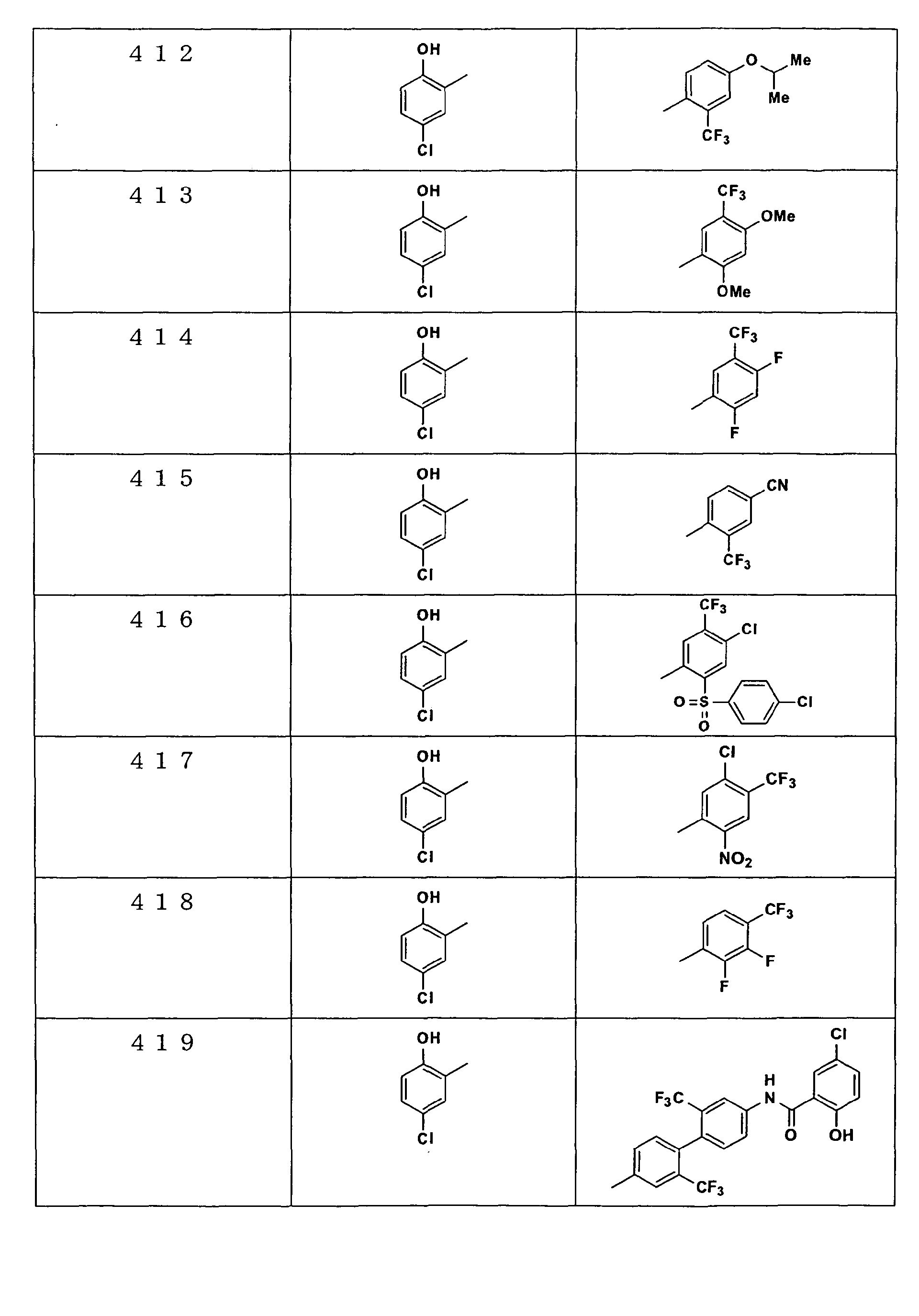

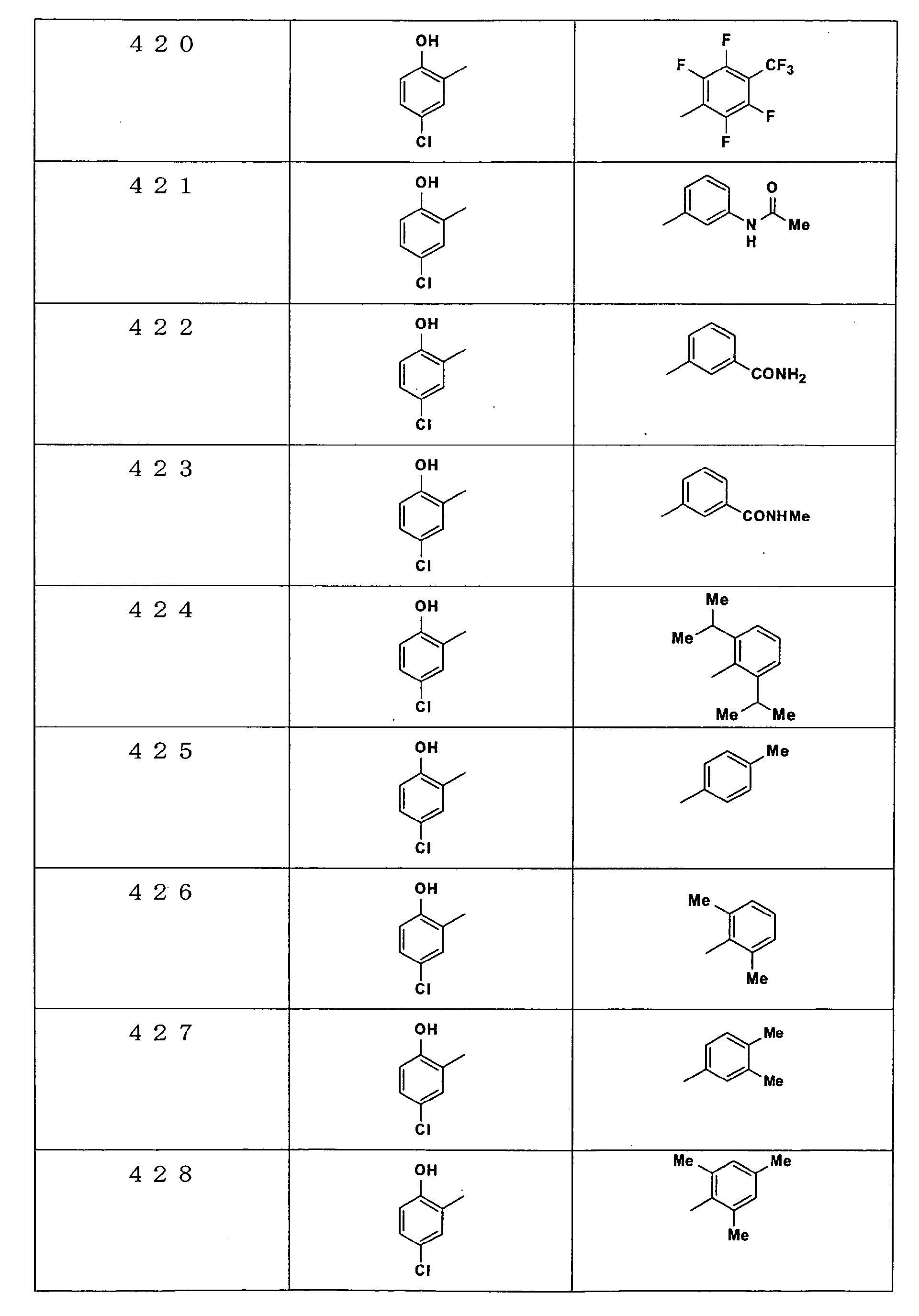

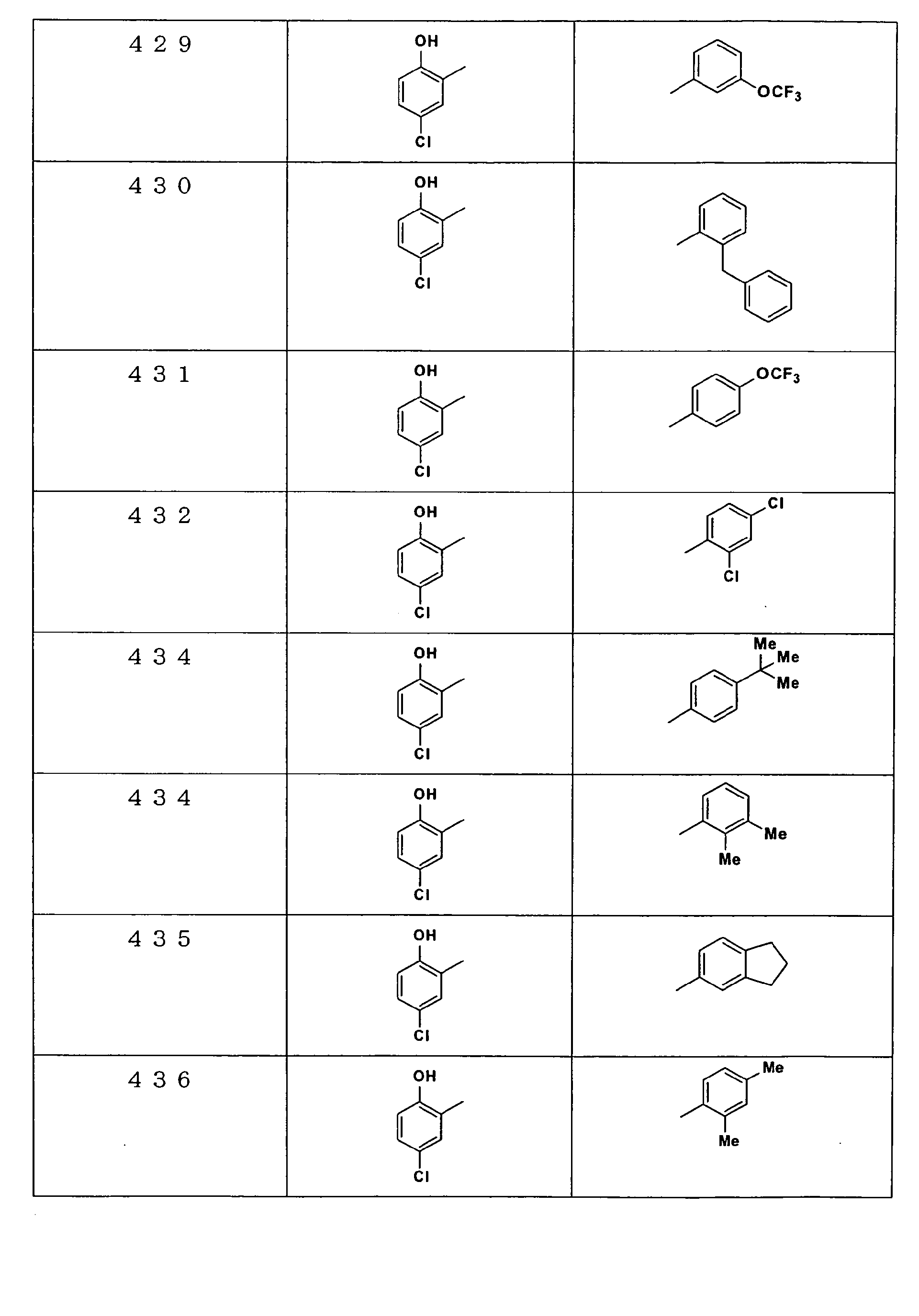

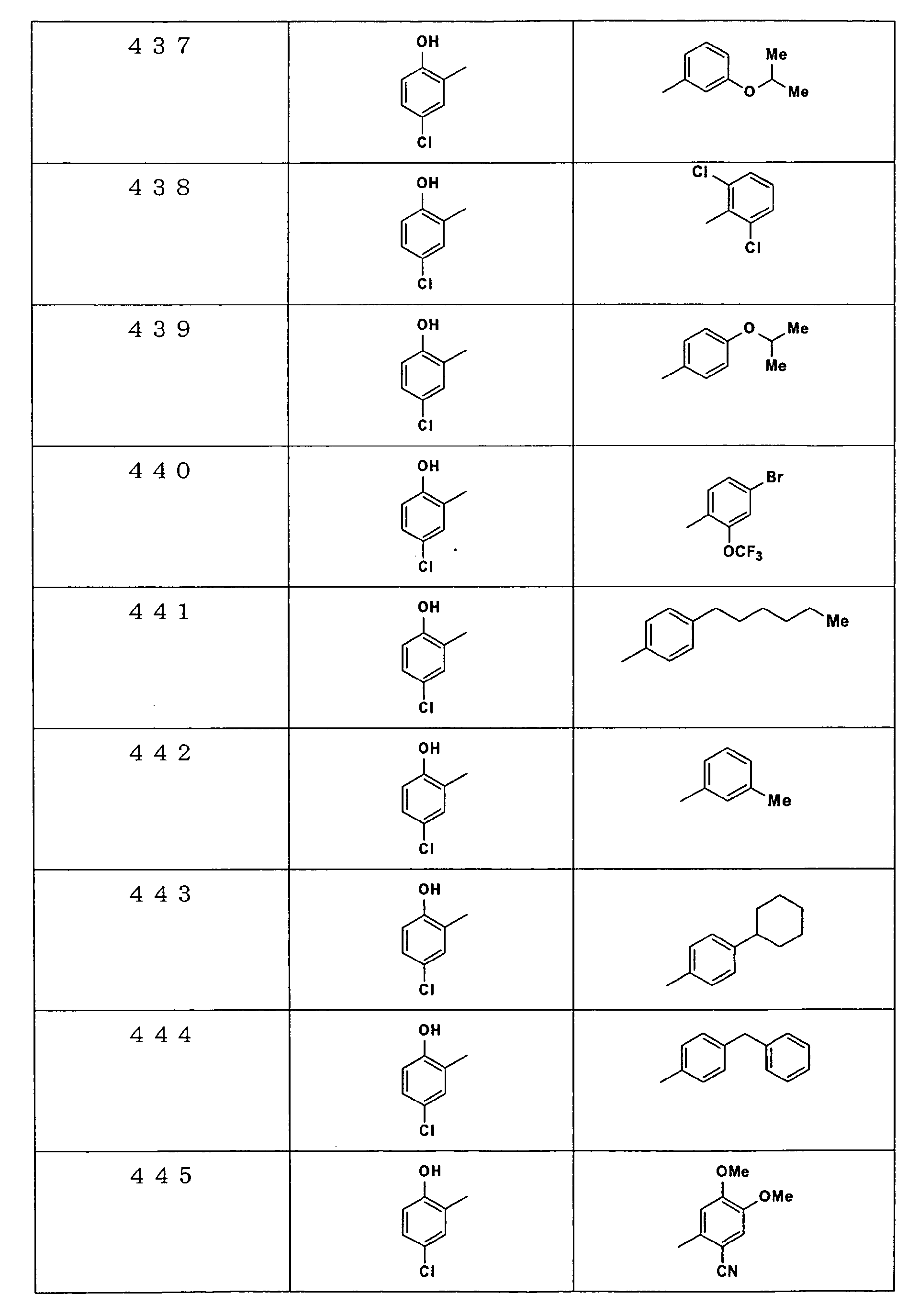

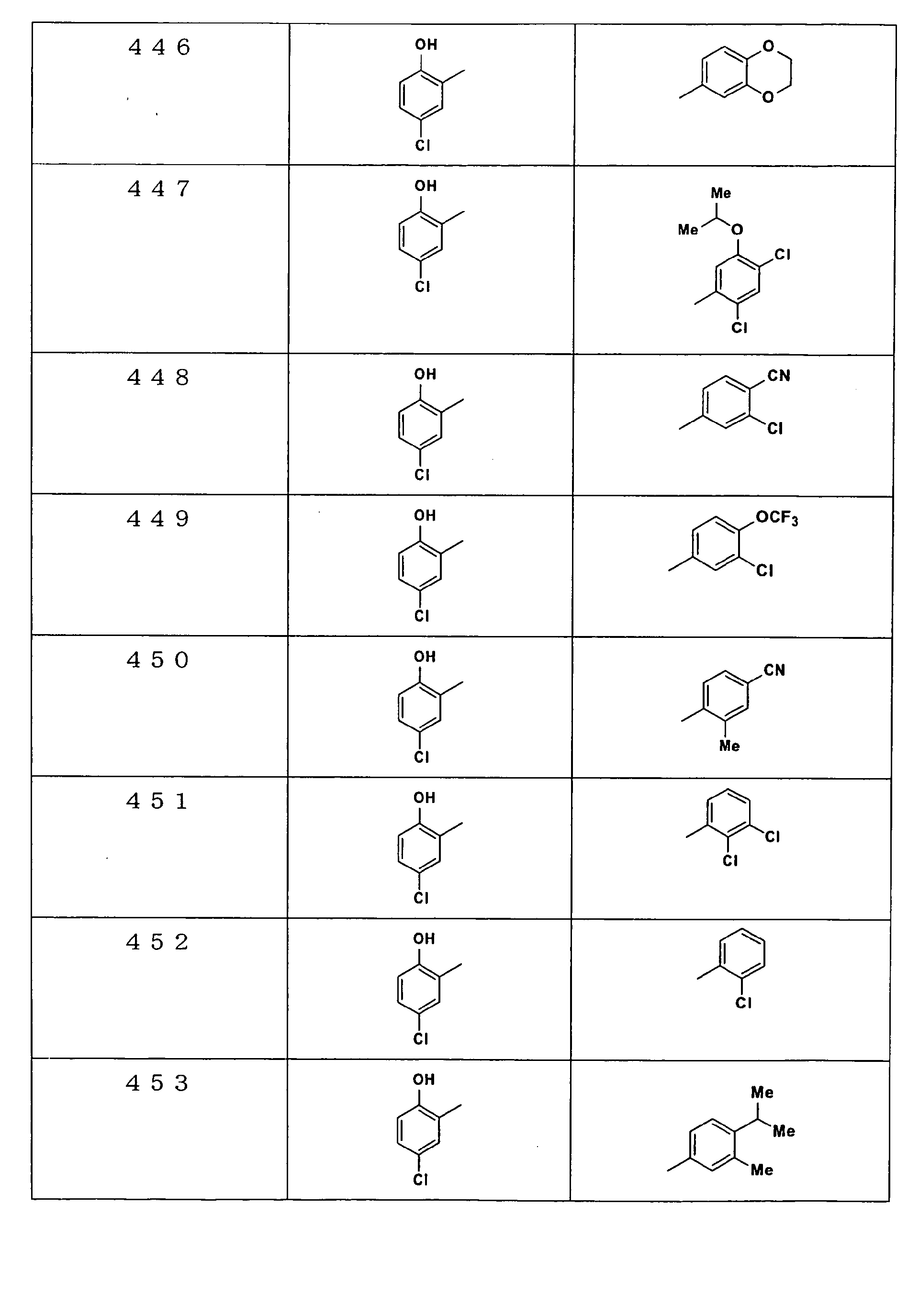

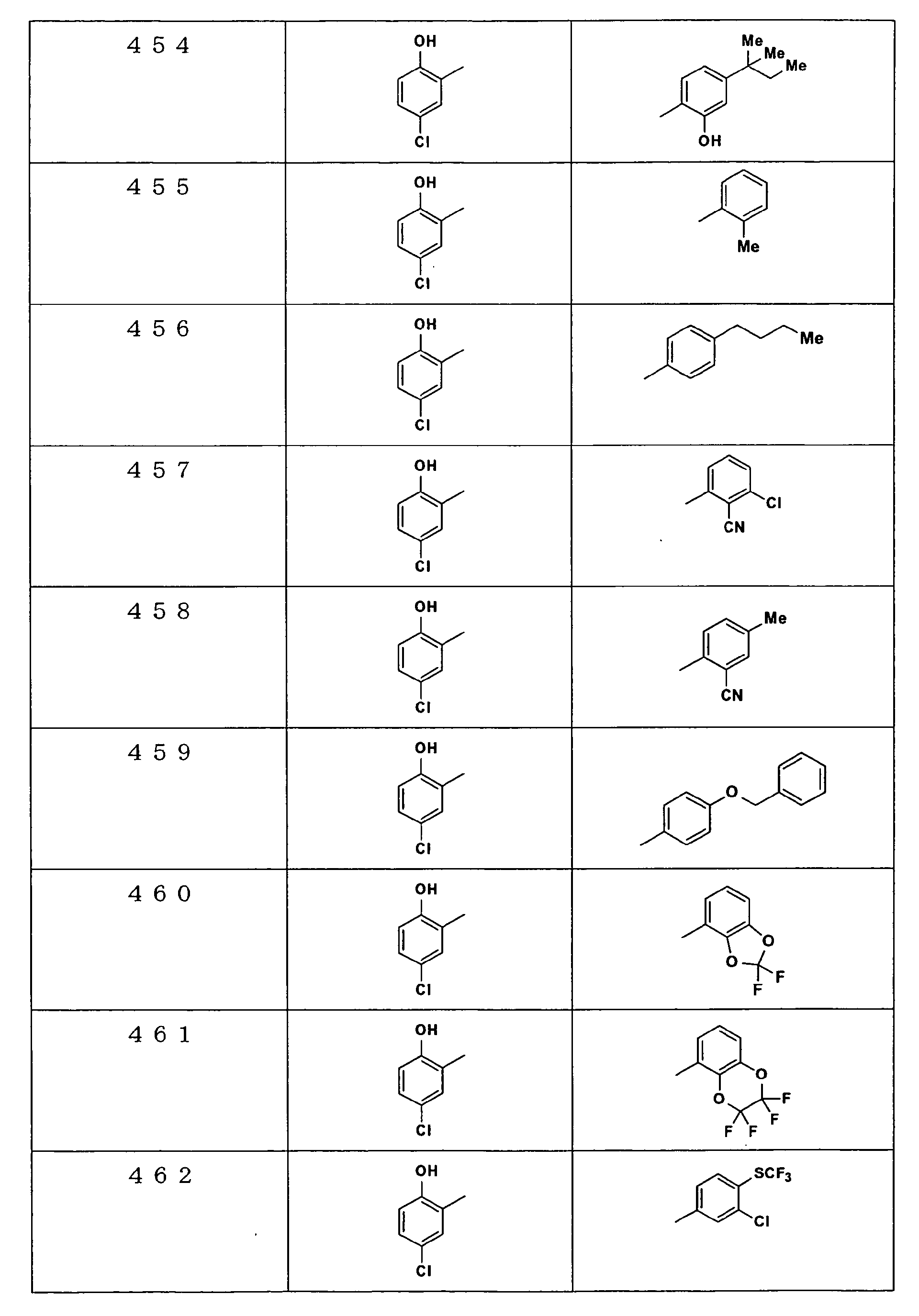

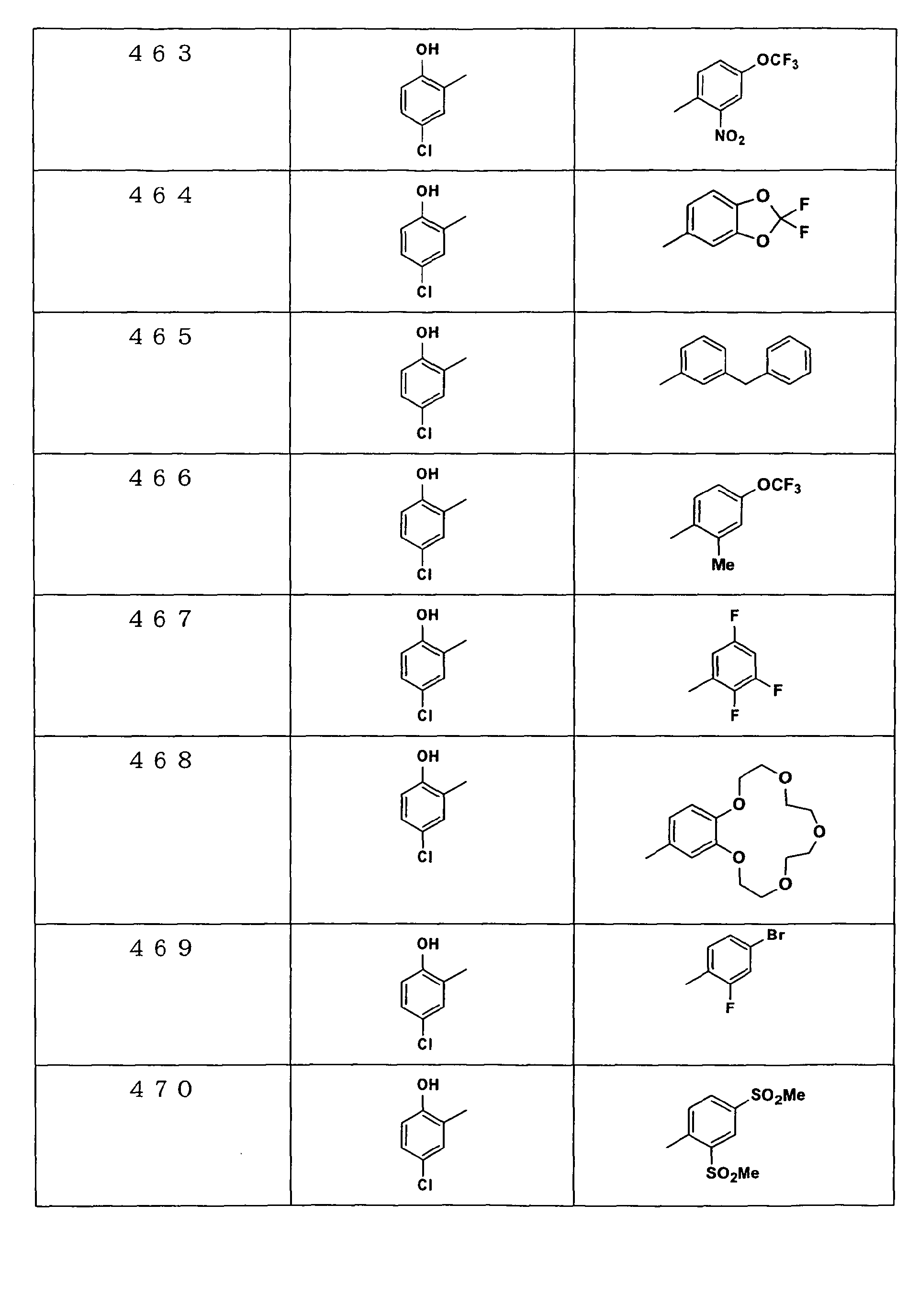

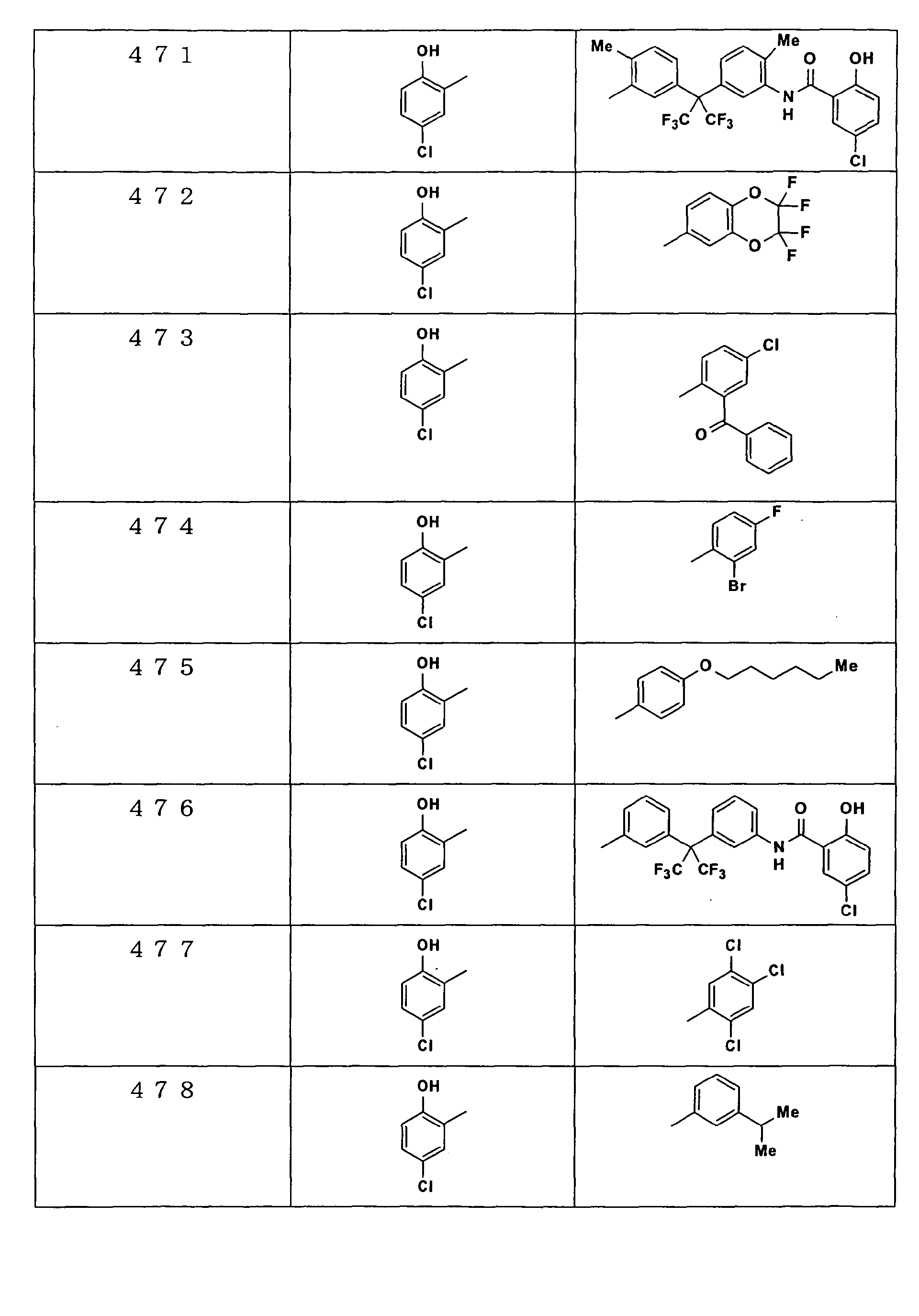

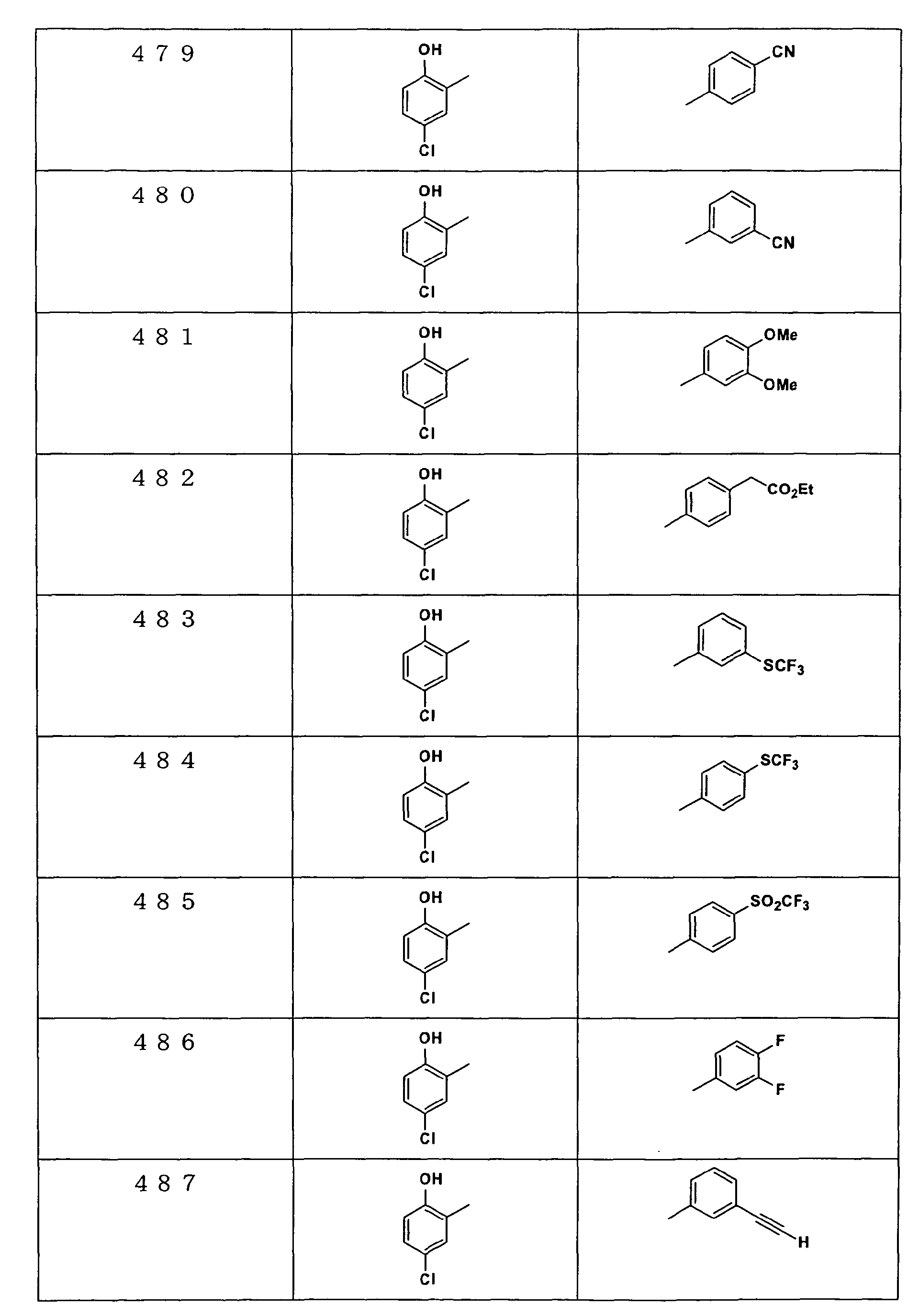

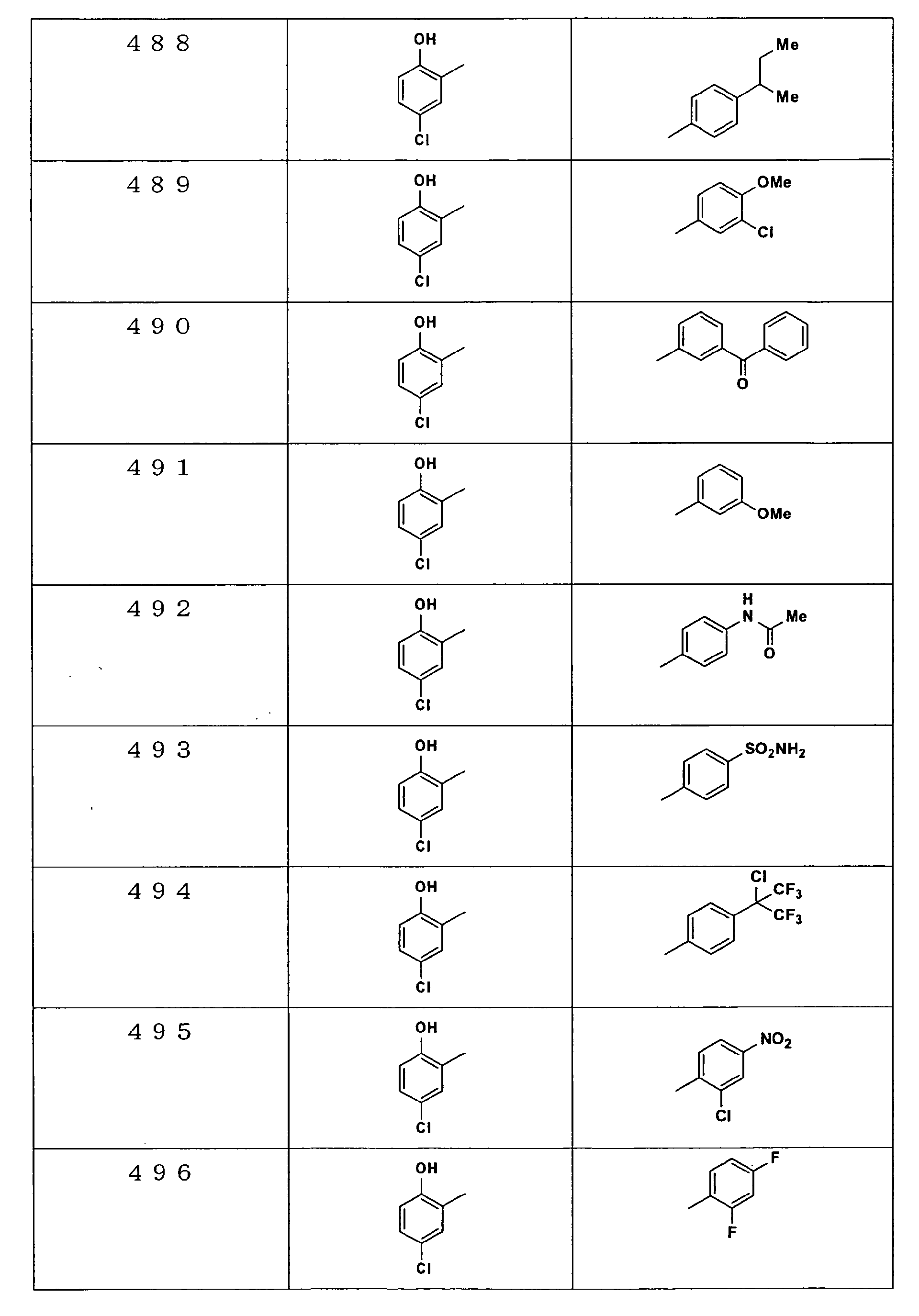

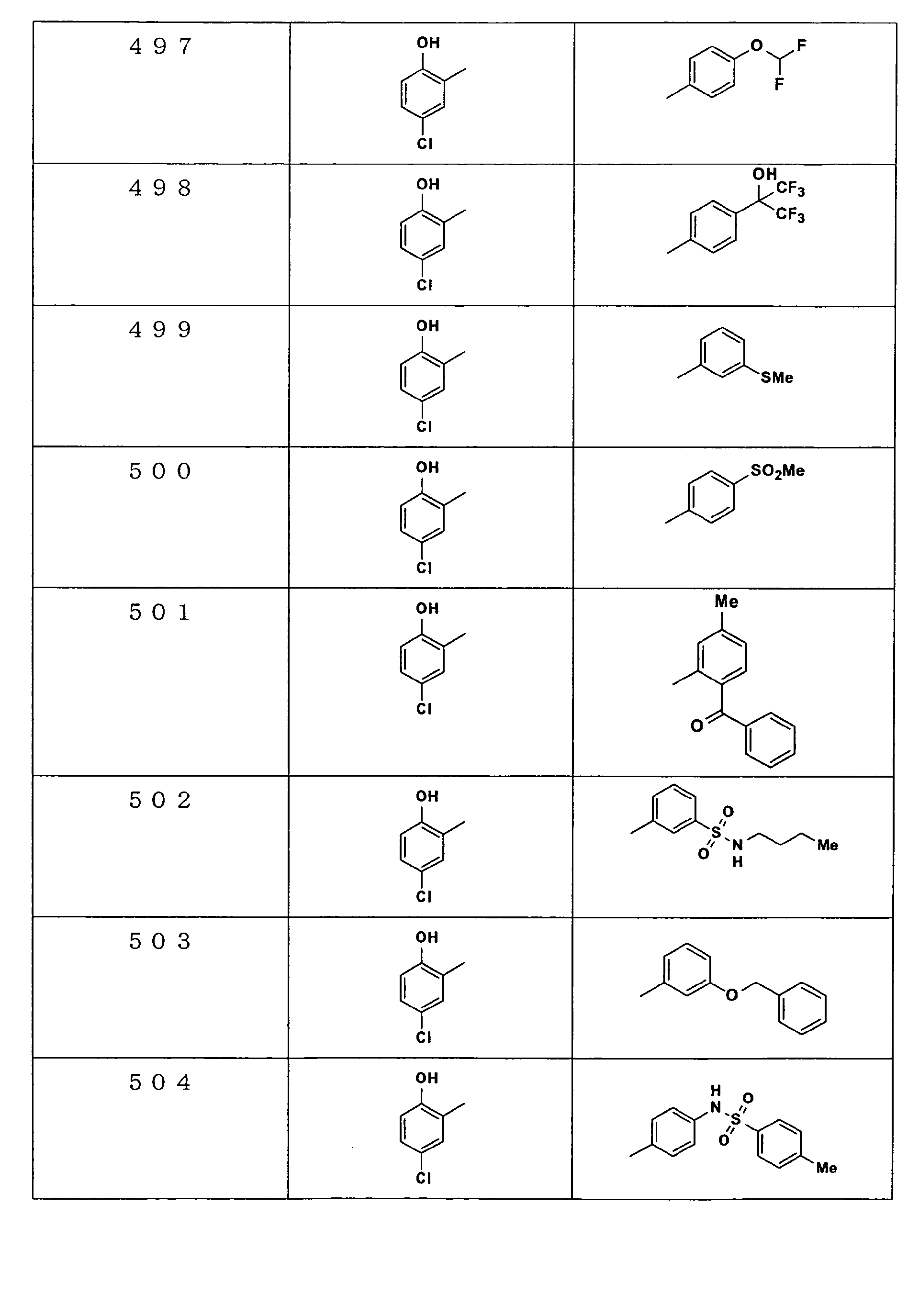

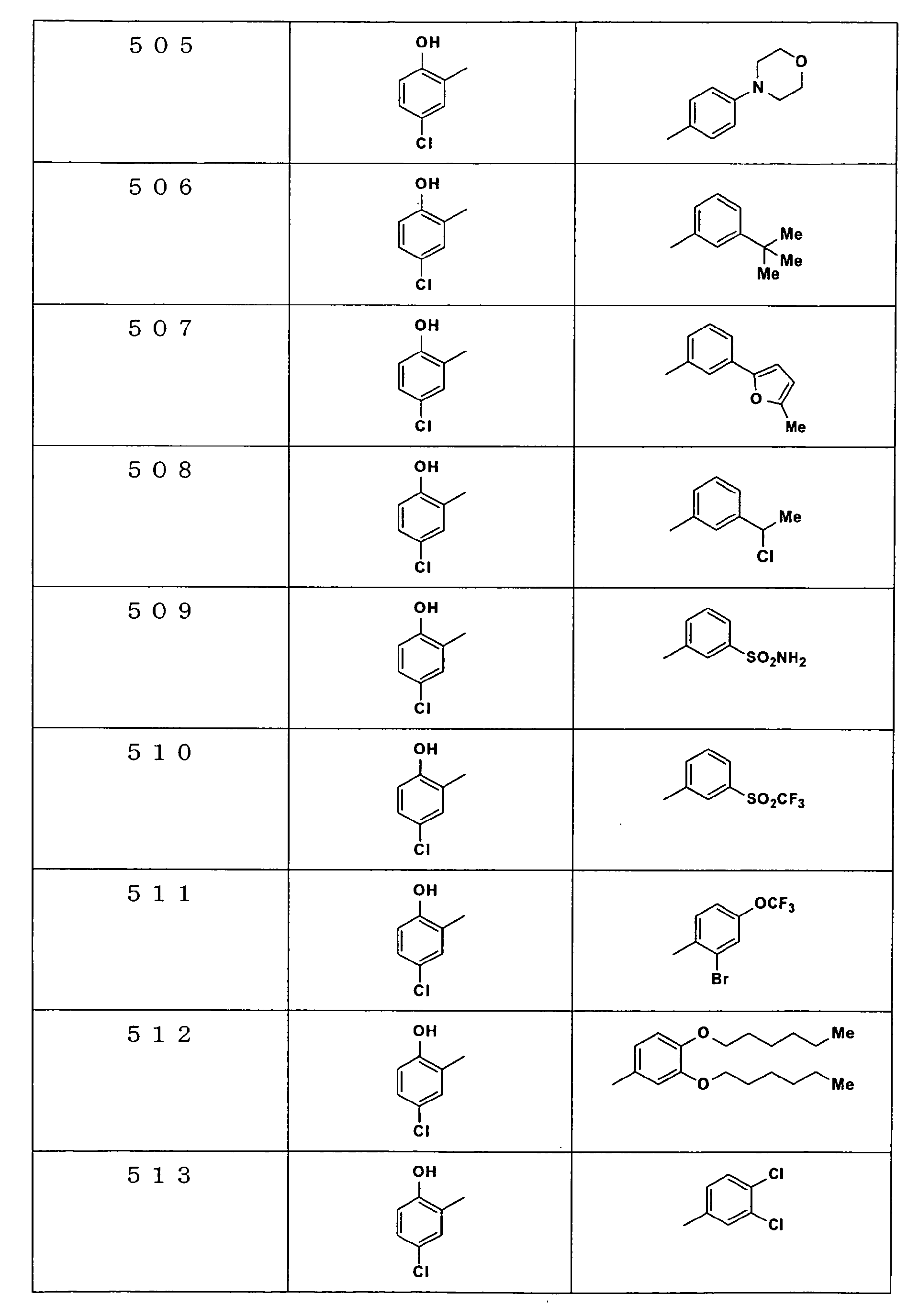

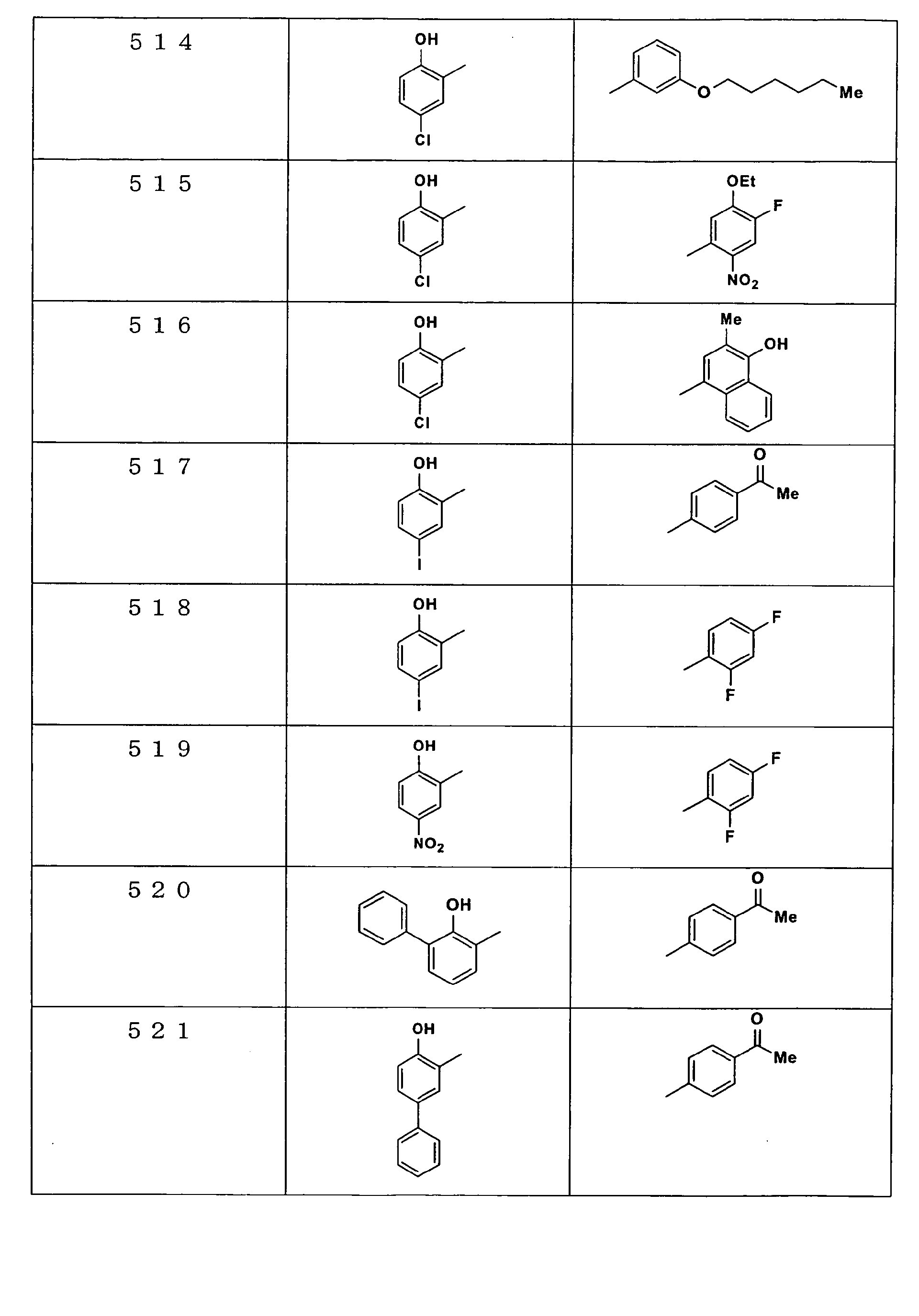

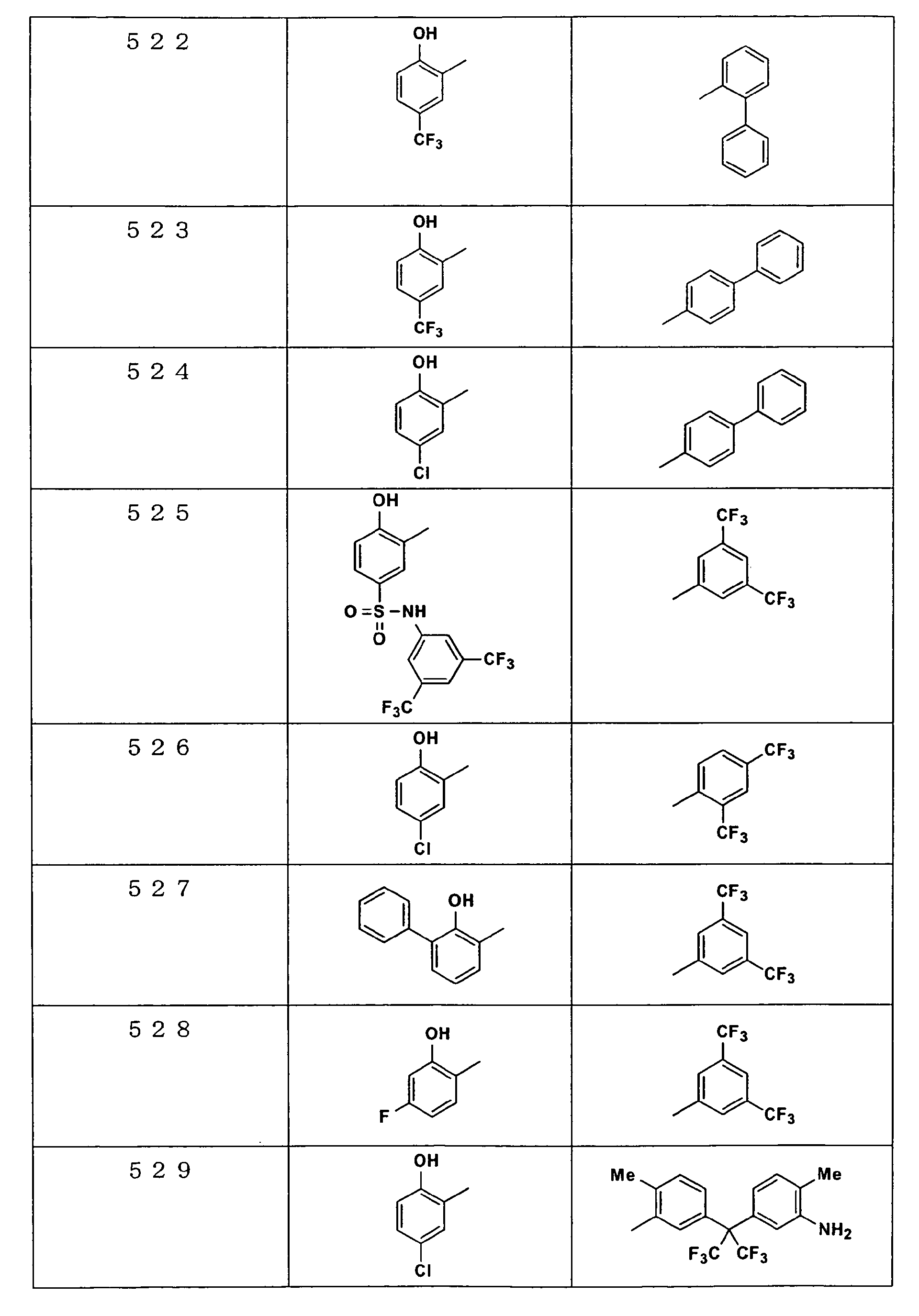

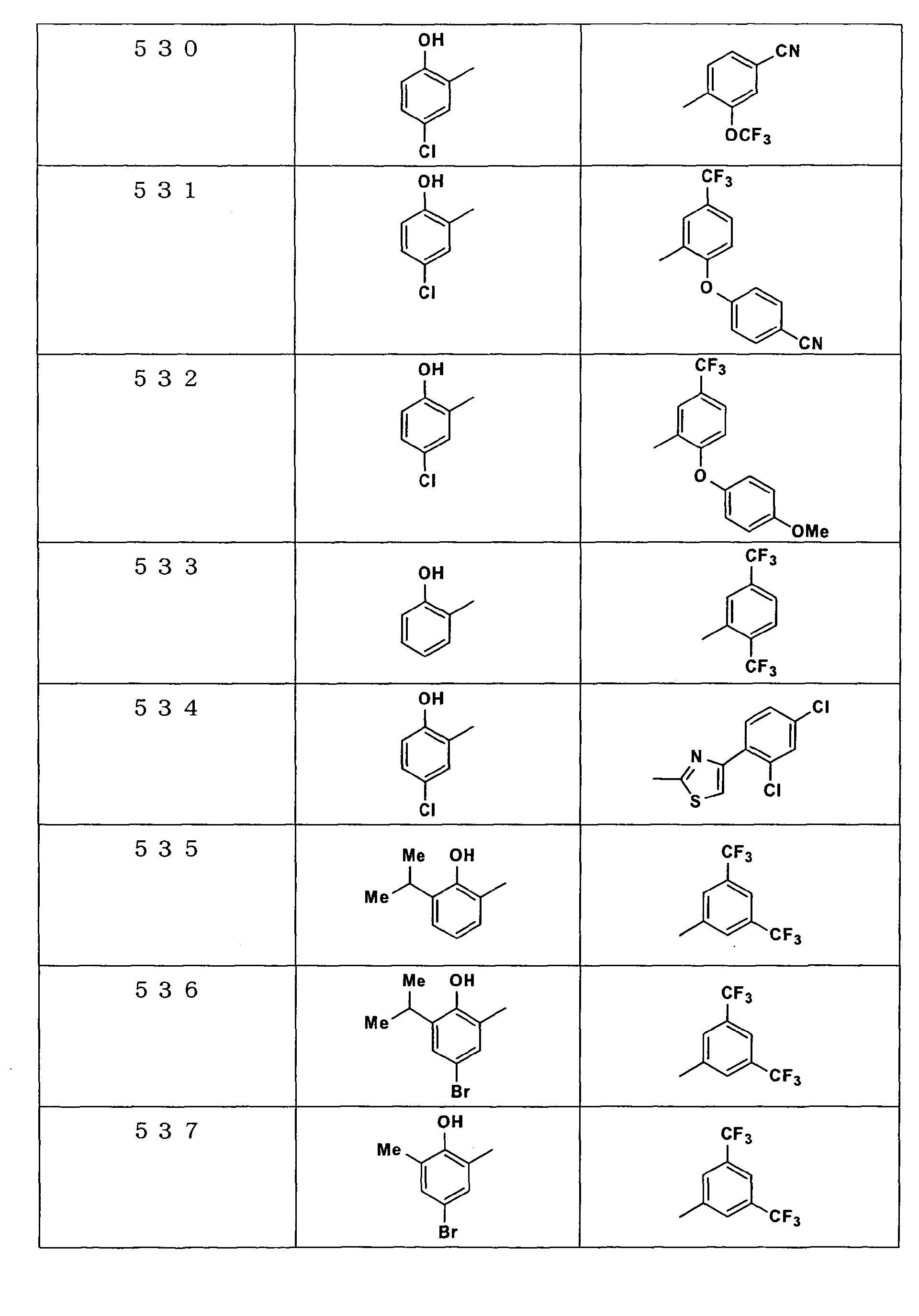

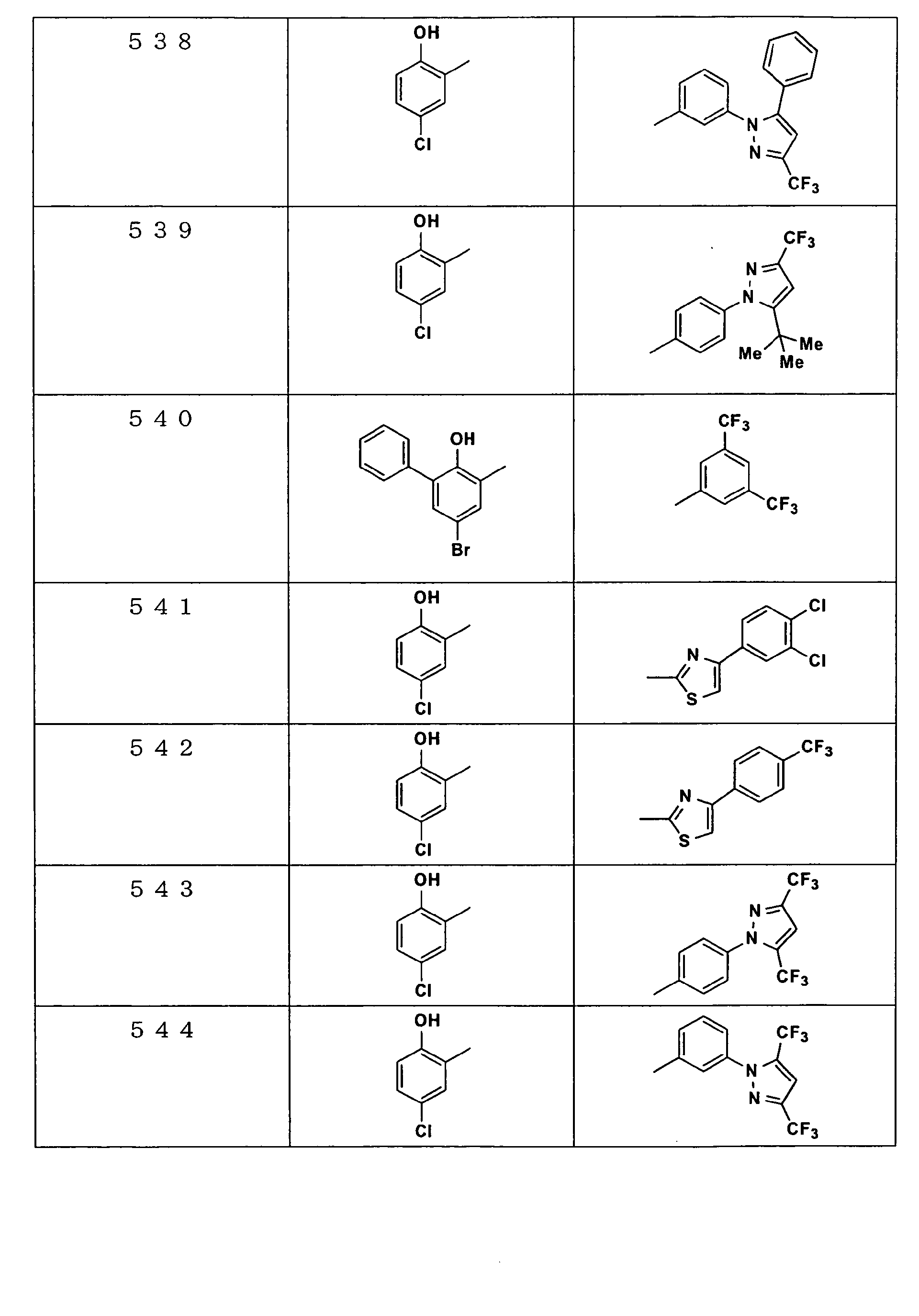

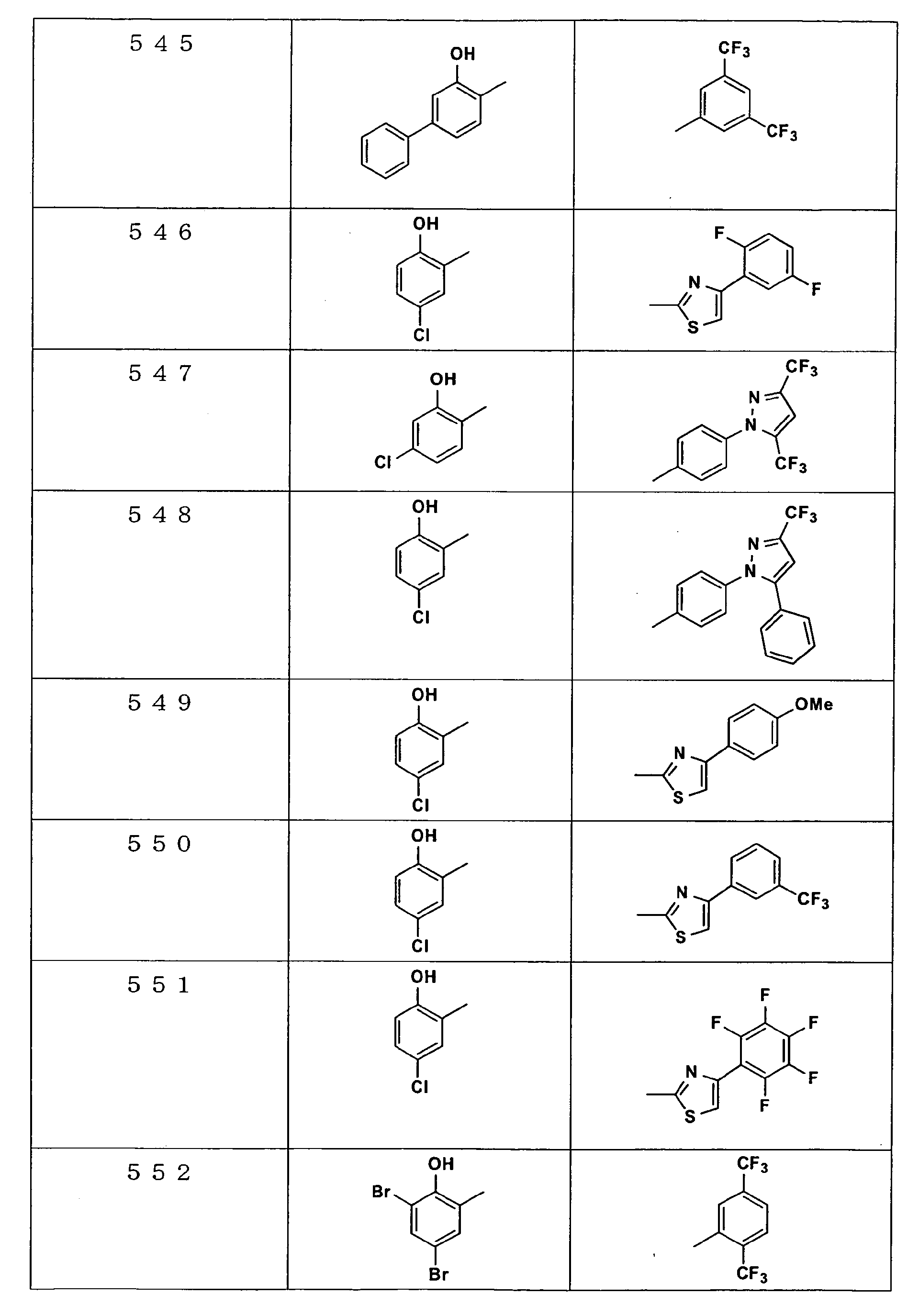

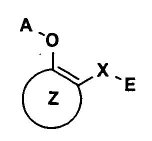

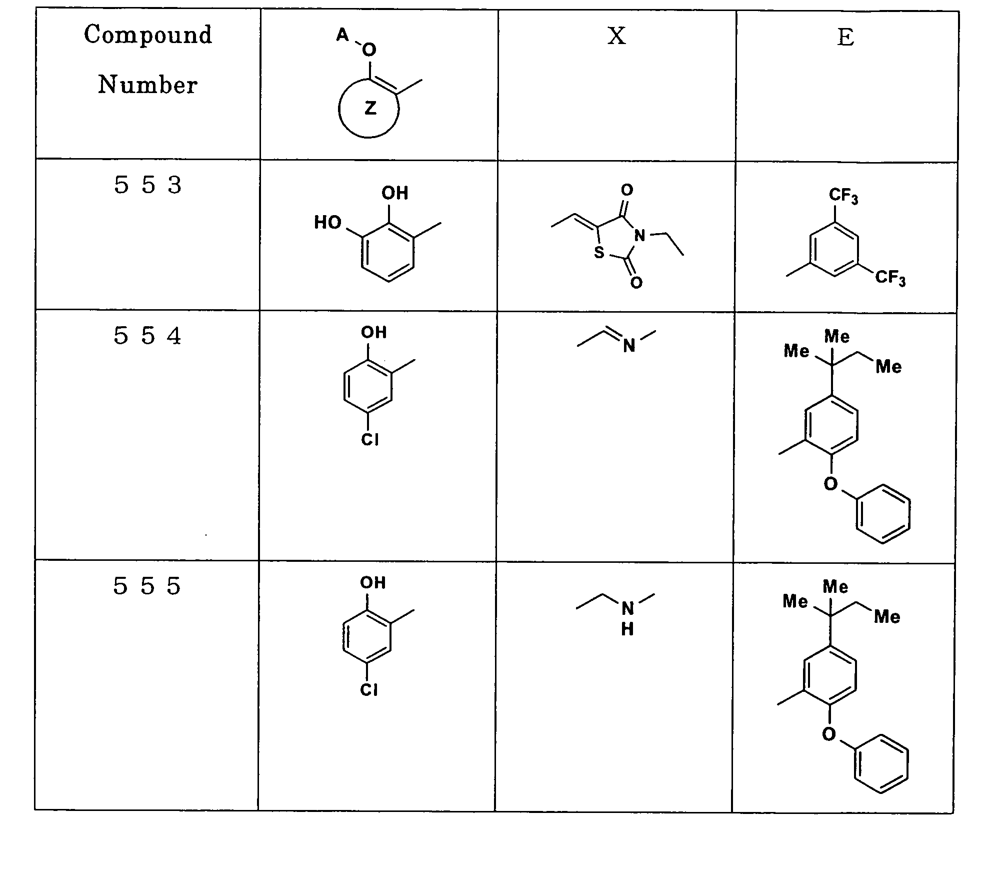

Methods for preparation of the compounds represented by the general formula (I) are not particularly limited. Reference to methods described in the pamphlet of International Publication W002/49632 may be useful.

The compounds represented by the general formula (I) can be prepared, for example, by methods shown bellow.

### < Method 1>

The compounds represented by the general formula (I), wherein X is -CONH- (the hydrogen atom on the nitrogen may be substituted) can be prepared, for example, by a method described in the reaction scheme 1. 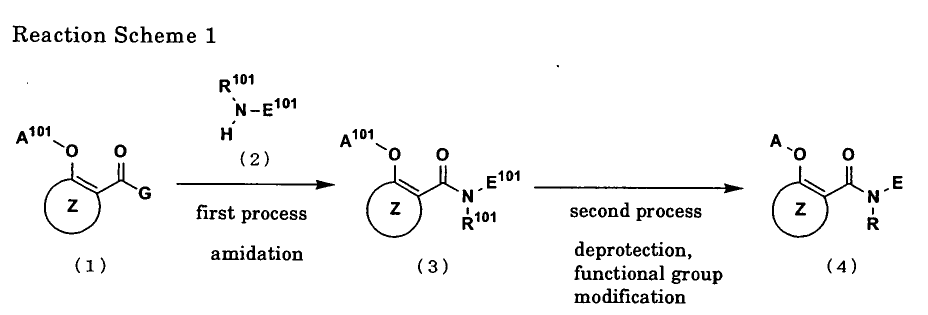 wherein each of A, ring Z, and E has the same meaning as that defined in the general formula (I), A¹⁰¹ represents a hydrogen atom or protecting groups of hydroxy group (preferably, an alkyl group such as methyl group and the like; an aralkyl group such as benzyl group and the like; an acetyl group, an alkoxyalkyl group such as methoxymethyl group and the like; a substituted silyl group such as trimethylsilyl group or the like), each of R and R¹⁰¹ represents a hydrogen atom, a C₁ to C₆ alkyl group or the like, E¹⁰¹ represents E or precursor of E in the definition of the general formula (I), G represents a hydroxy group, halogen atoms (preferably, a chlorine atom), a hydrocarbon-oxy group (preferably, an aryl-oxy group which may be substituted by halogen atom), an acyl-oxy group, an imido-oxy group or the like.

### (First Step)

The amide (3) can be prepared by dehydrocondensation of the carboxylic acid derivative (1) and the amine (2). This reaction is carried out at a reaction temperature of from 0°C to 180°C, without solvent or in an aprotic solvent, in the presence of an acid halogenating agent or a dehydrocondensing agent, and in the presence or absence of a base.

As the halogenating agent, examples include, for example, thionyl chloride, thionyl bromide, sulfuryl chloride, phosphorus oxychloride, phosphorus trichloride, phosphorus pentachloride or the like. When A¹⁰¹ is hydrogen atom, phosphorus trichloride is preferable, and when A¹⁰¹ is acetyl group or the like, phosphorus oxychloride is preferable. As the dehydrocondensing agent, examples include, for example, N,N'-dicyclohexylcarbodiimide, 1-ethyl-3-(3-dimethylaminopropyl)carbodiimide hydrochloride, diphenylphosphorylazide or the like. As the base, examples include inorganic bases such as sodium carbonate, potassium carbonate, sodium hydrogencarbonate or the like, or organic bases such as pyridine, triethylamine, N,N'-diethylaniline or the like. As the aprotic solvent, examples include dichloromethane, dichloroethane, chloroform, tetrahydrofuran, 1,4-dioxane, benzene, toluene, monochlorobenzene, o-dichlorobenzene, N,N'-dimethylformamide, N-methylpyrrolidone or the like, when the reaction is carried out in the presence of the acid halogenating agent, particularly, toluene, monochlorobenzene, o-dichlorobenzene are preferable.

A target compound can also be prepared, for example, by a method or similar method described in Journal of Medicinal Chemistry, (USA), 1998, Vol.41, No.16, p.2939-2945, in which the acid chloride is prepared and isolated beforehand from carboxylic acid, then the result is made to react with an amine having E¹⁰¹.

When G is hydroxy group, the reaction condition described in Archiv der Pharmazie, (Germany), 1998, Vol.331, No.1, p.3-6 can be used as a preferred reaction condition.

Kinds of carboxylic acid derivative (1) and amine (2) are not particularly limited, and new compounds synthesized by referring to well-known preparation method described in the literature or commercially available reagents can be used for the aforementioned reaction.

### (Second Step)

When the amide (3) has a protecting group and/or has a favorable substituent for functional group modification, for example, an amino group and a protected amino group or its precursor; a carboxy group and a protected carboxy group or its precursor; a hydroxy group and a protected hydroxy group or its precursor, the final target compound (4) can be prepared by a reaction for deprotection and/or functional group modification in this step. Various well-known methods can be used for the reaction. For the reaction of deprotection and functional group modification, for example, methods described in "Protective Groups in Organic Syntheses", (USA), Theodra W. Green, Peter G.M. Wuts, Eds., Third edition, Apr. in 1999, John Wiley & Sons, and "Handbook of Reagents for Organic Synthesis", (USA), 4 Volumes, Jun. in 1999, John Wiley & Sons can be used, and for the reaction of functional group modification, for example, methods described in "Palladium Reagents in Organic Syntheses", (USA), Richard F. Heck, 1985, Academic Press, and "Palladium Reagents and Catalysts: Innovations in Organic Synthesis", (USA), J. Tsuji, 1999, John Wiley & Sons, or the like can be used.

The aforementioned methods are applicable by appropriately combining raw materials even for the compounds wherein X is other connecting group, for example, -SO₂NH-, -NHCO-, -NHSO₂-, -CONHCH₂-, -CONHCH₂CH₂-, -CONHCH₂CONH-, -CONHNHCO-, -CONHNH CH₂-, -COO-, -CONHNH-; wherein the hydrogen atom on said connecting group may be substituted.

In the general formula (I), when X is the formula: -CONHCH₂- wherein the hydrogen atom on said connecting group may be substituted, the target compound can be prepared by using an amine represented by the formula: H₂N-CH₂-E¹⁰¹, wherein E¹⁰¹ has the same meaning as that defined above, instead of the amine (2).

In the general formula (I), when X is the formula: -CONHCH₂CH₂- wherein the hydrogen atom on said connecting group may be substituted, the target compound can be prepared by using an amine represented by the formula: H₂N-CH₂ CH₂-E¹⁰¹, wherein E¹⁰¹ has the same meaning as that defined above, instead of the amine (2).

In the general formula (I), when X is the formula: -SO₂NH-, the target compound can be prepared by using a sulfonyl chloride represented by the formula: A¹⁰¹-O-(ring Z)-SO₂Cl, wherein each of A¹⁰¹ and ring Z has the same meaning as that defined above, instead of the carboxylic acid derivative (1).

In the general formula (I), when X is the formula: -NHCO-, the target compound can be prepared by using an amine represented by the formula: A¹⁰¹-O-(ring Z)-NH₂, wherein each of A¹⁰¹ and ring Z has the same meaning as that defined above, and a carboxylic acid represented by the formula: E¹⁰¹-COOH, wherein -E¹⁰¹ has the same meaning as that defined above, or a carboxylic acid chloride represented by the formula: E^{101_}COCl, wherein -E¹⁰¹ has the same meaning as that defined above.

In the general formula (I), when X is the formula: -NHSO₂-, wherein said connecting group may be substituted, the target compound can be prepared by using an amine represented by the formula: HO-(ring Z)-NH₂, wherein ring Z has the same meaning as that defined above, and a sulfonyl chloride represented by the formula: E¹⁰¹-SO₂Cl, wherein E¹⁰¹ has the same meaning as that defined above.

In the general formula (I), when X is the formula: ^{_}CONHNHCO-, the target compound can be prepared by using a hydrazide represented by the formula: HO-(ring Z)-CONHNH₂, wherein ring Z has the same meaning as that defined above, and a carboxylic acid chloride represented by the formula: E¹⁰¹-COCl, wherein -E¹⁰¹ has the same meaning as that defined above.

In the general formula (I), when X is the formula: -COO-, the target compound can be prepared by using a phenol derivative represented by the formula: HO-E¹⁰¹, wherein -E¹⁰¹ has the same meaning as that defined above, instead of the amine (2).

In the general formula (I), when X is the formula: -CONHNH-, the target compound can be prepared by using a hydrazine represented by the formula: H₂N-NH -E¹⁰¹, wherein E¹⁰¹ has the same meaning as that defined above, instead of the amine (2).

In the general formula (I), when X is the formula: -CONHCH₂CONH-, the target compound can be prepared by using an amine represented by the formula: H₂N -CH₂CONH-E¹⁰¹, wherein E¹⁰¹ has the same meaning as that defined above, instead of the amine (2).

The amine represented by the formula: H₂N-CH₂CONH-E¹⁰¹, can be prepared, for example, by condensation of the amine (2) and a N-protected amino acid (for example, N-(tert-butoxycarbonyl)glycine), according to the aforementioned method 1, followed by a deprotection reaction.

In the general formula (I), when X is the following formula: 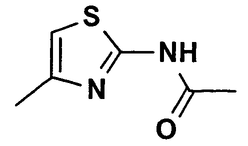 wherein said connecting group may be substituted, the target compound can be prepared by using an amine represented by the following formula: 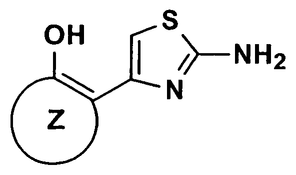 wherein ring Z has the same meaning as that defined above, and a carboxylic acid represented by the formula: E¹⁰¹-COOH, wherein E¹⁰¹ has the same meaning as that defined above, or a carboxylic acid chloride represented by the formula: E¹⁰¹-COCl, wherein E¹⁰¹ has the same meaning as that defined above.

The amine represented by the following formula: 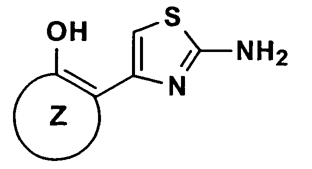 can be prepared, for example, by a method described in the reaction scheme 1-2. 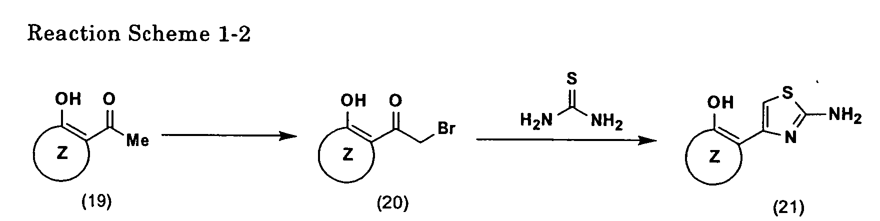 wherein ring Z has the same meaning as that defined above.

The bromoacetophenone (20) can be prepared by bromination of the acetophenone (19).

This reaction is carried out at a reaction temperature of from 0°C to 100°C in a solvent, in the presence of a brominating agent.

As the brominating agent, for example, phenyltrimethylammonium tribromide can preferably be used.

As the reaction solvent, any solvent can be used as long as it does not inhibit the reaction, for example, ethers such as tetrahydrofuran can be used.

The amine (21) can be prepared by reacting the bromoacetophenone (20) with thiourea.

This reaction is carried out at a reaction temperature of from 0°C to 120°C in a solvent.

As the reaction solvent, any solvent can be used as long as it does not inhibit the reaction, for example, alcohols such as ethanol can be used.

### < Method 2 >

The compounds represented by the general formula (I), wherein X is -CH₂NH- can be prepared, for example, by a method described in the reaction scheme 2. 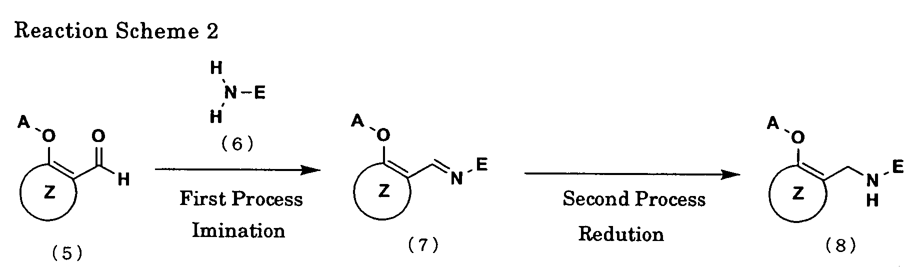 wherein each of A, ring Z, and E has the same meaning as that defined in the general formula (I).

The imine derivative of the formula (7) can be prepared by dehydrocondensation of the aldehyde (5) and the amine (6). This reaction is carried out at a reaction temperature of from 0°C to 100°C in a solvent, in the presence or absence of a dehydrating agent. As the dehydrating agent, examples include anhydrous magnesium sulfate, molecular sieves or the like. As the solvent, examples include inert solvent, and tetrahydrofuran, 1,4-dioxane, methanol, ethanol or the like are preferable.

The aforementioned methods are applicable by appropriately combining raw materials even for the compounds wherein X is other connecting group, for example, -CONHN=CH-, -CH=NNHCO-, -CHNNH-; wherein the hydrogen atom on said connecting group may be substituted.

In the general formula (I), when X is the formula: -CONHN=CH-, the target compound can be prepared by using a hydrazide represented by the formula: HO-(ring Z)-CONHNH₂, wherein ring Z has the same meaning as that defined above, and an aldehyde represented by the formula: E-CHO, wherein E has the same meaning as that defined above.

In the general formula (I), when X is the formula: -CH=NNHCO-, the target compound can be prepared by using an aldehyde represented by the formula: HO-(ring Z)-CHO, wherein ring Z has the same meaning as that defined above, and a hydrazide represented by the formula: E-CONHNH₂, wherein E has the same meaning as that defined above.

In the general formula (I), when X is the formula: -CH=NNH-, the target compound can be prepared by using an aldehyde represented by the formula: HO-(ring Z)-CHO, wherein ring Z has the same meaning as that defined above, and a hydrazine represented by the formula: E-NHNH₂, wherein E has the same meaning as that defined above.

The target compound (8) can be prepared by reduction of the imine derivative (7). This reaction is carried out at a reaction temperature of from 0°C to 100°C in a solvent, in the presence of a reducing agent. As the reducing agent, examples include sodium borohydride, lithium borohydride or the like. As the solvent, examples include inert solvent, and tetrahydrofuran, 1,4-dioxane, methanol, ethanol or the like are preferable. This reaction can also be carried out by a method of catalytic hydrogenation. As the catalyst, examples include palladium carbon, platinum carbon, palladium hydroxide, palladium black or the like. As solvent, examples include inert solvent, and tetrahydrofuran, 1,4-dioxane, methanol, ethanol or the like are preferable. The reaction is carried out at a reaction temperature of from 0°C to 200°C, and the hydrogen pressure may be an ordinary pressure or a positive pressure.

### <Method 3>

The compounds represented by the general formula (I), wherein X is -CH=CH- (the hydrogen atom on said connecting group may be substituted), can be prepared, for example, by methods described in the reaction scheme 3-1 or the reaction scheme 3-2. 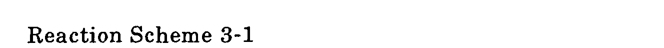 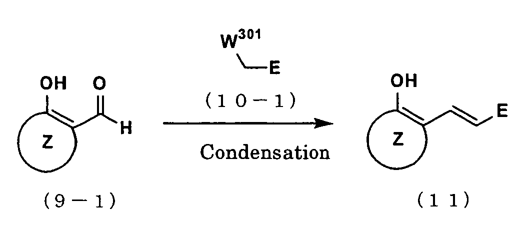 wherein each of ring Z and E has the same meaning as that defined in the general formula (I), W³⁰¹ represents O,O'-di-hydrocarbon-phosphono group or triarylphosphonium group

The target compound (11) can be prepared by dehydrocondensation of the aldehyde (9-1) and the phosphorus compound (10-1). This reaction is carried out in a solvent at a reaction temperature of from 0°C to the boiling point of the solvent, in the presence of a base. As the base, examples include inorganic base such as sodium carbonate, potassium carbonate, sodium hydrogencarbonate or the like, or organic base such as pyridine, triethylamine, N,N-diethylaniline or the like. As the solvent, examples include inert solvent, and tetrahydrofuran, 1,4-dioxane, methanol, ethanol, water or the like are preferable. 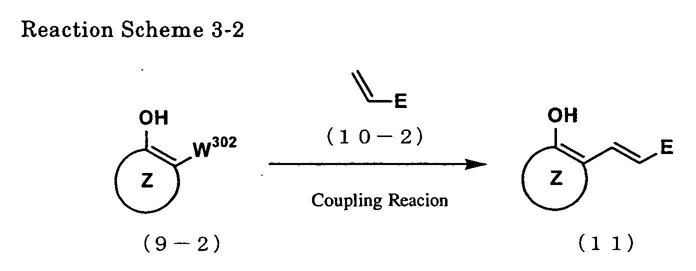 wherein each of ring Z and E has the same meaning as that defined in the general formula (I), W³⁰² represents halogen atoms (preferably, iodine atom and bromine atom), (trifluoromethanesulfonyl)oxy group and the like.

The target compound (11) can be prepared by reacting the halogenated compound (9-2) with the styrene compound (10-2) in the presence of a transition-metal complex catalyst. This reaction is carried out in a solvent at a reaction temperature of from 0°C to the boiling point of the solvent, in the presence or absence of a ligand and/or a base. As the transition-metal complex catalyst, examples include palladium catalyst such as palladium acetate and dichlorobis(triphenylphosphine)palladium. As the ligand, examples include phosphine ligand such as triphenylphosphine. As the base, examples include inorganic base such as sodium carbonate, potassium carbonate, and sodium hydrogen carbonate, or organic base such as pyridine, triethylamine, and N,N-diethylaniline. As the solvent, examples include inert solvents, and N,N-dimethylformamide, tetrahydrofuran, 1,4-dioxane or the like are preferable.

### < Method 4 >

The compounds represented by the general formula (I), wherein X is -COCH=CH- and -COCH₂CH₂- (the hydrogen atom on said connecting group may be substituted), can be prepared, for example, by a method described in the reaction scheme 4. 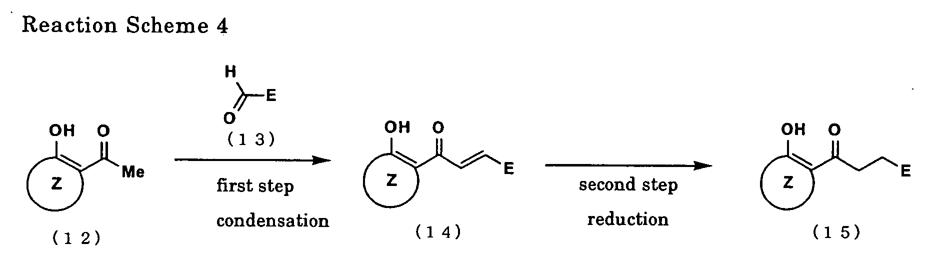 wherein each of rings Z and E has the same meaning as that defined in the general formula (I).

The target compound enone (14) can be prepared by dehydrocondensation of the ketone (12) and the aldehyde (13). This reaction is carried out in a solvent at a reaction temperature of from 0°C to the boiling point of the solvent, in the presence of a base. As the base, examples include inorganic base such as sodium hydroxide, potassium hydroxide, sodium carbonate, potassium carbonate, sodium hydrogencarbonate or the like, or organic base such as pyridine, triethylamine, N,N-diethylaniline or the like. Examples include inert solvent, and tetrahydrofuran, 1,4-dioxane, methanol, ethanol, water or the like are preferable.

Next, the target compound (15) can be prepared by reduction of the enone (14). This reaction is carried out at a reaction temperature of from 0°C to 100°C in solvent, in the presence of a reducing agent. As the reducing agent, examples include sodium borohydride, lithium borohydride or the like. As the solvent, examples include inert solvent, and tetrahydrofuran, 1,4-dioxane, methanol, ethanol or the like are preferable. Moreover, this reaction is carried out by a method of catalytic hydrogenation also. As the catalyst, examples include palladium carbon, platinum carbon, palladium hydroxide, palladium black or the like. As solvent, examples include inert solvent, and tetrahydrofuran, 1,4-dioxane, methanol, ethanol or the like are preferable. The reaction is carried out at a reaction temperature of from 0°C to 200°C, and the hydrogen pressure is at normal pressure or applied pressure.

### < Method 5 >

The compounds represented by the general formula (I), wherein X is -NHCONH- (the hydrogen atom on said connecting group may be substituted), can be prepared, for example, by a method described in the reaction scheme 5. 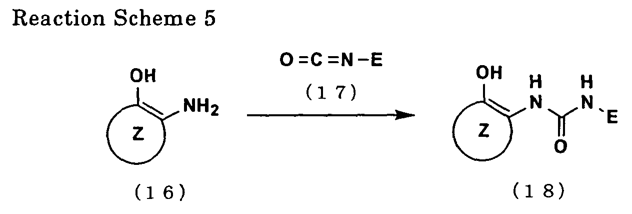 wherein each of ring Z and E has the same meaning as that defined in the general formula (I).

First, the target compound urea (18) can be prepared by reacting the amine (16) with the isocyanate (17). This reaction is carried out in a solvent at a reaction temperature of from 0°C to the boiling point of the solvent, in the presence or absence of a base. As the base, examples include inorganic base such as sodium hydroxide, potassium hydroxide, sodium carbonate, potassium carbonate, sodium hydrogencarbonate or the like, or organic base such as pyridine, triethylamine, N,N-diethylaniline or the like. Examples include inert solvent, and tetrahydrofuran, 1,4-dioxane, methanol, ethanol, water or the like are preferable.

### <Method 6>

The compounds represented by the general formula (I), wherein X is the formula: -CONHNHCH₂- (the hydrogen atom on said connecting group may be substituted), can be prepared, for example, by a method described in the reaction scheme 6. 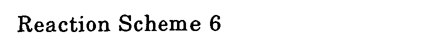 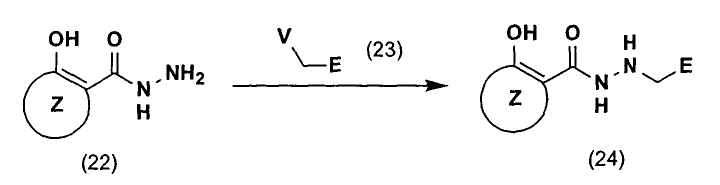 wherein each of ring Z and E has the same meaning as that defined above, and V represents a leaving group such as halogen atom.

The target compound hydrazide (24) can be prepared by reacting the hydrazide (22) with the benzyl derivative (23).

This reaction is carried out at a reaction temperature of from 0°C to 180°C in a solvent, in the presence or absence of a base.

As the base, for example, organic base such as pyridine, triethylamine or the like can preferably be used.

As the reaction solvent, any solvent can be used as long as it does not inhibit the reaction, for example, halogenated solvent such as dichloromethane; ethers such as tetrahydrofuran; and hydrocarbon solvent such as toluene can be used.

### < Method 7 >

The compounds represented by the general formula (I), wherein X is the formula: 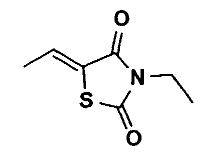 can be prepared, for example, by a method described in the reaction scheme 7. 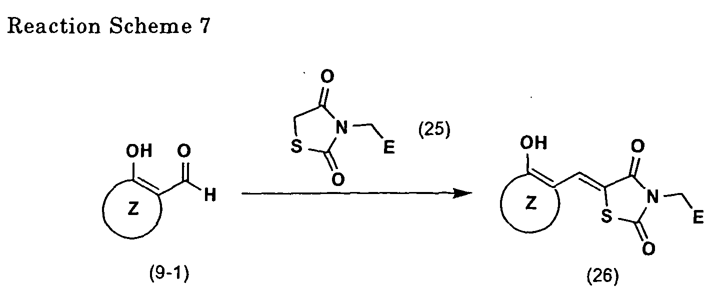 wherein each of ring Z and E has the same meaning as that defined above.

The target compound 5-(benzylidene)-3-benzylthiazolidin-2,4-dione derivative (26) can be prepared by reacting the aldehyde (9-1) with the 3-benzylthiazolidin-2,4-dione derivative (25).

This reaction is carried out at a reaction temperature of from 0°C to 180°C in a solvent, in the presence of a catalyst. As the catalyst, for example, a mixture of piperidine/acetic acid can preferably be used. As the reaction solvent, any solvent can be used as long as it does not inhibit the reaction, for example, hydrocarbon solvent such as toluene can be used.

The 3-benzylthiazolidine-2,4-dione derivative represented by the following formula: 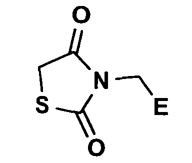 wherein E has the same meaning as that defined above, can be prepared, for example, by a method described in the reaction scheme 7-1. 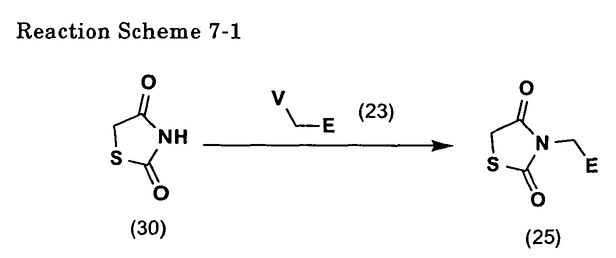 wherein each of E and V has the same meaning as that defined above.

The target compound 3-benzylthiazolidine-2,4-dione derivative (28) can be prepared by reacting thiazolidine-2,4-dione (30) with the benzyl derivative (23).

This reaction is carried out at a reaction temperature of from 0°C to 180°C in a solvent, in the presence of a base. As the base, for example, inorganic base such as sodium hydroxide, potassium carbonate or the like, or organic base such as pyridine, triethylamine or the like can preferably be used.

As the reaction solvent, any solvent can be used as long as it does not inhibit the reaction, for example, water; alcohols such as ethanol or the like; halogenated solvent such as dichloromethane or the like; ethers such as tetrahydrofuran or the like; or amides such as N,N-dimethylformamide or the like can be used.

The compounds represented by the general formula (I) prepared by the aforementioned methods can be isolated and purified by methods widely known by those skilled in the art, for example, extraction, precipitation, fractional chromatography, fractional crystallization, suspension and washing, and recrystallization. Furthermore, each of the pharmaceutically acceptable salt of the compound of the present invention, the hydrate thereof and the solvate thereof can be prepared by methods widely known by those skilled in the art.

In the examples of the specification, preparation methods of typical compounds included in the general formula (I) are explained in details. Therefore, those skilled in the art can prepare any compound fall within the general formula (I) by referring to the explanations of the aforementioned general preparation methods and those of specific preparation methods of the examples, by choosing appropriate reaction raw materials, reaction reagents, and reaction conditions, and by adding appropriate modification and alteration of these methods, if necessary.

The substances selected from the group consisting of a compound represented by the general formula (I) and a pharmacologically acceptable salt thereof, and a hydrate thereof and a solvate thereof have inhibitory activity against IKK-*β* or MEKK-1, and they are useful as an active ingredient of a medicament having inhibitory activity against IKK-*β* or MEKK-1. Furthermore, since the aforementioned substances have inhibitory activity against kinases structurally similar to IKK-β or MEKK-1, they are also useful as an active ingredient of a medicament having inhibitory activity against kinases structurally similar to IKK-β or MEKK-1. When IKK-β or MEKK-1 is herein referred to, those included are naturally-derived IKK-β or MEKK-1, as well as proteins that are amino acid-mutant generated by a technique such as gene recombination and have substantially the same biological functions as those of naturally-derived IKK-β or MEKK-1. Moreover, examples of the kinases structurally similar to IKK-β or MEKK-1 include kinases which have similar ligand binding sites to those of IKK-β or MEKK-1.

The medicament of the present invention can induce the inhibition of the activation of NF-κB and the inhibition of the production and release of inflammatory cytokines by inhibiting IKK-β and/or MEKK-1 or kinases structurally similar thereto. Furthermore, the medicament of the present invention induces the inhibition of an expression of genes of one or more substances selected from a group consisting of tumor necrosis factor (TNF), interleukin-1, interleukin-2, interleukin-6, interleukin-8, granulocyte colony-stimulating factor, interferon β, cell adhesion factor ICAM-1, VCAM-1, and ELAM-1, nitricoxide synthetase, major histocompatibility antigen family class I, major histocompatibility antigen family class II, β 2-microglobulin, immunoglobulin light chain, serum amyloid A, angiotensinogen, complement B, complement C4, c-myc, transcript derived from HIV gene, transcript derived from HTLV-1 gene, transcript derived from simian virus 40 gene, transcript derived from cytomegalovirus gene, and transcript derived from adenovirus gene by inhibiting IKK-β and/or MEKK-1 or kinases structurally similar thereto. Therefore, the medicament of the present invention can be used for the purpose of preventive and/or therapeutic treatment of diseases caused by NF-κB activation and inflammatory cytokine overproduction as a medicament for an inhibition of IKK-β and/or MEKK-1 or kinases structurally similar thereto.

The medicament of the present invention is useful for the preventive and/or therapeutic treatment of the following diseases wherein NF-κB activation and/or inflammatory cytokine are believed to be involved, for example, autoimmune diseases such as chronic rheumatism, osteoarthritis, systematic lupus erythematosus, systematic scleroderma, polymyositis, Sjoegren's syndrome, vasculitis syndrome, antiphospholipid syndrome, Still's disease, Behcet's disease, periarteritis nodosa, ulcerative colitis, Crohn's disease, active chronic hepatitis, glomerulonephritis, and chronic nephritis, chronic pancreatitis, gout, atherosclerosis, multiple sclerosis, arteriosclerosis, endothelial hypertrophy, psoriasis, psoriatic arthritis, contact dermatitis, atopic dermatitis, pruritus, allergic disease such as pollinosis, asthma, bronchitis, interstitial pneumonia, lung disease involving granuloma, chronic obstructive lung disease, chronic pulmonary thromboembolism, inflammatory colitis, insulin resistance, obesity, diabetes and its complications (nephropathy, retinopathy, neurosis, hyperinsulinemia, arteriosclerosis, hypertention, peripheral vessel obstruction, etc.) diseases involving abnormal vascular proliferation such as hyperlipemia, retinopathy, and pneumonia, Alzheimer's disease, encephalomyelitis, epilepsy, acute hepatitis, chronic hepatitis, drug induced toxic hepatopathy, alcoholic hepatitis, viral hepatitis, icterus , cirrhosis, hepatic insufficiency, atrial myxoma, Caslemann's syndrome, mesangial nephritis, kidney cancer, lung cancer, liver cancer, breast cancer, uterine cancer, pancreatic cancer, other solid cancer, sarcoma, osteosarcoma, metastatic invasion of cancer, canceration of inflammatory focus, cancerous cachexia, metastasis of cancer, leukemia such as acute myeloblastic leukemia, multiple myeloma, Lennert's lymphoma, malignant lymphoma, development of carcinostatic resistance of cancer, canceration of foci such as viral hepatitis and cirrhosis, canceration from polyp of colon, brain tumor, nervous tumor, sarcoidosis, endotoxic shock, sepsis, cytomegaloviral pneumonia, cytomegaloviral retinopathy, adenoviral cold, adenoviral pool fever, adenoviral ophthalmia, conjunctivitis, AIDS, uveitis, periodontal disease, diseases or complications provoked by infections of other bacteria, viruses, and mycetes, complications after surgery such as generalized inflammatory symptoms, restenosis after percutaneous tubal coronary artery plastic surgery, reperfusion disorders after vascular occulusion opening such as ischemia reperfusion disorders, organ transplantation rejection and reperfusion disorders of heart, liver, kidney and the like, pruritus, alopecia, anorexia, malaise, chronic fatigue syndrome and the like. Furthermore, inflammatory cytokine and NF-κB are involved in differentiation and activation of osteoclast, and consequently, the medicament of the present invention is also useful for preventive and/or therapeutic treatment of metabolic bone diseases or the like such as osteoporosis and osteocarcinomic pain or the like. The medicament may also be used for prevention of deterioration of an organ during organ conservation before transplantation.

As the active ingredient of the medicament on the present invention, one or more kinds of substances selected from the group consisting of the compound represented by the general formula (I) and a pharmacologically acceptable salt thereof, and a hydrate thereof and a solvate thereof may be used. The aforementioned substance, per se, may be administered as the medicament of the present invention, however, preferably, the medicament of the present invention is provided in the form of a pharmaceutical composition comprising the aforementioned substance which is an active ingredient together with one or more pharmacologically acceptable pharmaceutical additives. In the aforementioned pharmaceutical compositions, a ratio of the active ingredient to the pharmaceutical additives is 1 weight % to 90 weight %.

The pharmaceutical compositions of the present invention may be administered as pharmaceutical compositions for oral administration, for example, granules, subtilized granules, powders, hard capsules, soft capsules, syrup, emulsion, suspension, or solution, or may be administered as pharmaceutical compositions for parenteral administration, for example, injections for intravenous administration, intramuscular administration, or subcutaneous administration, drip infusions, suppositories, percutaneous absorbent, transmucosal absorption preparations, nasal drops, ear drops, instillation, and inhalants. Preparations made as pharmaceutical compositions in a form of powder may be dissolved when necessary and used as injections or drip infusions.

For preparation of pharmaceutical compositions, solid or liquid pharmaceutical additives may be used. Pharmaceutical additives may either be organic or inorganic. When an oral solid preparation is prepared, an excipient is added to the active ingredient, and further binders, disintegrator, lubricant, colorant, corrigent are added, if necessary, to manufacture preparations in the forms of tablets, coating tablets, granules, powders, capsules and the like by ordinary procedures. Examples of the excipient include lactose, sucrose, saccharose, glucose, corn starch, starch, talc, sorbit, crystal cellulose, dextrin, kaolin, calcium carbonate, and silicon dioxide. Examples of the binder include, for example, polyvinyl alcohol, polyvinyl ether, ethyl cellulose, methyl cellulose, gum Arabic, tragacanth, gelatine, shellac, hydroxypropyl cellulose, hydroxypropyl methyl cellulose, calcium citrate, dextrin, and pectin. Examples of the lubricant include, for example, magnesium stearate, talc, polyethylene glycol, silica, and hydrogenated vegetable oil. As the coloring agent, any material can be used which are approved to be added to ordinary pharmaceuticals. As the corrigent, cocoa powder, menthol, aromatic acid, peppermint oil, d-borneol, cinnamon powder and the like can be used. These tables and granules may be applied with sugarcoating, gelatin coating, or an appropriate coating, if necessary. Preservatives, antioxidant and the like may be added, if required.

For liquid preparations for oral administration such as emulsions, syrups, suspensions, and solutions, ordinary used inactive diluents, for example, water or vegetable oil may be used. For these preparations, besides inactive diluents, adjuvants such as wetting agents, suspending aids, sweating agents, flavoring agents, coloring agents or preservatives may be blended. After a liquid preparation is manufactured, the preparation may be filled in capsules made of a absorbable substance such as gelatin. Examples of solvents or suspending agents used for the preparations of parenteral administration such as injections or suppositories include, for example, water, propylene glycol, polyethylene glycol, benzyl alcohol, ethyl oleate, and lecithin. Examples of base materials used for preparation of suppositories include, for example, cacao butter, emulsified cacao butter, lauric fat, and witepsol. Methods for preparation of the aforementioned preparations are not limited, and any method ordinarily used in the art may be used.

When the composition are prepared in the form of injections, carriers such as, for example, diluents including water, ethanol, macrogol, propylene glycol, citric acid, acetic acid, phosphoric acid, lactic acid, sodium lactate, sulfuric acid and sodium hydroxide, pH modifiers and buffer solutions including sodium citrate, sodium acetate and sodium phosphate, stabilizers such as sodium pyrosulfite, ethylenediaminetetraacetic acid, thioglycolic acid and thiolactate may be used. For the preparation, a sufficient amount of a salt, glucose, mannitol or glycerin may be blended in the preparation to manufacture an isotonic solution, and an ordinary solubilizer, a soothing agent, or a topical anesthetic may be used.

When the preparation in the form of an ointment such as a paste, a cream, and a gel is manufactured, an ordinarily used base material, a stabilizer, a wetting agent, and a preservative may be blended, if necessary, and may be prepared by mixing the components by a common method. As the base material, for example, white petrolatum, polyethylene, paraffin, glycerin, cellulose derivatives, polyethylene glycol, silicon, and bentonite may be used. As the preservative, paraoxy methyl benzoate, paraoxy ethyl benzoate, paraoxy propyl benzoate and the like may be used. When the preparation in the form of a patch is manufactured, the aforementioned ointment, cream gel, or paste and the like may be applied by a common method to an ordinary support. As the support, fabric made of cotton, span rayon, and synthetic fibersor or nonwoven fabric, and a film or a foam sheet such as made of soft vinyl chloride, polyethylene, and polyurethane and the like may be preferably used.

A dose of the medicament of the present invention is not particularly limited. For oral administration, a dose may generally be 0.01 to 5,000 mg per day for an adult as the weight of the compound of the present invention. It is preferred to increase or decrease the above dose appropriately depending on the age, pathological conditions, and symptoms of a patient. The above dose may be administered once a day or 2 to 3 times a day as divided portions with appropriate intervals, or intermittent administration for every several days may be applied. When the medicament is used as an injection, the dose may be 0.001 to 100 mg per day for an adult as the weight of the compound of the present invention.

### Examples

The present invention will be explained more specifically with reference to the following examples. However the scope of the present invention is not limited to the following examples. The compound number in the following examples correspond to those in the table shown above. And the commercially available compounds, which were purchased and used for the examinations, are contained in these examples. As for such compounds, the suppliers of the reagents and the catalog code numbers are shown.

### Example 1: Preparation of the compound of Compound No. 1.

Under argon atmosphere, 1-(3-dimethylaminopropyl)-3-ethylcarbodiimide hydrochloride (it is abbreviated as WSC · HCl hereafter.; 192mg, 1mmol) was added to a mixture of 5-bromosalicylic acid(217mg, 1mmol), 3,5-bis(trifluoromethyl)benzylamine(243mg, 1mmol), 4-dimethylaminopyridine(12mg, 0.1mmol) and tetrahydrofuran(10mL), and the mixture was stirred at room temperature for 1 hour. The reaction mixture was poured into diluted hydrochloric acid and extracted with ethyl acetate. After the organic layer was washed with water and brine, dried over anhydrous magnesium sulfate, the residue obtained by evaporation under reduced pressure was purified by chromatography on silica gel(n-hexane:ethyl acetate=4:1) to give the title compound(244.8mg, 55.4%) as a white solid.
¹H-NMR(DMSO-d₆): δ4.69(2H, d, J=5.7Hz), 6.93(1H, d, J=8.7Hz), 7.56(1H, dd, J=8.7, 2.4Hz), 8.02(1H, d, J=2.4Hz), 8.06(3H, s), 9.41(1H, t, J=5.7Hz), 12.13(1H, s).

### Example 2: Preparation of the compound of Compound No. 2.

### (1) 2-Acetoxy-N-(2-phenethyl)benzamide.

O-Acetylsalicyloyl chloride(0.20g, 1.00mmol) was dissolved in benzene(8mL). Phenethylamine(0.12g, 1.00mmol) and pyridine(0.3mL) were added, and the mixture was stirred at room temperature for 2 hours. The reaction mixture was poured into diluted hydrochloric acid and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=2:1→1:1) to give the title compound(155.5mg, 54.9%) as a white crystal.
¹H-NMR(CDCl₃): δ 2.09(3H, s), 2.92(2H, t, J=6.8Hz), 3.71(2H, q, J=6.8Hz), 6.32(1H, brs),7.07(1H, dd, J=8.4, 1.2Hz), 7.23-7.35(6H, m), 7.44(1H, ddd, J=8.0, 7.6, 1.6Hz), 7.73(1H, dd, J=7.6, 1.6Hz).

When the preparation method described in Example 2(1) is referred in the following examples, organic bases such as pyridine, triethylamine or the like were used as the base. As the reaction solvent, solvents such as dichloromethane, tetrahydrofuran, benzene or the like were used alone or as a mixture.

### (2) 2-Hydroxy-N-(2-phenethyl)benzamide.

Methanol(5mL) and 2N sodium hydroxide(0.1mL) were added to 2-acetoxy-N-(2-phenethyl)benzamide(155.5mg), and the mixture was stirred at room temperature for 30 minutes. The reaction mixture was poured into diluted hydrochloric acid and extracted with ethyl acetate. After the organic layer was washed with water and brine, dried over anhydrous sodium sulfate, the residue obtained by evaporation under reduced pressure was crystallized(dichloromethane/hexane) to give the title compound(106.9mg, 80.7%) as a white solid.
¹H-NMR(DMSO-d₆): δ 2.86(2H, t, J=7.6Hz), 3.52(1H, q, J=7.6Hz),6.84-6.88(2H, m), 7.18-7.31(5H, m), 7.37(1H, ddd, J=8.4, 7.2, 1.6Hz), 7.80(1H, dd, J=8.4, 1.6Hz), 8.84(1H, s), 12.51(1H, s).

When the method described in Example 2(2) is referred in the following examples, inorganic bases such as sodium hydroxide, potassium carbonate or the like were used as the base. As the reaction solvent, solvents such as water, methanol, ethanol, tetrahydrofuran or the like were used alone or as a mixture.

### (3) 5-Bromo-2-hydroxy-N-(2-phenethyl)benzamide(Compound No. 2).

Carbon tetrachloride(5mL), iron powder(0.03g) and bromine(25 µl, 0.48mmol) were added to 2-hydroxy-N-(2-phenethyl)benzamide(79.6mg, 0.33mmol), and the mixture was stirred at room temperature for 1 hour. The reaction mixture was poured into aqueous sodium hydrogen sulfite and extracted with ethyl acetate. After the organic layer was washed with brine, dried over anhydrous sodium sulfate, the residue obtained by evaporation under reduced pressure was purified by chromatography on silica gel(n-hexane:ethyl acetate=5:1) to give the title compound(62mg, 58.7%) as a white powder.
¹H-NMR(DMSO-d₆): δ2.85(2H, t, J=7.6Hz), 3.52(1H, q, J=7.6Hz),6.87(1H, d, J=8.8Hz), 7.18-7.31(5H, m), 7.52(1H, dd, J=8.8, 2.4Hz), 8.01(1H, d, J=2.4Hz), 8.90(1H, s), 12.51(1H, s).

### Example 3: Preparation of the compound of Compound No. 3.

WSC · HCl(96mg, 0.5mmol) was added to a solution of 5-bromosalicylic acid(109mg, 0.5mmol), 2-amino-5-(morpholino)carbonylindane(141mg, 0.5mmol) and triethylamine(70 µL, 0.5mmol) in dichloromethane(5mL), and the mixture was stirred at 40°C for 1.5 hours. After cooling, the reaction mixture was diluted with ethyl acetate, washed successively with 2N hydrochloric acid, water, and brine, dried over anhydrous magnesium sulfate, concentrated, and the residue was purified by column chromatography on silica gel(dichloromethane:methanol=19:1) to give the title compound(26mg, 11.9%) as a white crystal.
¹H-NMR(CDCl₃): δ2.66(1H, dd, J=16.2, 7.2Hz), 2.82(1H, dd, J=16.2, 7.2Hz), 3.16-3.25(2H, m), 3.43-3.86(8H, m), 4.79-4.92(1H, m), 6.88(1H, d, J=8.7Hz), 7.14-7.15(3H, m), 7.46(1H, dd, J=8.7, 2.4Hz), 7.74(1H, d, J=7.8Hz), 7.84(1H, d, J=2.4Hz).

### [2-Amino-5-(morpholino)carbonylindane: Refer to "Chemical and Pharmaceutical Bulletin", 2000, Vol.48, p.131.]

### Example 4: The compound of Compound No. 4.

This compound is a commercially available compound.
Supplier: Apin Chemicals.
Catalog code number: N 0100D.

### Example 5: The compound of Compound No. 5.

This compound is a commercially available compound.
Supplier: Specs.
Catalog code number: AI-233/31581024.

### Example 6: The compound of Compound No. 6.

This compound is a commercially available compound.
Supplier: Maybridge.
Catalog code number: RJC 00106.

### Example 7: The compound of Compound No. 7.

This compound is a commercially available compound.
Supplier: Maybridge.
Catalog code number: BTB 13230.

### Example 8: The compound of Compound No. 8.

This compound is a commercially available compound.
Supplier: Maybridge.
Catalog code number: BTB 114482.

### Example 9: Preparation of the compound of Compound No. 9.

5-Chlorosalicylaldehyde(313mg, 2mmol) and 4-chlorobenzyltriphenylphosphonium chloride(847mg, 2mmol) were dissolved in N,N-dimethylformamide(20mL). Potassium carbonate(1.382g, 10mmol) dissolved in water(10mL) was added, and the mixture was refluxed for 5 hours. After cooling, the reaction mixture was poured into 2N hydrochloric acid and extracted with ethyl acetate. After the organic layer was washed with water and brine, dried over anhydrous magnesium sulfate, the residue obtained by evaporation under reduced pressure was purified by chromatography on silica gel(n-hexane:ethyl acetate=3:1) to give the title compound(44.6mg, 8.4%) as a light gray solid.
¹H-NMR(CDCl₃): δ 5.04(1H, s), 6.74(1H, d, J=9.0Hz), 7.05(1H, d, J=16.5Hz), 7.10(1H, dd, J=8.4, 2.4Hz), 7.26(1H, d, J=16.5Hz), 7.33(2H, d, J=8.4Hz), 7.45(2H, d, J=8.4Hz), 7.49(1H, d, J=2.4Hz).

### Example 10: Preparation of the compound of Compound No. 10.

### (1) 5-Bromo-N-(3,5-dichlorophenyl)-2-methoxybenzenesulfonamide.

5-Bromo-2-methoxybenzenesulfonyl chloride(857mg, 3mmol) was dissolved in dichloromethane(6mL). A solution of 3,5-dichloroaniline(510mg, 3.15mmol) and pyridine(261mg, 3.3mmol) in dichloromethane(2mL) was added dropwise under ice cooling and argon atmosphere, and the mixture was stirred at room temperature for 6 hours. After the reaction mixture was diluted with dichloromethane, washed successively with 2N hydrochloric acid, water, and brine, dried over anhydrous magnesium sulfate, the solvent was evaporated under reduced pressure. The obtained residue was crystallized from n-hexane-ethyl acetate to give 5-bromo-2-methoxy-N-(3,5-dichloro)benzenesulfonamide(900mg, 73.0%) as a white crystal.
¹H-NMR(DMSO-d₆): δ 4.03(3H, s), 6.92(1H, d, J=9.0Hz), 7.01(2H, d, J=1.8Hz), 7.07-7.08(1H, m), 7.24(1H, brs), 7.63(1H, dd, J=8.7, 2.4Hz), 7.99(1H, d, J=2.4Hz). (2) 5-Bromo-N-(3,5-dichlorophenyl)-2-hydroxybenzenesulfonamide(Compound No. 10).

A mixture of the white crystal of 5-Bromo-N-(3,5-dichlorophenyl)-2-methoxybenzenesulfonamide(206mg, 0.5mmol), lithium iodide(134mg, 1mmol) and 2,4,6-collidine(5mL) was refluxed for 30 minutes under argon atmosphere. After cooling to room temperature, the reaction mixture was poured into 2N hydrochloric acid and extracted with ethyl acetate. After the ethyl acetate layer was washed successively with water and brine, dried over anhydrous magnesium sulfate, the solvent was evaporated under reduced pressure. The obtained residue was crystallized from n-hexane-ethyl acetate to give the title compound(90mg, 45.3%) as a white crystal.
mp 158-159°C.
¹H-NMR(DMSO-d₆): δ6.92(1H, d, J=8.7Hz), 7.11(2H, d, J=2.1Hz), 7.21-7.22(1H, m), 7.62(1H, dd, J=8.7, 2.7Hz), 7.80(1H, d, J=2.4Hz), 10.70(1H, br), 11.37(1H, br).

### Example 11: Preparation of the compound of Compound No. 11.

2-Aminophenol(120mg, 1.1mmol) was dissolved in dichloromethane(5mL). A solution of 3,5-bis(trifluoromethyl)benzoyl chloride(300mg, 1.1mmol) in dichloromethane(3mL) and pyridine(0.5mL) was added dropwise under ice cooling and argon atmosphere, and the mixture was stirred at room temperature for 1 hour. The reaction mixture was poured into 2N hydrochloric acid and extracted with ethyl acetate. After the ethyl acetate layer was washed successively with water and brine, dried over anhydrous magnesium sulfate, the solvent was evaporated under reduced pressure. The obtained residue was dissolved in ethanol(5mL). 2N Sodium hydroxide(0.1mL, 0.2mmol) was added dropwise, and the mixture was stirred at room temperature for 30 minutes. The reaction mixture was poured into 2N hydrochloric acid and extracted with ethyl acetate. After the ethyl acetate layer was washed successively with water and brine, dried over anhydrous sodium sulfate, the solvent was evaporated under reduced pressure. The obtained residue was purified by column chromatography on silica gel(n-hexane:ethyl acetate=4:1) to give the title compound(288mg, 73.6%) as a light pink crystal.
mp 183°C(dec.).
¹H-NMR(DMSO-d₆): δ 6.83(1H, td, J=8.0, 1.2Hz), 6.93(1H, dd, J=8.0, 1.2Hz), 7.08(1H, td, J=8.0, 1.6Hz), 7.50(1H, d, J=8.0Hz), 8.35(2H, s), 9.61(1H, s), 10.15(1H, s).

### Example 12: Preparation of the compound of Compound No. 12.

2-Amino-4-chlorophenol(316mg, 2.2mmol) and triethylamine(243mg, 2.4mmol) were dissolved in dichloromethane(8mL). A solution of 3,5-dichlorobenzoyl chloride(419mg, 2mmol) in dichloromethane(2mL) was added dropwise under ice cooling and argon atmosphere, and the mixture was stirred at room temperature for 15 hours. After the reaction mixture was diluted with ethyl acetate, washed successively with water and brine, dried over anhydrous magnesium sulfate, the solvent was evaporated under reduced pressure. The obtained residue was purified by column chromatography on silica gel(n-hexane:ethyl acetate=3:1) to give a light brown solid. The solid was suspended and washed with n-hexane-ethyl acetate under heating at reflux to give the title compound(205mg, 32.4%) as a white crystal.
mp 251-252°C.
¹H-NMR(DMSO-d₆): δ 6.93(1H, d, J=9.0Hz), 7.11(1H, dd, J=8.7, 2.7Hz), 7.67(2H, d, J=2.7Hz), 7.86-7.87(1H, m), 7.97(1H, d, J=1.8Hz), 9.85(1H, s), 10.03(1H, s).

### Example 13: Preparation of the compound of Compound No. 13.

2-Amino-4-chlorophenol(287mg, 2mmol) and 3,5-dichlorobenzenesulfonyl chloride(540mg, 2.2mmol) were dissolved in dichloromethane(4mL). Pyridine(1mL) was added dropwise under ice cooling and argon atmosphere, and the mixture was stirred at room temperature for 1 hour. The reaction mixture was poured into 2N hydrochloric acid and extracted with ethyl acetate. After the ethyl acetate layer was washed successively with water and brine, dried over anhydrous magnesium sulfate, the solvent was evaporated under reduced pressure. The obtained residue was purified by column chromatography on silica gel(n-hexane:ethyl acetate=3:1→1:1) to give a reddish brown solid. The solid was crystallized from n-hexane-ethyl acetate to give the title compound(445mg, 63.1%) as a slight dark brown crystal.
mp 190-191°C.
¹H-NMR(DMSO-d₆): δ6.68(1H, d, J=9.0Hz), 7.08(1H, dd, J=8.7, 2.7Hz), 7.17(1H, d, J=2.4Hz), 7.70(2H, d, J=1.8Hz), 7.95-7.96(1H, m), 10.00(1H, s), 10.06(1H, s).

### Example 14: Preparation of the compound of Compound No. 14.

### (1) 4-Bromo-2-[(3,5-diphenylimino)methyl]phenol.

A mixture of 5-bromosalicylaldehyde(1.01g, 5mmol), 3,5-dichloroaniline(810mg, 5mmol) and ethanol(25mL) was refluxed for 1 hour under argon atmosphere. After the reaction mixture was cooled to room temperature, the separated crystal was filtered to give the title compound(1.52g, 88.2%) as an orange crystal.
mp 161-163°C.
¹H-NMR(CDCl₃): δ6.94(1H, d, J=9.0Hz), 7.16(2H, d, J=1.8Hz), 7.30-7.31(1H, m), 7.47-7.53(2H, m), 8.51(1H, s).

### (2) N-[(5-Bromo-2-hydroxyphenyl)methyl]-3,5-dichloroaniline(Compound No. 14).

4-Bromo-2-[(3,5-diphenylimino)methyl]phenol(1.04g, 3mmol) was dissolved in tetrahydrofuran(12mL) and ethanol(6mL). Sodium borohydride(113mg, 3mmol) was added under ice cooling and argon atmosphere, and the mixture was stirred at room temperature for 12 hours. Acetone(10mL) was added to the reaction mixture. Water was added to the residue obtained by concentration under reduced pressure, and it was extracted with dichloromethane. After the dichloromethane layer was washed successively with water and brine, dried over anhydrous magnesium sulfate, the solvent was evaporated under reduced pressure. The obtained residue was purified by column chromatography on silica gel(n-hexane:ethyl acetate=4:1) to give a light yellow viscous material. This was crystallized by n-hexane to give the title compound(971mg, 93.3%) as a white crystal.
mp 125-126°C.
¹H-NMR(CDCl₃): δ4.31(2H, s), 6.64(2H, d, J=1.8Hz), 6.74-6.77(1H,m), 6.84-6.85(1H, m), 7.30-7.34(2H, m).

### Example 15: The compound of Compound No. 15.

This compound is a commercially available compound.
Supplier: Sigma-Aldrich.
Catalog code number: S3203-5.

### Example 16: Preparation of the compound of Compound No. 16.

A mixture of 5-chlorosalicylic acid(173mg, lmmol), 3,5-bis(trifluoromethyl)-N-methylaniline(243mg, lmmol), phosphorus trichloride(44 µl, 0.5mmol) and monochlorobenzene(5mL) was refluxed for 3 hours under argon atmosphere. After the reaction mixture was cooled to room temperature, n-hexane(50mL) was added, and the separated crude crystal was filtered and dissolved in ethyl acetate(50mL). After the ethyl acetate solution was washed successively with water and brine, dried over anhydrous sodium sulfate, the solvent was evaporated under reduced pressure. The obtained residue was purified by column chromatography on silica gel(n-hexane:ethyl acetate=2:1) to give the title compound(75mg, 18.9%) as a white crystal.
¹H-NMR(CDCl₃): δ 3.57(3H, s), 6.59(1H, d, J=2.4Hz), 6.94(1H, d, J=9.0Hz), 7.21(1H, dd, J=9.0, 2.7Hz), 7.58(2H, s), 7.80(1H, s), 10.00(1H, brs).

When the method described in Example 16 is referred in the following examples, phosphorus trichloride was used as the acid halogenating agent. As the reaction solvent, solvents such as monochlorobenzene, toluene or the like were used.

### Example 17: Preparation of the compound of Compound No. 17.

Using 5-bromosalicylic acid and 7-trifluoromethyl-1,2,3,4-tetrahydroquinoline as the raw materials, the same operation as the Example 16 gave the title compound.
Yield: 42.0%.
¹H-NMR(CDCl₃): δ 2.08(2H, m), 2.92(2H, t, J=6.6Hz), 3.95(2H, t, J=6.6Hz), 6.91-6.94(2H, m), 7.14(1H, s), 7.32-7.35(2H, m), 7.40(1H, dd, J=8.7, 2.4Hz), 10.06(1H, s).

### Example 18: Preparation of the compound of Compound No. 18.

Using 2-hydroxynaphthalene-1-carboxylic acid and 3,5-dichloroaniline as the raw materials, the same operation as the Example 16 gave the title compound.
Yield: 51.2%.
mp 246-248°C.
¹H-NMR(DMSO-d₆): δ7.26(1H, d, J=9.3Hz), 7.31-7.37(2H, m), 7.44-7.50(1H, m), 7.65-7.68(1H, m), 7.85-7.90(4H, m), 10.23(1H, s), 10.74(1H, s).

### Example 19: Preparation of the compound of Compound No. 19.

Using 3-hydroxynaphthalene-2-carboxylic acid and 3,5-dichloroaniline as the raw materials, the same operation as the Example 16 gave the title compound.
Yield: 44.3%.
mp 254-255°C.
¹H-NMR(DMSO-d₆): δ 7.34-7.39(3H, m), 7.49-7.54(1H, m), 7.76-7.79(1H, m), 7.89(2H, d, J=1.8Hz), 7.92(1H, m), 8.39(1H, s), 10.75(1H, s), 11.01(1H, s).

### Example 20: The compound of Compound No. 20.

This compound is a commercially available compound.
Supplier: Sigma-Aldrich.
Catalog code number: S01361-8.

### Example 21: Preparation of the compound of Compound No. 21.

Using 1-hydroxynaphthalene-2-carboxylic acid and 3,5-bis(trifluoromethyl)aniline as the raw materials, the same operation as the Example 16 gave the title compound.
Yield: 65.5%.
¹H-NMR(DMSO-d₆): δ7.51(1H, d, J=9.0Hz), 7.60(1H, td, J=7.8, 0.9Hz), 7.70(1H, td, J=7.8, 0.9Hz), 7.89(1H, s), 7.93(1H, d, J=8.4Hz), 8.09(1H, d, J=9.0Hz), 8.33(1H, d, J=8.7Hz), 8.51(2H, s), 10.92(1H, s), 13.36(1H, s).

### Example 22: The compound of Compound No. 22.

This compound is a commercially available compound.
Supplier: Sigma-Aldrich.
Catalog code number: S58026-0.

### Example 23: The compound of Compound No. 23.

This compound is a commercially available compound.
Supplier: Sigma-Aldrich.
Catalog code number: S63263-5.

### Example 24: Preparation of the compound of Compound No. 24.

5-Chloro-2-hydroxynicotinic acid(174mg, 1mmol), 3,5-bis(trifluoromethyl)aniline(275mg, 1.2mmol) and pyridine(316mg, 4mmol) were dissolved in tetrahydrofuran(20mL) and dichloromethane(10mL). Phosphorus oxychloride(0.112ml, 1.2mmol) was added, and the mixture was stirred at room temperature for 2 hours. The reaction mixture was poured into ethyl acetate(100mL) and 0.2N hydrochloric acid(100mL), filtered through celite after stirring for 30 minutes, and the water layer of the filtrate was extracted with ethyl acetate. After the combined ethyl acetate layer was washed successively with water and brine, dried over anhydrous magnesium sulfate, the solvent was evaporated under reduced pressure. The residue was purified by column chromatography on silica gel(n-hexane:ethyl acetate=2:1→1:1) to give a light yellow solid. This was suspended and washed with ethanol under heating at reflux to give the title compound(183mg, 47.6%) as a white crystal.
mp >270°C.
¹H-NMR(DMSO-d₆): δ7.83(1H, s), 8.15(1H, d, J=3.3Hz), 8.36(1H, d, J=3.0Hz), 8.40(2H, s), 12.43(1H, s).

When the preparation method described in Example 24 is referred in the following examples, phosphorus oxychloride was used as the acid halogenating agent. Pyridine was used as the base. As the reaction solvent, solvents such as dichloromethane, tetrahydrofuran or the like were used alone or as a mixture.

### Example 25: Preparation of the compound of Compound No. 25.

Using 5-chloro-2-hydroxynicotinic acid and 2-chloro-5-(trifluoromethyl)aniline as the raw materials, the same operation as the Example 24 gave the title compound.
Yield: 42.9%.
¹H-NMR(DMSO-d₆): δ7.52(1H, dd, J=8.4, 2.1Hz), 7.81(1H, d, J=8.4Hz), 8.16(1H, s), 8.39(1H, d, J=2.7Hz), 8.96(1H, d, J=2.1Hz), 12.76(1H, s), 13.23(1H, s).

### Example 26: Preparation of the compound of Compound No. 26.

Using 5-chloro-2-hydroxynicotinic acid and 3,5-bis[(1,1-dimethyl)ethyl]aniline as the raw materials, the same operation as the Example 24 gave the title compound.
Yield: 59.1%.
¹H-NMR(DMSO-d₆): δ 1.29(18H, s), 7.18(1H, t, J=1.8Hz), 7.52(2H.d, J=1.8Hz), 8.07(1H, d, J=2.4Hz), 8.35(1H, d, J=3.3Hz), 11.92(1H, s), 13.10(1H, s).

### Example 27: Preparation of the compound of Compound No. 27.

Using 3-hydroxypyridine-2-carboxylic acid and 3,5-bis(trifluoromethyl)aniline as the raw materials, the same operation as the Example 24 gave the title compound.
Yield: 45.0%.
¹H-NMR(CDCl₃): δ7.40(1H, dd, J=8.4, 1.8Hz), 7.46(1H, dd, J=8.4, 4.2Hz), 7.68(1H, s), 8.16(1H, dd, J=4.2, 1.2Hz), 8.25(2H, s), 10.24(1H, s), 11.42(1H, s).

### Example 28: Preparation of the compound of Compound No. 28.

Under argon atmosphere, 3,5-bis(trifluoromethyl)phenylisocyanate(255mg, 1.0mmol) was dissolved in tetrahydrofuran(5mL). A solution of 6-chloro-oxindole(184mg, 1.1mmol) in tetrahydrofuran(5ml) and triethylamine(0.3mL) were added, and the mixture was stirred at room temperature for 4 hours. The reaction mixture was poured into diluted hydrochloric acid and extracted with ethyl acetate. After the organic layer was washed with water and brine, dried over anhydrous magnesium sulfate, the residue obtained by evaporation under reduced pressure was purified by chromatography on silica gel(n-hexane:ethyl acetate=4:1) to give the title compound(172.2mg, 40.7%) as a pink solid.
¹H-NMR(DMSO-d₆): δ 3.97(2H, s), 7.29(1H, dd, J=8.1, 2.1Hz), 7.41(1H, d, J=8.1Hz), 7.88(1H, s), 8.04(1H, d, J=2.1Hz), 8.38(2H, s), 10.93(1H, s).

### Example 29: Preparation of the compound of Compound No. 29.

Using 3-hydroxyquinoxaline-2-carboxylic acid and 3,5-bis(trifluoromethyl)aniline as the raw materials, the same operation as the Example 16 gave the title compound.
Yield: 2.7%.
¹H-NMR(DMSO-d₆): δ7.40-7.45(2H, m), 7.69(1H, td, J=8.4, 1.5Hz), 7.90-7.93(2H, m), 8.41(2H, s), 11.64(1H, s), 13.02(1H, s).

### Example 30: The compound of Compound No. 30.

This compound is a commercially available compound.
Supplier: Sigma-Aldrich.
Catalog code number: S83846-2.

### Example 31: The compound of Compound No. 31.

This compound is a commercially available compound.
Supplier: Maybridge.
Catalog code number: RDR 01818.

### Example 32: Preparation of the compound of Compound No. 32.

Using 5-chlorosalicylic acid and 1-naphthylamine as the raw materials, the same operation as the Example 16 gave the title compound.
Yield: 65.0%.
¹H-NMR(DMSO-d₆): δ 7.09(1H, d, J=8.7Hz), 7.51-7.61(4H, m), 7.85(1H, d, J=8.4Hz), 7.96(1H, d, J=7.5Hz), 7.99-8.05(2H, m), 8.13(1H, d, J=2.7Hz), 10.88(1H, s), 12.31(1H, s).

### Example 33: Preparation of the compound of Compound No. 33.

Using 5-chlorosalicylic acid and 4-methoxy-2-naphthylamine as the raw materials, the same operation as the Example 16 gave the title compound.
Yield: 84.3%.
¹H-NMR(DMSO-d₆): δ 3.99(3H, s), 7.05(1H, d, J=9.0Hz), 7.30(1H, d, J=1.5Hz), 7.39-7.45(1H, m), 7.48-7.54(2H, m), 7.83(1H, d, J=7.8Hz), 8.00(1H, s), 8.02(1H, d, J=2.4Hz), 8.09(1H, d, J=7.8Hz), 10.54(1H, s), 11.88(1H, s).

### Example 34: Preparation of the compound of Compound No. 34.

### (1) 2-Acetoxy-5-chlorobenzoic acid.

Concentrated sulfuric acid(0.08mL) was added slowly to a mixture of 5-chlorosalicylic acid(13.35g, 77mmol) and acetic anhydride(20mL). After the reaction mixture was solidified, it was poured into ice water and extracted with ethyl acetate. The organic layer was washed with water and brine, and dried over anhydrous sodium sulfate. The residue obtained by evaporation of the solvent under reduced pressure was washed with n-hexane under suspension to give the title compound(15.44g, 93.0%) as a white crystal.
¹H-NMR(DMSO-d₆): δ2.25(3H, s), 7.27(1H, d, J=8.7Hz), 7.72(1H, dd, J=8.7, 2.7Hz), 7.89(1H, d, J=2.7Hz), 13.47(1H, s).

### (2) 2-Acetoxy-5-chloro-N-(1-methoxynaphthalen-3-yl)benzamide(Compound No. 34).

Using 2-acetoxy-5-chlorobenzoic acid and 4-methoxy-2-naphthylamine as the raw materials, the same operation as the Example 24 gave the title compound.
Yield: 39.9%, red solid.
¹H-NMR(DMSO-d₆): δ 2.23(3H, s), 3.96(3H, s), 7.23(1H, d, J=1.2Hz), 7.34(1H, d, J=8.7Hz), 7.40(1H, dt, J=8.1, 1.2Hz), 7.50(1H, dt, J=8.1, 1.5Hz), 7.67(1H, dd, J=8.7, 2.7Hz), 7.81(1H, d, J=8.7Hz), 7.82(1H, d, J=3.0Hz), 8.02(1H, s), 8.08(1H, d, J=8.7Hz), 10.58(1H, s).

### Example 35: Preparation of the compound of Compound No. 35.

Using 5-chlorosalicylic acid and 2-amino-4,5,6,7-tetrahydrobenzo[b]thiophene-3-carboxylic acid ethyl ester as the raw materials, the same operation as the Example 16 gave the title compound.
Yield: 49.6%.
¹H-NMR(DMSO-d₆): δ 1.32(3H, t, J=7.2Hz), 1.74(4H, br), 2.63(2H, br), 2.75(2H, br), 4.30(2H, q, J=7.2Hz), 7.05(1H, d, J=9.0Hz), 7.50(1H, dd, J=8.7, 3.0Hz), 7.92(1H, d, J=3.0Hz), 12.23(1H, s), 13.07(1H, s).

### Example 36: Preparation of the compound of Compound No. 36.

Using 5-bromosalicylic acid and 3-amino-5-phenylpyrazole as the raw materials, the same operation as the Example 16 gave the title compound.
Yield: 9.2%.
¹H-NMR(DMSO-d₆): δ 6.98(1H, d, J=8.8Hz), 7.01(1H, s),7.35(1H, t, J=7.6Hz), 7.46(2H, t, J=7.6Hz), 7.58(1H, dd, J=8.8, 2.8Hz), 7.74-7.76(2H, m), 8.19(1H, s), 10.86(1H, s), 12.09(1H, s), 13.00(1H, brs).

### Example 37: Preparation of the compound of Compound No. 37.

### (1) 2-Amino-4,5-diethyloxazole.

Propioin(1.03g, 8.87mmol) was dissolved in ethanol(15mL). Cyanamide(0.75g, 17.7mmol) and sodium ethoxide(1.21g, 17.7mmol) were added, and the mixture was stirred at room temperature for 3.5 hours. The reaction mixture was poured into water and extracted with ethyl acetate. After the organic layer was washed with water and brine, dried over anhydrous sodium sulfate, the residue obtained by evaporation under reduced pressure was purified by chromatography on silica gel(dichloromethane:methanol=9:1) to give the title compound(369.2mg, 29.7%) as an yellow amorphous.
¹H-NMR(DMSO-d₆): δ 1.04(3H, t, J=7.5Hz), 1.06(3H, t, J=7.5Hz), 2.20(2H, q, J=7.5Hz), 2.43(2H, q, J=7.5Hz), 6.15(2H, s).

### (2) 2-Acetoxy-5-bromo-N-(4,5-diethyloxazol-2-yl)benzamide.

Using 2-acetoxy-5-bromobenzoic acid and 2-amino-4,5-diethyloxazole as the raw materials, the same operation as the Example 24 gave the title compound.
Yield: 22.0%.
¹H-NMR(CDCl₃): δ1.22(3H, t, J=7.5Hz), 1.23(3H, t, J=7.5Hz), 2.38(3H, s), 2.48(2H, q, J=7.5Hz), 2.57(2H, q, J=7.5Hz), 6.96(1H, d, J=8.7Hz), 7.58(1H, dd, J=8.7, 2.7Hz), 8.32(1H, s), 11.40(1H, br).

### [2-Acetoxy-5-bromosalicylic acid: It was obtained, using 5-bromosalicylic acid and acetic anhydride as the raw materials, by the same operation as the Example 34(1) with reference to "Europian Journal of Medicinal Chemistry", 1996, Vol.31, p.861-874.]

### (3) 5-Bromo-N-(4,5-diethyloxazol-2-yl)-2-hydroxybenzamide(Compound No. 37).

Using 2-acetoxy-5-bromo-N-(4,5-diethyloxazol-2-yl)benzamide as the raw material, the same operation as the Example 2(2) gave the title compound.
Yield: 70.2%.
¹H-NMR(CDCl₃)δ:1.25(3H, t, J=7.5Hz), 1.26(3H, t, J=7.5Hz), 2.52(2H, q, J=7.5Hz), 2.60(2H, q, J=7.5Hz), 6.84(1H, d, J=8.7Hz), 7.43(1H, dd, J=8.7, 3.0Hz), 8.17(1H, d, J=3.0Hz), 11.35(1H, br), 12.83(1H, br).

### Example 38: Preparation of the compound of Compound No. 38.

Using 5-bromosalicylic acid and 2-amino-4,5-diphenyloxazole as the raw materials, the same operation as the Example 16 gave the title compound.
Yield: 32.6%.
mp 188-189°C.
¹H-NMR(DMSO-d₆): δ6.98(1H, d, J=8.7Hz), 7.40-7.49(6H, m), 7.53-7.56(2H, m), 7.59-7.63(3H, m), 8.01(1H, d, J=2.4Hz), 11.80(2H, brs).

### [2-Amino-4,5-diphenyloxazole: Refer to "Zhournal Organicheskoi Khimii: Russian Journal of Organic Chemistry", (Russia), 1980, Vol.16, p.2185.]

### Example 39: Preparation of the compound of Compound No. 39.

### (1) 2-Amino-4,5-bis(furan-2-yl)oxazole.

Furoin(0.50g, 2.60mmol) was dissolved in ethanol(15mL).
Cyanamide(218.8mg, 5.20mmol) and sodium ethoxide(530.8mg, 7.80mmol) were added, and the mixture was stirred at room temperature for 2 hours. The reaction mixture was poured into water and extracted with ethyl acetate. After the organic layer was washed with water and brine, dried over anhydrous sodium sulfate, the residue obtained by evaporation under reduced pressure was purified by chromatography on silica gel(hexane:ethyl acetate=1:1→1:2) to give the title compound(175.0mg, 31.1%) as a dark brown crystal.
¹H-NMR(DMSO-d₆): δ 6.59(1H, dd, J=3.3, 2.1Hz), 6.62(1H, dd, J=3.3, 2.1Hz), 6.73(1H, dd, J=3.3, 0.6Hz), 6.80(1H, dd, J=3.3, 0.9Hz), 7.05(2H, s), 7.75-7.76(2H, m).

### (2) 5-Bromo-N-[4,5-bis(furan-2-yl)oxazol-2-yl]-2-hydroxybenzamide(Compound No. 39).

Using 5-bromosalicylic acid and 2-amino-4,5-bis(furan-2-yl)oxazole as the raw materials, the same operation as the Example 16 gave the title compound.
Yield: 12.9%.
¹H-NMR(DMSO-d₆): δ6.65(1H, dd, J=3.6, 1.8Hz), 6.68(1H, dd, J=3.6, 1.8Hz), 6.75(1H, d, J=8, 7Hz), 6.92(1H, dd, J=3.6, 0.9Hz), 6.93(1H, d, J=3.3Hz), 7.37(1H, dd, J=8.7, 2.7Hz), 7.80(1H, dd, J=1.8, 0.9Hz), 7.84(1H, dd, J=1.8, 0.9Hz), 7.92(1H, d, J=3.0Hz), 14.88(2H, br).

### Example 40: Preparation of the compound of Compound No. 40.

### (1) 2-Acetoxy-N-(5-trifluoromethyl-1,3,4-thiadiazol-2-yl)benzamide.

Using O-acetylsalicyloyl chloride and 2-amino-5-(trifluoromethyl)-1,3,4-thiadiazole as the raw materials, the same operation as the Example 2(1) gave the title compound.
Yield: 51.1%.
¹H-NMR(DMSO-d₆): δ 2.23(3H, s), 7.32(1H, dd, J=8.0, 1.2Hz),7.45(1H, td, J=7.6, 1.2Hz), 7.69(1H, td, J=8.0, 2.0Hz), 7.87(1H, dd, J=8.0, 2.0Hz), 13.75(1H, brs).

### (2) 2-Hydroxy-N-(5-trifluoromethyl-1,3,4-thiadiazol-2-yl)benzamide(Compound No. 40).

Using 2-acetoxy-N-(5-trifluoromethyl-1,3,4-thiadiazol-2-yl)benzamide as the raw material, the same operation as the Example 2(2) gave the title compound.
Yield: 92.9%.
¹H-NMR(DMSO-d₆): δ7.00(1H, td, J=8.0, 0.8Hz),7.06(1H, d, J=8.4Hz), 7.51(1H, ddd, J=8.4, 7.6, 2.0Hz), 7.92(1H, dd, J=8.0, 1.6Hz), 12.16(1H, br).

### Example 41: Preparation of the compound of Compound No. 41.

Using 5-bromosalicylic acid and 2-amino-5-trifluoromethyl-1,3,4-thiadiazole as the raw materials, the same operation as the Example 16 gave the title compound.
Yield: 80.2%.
¹H-NMR(DMSO-d₆): δ 7.01(1H, d, J=9.0Hz), 7.63(1H, dd, J=8.7, 2.7Hz), 7.97(1H, d, J=2.4Hz).

### Example 42: Preparation of the compound of Compound No. 42.

Using 5-chlorosalicylic acid and 5-amino-2-chloropyridine as the raw materials, the same operation as the Example 16 gave the title compound.
Yield: 12.2%.
¹H-NMR(DMSO-d₆): δ7.04(1H, d, J=9.0Hz), 7.49(1H, dd, J=9.0, 3.0Hz), 7.54(1H, d, J=8.4Hz), 7.88(1H, d, J=2.7Hz), 8.21(1H, dd, J=8.7, 2.7Hz), 8.74(1H, d, J=2.7Hz), 10.62(1H, s), 11.57(1H, s).

### Example 43: Preparation of the compound of Compound No. 43.

Using 5-chlorosalicylic acid and 2-amino-6-chloro-4-methoxypyrimidine as the raw materials, the same operation as the Example 16 gave the title compound.
Yield: 2.2%, white solid.
¹H-NMR(DMSO-d₆): δ 3.86(3H, s), 6.85(1H, s), 7.01(1H, d, J=9.0Hz), 7.47(1H, dd, J=9.0, 3.0Hz), 7.81(1H, d, J=3.0Hz), 11.08(1H, s), 11.65(1H, s).

### Example 44: Preparation of the compound of Compound No. 44.

Using 2-acetoxy-5-chlorobenzoic acid and 5-aminoindole as the raw materials, the same operation as the Example 24 gave the title compound.
Yield: 13.3%.
¹H-NMR(DMSO-d₆): δ 2.20(3H, s), 6.41(1H, t, J=2.1Hz), 7.27-7.36(4H, m), 7.63(1H, dd, J=8.7, 2.7Hz), 7.74(1H, d, J=2.7Hz), 7.93(1H, s), 10.21(1H, s), 11.04(1H, s).

### Example 45: The compound of Compound No. 45.

This compound is a commercially available compound.
Supplier: Peakdale.
Catalog code number: PFC-0448.

### Example 46: Preparation of the compound of Compound No. 46.

Using 5-chlorosalicylic acid and 3-aminoquinoline as the raw materials, the same operation as the Example 16 gave the title compound.
Yield: 4.3%.
¹H-NMR(DMSO-d₆): δ 7.07(1H, d, J=8.7Hz), 7.51(1H, dd, J=9.0, 3.0Hz), 7.61(1H, dt, J=7.8, 1.2Hz), 7.70(1H, dt, J=7.8, 1.5Hz), 7.98(2H, d, J=3.0Hz), 8.01(1H, s), 8.82(1H, d, J=2.4Hz), 10.80(1H, s), 11.74(1H, s).

### Example 47: Preparation of the compound of Compound No. 47.

Using 5-chlorosalicylic acid and 3-amino-9-ethylcarbazole as the raw materials, the same operation as the Example 16 gave the title compound.
Yield: 64.6%.
¹H-NMR(DMSO-d₆): δ 1.33(3H, t, J=7.0Hz), 4.46(2H, q, J=7.0Hz), 7.04(1H, d, J=9.0Hz), 7.21(1H, t, J=7.3Hz), 7.45-7.52(2H, m), 7.64-7.65(2H, m), 7.70(1H, d, J=8.4, 1.9Hz), 8.11-8.15(2H, m), 8.49(1H, d, J=1.9Hz), 10.55(1H, s), 12.22(1H, s).

### Example 48: Preparation of the compound of Compound No. 95.

Using O-acetylsalicyloyl chloride and 3,5-bis(trifluoromethyl)aniline as the raw materials, the same operation as the Example 2(1) gave the title compound.
Yield: 84.2%.
¹H-NMR(DMSO-d₆): δ2.36(3H, s), 7.19(1H, dd, J=8.0, 1.2Hz), 7.39(1H, td, J=7.6, 1.2Hz), 7.57(1H, ddd, J=8.0, 7.6, 1.6Hz), 7.65(1H, s), 7.83(1H, dd, J=8.0, 1.6Hz), 8.11(2H, s), 8.31(1H, s).

### Example 49: Preparation of the compound of Compound No. 48.

Using 2-acetoxy-N-[3,5-bis(trifluoromethyl)phenyl]benzamide(Compound No. 95) as the raw material, the same operation as the Example 2(2) gave the title compound.
Yield: 45.1%.
¹H-NMR(DMSO-d₆): δ6.96-7.02(2H, m), 7.45(1H, ddd, J=8.0, 7.2, 1.6Hz), 7.81(1H, s), 7.87(1H, dd, J=8.0, 1.6Hz), 8.46(2H, s), 10.80(1H, s), 11.26(1H, s).

### Example 50: Preparation of the compound of Compound No. 49.

Using 5-fluorosalicylic acid and 3,5-bis(trifluoromethyl)aniline as the raw materials, the same operation as the Example 16 gave the title compound.
Yield: 58.7%.
¹H-NMR(DMSO-d₆): δ 7.04(1H, ddd, J=9.0, 4.5, 1.2Hz), 7.30-7.37(1H, m), 7.66(1H, ddd, J=9.0, 3.3, 1.2Hz), 7.84(1H, s), 8.46(2H, s), 10.85(1H, s), 11.21(1H, brs).

### Example 51: Preparation of the compound of Compound No. 50.

Using 5-chlorosalicylic acid and 3,5-bis(trifluoromethyl)aniline as the raw materials, the same operation as the Example 16 gave the title compound.
Yield: 85.5%.
¹H-NMR(DMSO-d₆): δ7.05(1H, d, J=8.7Hz), 7.49(1H, dd, J=8.7, 2.7Hz), 7.85(1H, s), 7.87(1H, d, J=2.7Hz), 8.45(2H, s), 10.85(1H, s), 11.39(1H, s).

### Example 52: Preparation of the compound of Compound No. 51.

Using 5-bromosalicylic acid and 3,5-bis(trifluoromethyl)aniline as the raw materials, the same operation as the Example 16 gave the title compound.
Yield: 88.5%.
¹H-NMR(DMSO-d₆): δ6.98(1H, d, J=8.8Hz), 7.59(1H, dd, J=8.8, 2.8Hz), 7.83(1H, s), 7.98(1H, d, J=2.8Hz), 8.43(2H, s), 10.82(1H, s), 11.37(1H, s).

This compound was obtained also by the following preparation method.

Iron powder(30mg, 0.54mmol) and bromine(0.02mL, 0.39mmol) were added to a solution of 2-acetoxy-N-[3,5-bis(trifluoromethyl)]benzamide(Compound No. 95; 100mg, 0.25mmol) in carbon tetrachloride(8mL), and the mixture was stirred at 50°C for 4 hours. After the reaction mixture was cooled to room temperature, it was poured into aqueous NaHSO₄ and extracted with ethyl acetate. The ethyl acetate layer was washed with water and brine, and dried over anhydrous sodium sulfate. The residue obtained by evaporation of the solvent under reduced pressure was purified by column chromatography on silica gel(n-hexane:ethyl acetate=4:1) to give the title compound(600mg, 54.9%) as a white solid.

### Example 53: Preparation of the compound of Compound No. 52.

Using 5-iodosalicylic acid and 3,5-bis(trifluoromethyl)aniline as the raw materials, the same operation as the Example 16 gave the title compound.
Yield: 62.2%.
¹H-NMR(DMSO-d₆): δ 6.86(1H, d, J=8.4Hz), 7.74(1H, dd, J=8.7, 2.4Hz), 7.84(1H, s), 8.13(1H, d, J=2.1Hz), 8.84(2H, s), 10.82(1H, s), 11.41(1H, s).

### Example 54: Preparation of the compound of Compound No. 53.

Using 5-nitrosalicylic acid and 3,5-bis(trifluoromethyl)aniline as the raw materials, the same operation as the Example 16 gave the title compound.
Yield: 57.2%.
¹H-NMR(DMSO-d₆): δ 7.18(1H, d, J=9.0Hz), 7.86(1H, s), 8.31(1H, dd, J=9.0, 3.0Hz), 8.45(2H, s), 8.70(1H, d, J=3.0Hz), 11.12(1H, s).

### Example 55: Preparation of the compound of Compound No. 54.

### (1) 2-Benzyloxy-5-formylbenzoic acid benzyl ester.

A mixture of 5-formylsalicylic acid(4.98g, 30mmol), benzyl bromide(15.39g, 90mmol), potassium carbonate(16.59g, 120mmol), and methyl ethyl ketone(350mL) was refluxed for 8 hours. After cooling, the solvent was evaporated under reduced pressure. 2N Hydrochloric acid was added to the residue, and the mixture was extracted with ethyl acetate. The layer was washed with water and brine, and dried over anhydrous magnesium sulfate. The residue obtained by evaporation of the solvent under reduced pressure was purified by column chromatography on silica gel(n-hexane:ethyl acetate=3:1), suspended and washed with isopropyl ether under heating at reflux to give the title compound(5.98g, 57.5%) as a white solid.
¹H-NMR(CDCl₃): δ 5.27(2H, s), 5.37(2H, s), 7.15(1H, d, J=9.0Hz), 7.26-7.46(10H, m), 7.99(1H, dd, J=9.0, 2.4Hz), 8.36(1H, d, J=2.4Hz), 9.91(1H, s).

### (2) 2-Benzyloxy-5-cyanobenzoic acid benzyl ester.

A mixture of 2-benzyloxy-5-formylbenzoic acid benzyl ester(693mg, 2mmol), hydroxylamine hydrochloride(167mg, 2.4mmol), and N-methylpyrrolidone(3mL) was stirred at 115°C for 4 hours. After the reaction mixture was cooled, 2N hydrochloric acid(5mL) and water(30mL) were added and the mixture was extracted with ethyl acetate. The organic layer was washed with 2N aqueous sodium hydroxide, water, and brine, and dried over anhydrous magnesium sulfate. The residue obtained by evaporation of the solvent under reduced pressure was suspended and washed with isopropyl ether under heating at reflux to give the title compound(527mg, 76.7%) as a white solid.
¹H-NMR(CDCl₃): δ 5.23(2H, s), 5.35(2H, s), 7.08(1H, d, J=8.7Hz), 7.33-7, 43(10H, m), 7.70(1H, dd, J=8.7, 2.4Hz), 8.13(1H, d, J=2.4Hz).

### (3) 5-Cyanosalicylic acid.

Ethanol(10mL) and tetrahydrofuran(10mL) were added to 2-benzyloxy-5-cyanobenzoic acid benzyl ester(446mg, 1.3mmol) and 5% palladium on carbon(45mg), and the mixture was hydrogenated at room temperature for 2 hours. After the insoluble matter was filtered off, the solvent was evaporated under reduced pressure to give the title compound(212mg, 100.0%) as a white solid.
¹H-NMR(DMSO-d₆): δ 7.02(1H, d, J=8.7Hz), 7.82(1H, dd, J=8.7, 2.4Hz), 8.12(1H, d, J=2.1Hz).

### (4) N-[3,5-Bis(trifluoromethyl)phenyl]-5-cyano-2-hydroxybenzamide(Compound No. 54).

Using 5-cyanosalicylic acid and 3,5-bis(trifluoromethyl)aniline as the raw materials, the same operation as the Example 16 gave the title compound.
Yield: 16.6%.
¹H-NMR(DMSO-d₆): δ 7.15(1H, d, J=8.7Hz), 7.85(1H, s), 7.86(1H, dd, J=8.7, 2.1Hz), 8.22(1H, d, J=2.4Hz), 8.43(2H, s), 10.93(1H, s), 12.00(1H, brs).

### Example 56: Preparation of the compound of Compound No. 55.

Using 5-methylsalicylic acid and 3,5-bis(trifluoromethyl)aniline as the raw materials, the same operation as the Example 16 gave the title compound.
Yield: 54.9%.
¹H-NMR(DMSO-d₆): δ 6.92(1H, d, J=8.7Hz), 7.28(1H, dd, J=8.7, 1.8Hz), 7.71(1H, d, J=1.8Hz), 7.82(1H, s), 8.47(2H, s), 10.80(1H, s), 11.14(1H, s).

### Example 57: Preparation of the compound of Compound No. 56.

### (1) 5-[(1,1-Dimethyl)ethyl]salicylic acid.

Sulfamic acid(1.76g, 18.1mmol) and sodium dihydrogenphosphate(7.33g, 47mmol) were added to a solution of 5-[(1,1-dimethyl)ethyl]-2-hydroxybenzaldehyde(2.15g, 12.1mmol) in 1,4-dioxane(100mL) and water(40mL). A solution of sodium chlorite(1.76g, 15.5mmol) in water(10mL) was added to the mixture under ice cooling, and it was stirred for 1 hour. Then, sodium sulfite(1.80g, 14.3mmol) was added to the mixture, and it was stirred for 30 minutes. Concentrated hydrochloric acid was added to the reaction mixture, and pH was adjusted to 1. The residue obtained by evaporation of 1,4-dioxane under reduced pressure was extracted with ethyl acetate. The organic layer was washed with water and brine, and dried over anhydrous magnesium sulfate. The residue obtained by evaporation of the solvent under reduced pressure was washed with n-hexane under suspension to give the title compound(1.81g, 77.4%) as a white powder.
¹H-NMR(DMSO-d₆): δ 1.26(9H, s), 6.90(1H, d, J=9.0Hz), 7.58(1H, dd, J=8.7, 2.4Hz), 7.75(1H, d, J=2.4Hz), 11.07(1H, brs).

### (2) N-[3,5-Bis(trifluoromethyl)phenyl]-5-[(1,1-dimethyl)ethyl]-2-hydroxybenzamide (Compound No. 56).

Using 5-[(1,1-dimethyl)ethyl]salicylic acid and 3,5-bis(trifluoromethyl)aniline as the raw materials, the same operation as the Example 16 gave the title compound.
Yield: 53.8%.
¹H-NMR(DMSO-d₆): δ 1.30(9H, s), 6.96(1H, d, J=8.7Hz), 7.50(1H, dd, J=8.7, 2.4Hz), 7.82(1H, d, J=2.4Hz), 7.83(1H, s), 8.46(2H, s), 10.80(1H, s)11.12(1H, s).

### Example 58: Preparation of the compound of Compound No. 78.

### (1) 5-Acetyl-2-benzyloxybenzoic acid methyl ester.

A mixture of 5-acetylsalicylic acid methyl ester(13.59g, 70mmol), benzyl bromide(17.96g, 105mmol), potassium carbonate(19.35g, 140mmol) and methyl ethyl ketone(350mL) was refluxed for 8 hours. After cooling, the solvent was evaporated under reduced pressure. 2N Hydrochloric acid was added to the residue, and it was extracted with ethyl acetate. After the ethyl acetate layer was washed with water and brine, dried over anhydrous magnesium sulfate and concentrated, the residue was recrystallized from isopropyl ether to give the title compound(14.20g, 71.4%) as a white solid.
¹H-NMR(CDCl₃): δ2.58(3H, s), 3.93(3H, s), 5.27(2H, s), 7.07(1H, d, J=8.7Hz), 7.26-7.43(3H, m), 7.47-7.50(2H, m), 8.07(1H, dd, J=8.7, 2.4Hz), 8.44(1H, d, J=2.4Hz).

### (2) 5-Acetyl-2-benzyloxybenzoic acid.

5-Acetyl-2-benzyloxybenzoic acid methyl ester(5.69g, 20mmol) was dissolved in a mixed solvent of methanol(20mL) and tetrahydrofuran(20mL). 2N Sodium hydroxide(11mL) was added dropwise, and the mixture was stirred for 8 hours. The solvent was evaporated under reduced pressure. 2N Hydrochloric acid was added to the residue, and it was extracted with dichloromethane. After the dichloromethane layer was washed with water and brine, dried over anhydrous magnesium sulfate and concentrated, the residue was washed with isopropyl ether to give the title compound(4.92g, 91.0%) as a white solid.
¹H-NMR(DMSO-d₆): δ 2.55(3H, s), 5.32(2H, s), 7.30-7.43(4H, m), 7.49-7.52(2H, m), 8.09(1H, dd, J=9.0, 2.7Hz), 8.22(1H, d, J=2.4Hz).

### (3) 5-Acetyl-2-benzyloxy-N-[3,5-bis(trifluoromethyl)phenyl]benzamide.

Using 5-acetyl-2-benzyloxybenzoic acid and 3,5-bis(trifluoromethyl)aniline as the raw materials, the same operation as the Example 24 gave the title compound.
Yield: 63.1%.
¹H-NMR(DMSO-d₆): δ 2.57(3H, s), 7.11(1H, d, J=8.7Hz), 7.86(1H, s), 8.05(1H, dd, J=8.4, 2.1Hz), 8.44(1H, d, J=2.1Hz), 8.47(2H, s), 10.96(1H, s), 11.97(1H, brs).

### (4) 5-Acetyl-N-[3,5-bis(trifluoromethyl)phenyl]-2-hydroxybenzamide(Compound No. 78).

Ethanol(6mL) and tetrahydrofuran(72mL) were added to 5-acetyl-2-benzyloxy-N-[3,5-bis(trifluoromethyl)phenyl]benzamide(602mg, 1.25mmol) and 5% palladium on carbon(60mg), and the mixture was hydrogenated at room temperature for 30 minutes. After the insoluble matter was filtered off, the solvent was evaporated under reduced pressure and the residue was recrystallized from n-hexane-ethyl acetate to give the title compound(230mg, 47.0%) as a white solid.
¹H-NMR(DMSO-d₆): δ 2.59(3H, s), 5.35(2H, s), 7.32-7.36(3H, m), 7.43(1H, d, J=8.7Hz), 7.52-7.55(2H, m), 7.82(1H, s), 8.16(1H, dd, J=8.7, 2.4Hz), 8.25(1H, d, J=2.4Hz), 8.31(2H, s), 10.89(1H, s).

### Example 59: Preparation of the compound of Compound No. 57.

5-Acetyl-N-[3,5-bis(trifluoromethyl)phenyl]-2-hydroxybenzamide(Compound No. 78; 50.5mg, 0.13mmol) was suspended in ethanol(2mL). Sodium borohydride(23.6mg, 0.62mmol) was added, and the mixture was stirred at room temperature for 12 hours. The reaction mixture was poured into diluted hydrochloric acid and extracted with ethyl acetate. After the organic layer was washed with water and brine, dried over anhydrous sodium sulfate, the residue obtained by evaporation under reduced pressure was washed with isopropyl ether/n-hexane under suspension to give the title compound(39.7mg, 78.3%) as a white powder.
¹H-NMR(DMSO-d₆): δ 1.34(3H, d, J=6.3Hz), 4.71(1H, q, J=6.3Hz), 5.18(1H, brs), 6.97(1H, d, J=8.4Hz), 7.44(1H, dd, J=8.4, 2.1Hz), 7.84(1H, s), 7.86(1H, d, J=2.1Hz), 8.48(2H, s), 10.85(1H, s), 11.32(1H, s).

### Example 60: Preparation of the compound of Compound No. 58.

5-Acetyl-N-[3,5-bis(trifluoromethyl)phenyl]-2-hydroxybenzamide(Compound No. 78; 100.0mg, 0.26mmol) was dissolved in ethanol(3mL). Pyridine(45µl, 0.56mmol) and O-methylhydroxylamine hydrochloride(25.8mg, 0.31mmol) were added, and the mixture was refluxed for 1 hour. After cooling, the reaction mixture was poured into diluted hydrochloric acid and extracted with ethyl acetate. After the organic layer was washed with water and brine, dried over anhydrous sodium sulfate, the residue obtained by evaporation under reduced pressure was purified by chromatography on silica gel(hexane:ethyl acetate=4:1) to give the title compound(102.1mg, 95.3%) as a white crystal.
¹H-NMR(DMSO-d₆): δ 2.19(3H, s), 3.91(3H, s), 7.05(1H, d, J=8.7Hz),7.77(1H, dd, J=8.7, 2.4Hz), 7.85(1H, s), 8.09(1H, d, J=2.4Hz), 8.47(2H, s), 10.87(1H, s), 11.48(1H, s).

### Example 61: Preparation of the compound of Compound No. 59.

Using 5-acetyl-N-[3,5-bis(trifluoromethyl)phenyl]-2-hydroxybenzamide(Compound No. 78) and O-benzylhydroxylamine hydrochloride as the raw materials, the same operation as the Example 60 gave the title compound.
Yield: 79.9%.
¹H-NMR(DMSO-d₆): δ 2.24(3H, s), 5.20(2H, s), 7.04(1H, d, J=8.7Hz), 7.29-7.47(5H, m), 7.76(1H, dd, J=8.7, 2.4Hz), 7.85(1H, s), 8.07(1H, d, J=2.1Hz), 8.46(2H, s), 10.87(1H, s), 11.47(1H, s).

### Example 62: Preparation of the compound of Compound No. 60.

### (1) 5-(2,2-Dicyanoethen-1-yl)-2-hydroxybenzoic acid.

Malononitrile(132mg, 2mmol) was dissolved in ethanol(6mL), and 5-formylsalicylic acid (332mg, 2mmol) was added. After cooling with ice bath, benzylamine(0.1mL) was added and the mixture was stirred at room temperature for 2 hours. The separated yellow crystal was filtered and recrystallized (ethanol) to give the title compound(139.9mg, 32.7%) as a light yellow solid.
¹H-NMR(DMSO-d₆): δ 7.12(1H, d, J=8.7Hz), 8.09(1H, dd, J=8.7, 2.4Hz), 8.41(1H, s), 8.50(1H, d, J=2.4Hz).

### (2) N-[3,5-Bis(trifluoromethyl)phenyl]-5-(2,2-dicyanoethen-1-yl)-2-hydroxybenzamide (Compound No. 60).

Using 5-(2,2-dicyanoethen-1-yl)-2-hydroxybenzoic acid and 3,5-bis(trifluoromethyl)aniline as the raw materials, the same operation as the Example 16 gave the title compound.
Yield: 9.1%.
¹H-NMR(DMSO-d₆): δ 7.13(1H, d, J=9.0Hz), 7.83(1H, s), 8.04(1H, dd, J=9.0, 2.4Hz), 8.36(1H, s), 8.38(1H, d, J=2.4Hz), 8.43(2H, s), 11.43(1H, s).

### Example 63: Preparation of the compound of Compound No. 62.

### (1) 5-[(2-Cyano-2-methoxycarbonyl)ethen-1-yl]-2-hydroxybenzoic acid.

Triethylamine(0.2ml) was added to a mixture of 5-formylsalicylic acid(332mg, 2mmol). Cyanoacetic acid methyl ester(198mg, 2mmol) and acetic acid(6mL), and the mixture was refluxed for 5 hours. After cooling, the reaction mixture was poured into water, and the separated crystal was filtered and recrystallized (n-hexane) to give the title compound(327.7mg, 66.3%) as a light yellow solid.
¹H-NMR(DMSO-d₆): δ 3.85(3H, s), 7.15(1H, d, J=8.7Hz), 8.20(1H, dd, J=8.7, 2.4Hz), 8.37(1H, s), 8.66(1H, d, J=2.4Hz).

### (2) 3-({N-[3,5-Bis(trifluoromethyl)phenyl]carbamoyl}-4-hydroxyphenyl)-2-cyanoacrylic acid methyl ester(Compound No. 62).

Using 5-[(2-cyano-2-methoxycarbonyl)ethen-1-yl]-2-hydroxybenzoic acid and 3,5-bis(trifluoromethyl)aniline as the raw materials, the same operation as the Example 16 gave the title compound.
Yield: 66.3%.
¹H-NMR(DMSO-d₆): δ 3.85(3H, s), 7.19(1H, d, J=9.0Hz), 7.85(1H, s), 8.20(1H, dd, J=8.7, 2.1Hz), 8.33(1H, s), 8.45(2H, s), 8.50(1H, d, J=2.1Hz), 11.00(1H, s), 11.03(1H, s).

### Example 64: Preparation of the compound of Compound No. 61.

3-({N-[3,5-Bis(trifluoromethyl)phenyl]carbamoyl}-4-hydroxyphenyl)-2-cyanoacrylic acid methyl ester(Compound No. 62; 50mg, 0.11mmol) was dissolved in ethanol(5mL). 2N Sodium hydroxide(0.11ml, 0.22mmol) was added, and the mixture was stirred at room temperature for 3 hours. The reaction mixture was poured into diluted hydrochloric acid and extracted with ethyl acetate. After the organic layer was washed with brine, dried over anhydrous magnesium sulfate, the residue obtained by evaporation under reduced pressure was recrystallized (ethyl acetate) to give the title compound(13.5mg, 30.4%) as a light yellow solid.
¹H-NMR(DMSO-d₆): δ 7.12(1H, d, J=8.4Hz), 7.84(1H, s), 7.94(1H, dd, J=8.4, 2.1Hz), 8.38(1H, d, J=2.1Hz), 8.45(2H, s), 9.87(1H, s), 11.41(1H, s).

### Example 65: Preparation of the compound of Compound No. 63.

A mixture of N-[3,5-bis(trifluoromethyl)phenyl]-2-hydroxy-5-iodobenzamide(Compound No. 52; 475mg, 1mmol), styrene(130mg, 1.25mmol), palladium acetate(4.5mg, 0.02mmol), tris(ortho-tolyl)phosphine(12.2mg, 0.04mmol), diisopropylamine(388mg, 3mmol) and N,N-dimethylformamide(2mL) was refluxed for 8 hours. After cooling, water was added to the reaction mixture, and it was extracted with ethyl acetate. After the ethyl acetate layer was washed with water and brine, dried over anhydrous magnesium sulfate and concentrated, the residue was purified by column chromatography on silica gel(n-hexane:isopropyl ether=2:1→1:1) to give the title compound(173mg, 38.3%) as a pale yellow solid.
¹H-NMR(DMSO-d₆): δ 7.04(1H, d, J=8.4Hz), 7.20-7.29(3H, m), 7.38(2H, t, J=7.5Hz), 7.59(2H, d, J=7.5Hz), 7.72(1H, dd, J=8.4, 2.1Hz), 7.86(1H, s), 8.07(1H, d, J=2.1Hz), 8.49(2H, s), 10.89(1H, s), 11.33(1H, brs).

### Example 66: Preparation of the compound of Compound No. 66.

N-[3,5-Bis(trifluoromethyl)phenyl]-2-hydroxy-5-iodobenzamide(Compound No. 52; 950mg, 2mmol) and trimethylsilylacetylene(246mg, 2.5mmol) were dissolved in triethylamine(2mL) and N,N-dimethylformamide(4mL).
Tetrakis(triphenylphosphine)palladium(23mg, 0.02mmol) and cuprous iodide(4mg, 0.02mmol) were added under argon atmosphere, and the mixture was stirred at 40°C for 2 hours. After cooling to room temperature, the reaction mixture was poured into ethyl acetate(100mL) and 1N citric acid(100mL), stirred, and filtered through celite. After the ethyl acetate layer was washed successively with water and brine, dried over anhydrous magnesium sulfate, the solvent was evaporated under reduced pressure. The obtained residue was purified by column chromatography on silica gel(n-hexane:ethyl acetate=19:1) to give a light orange solid. This was crystallized by n-hexane to give the title compound(286mg, 32.1%) as a white crystal.
¹H-NMR(DMSO-d₆): δ 0.23(9H, s), 7.00(1H, d, J=8.7Hz), 7.54(1H, dd, J=8.7, 2.4Hz), 7.85(1H, s), 7.98(1H, d, J=2.1Hz), 8.46(2H, s), 10.86(1H, s), 11.69(1H, s).

### Example 67: Preparation of the compound of Compound No. 64.

N-[3,5-Bis(trifluoromethyl)phenyl]-2-hydroxy-5-[(trimethylsilyl)ethynyl]benzamide(Compound No. 66; 233mg, 0.5mmol) was dissolved in methanol(1mL). 2N Sodium hydroxide(1mL) was added, and the mixture was stirred at room temperature for 1 hour. The reaction mixture was poured into 2N hydrochloric acid and extracted with ethyl acetate. After the ethyl acetate layer was washed successively with water and brine, dried over anhydrous magnesium sulfate, the solvent was evaporated under reduced pressure. The obtained residue was crystallized from ethanol-water to give the title compound(67mg, 35.9%) as a light gray crystal.
¹H-NMR(DMSO-d₆): δ 4.11(1H, s), 7.02(1H, d, J=8.4Hz), 7.55(1H, dd, J=8.4, 2.1Hz), 7.85(1H, s), 7.98(1H, d, J=2.1Hz), 8.46(2H, s), 8.46(2H, s), 10.86(1H, s), 11.62(1H, s).

### Example 68: Preparation of the compound of Compound No. 65.

Using N-[3,5-bis(trifluoromethyl)phenyl]-2-hydroxy-5-iodobenzamide (Compound No. 52) and phenylacetylene as the raw materials, the same operation as the Example 66 gave the title compound.
Yield: 40.8%.
¹H-NMR(DMSO-d₆): δ7.06(1H, d, J=8.4Hz), 7.42-7.46(3H, m), 7.53-7.57(2H, m), 7.64(1H, dd, J=8.7, 2.1Hz), 7.86(1H, s), 8.06(1H, d, J=2.1Hz), 8.48(2H, s), 10.94(1H, s), 11.64(1H, brs).

### Example 69: Preparation of the compound of Compound No. 67.

N-[3,5-Bis(trifluoromethyl)phenyl]-2-hydroxy-5-iodobenzamide(Compound No. 52; 200mg, 0.42mmol) was dissolved in 1,2-dimethoxyethane(3mL).
Tetrakis(triphenylphosphine)palladium(16mg, 0.0014mmol) was added under argon atmosphere, and the mixture was stirred at room temperature for 5 minutes. Then dihydroxyphenylborane(57mg, 0.47mmol) and 1M sodium carbonate(1.3mL) were added and the mixture was refluxed for 2 hours. After cooling to room temperature, the reaction mixture was poured into diluted hydrochloric acid and extracted with ethyl acetate. After the ethyl acetate layer was washed successively with water and brine, dried over anhydrous sodium sulfate, the solvent was evaporated under reduced pressure. The obtained residue was purified by column chromatography on silica gel(n-hexane:ethyl acetate=6:1→3:1) to give the title compound(109mg, 61.1%) as a white crystal.
¹H-NMR(DMSO-d₆): δ 7.12(1H, d, J=8.7Hz), 7.33-7.38(1H, m), 7.48(2H, t, J=7.5Hz), 7.67-7.70(2H, m), 7.79(1H, dd, J=8.4, 2.4Hz), 7.87(1H, s), 8.17(1H, d, J=2.4Hz), 8.49(2H, s), 10.92(1H, s), 11.41(1H, s).

### Example 70: Preparation of the compound of Compound No. 68.

Using N-[3,5-bis(trifluoromethyl)phenyl]-2-hydroxy-5-(phenylethynyl)benzamide (Compound No. 65) as the raw material, the same operation as the Example 58(4) gave the title compound.
Yield: 86.2%.
¹H-NMR(DMSO-d₆): δ 2.88(4H, s), 6.93(1H, d, J=8.1Hz), 7.15-7.34(6H, m), 7.76(1H, d, J=2.4Hz), 7.84(1H, s), 8.47(2H, s), 10.79(1H, s), 11.15(1H, s).

### Example 71: Preparation of the compound of Compound No. 69.

Using 2-hydroxy-5-(trifluoromethyl)benzoic acid and 3,5-bis(trifluoromethyl)aniline as the raw materials, the same operation as the Example 16 gave the title compound.
Yield: 44.7%.
¹H-NMR(CDCl₃): δ 7.17(1H, d, J=9.0Hz) 7.72-7.75(2H, m), 7.86(1H, s), 8.17(2H, s), 8.35(1H, s) 11.88(1H, s).

### [2-Hydroxy-5-(trifluoromethyl)benzoic acid: Refer to "Chemical and Pharmaceutical Bulletin", 1996, Vol.44, p.734.]

### Example 72: Preparation of the compound of Compound No. 70.

Using 2-hydroxy-5-(pentafluoroethyl)benzoic acid and 3,5-bis(trifluoromethyl)aniline as the raw materials, the same operation as the Example 16 gave the title compound.
¹H-NMR(CDCl₃): δ7.19(1H, d, J=9.0Hz) 7.70(1H, dd, J=8.7, 2.1Hz), 7.81(1H, d, J=2.1Hz), 8.17(2H, s), 8.37(1H, s), 11.92(1H, s).

### [2-Hydroxy-5-(pentafluoromethyl)benzoic acid: Refer to "Chemical and Pharmaceutical Bulletin", 1996, Vol.44, p.734.]

### Example 73: Preparation of the compound of Compound No. 71.

Using 2-hydroxy-5-(pyrrol-1-yl)benzoic acid and 3,5-bis(trifluoromethyl)aniline as the raw materials, the same operation as the Example 16 gave the title compound.
Yield: 57.8%.
¹H-NMR(DMSO-d₆): δ 6.27(2H, dd, J=2.4, 1.8Hz), 7.10(1H, d, J=9.0Hz), 7.29(2H, dd, J=2.4, 1.8Hz), 7.66(1H, dd, J=9.0, 2.7Hz), 7.86(1H, s), 7.98(1H, d, J=2.4Hz), 8.47(2H, s), 10.89(1H, s), 11.24(1H, s).

### Example 74: Preparation of the compound of Compound No. 72.

Using N-[3,5-bis(trifluoromethyl)phenyl]-2-hydroxy-5-iodobenzamide (Compound No. 52) and 2-thiopheneboronic acid as the raw materials, the same operation as the Example 69 gave the title compound.
Yield: 44.4%.
¹H-NMR(DMSO-d₆): δ 7.08(1H, d, J=8.4Hz), 7.14(1H, dd, J=5.4, 3.6Hz), 7.45(1H, dd, J=3.6, 1.2Hz), 7.51(1H, dd, J=5.1, 0.9Hz), 7.75(1H, dd, J=8.4, 2.4Hz), 7.59(1H, s), 8.08(1H, d, J=2.4Hz), 8.48(2H, s), 10.91(1H, s), 11.38(1H, s).

### Example 75: Preparation of the compound of Compound No. 73.

Using N-[3,5-bis(trifluoromethyl)phenyl]-2-hydroxy-5-iodobenzamide (Compound No. 52) and 3-thiopheneboronic acid as the raw materials, the same operation as the Example 69 gave the title compound.
Yield: 38.7%.
¹H-NMR(DMSO-d₆): δ 7.06(1H, d, J=8.7Hz), 7.57(1H, dd, J=4.8, 1.5Hz), 7.66(1H, dd, J=4.8, 3.0Hz), 7.81-7.84(2H, m), 7.86(1H, s), 8.18(1H, d, J=2.1Hz), 8.49(2H, s), 10.90(1H, s), 11.33(1H, s).

### Example 76: Preparation of the compound of Compound No. 74.

### (1) 2-Benzyloxy-5-(2-bromoacetyl)-N-[3,5-bis(trifluoromethyl)phenyl]benzamide.

5-Acetyl-2-benzyloxy-N-[3,5-bis(trifluoromethyl)phenyl]benzamide(compound of Example 58(3); 4.81g, 10mmol) was dissolved in tetrahydrofuran(30ml). Phenyltrimethylammonium tribromide(3.75g, 10mmol) was added, and the mixture was stirred at room temperature for 12 hours. The reaction mixture was poured into water and extracted with ethyl acetate. After the organic layer was washed with aqueous sodium hydrogen sulfite, water and brine, dried over anhydrous magnesium sulfate, the residue obtained by evaporation under reduced pressure was purified by chromatography on silica gel(n-hexane:ethyl acetate=4:1), and recrystallized(ethyl acetate/n-hexane) to give the title compound(2.39g, 42.7%) as a white solid.
¹H-NMR(DMSO-d₆): δ4.91(2H, s), 5.36(2H, s), 7.32-7.35(3H, m), 7.47(1H, d, J=9.0Hz), 7.52-7.56(2H, m), 7.82(1H, s), 8.21(1H, dd, J=8.7, 2.4Hz), 8.29(1H, d, J=2.4Hz), 8.31(2H, s), 10.91(1H, s).

### (2) 2-Benzyloxy-N-[3,5-bis(trifluoromethyl)phenyl]-5-(2-methylthiazol-4-yl)benzamide.

A mixture of 2-benzyloxy-5-(2-bromoacetyl)-N-[3,5-bis(trifluoromethyl)phenyl]benzamide(280mg, 0.5mmol), thioacetamide(41mg, 0.55mmol), sodium hydrogen carbonate(50mg, 0.6mmol) and ethanol(15mL) was refluxed for 1 hour. The reaction mixture was poured into water, neutralized by sodium hydrogen carbonate, and extracted with ethyl acetate. After the organic layer was washed with water and brine, dried over anhydrous magnesium sulfate, the residue obtained by evaporation under reduced pressure was purified by chromatography on silica gel(hexane:ethyl acetate=4:1) to give the title compound(181mg, 67.5%) as a white solid.
¹H-NMR(DMSO-d₆): δ 2.72(3H, s), 5.29(2H, s), 7.33-7.36(3H, m), 7.40(1H, d, J=9.0Hz), 7.54-7.57(2H, m), 7.81(1H, s), 7.94(1H, s), 8.12(1H, dd, J=8.7, 2.1Hz), 8.27(1H, d, J=2.1Hz), 8.31(2H, s), 10.86(1H, s).

### (3) N-[3,5-Bis(trifluoromethyl)phenyl]-2-hydroxy-5-(2-methylthiazol-4-yl)benzamide (Compound No. 74).

2-Benzyloxy-N-[3,5-bis(trifluoromethyl)phenyl]-5-(2-methylthiazol-4-yl)benzamide(160mg, 0.3mmol) and 10% Pd-C(240mg) were dissolved in ethanol(10ml) and stirred for 3.5 hours under hydrogen atmosphere. The reaction mixture was filtered and the filtrate was evaporated under reduced pressure to give the title compound(103.4mg, 79.2%) as a white solid.
¹H-NMR(DMSO-d₆): δ 2.72(3H, s), 7.08(1H, d, J=8.7Hz), 7.83(1H, s), 7.85(1H, s), 8.01(1H, dd, J=8.7, 2.4Hz), 8.42(1H, d, J=2.1Hz), 8.50(2H, s), 10.96(1H, s), 11.40(1H, s).

### Example 77: Preparation of the compound of Compound No. 75.

A mixture of 2-benzyloxy-5-(2-bromoacetyl)-N-[3,5-bis(trifluoromethyl)phenyl]benzamide (compound of Example 58(3); 280mg, 0.5mmol), 2-aminopyridine(51.8mg, 0.55mmol), sodium hydrogen carbonate(50mg, 0.6mmol) and ethanol(10mL) was refluxed for 2 hours. After cooling, the reaction mixture was poured into aqueous sodium hydrogen carbonate and extracted with ethyl acetate. After the organic layer was washed with water and brine, dried over anhydrous magnesium sulfate, the residue obtained by evaporation under reduced pressure was purified by chromatography on silica gel(n-hexane:ethyl acetate=1:2) to give a white solid(130.3mg, 45.9%). Then, a mixture of this solid(108mg, 0.19mmol), 10% Pd-C(11mg), ethanol(8mL) and ethyl acetate(8mL) was stirred for 7 hours under hydrogen atmosphere. The reaction mixture was filtered and the residue obtained by evaporation of the filtrate under reduced pressur was purified by chromatography on silica gel(n-hexane:ethyl acetate=1:3) to give the title compound(18.3mg, 20.2%) as a white solid.
¹H-NMR(DMSO-d₆): δ 6.90(1H, dt, J=6.6, 0.9Hz), 7.10(1H, d, J=8.7Hz), 7.25(1H, m), 7.57(1H, d, J=9.0Hz), 7.86(1H, s), 8.04(1H, dd, J=8.7, 2.1Hz), 8.35(1H, s), 8.48-8.56(4H, m), 11.00(1H, s), 11.41(1H, s).

### Example 78: Preparation of the compound of Compound No. 76.

### (1) N-[3,5-Bis(trifluoromethyl)phenyl]-5-iodo-2-methoxymethoxybenzamide.

A mixture of N-[3,5-bis(trifluoromethyl)phenyl]-2-hydroxy-5-iodobenzamide(Compound No. 52; 4.75g, 10mmol), chloromethyl methyl ether(1.14ml, 15mmol), potassium carbonate(2.76g, 20mmol) and acetone(50mL) was refluxed for 8 hours. The reaction mixture was poured into diluted hydrochloric acid and extracted with ethyl acetate. After the organic layer was washed with water and brine, dried over anhydrous magnesium sulfate, the residue obtained by evaporation under reduced pressure was purified by chromatography on silica gel(n-hexane:ethyl acetate=3:1), and recrystallized(n-hexane/ethyl acetate) to give the title compound(3.96g, 76.3%) as a white solid.
¹H-NMR(DMSO-d₆): δ 3.38(3H, s), 5.28(2H, s), 7.12(1H, d, J=9.0Hz), 7.81(1H, s), 7.82(1H, dd, J=8.7, 2.4Hz), 7.88(1H, d, J=2.4Hz), 8.40(2H, s), 10.87(1H, s).

### (2) N-[3,5-Bis(trifluoromethyl)phenyl]-2-methoxymethoxy-5-(pyridin-2-yl)benzamide.

N-[3,5-Bis(trifluoromethyl)phenyl]-5-iodo-2-methoxymethoxybenzamide(0.20g, 0.39mmol) was dissolved in N,N-dimethylformamide(8ml). Tri-n-butyl(2-pyridyl)tin (0.13ml, 0.41mmol) and dichlorobis(triphenylphosphine)palladium(32.1mg, 0.05mmol) were added, and the mixture was stirred at 100°C for 1.5 hours. After cooling, the reaction mixture was poured into water and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=2:1→1:1) to give the title compound(37.9mg, 20.8%) as a white powder.
¹H-NMR(CDCl₃): δ 3.64(3H, s), 5.53(2H, s), 7.23-7.28(1H, m),7.36(1H, d, J=8.7Hz), 7.65(1H, s), 7.77-7.84(2H, m), 8.20(2H, s), 8.31(1H, dd, J=8.7, 2.4Hz), 8.68-8.70(1H, m), 8.83(1H, d, J=2.4Hz), 10.12(1H, s).

### (3) N-[3,5-Bis(trifluoromethyl)phenyl]-2-hydroxy-5-(pyridin-2-yl)benzamide (Compound No. 76).

Methanol(3ml) and concentrated hydrochloric acid(0.5ml) were added to N-[3,5-bis(trifluoromethyl)phenyl]-2-methoxymethoxy-5-(pyridin-2-yl)benzamide(37.9 mg, 0.08mmol), and the mixture was refluxed for 2 hours. After cooling, the reaction mixture was poured into saturated aqueous sodium hydrogen carbonate and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=2:1) to give the title compound(16.2mg, 47.2%) as a white powder.
¹H-NMR(DMSO-d₆): δ 7.13(1H, d, J=8.4Hz), 7.33(1H, ddd, J=7.5, 6.3, 1.2Hz), 7.86-7.91(2H, m), 7.97(1H, d, J=7.8Hz), 8.20(1H, dd, J=8.7, 2.1Hz), 8.50(2H, s), 8.59(1H, d, J=2.4Hz), 8.64-8.66(1H, m), 10.97(1H, s), 11.53(1H, s).

### Example 79: Preparation of the compound of Compound No. 77.

Using 5-methoxysalicylic acid and 3,5-bis(trifluoromethyl)aniline as the raw materials, the same operation as the Example 16 gave the title compound.
Yield: 56.8%.
¹H-NMR(DMSO-d₆): δ 3.77(3H, s), 6.97(1H, d, J=9.0Hz), 7.10(1H, dd, J=9.0, 3.0Hz), 7.43(1H, d, J=3.0Hz), 7.84(1H, s), 8.47(2H, s), 10.84(1H, s), 10.91(1H, s).

### Example 80: Preparation of the compound of Compound No. 79.

### (1) 5-Acetyl-2-methoxybenzoic acid methyl ester.

A mixture of 5-acetylsalicylic acid methyl ester(5.00g, 25.7mmol), sodium carbonate(7.10g, 51.4mmol) and N,N-dimethylformamide(25mL) was cooled with ice bath. Methyl iodide(2.5mL, 40.1mmol) was added, and the mixture was stirred at room temperature for 3 hours. The reaction mixture was poured into water, neutralized by hydrochloric acid, and extracted with ethyl acetate. After the organic layer was washed with water and brine, dried over anhydrous sodium sulfate, the residue obtained by evaporation under reduced pressure was washed under suspension(isopropyl ether/n-hexane) to give the title compound(5.17g, 96.5%) as a white crystal.
¹H-NMR(CDCl₃): δ2.59(3H, s), 3.92(3H, s), 3.99(3H, s), 7.04(1H, d, J=8.7Hz), 8.12(1H, dd, J=8.7, 2.4Hz), 8.41(1H, d, J=2.4Hz).

### (2) 5-Isobutyryl-2-methoxybenzoic acid methyl ester.

A mixture of 5-acetyl-2-methoxybenzoic acid methyl ester(0.50g, 2.40mmol), potassium tert-butoxide(0.81g, 7.22mmol) and tetrahydrofuran(10mL) was cooled with ice bath. Methyl iodide(0.5mL, 8.03mmol) was added, and the mixture was stirred at room temperature for 1 hour. The reaction mixture was poured into water, neutralized by hydrochloric acid, and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=3:1→2:1) to give the title compound(143.1mg, 25.2%) as a light yellow oil.
¹H-NMR(CDCl₃): δ 1.22(6H, d, J=6.9Hz), 3.52(1H, m), 3.92(3H, s), 3.98(3H, s), 7.05(1H, d, J=8.7Hz), 8.13(1H, dd, J=8.7, 2.4Hz), 8.42(1H, d, J=2.4Hz).

### (3) 5-Isobutyryl-2-methoxybenzoic acid.

5-Isobutyryl-2-methoxybenzoic acid methyl ester(143.1mg, 0.60mmol) was dissolved in methanol(5mL). 2N Aqueous sodium hydroxide(1ml) was added, and the mixture was refluxed for 1 hour. After cooling, the reaction mixture was poured into 2N hydrochloric acid and extracted with ethyl acetate. The organic layer was washed with water and brine, dried over anhydrous sodium sulfate, and evaporated under reduced pressure to give the title compound(134mg, yield: quantitative) as a white crystal.
¹H-NMR(CDCl₃): δ 1.22(6H, d, J=6.9Hz), 3.59(1H, m), 4.15(3H, s), 7.16(1H, d, J=8.7Hz), 8.24(1H, dd, J=8.7, 2.4Hz), 8.73(1H, d, J=2.1Hz).

### (4) 5-Isobutyryl-N-[3,5-bis(trifluoromethyl)phenyl]-2-methoxybenzamide.

Using 5-isobutyryl-2-methoxybenzoic acid and 3,5-bis(trifluoromethyl)aniline as the raw materials, the same operation as the Example 16 gave the title compound.
Yield: 61.4%.
¹H-NMR(CDCl₃): δ 1.23(6H, d, J=6.9Hz), 3.64(1H, m), 4.20(3H, s), 7.18(1H, d, J=8.7Hz), 7.65(1H, s), 8.19(2H, s), 8.22(1H, dd, J=8.7, 2.1Hz), 8.88(1H, d, J=2.1Hz), 9.98(1H, s).

### (5) N-[3,5-Bis(trifluoromethyl)phenyl]-2-hydroxy-5-isobutyrylbenzamide(Compound No. 79).

A mixture of 5-isobutyryl-N-[3,5-bis(trifluoromethyl)phenyl]-2-methoxybenzamide (143.4mg, 0.33mmol), 2,4,6-collidine(3ml) and lithium iodide(53.1mg, 0.40mmol) was refluxed for 1 hour. After cooling, the reaction mixture was poured into 2N hydrochloric acid and extracted with ethyl acetate. After the organic layer was washed with brine, dried over anhydrous sodium sulfate, the residue obtained by evaporation under reduced pressure was purified by chromatography on silica gel(n-hexane:ethyl acetate=3:1) and crystallized(ethyl acetate/isopropyl ether) to give the title compound(90.3mg, 65.3%) as a white crystal.
¹H-NMR(DMSO-d₆): δ 1.12(6H, d, J=6.9Hz), 3.66(1H, m), 7.12(1H, d, J=8.4Hz), 7.85(1H, s), 8.07(1H, dd, J=8.4, 2.4Hz), 8.45(1H, d, J=2.4Hz), 8.47(2H, s), 10.93(1H, s), 11.95(1H, brs).

### Example 81: Preparation of the compound of Compound No. 81.

Using 4-hydroxyisophthalic acid 1-methyl ester and 3,5-bis(trifluoromethyl)aniline as the raw materials, the same operation as the Example 16 gave the title compound.
Yield: 91.5%.
¹H-NMR(DMSO-d₆): δ 3.85(3H, s), 7.12(1H, d, J=8.4Hz), 7.86(1H, s), 8.02(1H, dd, J=8.7, 2.4Hz), 8.46-8.47(3H, m), 10.96(1H, s), 12.03(1H, brs).

### [4-Hydroxyisophthalic acid 1-methyl ester: Refer to "Journal of the Chemical Society", (England), 1956, p.3099-3107.]

### Example 82: Preparation of the compound of Compound No. 80.

N-[3,5-Bis(trifluoromethyl)phenyl]-4-hydroxyisophthalamic acid methyl ester(Comound No. 81; 2.85g, 7mmol) was suspended in a mixed solvent of methanol(14mL) and tetrahydrofuran(14mL). 2N Aqueous sodium hydroxide(14mL) was added, and the mixture was refluxed for 2 hours. After cooling, 2N hydrochloric acid(20ml) was added to the reaction mixture and the separated solid was filtered, washed with water, dried to give the title compound(2.68g, 97.4%) as a white crystal.
¹H-NMR(DMSO-d₆): δ 7.10(1H, d, J=8.7Hz), 7.82(1H, s), 7.86(1H, s), 8.01(1H, dd, J=8.7, 2.4Hz), 8.47(2H, s), 8.48(1H, d, J=2.4Hz), 10.97(1H, s), 11.98(1H, brs).

When the method described in Example 82 is referred in the following examples, inorganic bases such as sodium hydroxide, potassium carbonate or the like were used as the base. As the reaction solvent, solvents such as water, methanol, ethanol, tetrahydrofuran or the like were used alone or as a mixture.

### Example 83: Preparation of the compound of Compound No. 82.

Using 4-hydroxyisophthalic acid(182mg, 1mmol), 3,5-bis(trifluoromethyl)aniline(687mg, 3mmol), phosphorus trichloride(87 µl; 1mmol) and toluene(10mL), the same operation as the Example 16 gave the title compound(151mg, 25.0%) as a white crystal.
¹H-NMR(DMSO-d₆): δ 7.18(1H, d, J=8.7Hz), 7.82(1H, s), 7.86(1H, s), 8.11(1H, dd, J=8.7, 2.4Hz), 8.50(2H, s), 8.54(2H, s), 8.56(1H, d, J=2.4Hz), 10.79(1H, s), 10.99(1H, s), 11.84(1H, brs).

### Example 84: Preparation of the compound of Compound No. 83.

### (1) 4-Benzyloxy-N-[3,5-bis(trifluoromethyl)phenyl]isophthalamic acid methyl ester.

Sodium hydride(60%; 1.04g, 26mmol) was washed with n-hexane, and suspended in N,N-dimethylformamide(100mL). A solution of N-[3,5-bis(trifluoromethyl)phenyl]-4-hydroxyisophthalamic acid methyl ester(Compound No. 81; 8.15g, 20mmol) in N,N-dimethylformamide(100mL) was added dropwise under cooling with ice bath. After the addition was finished, the mixture was stirred at room temperature for 1 hour. A solution of benzyl bromide(4.45g, 26mmol) in N,N-dimethylformamide(10mL) was added, and the mixture was stirred at 60°C for 3 hours. After cooling, the reaction mixture was poured into ice and water, and extracted with ethyl acetate. After the organic layer was washed with water and brine, dried over anhydrous magnesium sulfate, the residue obtained by evaporation under reduced pressure was recrystallized(ethyl acetate/n-hexane) to give the title compound(5.38g, 54.1%) as a white solid.
¹H-NMR(DMSO-d₆): δ 3.87(3H, s), 5.33(2H, s), 7.33-7.36(3H, m), 7.46(1H, d, J=8.7Hz), 7.53-7.56(2H, m), 7.82(1H, s), 8.15(1H, dd, J=8.7, 2.1Hz), 8.25(1H, d, J=2.1Hz)8.28(2H, s), 10.87(1H, s).

### (2) 4-Benzyloxy-N-[3,5-bis(trifluoromethyl)phenyl]isophthalamic acid.

Using 4-benzyloxy-N-[3,5-bis(trifluoromethyl)phenyl]isophthalamic acid methyl ester as the raw material, the same operation as the Example 82 gave the title compound.
Yield: 79.7%.
¹H-NMR(DMSO-d₆): δ 5.32(2H, s), 7.32-7.34(3H, m), 7.43(1H, d, J=8.7Hz), 7.52-7.56(2H, m), 7.81(1H, s), 8.12(1H, dd, J=8.7, 2.1Hz), 8.22(1H, d, J=2.1Hz), 8.28(2H, s), 10.85(1H, s), 13.81(1H, brs).

### (3) 4-Benzyloxy-N³-[3,5-bis(trifluoromethyl)phenyl]-N¹,N¹-dimethylisophthalamide.

WSC · HCl(95mg, 0.50mmol) was added to a solution of 4-benzyloxy-N-[3,5-bis(trifluoromethyl)phenyl]isophthalamic acid(242mg, 0.50mmol), dimethylamine hydrochloride(41mg, 0.50mmol) and triethylamine(51mg, 0.50mmol) in tetrahydrofuran(5mL) under ice cooling, and the mixture was stirred at room temperature for 3 hours. The reaction mixture was poured into water and extracted with ethyl acetate. After the organic layer was washed with diluted hydrochloric acid, water and brine, dried over anhydrous magnesium sulfate, the residue obtained by evaporation of the solvent under reduced pressure was purified by chromatography on silica gel(hexane:ethyl acetate=1:4) to give the title compound(165mg, 64.9%) as a white solid.
¹H-NMR(DMSO-d₆): δ 2.99(6H, s)5.29(2H, s), 7.32-7.38(4H, m), 7.52-7.56(2H, m), 7.64(1H, dd, J=8.7, 2.1Hz), 7.73(1H, d, J=2.1Hz), 7.80(1H, s), 8.28(2H, s), 10.83(1H, s).

When the method described in Example 84(3) is referred in the following examples, organic bases such as pyridine, triethylamine or the like were used as the base. As the reaction solvent, solvents such as dichloromethane, tetrahydrofuran or the like were used alone or as a mixture.

### (4) N³-[3,5-bis(trifluoromethyl)phenyl]-4-hydroxy-N¹,N¹-dimethylisophthalamide (Compound No. 83).

A solution of 4-benzyloxy-N³-[3,5-bis(trifluoromethyl)phenyl]-N¹,N¹-dimethylisophthalamide(141mg, 0.28mmol) and 5% Pd-C(14mg) in a mixed solvent of ethanol(5ml) and ethyl acetate(5ml) was stirred at room temperature for 1 hour under hydrogen atmosphere. The reaction mixture was filtered and the filtrate was evaporated under reduced pressure to give the title compound(106mg, 91.2%) as a white solid.
¹H-NMR(DMSO-d₆): δ 2.98(6H, s), 7.02(1H, d, J=8.7Hz), 7.52(1H, dd, J=8.7, 2.1Hz), 7.84(1H, s), 7.95(1H, d, J=2.1Hz), 8.46(2H, s), 11.10(1H, brs), 11.63(1H, brs).

### Example 85: Preparation of the compound of Compound No. 84.

### (1) 2-Benzyloxy-N-[3,5-bis(trifluoromethyl)phenyl]-5-(piperidine-1-carbonyl)benzamide.

Using 4-benzyloxy-N-[3,5-bis(trifluoromethyl)phenyl]isophthalamic acid(compound of Example 84(2)) and piperidine as the raw materials, the same operation as the Example 84(3) gave the title compound.
Yield: 56.4%.
¹H-NMR(CDCl₃): δ 1.53-1.70(6H, m), 3.44(2H, brs), 3.70(2H, brs), 5.26(2H, s), 7.24(1H, d, J=8.7Hz), 7.26(1H, s), 7.52-7.58(5H, m), 7.66(2H, s), 7.74(1H, dd, J=8.7, 2.4Hz), 8.37(1H, d, J=2.1Hz), 10.27(1H, s).

### (2) N-[3,5-Bis(trifluoromethyl)phenyl]-2-hydroxy-5-(piperidine-1-carbonyl)benzamide (Compound No. 84).

Using 2-benzyloxy-N-[3,5-bis(trifluoromethyl)phenyl]-5-(piperidine-1-carbonyl)benzamide as the raw material, the same operation as the Example 84(4) gave the title compound.
Yield: 96.3%, white solid.
¹H-NMR(DMSO-d₆): δ 1.51(4H, brs), 1.60-1.65(2H, m), 3.47(4H, brs), 7.04(1H, d, J=8.4Hz), 7.48(1H, dd, J=8.4, 2.1Hz), 7.85(1H, s), 7.92(1H, d, J=2.1Hz), 8.46(2H, s), 10.99(1H, s), 11.64(1H, brs).

### Example 86: Preparation of the compound of Compound No. 85.

### (1) 2-Benzyloxy-5-(4-benzylpiperidine-1-carbonyl)-N-[3,5-bis(trifluoromethyl)phenyl]benzamide.

Using 4-benzyloxy-N-[3,5-bis(trifluoromethyl)phenyl]isophthalamic acid(compound of Example 84(2)) and 4-benzylpiperidine as the raw materials, the same operation as the Example 84(3) gave the title compound.
Yield: 76.7%.
¹H-NMR(CD₃OD): δ1.18-1.38(2H, m), 1.67(1H, brs), 1.74(1H, brs), 1.84-1.93(1H, m), 2.60(2H, d, J=7.2Hz), 2.83(1H, brs), 3.10(1H, brs), 3.78(1H, brs), 4.59(1H, brs), 5.34(2H, s), 7.15-7.18(3H, m), 7.24-7.28(2H, m), 7.40-7.46(4H, m), 7.57-7.63(3H, m), 7.65(1H, dd, J=8.7, 2.4Hz), 7.96(2H, s), 8.05(1H, d, J=2.1Hz).

### (2) N-[3,5-Bis(trifluoromethyl)phenyl]-2-hydroxy-5-(4-benzylpiperidine-1-carbonyl)benzamide(Compound No. 85).

Using 2-benzyloxy-5-(4-benzylpiperidine-1-carbonyl)-N-[3,5-bis(trifluoromethyl)phenyl]-benzamide as the raw material, the same operation as the Example 84(4) gave the title compound.
Yield: 54.3%, white solid.
¹H-NMR(DMSO-d₆): δ1.08-1.22(2H, m), 1.59-1.62(2H, m), 1.77-1.80(1H, m), 2.50-2.55(2H, m), 2.87(2H, brs), 3.75(1H, br), 4.39(1H, br), 7.06(1H, d, J=8.4Hz), 7.17-7.20(3H, m), 7.28(2H, t, J=7.2Hz), 7.49(1H, dd, J=8.4, 2.1Hz), 7.84(1H, s), 7.93(1H, d, J=2.1Hz), 8.47(2H, s), 10.89(1H, s), 11.65(1H, s).

### Example 87: Preparation of the compound of Compound No. 86.

### (1) 2-Methoxy-5-sulfamoylbenzoic acid.

Methyl 2-methoxy-5-sulfamoylbenzoate(4.91g, 20mmol) was dissolved in methanol(30mL). 2N Aqueous sodium hydroxide(30mL, 60mmol) was added, and the mixture was stirred at room temperature for 1 hour. The reaction mixture was poured into 2N hydrochloric acid, and the separated solid was filtered to give the title compound(4.55g, 98.3%) as a white solid.
¹H-NMR(DMSO-d₆): δ 3.89(3H, s), 7.30(1H, d, J=8.7Hz), 7.32(2H, s), 7.92(1H, dd, J=8.7, 2.7Hz), 8.09(1H, d, J=2.7Hz), 13.03(1H, br).

### (2) N-[3,5-Bis(trifluoromethyl)phenyl]-2-methoxy-5-sufamoylbenzamide.

Using 2-methoxy-5-sulfamoylbenzoic acid and 3,5-bis(trifluoromethyl)aniline as the raw materials, the same operation as the Example 24 gave the title compound.
Yield: 24.2%.
¹H-NMR(DMSO-d₆): δ 3.97(3H, s), 7.38(2H, s), 7.39(1H, d, J=8.7Hz), 7.85(1H, s), 7.96(1H, dd, J=8.7, 2.4Hz), 8.06(1H, d, J=2.4Hz), 8.43(2H, s), 10.87(1H, s).

### (3) N-[3,5-Bis(trifluoromethyl)phenyl]-5-dimethylsufamoyl-2-methoxybenzamide.

A suspension of N-[3,5-bis(trifluoromethyl)phenyl]-2-methoxy-5-sufamoylbenzamide(442mg, 1.0mmol), methyl iodide(710mg, 5.0mmol) and sodium carbonate(415mg, 3.0mmol) in acetonitrile(10mL) was refluxed for 3 hours. After cooling to room temperature, the reaction mixture was poured into water and extracted with ethyl acetate. After the organic layer was washed with water and brine, dried over anhydrous magnesium sulfate, the residue obtained by evaporation of the solvent under reduced pressure was recrystallized from a mixed solvent of n-hexane and ethyl acetate(2:1) to give the title compound(207mg, 44.1%) as a white solid.
¹H-NMR(DMSO-d₆): δ 2.62(6H, s), 3.99(3H, s), 7.45(1H, d, J=9.0Hz), 7.85(1H, s), 7.91(1H, dd, J=8.7, 2.4Hz), 7.95(1H, d, J=2.4Hz)8.43(2H, s), 10.90(1H, s).

### (4) N-[3,5-Bis(trifluoromethyl)phenyl]-5-dimethylsufamoyl-2-hydroxybenzamide (Compound No. 86).

Using N-[3,5-bis(trifluoromethyl)phenyl]-5-dimethylsufamoyl-2-methoxybenzamide as the raw material, the same operation as the Example 80(5) gave the title compound.
Yield: 45.5%.
¹H-NMR(DMSO-d₆): δ 2.61(6H, s), 7.20(1H, d, J=8.7Hz), 7.77(1H, dd, J=8.7, 2.1Hz), 7.86(1H, s), 8.14(1H, d, J=2.1Hz)8.45(2H, s), 11.16(1H, s), 12.15(1H, br).

### Example 88: Preparation of the compound of Compound No. 87.

### (1) N-[3,5-Bis(trifluoromethyl)phenyl]-2-methoxy-5-(pyrrole-1-sulfonyl)benzamide.

A mixture of N-[3,5-bis(trifluoromethyl)phenyl]-2-methoxy-5-sulfamoylbenzamide(compound of Example 87(2); 442mg, 1mmol), 2,5-dimethoxytetrahydrofuran(159mg, 1.2mmol) and acetic acid(5mL) was refluxed for 2 hours. After cooling, the reaction mixture was poured into water and extracted with ethyl acetate. After the organic layer was washed with water, saturated aqueous sodium hydrogen carbonate and brine, dried over anhydrous magnesium sulfate, the residue obtained by evaporation of the solvent under reduced pressure was purified by chromatography on silica gel(n-hexane:ethyl acetate=3:2) to give the title compound(436.5mg, 88.6%) as a white solid.
¹H-NMR(DMSO-d₆): δ 3.96(3H, s), 6.36(2H, dd, J=2.4, 2.1Hz), 7.37(2H, dd, J=2.4, 2.1Hz), 7.42(1H, d, J=9.0Hz), 7.85(1H, s), 8.80(1H, dd, J=9.0, 2.4Hz)8.18(1H, d, J=2.7Hz), 8.38(2H, s), 10.92(1H, s).

### (2) N-[3,5-Bis(trifluoromethyl)phenyl]-2-hydroxy-5-(pyrrole-1-sulfonyl)benzamide (Compound No. 87).

Using N-[3,5-bis(trifluoromethyl)phenyl]-2-methoxy-5-(pyrrole-1-sulfonyl)benzamide as the raw material, the same operation as the Example 80(5) gave the title compound.
Yield: 79.4%.
¹H-NMR(DMSO-d₆): δ 6.36(2H, dd, J=2.4, 2.1Hz), 7.18(1H, d, J=9.0Hz), 7.34(2H, dd, J=2.4, 2.1Hz), 7.86(1H, s), 7.99(1H, dd, J=9.0, 2.7Hz)8.31(1H, d, J=2.7Hz), 8.42(2H, s), 10.98(1H, s).

### Example 89: Preparation of the compound of Compound No. 88.

Using N-[3,5-bis(trifluoromethyl)phenyl]-2-hydroxy-5-nitrobenzamide (Compound No. 53) as the raw material, the same operation as the Example 84(4) gave the title compound.
Yield: 98.0%.
¹H-NMR(DMSO-d₆): δ 4.79(2H, brs), 6.76(1H, d, J=2.1Hz), 6.76(1H, s), 7.09(1H, dd, J=2.1, 1.2Hz), 7.80(1H, s), 8.45(2H, s), 10.30(1H, br), 10.84(1H, s).

### Example 90: Preparation of the compound of Compound No. 89.

Using 5-dimethylaminosalicylic acid and 3,5-bis(trifluoromethyl)aniline as the raw materials, the same operation as the Example 16 gave the title compound.
Yield: 28.8%.
¹H-NMR(DMSO-d₆): δ 2.85(6H, s), 6.92(1H, d, J=9.0Hz), 7.01(1H, dd, J=8.7, 3.0Hz), 7.22(1H, d, J=3.0Hz), 7.84(1H, s), 8.47(2H, s), 10.62(1H, s), 10.83(1H, s).

### Example 91: Preparation of the compound of Compound No. 90.

Under argon atmosphere, a mixture of 5-amino-N-[3,5-bis(trifluoromethyl)phenyl]-2-hydroxybenzamide(Compound No. 88; 364mg, 1mmol), pyridine(95mg, 1.2mmol) and tetrahydrofuran(10mL) was cooled on ice. Benzoyl chloride(155mg, 1.1mmol) was added, and the mixture was stirred for 1 hour. The reaction mixture was poured into water and extracted with ethyl acetate. After the organic layer was washed with water and brine, dried over anhydrous magnesium sulfate, the residue obtained by evaporation under reduced pressure was purified by chromatography on silica gel(n-hexane:ethyl acetate=4:1) to give the title compound(121mg, 25.7%) as a white solid.
¹H-NMR(DMSO-d₆): δ 7.04(1H, d, J=8.7Hz), 7.51-7.62(3H, m), 7.81(1H, dd, J=8.7, 2.4Hz), 7.83(1H, s), 7.98(2H, d, J=7.2Hz), 8.22(1H, d, J=2.4Hz), 8.49(2H, s), 10.27(1H, s), 10.89(1H, s), 11.07(1H, s).

### Example 92: Preparation of the compound of Compound No. 91.

5-Amino-N-[3,5-bis(trifluoromethyl)phenyl]-2-hydroxybenzamide(Compound No. 88; 100.2mg, 0.28mmol) was dissolved in acetonitrile(4ml).
4-Dimethylaminopyridine(3mg) and phenylisocyanate(30 µl, 0.28mmol) were added, and the mixture was stirred at 60°C for 5 minutes. The reaction mixture was concentrated and the residue was purified by chromatography on silica gel(n-hexane:ethyl acetate=1:1) to give the title compound(54.8mg, 41.2%) as a light brown solid.
¹H-NMR(DMSO-d₆): δ6.93-6.98(1H, m), 6.97(1H, d, J=9.3Hz),7.27(2H, t, J=7.8Hz), 7.34-7.46(2H, m), 7.50(1H, dd, J=9.0, 2.4Hz), 7.83(1H, s), 7.88(1H, s), 8.47(2H, s), 8.56(1H, s), 8.63(1H, s), 10.87(1H, s), 10.89(1H, s).

### Example 93: Preparation of the compound of Compound No. 92.

Using 5-amino-N-[3,5-bis(trifluoromethyl)phenyl]-2-hydroxybenzamide (Compound No. 88) and phenylisothiocyanate as the raw materials, the same operation as the Example 92 gave the title compound.
Yield: 66.3%.
¹H-NMR(DMSO-d₆): δ 7.00(1H, d, J=8.4Hz), 7.13(1H, tt, J=7.5, 1.2Hz),7.34(2H, t, J=7.8Hz), 7.45-7.51(3H, m), 7.84(1H, s), 7.87(1H, d, J=2.7Hz), 8.47(2H, s), 9.65(1H, s), 9.74(1H, s), 10.84(1H, s), 11.32(1H, s).

### Example 94: Preparation of the compound of Compound No. 93.

Using 5-[(4-nitrophenyl)diazenyl]salicylic acid and 3,5-bis(trifluoromethyl)aniline as the raw materials, the same operation as the Example 16 gave the title compound.
Yield: 11.3%.
¹H-NMR(DMSO-d₆): δ7.23(1H, d, J=9.0Hz), 7.87(1H, s),8.06(2H, d, J=9.0Hz), 8.10(1H, dd, J=9.0, 2.4Hz), 8.44(2H, d, J=9.0Hz), 8.50(2H, s), 8.53(1H, d, J=2.4Hz), 11.13(1H, s), 12.14(1H, br).

### Example 95: Preparation of the compound of Compound No. 94.

Using 5-({[(4-pyridin-2-yl)sulfamoyl]phenyl}diazenyl)salicylic acid and 3,5-bis(trifluoromethyl)aniline as the raw materials, the same operation as the Example 16 gave the title compound.
Yield: 7.9%.
¹H-NMR(DMSO-d₆): δ 6.87(1H, t, J=6.0Hz), 7.22(1H, d, J=8.7Hz), 7.21-7.23(1H, m), 7.77(1H, t, J=8.4Hz), 7.87(1H, s), 7.95-7.98(3H, m), 8.03-8.07(4H, m), 8.47(1H, d, J=2.4Hz), 8.49(2H, s), 11.14(1H, s), 12.03(1H, br).

### Example 96: Preparation of the compound of Compound No. 96.

N-[3,5-Bis(trifluoromethyl)phenyl]-5-chloro-2-hydroxybenzamide(Compound No. 50; 1.51g, 3mmol) and pyridine(285mg, 3.6mmol) were dissolved in tetrahydrofuran(6mL). Acetyl chloride(234mg, 3.3mmol) was added dropwise under ice cooling, and the mixture was stirred at room temperature for 1 hour. The solvent was evaporated under reduced pressure. 2 N hydrochloric acid was added to the residue, and it was extracted with ethyl acetate. After the ethyl acetate layer was washed with water and brine, dried over anhydrous magnesium sulfate and concentrated, the residue was recrystallized from n-hexane/ethyl acetate to give the title compound(1.06g, 83.0%) as a white solid.
¹H-NMR(DMSO-d₆): δ 2.22(3H, s), 7.35(1H, d, J=9.0Hz), 7.71(1H, dd, J=8.7, 2.7Hz), 7.85(1H, s), 7.88(1H, d, J=2.7Hz), 8.37(2H, s), 11.05(1H, brs).

When the method described in Example 96 is referred in the following examples, organic bases such as pyridine, triethylamine or the like were used as the base. As the reaction solvent, solvents such as dichloromethane, tetrahydrofuran, benzene or the like were used alone or as a mixture.

### Example 97: Preparation of the compound of Compound No. 97.

### (1) 4-Acetylamino-5-chloro-2-methoxybenzoic acid.

Using 4-acetylamino-5-chloro-2-methoxybenzoic acid methyl ester as the raw material, the same operation as the Example 82 gave the title compound.
Yield: 88.0%.
¹H-NMR(DMSO-d₆): δ 2.16(3H, s), 3.78(3H, s), 7.72(1H, s), 7.77(1H, s), 9.57(1H, s), 12.74(1H, s).

### (2) 4-Acetylamino-N-[3,5-bis(trifluoromethyl)phenyl]-5-chloro-2-methoxybenzamide.

Using 4-acetylamino-5-chloro-2-methoxybenzoic acid and 3,5-bis(trifluoromethyl)aniline as the raw materials, the same operation as the Example 24 gave the title compound.
Yield: 23.8%.
¹H-NMR(DMSO-d₆): δ 2.17(3H, s), 3.89(3H, s), 7.77-7.82(3H, m), 8.45-8.49(2H, m), 9.66(1H, s), 10.68(1H, s).

### (3) 4-Acetylamino-N-[3,5-bis(trifluoromethyl)phenyl]-5-chloro-2-hydoxybenzamide (Compound No. 97).

Using 4-acetylamino-N-[3,5-bis(trifluoromethyl)phenyl]-5-chloro-2-methoxybenzamide as the raw material, the same operation as the Example 80(5) gave the title compound.
Yield: 72.8%.
¹H-NMR(DMSO-d₆): δ2.17(3H, s), 7.75(1H, s), 7.82(1H, s), 7.95(1H, s), 8.44(2H, s), 9.45(1H, s), 11.16(1H, brs), 11.63(1H, brs).

### Example 98: Preparation of the compound of Compound No. 98.

Using 4-chlorosalicylic acid and 3,5-bis(trifluoromethyl)aniline as the raw materials, the same operation as the Example 16 gave the title compound.
Yield: 55.8%.
¹H-NMR(DMSO-d₆): δ 7.05-7.08(2H, m), 7.84-7.87(2H, m), 8.45(2H, s), 10.84(1H, s)11.64(1H, brs).

### Example 99: Preparation of the compound of Compound No. 99.

Using 5-chlorosalicylic acid and 3,5-bis(trifluoromethyl)-2-bromoaniline as the raw materials, the same operation as the Example 16 gave the title compound.
Yield: 14.5%.
¹H-NMR(DMSO-d₆): δ 7.11(1H, d, J=9.0Hz), 7.53(1H, dd, J=9.0, 2.7Hz), 7.91(1H, d, J=1.8Hz), 7.98(1H, d, J=2.7Hz), 9.03(1H, d, J=1.8Hz), 11.26(1H, brs).

### Example 100: Preparation of the compound of Compound No. 100.

Using 5-chlorosalicylic acid and 2,5-bis(trifluoromethyl)aniline as the raw materials, the same operation as the Example 16 gave the title compound.
Yield: 3.6%.
¹H-NMR(CDCl₃): δ 7.03(1H, d, J=8.7Hz), 7.43-7.48(2H, m), 6.61(1H, d, J=8.1Hz), 7.85(1H, d, J=8.4Hz), 8.36(1H, br s), 8.60(1H, s), 11.31(1H, s).

### Example 101: Preparation of the compound of Compound No. 101.

Using 5-bromosalicylic acid and 2,5-bis(trifluoromethyl)aniline as the raw materials, the same operation as the Example 16 gave the title compound.
Yield: 24.0%.
¹H-NMR(DMSO-d₆): δ 7.03(1H, d, J=8.7Hz), 7.65(1H, dd, J=8.7, 2.7Hz), 7.76(1H, d, J=8.4Hz), 8.03(1H, d, J=8.1Hz)8.11(1H, d, J=2.7Hz), 8.74(1H, s), 11.02(1H, s), 12.34(1H, s).

### Example 102: Preparation of the compound of Compound No. 102.

Using 5-methylsalicylic acid and 2,5-bis(trifluoromethyl)aniline as the raw materials, the same operation as the Example 16 gave the title compound.
Yield: 1.5%.
¹H-NMR(CDCl₃): δ 2.36(3H, s), 6.97(1H, d, J=8.4Hz), 7.23(1H, s), 7.32(1H, dd, J=8.4, 1.5Hz), 7.57(1H, d, J=8.4Hz), 7.83(1H, d, J=8.4Hz), 8.46(1H, s), 8.69(1H, s), 11.19(1H, s).

### Example 103: Preparation of the compound of Compound No. 103.

Using N-[2,5-bis(trifluoromethyl)phenyl]-5-chloro-2-hydroxybenzamide (Compound No. 100) and acetyl chloride as the raw materials, the same operation as the Example 96 gave the title compound.
Yield: 6.6%.
¹H-NMR(CDCl₃): δ 2.35(3H, s), 7.17(1H, d, J=8.7Hz),7.54(1H, dd, J=8.7, 2.4Hz), 7.55(1H, d, J=8.1Hz), 7.80(1H, d, J=8.1Hz), 7.95(1H, d, J=2.4Hz), 8.60(1H, s), 8.73(1H, s).

### Example 104: Preparation of the compound of Compound No. 104.

Using 5-chlorosalicylic acid and 2-(trifluoromethyl)aniline as the raw materials, the same operation as the Example 16 gave the title compound.
Yield: 58.0%.
¹H-NMR(DMSO-d₆): δ 7.07(1H, d, J=8.7Hz), 7.42(1H, t, J=7.5Hz), 7.52(1H, dd, J=8.7, 2.7Hz), 7.74(1H, t, J=8.1Hz), 7.77(1H, t, J=8.1Hz), 7.99(1H, d, J=2.7Hz), 8.18(1H, d, J=8.1Hz), 10.76(1H, s), 12.22(1H, s).

### Example 105: Preparation of the compound of Compound No. 105.

Using 5-chlorosalicylic acid and 4-chloro-2-(trifluoromethyl)aniline as the raw materials, the same operation as the Example 16 gave the title compound.
Yield: 21.5%.
¹H-NMR(DMSO-d₆): δ 7.07(1H, d, J=8.7Hz), 7.52(1H, dd, J=8.7, 2.7Hz), 7.80-7.85(2H, m), 7.97(1H, d, J=2.7Hz), 8.26(1H, d, J=8.4Hz), 10.80(1H, s), 12.26(1H, s).

### Example 106: Preparation of the compound of Compound No. 106.

Using 5-bromosalicylic acid and 3-(trifluoromethyl)aniline as the raw materials, the same operation as the Example 16 gave the title compound.
Yield: 50.3%.
¹H-NMR(DMSO-d₆): δ6.98(1H, d, J=8.7Hz), 7.48-7.52(1H, m), 7.59(1H, dd, J=8.7, 2.7Hz), 7.62(1H, t, J=8.1Hz), 7.92-7.96(1H, m), 8.02(1H, d, J=2.4Hz), 8.20(1H, s), 10.64(1H, s), 11.60(1H, s).

### Example 107: Preparation of the compound of Compound No. 107.

Using 5-chlorosalicylic acid and 2-fluoro-3-(trifluoromethyl)aniline as the raw materials, the same operation as the Example 16 gave the title compound.
Yield: 71.7%, white solid.
¹H-NMR(DMSO-d₆): δ 7.07(1H, d, J=9.0Hz), 7.46(1H, t, J=7.8Hz), 7.52(1H, dd, J=9.0, 2.7Hz), 7.58(1H, t, J=7.2Hz), 7.96(1H, d, J=2.7Hz), 8.49(1H, t, J=7.2Hz), 10.82(1H, s), 12.13(1H, brs).

### Example 108: Preparation of the compound of Compound No. 108.

Using 5-chlorosalicylic acid and 4-fluoro-3-(trifluoromethyl)aniline as the raw materials, the same operation as the Example 16 gave the title compound.
Yield: 72.1%, white solid.
¹H-NMR(DMSO-d₆):7.03(1H, d, J=9.0Hz), 7.48(1H, dd, J=8.7, 2.7Hz), 7.56(1H, d, J=9.9Hz), 7.90(1H, d, J=2.7Hz), 7.99-8.03(1H, m), 8.21(1H, dd, J=6.6, 2.4Hz), 10.63(1H, s), 11.58(1H, s).

### Example 109: Preparation of the compound of Compound No. 109.

Using 5-bromosalicylic acid and 4-chloro-3-(trifluoromethyl)aniline as the raw materials, the same operation as the Example 16 gave the title compound.
Yield: 37.4%.
¹H-NMR(DMSO-d₆): δ 6.98(1H, d, J=8.7Hz), 7.59(1H, dd, J=8.7, 2.4Hz), 7.73(1H, d, J=8.7Hz), 7.98(1H, d, J=2.4Hz), 8.00(1H, dd, J=8.7, 2.4Hz), 8.31(1H, d, J=2.4Hz), 10.68(1H, s), 11.52(1H, brs).

### Example 110: Preparation of the compound of Compound No. 110.

Using 5-chlorosalicylic acid and 3-fluoro-5-(trifluoromethyl)aniline as the raw materials, the same operation as the Example 16 gave the title compound.
Yield: 62.0%.
¹H-NMR(DMSO-d₆): δ 7.04(1H, d, J=8.7Hz), 7.42(1H, d, J=8.4Hz), 7.48(1H, dd, J=9.0, 3.0Hz), 7.85(1H, d, J=2.4Hz), 7.94(1H, dd, J=11.4, 2.1Hz), 7.99(1H, s), 10.73(1H, s), 11.46(1H, s).

### Example 111: Preparation of the compound of Compound No. 111.

Using 5-bromosalicylic acid and 3-bromo-5-(trifluoromethyl)aniline as the raw materials, the same operation as the Example 16 gave the title compound.
Yield: 73.3%.
¹H-NMR(DMSO-d₆): δ6.99(1H, d, J=9.0Hz), 7.60(1H, dd, J=9.0, 2.4Hz), 7.72(1H, s), 7.97(1H, d, J=2.7Hz), 8.16(1H, s), 8.28(1H, s), 10.69(1H, s), 11.45(1H, s).

### Example 112: Preparation of the compound of Compound No. 112.

Using 5-chlorosalicylic acid and 2-fluoro-5-(trifluoromethyl)aniline as the raw materials, the same operation as the Example 16 gave the title compound.
Yield: 77.9%.
¹H-NMR(DMSO-d₆): δ7.07(1H, d, J=9.0Hz), 7.52(1H, dd, J=9.0, 2.7Hz), 7.58-7.61(2H, m), 7.95(1H, d, J=2.7Hz), 8.71(1H, d, J=7.5Hz), 10.90(1H, s), 12.23(1H, s).

### Example 113: Preparation of the compound of Compound No. 113.

Using 5-chlorosalicylic acid and 2-chloro-5-(trifluoromethyl)aniline as the raw materials, the same operation as the Example 16 gave the title compound.
Yield: 49.1%.
¹H-NMR(DMSO-d₆): δ 7.09(1H, d, J=9.0Hz), 7.53(1H, dd, J=9.0, 3.0Hz), 7.55(1H, dd, J=8.4, 2.7Hz), 7.83(1H, d, J=8.4Hz), 7.98(1H, d, J=3.0Hz), 8.88(1H, d, J=2.7Hz), 11.14(1H, s), 12.39(1H, s).

### Example 114: Preparation of the compound of Compound No. 114.

Using 5-bromosalicylic acid and 2-chloro-5-(trifluoromethyl)aniline as the raw materials, the same operation as the Example 16 gave the title compound.
Yield: 34.2%.
¹H-NMR(DMSO-d₆): δ 7.04(1H, d, J=8.7Hz), 7.56(1H, ddd, J=8.1, 2.4, 1.2Hz), 7.64(1H, dd, J=8.7, 2.7Hz), 7.83(1H, dd, J=8.1, 1.2Hz), 8.11(1H, d, J=2.7Hz), 8.87(1H, d, J=2.4Hz), 11.12(1H, s), 12.42(1H, s).

### Example 115: Preparation of the compound of Compound No. 115.

Using 5-chlorosalicylic acid and 4-nitro-3-(trifluoromethyl)aniline as the raw materials, the same operation as the Example 16 gave the title compound.
Yield: 44.8%.
¹H-NMR(DMSO-d₆): δ7.04(1H, d, J=9.0Hz), 7.49(1H, dd, J=9.0, 2.7Hz), 7.81(1H, d, J=2.7Hz), 8.23-8.24(2H, m), 8.43(1H, d, J=1.2Hz), 11.02(1H, s), 11.30(1H, br).

### Example 116: Preparation of the compound of Compound No. 116.

Using 5-chlorosalicylic acid and 2-nitro-5-(trifluoromethyl)aniline as the raw materials, the same operation as the Example 16 gave the title compound.
Yield: 8.1%.
¹H-NMR(DMSO-d₆): δ 7.08(1H, d, J=9.0Hz), 7.53(1H, dd, J=8.7, 2.7Hz), 7.73(1H, dd, J=8.4, 1.8Hz), 7.95(1H, d, J=3.0Hz), 8.36(1H, d, J=8.7Hz), 9.01(1H, d, J=1.8Hz), 12.04(1H, s), 12.20(1H, s).

### Example 117: Preparation of the compound of Compound No. 117.

Using 5-bromosalicylic acid and 4-cyano-3-(trifluoromethyl)aniline as the raw materials, the same operation as the Example 16 gave the title compound.
Yield: 49.7%.
¹H-NMR(DMSO-d₆): δ 6.99(1H, d, J=8.7Hz), 7.60(1H, dd, J=8.7, 2.4Hz), 7.92(1H, d, J=2.7Hz), 8.16(2H, s), 8.42(1H, s), 10.93(1H, s), 11.36(1H, s).

### Example 118: Preparation of the compound of Compound No. 118.

Using 5-chlorosalicylic acid and 2-methyl-3-(trifluoromethyl)aniline as the raw materials, the same operation as the Example 16 gave the title compound.
Yield: 14.5%.
¹H-NMR(DMSO-d₆): δ2.36(3H, d, J=1.2Hz), 7.05(1H, d, J=8.7Hz), 7.46(1H, t, J=8.1Hz), 7.50(1H, dd, J=8.7, 2.7Hz), 7.60(1H, d, J=7.2Hz), 7.99(1H, d, J=7.2Hz), 8.00(1H, d, J=2.4Hz), 10.43(1H, s), 12.08(1H, s).

### Example 119: Preparation of the compound of Compound No. 119.

Using 5-chlorosalicylic acid and 4-methyl-3-(trifluoromethyl)aniline as the raw materials, the same operation as the Example 16 gave the title compound.
Yield: 80.2%.
¹H-NMR(DMSO-d₆): δ 7.01(1H, d, J=8.7Hz), 7.44(1H, d, J=8.4Hz), 7.47(1H, dd, J=9.0, 2.7Hz), 7.84(1H, dd, J=8.4, 2.1Hz), 7.92(1H, d, J=2.7Hz), 8.13(1H, d, J=2.1Hz), 10.65(1H, s), 11.68(1H, br).

### Example 120: Preparation of the compound of Compound No. 120.

Using 5-chlorosalicylic acid and 2-methyl-5-(trifluoromethyl)aniline as the raw materials, the same operation as the Example 16 gave the title compound.
Yield: 73.3%.
¹H-NMR(DMSO-d₆): δ2.39(3H, s), 7.07(1H, d, J=8.7Hz), 7.44-7.54(3H, m), 7.99(1H, d, J=3.0Hz), 8.43(1H, s), 10.52(1H, s), 12.17(1H, brs).

### Example 121: Preparation of the compound of Compound No. 121.

Using 5-chlorosalicylic acid and 4-methoxy-3-(trifluoromethyl)aniline as the raw materials, the same operation as the Example 16 gave the title compound.
Yield: 79.1%.
¹H-NMR(DMSO-d₆): δ 3.89(3H, s), 7.02(1H, d, J=9.0Hz), 7.30(1H, d, J=9.0Hz), 7.48(1H, dd, J=9.0, 3.0Hz), 7.92(1H, dd, J=9.0, 2.4Hz), 7.96(1H, d, J=2.7Hz), 8.04(1H, d, J=2.4Hz), 10.47(1H, s), 11.78(1H, s).

### Example 122: Preparation of the compound of Compound No. 122.

Using 5-bromosalicylic acid and 3-methoxy-5-(trifluoromethyl)aniline as the raw materials, the same operation as the Example 16 gave the title compound.
Yield: 58.8%.
¹H-NMR(DMSO-d₆): δ 3.85(3H, s), 6.98(1H, d, J=8.7Hz), 7.03(1H, s), 7.57-7.61(2H, m), 7.77(1H, s), 8.00(1H, d, J=2.4Hz), 10.57(1H, s), 11.56(1H, s).

### Example 123: Preparation of the compound of Compound No. 123.

Using 5-bromosalicylic acid and 2-methoxy-5-(trifluoromethyl)aniline as the raw materials, the same operation as the Example 16 gave the title compound.
Yield: 71.3%.
¹H-NMR(DMSO-d₆): δ 3.99(3H, s), 7.03(1H, d, J=9.0Hz), 7.30(1H, d, J=8.7Hz), 7.47-7.51(1H, m), 7.61(1H, dd, J=9.0, 2.4Hz), 8.10(1H, d, J=2.4Hz), 8.82(1H, d, J=2.1Hz)11.03(1H, s), 12.19(1H, s).

### Example 124: Preparation of the compound of Compound No. 124.

Using 5-chlorosalicylic acid and 2-methoxy-5-(trifluoromethyl)aniline as the raw materials, the same operation as the Example 16 gave the title compound.
Yield: 83.4%.
¹H-NMR(DMSO-d₆): δ4.00(3H, s), 7.08(1H, d, J=9.0Hz), 7.30(1H, d, J=8.7Hz), 7.47-7.52(2H, m), 7.97(1H, d, J=2.7Hz), 8.83(1H, d, J=2.4Hz), 11.05(1H, s), 12.17(1H, s).

### Example 125: Preparation of the compound of Compound No. 125.

Using 5-chlorosalicylic acid and 2-methylsulfanyl-5-(trifluoromethyl)aniline as the raw materials, the same operation as the Example 16 gave the title compound.
Yield: 79.2%.
¹H-NMR(DMSO-d₆): δ 2.57(3H, s), 7.07(1H, d, J=8.7Hz), 7.52(1H, dd, J=8.7, 2.4Hz), 7.55(1H, dd, J=8.4, 1.5Hz), 7.63(1H, d, J=8.1Hz), 8.00(1H, d, J=2.4Hz), 8.48(1H, d, J=1.5Hz), 10.79(1H, s), 12.26(1H, s).

### Example 126: Preparation of the compound of Compound No. 126.

Using 5-bromosalicylic acid and 2-(1-pyrrolidinyl)-5-(trifluoromethyl)aniline as the raw materials, the same operation as the Example 16 gave the title compound.
Yield: 44.5%.
¹H-NMR(DMSO-d₆): δ 1.86-1.91(4H, m), 3.20-3.26(4H, m), 6.99(1H, d, J=8.7Hz), 7.07(1H, d, J=8.7Hz), 7.43(1H, dd, J=8.7, 2.1Hz), 7.62(1H, dd, J=8.7, 2.4Hz), 7.94(1H, d, J=2.1Hz), 8.17(1H, d, J=2.4Hz), 10.54(1H, s), 12.21(1H, s).

### Example 127: Preparation of the compound of Compound No. 127.

Using 5-bromosalicylic acid and 2-morpholino-5-(trifluoromethyl)aniline as the raw materials, the same operation as the Example 16 gave the title compound.
Yield: 65.9%.
¹H-NMR(DMSO-d₆): δ 2.90(4H, dd, J=4.5, 4.2Hz), 3.84(4H, dd, J=4.8, 4.2Hz), 7.09(1H, d, J=8.4Hz), 7.48(2H, s), 7.61(1H, dd, J=8.4, 2.7Hz), 8.13(1H, d, J=2.7Hz), 8.90(1H, s), 11.21(1H, s), 12.04(1H, s).

### Example 128: Preparation of the compound of Compound No. 128.

Using 5-chlorosalicylic acid and 4-(trifluoromethyl)aniline as the raw materials, the same operation as the Example 16 gave the title compound.
Yield: 75.0%, white solid
¹H-NMR(DMSO-d₆): δ7.04(1H, d, J=9.0Hz), 7.48(1H, dd, J=8.7, 2.7Hz), 7.74(2H, d, J=8.7Hz), 7.90(1H, d, J=2.7Hz), 7.95(2H, d, J=9.0Hz), 10.65(1H, s), 11.59(1H, s).

### Example 129: Preparation of the compound of Compound No. 129.

Using 5-bromosalicylic acid and 2-chloro-4-(trifluoromethyl)aniline as the raw materials, the same operation as the Example 16 gave the title compound.
Yield: 34.9%.
¹H-NMR(DMSO-d₆): δ7.04(1H, d, J=8.7Hz), 7.64(1H, dd, J=8.7, 2.7Hz), 7.79(1H, dd, J=9.0, 2.1Hz), 7.99(1H, d, J=2.1Hz), 8.11(1H, d, J=2.4Hz), 8.73(1H, d, J=9.0Hz), 11.15(1H, s), 12.42(1H, s).

### Example 130: Preparation of the compound of Compound No. 130.

Using 5-chloro-N-[2-chloro-5-(trifluoromethyl)phenyl]-2-hydroxybenzamide (Compound No. 113) and acetyl chloride as the raw materials, the same operation as the Example 96 gave the title compound.
Yield: 34.0%.
¹H-NMR(CDCl₃): δ2.39(3H, s), 7.16(1H, d, J=8.7Hz),7.37(1H, ddd, J=8.7, 2.4, 0.6Hz), 7.51-7.56(2H, m), 7.97(1H, d, J=3.0Hz), 8.85(1H, s), 8.94(1H, d, J=1.8Hz).

### Example 131: Preparation of the compound of Compound No. 131.

Using 5-nitrosalicylic acid and 2-chloro-5-(trifluoromethyl)aniline as the raw materials, the same operation as the Example 16 gave the title compound.
Yield: 31.1%.
¹H-NMR(DMSO-d₆): δ6.98(1H, d, J=9.3Hz), 7.52(1H, dd, J=8.4, 2.1Hz), 7.81(1H, d, J=8.4Hz), 8.21(1H, dd, J=9.0, 3.3Hz), 8.82(1H, d, J=3.0Hz), 8.93(1H, d, J=2.4Hz), 12.18(1H, s).

### Example 132: Preparation of the compound of Compound No. 132.

Using 5-methylsalicylic acid and 2-chloro-5-(trifluoromethyl)aniline as the raw materials, the same operation as the Example 16 gave the title compound.
Yield: 15.8%.
¹H-NMR(CDCl₃): δ 2.36(3H, s), 6.95(1H, d, J=8.1Hz), 7.26-7.31(2H, m), 7.37(1H, dd, J=8.4, 1.8Hz), 7.56(1H, d, J=8.4Hz), 8.65(1H, br s), 8.80(1H, d, J=1.8Hz), 11.33(1H, br s).

### Example 133: Preparation of the compound of Compound No. 133.

Using 5-methoxysalicylic acid and 2-chloro-5-(trifluoromethyl)aniline as the raw materials, the same operation as the Example 16 gave the title compound.
Yield: 56.4%.
¹H-NMR(DMSO-d₆): δ3.77(3H, s), 6.91(1H, d, J=9.0Hz), 7.07(1H, dd, J=8.7, 3.0Hz), 7.20(1H, t, J=1.8Hz), 7.52-7.54(3H, m), 10.33(1H, s), 11.44(1H, s).

### Example 134: Preparation of the compound of Compound No. 134.

Using 5-methylsalicylic acid and 4-chloro-3-(trifluoromethyl)aniline as the raw materials, the same operation as the Example 16 gave the title compound.
Yield: 70.4%.
¹H-NMR(DMSO-d₆): δ2.29(3H, s), 6.91(1H, d, J=8.3Hz), 7.27(1H, ddd, J=8.3, 2.2, 0.6Hz), 7.71(1H, d, J=2.2Hz), 7.72(1H, d, J=8.5Hz), 8.02(1H, dd, J=8.5, 2.5Hz), 8.33(1H, d, J=2.5Hz), 10.64(1H, s), 11.25(1H, s).

### Example 135: Preparation of the compound of Compound No. 135.

Using 5-methylsalicylic acid and 4-methyl-3-(trifluoromethyl)aniline as the raw materials, the same operation as the Example 16 gave the title compound.
Yield: 63.7%.
¹H-NMR(DMSO-d₆): δ 2.29(3H, s), 2.42(3H, s), 6.89(1H, d, J=8.4Hz), 7.26(1H, ddd, J=8.4, 2.1, 0.6Hz), 7.44(1H, d, J=8.1Hz), 7.75(1H, d, J=2.1Hz), 7.86(1H, dd, J=8.4, 1.8Hz), 8.13(1H.d, J=2.1Hz), 10.50(1H, s), 11.42(1H, s).

### Example 136: Preparation of the compound of Compound No. 136.

Using 5-methylsalicylic acid and 2-methyl-5-(trifluoromethyl)aniline as the raw materials, the same operation as the Example 16 gave the title compound.
Yield: 14.2%, white solid.
¹H-NMR(DMSO-d₆): δ2.29(3H, s), 2.38(3H, s), 6.94(1H, d, J=8.4Hz), 7.27(1H, ddd, J=8.4, 2.4, 0.6Hz), 7.44(1H, dd, J=8.1, 1.5Hz), 7.52(1H, d, J=7.8Hz), 7.84(1H, d, J=2.4Hz), 8.46(1H, d, J=1.5Hz), 10.55(1H, s), 11.72(1H, s).

### Example 137: Preparation of the compound of Compound No. 137.

Using 5-methylsalicylic acid and 4-methoxy-3-(trifluoromethyl)aniline as the raw materials, the same operation as the Example 16 gave the title compound.
Yield: 65.1%, slightly yellow solid.
¹H-NMR(DMSO-d₆): δ 2.35(3H, s), 3.89(3H, s), 6.88(1H, d, J=8.4Hz), 7.26(1H, dd, J=8.1, 1.8Hz), 7.30(1H, d, J=8.4Hz), 7.77(1H, d, J=2.1Hz), 7.92(1H, dd, J=9.0, 2.7Hz), 8.04(1H, d, J=2.7Hz), 10.42(1H, s), 11.54(1H, s).

### Example 138: Preparation of the compound of Compound No. 138.

Using 5-methylsalicylic acid and 2-methoxy-5-(trifluoromethyl)aniline as the raw materials, the same operation as the Example 16 gave the title compound.
Yield: 77.9%.
¹H-NMR(CDCl₃): δ 2.35(3H, s), 4.02(3H, s), 6.93(1H, d, J=9.0Hz), 6.98(1H, d, J=8.4Hz), 7.25-7.28(2H, m), 7.36(1H, ddd, J=8.4, 2.1, 0.9Hz), 8.65(1H, br s), 8.73(1H, d, J=2.1Hz), 11.69(1H, s).

### Example 139: Preparation of the compound of Compound No. 139.

Using 5-bromosalicylic acid and aniline as the raw materials, the same operation as the Example 16 gave the title compound.
Yield: 68.8%.
mp 229-230°C.
¹H-NMR(DMSO-d₆): δ6.96(1H, d, J=9.0Hz), 7.12-7.18(1H, m), 7.35-7.41(2H, m), 7.58(1H, dd, J=8.7, 2.7Hz), 7.67-7.71(2H, m), 8.08(1H, d, J=2.7Hz), 10.43(1H, s), 11.87(1H, s).

### Example 140: Preparation of the compound of Compound No. 140.

Using 5-bromosalicylic acid and 3-chloroaniline as the raw materials, the same operation as the Example 16 gave the title compound.
Yield: 63.1%.
mp 231-232°C.
¹H-NMR(DMSO-d₆): δ 6.97(1H, d, J=8.7Hz), 7.19-7.22(1H, m), 7.38-7.43(1H, m), 7.57-7.63(2H, m), 7.91-7.92(1H, m), 8.01(1H, d, J=2.7Hz), 10.49(1H, s), 11.64(1H, s).

### Example 141: The compound of Compound No. 141

This compound is a commercially available compound.
Supplier: Tokyo Kasei.
Catalog code number: B0897.

### Example 142: Preparation of the compound of Compound No. 142.

Using 5-chlorosalicylic acid and 2,5-dichloroaniline as the raw materials, the same operation as the Example 16 gave the title compound.
Yield: 10.8%.
¹H-NMR(DMSO-d₆): δ 7.08(1H, d, J=9.0Hz), 7.24-7.28(1H, m), 7.50-7.54(1H, m), 7.61(1H, dd, J=9.0, 3.0Hz), 7.97(1H, d, J=2.7Hz), 8.58(1H, d, J=2.4Hz), 11.02(1H, s), 12.35(1H, brs).

### Example 143: Preparation of the compound of Compound No. 143.

Using 5-bromosalicylic acid and 3,4-dichloroaniline as the raw materials, the same operation as the Example 16 gave the title compound.
Yield: 58.2%.
mp 249-251°C.
¹H-NMR(DMSO-d₆): δ 6.97(1H, d, J=8.7Hz), 7.57-7.70(3H, m), 7.98(1H, d, J=2.7Hz), 8.10(1H, d, J=2.4Hz), 10.54(1H, s), 11.55(1H, s).

### Example 144: Preparation of the compound of Compound No. 144.

Using 5-bromosalicylic acid and 3,5-difluoroaniline as the raw materials, the same operation as the Example 16 gave the title compound.
Yield: 36.3%.
mp 259-261°C.
¹H-NMR(DMSO-d₆): δ 6.96-7.04(2H, m), 7.45-7.54(2H, m), 7.58(1H, dd, J=8.7, 2.7Hz), 7.94(1H, d, J=2.7Hz), 10.60(1H, s) 11.48(1H, s).

### Example 145: Preparation of the compound of Compound No. 172.

Using O-acetylsalicyloyl chloride and 3,5-dichloroaniline as the raw materials, the same operation as the Example 2(1) gave the title compound.
Yield: 73.5%.
mp 167-168°C.
¹H-NMR(CDCl₃): δ2.35(3H, s), 7.14-7.18(2H, m), 7.35-7.40(1H, m), 7.52-7.57(3H, m), 7.81(1H, dd, J=7.8, 1.8Hz), 8.05(1H, brs).

### Example 146: Preparation of the compound of Compound No. 145.

Using 2-acetoxy-N-(3,5-dichlorophenyl)benzamide(Compound No. 172) as the raw material, the same operation as the Example 2(2) gave the title compound.
Yield: 60.3%.
mp 218-219°C.
¹H-NMR(DMSO-d₆): δ 6.95-7.02(2H, m), 7.35-7.36(1H, m), 7.42-7.47(1H, m), 7.83-7.87(3H, m), 10.54(1H, s), 11.35(1H, s).

### Example 147: Preparation of the compound of Compound No. 146.

Using 5-fluorosalicylic acid and 3,5-dichloroaniline as the raw materials, the same operation as the Example 16 gave the title compound.
Yield: 33.3%.
mp 258-260°C.
¹H-NMR(DMSO-d₆): δ7.00-7.05(1H, m), 7.28-7.37(2H, m), 7.63(1H, dd, J=9.3, 3.3Hz), 7.84(2H, d, J=2.1Hz), 10.56(1H, s), 11.23(1H, s).

### Example 148: Preparation of the compound of Compound No. 147.

Using 5-chlorosalicylic acid and 3,5-dichloroaniline as the raw materials, the same operation as the Example 16 gave the title compound.
Yield: 41.2%.
¹H-NMR(DMSO-d₆): δ 7.03(1H, d, J=9.0Hz), 7.36-7.37(1H, m), 7.48(1H, dd, J=8.7, 2.7Hz), 7.83-7.84(3H, m), 10.56(1H, s), 11.44(1H, s).

### Example 149: Preparation of the compound of Compound No. 148.

Using 5-bromosalicylic acid and 3,5-dichloroaniline as the raw materials, the same operation as the Example 16 gave the title compound.
Yield: 61.6%.
mp 243-244°C.
¹H-NMR(DMSO-d₆): δ 6.98(1H, d, J=8.7Hz), 7.36-7.37(1H, m), 7.59(1H, dd, J=9.0, 2.4Hz), 7.83(2H, d, J=1.8Hz), 7.95(1H, d, J=2.4Hz), 10.56(1H, s), 11.46(1H, s).

### Example 150: Preparation of the compound of Compound No. 149.

Using 5-iodosalicylic acid and 3,5-dichloroaniline as the raw materials, the same operation as the Example 16 gave the title compound.
Yield: 65.4%.
mp 244-245°C.
¹H-NMR(DMSO-d₆): δ6.84(1H, d, J=9.0Hz), 7.35-7.37(1H, m), 7.72(1H, dd, J=9.0, 2.1Hz), 7.83(2H, d, J=1.8Hz), 8.09(1H, d, J=2.1Hz), 10.55(1H, s), 11.45(1H, s).

### Example 151: Preparation of the compound of Compound No. 150.

Using 3,5-dibromosalicylic acid and 3,5-dichloroaniline as the raw materials, the same operation as the Example 16 gave the title compound.
Yield: 44.2%.
mp 181-182°C.
¹H-NMR(DMSO-d₆): δ 7.42-7.43(1H, m), 7.80(2H, d, J=1.8Hz), 8.03(1H, d, J=2.1Hz), 8.17(1H, d, J=2.1Hz), 10.82(1H, s).

### Example 152: Preparation of the compound of Compound No. 151.

Using 4-chlorosalicylic acid and 3,5-dichloroaniline as the raw materials, the same operation as the Example 16 gave the title compound.
Yield: 57.2%.
mp 255-256°C.
¹H-NMR(DMSO-d₆): δ 7.03-7.06(2H, m), 7.34-7.36(1H, m), 7.82-7.85(3H,m), 10.51(1H, s), 11.70(1H, brs).

### Example 153: Preparation of the compound of Compound No. 152.

Using 5-nitrosalicylic acid and 3,5-dichloroaniline as the raw materials, the same operation as the Example 16 gave the title compound.
Yield: 83.1%.
mp 232-233°C.
¹H-NMR(DMSO-d₆): δ 7.16(1H, d, J=9.6Hz), 7.37-7.39(1H, m), 7.84(1H, d, J=2.1Hz), 8.29(1H, dd, J=9.0, 3.0Hz), 8.65(1H, d, J=3.0Hz), 10.83(1H, s).

### Example 154: Preparation of the compound of Compound No. 153.

Using 5-methylsalicylic acid and 3,5-dichloroaniline as the raw materials, the same operation as the Example 16 gave the title compound.
Yield: 71.0%.
mp 216-217°C.
¹H-NMR(DMSO-d₆): δ 2.28(3H, s), 6.90(1H, d, J=8.4Hz), 7.26(1H, dd, J=8.7, 1.8Hz), 7.34-7.36(1H, m), 7.67(1H, d, J=1.5Hz), 7.85(2H, d, J=1.8Hz), 10.52(1H, s), 11.15(1H, s).

### Example 155: Preparation of the compound of Compound No. 154.

Using 5-methoxysalicylic acid and 3,5-dichloroaniline as the raw materials, the same operation as the Example 16 gave the title compound.
Yield: 29.8%.
mp 230-232°C.
¹H-NMR(DMSO-d₆): δ3.76(3H, s), 6.95(1H, d, J=8.7Hz), 7.08(1H, dd, J=9.0, 3.0Hz), 7.35-7.36(1H, m), 7.40(1H, d, J=3.0Hz), 7.85(2H, d, J=1.5Hz), 10.55(1H, s), 10.95(1H, s).

### Example 156: Preparation of the compound of Compound No. 155.

Using 5-bromosalicylic acid and 3,4,5-trichloroaniline as the raw materials, the same operation as the Example 16 gave the title compound.
Yield: 78.6%.
mp 297-299°C.
¹H-NMR(DMSO-d₆): δ 6.98(1H, d, J=9.0Hz), 7.58(1H, dd, J=8.4, 2.4Hz), 7.95(1H, d, J=2.4Hz), 8.03(1H, s), 10.58(1H, s), 11.49(1H, s).

### Example 157: Preparation of the compound of Compound No. 156.

Using 5-bromosalicylic acid and 3,5-dichloro-4-hydroxyaniline as the raw materials, the same operation as the Example 16 gave the title compound.
Yield: 22.5%.
¹H-NMR(DMSO-d₆): δ 6.96(1H, d, J=8.7Hz), 7.58(1H, dd, J=8.7, 2.4Hz), 7.76(2H, s), 8.01(1H, d, J=2.4Hz), 10.03(1H, s), 10.36(1H, s), 11.67(1H, brs).

### Example 158: Preparation of the compound of Compound No. 157.

Using 5-chlorosalicylic acid and 2,3,4,5,6-pentafluoroaniline as the raw materials, the same operation as the Example 16 gave the title compound.
Yield: 58.6%.
¹H-NMR(DMSO-d₆): δ7.07(1H, d, J=8.7Hz), 7.53(1H, dd, J=8.7, 2.7Hz), 7.91(1H, d, J=2.7Hz), 10.38(1H, brs), 11.74(1H, brs).

### Example 159: Preparation of the compound of Compound No. 158.

Using 5-bromosalicylic acid and 3,5-dinitroaniline as the raw materials, the same operation as the Example 16 gave the title compound.
Yield: 32.2%.
mp 258-260°C.
¹H-NMR(DMSO-d₆): δ6.98-7.02(1H, m), 7.59-7.63(1H, m), 7.96-7.97(1H, m), 8.56-8.58(1H, m), 9.03-9.05(2H, m), 11.04(1H, s), 11.39(1H, brs).

### Example 160: Preparation of the compound of Compound No. 159.

Using 5-chlorosalicylic acid and 2,5-bis[(1,1-dimethyl)ethyl]aniline as the raw materials, the same operation as the Example 16 gave the title compound.
Yield: 75.7%.
¹H-NMR(DMSO-d₆): δ1.27(9H, s), 1.33(9H, s), 7.04(1H, d, J=9.0Hz), 7.26(1H, dd, J=8.4, 2.1Hz), 7.35-7.38(2H, m), 7.49(1H, dd, J=8.7, 2.7Hz), 8.07(1H, d, J=2.4Hz), 10.22(1H, s), 12.38(1H, br s).

### Example 161: Preparation of the compound of Compound No. 160.

Using 5-chlorosalicylic acid and 5-[(1,1-dimethyl)ethyl]-2-methoxyaniline as the raw materials, the same operation as the Example 16 gave the title compound.
Yield: 89.5%.
¹H-NMR(DMSO-d₆): δ 1.28(9H, s), 3.33(3H, s), 7.01(1H, d, J=8.7Hz), 7.05(1H, d, J=9.0Hz), 7.11(1H, dd, J=8.7, 2.4Hz), 7.47(1H, dd, J=9.0, 3.0Hz), 7.99(1H, d, J=3.0Hz), 8.49(1H, d, J=2.4Hz), 10.78(1H, s), 12.03(1H, s).

### Example 162: Preparation of the compound of Compound No. 161.

Using 5-bromosalicylic acid and 3,5-dimethylaniline as the raw materials, the same operation as the Example 16 gave the title compound.
Yield: 58.1%.
mp 188-190°C.
¹H-NMR(DMSO-d₆): δ 2.28(6H, s), 6.80(1H, s), 6.96(1H, d, J=8.7Hz), 7.33(2H, s), 7.58(1H, dd, J=9.0, 2.4Hz), 8.10(1H, d, J=2.4Hz), 10.29(1H, s), 11.93(1H, brs).

### Example 163: Preparation of the compound of Compound No. 162.

Using 5-chlorosalicylic acid and 3,5-bis[(1,1-dimethyl)ethyl]aniline as the raw materials, the same operation as the Example 16 gave the title compound.
Yield: 34.1%.
¹H-NMR(CDCl₃): δ1.26(18H, s), 6.99(1H, d, J=8.7Hz), 7.29(1H, t, J=1.8Hz), 7.39(1, dd, J=9.0, 2.4Hz), 7.41(2H, d, J=1.5Hz), 7.51(1H, d, J=2.1Hz), 7.81(1H, br s), 12.01(1H, s).

### Example 164: Preparation of the compound of Compound No. 163.

Using 5-bromosalicylic acid and 3,5-bis[(1,1-dimethyl)ethyl]aniline as the raw materials, the same operation as the Example 16 gave the title compound.
Yield: 45.2%.
¹H-NMR(DMSO-d₆): δ 1.30(18H, s), 6.95(1H, d, J=8.7Hz), 7.20(1H, t, J=1.5Hz), 7.56(2H, d, J=1.5Hz), 7.58(1H, dd, J=8.7, 2.4Hz), 8.12(1H, d, J=2.7Hz), 10.39(1H, s), 11.98(1H, s).

### Example 165: Preparation of the compound of Compound No. 164.

Using 5-chlorosalicylic acid and 2-amino-3,5,5,8,8-pentamethyl-5,6,7,8-tetrahydronaphthalene as the raw materials, the same operation as the Example 16 gave the title compound.
Yield: 77.5%.
¹H-NMR(DMSO-d₆): δ1.23(6H, s), 1.24(6H, s), 1.64(4H, s), 2.19(3H, s), 7.13(1H, d, J=9.0Hz), 7.20(1H, s), 7.49(1H, dd, J=8.7, 2.7Hz), 7.67(1H, s), 8.04(1H, d, J=2.7Hz), 10.23(1H, s), 12.26(1H, s).

### Example 166: Preparation of the compound of Compound No. 165.

Using 5-chlorosalicylic acid and 3-aminobiphenyl as the raw materials, the same operation as the Example 16 gave the title compound.
Yield: 75.6%.
¹H-NMR(DMSO-d₆): δ7.04(1H, d, J=8.7Hz), 7.35-7.44(1H, m), 7.45-7.54(5H, m), 7.65-7.68(2H, m), 7.72(1H, dt, J=7.2, 2.1Hz).7.99(1H, d, J=3.0Hz), 8.03(1H, m), 10.50(1H, s), 11.83(1H, brs).

### Example 167: Preparation of the compound of Compound No. 166.

Using 5-chlorosalicylic acid and 3-amino-4-methoxybiphenyl as the raw materials, the same operation as the Example 16 gave the title compound.
Yield: 37.0%.
¹H-NMR(DMSO-d₆): δ3.95(3H, s), 7.08(1H, d, J=8.7Hz), 7.20(1H, d, J=8.4Hz), 7.34(1H, t, J=7.2Hz), 7.40-7.50(4H, m), 7.62(1H, d, J=8.7Hz), 8.00(1H, d, J=3.0Hz), 8.77(1H, d, J=2.1Hz), 10.92(1H, s), 12.09(1H, s).

### Example 168: Preparation of the compound of Compound No. 167.

Using 5-bromosalicylic acid and 2,5-dimethoxyaniline as the raw materials, the same operation as the Example 16 gave the title compound.
Yield: 39.7%.
¹H-NMR(DMSO-d₆): δ 3.72(3H, s), 3.84(3H, s), 6.66(1H, ddd, J=9.0, 3.0, 0.6Hz), 6.99-7.03(2H, m), 7.58(1H, ddd, J=9.0, 2.7, 0.6Hz), 8.10(1H, dd, J=2.4, 0.6Hz), 8.12(1H, d, J=3.0Hz), 10.87(1H, s), 12.08(1H, s).

### Example 169: Preparation of the compound of Compound No. 168.

Using 5-bromosalicylic acid and 3,5-dimethoxyaniline as the raw materials, the same operation as the Example 16 gave the title compound.
Yield: 40.3%.
mp 207-209°C.
¹H-NMR(DMSO-d₆): δ 3.75(6H, s), 6.30-6.32(1H, m), 6.94-6.97(3H, m), 7.57(1H, dd, J=8.7, 2.4Hz), 8.04(1H, d, J=2.4Hz), 10.32(1H, s), 11.78(1H, s).

### Example 170: Preparation of the compound of Compound No. 169.

Using 5-chlorosalicylic acid and 3-acetylaniline as the raw materials, the same operation as the Example 16 gave the title compound.
Yield: 80.0%.
¹H-NMR(DMSO-d₆): δ 2.60(3H, s), 7.03(1H, d, J=9.0Hz), 7.49(1H, dd, J=9.0, 3.0Hz), 7.54(1H, t, J=8.1Hz), 7.76(1H, dq, J=7.8, 0.9Hz), 7.96-8.00(2H, m), 8.30(1H, t, J=1.8Hz), 10.56(1H, s), 11.75(1H, s).

### Example 171: Preparation of the compound of Compound No. 170.

Using 5-bromosalicylic acid and 5-aminoisophthalic acid dimethyl ester as the raw materials, the same operation as the Example 16 gave the title compound.
Yield: 74.1%.
mp 254-256°C.
¹H-NMR(DMSO-d₆): δ 3.92(6H, s), 6.97(1H, d, J=9.0Hz), 7.60(1H, dd, J=9.0, 2.4Hz), 8.06(1H, d, J=2.4Hz), 8.24-8.25(1H, m), 8.62(2H, m), 10.71(1H, s), 11.57(1H, s).

### Example 172: The compound of Compound No. 171.

This compound is a commercially available compound.
Supplier: Maybridge.
Catalog code number: RDR 01434

### Example 173: Preparation of the compound of Compound No. 173.

Using 5-methylsalicylic acid and 2,5-bis[(1,1-dimethyl)ethyl]aniline as the raw materials, the same operation as the Example 16 gave the title compound.
Yield: 61.1%.
¹H-NMR(DMSO-d₆): δ 1.27(9H, s), 1.33(9H, s), 2.28(3H, s), 6.89(1H, d, J=8.1Hz), 7.24(1H, d, J=2.1Hz), 7.27(1H, d, J=2.1Hz), 7.32(1H, d, J=2.4Hz), 7.37(1H, d, J=8.4Hz), 7.88(1H, d, J=1.5Hz), 10.15(1H, s), 11.98(1H, br s).

### Example 174: Preparation of the compound of Compound No. 174.

Using N-{3,5-bis[(1,1-dimethyl)ethyl]phenyl}-5-chloro-2-hydroxybenzamide(Compound No. 162) and acetyl chloride as the raw materials, the same operation as the Example 96 gave the title compound.
Yield: 66.1%.
¹H-NMR(CDCl₃): δ1.34(18H, s), 2.36(3H, s), 7.12(1H, d, J=8.4Hz),7.25(1H, d, J=1.5Hz), 7.44(2H, d, J=1.2Hz), 7.47(1H, dd, J=8.7, 2.7Hz), 7.87(1H, d, J=2.4Hz), 7.98(1H, s).

### Example 175: Preparation of the compound of Compound No. 175.

Using 5-nitrosalicylic acid and 3,5-bis[(1,1-dimethyl)ethyl]aniline as the raw materials, the same operation as the Example 16 gave the title compound.
Yield: 46.7%.
¹H-NMR(CDCl₃): δ 1.37(18H, s), 7.13(1H, d, J=9.3Hz), 7.32(1H, t, J=1.8Hz), 7.46(2H, d, J=1.8Hz), 8.07(1H, s), 8.33(1H, dd, J=9.3, 2.1Hz), 8.59(1H, d, J=2.4Hz), 13.14(1H, s).

### Example 176: Preparation of the compound of Compound No. 176.

Using 5-methylsalicylic acid and 3,5-bis[(1,1-dimethyl)ethyl]aniline as the raw materials, the same operation as the Example 16 gave the title compound.
Yield: 16.3%.
¹H-NMR(CDCl₃): δ 1.35(18H, s), 2.35(3H, s), 6.94(1H, d, H=8.4Hz), 7.23-7.28(2H, m), 7.31(1H, s), 7.42(1H, d, J=1.8Hz), 7.88(1H, s), 11.86(1H, s).

### Example 177: Preparation of the compound of Compound No. 177.

Using 5-methoxysalicylic acid and 3,5-bis[(1,1-dimethyl)ethyl]aniline as the raw materials, the same operation as the Example 16 gave the title compound.
Yield: 12.7%.
¹H-NMR(DMSO-d₆): δ 1.30(18H, s), 3.77(3H, s), 6.91(1H, d, J=9.0Hz), 7.07(1H, dd, J=8.7, 3.0Hz), 7.19-7.20(1H, m), 7.52-7.54(3H, m), 10.33(1H, s), 11.44(1H, s).

### Example 178: Preparation of the compound of Compound No. 178.

Using 5-chloro-N-{5-[(1,1-dimethyl)ethyl]-2-methoxyphenyl}-2-hydroxybenzamide(Compound No. 160) and acetyl chloride as the raw materials, the same operation as the Example 96 gave the title compound.
Yield: 87.5%.
¹H-NMR(CDCl₃): δ 1.35(9H, s), 2.37(3H, s), 3.91(3H, s), 6.86(1H, d, J=8.7Hz),7.12(1H, dd, J=8.7, 2.4Hz), 7.13(1H, d, J=9.0Hz), 7.47(1H, dd, J=9.0, 2.4Hz), 8.02(1H, d, J=2.7Hz), 8.66(1H, d, J=2.4Hz), 8.93(1H, s).

### Example 179: Preparation of the compound of Compound No. 179.

Using 5-methylsalicylic acid and 5-[(1,1-dimethyl)ethyl]-2-methoxyaniline as the raw materials, the same operation as the Example 16 gave the title compound.
Yield: 84.7%.
¹H-NMR(CDCl₃): δ 1.35(9H, s), 2.34(3H, s), 3.93(3H, s), 6.86(1H, d, J=8.7Hz), 6.93(1H, d, J=8.4Hz), 7.12(1H, dd, J=8.7, 2.4Hz), 7.24(1H, dd, J=8.4, 1.8Hz), 7.27(1H, br s), 8.48(1H, d, J=2.4Hz), 8.61(1H, brs), 11.95(1H, s).

### Example 180: Preparation of the compound of Compound No. 179.

Using 5-bromosalicylic acid and 2-aminothiazole as the raw materials, the same operation as the Example 16 gave the title compound.
Yield: 12.0%.
mp 212°C(dec.).
¹H-NMR(DMSO-d₆): δ 6.94(1H, brd, J=8.0Hz), 7.25(1H, brd, J=3.2Hz), 7.56(2H, m), 8.05(1H, d, J=2.8Hz).

### Example 181: Preparation of the compound of Compound No. 186.

### (1) 2-Amino-4-[(1,1-dimethyl)ethyl]thiazole.

A mixture of 1-bromo-3,3-dimethyl-2-butanone(5.03g, 28.1mmol), thiourea(2.35g, 30.9mmol) and ethanol(30mL) was refluxed for 1.5 hours. After cooling, the reaction mixture was poured into saturated aqueous sodium hydrogen carbonate and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=2:1→1:1) to give the title compound(3.99g, 90.9%) as an yellowish white powder.
¹H-NMR(CDCl₃): δ 1.26(9H, s), 4.96(2H, brs), 6.09(1H, s).

When the method described in Example 181(1) is referred in the following examples, solvents such as ethanol or the like were used as the reaction solvent.

### (2) 2-Acetoxy-5-bromo-N-{4-[(1,1-dimethyl)ethyl]thiazol-2-yl}benzamide.

Using 2-acetoxy-5-bromobenzoic acid and 2-amino-4-[(1,1-dimethyl)ethyl]thiazole as the raw materials, the same operation as the Example 24 gave the title compound.
Yield: 59.4%.
¹H-NMR(CDCl₃): δ 1.31(9H, s), 2.44(3H, s), 6.60(1H, s), 7.13(1H, d, J=8.4Hz), 7.68(1H, dd, J=8.7, 2.4Hz), 8.17(1H, d, J=2.4Hz), 9.72(1H, brs).

### (3) 5-Bromo-N-{4-[(1,1-dimethyl)ethyl]thiazol-2-yl}-2-hydroxybenzamide(Compound No. 186).

2-Acetoxy-5-bromo-N-{4-[(1,1-dimethyl)ethyl]thiazol-2-yl}benzamide(100.1mg, 0.25mmol) was dissolved in tetrahydrofuran(3mL). 2N Sodium hydroxide(0.2ml) was added, and the mixture was stirred at room temperature for 20 minutes. The reaction mixture was poured into diluted hydrochloric acid and extracted with ethyl acetate. After the organic layer was washed with brine, dried over anhydrous sodium sulfate, the residue obtained by evaporation under reduced pressure was crystallized(isopropyl ether/n-hexane) to give the title compound(70.1mg, 78.9%) as a white powder.
¹H-NMR(DMSO-d₆): δ1.30(9H, s), 6.80(1H, brs), 6.95(1H, brs), 7.57(1H, brs), 8.06(1H, d, J=2.4Hz), 11.82(1H, brs), 13.27(1H, brs).

### Example 182: Preparation of the compound of Compound No. 181.

### (1) 2-Acetoxy-5-bromo-N-{5-bromo-4-[(1,1-dimethyl)ethyl]thiazol-2-yl}benzamide.

2-Acetoxy-5-bromo-N-{4-[(1,1-dimethyl)ethyl]thiazol-2-yl}benzamide (compound of Example 181(2); 0.20g, 0.50mmol) was dissolved in acetonitrile(10mL). N-Bromosuccinimide(97.9mg, 0.55mmol) was added, and the mixture was stirred at room temperature for 1 hour. The reaction mixture was concentrated under reduced pressure, and the obtained residue was purified by chromatography on silica gel(n-hexane:ethyl acetate=3:1) to give the title compound as a crude product.

### (2) 5-Bromo-N-{5-bromo-4-[(1,1-dimethyl)ethyl]thiazol-2-yl}-2-hydroxybenzamide (Compound No. 181).

Using 2-acetoxy-5-bromo-N-{5-bromo-4-[(1,1-dimethyl)ethyl]thiazol-2-yl}benzamide as the raw material, the same operation as the Example 2(2) gave the title compound.
Yield: 90.9%(2 steps).
¹H-NMR(DMSO-d₆): δ 1.42(9H, s), 6.99(1H, d, J=8.7Hz), 7.61(1H, dd, J=8.7, 2.7Hz), 8.02(1H, d, J=2.4Hz), 11.79(1H, brs), 12.00(1H, brs).

### Example 183: Preparation of the compound of Compound No. 182.

Using 5-bromosalicylic acid and 2-amino-5-bromo-4-(trifluoromethyl)thiazole as the raw materials, the same operation as the Example 16 gave the title compound.
Yield: 22.4%.
mp 215°C(dec.).
¹H-NMR(DMSO-d₆): δ 7.00(1H, d, J=8.8Hz), 7.61(1H, dd, J=8.8, 2.8Hz), 7.97(1H, d, J=2.4Hz).

### [2-Amino-5-bromo-4-(trifluoromethyl)thiazole: Refer to "Journal of Heterocyclic Chemistry", (USA), 1991, Vol.28, p.1017.]

### Example 184: Preparation of the compound of Compound No. 183.

### (1) α-Bromo-pivaloylacetonitrile.

Pivaloylacetonitrile(1.00g, 7.99mmol) was dissolved in carbon tetrachloride(15mL). N-Bromosuccinimide(1.42g, 7.99mmol) was added, and the mixture was refluxed for 15 minutes. After cooling, the insoluble matter was filtered off, and the residue obtained by evaporation of the filtrate under reduced pressure was purified by chromatography on silica gel(n-hexane:ethyl acetate=4:1) to give the title compound(1.43g, 87.9%) as an yellowish brown oil.
¹H-NMR(CDCl₃): δ1.33(9H, s), 5.10(1H, s).

When the method described in Example 184(1) is referred in the following examples, N-bromosuccinimide was used as the brominating agent. As the reaction solvent, solvents such as carbon tetrachloride or the like were used.

### (2) 2-Amino-5-cyano-4-[(1,1-dimethyl)ethyl]thiazole.

Using α-bromo-pivaloylacetonitrile and thiourea as the raw materials, the same operation as the Example 181(1) gave the title compound.
Yield: 66.3%.
¹H-NMR(CDCl₃): δ 1.41(9H, s), 5.32(2H, s).

### (3) 5-Chloro-N-{5-cyano-4-[(1,1-dimethyl)ethyl]thiazol-2-yl}-2-hydroxybenzamide (Compound No. 183).

Using 5-chlorosalicylic acid and 2-amino-5-cyano-4-[(1,1-dimethyl)ethyl]thiazole as the raw materials, the same operation as the Example 16 gave the title compound.
Yield: 63.4%.
¹H-NMR(DMSO-d₆): δ 1.43(9H, s), 7.06(1H, d, J=8.7Hz), 7.51(1H, dd, J=8.7, 3.0Hz), 7.85(1H, d, J=2.7Hz), 12.31(2H, br).

### Example 185: Preparation of the compound of Compound No. 184.

Using 5-bromosalicylic acid and 2-amino-5-cyano-4-[(1,1-dimethyl)ethyl]thiazole(compound of Example 184(2)) as the raw materials, the same operation as the Example 16 gave the title compound.
Yield: 61.3%.
¹H-NMR(DMSO-d₆): δ 1.43(9H, s), 7.00(1H, d, J=8.7Hz), 7.62(1H, dd, J=8.7, 2.7Hz), 7.97(1H, d, J=2.7Hz), 11.75(1H, br), 12.43(1H, br).

### Example 186: Preparation of the compound of Compound No. 185.

Using 5-bromosalicylic acid and 2-amino-5-methylthiazole as the raw materials, the same operation as the Example 16 gave the title compound.
Yield: 12.9%.
¹H-NMR(DMSO-d₆): δ 2.33(3H, s), 6.91(1H, d, J=7.6Hz), 7.26(1H, s), 7.54(1H, d, J=9.6Hz), 8.03(1H, d, J=2.8Hz).

### Example 187: Preparation of the compound of Compound No. 187.

Using 5-bromosalicylic acid and 2-amino-4,5-dimethylthiazole as the raw materials, the same operation as the Example 16 gave the title compound.
Yield: 14.4%.
¹H-NMR(DMSO-d₆): δ 2.18(3H, s), 2.22(3H, s), 6.89(1H, d, J=8.8Hz), 7.51(1H, d, J=6.8Hz), 8.02(1H, d, J=2.8Hz), 13.23(1H, brs).

### Example 188: Preparation of the compound of Compound No. 188.

Using 5-bromosalicylic acid and 2-amino-5-methyl-4-phenylthiazole as the raw materials, the same operation as the Example 16 gave the title compound.
Yield: 27.7%.
mp 243-244°C.
¹H-NMR(CD₃OD): δ 2.47(3H, s), 6.92(1H, d, J=8.7Hz), 7.36-7.41(1H, m), 7.44-7.50(2H, m), 7.53(1H, dd, J=9.0, 2.7Hz), 7.57-7.61(2H, m), 8.16(1H, d, J=2.7Hz).

### [2-Amino-5-methyl-4-phenylthiazole: Refer to "Yakugaku Zasshi: Journal of The Pharmaceutical Society of Japan", 1961, Vol.81, p.1456.]

### Example 189: Preparation of the compound of Compound No. 189.

Using (4-fluorophenyl)acetone as the raw material, the same operation as the Examples 184(1)-(3) gave the title compound.
Yield: 28.8%(3 steps).

### (1) α-Bromo-(4-fluorophenyl)acetone.

¹H-NMR(CDCl₃): δ 2.33(3H, s), 5.41(1H, s), 7.07(2H, t, J=8.7Hz), 7.43(2H, dd, J=8.7, 5.1Hz).

### (2) 2-Amino-4-methyl-5-(4-fluorophenyl)thiazole.

¹H-NMR(CDCl₃): δ 2.27(3H, s), 4.88(2H, s), 7.07(2H, t, J=8.7Hz), 7.32(2H, dd, J=8.7, 5.4Hz).

### (3) 5-Bromo-N-[4-methyl-5-(4-fluorophenyl)thiazol-2-yl]-2-hydroxybenzamide (Compound No. 189).

¹H-NMR(DMSO-d₆): δ2.36(3H, s), 6.95(1H, d, J=8.4Hz), 7.33(2H, t, J=8.7Hz), 7.52-7.59(3H, m), 8.06(1H, d, J=3.0Hz), 12.01-13.65(2H, br).

### Example 190: Preparation of the compound of Compound No. 190.

Using 3-(trifluoromethyl)phenylacetone as the raw material, the same operation as the Examples 184(1)-(3) gave the title compound.
Yield: 39.8%(3 steps).

### (1) α-Bromo-3-(trifluoromethyl)phenylacetone.

¹H-NMR(CDCl₃): δ2.38(3H, s), 5.43(1H, s), 7.52(1H, t, J=7.8Hz), 7.61-7.66(2H, m), 7.69-7.70(1H, m).

### (2) 2-Amino-4-methyl-5-[3-(trifluoromethyl)phenyl]thiazole.

¹H-NMR(CDCl₃): δ 2.32(3H, s), 4.95(2H, s), 7.46-7.56(3H, m), 7.59-7.61(1H, m).

### (3) 5-Bromo-N-{4-methyl-5-[3-(trifluoromethyl)phenyl]thiazol-2-yl}-2-hydroxybenzamide(Compound No. 190).

¹H-NMR(DMSO-d₆): δ2.40(3H, s), 6.97(1H, d, J=8.7Hz), 7.59(1H, dd, J=8.7, 2.4Hz), 7.71-7.84(4H, m), (2H, m), 8.06(1H, d, J=2.4Hz), 12.09(1H, br), 12.91-13.63(1H, br).

### Example 191: Preparation of the compound of Compound No. 191.

Using 2,2-dimethyl-3-hexanone as the raw material, the same operation as the Examples 184(1)-(3) gave the title compound.
Yield: 17.0%(3 steps).

### (2) 2-Amino-4-[(1,1-dimethyl)ethyl]-5-ethylthiazole.

¹H-NMR(CDCl₃): δ1.21(3H, t, J=7.5Hz), 1.32(9H, s), 2.79(2H, q, J=7.5Hz), 4.63(2H, brs).

### (3) 5-Bromo-N-{4-[(1,1-dimethyl)ethyl]-5-ethylthiazol-2-yl}-2-hydroxybenzamide (Compound No. 191).

¹H-NMR(CDCl₃): δ1.32(3H, t, J=7.5Hz), 1.41(9H, s), 2.88(2H, q, J=7.5Hz), 6.84(1H, d, J=9.0Hz), 7.44(1H, dd, J=8.7, 2.4Hz), 8.05(1H, d, J=2.7Hz), 11.46(2H, br).

### Example 192: Preparation of the compound of Compound No. 192.

Using 5-bromosalicylic acid and 2-amino-4-ethyl-5-phenylthiazole as the raw materials, the same operation as the Example 16 gave the title compound.
Yield: 17.4%.
mp 224-225°C.
¹H-NMR(DMSO-d₆): δ 1.24(3H, t, J=7.6Hz), 2.70(2H, q, J=7.6Hz), 6.95(1H, brd, J=7.6Hz), 7.39-7.42(1H, m), 7.45-7.51(4H, m), 7.56(1H, brd, J=8.0Hz), 8.06(1H, d, J=2.8Hz), 11.98(1H, brs).

### Example 193: Preparation of the compound of Compound No. 193.

Using benzyl isopropyl ketone as the raw material, the same operation as the Examples 184(1)-(3) gave the title compound.
Yield: 4.4%(3 steps).

### (2) 2-Amino-4-isopropyl-5-phenylthiazole.

¹H-NMR(CDCl₃): δ 1.23(6H, d, J=6.6Hz), 3.05(1H, m), 4.94(2H, s), 7.28-7.41(5H, m).

### (3) 5-Bromo-N-(4-isopropyl-5-phenylthiazol-2-yl)-2-hydroxybenzamide(Compound No. 193).

### ¹H-NMR(DMSO-d₆): δ 1.26(6H, d, J=6.0Hz), 3.15(1H, m), 6.98(1H, brs), 7.43-7.53(5H, m), 7.59(1H, brs), 8.08(1H, d, J=2.7Hz), 11.90(1H, brd), 13.33(1H, brd).

### Example 194: Preparation of the compound of Compound No. 194.

Using 1-phenyl-2-hexanone as the raw material, the same operation as the Examples 184(1)-(3) gave the title compound.
Yield: 52.6%(3 steps).

### (1) α-Bromo-1-phenyl-2-hexanone.

¹H-NMR(CDCl₃): δ 0.85(3H, t, J=7.2Hz), 1.19-1.32(2H, m), 1, 50-1.60(2H, m), 2.59(2H, td, J=7.5, 3.9Hz), 5.44(1H, s), 7.34-7.45(5H, m).

### (2) 2-Amino-4-butyl-5-phenylthiazole.

¹H-NMR(CDCl₃): δ0.89(3H, t, J=7.5Hz), 1.28-1.41(2H, m), 1.61-1.71(2H, m), 2.56-2.61(2H, m), 4.87(2H, s), 7.25-7.40(5H, m).

### (3) 5-Bromo-N-(4-butyl-5-phenylthiazol-2-yl)-2-hydroxybenzamide(Compound No. 194).

¹H-NMR(DMSO-d₆): δ 0.85(3H, t, J=7.2Hz), 1.23-1.35(2H, m), 1.59-1.69(2H, m), 2.70(2H, t, J=7.2Hz), 6.96(1H, d, J=6.9Hz), 7.39-7.59(6H, m), 8.07(1H, d, J=2.4Hz), 11.93(1H, br), 13.18-13.59(1H, br).

### Example 195: Preparation of the compound of Compound No. 195.

### (1) 4-Bromo-2,2,6,6-tetramethyl-3,5-heptanedione [α-Bromo-dipivaloylmethane].

2,2,6,6-Tetramethyl-3,5-heptanedione(dipivaloylmethane; 1.00g, 5.42mmol) was dissolved in carbon tetrachloride(10mL). N-Bromosuccinimide(965.8mg, 5.42mmol) was added, and the mixture was refluxed for 2 hours. After cooling, the insoluble matter was filtered off, and the filtrate was evaporated under reduced pressure to give the title compound(1.42g, quant.) as a white crystal.
¹H-NMR(CDCl₃): δ1.27(18H, s), 5.67(1H, s).

When the method described in Example 195(1) is referred in the following examples, N-bromosuccinimide was used as the brominating agent. As the reaction solvent, solvents such as carbon tetrachloride or the like were used.

### (2) 2-Amino-4-[(1,1-dimethyl)ethyl]-5-[(2,2-dimethyl)propionyl]thiazole.

A mixture of 4-bromo-2,2,6,6-tetramethyl-3,5-heptanedione(α-bromodipivaloylmethane; 1.42g, 5.40mmol), thiourea(451.8mg, 5.94mmol) and ethanol(15mL) was refluxed for 2 hours. After cooling, the reaction mixture was poured into saturated aqueous sodium hydrogen carbonate and extracted with ethyl acetate. After the organic layer was washed with water and brine, dried over anhydrous sodium sulfate, the residue obtained by evaporation under reduced pressure was crystallized(dichloromethane/hexane) to give the title compound(1.23g, 94.5%) as a white crystal.
¹H-NMR(CDCl₃): δ1.26(9H, s), 1.29(9H, s), 5.03(2H, s).

### (3) 5-Chloro-N-{4-[(1,1-dimethyl)ethyl]-5-[(2,2-dimethyl)propionyl]thiazol-2-yl}-2-hydroxybenzamide(Compound No. 195).

A mixture of 5-chlorosalicylic acid(143.6mg, 0.83mmol), 2-amino-4-[(1,1-dimethyl)ethyl]-5-[(2,2-dimethyl)propionyl]thiazole(200.0mg, 0.83mmol), phophorus trichloride(40µl, 0.46mmol) and chlorobenzene(4mL) was refluxed for 3 hours. The residue obtained by concentration of the reaction mixture under reduced pressure was purified by chromatography on silica gel(n-hexane:ethyl acetate=3:1) to give the title compound(159.1mg, 48.4%) as a white powder.
¹H-NMR(CDCl₃): δ1.33(9H, s), 1.35(9H, s), 6.99(1H, d, J=8.7Hz), 7.43(1H, dd, J=9.0, 2.7Hz), 7.70(1H, d, J=2.7Hz), 10.52(2H, br).

When the method described in Example 195(3) is referred in the following examples, phophorus trichloride was used as the acid halogenating agent. As the reaction solvent, solvents such as monochlorobenzene, toluene or the like were used.

### Example 196: Preparation of the compound of Compound No. 196.

Using 5-bromosalicylic acid and 2-amino-4-[(1,1-dimethyl)ethyl]-5-[(2,2-dimethyl)propionyl]thiazole(compound of Example 195(2)) as the raw materials, the same operation as the Example 195(3) gave the title compound.
Yield: 23.8%.
¹H-NMR(CDCl₃): δ1.33(9H, s), 1.35(9H, s), 6.94(1H, d, J=8, 7Hz), 7.55(1H, dd, J=8.7, 2.1Hz), 7.85(1H, d, J=2.1Hz), 10.51(2H, br).

### Example 197: Preparation of the compound of Compound No. 197.

Using pivaloylacetic acid ethyl ester as the raw material, the same operation as the Examples 195(1)-(3) gave the title compound.
Yield: 45.7%(3 steps).

### (1) α-Bromo-pivaloylacetic acid ethyl ester.

¹H-NMR(CDCl₃): δ1.28(9H, s), 1.29(3H, t, J=7.2Hz), 4.26(2H, q, J=7.2Hz), 5.24(1H, s).

### (2) 2-Amino-4-[(1,1-dimethyl)ethyl]thiazole-5-carboxylic acid ethyl ester.

¹H-NMR(CDCl₃): δ1.32(3H, t, J=7.2Hz), 1.43(9H, s), 4.24(2H, q, J=7.2Hz), 5.18(2H, s).

### (3) 2-(5-Bromo-2-hydroxybenzoyl)amino-4-[(1,1-dimethyl)ethyl]thiazole-5-carboxylic acid ethyl ester(Compound No. 197).

¹H-NMR(DMSO-d₆): δ 1.30(3H, t, J=7.2Hz), 1.44(9H, s), 4.27(2H, q, J=6.9Hz), 7.00(1H, d, J=8.7Hz), 7.63(1H, dd, J=8.7, 2.7Hz), 8.02(1H, d, J=2.4Hz), 11.80(1H, br), 12.12(1H, br).

### Example 198: Preparation of the compound of Compound No. 198.

### (1) 2-Amino-5-bromo-4-[(1,1-dimethyl)ethyl]thiazole.

2-Amino-4-[(1,1-dimethyl)ethyl]thiazole(compound of Example 181(1); 0.87g, 5.6mmol) was dissolved in carbon tetrachloride(9mL). N-Bromosuccinimide(1.00g, 5.6mmol) was added, and the mixture was stirred at room temperature for 1 hour. Hexane was added to the reaction mixture. The insoluble matter was filtered off, and the residue obtained by evaporation of the filtrate under reduced pressure was purified by chromatography on silica gel(hexane:ethyl acetate=2:1) to give the title compound(1.23g, 93.7%) as an yellowish gray powder.
¹H-NMR(CDCl₃): δ 1.39(9H, s), 4.81(2H, brs).

### (2) 2-Amino-4-[(1,1-dimethyl)ethyl]-5-piperidinothiazole.

A mixture of 2-amino-5-bromo-4-[(1,1-dimethyl)ethyl]thiazole(0.10g, 0.42mmol), piperidine(0.1mL), potassium carbonate(0.20g) and acetonitrile(4mL) was refluxed for 3 hours. The reaction mixture was poured into water and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=2:1) to give the title compound(80.7mg, 79.3%) as an yellow crystal.
¹H-NMR(CDCl₃): δ1.32(9H, s), 1.64(4H, t, J=5.7Hz), 1.71-1.77(2H, m), 2.35(2H, brs), 2.99(2H, brs), 4.68(2H, s).

When the preparation method described in Example 198(2) is referred in the following examples, bases such as potassium carbonate or the like were used as the base. As the reaction solvent, solvents such as acetonitrile or the like were used.

### (3) 2-Acetoxy-5-bromo-N-{4-[(1,1-dimethyl)ethyl]-5-piperidinothiazol-2-yl}benzamide.

Under argon atmosphere, phosphorus oxychloride(46µl, 0.50mmol) was added to a mixture of 2-acetoxy-5-bromobenzoic acid(90.3mg, 0.35mmol), 2-amino-4-[(1,1-dimethyl)ethyl]-5-piperidinothiazole(80.7mg, 0.34mmol), pyridine(0.1mL) and tetrahydrofuran(3mL), and the mixture was stirred at room temperature for 2 hours. The reaction mixture was poured into 2N hydrochloric acid and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=3:1) to give the title compound(84.3mg) as a crude product.

When the preparation method described in Example 198(3) is referred in the following examples, phosphorus oxychloride was used as the acid halogenating agent. As the reaction base, pyridine was used. As the reaction solvent, solvents such as dichloromethane, tetrahydrofuran or the like were used.

### (4) 5-Bromo-N-{4-[(1,1-dimethyl)ethyl]-5-piperidinothiazol-2-yl}-2-hydroxybenzamide (Compound No. 198).

2-Acetoxy-5-bromo-N-{4-[(1,1-dimethyl)ethyl]-5-piperidinothiazol-2-yl}benzamide(crude product, 84.3mg) was dissolved in ethanol(3mL). 2N Aqueous sodium hydroxide(0.1mL) was added, and the mixture was stirred at room temperature for 1 hour. The reaction mixture was poured into 2N hydrochloric acid and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=4:1) to give the title compound(54.1mg, 36.3%; 2 steps) as a white powder.
¹H-NMR(CDCl₃): δ 1.41(9H, s), 1.56(2H, brs), 1.67-1.74(4H, m), 2.79(4H, brs), 6.85(1H, d, J=9.0Hz), 7.45(1H, dd, J=9.0, 2.4Hz), 8.06(1H, d, J=2.4Hz), 11.70(2H, br).

When the preparation method described in Example 198(4) is referred in the following examples, inorganic bases such as sodium hydroxide, potassium carbonate or the like were used as the base. As the reaction solvent, solvents such as water, methanol, ethanol, tetrahydrofuran or the like were used alone or as a mixture.

### Example 199: Preparation of the compound of Compound No. 199.

Using 2-amino-5-bromo-4-[(1,1-dimethyl)ethyl]thiazole(compound of Example 198(1)) and morpholine as the raw materials, the same operation as the Examples 198(2)-(4) gave the title compound.
Yield: 17.1%.

### (2) 2-Amino-4-[(1,1-dimethyl)ethyl]-5-morpholinothiazole.

¹H-NMR(CDCl₃): δ1.33(9H, s), 2.76(4H, brs), 3.79(4H, brs), 4.66(2H, s).

### (3) 2-Acetoxy-5-bromo-N-{4-[(1,1-dimethyl)ethyl]-5-morpholinothiazol-2-yl}benzamide.

The product was used for the next reaction as a crude product.

### (4) 5-Bromo-N-{4-[(1,1-dimethyl)ethyl]-5-morpholinothiazol-2-yl}-2-hydroxybenzamide (Compound No. 199).

¹H-NMR(CDCl₃): δ 1.24(9H, s), 2.89(4H, dd, J=4.8, 4.2Hz), 3.83(4H, dd, J=4.5, 4.2Hz), 6.89(1H, d, J=9.0Hz), 7.49(1H, dd, J=9.0, 2.4Hz), 7.98(1H, d, J=2.1Hz), 11.20(2H, br).

### Example 200: Preparation of the compound of Compound No. 200.

Using 2-amino-5-bromo-4-[(1,1-dimethyl)ethyl]thiazole(compound of Example 198(1)) and 4-methylpiperazine as the raw materials, the same operation as the Examples 198(2)-(4) gave the title compound.
Yield: 6.9%.

### (2) 2-Amino-4-[(1,1-dimethyl)ethyl]-5-(4-methylpiperazin-1-yl)thiazole.

¹H-NMR(DMSO-d₆): δ1.25(9H, s), 2.12(2H, brs), 2.19(3H, s), 2.57(2H, brs), 2.72(4H, brs), 6.51(2H, s).

### (3) 2-Acetoxy-N-{4-[(1,1-dimethyl)ethyl]-5-(4-methylpiperazin-1-yl)thiazol-2-yl}benzamide.

The product was used for the next reaction as a crude product.

### (4) 5-Bromo-N-{4-[(1,1-dimethyl)ethyl]-5-(4-methylpiperazin-1-yl)thiazol-2-yl}-2-hydroxybenzamide(Compound No. 200).

¹H-NMR(CD₃OD): δ1.41(9H, s), 2.55(3H, s), 2.87(4H, brs), 3.03(4H, brs), 6.88(1H, d, J=8.7Hz), 7.49(1H, dd, J=8.7, 2.7Hz), 8.11(1H, d, J=2.7Hz).

### Example 201: Preparation of the compound of Compound No. 201.

Using 2-amino-5-bromo-4-[(1,1-dimethyl)ethyl]thiazole(compound of Example 198(1)) and 4-phenylpiperazine as the raw materials, the same operation as the Examples 198(2)-(4) gave the title compound.
Yield: 6.9%.

### (2) 2-Amino-4-[(1,1-dimethyl)ethyl]-5-(4-phenylpiperazin-1-yl)thiazole.

¹H-NMR(CDCl₃): δ 1.34(9H, s), 2.80(2H, brs), 3.03(4H, brs), 3.55(2H, brs), 4.69(2H, s), 6.88(1H, tt, J=7.2, 1.2Hz), 6.95(2H, dd, J=9.0, 1.2Hz), 7.28(2H, dd, J=8.7, 7.2Hz).

### (3) 2-Acetoxy-5-bromo-N-{4-[(1,1-dimethyl)ethyl]-5-(4-phenylpiperazin-1-yl)thiazol-2-yl}benzamide.

The product was used for the next reaction as a crude product.

### (4) 5-Bromo-N-{4-[(1,1-dimethyl)ethyl]-5-(4-phenylpiperazin-1-yl)thiazol-2-yl}-2-hydroxybenzamide(Compound No. 201).

¹H-NMR(DMSO-d₆): δ1.39(9H, s), 2.97(4H, s), 3.30(4H, s), 6.82(1H, t, J=7.5Hz), 6.97(2H, brs), 6.99(2H, t, J=7.5Hz), 7.58(1H, brs), 8.05(1H, d, J=2.4Hz), 11.69(1H, brs), 11.82(1H, brs).

### Example 202: Preparation of the compound of Compound No. 202.

Using 5-bromosalicylic acid and 2-amino-4-phenylthiazole as the raw materials, the same operation as the Example 195(3) gave the title compound.
Yield: 16.0%.
mp 239°C(dec.).
¹H-NMR(DMSO-d₆): δ 7.02(1H, d, J=8.4Hz), 7.34(1H, t, J=7.6Hz), 7.44(2H, t, J=7.6Hz), 7.62(1H, dd, J=8.4, 2.8Hz), 7.67(1H, s), 7.92(2H, d, J=7.2Hz), 8.08(1H, d, J=2.8Hz), 11.88(1H, brs), 12.05(1H, brs).

### Example 203: Preparation of the compound of Compound No. 203.

### (1) {2-[(5-Bromo-2-hydroxybenzoyl)amino]-4-phenylthiazol-5-yl}acetic acid methyl ester.

Using 5-bromosalicylic acid and 2-amino-4-phenylthiazole-5-acetic acid methyl ester as the raw materials, the same operation as the Example 195(3) gave the title compound.
Yield: 32.1%.
mp 288.5-229.5°C.
¹H-NMR(DMSO-d₆): δ 3.66(3H, s), 3.95(2H, s), 6.99(1H, d, J=8.0Hz), 7.42(1H, d, J=6.0Hz), 7.48(2H, brt, J=7.6Hz), 7.56-7.61(3H, m), 8.07(1H, d, J=2.4Hz), 11.85(1H, brs), 11.98(1H, brs).

### (2) {2-[(5-Bromo-2-hydroxybenzoyl)amino]-4-phenylthiazol-5-yl}acetic acid(Compound No. 203).

{2-[(5-Bromo-2-hydroxybenzoyl)amino]-4-phenylthiazol-5-yl}acetic acid methyl ester(75mg, 0.17mmol) was dissolved in methanol(5mL). 2N Sodium hydroxide(0.5mL, lmmol) was added, and the mixture was stirred at room temperature for 12 hours. The reaction mixture was poured into 2N hydrochloric acid and extracted with ethyl acetate. After the ethyl acetate layer was washed successively with water and brine, dried over anhydrous sodium sulfate, the solvent was evaporated under reduced pressure. The obtained residue was suspended and washed with n-hexane-ethyl acetate under heating at reflux to give the title compound(56mg, 77.3%) as a light yellow white crystal.
mp 284-286°C.
¹H-NMR(DMSO-d₆): δ 3.84(2H, s), 6.98(1H, d, J=8.8Hz), 7.42(1H, d, J=6.8Hz), 7.49(2H, t, J=7.6Hz), 7.58-7.61(3H, m), 8.07(1H, d, J=2.8Hz), 12.25(H, brs).

### Example 204: Preparation of the compound of Compound No. 204.

Using 5-bromosalicylic acid and 2-amino-4,5-diphenylthiazole as the raw materials, the same operation as the Example 195(3) gave the title compound.
Yield: 25.9%.
mp 262-263°C.
¹H-NMR(DMSO-d₆): δ7.02(1H, d, J=8.1Hz), 7.34-7.47(10H, m), 7.63(1H, d, J=6.9Hz), 8.08(1H, d, J=2.4Hz), 11.88(1H, brs), 12.08(1H, brs).

### [2-Amino-4,5-diphenylthiazole: Refer to "Nihon Kagaku Zasshi", 1962, Vol.83, p.209.]

### Example 205: Preparation of the compound of Compound No. 205.

Using 5-bromosalicylic acid and 2-amino-4-benzyl-5-phenylthiazole as the raw materials, the same operation as the Example 195(3) gave the title compound.
Yield: 28.1%.
mp 198-200°C.
¹H-NMR(DMSO-d₆): δ4.08(2H, s), 6.95(1H, d, J=8.8Hz), 7.15-7.22(3H, m), 7.30(2H, t, J=7.6Hz), 7.38-7.43(1H, m), 7.47(4H, d, J=4.4Hz), 7.57(1H, brd, J=8.8Hz), 8.05(1H, d, J=2.4Hz), 11.98(1H, brs).

### [2-Amino-4-benzyl-5-phenylthiazole: Refer to "Chemical and Pharmaceutical Bulletin", 1962, Vol.10, p.376.]

### Example 206: Preparation of the compound of Compound No. 206.

Using 5-bromosalicylic acid and 2-amino-5-phenyl-4-(trifluoromethyl)thiazole as the raw materials, the same operation as the Example 195(3) gave the title compound.
Yield: 33.2%.
mp 250°C(dec.). ¹H-NMR(DMSO-d₆): δ 7.02(1H, d, J=8.8Hz), 7.51(5H, s), 7.63(1H, dd, J=8.8, 2.4Hz), 8.02(1H, d, J=2.8Hz), 12.38(1H, brs).

### Example 207: Preparation of the compound of Compound No. 207.

Using 1-phenyl-1,3-butanedione as the raw material, the same operation as the Examples 195(1)-(3) gave the title compound.
Yield: 8.9%(3 steps).

### (1) α-Bromo-1-phenyl-1,3-butanedione.

¹H-NMR(CDCl₃): δ2.46(3H, s), 5.62(1H, s), 7.48-7.54(2H, m), 7.64(1H, tt, J=7.5, 2.1Hz), 7.97-8.01(2H, m).

### (2) 2-Amino-5-acetyl-4-phenylthiazole.

¹H-NMR(DMSO-d₆): δ2.18(3H, s), 7.50-7.55(2H, m), 7.59-7.68(3H, m), 8.69(2H, brs).

### (3) 5-Bromo-N-(5-acetyl-4-phenylthiazol-2-yl)-2-hydroxybenzamide(Compound No. 207).

¹H-NMR(DMSO-d₆): δ 2.44(3H, s), 6.99(1H, d, J=9.0Hz), 7.55-7.71(4H, m), 7.76-7.80(2H, m), 8.01(1H, d, J=2.4Hz), 12.36(2H, br).

### Example 208: Preparation of the compound of Compound No. 208.

Using 1,3-diphenyl-1,3-propanedione as the raw material, the same operation as the Examples 195(1)-(3) gave the title compound.
Yield: 49.7%.

### (1) α-Bromo-1,3-diphenyl-1,3-propanedione.

¹H-NMR(CDCl₃): δ 6.55(1H, s), 7.45-7.50(4H, m), 7.61(2H, tt, J=7.2, 2.1Hz), 7.98-8.01(4H, m).

### (2) 2-Amino-5-benzoyl-4-phenylthiazole.

¹H-NMR(DMSO-d₆): δ 7.04-7.18(5H, m), 7.22-7.32(3H, m), 7.35-7.38(2H, m), 8.02(2H, s).

### (3) 5-Bromo-N-(5-benzoyl-4-phenylthiazol-2-yl)-2-hydroxybenzamide(Compound No. 208).

¹H-NMR(DMSO-d₆): δ 7.03(1H, d, J=8.7Hz), 7.17-7.30(5H, m), 7.39-7.47(3H, m), 7.57-7.60(2H, m), 7.64(1H, dd, J=8.7, 2.7Hz), 8.05(1H, d, J=2.4Hz), 11.82(1H, brs), 12.35(1H, brs).

### Example 209: Preparation of the compound of Compound No. 210.

Using 5-chlorosalicylic acid and 2-amino-4-phenylthiazole-5-carboxylic acid ethyl ester as the raw materials, the same operation as the Example 195(3) gave the title compound.
Yield: 69.4%.
¹H-NMR(DMSO-d₆): δ1.22(3H, t, J=7.5Hz), 4.21(2H, q, J=7.5Hz), 7.07(1H, d, J=8.7Hz), 7.43-7.47(3H, m), 7.53(1H, dd, J=8.7, 2.4Hz), 7.70-7.74(2H, m), 7.92(1H, d, J=3.0Hz), 11.88(1H, br), 12.29(1H, brs).

### Example 210: Preparation of the compound of Compound No. 209.

Using 5-bromosalicylic acid and 2-amino-4-phenylthiazole-5-carboxylic acid ethyl ester as the raw materials, the same operation as the Example 195(3) gave the title compound.
Yield: 28.6%.
mp 197-199°C.
¹H-NMR(DMSO-d₆): δ 1.21(3H, t, J=6.8Hz), 4.20(2H, q, J=6.8Hz), 7.01(1H, d, J=8.8Hz), 7.43-7.48(3H, m), 7.63(1H, dd, J=8.8, 2.4Hz), 7.70-7.72(2H, m), 8.04(1H, d, J=2.4Hz), 12.33(1H, brs).

### Example 211: Preparation of the compound of Compound No. 211.

Using pentafluorobenzoylacetic acid ethyl ester as the raw material, the same operation as the Examples 195(1)-(3) gave the title compound.
Yield: 40.0%(3 steps).

### (1) α-Bromo-pentafluorobenzoylacetic acid ethyl ester.

It was used for the next reaction as a crude product.

### (2) 2-Amino-4-(pentafluorophenyl)thiazole-5-carboxylic acid ethyl ester.

¹H-NMR(CDCl₃): δ 1.23(3H, t, J=7.2Hz), 4.21(2H, q, J=7.2Hz), 5.41(2H, s).

### (3) Ethyl 2-(5-bromo-2-hydroxybenzoyl)amino-4-(pentafluorophenyl)thiazole-5-carboxylate(Compound No. 211).

¹H-NMR(DMSO-d₆): δ1.20(3H, t, J=7.2Hz), 2.51(2H, q, J=7.2Hz), 7.02(1H, d, J=8.7Hz), 7.64(1H, dd, J=8.7, 2.7Hz), 7.90(1H, d, J=3.0Hz), 11.92(1H, br), 12.58(1H, br).

### Example 212: Preparation of the compound of Compound No. 212.

### (1) 2-(5-Bromo-2-hydroxybenzoyl)amino-4-phenylthiazole-5-carboxylic acid.

Using 2-(5-bromo-2-hydroxybenzoyl)amino-4-phenylthiazole-5-carboxylic acid ethyl ester(compound No. 209) as the raw material, the same operation as the Example 82 gave the title compound.
Yield: 67.0%.
¹H-NMR(DMSO-d₆): δ 7.00(1H, d, J=8.8Hz), 7.42-7.44(3H, m), 7.62(1H, dd, J=8.8, 2.4Hz), 7.70-7.72(2H, m), 8.04(1H, d, J=2.4Hz), 12.31(1H, brs), 12.99(1H, brs).

### (2) [2-(5-Bromo-2-hydroxybenzoyl)amino-4-phenylthiazol-5-yl]-N-methylcarboxamide (Compound No. 212).

A mixure of 2-(5-bromo-2-hydroxybenzoyl)amino-4-phenylthiazole-5-carboxylic acid(0.20g, 0.48mmol), methylamine 40% methanol solution(0.2ml), 1-hydroxybenzotriazole hydrate(96.7mg, 0.72mmol), WSC · HCl(137.2mg, 0.72mmol) and tetrahydrofuran(15mL) was stirred at room temperature for 18 hours. The reaction mixture was poured into 2N hydrochloric acid and extracted with ethyl acetate. After the organic layer was washed with water and brine, dried over anhydrous sodium sulfate, the residue obtained by evaporation under reduced pressure was purified by chromatography on silica gel(n-hexane:ethyl acetate=1:2), and crystallized(dichloromethane/n-hexane) to give the title compound(87.9mg, 42.6%) as a white powder.
¹H-NMR(DMSO-d₆): δ 2.70(3H, d, J=4.5Hz), 7.02(1H, d, J=9.0Hz), 7.40-7.48(3H, m), 7.63(1H, dd, J=9.0, 2.4Hz), 7.68-7.71(2H, m), 8.06(1H, d, J=2.4Hz), 8.16(1H, t, J=4.5Hz), 11.88(1H, br), 12.15(1H, brs).

When the method described in Example 212(2) is referred in the following examples, WSC · HCl and 1-hydroxybenzotriazole hydrate were used as the dehydrocondensating agent. As the reaction solvent, solvents such as tetrahydrofuran or the like were used.

### Example 213: Preparation of the compound of Compound No. 213.

Using 2-(5-bromo-2-hydroxybenzoyl)amino-4-phenylthiazole-5-carboxylic acid (compound of Example 212(1)) and 70% aqueous ethylamine solution as the raw materials, the same operation as the Example 212(2) gave the title compound.
Yield: 62.5%.
¹H-NMR(DMSO-d₆): δ 1.05(3H, t, J=6.9Hz), 3.15-3.24(2H, m), 7.02(1H, d, J=8.7Hz), 7.40-7.47(3H, m), 7.63(1H, dd, J=8.7, 3.0Hz), 7.69-7.72(2H, m), 8.06(1H, d, J=2.4Hz), 8.20(1H, t, J=5.4Hz), 11.84(1H, br), 12.14(1H, brs).

### Example 214: Preparation of the compound of Compound No. 214.

Using 2-(5-bromo-2-hydroxybenzoyl)amino-4-phenylthiazole-5-carboxylic acid (compound of Example 212(1)) and isopropylamine as the raw materials, the same operation as the Example 212(2) gave the title compound.
Yield: 23.9%.
¹H-NMR(DMSO-d₆): δ 1.07(6H, d, J=6.3Hz), 4.02(1H, m), 7.02(1H, d, J=9.0Hz), 7.40-7.52(3H, m), 7.64(1H, dd, J=8.7, 2.7Hz), 7.69-7.73(2H, m), 8.06(1H, d, J=2.7Hz), 11.89(1H, br), 12.14(1H, brs).

### Example 215: Preparation of the compound of Compound No. 215.

Using 2-(5-bromo-2-hydroxybenzoyl)amino-4-phenylthiazole-5-carboxylic acid (compound of Example 212(1)) and 2-phenethylamine as the raw materials, the same operation as the Example 212(2) gave the title compound.
Yield: 62.2%.
¹H-NMR(DMSO-d₆): δ 2.78(2H, t, J=7.5Hz), 3.43(2H, q, J=7.5Hz), 7.02(1H, d, J=9.0Hz), 7.19-7.24(3H, m), 7.27-7.33(2H, m), 7.39-7.41(3H, m), 7.61-7.65(3H, m), 8.06(1H, d, J=2.4Hz), 8.25(1H, t, J=6.0Hz), 11.85(1H, brs), 12.15(1H, brs).

### Example 216: Preparation of the compound of Compound No. 216.

Using 5-bromosalicylic acid and 2-amino-4-(trifluoromethyl)thiazole-5-carboxylic acid ethyl ester as the raw materials, the same operation as the Example 195(3) gave the title compound.
Yield: 88.7%.
¹H-NMR(DMSO-d₆): δ1.32(3H, t, J=7.2Hz), 4.33(2H, q, J=7.2Hz), 7.01(1H, d, J=8.7Hz), 7.63(1H, dd, J=8.7, 2.7Hz), 7.98(1H, d, J=2.4Hz), 12.64(1H, br).

### Example 217: Preparation of the compound of Compound No. 217.

Using 5-chloro-N-{4-[(1,1-dimethyl)ethyl]-5-[(2,2-dimethyl)propionyl]thiazol-2-yl}-2-hydroxybenzamide(compound No. 195) and acetyl chloride as the raw materials, the same operation as the Example 96 gave the title compound.
Yield: 65.3%.
¹H-NMR(CDCl₃): δ1.32(9H, s), 1.33(9H,s),2.46(3H, s), 7.22(1H, d, J=8.4Hz), 7.56(1H, dd, J=8.7, 2.4Hz), 8.05(1H, d, J=2.7Hz), 9.82(1H, brs).

### Example 218: Preparation of the compound of Compound No. 218.

Using 4-hydroxybiphenyl-3-carboxylic acid and 2-amino-4-phenylthiazole-5-carboxylic acid ethyl ester as the raw materials, the same operation as the Example 195(3) gave the title compound.
Yield: 61.7%.
mp 207-208°C.
¹H-NMR(DMSO-d₆): δ1.23(3H, t, J=7.2Hz), 4.22(2H, q, J=7.2Hz), 7.16(1H, d, J=8.7Hz), 7.36(1H, t, J=7.5Hz), 7.45-7.50(5H, m), 7.69-7.76(4H, m), 7.85(1H, dd, J=8.7, 2.4Hz), 8.31(1H, d, J=2.4Hz), 11.73(1H, brs), 12.60(1H, brs).

### [4-Hydroxybiphenyl-3-carboxylic acid: Refer to "Tetrahedron", 1997, Vol.53, p.11437.]

### Example 219: Preparation of the compound of Compound No. 219.

Using (4'-fluoro-4-hydroxybiphenyl)-3-carboxylic acid and 2-amino-4-phenylthiazole-5-carboxylic acid ethyl ester as the raw materials, the same operation as the Example 195(3) gave the title compound.
Yield: 62.7%.
mp 237-238°C.
¹H-NMR(DMSO-d₆): δ 1.22(3H, t, J=7.2Hz), 4.21(2H, q, J=7.2Hz), 7.13(1H, d, J=8.4Hz), 7.28(2H, t, J=8.8Hz), 7.44-7.45(3H, m), 7.71-7.75(4H, m), 7.81(1H, dd, J=8.8, 2.4Hz), 8.27(1H, d, J=2.4Hz), 11.67(1H, brs), 12.58(1H, brs).

### [(4'-Fluoro-4-hydroxybiphenyl)-3-carboxylic acid: Refer to "Tetrahedron", 1997, Vol.53, p.11437.]

### Example 220: Preparation of the compound of Compound No. 220.

Using (2',4'-difluoro-4-hydroxybiphenyl)-3-carboxylic acid and 2-amino-4-phenylthiazole-5-carboxylic acid ethyl ester as the raw materials, the same operation as the Example 195(3) gave the title compound.
Yield: 45.6%.
mp 206-207°C.
¹H-NMR(DMSO-d₆): δ1.22(3H, t, J=7.2Hz), 4.22(2H, q, J=7, 2Hz), 7.17(1H, d, J=9.0Hz), 7.21(1H, td, J=8.7, 2.4Hz), 7.38(1H, ddd, J=11.7, 9.3, 2.4Hz), 7.44-7.46(3H, m), 7.60-7.75(4H, m), 8.13-8.14(1H, m), 11.86(1H, brs), 12.46(1H, brs).

### Example 221: Preparation of the compound of Compound No. 221.

### (1) [4-Hydroxy-4'-(trifluoromethyl)biphenyl]-3-carboxylic acid.

A mixture of 5-bromosalicylic acid(500mg, 2.30mmol), dihydroxy-4-(trifluoromethyl)phenylborane(488mg, 2.57mmol), palladium acetate(10mg, 0.040mmol) and 1M sodium carbonate(7mL) was stirred at 80°C for 1 hour. The reaction mixture was poured into 2N hydrochloric acid and extracted with ethyl acetate. After the ethyl acetate layer was washed successively with water and brine, dried over anhydrous sodium sulfate, the solvent was evaporated under reduced pressure. According to the fixed procedure, the obtained residue was methyl-esterified by trimethylsilyldiazomethane and methanol, and purified by column chromatography on silica gel(n-hexane:ethyl acetate=5:1) to give a colourless liquid(563mg). This liquid was dissolved in methanol(10mL). 2N Sodium hydroxide(3mL) was added, and the mixture was stirred at 60°C for 1 hour. After the reaction mixture was cooled to room temperature, it was poured into 2N hydrochloric acid and extracted with ethyl acetate. After the ethyl acetate layer was washed successively with water and saturted brine, dried over anhydrous magnesium sulfate, the solvent was evaporated under reduced pressure. The obtained residue was suspended and washed with n-hexane-dichloromethane under heating at reflux to give the title compound(458mg, 70.4%) as a white crystal.
mp 185°C(dec).
¹H-NMR(DMSO-d₆): δ 7.09(1H, d, J=8.8Hz), 7.77(2H, d, J=8.0Hz), 7.85(2H, d, J=8.0Hz), 7.90(1H, dd, J=8.8, 2.0Hz), 8.10(1H, d, J=2.4Hz), 11.80(1H, brs).

### (2) 2-{[4-Hydroxy-4'-(trifluoromethyl)biphenyl]-3-carbonyl}amino-4-phenylthiazole-5-carboxylic acid ethyl ester(Compound No. 221).

Using [4-hydroxy-4'-(trifluoromethyl)biphenyl]-3-carboxylic acid and 2-amino-4-phenylthiazole-5-carboxylic acid ethyl ester as the raw materials, the same operation as the Example 195(3) gave the title compound.
Yield: 41.7%.
mp 236-237°C.
¹H-NMR(DMSO-d₆): δ 1.22(3H, t, J=7.2Hz), 4.21(2H, q, J=7.2Hz), 7.18(1H, d, J=8.8Hz), 7.44-7.45(3H, m), 7.72-7.74(2H, m), 7.81(2H, d, J=8.4Hz), 7.91(1H, dd, J=8.8, 2.4Hz), 7.93(2H, d, J=8.4Hz), 8.36(1H, d, J=2.4Hz), 11.78(1H, brs), 12.62(1H, brs).

### Example 222: Preparation of the compound of Compound No. 222.

Using 2-hydroxy-5-(1-pyrrolyl)benzoic acid and 2-amino-4-phenylthiazole-5-carboxylic acid ethyl ester as the raw materials, the same operation as the Example 195(3) gave the title compound.
Yield: 55.0%.
¹H-NMR(DMSO-d₆): δ 1.22(3H, t, J=7.2Hz), 4.22(2H, q, J=7.2Hz), 6.26(2H, t, J=2.1Hz), 7.13(1H, d, J=8.7Hz), 7.32(2H, t, J=2.1Hz), 7.43-7.47(3H, m), 7.70-7.75(3H, m), 8.09(1H, d, J=2.7Hz), 11.58(1H, brs), 12.55(1H, brs).

### Example 223: Preparation of the compound of Compound No. 223.

### (1) 2-Hydroxy-5-(2-thienyl)benzoic acid.

5-Bromosalicylic acid(500mg, 2.30mmol) was dissolved in 1,2-dimethoxyethane(5mL). Tetrakis(triphenylphosphine)palladium(80mg, 0.07mmol) was added under argon atmosphere, and the mixture was stirred at room temperature for 10 minutes. Then dihydroxy-2-thienylborane(324mg, 2.53mmol) and 1M sodium carbonate(7mL) were added, and the mixture was refluxed for 2 hours. After the reaction mixture was cooled to room temperature, it was poured into 2N hydrochloric acid and extracted with ethyl acetate. After the ethyl acetate layer was washed successively with water and brine, dried over anhydrous sodium sulfate, the solvent was evaporated under reduced pressure. According to the fixed procedure, the obtained residue was methyl-esterified by trimethylsilyldiazomethane and methanol, and purified by column chromatography on silica gel(n-hexane:ethyl acetate=5:1) to give an yellow liquid(277mg). This was dissolved in methanol(5mL). 2N Sodium hydroxide(1.5mL) was added, and the mixture was stirred at 60°C for 1 hour. After the reaction mixture was cooled to room temperature, it was poured into 2N hydrochloric acid and extracted with ethyl acetate. After the ethyl acetate layer was washed successively with water and brine, dried over anhydrous magnesium sulfate, the residue obtained by evaporation of the solvent under reduced pressure was crystallized from n-hexane-dichloromethane to give the title compound(58mg, 11.5%) as a white crystal.
¹H-NMR(DMSO-d₆): δ6.95(1H, d, J=8.8Hz), 7.09(1H, dd, J=4.8, 3.6Hz), 7.37(1H, dd, J=4.0, 1.2Hz), 7.45(1H, dd, J=5.2, 1.2Hz), 7.74(1H, dd, J=8.8, 2.8Hz), 7.96(1H, d, J=2.8Hz).

### (2) 2-[2-Hydroxy-5-(2-thienyl)benzoyl]amino-4-phenylthiazole-5-carboxylic acid ethyl ester(Compound No. 223).

Using 2-hydroxy-5-(2-thienyl)benzoic acid and 2-amino-4-phenylthiazole-5-carboxylic acid ethyl ester as the raw materials, the same operation as the Example 195(3) gave the title compound.
Yield: 58.2%.
mp 213-214°C.
¹H-NMR(DMSO-d₆): δ 1.22(3H, t, J=7.2Hz), 4.21(2H, q, J=7.2Hz), 7.10(1H, d, J=9.2Hz), 7.12(1H, dd, J=4.8, 3.6Hz), 7.44-7.46(4H, m), 7.50(1H, dd, J=4.8, 1.2Hz), 7.71-7.74(2H, m), 7.79(1H, dd, J=8.8, 2.4Hz), 8.21(1H, d, J=2.4Hz), 11.78(1H, brs), 12.44(1H, brs).

### Example 301: Preparation of the compound of Compound No. 301.

### (1) 5-Chloro-2-methoxy-β-phenylstyrene.

Palladium acetate(21mg, 7mol%) was added to a solution of 2-bromo-4-chloroanisole(300mg, 1.4mmol), styrene(211mg, 2mmol), triethylamine(13 µL, 0.1mmol) and triphenylphosphine(50mg, 1.9mmol) in acetonitrile(6mL), and the mixture was refluxed for 8 hours under argon atmosphere. After the reaction mixture was cooled to room temperature, the solvent was concentrated under reduced pressure and the obtained residue was diluted with ethyl acetate(15mL). After the solution was washed successively with 2N hydrochloric acid, water and brine, dried over anhydrous sodium sulfate, the residue obtained by evaporation of the solvent under reduced pressure was purified by column chromatography on silica gel(n-hexane:ethyl acetate=10:1) to give the title compound(118mg, 35.6%) as a white powder.
¹H-NMR(CDCl₃):d 3.85(3H, s), 6.80(1H, d, J=8.8Hz), 7.08(1H, d, J=16.8Hz), 7.17(1H, dd, J=8.8, 2.5Hz), 7.20-7.42(4H, m), 7.51-7.55(3H, m).

### (2) 4-Chloro-2-styrylphenol(Compound No. 301).

Under argon atmosphere, 1mol/L boron tribromide/dichloromethane solution(0.5mL, 0.5mmol) was added to a solution of 5-chloro-2-methoxy-β-phenylstyrene(80mg, 0.3mmol) in dichloromethane(2mL) at room temperature, and the mixture was stirred for 12 hours. The reaction mixture was diluted with ethyl acetate(15mL), and after it was washed successively with water and brine, dried over anhydrous sodium sulfate, the residue obtained by evaporation of the solvent under reduced pressure was purified by column chromatography on silica gel(n-hexane:ethyl acetate=3:1) to give the title compound(34.2mg, 45.4%) as a white powder.
¹H-NMR(CDCl₃):d 4.95(1H, brs), 6.74(1H, d, J=8.7Hz), 7.09(1H, dd, =8.7, 2.4Hz), 7.10(1H, d, J=16.2Hz), 7.28-7.39(4H, m), 7.49-7.54(3H, m).

### Example 302: Preparation of the compound of Compound No. 302.

### (1) (S)-2-Amino-3-phenyl-N-[3,5-bis(trifluoromethyl)phenyl]propionamide.

A mixture of 3,5-bis(trifluoromethyl)aniline(0.20g, 0.87mmol), N-(tert-butoxycarbonyl)-L-phenylalanine(254.8mg, 0.96mmol), phosphorus trichloride(40 µL, 0.46mmol) and toluene(4mL) was stirred at 80°C for 1.5 hours under argon atmosphere. After the reaction mixture was cooled to room temperature, it was poured into aqueous sodium hydrogen carbonate and extracted with ethyl acetate. After the ethyl acetate layer was washed with brine, dried over anhydrous sodium sulfate, the residue obtained by evaporation of the solvent under reduced pressure was crystallized by isopropyl ether/n-hexane to give the title compound(333.7mg, 92.9%) as an yellow white powder.
¹H-NMR(DMSO-d₆): δ 3.13(1H, dd, J=13.8, 8.1Hz), 3.29(1H, dd, J=13.8, 6.0Hz), 4.37(1H, s), 7.25-7.38(5H, m), 7.86(1H, s), 8.30(2H, s), 8.48(3H, s), 11.95(1H, s).

When the method described in Example 302(1) is referred in the following examples, phosphorus trichloride was used as the acid halogenating agent. As the reaction solvent, solvents such as toluene, monochlorobenzene or the like were used.

### (2) (S)-2-Acetoxy-5-chloro-N-(2-phenyl-1-{[3,5-bis(trifluoromethyl)phenyl]carbamoyl}ethyl)benzamide.

WSC · HCl(184mg, 0.96mmol) was added to a solution of 2-acetoxy-5-chlorobenzoic acid(104mg, 0.48mmol), (S)-2-amino-3-phenyl-N-[3,5-bis(trifluoromethyl)phenyl]propionamide(0.20g, 0.48mmol) and 1-hydroxybenzotriazole(71.4mg, 0.53mmol) in N,N-dimethylformamide(4mL), and the mixture was stirred at room temperature for 3 hours. The reaction mixture was poured into diluted hydrochloric acid and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=3:1→2:1) to give the title compound(141.4mg, 51.4%) as a white crystal.
¹H-NMR(DMSO-d₆): δ2.05(3H, s), 3.04(1H, dd, J=13.8, 9.9Hz), 3.19(1H, dd.J=13.8, 4.8Hz), 4.73-4.81(1H, m), 7.22-7.35(6H, m), 7.54(1H, d, J=2.4Hz), 7.60(1H, dd, J=8.7, 2.4Hz), 7.81(1H, s), 8.27(2H, s), 8.91(1H, d, J=7.8Hz), 10.81(1H, s).

When the method described in Example 302(2) is referred in the following examples, WSC · HCl and 1-hydroxybenzotriazole hydrate were used as the dehydrocondensating agent. As the reaction solvent, solvents such as N,N-dimethylformamide or the like were used.

### (3) (S)-5-Chloro-2-hydroxy-N-(2-phenyl-1-{[3,5-bis(trifluoromethyl)phenyl]carbamoyl}ethyl)benzamide(Compound No. 302).

5N Aqueous sodium hydroxide(0.2mL) was added to a solution of (S)-2-acetoxy-5-chloro-N-(2-phenyl-1-{[3,5-bis(trifluoromethyl)phenyl]carbamoyl}ethyl)benzamide(141.4mg, 0.25mmol) in a mixed solvent of methanol/tetrahydrofuran(2mL+2mL), and the mixture was stirred at room temperature for 20 minutes. The reaction mixture was poured into diluted hydrochloric acid and extracted with ethyl acetate. After the ethyl acetate layer was washed successively with water and brine, dried over anhydrous sodium sulfate, the residue obtained by evaporation of the solvent under reduced pressure was crystallized by ethyl acetate/isopropyl ether/n-hexane to give the title compound(74.4mg, 56.8%) as a white powder.
¹H-NMR(DMSO-d₆): δ 3.13(1H, dd, J=13.8, 9.0Hz), 3.26(1H, dd, J=14.1, 4.8Hz), 4.85-4.92(1H, m), 6.95(1H, d, J=8.7Hz), 7.19-7.23(1H, m), 7.26-7.31(4H, m), 7.45(1H, dd, J=8.7, 2.4Hz), 7.81(1H, s), 7.97(1H, d, J=2.4Hz), 8.26(2H, s), 9.12(1H, d, J=7.2Hz), 10.89(1H, s), 12.01(1H, s).

When the method described in Example 302(3) is referred in the following examples, inorganic bases such as sodium hydroxide, potassium carbonate or the like were used as the base. As the reaction solvent, solvents such as water, methanol, ethanol, tetrahydrofuran or the like were used alone or as a mixture.

### Example 303: Preparation of the compound of Compound No. 303.

### (1) [1-({[3,5-Bis(trifluoromethyl)phenyl]amino}carbonyl)methyl]carbamic acid 1,1-dimethyl ester.

Under argon atmosphere, N-(tert-butoxycarbonyl)glycine(183.5mg, 1.05mmol) and triethylamine(0.25mL, 1.79mmol) were added to a solution of 3,5-bis(trifluoromethyl)aniline(0.20g, 0.87mmol) in tetrahydrofuran(4mL), and after cooling with ice bath, phosphorus oxychloride(96 µL, 1.05mmol) was added and the mixture was stirred at room temperature for 5 hours. The reaction mixture was poured into water and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=2:1→3:2) to give the title compound(101.9mg, 30.3%) as a white crystal.
¹H-NMR(CDCl₃): δ 1.49(9H, s), 3.99(2H, d, J=6.0Hz), 5.37(1H, t, J=6.0Hz), 7.57(1H, s), 8.00(2H, s), 9.06(1H, brs).

### (2) 2-Amino-N-[3,5-bis(trifluoromethyl)phenyl]acetamide hydrochloride.

4N Hydrochloric acid/ethyl acetate solution(1mL) was added to [1-({[3,5-bis(trifluoromethyl)phenyl]amino}carbonyl)methyl]carbamic acid 1,1-dimethyl ester(101.9mg, 0.26mmol), and the mixture was stirred at room temperature for 1 hour. n-Hexane(15mL) was added to the reaction mixture and the separated white solid was filtered to give the title compound(80.8mg, 96.4%) as a white powder.
¹H-NMR(CD₃OD): δ 3.89(2H, s), 7.71(1H, s), 8.22(2H, s).

### (3) 2-Acetoxy-5-chloro-N-({[3,5-bis(trifluoromethyl)phenyl]carbamoyl}methyl)benzamide.

WSC · HCl(95.9mg, 0.5mmol) was added to a solution of 2-acetoxy-5-chlorobenzoic acid(59.1mg, 0.28mmol), 2-amino-N-[3,5-bis(trifluoromethyl)phenyl]acetamide hydrochloride (80.8mg, 0.25mmol) and 1-hydroxybenzotriazole(37.2mg, 0.28mmol) in N,N-dimethylformamide(3mL), and the mixture was stirred at room temperature for 3 hours. The reaction mixture was poured into diluted hydrochloric acid and extracted with ethyl acetate. After the ethyl acetate layer was washed with brine, dried over anhydrous sodium sulfate, the residue obtained by evaporation of the solvent under reduced pressure was purified by column chromatography on silica gel(n-hexane:ethyl acetate=3:2→1:1) to give the title compound(83.7mg, 69.3%) as a white crystal.
¹H-NMR(CDCl₃): δ 2.40(3H, s), 4.40(2H, d, J=5.4Hz), 7.17(1H, d.J=8.4Hz), 7.40(1H, t, J=5.4Hz), 7.53(1H, dd, J=8.4, 2.4Hz), 7.62(1H, s), 7.82(1H, d, J=2.4Hz), 8.19(2H, s), 9.20(1H, s).

### (4) 5-Chloro-2-hydroxy-N-({[3,5-bis(trifluoromethyl)phenyl]carbamoyl}methyl)benzamide

### (Compound No. 303).

5N Aqueous sodium hydroxide(0.1mL) was added to a solution of 2-acetoxy-5-chloro-N-({[3,5-bis(trifluoromethyl)phenyl]carbamoyl}methyl)benzamide (83.7mg, 0.17mmol) in methanol/tetrahydrofuran(2mL+1mL), and the mixture was stirred at room temperature for 20 minutes. The reaction mixture was poured into diluted hydrochloric acid and extracted with ethyl acetate. After the ethyl acetate layer was washed with water and brine, dried over anhydrous sodium sulfate, the residue obtained by evaporation of the solvent under reduced pressure was purified by column chromatography on silica gel(n-hexane:ethyl acetate=2:1) and washed with n-hexane under suspension to give the title compound(47.7mg, 63.7%) as a white crystal.
¹H-NMR(DMSO-d₆): δ 4.18(2H, d, J=5.4Hz), 7.00(1H, d, J=9.0Hz), 7.47(1H, dd, J=9.0, 2.7Hz), 7.80(1H, s), 7.96(1H, d, J=2.7Hz), 8.27(2H, s), 9.25(1H, t, J=5.4Hz), 10.78(1H, s), 12.14(1H, s).

### Example 304: Preparation of the compound of Compound No. 304.

### (1) 5-Chlorosalicylhydrazide.

A mixture of 5-chloro-2-hydroxybenzoic acid methyl ester(0.50g, 2.7mmol), hydrazine monohydrate(0.3mL, 6.2mmol) and ethanol(5mL) was refluxed for 6 hours. After the reaction mixture was cooled to room temperature, n-hexane was added and the separated crystal was filtered to give the title compound(395.9mg, 79.2%) as a white crystal.
¹H-NMR(DMSO-d₆): δ 6.90(1H, d, J=8.7Hz), 7.38(1H, dd, J=8.7, 2.7Hz), 7.85(1H, d, J=8.7Hz), 10.23(brs).

### (2) 5-Chlorosalicylic acid [3,5-bis(trifluoromethyl)benzylidene]hydrazide(Compound No. 304).

A mixture of 5-chlorosalicylhydrazide(213.9mg, 1.2mmol), 3,5-bis(trifluoromethyl)benzaldehyde(190 µL, 1.2mmol), concentrated sulfric acid(3 drops) and ethanol(5mL) was refluxed for 30 minutes. 3,5-Bis(trifluoromethyl)benzaldehyde(100 µL, 0.61mmol) was added and the mixture was refluxed for further 1 hour. After the reaction mixture was cooled to room temperature, it was poured into water and extracted with ethyl acetate. After the ethyl acetate layer was washed with brine, dried over sodium sulfate, the residue obtained by evaporation of the solvent under reduced pressure was purified by column chromatography on silica gel(n-hexane:ethyl acetate=3:1→2:1) and washed with n-hexane under suspension to give the title compound(362.6mg, 76.8%) as a white powder.
¹H-NMR(DMSO-d₆): δ 7.03(1H, d, J=9.0Hz), 7.49(1H, dd, J=9.0, 2.7Hz), 7.86(1H, d, J=3.0Hz), 8.20(1H, s), 8.40(2H, s), 8.59(1H, s), 11.65(1H, s), 12.14(1H, s).

### Example 305: Preparation of the compound of Compound No. 305.

### (1) (S)-2-Amino-4-methyl-N-[3,5-bis(trifluoromethyl)phenyl]pentanamide.

Using N-(tert-butoxycarbonyl)-L-leucine and 3,5-bis(trifluoromethyl)aniline as the raw materials, the same operation as the Example 302(1) gave the title compound.
Yield: 25.2%.
¹H-NMR(CDCl₃): δ 0.98(3H, d, J=6.3Hz), 1.01(3H, d, J=6.3Hz), 1.39-1.48(1H, m), 1.74-1.89(2H, m), 3.55(1H, dd, J=9.9, 3.6Hz), 7.58(1H, s), 8.12(2H, s), 10.01(1H, s).

### (2) (S)-5-Chloro-2-hydroxy-N-(3-methyl-1-{[3,5-bis(trifluoromethyl)phenyl]carbamoyl}butyl)benzamide(Compound No. 305).

Using 2-acetoxy-5-chlorobenzoic acid and (S)-2-amino-4-methyl-N-[3,5-bis-(trifluoromethyl)phenyl]pentanamide as the raw materials, the same operation as the Example 302(2)-(3) gave the title compound.
Yield: 24.8%(2 steps).
¹H-NMR(DMSO-d₆): δ 0.95(3H, d, J=5.7Hz), 0.97(3H, d, J=6.0Hz), 1.65-1.84(3H, m), 4.65-4.72(1H, m), 6.98(1H, d, J=9.0Hz), 7.47(1H, dd, J=8.7, 2.4Hz), 7.79(1H, s), 8.06(1H, d, J=2.7Hz), 8.32(2H, s), 9.03(1H, d, J=8.1Hz), 10.85(1H, s), 12.20(1H, s).

### Example 306: Preparation of the compound of Compound No. 306.

Using 5-chlorosalicylaldehyde and 3,5-bis(trifluoromethyl)benzhydrazide as the raw materials, the same operation as the Example 304(2) gave the title compound.
Yield: 24.7%.
¹H-NMR(DMSO-d₆): δ 6.97(1H, d, J=8.7Hz), 7.34(1H, dd, J=9.0, 2.7Hz), 7.73(1H, d, J=2.4Hz), 8.41(1H, s), 8.59(2H, s), 8.67(1H, s), 11.07(1H, s), 12.45(1H, s).

### Example 307: Preparation of the compound of Compound No. 307.

Using 5-chlorosalicylic acid and 3,5-bis(trifluoromethyl)phenethylamine as the raw materials, the same operation as the Example 16 gave the title compound.
Yield: 30.2%.
¹H-NMR(CDCl₃): δ3.10(2H, t, J=6.9Hz), 3.71-3.77(2H, m), 6.34(1H, brs), 6.95(1H, d, J=8.7Hz), 7.23(1H, d, J=2.7Hz), 7.36(1H, dd, J=8.7, 2.4Hz), 7.70(2H, s), 7.80(1H, s), 12.06(1H, s).

### Example 308: Preparation of the compound of Compound No. 308.

A mixture of 3-hydroxyphthalic anhydride(100mg, 0.6mmol), 3,5-bis(trifluoromethyl)aniline(168mg, 0.7mmol) and acetic acid(5mL) was refluxed for 6 hours under argon atmosphere. After the reaction mixture was cooled to room temperature, acetic acid was evaporated under reduced pressure and the obtained residue was dissolved in ethyl acetate(15mL). After the ethyl acetate solution was washed successively with water and brine, dried over anhydrous sodium sulfate, the residue obtained by evaporation of the solvent under reduced pressure was purified by column chromatography on silica gel(n-hexane:ethyl acetate=3:1) to give the title compound(100mg, 43.7%) as a white powder.
¹H-NMR(DMSO-d₆): δ7.31(1H, d, J=8.1Hz),7.42(1H, d, J=7.5Hz), 7.72(1H, dd, J=8.1, 7.5Hz), 8.21(1H, s), 8.24(2H, s), 11.28(1H, s).

### Example 309: Preparation of the compound of Compound No. 309.

3,5-Bis(trifluoromethyl)phenylisocyanate(180 µL, 1.04mmol) was added to a solution of 2-amino-4-chlorophenol(143.6mg, 1mmol) in a mixed solvent of tetrahydrofuran/toluene(0.5mL+4.5mL), and the mixture was stirred at 100°C for 1 hour. After the reaction mixture was cooled to room temperature, the residue obtained by evaporation of the solvent under reduced pressure was purified by column chromatography on silica gel(n-hexane:ethyl acetate=1:1) and crystallized by isopropyl ether/n-hexane to give the title compound(288.5mg, 72.4%) as a light yellowish brown powder.
¹H-NMR(DMSO-d₆): δ 6.84-6.91(2H, m), 7.67(1H, s), 8.06(2H, s), 8.14(1H, d, J=2.1Hz), 8.45(1H, s), 10.10(1H, s), 10.44(1H, s).

### Example 310: Preparation of the compound of Compound No. 310.

### (1) 5-Chloro-2-methoxy-β-[3,5-bis(trifluoromethyl)phenyl]styrene.

A solution of sodium nitrite(57mg, 0.8mmol) in water(1mL) was added to a solution of 2-amino-4-chloroanisole(131mg, 0.8mmol) in 48% hydrogen tetrafluoroborate(0.3mL) under ice cooling and argon atmosphere. After the mixture was stirred at 0°C for 1 hour, a solution of 3,5-bis(trifluoromethyl)styrene(100mg, 0.4mmol) in methanol(3mL) was added and the mixture was stirred at 50°C for 1 hour. After the reaction mixture was cooled to room temperature, the residue obtained by evaporation of the solvent under reduced pressure was diluted with ethyl acetate. After the solution was washed successively with 2N hydrochloric acid, water and brine, dried over anhydrous sodium sulfate, the residue obtained by evaporation of the solvent under reduced pressure was purified by column chromatography on silica gel(n-hexane:ethyl acetate=5:1) to give the title compound(52.8mg, 33.3%) as a white powder.
¹H-NMR(CDCl₃): δ 3.85(3H, s), 6.80(1H, d, J=8.8Hz), 7.08(1H, d, J=16.8Hz), 7.17(1H, dd, J=8.8, 2.5Hz), 7.20-7.42(4H, m), 7.51-7.55(3H, m).

### (2) 4-Chloro-2-[3,5-bis(trifluoromethyl)styryl]phenol(Compound No. 310).

Using 5-chloro-2-methoxy-β-[3,5-bis(trifluoromethyl)phenyl]styrene as the raw material, the same operation as the Example 301(2) gave the title compound.
Yield: 18.1%.
¹H-NMR(CDCl₃): δ 5.16(1H, brs), 6.76(1H, d,J=8.4Hz), 7.15(1H, dd, J=8.4, 2.7Hz), 7.19(1H, d, J=16.5Hz), 7.45(1H, d, J=15.5Hz), 7.53(1H, d, J=2.4Hz), 7.76(1H, s), 7.93(2H, s).

### Example 311: Preparation of the compound of Compound No. 311.

Using 5-chlorosalicylic acid and 2-aminoindane as the raw materials, the same operation as the Example 16 gave the title compound.
Yield: 45.3%.
¹H-NMR(DMSO-d₆): δ 2.98(2H, dd, J=16.2, 5.7Hz), 3.29(2H, dd, J=16.2, 7.5Hz), 4.69-4.79(1H, m), 6.93(1H, d, J=8.7Hz), 7.16-7.20(2H, m), 7.23-7.28(2H, m), 7.43(1H, dd, J=8.7, 2.4Hz), 8.02(1H, d, J=2.4Hz), 9.03(1H, d, J=6.9Hz), 12.66(1H, s).

### Example 312: Preparation of the compound of Compound No. 312.

### (1) 4-Chloro-2-({[3,5-bis(trifluoromethyl)phenyl]imino}methyl)phenol.

Using 5-chlorosalicylaldehyde and 3,5-bis(trifluoromethyl)aniline as the raw materials, the same operation as the Example 14(1) gave the title compound.
Yield: 76.6%.
¹H-NMR(DMSO-d₆): δ 7.04(1H, d, J=9.0Hz), 7.50(1H, dd, J=9.0, 2.7Hz), 7.80(1H, d, J=2.7Hz), 8.01(1H, s), 8.12(2H, s), 9.03(1H, s), 12.09(1H, brs).

### (2) N-[(5-Chloro-2-hydroxyphenyl)methyl]-3,5-bis(trifluoromethyl)aniline(Compound No. 312).

Using 4-chloro-2-({[3,5-bis(trifluoromethyl)phenyl]imino}methyl)phenol as the raw material, the same operation as the Example 14(2) gave the title compound.
Yield: 78.1%.
¹H-NMR(CDCl₃): δ4.40(3H, s), 6.27(1H, s), 6.80(1H, d, J=8.4Hz), 7.11(2H, s), 7.17-7.20(2H, m), 7.30(1H, s).

### Example 313: Preparation of the compound of Compound No. 313.

WSC · HCl(138mg, 0.7mmol) was added to a solution of N-[(5-chloro-2-hydroxyphenyl)methyl]-3,5-bis(trifluoromethyl)aniline(Compound No. 312; 88.8mg, 0.24mmol) and acetic acid(43mg, 0.7mmol) in dichloromethane(2mL) under argon atmosphere, and the mixture was stirred at room temperature for 12 hours. After the reaction mixture was diluted with ethyl acetate, washed successively with water and brine, dried over anhydrous sodium sulfate, the residue obtained by evaporation of the solvent under reduced pressure was purified by column chromatography on silica gel(n-hexane:ethyl acetate=3:1) to give the title compound(69mg, 70.4%) as a white powder.
¹H-NMR(CDCl₃): δ 1.92(3H, s), 4.73(2H, s), 6.54(1H, d, J=2.4Hz), 6.95(1H, d, J=8.4Hz), 7.22(1H, dd, J=8.7, 2.4Hz), 7.53(2H, s), 7.99(1H, s), 9.21(1H, s).

### Example 314: Preparation of the compound of Compound No. 314.

3,5-Bis(trifluoromethyl)benzoyl chloride(100 µL, 0.55mmol) was added to a solution of 5-chlorosalicylhydrazide(compound of Example 304(1); 0.1g, 0.53mmol) in pyridine(3mL) and the mixture was stirred at room temperature for 6 hours. The reaction mixture was poured into 2N hydrochloric acid and extracted with ethyl acetate. After the ethyl acetate layer was washed with brine and dried over anhydrous sodium sulfate, the residue obtained by evaporation of the solvent under reduced pressure was washed with ethyl acetate/isopropyl ether/n-hexane under suspension to give the title compound(169mg, 74.7%) as a white powder.
¹H-NMR(DMSO-d₆): δ7.04(1H, d, J=9.0Hz), 7.51(1H, dd, J=8.7, 2.4Hz), 7.92(1H, d, J=2.4Hz), 8.43(1H, s), 8.57(2H, s), 10.79(1H, s), 11.37(1H, s), 11.81(1H, s).

### Example 315: Preparation of the compound of Compound No. 315.

A mixture of 5-chlorosalicylhydrazide(compound of Example 304(1); 0.10g, 0.53mmol), 3,5-bis(trifluoromethyl)benzyl bromide(120 µL, 0.65mmol), triethylamine(0.2mL, 1.43mmol) and toluene(4mL) was stirred at 100°C for 2 hours. After the reaction mixture was cooled to room temperature, it was poured into diluted hydrochloric acid and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=3:1) and crystallized by n-hexane to give the title compound(45.6mg, 20.9%) as a white powder.
¹H-NMR(CDCl₃): δ 4.22(2H, d, J=4.8Hz), 5.13(1H, q, J=4.8Hz), 6.96(1H, d, J=8.7Hz), 7.23(1H, d, J=2.4Hz), 7.37(1H, dd, J=9.0, 2.4Hz), 7.69(1H, d, J=4.8Hz), 7.85(1H, s), 7.88(2H, s), 11.54(1H, s).

### Example 316: Preparation of the compound of Compound No. 316.

A mixture of 5-chlorosalicylic acid(172.6mg, 1mmol), 3,5-bis(trifluoromethyl)phenol(152 µL, 1mmol), phosphorus oxychloride(40 µL, 0.43mmol) and xylene(3mL) was stirred at 140°C for 2 hours. After the reaction mixture was cooled to room temperature, it was poured into water and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=10:1→5:1) to give the title compound(53.6mg, 13.9%) as a white crystal.
¹H-NMR(CDCl₃): δ7.04(1H, d, J=9.0Hz), 7.54(1H, dd, J=9.0, 2.7Hz), 7.75(2H, s), 7.86(1H, s), 8.02(1H, d, J=2.7Hz), 10.09(1H, s).

### Example 317: Preparation of the compound of Compound No. 317.

WSC · HCl(30.9mg, 0.2mmol) was added to a solution of 5-chlorosalicylic acid(35mg, 0.2mmol) and 3,5-bis(trifluoromethyl)phenylhydrazine(50mg, 0.2mmol) in dichloromethane(2mL) under argon atmosphere, and the mixture was stirred at room temperature for 1 hour. After the reaction mixture was diluted with ethyl acetate, washed successively with water and brine, dried over anhydrous sodium sulfate, the residue obtained by evaporation of the solvent under reduced pressure was purified by column chromatography on silica gel(n-hexane:ethyl acetate=3:1) to give the title compound(56.3mg, 69.6%) as a white powder.
¹H-NMR(CDCl₃): δ6.61(1H, d, J=2.7Hz), 6.99(1H, d, J=8.7Hz), 7.28(2H, s), 7.41-7.45(2H, m), 7.62(1H, d, J=2.4Hz), 8.53(1H, brs), 11.11(1H, s).

### Example 318: Preparation of the compound of Compound No. 318.

### (1) 2-Bromo-1-(5-chloro-2-hydroxyphenyl)ethanone.

Phenyltrimethylammonium tribromide(0.44g, 1.17mmol) was added to a solution of 5'-chloro-2'-hydroxyacetophenone(0.20g, 1.17mmol) in tetrahydrofuran(6mL) and the mixture was stirred at room temperature for 8 hours. The reaction mixture was poured into water and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=5:1) to give the title compound(220.7mg, 75.6%) as an yellow oil.
¹H-NMR(CDCl₃): δ 4.41(2H, s), 7.00(1H, d, J=9.3Hz), 7.47(1H, dd, J=8.7, 2.4Hz), 7.71(1H, d, J=2.7Hz), 11.63(1H, s).

### (2) 2-(2-Aminothiazol-4-yl)-4-chlorophenol.

A mixture of 2-bromo-1-(5-chloro-2-hydroxyphenyl)ethanone(156.9mg, 0.63mmol), thiourea(47.9mg, 0.63mmol) and ethanol(3mL) was refluxed for 2 hours. After the reaction mixture was cooled to room temperature, it was poured into saturated sodium hydrogen carbonate solution and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=2:1) to give the title compound(98.6mg, 64.5%) as a light yellowish white powder.
¹H-NMR(DMSO-d₆): δ 6.85(1H, d, J=8.7Hz), 7.14(1H, dd, J=8.7, 3.0Hz), 7.25(1H, s), 7.48(2H, s), 7.79(1H, d, J=3.0Hz), 11.95(1H, s).

### (3) N-[4-(5-Chloro-2-hydroxymethyl)thiazol-2-yl]-[3,5-bis(trifluoromethyl)phenyl]benzamide(Compound No. 318).

Phosphorus trichloride(36 µL, 0.41mmol) was added to a mixture of 2-(2-aminothiazol-4-yl)-4-chlorophenol(98.6mg, 0.41mmol), 3,5-bis(trifluoromethyl)benzoid acid(104.9mg, 0.41mmol), chlorobenzene(3mL) and N-methyl-2-pyrrolidinone(3mL), and the mixture was refluxed for 3 hours. After the reaction mixture was cooled to room temperature, it was poured into water and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=4:1→2:1) and washed with isopropyl ether/n-hexane under suspension to give the title compound(19.6mg, 10.3%) as a white powder.
¹H-NMR(DMSO-d₆): δ6.98(1H, d, J=8.4Hz), 7.21(1H, dd, J=8.7, 2.7Hz), 7.95(1H, s), 8.08(1H, d, J=2.7Hz), 8.45(1H, s), 8.77(2H, s), 10.90(1H, s), 13.15(1H, s).

### Example 319: Preparation of the compound of Compound No. 319.

### (1) 3-[3,5-Bis(trifluoromethyl)benzyl]thiazolidine-2,4-dione.

5N Aqueous sodium hydroxide(0.5mL) was added to a mixture of 2,4-thiazolidinedione(198.7mg, 1.69mmol), 3,5-bis(trifluoromethyl)benzyl bromide(0.50g, 1.63mmol) and ethanol(5mL), and the mixture was refluxed for 4 hours. After the reaction mixture was cooled to room temperature, it was poured into water and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=3:1→2:1) to give the title compound(405.6mg, 72.5%) as a white crystal.
¹H-NMR(CDCl₃): δ4.01(2H, s), 4.87(2H, s), 7.84(1H, s), 7.86(2H, s).

### (2) 5-(5-Chloro-2-hydroxybenzylidene)-3-[3,5-bis(trifluoromethyl)benzyl]thiazolidine-2,4-dione(Compound No. 319).

A mixture of 3-[3,5-bis(trifluoromethyl)benzyl]thiazolidine-2,4-dione(0.20g, 0.58mmol), piperidine(3 drops), acetic acid(3 drops) and toluene(5mL) was stirred at room temperature for 10 minutes, then 5-chlorosalicylaldehyde(92.3mg, 0.59mmol) was added and the mixture was refluxed for 1 hour. After the reaction mixture was cooled to room temperature, it was poured into water and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=2:1→3:2) to give the title compound(173.2mg, 62.0%) as a light yellow powder.
¹H-NMR(DMSO-d₆): δ5.03(2H, s), 7.00(1H, d, J=9.0Hz), 7.33(1H, d, J=2.4Hz), 7.38(1H, dd, J=8.7, 2.7Hz), 8.03(1H, s), 8.05(2H, s), 8.07(1H, s), 10.95(1H, s).

### Example 320: Preparation of the compound of Compound No. 320.

A mixture of 3-hydroxyphthalic anhydride(33.5mg, 0.2mmol), 3,5-bis(trifluoromethyl)benzyl amine(62mg, 0.2mmol) and chlorobenzene(5mL) was refluxed for 3 hours under argon atmosphere. After the reaction mixture was cooled to room temperature, the solvent was evaporated under reduced pressure and the obtained residue was crystallized from n-hexane/ethyl acetate to give the title compound(68.5mg, 85.2%) as a white crystal.
¹H-NMR(CDCl₃): δ4.90(2H, s), 7.19(1H, dd, J=8.4, 0.6Hz), 7.41(1H, dd, J=7.2, 0.6Hz), 7.61(1H, dd, J=8.4, 7.2Hz), 7.75(1H, brs), 7.82(1H, brs), 7.86(2H, s).

### Example 321: Preparation of the compound of Compound No. 321.

A mixture of 5-chlorosalicylaldehyde(150mg, 1mmol), 3,5-bis(trifluoromethyl)phenylhydrazine(200mg, 0.9mmol) and methanol(5mL) was refluxed for 1 hour under argon atmosphere. After the reaction mixture was cooled to room temperature, methanol was evaporated under reduced pressure and the obtained residue was crystallized from n-hexane/ethyl acetate to give the title compound(224mg, 66.6%) as a white powder.
¹H-NMR(CDCl₃): δ 6.97(1H, d, J=8.7Hz), 7.17(1H,d,J=2.4Hz), 7.24(1H, dd, J=9.0, 2.7Hz), 7.35(2H, s), 7.41(1H, s), 7.82(1H, s), 7.87(1H, s), 10.29(1H, s).

### Example 322: Preparation of the compound of Compound No. 322.

Using 6-hydroxysalicylic acid and 3,5-bis(trifluoromethyl)aniline as the raw materials, the same operation as the Example 16 gave the title compound.
Yield: 86.9%.
¹H-NMR(DMSO-d₆): δ6.36(2H,d,J=8.4Hz), 7.13(1H,t,J=8.4Hz),7.79(1H, s),8.38(2H, s),11.40(2H,brs),11.96(1H, brs).

### Example 323: Preparation of the compound of Compound No. 323.

Using 4-methylsalicylic acid and 3,5-bis(trifluoromethyl)aniline as the raw materials, the same operation as the Example 16 gave the title compound.
Yield: 42.9%.
¹H-NMR(DMSO-d₆): δ2.32(3H, s)6.82(1H, d, J=6.6Hz)6.84(1H, s)7.83(1H, s)7.84(1H, d, J=8.5Hz)8.47(2H, s)10.76(1H, s)11.44(1H, s).

### Example 324: Preparation of the compound of Compound No. 324.

Using 5-bromo-4-hydroxysalicylic acid and 3,5-bis(trifluoromethyl)aniline as the raw material, the same operation as the Example 16 gave the title compound.
Yield: 82.4%.
¹H-NMR(CDCl₃): δ5.89(1H, s)6.70(1H, s)7.69(2H, s)7.95(1H, s)8.12(2H, s)11.62(1H, s).

### Example 325 Preparation of the compound of Compound No. 325.

Using 4-hydroxysalicylic acid and 3,5-bis(trifluoromethyl)aniline as the raw materials, the same operation as the Example 16 gave the title compound.
Yield: 29.9%.
¹H-NMR(DMSO-d₆): δ 6.37(1H, d, J=2.5Hz), 6.42(1H, dd, J=8.8, 2.5Hz), 7.81(1H, s), 7.86(1H, d, J=8.5Hz), 8.44(2H, s), 10.31(1H, s), 10.60(1H, s), 11.77(1H, s).

### Example 326: Preparation of the compound of Compound No. 326.

Using 3,5-dichlorosalicylic acid and 3,5-bis(trifluoromethyl)aniline as the raw materials, the same operation as the Example 16 gave the title compound.
Yield: 44.8%.
¹H-NMR(DMSO-d₆): δ 7.85(1H, d, J=2.5Hz), 7.91(1H, s), 8.01(1H, d, J=2.5Hz), 8.42(2H, s), 11.10(1H, s).

### Example 327: Preparation of the compound of Compound No. 327.

Using 3-hydroxysalicylic acid and 3,5-bis(trifluoromethyl)aniline as the raw materials, the same operation as the Example 16 gave the title compound.
Yield: 22.7%.
¹H-NMR(DMSO-d₆): δ 6.81(1H, t, J=8.0Hz), 7.01(1H, dd, J=8.0, 1.5Hz), 7.35(1H, dd, J=8.0, 1.5Hz), 7.84(1H, s), 8.46(2H, s), 9.56(1H, s), 10.79(1H, s), 10.90(1H, brs).

### Example 328: Preparation of the compound of Compound No. 328.

Using 3-methylsalicylic acid and 3,5-bis(trifluoromethyl)aniline as the raw materials, the same operation as the Example 16 gave the title compound.
Yield: 54.9%.
¹H-NMR(DMSO-d₆): δ 2.22(3H, s), 6.94(1H, t, J=7.4Hz), 7.42(1H, d, J=7.4Hz), 7.84-7.85(2H, m), 8.47(2H, s), 10.87(1H, s), 11.87(1H, s).

### Example 329: Preparation of the compound of Compound No. 329.

Using 3-methoxysalicylic acid and 3,5-bis(trifluoromethyl)aniline as the raw materials, the same operation as the Example 16 gave the title compound.
Yield: 34.6%.
¹H-NMR(DMSO-d₆): δ3.85(3H, s), 6.94(1H, t, J=8.0Hz), 7.20(1H, dd, J=8.0, 1.4Hz), 7.44(1H, dd, J=8.0, 1.4Hz), 7.84(1H, s), 8.45(2H, s), 10.82(1H, s), 10.94(1H, brs).

### Example 330: Preparation of the compound of Compound No. 330.

Using 5-[(1,1,3,3-tetramethyl)butyl]salicylic acid and 3,5-bis(trifluoromethyl)aniline as the raw materials, the same operation as the Example 16 gave the title compound.
Yield: 64.2%.
¹H-NMR(DMSO-d₆): δ0.70(9H, s), 1.35(6H, s), 1.72(2H, s), 6.95(1H, d, J=8.4Hz), 7.50(1H, dd, J=8.0, 2.1Hz), 7.83(1H, s), 7.84(1H, d, J=2.1Hz), 8.46(1H, s), 10.77(1H, s), 11.20(1H, s).

### Example 331: Preparation of the compound of Compound No. 331.

Using 3,5,6-trichlorosalicylic acid and 3,5-bis(trifluoromethyl)aniline as the raw materials, the same operation as the Example 16 gave the title compound.
Yield: 26.2%.
¹H-NMR(DMSO-d₆): δ 7.88(1H, s), 7.93(1H, s), 8.33(2H, s), 10.88(1H, s), 11.36(1H, s).

### Example 332: Preparation of the compound of Compound No. 332.

Using 3,5-bis[(1,1-dimethyl)ethyl]salicylic acid and 3,5-bis(trifluoromethyl)aniline as the raw materials, the same operation as the Example 16 gave the title compound.
Yield: 65.0%.
¹H-NMR(DMSO-d₆): δ 1.34(9H, s), 1.40(9H, s), 7.49(1H, d, J=2.2Hz), 7.82(1H, d, J=2.2Hz), 7.91(1H, s), 8.40(2H, s), 10.82(1H, s), 12.44(1H, s).

### Example 333: Preparation of the compound of Compound No. 333.

Using 6-fluorosalicylic acid and 3,5-bis(trifluoromethyl)aniline as the raw materials, the same operation as the Example 16 gave the title compound.
Yield: 35.9%.
¹H-NMR(DMSO-d₆): δ6.73-6.82(2H, m), 7.32(1H, ddd, J=1.4, 8.5, 15.3Hz), 7.83(1H, s), 8.39(2H, s), 10.50(1H, d, J=1.4Hz), 11.11(1H, s).

### Example 334: Preparation of the compound of Compound No. 334.

Using 3-chlorosalicylic acid and 3,5-bis(trifluoromethyl)aniline as the raw materials, the same operation as the Example 16 gave the title compound.
Yield: 61.3%.
¹H-NMR(DMSO-d₆): δ 7.05(1H, dd, J=7.6, 8.0Hz), 7.69(1H, dd, J=1.4, 13.3Hz), 7.90(1H, s), 7.93(1H, dd, J=1.4, 8.0Hz), 8.44(2H, s), 11.01(1H, s), 11.92(1H, br.s).

### Example 335: Preparation of the compound of Compound No. 335.

Using 4-methoxysalicylic acid and 3,5-bis(trifluoromethyl)aniline as the raw materials, the same operation as the Example 16 gave the title compound.
Yield: 14.2%.
¹H-NMR(DMSO-d₆): δ 3.81(3H, s), 6.54(1H, d, J=2.5Hz), 6.61(1H, dd, J=2.5, 8.8Hz), 7.83(1H, s), 7.95(1H, d, J=8.8Hz), 8.45(2H, s), 10.69(1H, s), 11.89(1H, s).

### Example 336: Preparation of the compound of Compound No. 336.

Using 6-methoxysalicylic acid and 3,5-bis(trifluoromethyl)aniline as the raw materials, the same operation as the Example 16 gave the title compound.
Yield: 63.1%.
¹H-NMR(DMSO-d₆): δ 3.24(3H, s), 6.03(1H, d, J=8.0Hz), 6.05(1H, d, J=8.5Hz), 6.71(1H, dd, J=8.2, 8.5Hz), 7.25(1H, s), 7.88(2H, s), 9.67(1H, s), 10.31(1H, s)

### Example 337: Preparation of the compound of Compound No. 337. Using

5-amino-N-[3,5-bis(trifluoromethyl)phenyl]-2-hydroxybenzamide(Compound No. 88) and methanesulfonyl chloride as the raw materials, the same operation as the Example 91 gave the title compound.
Yield: 22.6%.
¹H-NMR(DMSO-d₆): δ 2.93(3H, s), 7.02(1H, d, J=8.4Hz), 7.31(1H, dd, J=8.4, 2.7Hz), 7.68(1H, d, J=2.7Hz), 7.83(1H, s), 8.46(2H, s), 9.48(1H, s), 10.85(1H, s), 11.15(1H, s).

### Example 338: Preparation of the compound of Compound No. 338.

Using 5-amino-N-[3,5-bis(trifluoromethyl)phenyl]-2-hydroxybenzamide(Compound No. 88) and benzenesulfonyl chloride as the raw materials, the same operation as the Example 91 gave the title compound.
Yield: 45.3%.
¹H-NMR(DMSO-d₆): δ 6.89(1H, d, J=8.7Hz), 7.10(1H, dd, J=8.7, 2.7Hz), 7.51-7.64(4H, m), 7.68-7.71(2H, m), 7.81(1H, s), 8.42(2H, s), 10.03(1H, s), 10.87(1H, s), 11.13(1H, brs).

### Example 339: Preparation of the compound of Compound No. 339.

Using 5-amino-N-[3,5-bis(trifluoromethyl)phenyl]-2-hydroxybenzamide(Compound No. 88) and acetyl chloride as the raw materials, the same operation as the Example 91 gave the title compound.
Yield: 44.8%.
¹H-NMR(DMSO-d₆): δ 2.02(3H, s), 6.97(1H, d, J=8.7Hz), 7.61(1H, dd, J=8.7, 2.7Hz), 7.82(1H, s), 7.99(1H, d, J=2.7Hz), 8.46(2H, s), 9.90(1H, s), 10.85(1H, s), 10.94(1H, s).

### Example 340: Preparation of the compound of Compound No. 340.

Using N-[3,5-bis(trifluoromethyl)phenyl]-2-methoxy-5-sulfamoylbenzamide(compound of Example 87(2)) as the raw material, the same operation as the Example 80(5) gave the title compound.
Yield: 59.9%.
¹H-NMR(DMSO-d₆): δ 7.17(1H, d, J=8.7Hz), 7.31(2H, s), 7.85(1H, s), 7.86(1H, dd, J=8.4, 2.4Hz), 8.26(1H, d, J=2.7Hz), 8.47(2H, s), 10.95(1H, s), 11.90(1H, s).

### Example 341: Preparation of the compound of Compound No. 341.

Using 3-hydroxynaphthalene-2-carboxylic acid and 3,5-bis(trifluoromethyl)aniline as the raw materials, the same operation as the Example 16 gave the title compound.
Yield: 46.9%.
¹H-NMR(DMSO-d₆): δ7.36-7.41(2H, m), 7.50-7.55(1H, m), 7.79(1H, d, J=8.2Hz), 7.85(1H, d, J=0.6Hz), 7.96(1H, d, J=8.0Hz), 8.51(2H, s), 10.98(1H, s), 11.05(1H, s).

### Example 342: Preparation of the compound of Compound No. 342.

Using 2-hydroxynaphthalene-1-carboxylic acid and 3,5-bis(trifluoromethyl)aniline as the raw materials, the same operation as the Example 16 gave the title compound.
Yield: 30.2%.
¹H-NMR(DMSO-d₆): δ7.27(1H, d, J=8.8Hz), 7.32-7.38(1H, m), 7.45-7.50(1H, m), 7.72(1H, d, J=8.5Hz), 7.82-7.93(3H, m), 8.50(1H, s), 10.28(1H, s), 11.07(1H, brs).

### Example 343: Preparation of the compound of Compound No. 343.

### (1) 4-Bromo-3-hydroxythiophene-2-carboxylic acid.

A mixture of 4-bromothiophene-2-carboxylic acid methyl ester(500mg, 2.1mmol), sodium hydroxide(261mg, 6.3mmol) in a mixed solvent of methanol/water(2.5mL+2.5mL) was refluxed for 2 hours. After the reaction mixture was cooled to room temperature, 2N hydrochloric acid was added to adjust pH to 1, and it was diluted with ethyl acetate. After the ethyl acetate layer was washed successively with water and brine, dried over anhydrous sodium sulfate, the solvent was evaporated under reduced pressure to give the title compound(326mg, 69.4%) as a red brown powder.
¹H-NMR(CDCl₃): δ 4.05(1H, brs), 7.40(1H, s).

### (2) 4-Bromo-3-hydroxy-N-[3,5-bis(trifluoromethyl)phenyl]thiophene-2-carboxamide (Compound No. 343).

Using 4-bromo-3-hydroxythiophene-2-carboxylic acid and 3,5-bis(trifluoromethyl)aniline as the raw materials, the same operation as the Example 16 gave the title compound.
Yield: 82.4%.
¹H-NMR(CDCl₃): δ7.42(1H, s), 7.67(1H, brs), 7.78(1H, brs), 8.11(2H, s), 9.91(1H, brs).

### Example 344: Preparation of the compound of Compound No. 344.

Using 3,5-bis(trifluoromethyl)phenylisocyanate and oxindole as the raw materials, the same operation as the Example 28 gave the title compound.
Yield: 44.8%.
¹H-NMR(DMSO-d₆): δ 3.98(2H, s), 7.22(1H, td, J=7.8, 1.2Hz), 7.33-7.40(2H, m), 7.87(1H, s), 8.02(1H, d, J=7.8Hz), 8.38(2H, s), 11.00(1H, s).

### Example 345: Preparation of the compound of Compound No. 345.

Using 3,5-bis(trifluoromethyl)phenylisocyanate and 5-chlorooxindole as the raw materials, the same operation as the Example 28 gave the title compound.
Yield: 31.1%.
¹H-NMR(DMSO-d₆): δ3.99(2H, s), 7.41(1H, dd, J=8.7, 2.4Hz), 7.47(1H, d, J=2.1Hz), 7.87(1H, s), 8.01(1H, d, J=8.4Hz), 8.38(2H, s), 10.93(1H, s).

### Example 346: Preparation of the compound of Compound No. 346.

Using 5-chlorosalicylic acid and 3-bromo-5-(trifluoromethyl)aniline as the raw materials, the same operation as the Example 16 gave the title compound.
Yield: 37.1%.
¹H-NMR(DMSO-d₆): δ7.03(1H, d, J=9.3Hz), 7.48(1H, dd, J=8.7, 2.4Hz), 7.72(1H, s), 7.84(1H, d, J=2.7Hz), 8.16(1H, s), 8.28(1H, s), 10.69(1H, s), 11.42(1H, s).

### Example 347: Preparation of the compound of Compound No. 347.

Using 5-chlorosalicylic acid and 3-methoxy-5-(trifluoromethyl)aniline as the raw materials, the same operation as the Example 16 gave the title compound.
Yield: 68.0%.
¹H-NMR(DMSO-d₆): δ 3.85(3H, s), 7.02(1H, s), 7.03(1H, d, J=8.7Hz), 7.48(1H, dd, J=8.7, 2.7Hz), 7.61(1H, s), 7.77(1H, s), 7.88(1H, d, J=2.7Hz), 10.57(1H, s), 11.53(1H, s).

### Example 348: Preparation of the compound of Compound No. 348.

Using 5-chlorosalicylic acid and 2-morpholino-5-(trifluoromethyl)aniline as the raw materials, the same operation as the Example 16 gave the title compound.
Yield: 64.8%.
¹H-NMR(DMSO-d₆): δ2.90(4H, m), 3.84(4H, m), 7.15(1H, d, J=9.0Hz), 7.48(2H, s), 7.50(1H, dd, J=9.0, 2.7Hz), 8.00(1H, d, J=2.7Hz), 8.91(1H, s), 11.24(1H, s), 12.05(1H, s).

### Example 349: Preparation of the compound of Compound No. 349.

Using 5-chlorosalicylic acid and 2-bromo-5-(trifluoromethyl)aniline as the raw material, the same operation as the Example 16 gave the title compound.
Yield: 59.2%.
¹H-NMR(DMSO-d₆): δ 7.10(1H, d, J=8.7Hz), 7.48(1H, dd, J=8.4, 2.1Hz), 7.53(1H, dd, J=8.7, 3.0Hz), 7.97-7.99(2H, m), 8.81(1H, d, J=2.1Hz), 11.03(1H, s), 12.38(1H, s).

### Example 350: Preparation of the compound of Compound No. 350.

Using 5-chlorosalicylic acid and 3-amino-5-(trifluoromethyl)benzoic acid methyl ester as the raw materials, the same operation as the Example 16 gave the title compound.
Yield: 67.0%.
¹H-NMR(DMSO-d₆): δ3.91(3H, s), 7.02(1H, d, J=9.3Hz), 7.43(1H, dd, J=9.0, 2.4Hz), 7.57(1H, d, J=2.4Hz), 8.13(1H, s), 8.23(1H, s), 8.29(1H, s), 8.36(1H, s), 11.52(1H, s).

### Example 351: Preparation of the compound of Compound No. 351.

2N Aqueous sodium hydroxide(0.6mL) was added to a mixture of 5-chloro-2-hydroxy-N-[3-methoxycarbonyl-5-(trifluoromethyl)phenyl]benzamide (Compound No. 350; 105mg, 0.281mmol) and methanol(2.5mL), and the mixture was stirred at room temperature for 3 hours. Water was added to the reaction mixture and it was washed with ethyl acetate. After the water layer was acidified by addition of diluted hydrochloric acid, it was extracted with ethyl acetate. After the ethyl acetate layer was washed successively with water and brine, dried over anhydrous sodium sulfate, the residue obtained by evaporation of the solvent under reduced pressure was crystallized by isopropyl ether to give the title compound(100mg, 99.0%) as a white solid.
¹H-NMR(DMSO-d₆): δ 7.04(1H, d, J=9.0Hz), 7.49(1H, dd, J=8.7, 2.7Hz), 7.91(1H, d, J=2.7Hz), 7.93(1H, s), 8.43(1H, s), 8.59(1H, s), 10.78(1H, s), 11.48(1H, s).

### Example 352: Preparation of the compound of Compound No. 352.

Using 5-chlorosalicylic acid and 2-(2-naphthyloxy)-5-(trifluoromethyl)aniline as the raw materials, the same operation as the Example 16 gave the title compound.
Yield: 89.6%.
¹H-NMR(CDCl₃): δ6.94(1H, d, J=9.6Hz), 6.98(1H, d, J=9.2Hz), 7.25-7.41(4H, m), 7.48-7.57(3H, m), 7.81(1H, d, J=6.9Hz), 7.88(1H, d, J=6.9Hz), 7.95(1H, d, J=8.9Hz), 8.72(1H, s), 8.83(1H, d, J=2.0Hz), 11.70(1H, s).

### Example 353: Preparation of the compound of Compound No. 353.

Using 5-chlorosalicylic acid and 2-(2,4-dichlorophenoxy)-5-(trifluoromethyl)aniline as the raw materials, the same operation as the Example 16 gave the title compound.
Yield: 4.7%.
¹H-NMR(CDCl₃): δ 6.78(1H, d, J=8.9Hz), 7.02(1H, d, J=8.6Hz), 7.16(1H, d, J=8.6Hz), 7.33-7.38(3H, m), 7.42(1H, dd, J=8.6, 2.6Hz), 7.49(1H, d, J=2.6Hz)7.58(1H, d, J=2.3Hz), 8.66(1H, brs,), 8.82(1H, d, J=2.0Hz), 11.65(1H, s).

### Example 354: Preparation of the compound of Compound No. 354.

Using 5-chlorosalicylic acid and 2-[(4-trifluoromethyl)piperidino]-5-(trifluoromethyl)aniline as the raw materials, the same operation as the Example 16 gave the title compound.
Yield: 60.5%.
¹H-NMR(CDCl₃): δ1.85-2.05(2H, m), 2.15(2H, d, J=10.9Hz), 2.28(1H, m), 2.82(2H, t, J=11.0Hz), 3.16(2H, d, J=12.2Hz), 7.02(1H, d, J=8.9Hz), 7.31(1H, d, J=8.3Hz), 7.42(2H, m), 7.50(1H, d, J=2.6Hz), 8.75(1H, s), 9.60(1H, s), 11.94(1H, s)

### Example 355: Preparation of the compound of Compound No. 355.

Using 5-chlorosalicylic acid and 2-(2,2,2-trifluoroethoxy)-5-(trifluoromethyl)aniline as the raw materials, the same operation as the Example 16 gave the title compound.
Yield: 94.5%.
¹H-NMR(CDCl₃): δ4.58(2H, q, J=7.9Hz), 6.99-7.05(2H, m), 7.41-7.50(3H, m), 8.63(1H, brs), 8.79(1H, d, J=2.0Hz), 11.59(1H, s).

### Example 356: Preparation of the compound of Compound No. 356.

Using 5-chlorosalicylic acid and 2-(2-methoxyphenoxy)-5-(trifluoromethyl)aniline as the raw materials, the same operation as the Example 16 gave the title compound.
Yield: 80.6%.
¹H-NMR(DMSO-d₆): δ 3.74(3H, s), 6.70(1H, d, J=8.4Hz), 7.02(1H, d, J=8.7Hz), 7.07(1H, dd, J=1.5, 7.8Hz), 7.24-7.39(4H, m), 7.49(1H, dd, J=3.0, 8.7Hz), 8.00(1H, d, J=3.0Hz), 8.92(1H, d, J=2.1Hz), 11.36(1H, s), 12.18(1H, s).

### Example 357: Preparation of the compound of Compound No. 357.

Using 5-chlorosalicylic acid and 2-(4-chloro-3,5-dimethylphenoxy)-5-(trifluoromethyl)aniline as the raw materials, the same operation as the Example 16 gave the title compound.
Yield: 91.5%.
¹H-NMR(DMSO-d₆): δ 2.34(6H, s), 7.03(1H, d, J=8.8Hz), 7.05(1H, d, J=8.1Hz), 7.11(2H, s), 7.43-7.47(1H, m), 7.48(1H, dd, J=2.9, 8.8Hz), 7.97(1H, d, J=2.6Hz), 8.94(1H, d, J=2.2Hz), 11.25(1H, s), 12.12(1H, s).

### Example 358: Preparation of the compound of Compound No. 358.

Using 5-chlorosalicylic acid and 2-piperidino-5-(trifluoromethyl)aniline as the raw materials, the same operation as the Example 16 gave the title compound.
Yield: 73.7%.
¹H-NMR(CDCl₃): δ 1.68-1.72(2H, m), 1.80-1.88(4H, m), 2.89(4H, t, J=5.2Hz), 7.01(1H, d, J=8.7Hz), 7.31(1H, d, J=8.4Hz), 7.39-7.43(2H, m), 7.55(1H, d, J=2.4Hz), 8.73(1H, d, J=1.8Hz), 9.71(1H, s), 12.05(1H, s)

### Example 359: Preparation of the compound of Compound No. 359.

Using 5-chlorosalicylic acid and 2-(4-methylphenoxy)-5-(trifluoromethyl)aniline as the raw materials, the same operation as the Example 16 gave the title compound.
Yield: 67.3%.
¹H-NMR(DMSO-d₆): δ2.33(3H, s), 6.93(1H, d, J=8.8Hz), 7.03(1H, dd, J=0.5, 8.8Hz), 7.12(2H, d, J=8.2Hz), 7.29(2H, d, J=8.5Hz), 7.43(1H, dd, J=2.0, 8.6Hz), 7.48(1H, ddd, J=0.8, 2.7, 8.8Hz), 7.98(1H, dd, J=0.8, 2.7Hz), 8.94(1H, d, J=2.2Hz), 11.29(1H, s), 12.15(1H, s).

### Example 360: Preparation of the compound of Compound No. 360.

Using 5-chlorosalicylic acid and 2-(4-chlorophenoxy)-5-(trifluoromethyl)aniline as the raw materials, the same operation as the Example 16 gave the title compound.
Yield: 74.5%.
¹H-NMR(DMSO-d₆): δ 7.01(1H, d, J=8.8Hz), 7.06(1H, d, J=8.5Hz), 7.22(1H, d, J=8.5Hz), 7.43-7.48(2H, m), 7.50(2H, d, J=8.2Hz), 7.94(1H, dd, J=0.5, 2.7Hz), 8.92(1H, d, J=2.2Hz), 11.20(1H, s), 12.10(1H, s).

### Example 361: Preparation of the compound of Compound No. 361.

Using 5-bromo-2-hydroxy-N-[3,5-bis(methoxycarbonyl)phenyl]benzamide (Compound No. 170) as the raw material, the same operation as the Example 351 gave the title compound.
Yield: 89.0%.
¹H-NMR(DMSO-d₆): δ 6.98(1H, d, J=8.7Hz), 7.60(1H, dd, J=8.7, 2.4Hz), 7.24(1H, dd, J=8.7, 2.7Hz), 8.08(1H, d, J=2.7Hz), 8.24(1H, t, J=1.5Hz), 8.57(2H, d, J=1.2Hz), 10.67(1H, s), 11.64(1H, s).

### Example 362: Preparation of the compound of Compound No. 362.

Using 5-chlorosalicylic acid and 2-methyl-5-[(1-methyl)ethyl]aniline as the raw materials, the same operation as the Example 16 gave the title compound.
Yield: 19.1%.
¹H-NMR(CDCl₃): δ 1.26(6H, d, J=6.9Hz), 2.30(3H, s), 2.87-2.96(1H, m), 7.00(1H, d, J=8.7Hz), 7.08(1H, dd, J=7.8, 1.8Hz), 7.20(1H, d, J=7.8Hz), 7.40(1H, dd, J=8.7, 2.4Hz), 7.49(1H, d, J=2.7Hz), 7.50(1H, s), 7.71(1H, s), 11.99(1H, s).

### Example 363: Preparation of the compound of Compound No. 363.

Using 5-chlorosalicylic acid and 2,5-diethoxyaniline as the raw materials, the same operation as the Example 16 gave the title compound.
Yield: 59.2%.
¹H-NMR(DMSO-d₆): δ 1.32(3H, t, J=6.9Hz), 1.41(3H, t, J=6.9Hz), 3.97(2H, q, J=6.9Hz), 4.06(2H, q, J=6.9Hz), 6.61(1H, dd, J=9.0, 3.0Hz), 6.98(1H, d, J=8.7Hz), 7.10(1H, d, J=8.7Hz), 7.48(1H, dd, J=8.7, 2.7Hz), 7.97(1H, d, J=2.7Hz), 8.16(1H, d, J=3.0Hz), 10.96(1H, s), 11.91(1H, s).

### Example 364: Preparation of the compound of Compound No. 364.

Using 5-chlorosalicylic acid and 2,5-dimethylaniline as the raw materials, the same operation as the Example 16 gave the title compound.
Yield: 90.5%.
¹H-NMR(CDCl₃): δ2.28(3H, s), 2.35(3H, s), 6.99(1H, d, J=8.8Hz), 7.02(1H, brs), 7.15(1H, d, J=7.7Hz), 7.40(1H, dd, J=8.8, 2.5Hz), 7.45(1H, brs), 7.49(1H, d, J=2.5Hz)7.70(1H, br), 11.96(1H, brs).

### Example 365: Preparation of the compound of Compound No. 365.

Using 5-chlorosalicylic acid and 5-chloro-2-cyanoaniline as the raw materials, the same operation as the Example 16 gave the title compound.
Yield: 90.0%.
¹H-NMR(DMSO-d₆): δ7.09(1H, d, J=9.0Hz), 7.53(1H, dd, J=8.7, 3.0Hz), 7.82(1H, dd, J=8.7, 2.4Hz), 7.95(1H, d, J=3.0Hz), 8.07(1H, d, J=2.4Hz), 8.36(1H, d, J=9.0Hz), 11.11(1H, s), 12.36(1H, s).

### Example 366: Preparation of the compound of Compound No. 366.

Using 5-chlorosalicylic acid and 5-(N,N-diethylsulfamoyl)-2-methoxyaniline as the raw materials, the same operation as the Example 16 gave the title compound.
Yield: 44.8%.
¹H-NMR(CDCl₃): δ 1.17(6H, t, J=7.3Hz), 3.29(4H, q, J=7.3Hz), 4.05(3H, s), 7.00(2H, dd, J=2.3, 8.9Hz), 7.41(1H, dd, J=2.3, 8.9Hz), 7.48(1H, d, J=2.6Hz), 7.65(1H, dd, J=2.3, 8.6Hz), 8.56(1H, br.s), 8.84(1H, d, J=2.3Hz), 11.82(1H, s).

### Example 367: Preparation of the compound of Compound No. 367.

Using 5-chlorosalicylic acid and 2-chloro-5-nitroaniline as the raw materials, the same operation as the Example 16 gave the title compound.
Yield: 73.3%.
¹H-NMR(CD₃OD): δ 6.98(1H, d, J=8.6Hz), 7.43(1H, dd, J=2.6, 8.6Hz), 7.74(1H, d, J=8.9Hz), 7.99(1H, dd, J=3.0, 8.9Hz), 8.08(1H, d, J=2.6Hz), 9.51(1H, d, J=2.6Hz).

### Example 368: Preparation of the compound of Compound No. 368.

Using 5-chlorosalicylic acid and 5-(N-phenylcarbamoyl)-2-methoxyaniline as the raw material, the same operation as the Example 16 gave the title compound.
Yield: 40.3%.
¹H-NMR(DMSO-d₆): δ 3.99(3H, s), 7.09(2H, dd, J=6.6, 6.9Hz), 7.24(1H, d, J=8.6Hz), 7.35(2H, dd, 6.9, 7.3Hz), 7.49(1H, d, J=2.3, 8.9Hz), 7.77(3H, d, J=8.6Hz), 8.00(1H, s), 8.97(1H, s), 10.17(1H, s), 10.91(1H, s), 12.11(1H, s).

### Example 369: Preparation of the compound of Compound No. 369.

Using 5-chlorosalicylic acid and 2,5-dimethoxyaniline as the raw materials, the same operation as the Example 16 gave the title compound.
Yield: 73.9%.
¹H-NMR(CDCl₃): δ3.82(3H, s), 3.93(3H, s), 6.66(1H, dd, J=3.0, 8.9Hz), 6.86(1H, d, J=8.9Hz), 6.98(1H, d, J=8.9Hz), 7.39(1H, dd, J=2.6, 8.9Hz), 7.47(1H, d, J=2.6Hz), 8.08(1H, d, J=3.0Hz), 8.60(1H, br.s), 12.03(1H, s).

### Example 370: Preparation of the compound of Compound No. 370.

Using 5-chlorosalicylic acid and 5-acetylamino-2-methoxyaniline as the raw materials, the same operation as the Example 16 gave the title compound.
Yield: 16.9%.
¹H-NMR(DMSO-d₆): δ 2.01(3H, s), 3.85(3H, s), 7.03(2H, t, J=9.6Hz), 7.49(2H, dd, J=8.9, 9.2Hz), 7.96(1H, s), 8.51(1H, s), 9.87(1H, s), 10.82(1H, s), 12.03(1H, d, J=4.0Hz).

### Example 371: Preparation of the compound of Compound No. 371.

Using 5-chlorosalicylic acid and 5-methoxy-2-methylaniline as the raw materials, the same operation as the Example 16 gave the title compound.
Yield: 100%.
¹H-NMR(CDCl₃): δ 2.29(3H, s), 3.82(3H, s), 6.75(1H, dd, J=2.6, 8.2Hz), 7.00(1H, d, J=8.9Hz), 7.16(1H, d, J=8.6Hz), 7.38(1H, d, 2.3Hz), 7.41(1H, dd, J=2.3, 8.9Hz), 7.48(1H, d, J=2.3Hz), 7.70(1H, br.s), 11.92(1H, s).

### Example 372: Preparation of the compound of Compound No. 372.

Using 5-chlorosalicylic acid and 2,5-dibutoxyaniline as the raw materials, the same operation as the Example 16 gave the title compound.
Yield: 73.9%.
¹H-NMR(CDCl₃): δ 0.98(3H, t, J=7.2Hz), 1.05(3H, t, J=7.2Hz), 1.44-1.65(4H, m), 1.72-1.79(2H, m), 1.81-1.91(2H, m), 3.97(2H, t, J=6.3Hz), 4.07(2H, t, J=6.3Hz), 6.64(1H, dd, J=9.0, 3.0Hz), 6.85(1H, d, J=9.3Hz), 6.99(1H, d, J=9.0Hz), 7.39(1H, dd, J=8.7, 2.4Hz), 7.44(1H, d, J=2.7Hz), 8.08(1H, d, J=3.0Hz), 8.76(1H, s), 12.08(1H, s).

### Example 373: Preparation of the compound of Compound No. 373.

Using 5-chlorosalicylic acid and 2,5-diisopentyloxyaniline as the raw materials, the same operation as the Example 16 gave the title compound.
Yield: 59.7%.
¹H-NMR(CDCl₃): δ 0.97(6H, d, J=6.6Hz), 1.03(6H, d, 6.6Hz), 1.64-1.98(6H, m), 3.99(2H, t, J=6.6Hz), 4.09(2H, t, J=6.3Hz), 6.63(1H, dd, J=8.7, 3.0Hz), 6.85(1H, d, J=8.7Hz), 6.98(1H, d, J=8.7Hz), 7.38(1H, dd, J=9.0, 2.4Hz), 7.43(1H, d, J=2.7Hz), 8.09(1H, d, J=3.0Hz), 8.75(1H, s), 12.08(1H, s).

### Example 374: Preparation of the compound of Compound No. 374.

Using 5-chlorosalicylic acid and 5-carbamoyl-2-methoxyaniline as the raw materials, the same operation as the Example 16 gave the title compound.
Yield: 31.2%.
¹H-NMR(CD₃OD): δ4.86(3H, s), 6.93(1H, d, J=7.6Hz), 7.18(1H, d, J=8.6Hz), 7.35(1H, dd, J=3.0, 7.6Hz), 7.47(1H, dd, J=2.0, 8.6Hz), 8.00(1H, d, J=3.0Hz), 8.80(1H, d, J=2.0Hz).

### Example 375: Preparation of the compound of Compound No. 375.

Using 5-chlorosalicylic acid and 5-[(1,1-dimethyl)propyl]-2-phenoxyaniline as the raw materials, the same operation as the Example 16 gave the title compound.
Yield: 65.2%.
¹H-NMR(CDCl₃): δ0.69(3H, t, J=7.6Hz), 1.29(6H, s), 1.64(2H, q, J=7.6Hz), 6.91(1H, dd, J=1.7, 7.6Hz), 6.96(1H, d, J=8.9Hz), 7.03(2H, d, J=8.9Hz), 7.10(1H, dt, J=1.7, 7.6Hz), 7.16(1H, dt, J=1.7, 7.6Hz), 7.40-7.31(4H, m), 8.42(1H, dd, J=2.0, 7.9Hz), 8.53(1H, br.s)11.94(1H, s).

### Example 376: Preparation of the compound of Compound No. 376.

Using 5-chlorosalicylic acid and 2-hexyloxy-5-(methylsulfonyl)aniline as the raw materials, the same operation as the Example 16 gave the title compound.
Yield: 33.0%.
¹H-NMR(CDCl₃): δ 0.92(3H, t, J=6.9Hz), 1.40-1.59(6H, m), 1.90-2.01(2H, m), 3.09(3H, s), 4.22(2H, t, J=6.3Hz), 7.01(1H, d, J=8.9Hz), 7.06(1H, d, J=8.6Hz), 7.40-7.43(2H, m), 7.73(1H, dd, J=8.6, 2.3Hz), 8.74(1H, brs), 8.99(1H, d, J=2.3Hz), 11.76(1H, s).

### Example 377: Preparation of the compound of Compound No. 377.

Using 5-chlorosalicylic acid and 3'-amino-2,2,4'-trimethylpropiophenone as the raw materials, the same operation as the Example 16 gave the title compound.
Yield: 44.8%.
¹H-NMR(CDCl₃): δ 1.38(9H, s), 2.38(3H, s), 7.01(1H, d, J=8.9Hz), 7.31(1H, d, J=7.9Hz), 7.42(1H, dd, J=8.9, 2.6Hz), 7.53(1H, d, J=2.6Hz), 7.57(1H, dd, J=7.9, 2.0Hz), 7.83(1H, brs), 8.11(1H, d, J=2.0Hz), 11.82(1H, s).

### Example 378: Preparation of the compound of Compound No. 378.

Using 5-chlorosalicylic acid and 5-methoxy-2-(1-pyrrolyl)aniline as the raw materials, the same operation as the Example 16 gave the title compound.
Yield: 53.4%.
¹H-NMR(CDCl₃): δ 2.46(3H, s), 6.51-6.52(2H, m), 6.82-6.85(3H, m), 6.93(1H, d, J=8.9Hz), 7.06(1H, d, J=7.9Hz), 7.30(1H, d, J=7.9Hz), 7.32(1H, dd, J=2.3, 8.9Hz), 7.61(1H, s), 8.29(1H, s), 11.86(1H, br.s).

### Example 379: Preparation of the compound of Compound No. 379.

Using 5-chlorosalicylic acid and 5-chloro-2-tosylaniline as the raw materials, the same operation as the Example 16 gave the title compound.
Yield: 8.0%.
¹H-NMR(CDCl₃): δ 2.38(3H, s), 7.02(1H, d, J=8.9Hz), 7.25-7.31(3H, m), 7.46(1H, dd, J=2.6, 8.9Hz), 7.68(2H, d, J=8.6Hz), 7.74(1H, d, J=2.3Hz), 7.96(1H, d, J=8.6Hz), 8.56(1H, d, J=2.0Hz), 10.75(1H, s), 11.70(1H, s).

### Example 380: Preparation of the compound of Compound No. 380.

Using 5-chlorosalicylic acid and 2-chloro-5-tosylaniline as the raw materials, the same operation as the Example 16 gave the title compound.
Yield: 43.5%.
¹H-NMR(CDCl₃): δ2.38(3H, s), 7.02(1H, d, J=8.9Hz), 7.27(1H, d, J=7.9Hz), 7.29(1H, dd, J=2.0, 6.6Hz), 7.46(1H, dd, J=2.3, 8.9Hz), 7.68(2H, d, J=8.6Hz), 7.73(2H, d, J=2.3Hz), 7.97(1H, d, J=8.6Hz), 8.56(1H, d, J=2.0Hz), 10.73(1H, s), 11.71(1H, s).

### Example 381: Preparation of the compound of Compound No. 381.

Using 5-chlorosalicylic acid and 2-fluoro-5-(methylsulfonyl)aniline as the raw materials, the same operation as the Example 16 gave the title compound.
Yield: 28.8%.
¹H-NMR(CDCl₃): δ3.12(3H, s), 7.03(1H, d, J=8.9Hz), 7.38(1H, dd, J=8.6, 10.2Hz), 7.45(1H, dd, J=2.3, 8.9Hz), 7.53(1H, d, J=2.3Hz), 7.80(1H, ddd, J=2.3, 4.6, 8.6Hz), 8.25(1H, s), 8.98(1H, dd, J=2.3, 7.7Hz), 11.33(1H, br.s).

### Example 382: Preparation of the compound of Compound No. 382.

Using 5-chlorosalicylic acid and 2-methoxy-5-phenoxyaniline as the raw materials, the same operation as the Example 16 gave the title compound.
Yield: 77.0%.
¹H-NMR(CDCl₃): δ3.98(3H, s), 6.80(1H, d, J=8.8Hz), 6.90(1H, d, J=8.8Hz), 6.95-7.00(3H, m), 7.04-7.09(1H, m), 7.29-7.35(2H, m), 7.38(1H, dd, J=8.8, 2.6Hz), 7.47(1H, d, J=2.6Hz), 8.19(1H, d, J=2.9Hz), 8.61(1H, brs), 11.92(1H, s).

### Example 383: Preparation of the compound of Compound No. 383.

Using 5-chlorosalicylic acid and 3-amino-4-methylbiphenyl as the raw materials, the same operation as the Example 16 gave the title compound.
Yield: 47.7%.
¹H-NMR(DMSO-d₆): δ 2.33(3H, s), 7.06(1H, d, J=8.7Hz), 7.43-7.52(4H, m), 7.64-7.67(2H, m), 8.04(1H, d, J=2.7Hz), 8.19(1H, d, J=1.5Hz), 10.40(1H, s), 12.22(1H, s).

### Example 384: Preparation of the compound of Compound No. 384.

Using 5-chlorosalicylic acid and 5-(α,α-dimethylbenzyl)-2-methoxyaniline as the raw materials, the same operation as the Example 16 gave the title compound.
Yield: 89.0%.
¹H-NMR(CDCl₃): δ 1.72(6H, s), 3.93(3H, s), 6.83(1H, d, J=8.8Hz), 6.93(1H, dd, J=2.6, 8.8Hz), 6.96(1H, d, J=9.2Hz), 7.15-7.20(1H, m), 7.25-7.28(4H, m), 7.36(1H, dd, J=2.6, 8.8Hz), 7.46(1H, d, J=2.6Hz), 8.35(1H, d, J=2.6Hz), 8.51(1H, s), 12.04(1H, s).

### Example 385: Preparation of the compound of Compound No. 385.

Using 5-chlorosalicylic acid and 5-morpholino-2-nitroaniline as the raw materials, the same operation as the Example 16 gave the title compound.
Yield: 4.1%.
¹H-NMR(DMSO-d₆): δ 3.46-3.52(4H, m), 3.85-3.94(4H, m), 7.03(1H, d, J=8.8Hz), 7.47(1H, dd, J=2.9, 8.8Hz), 7.80(1H, dd, J=2.6, 8.8Hz), 7.82(1H, d, J=2.6Hz), 7.88(1H, d, J=8.8Hz), 8.20(1H, d, J=2.2Hz), 10.70(1H, s), 11.43(1H, s)

### Example 386: Preparation of the compound of Compound No. 386.

Using 5-chlorosalicylic acid and 5-fluoro-2-(1-imidazolyl)aniline as the raw materials, the same operation as the Example 16 gave the title compound.
Yield: 33.8%.
¹H-NMR(DMSO-d₆): δ6.99(1H, d, J=8.8Hz), 7.12-7.19(2H, m), 7.42-7.51(3H, m), 7.89(1H, d, J=2.8Hz), 7.93(1H, d, J=1.1Hz), 8.34(1H, dd, J=11.4, 2.8Hz), 10.39(1H, s), 11.76(1H, brs).

### Example 387: Preparation of the compound of Compound No. 387.

Using 5-chlorosalicylic acid and 2-butyl-5-nitroaniline as the raw materials, the same operation as the Example 16 gave the title compound.
Yield: 15.3%.
¹H-NMR(CDCl₃): δ 0.99(3H, t, J=7.3Hz), 1.39-1.51(2H, m), 1.59-1.73(2H, m), 2.71-2.79(2H, m), 7.03(1H, d, J=8.9Hz), 7.41-7.49(3H, m), 7.92(1H, s), 8.07(1H, dd, J=2.3, 8.4Hz), 8.75(1H, d, J=2.4Hz), 11.51(1H, s).

### Example 388: Preparation of the compound of Compound No. 388.

Using 5-chlorosalicylic acid and 5-[(1,1-dimethyl)propyl]-2-hydroxyaniline as the raw materials, the same operation as the Example 16 gave the title compound.
Yield: 36.0%.
¹H-NMR(CDCl₃): δ 0.70(3H, t, J=7.4Hz), 1.28(6H, s), 1.63(2H, q, J=7.4Hz), 6.97(1H, d, J=6.3Hz), 7.00(1H, d, J=6.6Hz), 7.08(1H, s), 7.14(1H, dd, J=2.5, 8.6Hz), 7.36(1H, d, J=2.2Hz), 7.42(1H, dd, J=2.5, 8.8Hz), 7.57(1H, d, J=2.5Hz), 8.28(1H, s), 11.44(1H, s).

### Example 389: Preparation of the compound of Compound No. 389.

Using 5-chlorosalicylic acid and 2-methoxy-5-methylaniline as the raw materials, the same operation as the Example 16 gave the title compound.
Yield: 74.2%.
¹H-NMR(DMSO-d₆): δ2.27(3H, s), 3.85(3H, s), 6.90(1H, dd, J=9.0, 2.4Hz), 6.98(1H, d, J=9.0Hz), 7.05(1H, d, J=9.0Hz), 7.47(1H, dd, J=9.0, 3.0Hz), 7.97(1H, d, J=3.0Hz), 8.24(1H, d, J=2.4Hz), 10.79(1H, s), 12.03(1H, s).

### Example 390: Preparation of the compound of Compound No. 390.

Using 5-chlorosalicylic acid and 2,5-difluoroaniline as the raw materials, the same operation as the Example 16 gave the title compound.
Yield: 81.5%.
¹H-NMR(DMSO-d₆): δ 6.98-7.07(1H, m), 7.07(1H, d, J=9.0Hz), 7.37-7.49(1H, m), 7.52(1H, dd, J=8.7, 3.0Hz), 7.95(1H, d, J=2.7Hz), 8.15-8.22(1H, m), 10.83(1H, s), 12.25(1H, s).

### Example 391: Preparation of the compound of Compound No. 391.

Using 5-chlorosalicylic acid and 3,5-difluoroaniline as the raw materials, the same operation as the Example 16 gave the title compound.
Yield: 82.0%.
¹H-NMR(DMSO-d₆): δ 7.00(1H, tt, J=9.3, 2.1), 7.03(1H, d, J=9.0Hz), 7.47(1H, dd, J=7.5, 2.7Hz), 7.49(1H, d, J=2.7Hz), 7.51(1H, d, J=2.1Hz), 7.82(1H, d, J=3.0Hz), 10.63(1H, s), 11.43(1H, brs).

### Example 392: Preparation of the compound of Compound No. 392.

Using 2-(5-bromo-2-hydroxybenzoyl)amino-4-[(1,1-dimethyl)ethyl]thiazole-5-carboxylic acid ethyl ester(Compound No. 197) as the raw material, the same operation as the Example 82 gave the title compound.
Yield: 85.5%.
¹H-NMR(DMSO-d₆): δ1.44(9H, s), 7.00(1H, d, J=9.0Hz), 7.62(1H, dd, J=9.0, 2.7Hz), 8.02(1H, d, J=2.4Hz), 11.83(1H, brs), 12.04(1H, brs), 12.98(1H, brs).

### Example 393: Preparation of the compound of Compound No. 393.

Using 5-bromosalicylic acid and 2-amino-4-phenylthiazole-5-acetic acid methyl ester as the raw materials, the same operation as the Example 195(3) gave the title compound. (This compound is the compound of Example 203(1).)
Yield: 32.1%.
mp 288.5-229.5°C.
¹H-NMR(DMSO-d₆): δ 3.66(3H, s), 3.95(2H, s), 6.99(1H, d, J=8.0Hz), 7.42(1H, d, J=6.0Hz), 7.48(2H, brt, J=7.6Hz), 7.56-7.61(3H, m), 8.07(1H, d, J=2.4Hz), 11.85(1H, brs), 11.98(1H, brs).

### Example 394: Preparation of the compound of Compound No. 394.

Using 2-(5-bromo-2-hydroxybenzoyl)amino-4-phenylthiazole-5-carboxylic acid ethyl ester(Compound No. 209) as the raw material, the same operation as the Example 82 gave the title compound. (This compound is the compound of Example 212(1).)
Yield: 67.0%.
¹H-NMR(DMSO-d₆): δ 7.00(1H, d, J=8.8Hz), 7.42-7.44(3H, m), 7.62(1H, dd, J=8.8, 2.4Hz), 7.70-7.72(2H, m), 8.04(1H, d, J=2.4Hz), 12.31(1H, brs), 12.99(1H, brs).

### Example 395: Preparation of the compound of Compound No. 395.

### (1) 2-Amino-4-[3,5-bis(trifluoromethyl)phenyl]thiazole.

Phenyltrimethylammonium tribromide(753mg, 2mmol) was added to a solution of 3',5'-bis(trifluoromethyl)acetophenone(0.51g, 2.0mmol) in tetrahydrofuran(5mL) and the mixture was stirred at room temperature for 5 hours. The reaction mixture was poured into water and extracted with ethyl acetate. After the ethyl acetate layer was washed with brine, dried over anhydrous sodium sulfate, ethanol(5mL) and thiourea(152mg, 2mmol) were added to the residue obtained by evaporation of the solvent under reduced pressure, and the mixture was refluxed for 30 minutes. After the reaction mixture was cooled to room temperature, it was poured into saturated aqueous sodium hydrogen carbonate and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=2:1) and washed with n-hexane under suspension to give the title compound(520.1mg, 83.3%) as a light yellow white crystal.
¹H-NMR(CDCl₃): δ5.03(2H, s), 6.93(1H, s), 7.77(1H, s), 8.23(2H, s).

### (2) 5-Chloro-2-hydroxy-N-{4-[3,5-bis(trifluoromethyl)phenyl]thiazol-2-yl}benzamide (Compound No. 395).

A mixture of 5-chlorosalicylic acid(172.6mg, 1mmol), 2-amino-4-[3,5-bis(trifluoromethyl)phenyl]thiazole(312.2mg, 1mmol), phosphorus trichloride(44µL, 0.5mmol) and monochlorobenzene(5mL) was refluxed for 4 hours. After the reaction mixture was cooled to room temperature, it was poured into water and extracted with ethyl acetate. After the ethyl acetate layer was washed with brine, dried over anhydrous sodium sulfate, the residue obtained by evaporation of the solvent under reduced pressure was purified by column chromatography on silica gel(n-hexane:ethyl acetate=3:1→2:1) to give the title compound(109.8mg, 23.5%) as a pale yellow white powder.
¹H-NMR(DMSO-d₆): δ7.08(1H, d, J=8.7Hz), 7.53(1H, dd, J=9.0, 3.0Hz), 7.94(1H, d, J=3.0Hz), 8.07(1H, s), 8.29(1H, s), 8.60(2H, s), 11.77(1H, s), 12.23(1H, s).

### Example 396: Preparation of the compound of Compound No. 396.

Using 5-chlorosalicylic acid and 3-aminopyridine as the raw materials, the same operation as the Example 16 gave the title compound.
Yield: 23.2%.
¹H-NMR(DMSO-d₆): δ7.02(1H, d, J=9.3Hz), 7.42(1H, ddd, J=9.0, 4.8, 0.6Hz), 7.47(1H, dd, J=8.7, 5.7Hz), 7.92(1H, d, J=2.7Hz), 8.15(1H, ddd, J=8.4, 2.4, 1.5Hz), 8.35(1H, dd, J=7.8, 1.5Hz), 8.86(1H, d, J=2.4Hz), 10.70(1H, s).

### Example 397: Preparation of the compound of Compound No. 397.

Using 5-chlorosalicylic acid and 2-amino-6-bromopyridine as the raw materials, the same operation as the Example 16 gave the title compound.
Yield: 12.3%.
¹H-NMR(DMSO-d₆): δ 7.07(1H, d, J=8.7Hz), 7.42(1H, d, J=7.8Hz), 7.51(1H, dd, J=8.7, 2.7Hz), 7.82(1H, t, J=7.5Hz), 7.94(1H, d, J=3.0Hz), 8.24(1H, d, J=7.8Hz), 10.95(1H, s), 11.97(1H, s).

### Example 398: Preparation of the compound of Compound No. 398.

### (1) 2-Acetoxy-5-chloro-N-(pyridazin-2-yl)benzamide.

Using 2-acetoxy-5-chlorobenzoic acid and 2-aminopyridazine as the raw materials, the same operation as the Example 198(3) gave the title compound.
Yield: 19.7%.
¹H-NMR(CDCl₃): δ 2.42(3H, s), 7.19(1H, d, J=8.7Hz), 7.54(1H, dd, J=8.7, 2.7Hz), 8.01(1H, d, J=2.4Hz), 8.28(1H, dd, J=2.4, 1.8Hz), 8.42(1H, d, J=2.4Hz), 9.09(1H, s), 9.66(1H, d, J=1.8Hz).

### (2) 5-Chloro-2-hydroxy-N-(pyridazin-2-yl)benzamide(Compound No. 398).

Using 2-acetoxy-5-chloro-N-(pyridazin-2-yl)benzamide as the raw material, the same operation as the Example 2(2) gave the title compound.
Yield: 72.6%.
¹H-NMR(DMSO-d₆): δ 7.09(1H, d, J=9.0Hz), 7.52(1H, dd, J=8.7, 2.7Hz), 7.96(1H, d, J=2.7Hz), 8.44-8.47(2H, m), 9.49(1H, s), 10.99(1H, s), 12.04(1H, s).

### Example 399: Preparation of the compound of Compound No. 399.

Using 5-bromosalicylic acid and 2-amino-5-bromopyrimidine as the raw materials, the same operation as the Example 16 gave the title compound.
Yield: 10.3%.
¹H-NMR(DMSO-d₆): δ 6.98(1H, d, J=8.8Hz), 7.59(1H, dd, J=8.8, 2.4Hz), 8.00(1H, d, J=2.8Hz), 8.86(2H, s), 11.09(1H, s), 11.79(1H, s).

### Example 400: Preparation of the compound of Compound No. 400.

Using 2-(5-bromo-2-hydroxybenzoyl)amino-4-phenylthiazole-5-carboxylic acid(Compound No. 394) and propylamine as the raw materials, the same operation as the Example 212(2) gave the title compound.
Yield: 23.1%.
¹H-NMR(DMSO-d₆): δ0.82(3H, t, J=7.5Hz), 1.39-1.51(2H, m), 3.13(2H, q, J=6.6Hz), 7.02(1H, d, J=9.0Hz), 7.40-7.48(3H, m), 7.63(1H, dd, J=8.7, 2.7Hz), 7.68-7.72(2H, m), 8.06(1H, d, J=2.7Hz), 8.18(1H, t, J=5.7Hz), 11.87(1H, brs), 12.14(1H, brs).

### Example 401: Preparation of the compound of Compound No. 401.

Using 5-chlorosalicylic acid and 2-methyl-3,5-bis(trifluoromethyl)aniline as the raw materials, the same operation as the Example 16 gave the title compound.
Yield: 15.0%.
¹H-NMR(DMSO-d₆): δ 2.49(3H, s), 7.07(1H, d, J=8.7Hz), 7.52(1H, dd, J=8.7, 2.8Hz), 7.84(1H, s), 7.97(1H, d, J=2.8Hz), 8.60(1H, s), 10.69(1H, brs), 12.07(1H, brs).

### Example 402: Preparation of the compound of Compound No. 402.

Using 5-chlorosalicylic acid and 4-chloro-3-(trifluoromethyl)aniline as the raw materials, the same operation as the Example 16 gave the title compound.
Yield: 66.5%.
¹H-NMR(DMSO-d₆): δ7.03(1H, d, J=8.7Hz), 7.48(1H, dd, J=8.7, 2.7Hz), 7.73(1H, d, J=8.7Hz), 7.86(1H, d, J=2.4Hz), 8.00(1H, dd, J=8.7, 2.4Hz), 8.32(1H, d, J=2.4Hz), 10.69(1H, s), 11.49(1H, s).

### Example 403: Preparation of the compound of Compound No. 403.

Using 5-chlorosalicylic acid and 4-isopropyl-2-(trifluoromethyl)aniline as the raw materials, the same operation as the Example 16 gave the title compound.
Yield: 33.4%.
¹H-NMR(DMSO-d₆): δ1.24(6H, d, J=6.6Hz), 2.97-3.06(1H, m), 7.06(1H, d, J=8.7Hz), 7.51(1H, dd, J=8.7, 2.7Hz), 7.61(1H, s), 7.62(1H, d, J=7.5Hz), 7.98(1H, d, J=2.7Hz), 8.03(1H, d, J=8.1Hz), 10.67(1H, s), 12.21(1H, s).

### Example 404: Preparation of the compound of Compound No. 404.

Using 5-chlorosalicylic acid and 3-(trifluoromethyl)aniline as the raw materials, the same operation as the Example 16 gave the title compound.
Yield: 68.5%.
¹H-NMR(DMSO-d₆): δ 7.03(1H, d, J=8.6Hz), 7.46-7.51(2H, m), 7.62(1H, t, J=7.9Hz), 7.90(1H, d, J=3.0Hz), 7.94(1H, d, J=9.2Hz), 8.21(1H, s), 10.64(1H, s), 11.58(1H, brs).

### Example 405: Preparation of the compound of Compound No. 405.

Using 5-chlorosalicylic acid and 2-nitro-4-(trifluoromethyl)aniline as the raw materials the same operation as the Example 16 gave the title compound.
Yield: 18.7%.
¹H-NMR(DMSO-d₆): δ7.08(1H, d, J=9.0Hz), 7.54(1H, dd, J=8.7, 2.7Hz), 7.94(1H, d, J=2.7Hz), 8.17(1H, dd, J=9.0, 2.4Hz), 8.46(1H, d, J=1.8Hz), 8.88(1H, d, J=9.0Hz), 12.19(1H, s), 12.25(1H, s).

### Example 406: Preparation of the compound of Compound No. 406.

Using 5-chlorosalicylic acid and 2,6-dichloro-4-(trifluoromethyl)aniline as the raw materials, the same operation as the Example 16 gave the title compound.
Yield: 22.1%.
¹H-NMR(DMSO-d₆): δ7.07(1H, d, J=8.7Hz), 7.55(1H, dd, J=8.7, 2.7Hz), 7.99(1H, d, J=2.4Hz), 8.10(2H, s), 10.62(1H, s), 11.88(1H, s).

### Example 407: Preparation of the compound of Compound No. 407.

Using 5-chlorosalicylic acid and 4-cyano-3-(trifluoromethyl)aniline as the raw materials, the same operation as the Example 16 gave the title compound.
Yield: 55.8%.
¹H-NMR(DMSO-d₆): δ 7.04(1H, d, J=8.7Hz), 7.49(1H, dd, J=8.7, 2.7Hz), 7.80(1H, d, J=2.7Hz), 8.17(2H, s), 8.43(1H, s), 10.94(1H, s), 11.34(1H, s).

### Example 408: Preparation of the compound of Compound No. 408.

Using 5-chlorosalicylic acid and 4-bromo-3-(trifluoromethyl)aniline as the raw materials, the same operation as the Example 16 gave the title compound.
Yield: 81.2%.
¹H-NMR(DMSO-d₆): δ 7.03(1H, d, J=8.7Hz), 7.48(1H, dd, J=9.0, 2.7Hz), 7.85-7.94(3H, m), 8.31(1H, d, J=1.8Hz), 10.67(1H, s), 11.48(1H, s).

### Example 409: Preparation of the compound of Compound No. 409.

Using 5-chlorosalicylic acid and 4-bromo-2-(trifluoromethyl)aniline as the raw materials, the same operation as the Example 16 gave the title compound.
Yield: 41.8%.
¹H-NMR(DMSO-d₆): δ 7.07(1H, d, J=8.7Hz), 7.52(1H, dd, J=9.0, 2.7Hz), 7.93-7.97(3H, m), 8.21(1H, d, J=9.3Hz), 10.81(1H, s), 12.28(1H, s).

### Example 410: Preparation of the compound of Compound No. 410.

Using 5-chlorosalicylic acid and 2-bromo-4-(trifluoromethyl)aniline as the raw materials, the same operation as the Example 16 gave the title compound.
Yield: 17.6%.
¹H-NMR(DMSO-d₆): δ 7.10(1H, d, J=9.0Hz), 7.53(1H, dd, J=8.7, 3.0Hz), 7.82(1H, dd, J=9.0, 1.8Hz), 7.98(1H, d, J=3.0Hz), 8.11(1H, d, J=1.5Hz), 8.67(1H, d, J=8.7Hz), 11.05(1H, s), 12.40(1H, s).

### Example 411: Preparation of the compound of Compound No. 411.

Using 5-chlorosalicylic acid and 4-fluoro-2-(trifluoromethyl)aniline as the raw materials, the same operation as the Example 16 gave the title compound.
Yield: 36.0%.
¹H-NMR(DMSO-d₆): δ 7.06(1H, d, J=9.0Hz), 7.52(1H, dd, J=8.7, 2.7Hz), 7.63(1H, td, J=8.7, 3.3Hz), 7.71(1H, dd, J=8.7, 3.0Hz), 7.97(1H, d, J=2.7Hz), 8.11(1H, dd, J=8.7, 5.1Hz), 10.67(1H, s), 12.20(1H, s).

### Example 412: Preparation of the compound of Compound No. 412.

Using 5-chlorosalicylic acid and 4-isopropyloxy-2-(trifluoromethyl)aniline as the raw materials, the same operation as the Example 16 gave the title compound.
Yield: 39.2%.
¹H-NMR(DMSO-d₆): δ1.29(6H, d, J=5.7Hz), 4.67-4.79(1H, m), 7.04(1H, d, J=9.0Hz), 7.22(1H, d, J=2.7Hz), 7.30(1H, dd, J=8.7, 2.7Hz), 7.51(1H, dd, J=8.7, 2.4Hz), 7.86(1H, d, J=9.0Hz), 7.99(1H, d, J=3.0Hz), 10.50(1H, s), 12.18(1H, s).

### Example 413: Preparation of the compound of Compound No. 413.

Using 5-chlorosalicylic acid and 2,4-dimethoxy-5-(trifluoromethyl)aniline as the raw materials, the same operation as the Example 16 gave the title compound.
Yield: 19.0%.
¹H-NMR(CDCl₃): δ 3.93(3H, s), 4.03(3H, s), 6.70(1H, s), 6.98(1H, d, J=8.9Hz), 7.39(1H, dd, J=8.9, 2.6Hz), 7.45(1H, d, J=2.6Hz), 8.29(1H, brs, ), 8.54(1H, s), 11.92(1H, s).

### Example 414: Preparation of the compound of Compound No. 414.

Using 5-chlorosalicylic acid and 2,4-difluoro-5-(trifluoromethyl)aniline as the raw materials, the same operation as the Example 16 gave the title compound.
Yield: 66.0%.
¹H-NMR(DMSO-d₆): δ 7.06(1H, d, J=8.8Hz), 7.51(1H, dd, J=8.8, 2.8Hz), 7.82(1H, t, J=10.7Hz), 7.94(1H, d, J=2.8Hz), 8.64(1H, d, J=8.0Hz), 10.78(1H, s), 12.37(1H, brs).

### Example 415: Preparation of the compound of Compound No. 415.

Using 5-chlorosalicylic acid and 4-cyano-2-(trifluoromethyl)aniline as the raw materials, the same operation as the Example 16 gave the title compound.
Yield: 24.8%.
¹H-NMR(DMSO-d₆): δ7.06(1H, d, J=8.8Hz), 7.52(1H, dd, J=2.8, 8.8Hz), 7.94(1H, d, J=2.8Hz), 8.17(1H, dd, J=1.8, 8.9Hz), 8.31(1H, d, J=2.1Hz), 8.63(1H, d, J=8.9Hz), 11.16(1H, s), 12.45(1H, br.s).

### Example 416: Preparation of the compound of Compound No. 416.

Using 5-chlorosalicylic acid and 4-chloro-2-(4-chlorobenzenesulfonyl)-5-(trifluoromethyl)aniline as the raw materials, the same operation as the Example 16 gave the title compound.
Yield: 8.5%.
¹H-NMR(CDCl₃): δ 6.98(1H, d, J=8.9Hz), 7.13(1H, d, J=2.6Hz), 7.22(2H, d, J=8.6Hz), 7.34(2H, d, J=8.6Hz), 7.40(1H, dd, J=2.3, 8.9Hz), 7.66(1H, s), 8.71(1H, s), 8.80(1H, s), 11.42(1H, s).

### Example 417: Preparation of the compound of Compound No. 417.

Using 5-chlorosalicylic acid and 5-chloro-2-nitro-4-(trifluoromethyl)aniline as the raw materials, the same operation as the Example 16 gave the title compound.
Yield: 22.8%.
¹H-NMR(DMSO-d₆): δ7.08(1H, d, J=8.8Hz), 7.55(1H, dd, J=8.8, 2.8Hz), 7.93(1H, d, J=2.8Hz), 8.52(1H, s), 9.13(1H, s), 12.38(1H, brs), 12.45(1H, s).

### Example 418: Preparation of the compound of Compound No. 418.

Using 5-chlorosalicylic acid and 2,3-difluoro-4-(trifluoromethyl)aniline as the raw materials, the same operation as the Example 16 gave the title compound.
Yield: 21.8%.
¹H-NMR(DMSO-d₆): δ 7.07(1H, d, J=8.8Hz), 7.53(1H, dd, J=2.9, 8.8Hz), 7.66(1H, dt, J=1.8, 7.7Hz), 7.93(1H, d, J=2.6Hz), 8.35(1H, t, J=7.7Hz), 11.02(1H, d, J=1.5Hz), 12.32(1H, s).

### Example 419: Preparation of the compound of Compound No. 419.

Using 5-chlorosalicylic acid and 4,4'-diamino-2,2'-bis(trifluoromethyl)biphenyl as the raw materials, the same operation as the Example 16 gave the title compound.
Yield: 35.9%.
¹H-NMR(DMSO-d₆): δ 7.05(2H, d, J=8.8Hz), 7.39(2H, d, J=8.5Hz), 7.49-7.51(2H, m), 7.91(2H, d, J=2.5Hz), 7.99(2H, dd, J=2.0, 8.5Hz), 8.31(2H, d, J=1.9Hz), 10.71(2H, s), 11.54(2H, s).

### Example 420: Preparation of the compound of Compound No. 420.

Using 5-chlorosalicylic acid and 2,3,5,6-tetrafluoro-4-(trifluoromethyl)aniline as the raw materials, the same operation as the Example 16 gave the title compound.
Yield: 42.5%.
¹H-NMR(DMSO-d₆): δ7.08(1H, d, J=8.8Hz), 7.53(1H, dd, J=2.9, 8.8Hz), 7.89(1H, d, J=2.6Hz), 10.65(1H, br.s), 11.76(1H, br.s).

### Example 421: Preparation of the compound of Compound No. 421.

Using 5-chlorosalicylic acid and 3'-aminoacetanilide as the raw materials, the same operation as the Example 16 gave the title compound.
Yield: 22.4%.
¹H-NMR(DMSO-d₆): δ2.05(3H, s), 7.01(1H, d, J=8.7Hz), 7.24-7.39(3H, m), 7.47(1H, dd, J=9.0, 3.0Hz), 7.97(1H, d, J=3.0Hz), 8.03(1H, s), 10.01(1H, s), 10.41(1H, s), 11.87(1H, s).

### Example 422: Preparation of the compound of Compound No. 422.

### (1) 2-Acetoxy-5-chloro-N-(3-carbamoylphenyl)benzamide.

Using 2-acetoxy-5-chlorobenzoic acid and 3-aminobenzamide as the raw materials, the same operation as the Example 24 gave the title compound.
Yield: 15.8%.
¹H-NMR(CDCl₃): δ 2.33(3H, s), 5.89(1H, brs), 6.31(1H, brs), 7.14(1H, d, J=9.0Hz), 7.42-7.49(2H, m), 7.55-7.58(1H, m), 7.80(1H, d, J=2.7Hz), 7.93(1H, d, J=8.1Hz), 8.07(1H, s), 8.71(1H, s).

### (2) 5-Chloro-2-hydroxy-N-(3-carbamoylphenyl)benzamide(Compound No. 422).

Using 2-acetoxy-5-chloro-N-(3-carbamoylphenyl)benzamide as the raw material, the same operation as the Example 2(2) gave the title compound.
Yield: 76.0%.
¹H-NMR(DMSO-d₆): δ 7.03(1H, d, J=8.7Hz), 7.40(1H, brs), 7.45(1H, t, J=7.5Hz), 7.48(1H, dd, J=8.7, 2.4Hz), 7.62-7.65(1H, m), 7.86-7.89(1H, m), 7.98-7.99(2H, m), 8.15(1H, t, J=1.8Hz), 10.51(1H, s), 11.85(1H, s).

### Example 423: Preparation of the compound of Compound No. 423.

Using 5-chlorosalicylic acid and 3-amino-N-methylbenzamide as the raw materials, the same operation as the Example 16 gave the title compound.
Yield: 19.3%.
¹H-NMR(DMSO-d₆): δ2.79(3H, d, J=4.5Hz), 7.03(1H, d, J=9.0Hz), 7.43-7.51(2H, m), 7.59(1H, dt, J=8.1, 1.5Hz), 7.87(1H, ddd, J=8.1, 2.1, 0.9Hz), 7.99(1H, d, J=2.4Hz), 8.15(1H, t, J=1.8Hz), 8.46(1H, d, J=4.2Hz), 10.52(1H, s), 11.84(1H, s).

### Example 424: Preparation of the compound of Compound No. 424.

Using 5-chlorosalicylic acid and 2,6-diisopropylaniline as the raw materials, the same operation as the Example 16 gave the title compound.
Yield: 52.5%.
¹H-NMR(DMSO-d₆): δ1.14(12H, s), 2.96-3.13(2H, m), 7.16(1H, d, J=8.7Hz), 7.23(1H, d, J=7.5Hz), 7.33(1H, dd, J=8.4, 6.6Hz), 7.52(1H, dd, J=8.7, 2.4Hz), 8.11(1H, d, J=2.4Hz), 10.09(1H, s), 12.40(1H, s).

### Example 425: Preparation of the compound of Compound No. 425.

Using 5-chlorosalicylic acid and 4-methylaniline as the raw materials, the same operation as the Example 16 gave the title compound.
Yield: 58.6%.
¹H-NMR(DMSO-d₆): δ 2.29(3H, s), 7.01(1H, d, J=8.7Hz), 7.18(1H, d, J=8.1Hz), 7.47(1H, dd, J=8.7, 2.7Hz), 7.58(1H, d, J=8.4Hz), 7.98(1H, d, J=2.7Hz), 10.35(1H, s), 11.94(1H, s).

### Example 426: Preparation of the compound of Compound No. 426.

Using 5-chlorosalicylic acid and 2,6-dimethylaniline as the raw materials, the same operation as the Example 16 gave the title compound.
Yield: 59.6%.
¹H-NMR(DMSO-d₆): δ 2.19(6H, s), 7.01(1H, d, J=9.0Hz), 7.15-7.16(2H, m), 7.50(1H, dd, J=9.0, 2.7Hz), 8.07(1H, d, J=2.7Hz), 10.03(1H, s), 10.10(1H, s), 12.29(1H, s).

### Example 427: Preparation of the compound of Compound No. 427.

Using 5-chlorosalicylic acid and 3,4-dimethylaniline as the raw materials, the same operation as the Example 16 gave the title compound.
Yield: 68.3%.
¹H-NMR(DMSO-d₆): δ 2.20(3H, s), 2.23(3H, s), 7.01(1H, d, J=9.0Hz), 7.13(1H, d, J=8.4Hz), 7.40-7.47(2H, m), 7.47(1H, dd, J=9.0, 2.7Hz), 7.99(1H, d, J=2.7Hz), 10.29(1H, s), 11.97(1H, brs).

### Example 428: Preparation of the compound of Compound No. 428.

Using 5-chlorosalicylic acid and 2,4,6-trimethylaniline as the raw materials, the same operation as the Example 16 gave the title compound.
Yield: 61.0%.
¹H-NMR(DMSO-d₆): δ 2.14(6H, s), 2.26(3H, s), 6.95(2H, s), 7.00(1H, d, J=9.3Hz), 7.48(1H, dd, J=8.7, 2.7Hz), 8.09(1H, d, J=2.4Hz), 10.03(1H, s), 12.37(1H, s).

### Example 429: Preparation of the compound of Compound No. 429.

Using 5-chlorosalicylic acid and 3-(trifluoromethoxy)aniline as the raw materials, the same operation as the Example 16 gave the title compound.
Yield: 41.4%.
¹H-NMR(CDCl₃): δ7.00(1H, d, J=9.0Hz), 7.09(1H, d, J=7.5Hz), 7.40-7.48(3H, m), 7.51(1H, d, J=2.4Hz), 7.64(1H, s), 7.94(1H, s), 11.66(1H, s).

### Example 430: Preparation of the compound of Compound No. 430.

Using 5-chlorosalicylic acid and 2-benzylaniline as the raw materials, the same operation as the Example 16 gave the title compound.
Yield: 93.3%.
¹H-NMR(CDCl₃): δ4.08(2H, s), 6.56(1H, d, J=2.5Hz), 6.92(1H, d, J=8.8Hz), 7.20-7.46(9H, m), 7.53(1H, brs), 7.85(1H, d, J=8.0Hz), 12.01(1H, brs).

### Example 431: Preparation of the compound of Compound No. 431.

Using 5-chlorosalicylic acid and 4-(trifluoromethoxy)aniline as the raw materials, the same operation as the Example 16 gave the title compound.
Yield: 20.4%.
¹H-NMR(DMSO-d₆): δ 7.03(1H, d, J=9.3Hz), 7.39(2H, d, J=9.0Hz), 7.48(1H, dd, J=9.0, 2.7Hz), 7.83(2H, d, J=9.3Hz), 7.92(1H, d, J=2.7Hz), 10.54(1H, s), 11.78(1H, s).

### Example 432: Preparation of the compound of Compound No. 432.

Using 5-chlorosalicylic acid and 2,4-dichloroaniline as the raw materials, the same operation as the Example 16 gave the title compound.
Yield: 60.0%.
¹H-NMR(DMSO-d₆): δ7.08(1H, d, J=8.7Hz), 7.48-7.54(2H, m), 7.75(1H, d, J=2.1Hz), 7.98(1H, d, J=2.7Hz), 8.44(1H, d, J=8.7Hz), 10.93(1H, s), 12.31(1H, s).

### Example 433: Preparation of the compound of Compound No. 433.

Using 5-chlorosalicylic acid and 4-(tert-butyl)aniline as the raw materials, the same operation as the Example 16 gave the title compound.
Yield: 69.0%.
¹H-NMR(DMSO-d₆): δ 1.29(9H, s), 7.01(1H, d, J=8.7Hz), 7.39(2H, d, J=8.4Hz), 7.47(1H, dd, J=8.7, 2.7Hz), 7.61(2H, d, J=8.4Hz), 7.99(1H, d, J=2.4Hz), 10.37(1H, s), 11.96(1H, s).

### Example 434: Preparation of the compound of Compound No. 434.

Using 5-chlorosalicylic acid and 2,3-dimethylaniline as the raw materials, the same operation as the Example 16 gave the title compound.
Yield: 79.5%.
¹H-NMR(DMSO-d₆): δ2.14(3H, s), 2.29(3H, s), 7.03(1H, d, J=9.0Hz), 7.06-7.15(2H, m), 7.46-7.51(2H, m), 8.05(1H, d, J=3.0Hz), 10.32(1H, s), 12.28(1H, s).

### Example 435: Preparation of the compound of Compound No. 435.

Using 5-chlorosalicylic acid and 5-aminoindane as the raw materials, the same operation as the Example 16 gave the title compound.
Yield: 80.7%.
¹H-NMR(DMSO-d₆): δ 1.98-2.08(2H, m), 2.81-2.89(4H, m), 7.01(1H, d, J=8.8Hz), 7.21(1H, d, J=8.0, Hz), 7.42(1H, dd, J=8.0, 1.9Hz), 7.48(1H, dd, J=8.8, 2.8Hz), 7.60(1H, s), 7.99(1H, d, J=2.8, Hz), 10.34(1H, s), 12.00(1H, brs).

### Example 436: Preparation of the compound of Compound No. 436.

Using 5-chlorosalicylic acid and 2,4-dimethylaniline as the raw materials, the same operation as the Example 16 gave the title compound.
Yield: 37.1%.
¹H-NMR(DMSO-d₆): δ 2.23(3H, s), 2.28(3H, s), 7.03(2H, d, J=8.7Hz), 7.10(1H, s), 7.49(1H, dd, J=9.0, 2.7Hz), 7.63(1H, d, J=8.1Hz), 8.03(1H, d, J=2.4Hz), 10.24(1H, s), 12.25(1H, s).

### Example 437: Preparation of the compound of Compound No. 437.

Using 5-chlorosalicylic acid and 3-isopropyloxyaniline as the raw materials, the same operation as the Example 16 gave the title compound.
Yield: 21.5%.
¹H-NMR(CDCl₃): δ 1.36(6H, d, J=6.0Hz), 4.52-4.64(1H, m), 6.75(1H, ddd, J=8.4, 2.4, 0.9Hz), 6.99(1H, d, J=8.7Hz), 7.03(1H, ddd, J=8.1, 2.1, 0.9Hz), 7.25-7.31(3H, m), 7.39(1H, dd, J=8.7, 2.4Hz), 7.49(1H, d, J=2.4Hz), 7.81(1H, s).

### Example 438: Preparation of the compound of Compound No. 438.

Using 5-chlorosalicylic acid and 2,6-dichloroaniline as the raw materials, the same operation as the Example 16 gave the title compound.
Yield: 10.3%.
¹H-NMR(DMSO-d₆): δ7.05(1H, d, J=8.7Hz), 7.43(1H, dd, J=8.7, 7.8Hz), 7.54(1H, dd, J=9.0, 2.7Hz), 7.62(1H, d, J=8.1Hz), 8.05(1H, d, J=2.4Hz), 10.52(1H, s), 12.01(1H, s).

### Example 439: Preparation of the compound of Compound No. 439.

Using 5-chlorosalicylic acid and 4-isopropyloxyaniline as the raw materials, the same operation as the Example 16 gave the title compound.
Yield: 76.8%.
¹H-NMR(DMSO-d₆): δ1.26(6H, d, J=6.3Hz), 4.52-4.64(1H, m), 6.93(2H, dt, J=9.0, 2.1Hz), 7.46(1H, dd, J=9.0, 2.7Hz), 7.58(2H, dt, J=9.0, 2.1Hz), 7.99(1H, d, J=3.0Hz), 10.36(1H, s), 11.83(IH, brs).

### Example 440: Preparation of the compound of Compound No. 440.

Using 5-chlorosalicylic acid and 4-bromo-2-(trifluoromethoxy)aniline as the raw materials, the same operation as the Example 16 gave the title compound.
Yield: 59.2%.
¹H-NMR(CDCl₃): δ 7.01(1H, d, J=9.3Hz), 7.42-7.52(4H, m), 8.23(1H, s), 8.31(1H, d, J=9.3Hz), 11.35(1H, s).

### Example 441: Preparation of the compound of Compound No. 441.

Using 5-chlorosalicylic acid and 4-butylaniline as the raw materials, the same operation as the Example 16 gave the title compound.
Yield: 77.6%
¹H-NMR(CDCl₃): δ 0.89(3H, t, J=6.9Hz), 1.27-1.36(6H, m), 1.56-1.64(2H, m), 2.61(2H, t, J=7.8Hz), 6.99(1H, d, J=9.0Hz), 7.21(2H, d, J=8.7Hz), 7.39(1H, dd, J=9.0, 2.7Hz), 7.44-7.49(3H, m), 7.80(1H, s), 11.96(1H, s).

### Example 442: Preparation of the compound of Compound No. 442.

Using 5-chlorosalicylic acid and 3-methylaniline as the raw materials, the same operation as the Example 16 gave the title compound.
Yield: 88.3%.
¹H-NMR(CDCl₃): δ 2.38(3H, s), 6.98(1H, d, J=8.8Hz), 7.03(1H, d, J=7.4Hz), 7.25-7.40(4H, m), 7.48(1H, d, J=2.2Hz), 7.83(1H, brs), 11.92(1H, brs).

### Example 443: Preparation of the compound of Compound No. 443.

Using 5-chlorosalicylic acid and 4-cyclohexylaniline as the raw materials, the same operation as the Example 16 gave the title compound.
Yield: 90.6%.
¹H-NMR(CDCl₃): δ1.15-1.47(5H, m), 1.56-1.87(5H, m), 2.40-2.53(2H, m), 7.01(1H, d, J=8.8Hz), 7.21(2H, d, J=8.5Hz), 7.47(1H, dd, J=8.8, 2.7Hz), 7.60(2H, d, J=8.5H), 8.00(1H, d, J=2.7Hz), 10.36(1H, s), 11.98(1H, brs).

### Example 444: Preparation of the compound of Compound No. 444.

Using 5-chlorosalicylic acid and 4-benzylaniline as the raw materials, the same operation as the Example 16 gave the title compound.
Yield: 90.3%.
¹H-NMR(DMSO-d₆): δ 3.93(2H, s), 7.01(1H, d, J=9.0Hz), 7.16-7.32(7H, m), 7.57(1H, dd, J=9.0, 2.7Hz), 7.61(2H, d, J=8.4Hz), 7.96(1H, d, J=2.4Hz), 10.37(1H, s).

### Example 445: Preparation of the compound of Compound No. 445.

Using 5-chlorosalicylic acid and 2-amino-4,5-dimethoxybenzonitrile as the raw materials, the same operation as the Example 16 gave the title compound.
Yield: 52.8%.
¹H-NMR(DMSO-d₆): δ 3.81(3H, s), 3.86(3H, s), 7.08(1H, d, J=8.7Hz), 7.40(1H, s), 7.52(1H, dd, J=8.7, 2.7Hz), 7.89(1H, s), 7.99(1H, d, J=3.0Hz), 10.93(1H, s), 12.31(1H, s).

### Example 446: Preparation of the compound of Compound No. 446.

Using 5-chlorosalicylic acid and 6-amino-1,4-benzodioxane as the raw materials, the same operation as the Example 16 gave the title compound.
Yield: 79.7%.
¹H-NMR(DMSO-d₆): δ 4.25(4H, s), 6.86(1H, d, J=8.8Hz), 7.00(1H, d, J=8.8Hz), 7.12(1H, dd, J=8.8, 2.5Hz), 7.33(1H, d, J=2.5Hz), 7.46(1H, dd, J=8.8, 2.5Hz), 7.97(1H, d, J=2.5Hz), 10.27(1H, s), 11.96(1H, s).

### Example 447: Preparation of the compound of Compound No. 447.

Using 5-chlorosalicylic acid and 2,4-dichloro-5-(isopropyloxy)aniline as the raw materials, the same operation as the Example 16 gave the title compound.
Yield: 76.1%.
¹H-NMR(DMSO-d₆): δ 1.35(6H, d, J=6.0Hz), 4.58-4.66(1H, m), 7.07(1H, d, J=9.0Hz), 7.51(1H, dd, J=8.7, 3.0Hz), 7.68(1H, s), 7.98(1H, d, J=3.0Hz), 8.35(1H, s), 10.94(1H, s), 12.34(1H, s).

### Example 448: Preparation of the compound of Compound No. 448.

Using 5-chlorosalicylic acid and 4-amino-2-chlorobenzonitrile as the raw materials, the same operation as the Example 16 gave the title compound.
Yield: 57.9%.
¹H-NMR(DMSO-d₆): δ7.04(1H, d, J=9.0Hz), 7.48(1H, dd, J=8.7, 2.7Hz), 7.78(1H, d, J=2.7Hz), 7.82(1H, dd, J=9.0, 2.1Hz), 7.97(1H, d, J=8.7Hz), 8.19(1H, d, J=2.1Hz), 10.79(1H, s), 11.38(1H, s).

### Example 449: Preparation of the compound of Compound No. 449.

Using 5-chlorosalicylic acid and 3-chloro-4-(trifluoromethoxy)aniline as the raw materials, the same operation as the Example 16 gave the title compound.
Yield: 50.6%.
¹H-NMR(DMSO-d₆): δ7.03(1H, d, J=8.7Hz), 7.48(1H, dd, J=8.7, 2.7Hz), 7.60(1H, dd, J=9.0, 1.5Hz), 7.76(1H, dd, J=9.0, 2.4Hz), 7.85(1H, d, J=3.0Hz), 8.13(1H, d, J=2.4Hz), 10.61(1H, s), 11.51(1H, s).

### Example 450: Preparation of the compound of Compound No. 450.

Using 5-chlorosalicylic acid and 4-amino-3-methylbenzonitrile as the raw materials, the same operation as the Example 16 gave the title compound.
Yield: 80.6%.
¹H-NMR(DMSO-d₆): δ 2.36(3H, s), 7.06(1H, d, J=8.7Hz), 7.49(1H, dd, J=8.7, 2.4Hz), 7.71(1H, dd, J=8.4, 1.8Hz), 7.77(1H, s), 7.95(1H, d, J=3.0Hz), 8.40(1H, d, J=8.4Hz), 10.76(1H, s), 12.31(1H, brs).

### Example 451: Preparation of the compound of Compound No. 451.

Using 5-chlorosalicylic acid and 2,3-dichloroaniline as the raw materials, the same operation as the Example 16 gave the title compound.
Yield: 37.1%.
¹H-NMR(DMSO-d₆): δ7.08(1H, d, J=9.0Hz), 7.40-7.48(2H, m), 7.52(1H, dd, J=9.0, 2.7Hz), 7.98(1H, d, J=2.7Hz), 8.40(1H, dd, J=7.2, 2.4Hz), 11.00(1H, s), 12.32(1H, s).

### Example 452: Preparation of the compound of Compound No. 452.

Using 5-chlorosalicylic acid and 2-chloroaniline as the raw materials, the same operation as the Example 16 gave the title compound.
Yield: 67.3%.
¹H-NMR(DMSO-d₆): δ 7.08(1H, d, J=8.7Hz), 7.20(1H, td, J=8.1, 1.8Hz), 7.40(1H, td, J=8.4, 1.8Hz), 7.52(1H, dd, J=8.7, 2.7Hz), 7.57(1H, dd, J=8.4, 1.8Hz), 8.00(1H, d, J=2.7Hz), 8.40(1H, dd, J=8.4, 1.8Hz), 10.89(1H, s), 12.27(1H, s).

### Example 453: Preparation of the compound of Compound No. 453.

Using 5-chlorosalicylic acid and 4-isopropyl-3-methylaniline as the raw materials, the same operation as the Example 16 gave the title compound.
Yield: 21.6%.
¹H-NMR(CDCl₃): δ 1.23(6H, d, J=6.9Hz), 2.36(3H, s), 3.12(1H, m), 6.89(1H, d, J=9.0Hz), 7.15-7.40(5H, m), 7.48(1H, d, J=2.1Hz), 7.83(1H, brs).

### Example 454: Preparation of the compound of Compound No. 454.

Using 5-chlorosalicylic acid and 2-amino-5-[(1,1-dimethyl)propyl]phenol as the raw materials, the same operation as the Example 16 gave the title compound.
Yield: 24.9%.
¹H-NMR(CDCl₃): δ0.69(3H, t, J=7.5Hz), 1.28(6H, s), 1.63(2H, q, J=7.5Hz), 6.98(1H, d, J=8.7Hz), 7.01(1H, d, J=9.0Hz), 7.06(1H, s), 7.15(1H, dd, =8.4, 2.4Hz), 7.35(1H, d, J=2.1Hz), 7.42(IH, dd, J=8.7, 2.4Hz), 7.56(1H, d, J=2.4Hz), 8.26(1H, s), 11.44(1H, s).

### Example 455: Preparation of the compound of Compound No. 455.

Using 5-chlorosalicylic acid and 2-methylaniline as the raw materials, the same operation as the Example 16 gave the title compound.
Yield: 64.7%.
¹H-NMR(DMSO-d₆): δ2.28(3H, s), 7.05(1H, d, J=8.7Hz), 7.13(1H, td, J=7.5, 1.5Hz), 7.22-7.30(2H, m), 7.50(1H, dd, J=9.0, 2.7Hz), 7.83(1H, d, J=7.8Hz), 8.03(1H, d, J=3.0Hz), 10.32(1H, s), 12.22(1H, s).

### Example 456: Preparation of the compound of Compound No. 456.

Using 5-chlorosalicylic acid and 4-butylaniline as the raw materials, the same operation as the Example 16 gave the title compound.
Yield: 82.1%.
¹H-NMR(DMSO-d₆): δ 0.90(3H, t, J=7.2Hz), 1.24-1.36(2H, m), 1.50-1.60(2H, m), 2.56(2H, t, J=7.2Hz), 7.01(1H, d, J=8.7Hz), 7.19(2H, d, J=8.7Hz), 7.47(1H, dd, J=8.7, 2.4Hz), 7.59(2H, d, J=8.4Hz), 7.98(1H, d, J=2.7Hz), 10.36(1H, s), 11.94(1H, s).

### Example 457: Preparation of the compound of Compound No. 457.

Using 5-chlorosalicylic acid and 2-amino-6-chlorobenzonitrile as the raw materials, the same operation as the Example 16 gave the title compound.
Yield: 12.7%.
¹H-NMR(DMSO-d₆): δ 7.09(1H, d, J=8.7Hz), 7.52(1H, d, J=8.1Hz), 7.53(1H, dd, J=9.0, 3.0Hz), 7.76(1H, t, J=8.7Hz), 7.95(1H, d, J=3.0Hz), 8.34(1H, d, J=8.4Hz), 11.17(1H, s), 12.39(1H, s).

### Example 458: Preparation of the compound of Compound No. 458.

Using 5-chlorosalicylic acid and 2-amino-5-methylbenzonitrile as the raw materials, the same operation as the Example 16 gave the title compound.
Yield: 9.0%.
¹H-NMR(CDCl₃): δ 2.48(3H, s), 7.01(1H, d, J=9.0Hz), 7.10(1H, dd, J=8.0, 0.9Hz), 7.44(1H, d, J=9.0, 2.4Hz), 7.56(1H, d, J=8.1Hz), 7.62(1H, d, J=2.4Hz), 8.22(1H, s), 8.54(1H, brs), 11.25(1H, brs).

### Example 459: Preparation of the compound of Compound No. 459.

Using 5-chlorosalicylic acid and 4-benzyloxyaniline as the raw materials, the same operation as the Example 16 gave the title compound.
Yield: 26.8%.
¹H-NMR(DMSO-d₆): δ5.11(2H, s), 6.99-7.05(3H, m), 7.33-7.49(6H, m), 7.60(2H, d, J=9.0Hz), 7.99(1H, d, J=2.7Hz), 10.33(1H, s), 12.02(1H, s).

### Example 460: Preparation of the compound of Compound No. 460.

Using 5-chlorosalicylic acid and 4-amino-2,2-difluorobenzo[1,3]dioxole as the raw materials, the same operation as the Example 16 gave the title compound.
Yield: 66.9%.
¹H-NMR(DMSO-d₆): δ7.05(1H, d, J=8.8Hz), 7.31-7.32(2H, m), 7.51(1H, dd, J=8.8, 2.8Hz), 7.70(1H, dd, J=5.6, 3.8Hz), 7.96(1H, d, J=2.8Hz), 10.59(1H, s), 12.05(1H, brs).

### Example 461: Preparation of the compound of Compound No. 461.

Using 5-chlorosalicylic acid and 5-amino-2,2,3,3-tetrafluoro-2,3-dihydrobenzo[1,4]dioxene as the raw materials, the same operation as the Example 16 gave the title compound.
Yield: 67.9%.
¹H-NMR(CDCl₃): δ6.99-7.03(2H, m), 7.21-7.27(2H, m), 7.45(1H, dd, J=8.9, 2.5Hz), 7.52(1H, d, J=2.5Hz), 8.13(1H, s), 11.44(1H, s).

### Example 462: Preparation of the compound of Compound No. 462.

Using 5-chlorosalicylic acid and 3-chloro-4-(trifluoromethyl)sulfanylaniline as the raw materials, the same operation as the Example 16 gave the title compound.
Yield: 52.3%.
¹H-NMR(DMSO-d₆): δ 7.03(1H, d, J=8.8Hz), 7.47(1H, dd, J=2.9, 8.8Hz), 7.80(1H, dd, J=2.6, 8.8Hz), 7.82(1H, d, J=2.6Hz), 7.88(1H, d, J=8.8Hz), 8.20(1H, d, J=2.2Hz), 10.70(1H, s), 11.43(1H, s).

### Example 463: Preparation of the compound of Compound No. 463.

Using 5-chlorosalicylic acid and 2-nitro-4-(trifluoromethoxy)aniline as the raw materials, the same operation as the Example 16 gave the title compound.
Yield: 68.4%.
¹H-NMR(DMSO-d₆): δ7.07(1H, d, J=8.8Hz), 7.52(1H, dd, J=2.6, 8.8Hz), 7.85-7.89(1H, m), 7.93(1H, d, J=2.6Hz), 8.17(1H, d, J=2.9Hz), 8.67(1H, d, J=9.5Hz), 11.92(1H, s), 12.14(1H, s).

### Example 464: Preparation of the compound of Compound No. 464.

Using 5-chlorosalicylic acid and 5-amino-2,2-difluorobenzo[1,3]dioxole as the raw materials, the same operation as the Example 16 gave the title compound.
Yield: 75.8%.
¹H-NMR(DMSO-d₆): δ 7.02(1H, d, J=8.8Hz), 7.42-7.43(2H, m), 7.48(1H, dd, J=8.8, 2.5Hz), 7.90(1H, d, J=2.5Hz), 10.54(1H, s), 11.69(1H, s).

### Example 465: Preparation of the compound of Compound No. 465.

Using 5-chlorosalicylic acid and 3-benzylaniline as the raw materials, the same operation as the Example 16 gave the title compound.
Yield: 66.4%.
¹H-NMR(CDCl₃): δ 3.99(2H, s), 6.97(1H, d, J=9.1Hz), 7.06(1H, d, J=7.4Hz), 7.18-7.48(8H, m), 7.37(1H, dd, J=9.1, 2.5Hz), 7.45(1H, d, J=2.5Hz), 7.80(1H, brs), 11.88(1H, s).

### Example 466: Preparation of the compound of Compound No. 466.

Using 5-chlorosalicylic acid and 2-nitro-4-(trifluoromethoxy)aniline as the raw materials, the same operation as the Example 16 gave the title compound.
Yield: 40.9%.
¹H-NMR(DMSO-d₆): δ2.33(3H, s), 7.05(1H, d, J=8.8Hz), 7.25(1H, dd, J=1.8, 8.8Hz), 7.33(1H, d, J=1.8Hz), 7.49(1H, dd, J=2.9, 8.8Hz), 7.97-8.00(2H, m), 10.37(1H, s), 12.15(1H, s).

### Example 467: Preparation of the compound of Compound No. 467.

Using 5-chlorosalicylic acid and 2,3,5-trifluoroaniline as the raw materials, the same operation as the Example 16 gave the title compound.
Yield: 54.2%.
¹H-NMR(DMSO-d₆): δ 7.06(1H, d, J=8.8Hz), 7.28-7.37(1H, m), 7.51(1H, dd, J=2.6, 8.8Hz), 7.92(1H, d, J=2.6Hz), 7.98-8.04(1H, m), 10.93(1H, s), 12.27(1H, br.s)

### Example 468: Preparation of the compound of Compound No. 468.

Using 5-chlorosalicylic acid and 4'-aminobenzo-15-crown-5 as the raw materials, the same operation as the Example 16 gave the title compound.
Yield: 45.1%.
¹H-NMR(CDCl₃): δ 3.74-3.77(8H, m), 3.90-3.92(4H, m), 4.10-4.15(4H, m), 6.83(1H, d, J=8.5Hz), 6.96-6.99(2H, m), 7.24(1H, d, J=2.5Hz), 7.36(1H, dd, J=2.5, 8.8Hz), 7.53(1H, s), 8.06(1H, br.s), 11.92(1H, s).

### Example 469: Preparation of the compound of Compound No. 469.

Using 5-chlorosalicylic acid and 4-bromo-2-fluoroaniline as the raw materials, the same operation as the Example 16 gave the title compound.
Yield: 45.1%.
¹H-NMR(DMSO-d₆): δ 7.05(1H, d, J=8.8Hz), 7.43-7.53(2H, m), 7.64-7.71(1H, m), 7.94(1H, d, J=1.5Hz), 8.20(1H, dd, J=8.4, 8.8Hz), 10.70(1H, s), 12.16(1H, s).

### Example 470: Preparation of the compound of Compound No. 470.

Using 5-chlorosalicylic acid and 2,4-bis(methanesulfonyl)aniline as the raw materials, the same operation as the Example 16 gave the title compound.
Yield: 7.2%.
¹H-NMR(CDCl₃): δ3.13(3H, s), 3.21(3H, s), 7.04(1H, d, J=8.9Hz), 7.48(1H, dd, J=2.2, 8.9Hz), 7.62(1H, d, J=2.2Hz), 8.24(1H, dd, J=2.4, 9.0Hz), 8.56(1H, d, J=2.4Hz), 8.91(1H, d, J=8.9Hz), 10.96(1H, s), 11.57(1H, s).

### Example 471: Preparation of the compound of Compound No. 471.

A mixture of 5-chlorosalicylic acid(87mg, 0.5mmol), 2,2-bis(3-amino-4-methylphenyl)-1,1,1,3,3,3-hexafluoropropane(363mg, lmmol), phosphorus trichloride(44 µL, 0.5mmol) and toluene(4mL) was refluxed for 4 hours. After the reaction mixture was cooled to room temperature, it was purified by column chromatography on silica gel(n-hexane:ethyl acetate=5:1) to give the white title compound(16mg, 4.9%). (The compound of Compound No. 529 described in the following Example 529 was obtained as a by-product.)
¹H-NMR(DMSO-d₆): δ 2.34(6H, s), 7.04(4H, d, J=8.8Hz), 7.39(2H, d, J=8.4Hz), 7.48(2H, dd, J=2.9, 8.8Hz), 7.96(2H, d, J=2.9Hz), 8.19(2H, s), 10.44(2H, s), 12.17(2H, s).

### Example 472: Preparation of the compound of Compound No. 472.

Using 5-chlorosalicylic acid and 6-amino-2,2,3,3-tetrafluoro-2,3-dihydrobenzo[1,4]dioxene as the raw materials, the same operation as the Example 16 gave the title compound.
Yield: 10.1%.
¹H-NMR(DMSO-d₆): δ 7.03(1H, d, J=8.8Hz), 7.48(1H, dd, J=9.0, 2.7Hz), 7.50(1H, d, J=9.0Hz), 7.59(1H, dd, J=8.8, 2.2Hz), 7.86(1H, d, J=2.7Hz), 7.92(1H, d, J=2.2Hz), 10.59(1H, s), 11.55(1H, s).

### Example 473: Preparation of the compound of Compound No. 473.

Using 5-chlorosalicylic acid and 2-amino-5-chlorobenzophenone as the raw materials, the same operation as the Example 16 gave the title compound.
Yield: 27.6%.
¹H-NMR(DMSO-d₆): δ 6.96(1H, d, J=8.7Hz), 7.43(1H, dd, J=8.7, 3.0Hz), 7.49-7.56(3H, m), 7.64-7.75(5H, m), 8.21(1H, d, J=9.3Hz), 11.21(1H, s), 11.83(1H, s).

### Example 474: Preparation of the compound of Compound No. 474.

Using 5-chlorosalicylic acid and 2-bromo-4-fluoroaniline as the raw materials, the same operation as the Example 16 gave the title compound.
Yield: 77.1%.
¹H-NMR(DMSO-d₆): δ7.07(1H, d, J=9.0Hz), 7.31-7.38(1H, m), 7.51(1H, dd, J=9.0, 3.0Hz), 7.72(1H, d, J=8.1, 3.0Hz), 8.00(1H, d, J=3.0Hz), 8.23(1H, dd, J=9.3, 5.4Hz), 10.70(1H, s), 12.24(1H, s).

### Example 475: Preparation of the compound of Compound No. 475.

Using 5-chlorosalicylic acid and 4-hexyloxyaniline as the raw materials, the same operation as the Example 16 gave the title compound.
Yield: 74.8%.
¹H-NMR(DMSO-d₆): δ 0.88(3H, t, J=6.6Hz), 1.28-1.46(6H, m), 2.49-2.52(2H, m), 3.95(2H, t, J=6.6Hz), 6.91-6.96(2H, m), 7.00(1H, d, J=8.8Hz), 7.46(1H, dd, J=8.8, 2.9Hz), 7.55-7.61(2H, m), 8.00(1H, d, J=2.9Hz), 10.31(1H, s), 12.03(1H, s).

### Example 476: Preparation of the compound of Compound No. 476.

Using 5-chlorosalicylic acid and 2,2-bis(3-aminophenyl)-1,1,1,3,3,3-hexafluoropropane as the raw materials, the same operation as the Example 16 gave the title compound.
Yield: 64.5%.
¹H-NMR(DMSO-d₆): δ 6.99(2H, d, J=8.8Hz), 7.11(2H, d, J=8.0Hz), 7.45(2H, dd, J=8.8, 2.6Hz), 7.50(2H, t, J=8.4Hz), 7.86(2H, d, J=2, 6Hz), 7.88-7.91(4H, m), 10.53(2H, s), 11.56(2H, s).

### Example 477: Preparation of the compound of Compound No. 477.

Using 5-chlorosalicylic acid and 2,4,5-trichloroaniline as the raw materials, the same operation as the Example 16 gave the title compound.
Yield: 38.9%.
¹H-NMR(CDCl₃): δ7.02(1H, d, J=8.6Hz), 7.46(1H, d, J=8.6Hz), 7.49(1H, s), 7.57(1H, s), 8.41(1H, br.s), 8.63(1H, s), 11.42(1H, s).

### Example 478: Preparation of the compound of Compound No. 478.

Using 5-chlorosalicylic acid and 3-isopropylaniline as the raw materials, the same operation as the Example 16 gave the title compound.
Yield: 55.3%.
¹H-NMR(DMSO-d₆): δ 1.22(6H, d, 6.9Hz), 2.76-2.94(1H, m), 7.01(1H, d, J=8.6Hz), 7.04(1H, d, J=7.9Hz), 7.29(1H, t, J=7.9Hz), 7.47(1H, dd, J=8.6, 2.6Hz), 7.54(1H, d, J=7.9Hz), 7.57(1H, s), 7.98(1H, d, J=2.6Hz), 10.37(1H, s), 11.90(1H, brs).

### Example 479: Preparation of the compound of Compound No. 479.

Using 5-chlorosalicylic acid and 4-aminobenzonitrile as the raw materials, the same operation as the Example 16 gave the title compound.
Yield: 45.6%.
¹H-NMR(DMSO-d₆): δ 7.03(1H, d, J=8.6Hz), 7.47(1H, dd, J=8.6, 2.6Hz), 7.83(1H, d, J=2.6Hz), 7.84(2H, d, J=8.9Hz), 7.92(2H, d, J=8.9Hz), 10.71(1H, s), 11.59(1H, brs).

### Example 480: Preparation of the compound of Compound No. 480.

Using 5-chlorosalicylic acid and 3-aminobenzonitrile as the raw materials, the same operation as the Example 16 gave the title compound.
Yield: 97.1%.
¹H-NMR(DMSO-d₆): δ7.03(1H, d, J=8.7Hz), 7.48(1H, dd, J=9.0, 2.7Hz), 7.56-7.63(2H, m), 7.88(1H, d, J=2.7Hz), 7.95-8.02(1H, m), 8.20-8.21(1H, m), 10.62(1H, s), 11.57(1H, s).

### Example 481: Preparation of the compound of Compound No. 481.

Using 5-chlorosalicylic acid and 3,4-dimethoxyaniline as the raw materials, the same operation as the Example 16 gave the title compound.
Yield: 73.3%.
¹H-NMR(DMSO-d₆): δ 3.75(3H, s), 3.76(3H, s), 6.95(1H, d, J=8.7Hz), 7.01(1H, d, J=9.0Hz), 7.24(1H, dd, J=8.7, 2.7Hz), 7.38(1H, d, J=2.1Hz), 7.47(1H, dd, J=8.7, 2.7Hz), 8.00(1H, d, J=2.4Hz), 10.30(1H, s), 12.01(1H, s).

### Example 482: Preparation of the compound of Compound No. 482.

Using 5-chlorosalicylic acid and 4-aminophenylacetic acid ethyl ester as the raw materials, the same operation as the Example 16 gave the title compound.
Yield: 66.1%.
¹H-NMR(DMSO-d₆): δ 1.19(3H, t, J=7.5Hz), 3.64(2H, s), 4.08(2H, q, J=7.2Hz), 7.01(1H, d, J=8.7Hz), 7.26(2H, d, J=8.7Hz), 7.47(1H, dd, J=8.7, 3.0Hz), 7.64(1H, d, J=8.4Hz), 7.96(1H, d, J=2.4Hz), 10.40(1H, s), 11.87(1H, s).

### Example 483: Preparation of the compound of Compound No. 483.

Using 5-chlorosalicylic acid and 3-[(trifluoromethyl)sulfanyl]aniline as the raw materials, the same operation as the Example 16 gave the title compound.
Yield: 67.1%.
¹H-NMR(CDCl₃): δ 7.01(1H, d, J=8.9Hz), 7.42(1H, dd, J=8.9, 2.3Hz), 7.47-7.53(2H, m), 7.51(1H, d, J=2.3Hz), 7.76(1H, dt, J=7.6Hz, 2.0Hz), 7.88(1H, brs), 7.92(1H, s), 11.64(1H, s).

### Example 484: Preparation of the compound of Compound No. 484.

Using 5-chlorosalicylic acid and 4-[(trifluoromethyl)sulfanyl]aniline as the raw materials, the same operation as the Example 16 gave the title compound.
Yield: 63.2%.
¹H-NMR(CDCl₃): δ 7.01(1H, d, J=8.9Hz), 7.43(1H, dd, J=8.9, 2.3Hz), 7.50(1H, d, J=2.3Hz), 7.70(4H, s), 7.90(1H, brs), 11.60(1H, s).

### Example 485: Preparation of the compound of Compound No. 485.

Using 5-chlorosalicylic acid and 4-(trifluoromethanesulfonyl)aniline as the raw materials, the same operation as the Example 16 gave the title compound.
Yield: 38.7%.
¹H-NMR(DMSO-d₆): δ 7.04(1H, d, J=8.6Hz), 7.49(1H, dd, J=8.6, 2.6Hz), 7.80(1H, d, J=2.6Hz), 8.12(2H, d, J=9.4Hz), 8.17(2H, d, J=9.4Hz), 8.16(1H, s), 10.95(1H, s), 11.37(1H, brs).

### Example 486: Preparation of the compound of Compound No. 486.

Using 5-chlorosalicylic acid and 3,4-difluoroaniline as the raw materials, the same operation as the Example 16 gave the title compound.
Yield: 75.4%.
¹H-NMR(DMSO-d₆): δ7.02(1H, d, J=8.9Hz), 7.39-7.51(3H, m), 7.85-7.93(2H, m), 10.51, (1H, s), 11.60(1H, s).

### Example 487: Preparation of the compound of Compound No. 487.

Using 5-chlorosalicylic acid and 3-ethynylaniline as the raw materials, the same operation as the Example 16 gave the title compound.
Yield: 35.8%.
¹H-NMR(DMSO-d₆): δ4.22(1H, s), 7.02(1H, d, J=8.6Hz), 7.25(1H, d, J=7.6Hz), 7.39(1H, t, J=7.6Hz), 7.47(1H, dd, J=8.6, 2.6Hz), 7.70(1H, d, J=7.6Hz), 7.89(1H, s), 7.91(1H, d, J=2.6Hz), 10.46(1H, s), 11.69(1H, brs).

### Example 488: Preparation of the compound of Compound No. 488.

Using 5-chlorosalicylic acid and 4-(sec-butyl)aniline as the raw materials, the same operation as the Example 16 gave the title compound.
Yield: 40.1%.
¹H-NMR(DMSO-d₆): δ 0.77(3H, t, 7.4Hz), 1.19(3H, d, 6.9Hz), 1.50-1.61(2H, m), 2.52-2.62(1H, m), 7.01(1H, d, J=8.9Hz), 7.20(2H, d, J=8.6Hz), 7.47(1H, dd, J=8.9, 2.6Hz), 7.60(2H, d, J=8.6Hz), 7.98(1H, d, J=2.6Hz), 10.36(1H, s), 11.94(1H, brs).

### Example 489: Preparation of the compound of Compound No. 489.

Using 5-chlorosalicylic acid and 3-chloro-4-methoxyaniline as the raw materials, the same operation as the Example 16 gave the title compound.
Yield: 75.7%.
¹H-NMR(CDCl₃): δ 6.98(2H, t, J=9.2Hz), 7.38-7.44(2H, m), 7.47(1H, d, J=2.6Hz), 7.66(1H, d, J=2.6Hz), 7.73(1H, br.s), 11.81(1H, s).

### Example 490: Preparation of the compound of Compound No. 490.

Using 5-chlorosalicylic acid and 3-aminobenzophenone as the raw materials, the same operation as the Example 16 gave the title compound.
Yield: 34.3%.
¹H-NMR(DMSO-d₆): δ7.02(1H, d, J=8.6Hz), 7.48(1H, dd, J=9.1, 2.6Hz), 7.52-7.62(4H, m), 7.68-7.79(3H, m), 7.93(1H, d, J=2.6Hz), 8.02(1H, d, J=7.9Hz), 8.16(1H, s), 10.60(1H, s), 11.68(1H, brs).

### Example 491: Preparation of the compound of Compound No. 491.

Using 5-chlorosalicylic acid and 3-methoxyaniline as the raw materials, the same operation as the Example 16 gave the title compound.
Yield: 23.5%.
¹H-NMR(DMSO-d₆): δ3.76(3H, s), 6.69-6.75(1H, m), 7.01(1H, d, J=8.6Hz), 7.25-7.28(2H, m), 7.39(1H, s), 7.47(1H, dd, J=8.6, 2.6Hz), 7.94(1H, d, J=2.6Hz), 10.39(1H, s), 11.81(1H, brs).

### Example 492: Preparation of the compound of Compound No. 492.

Using 5-chlorosalicylic acid and 4'-aminoacetanilide as the raw materials, the same operation as the Example 16 gave the title compound.
Yield: 36.2%.
¹H-NMR(DMSO-d₆): δ2.50(3H, s), 7.01(1H, d, J=8.6Hz), 7.47(1H, dd, J=8.6, 2.6Hz), 7.57(2H, d, J=9.1Hz), 7.61(2H, d, J=9.1Hz), 7.98(1H, d, J=2.6Hz), 9.95(1H, s), 10.38(1H, s), 11.99(1H, brs).

### Example 493: Preparation of the compound of Compound No. 493.

Using 5-chlorosalicylic acid and sulfanilamide as the raw materials, the same operation as the Example 16 gave the title compound.
Yield: 25.7%.
¹H-NMR(DMSO-d₆): δ7.03(1H, d, J=8.9Hz), 7.31(2H, s), 7.47(1H, dd, J=8.9, 2.3Hz), 7.81(2H, d, J=8.9Hz), 7.89(2H, d, J=8.9Hz), 7.89(1H, d, J=2.3Hz), 10.70(1H, s), 11.55(1H, brs).

### Example 494: Preparation of the compound of Compound No. 494.

Using 5-chlorosalicylic acid and 2-(4-aminophenyl)-1,1,1,3,3,3-hexafluoro-2-propanol as the raw materials, the same operation as the Example 16 gave the title compound. (The compound was obtained by separation from the mixture with the compound of Compound No. 498 described in the following Example 498.)
Yield: 11.7%.
¹H-NMR(DMSO-d₆): δ 7.02(1H, d, J=8.6Hz), 7.47(1H, dd, J=8.6, 2.6Hz), 7.68(2H, d, J=8.7Hz), 7.85(2H, d, J=8.7Hz), 7.91(1H, d, J=2.6Hz), 8.69(1H, s), 10.62(1H, s).

### Example 495: Preparation of the compound of Compound No. 495.

Using 5-chlorosalicylic acid and 2-chloro-4-nitroaniline as the raw materials, the same operation as the Example 16 gave the title compound.
Yield: 39.6%.
¹H-NMR(CDCl₃): δ 7.04(1H, d, J=8.9Hz), 7.47(1H, dd, J=2.3, 8.9Hz), 7.54(1H, d, J=2.3Hz), 8.25(1H, dd, J=2.6, 8.9Hz), 8.39(1H, d, J=2.3Hz), 8.73(1H, d, J=9.2Hz), 8.76(1H, br.s), 11.22(1H, s).

### Example 496: Preparation of the compound of Compound No. 496.

Using 5-chlorosalicylic acid and 2,4-difluoroaniline as the raw materials, the same operation as the Example 16 gave the title compound.
Yield: 67.8%.
¹H-NMR(DMSO-d₆): δ7.05(1H, dd, J=1.7, 8.9Hz), 7.15(1H, dt, J=1.7, 9.2Hz), 7.41(1H, ddd, J=2.3, 8.9, 9.2Hz), 7.51(1H, dt, J=2.3, 8.9Hz), 7.98(1H, d, J=2.3Hz), 8.11(1H, dd, J=8.9, 15.1Hz), 10.59(1H, s), 12.13(1H, s).

### Example 497: Preparation of the compound of Compound No. 497.

Using 5-chlorosalicylic acid and 4-(difluoromethoxy)aniline as the raw materials, the same operation as the Example 16 gave the title compound.
Yield: 85.9%.
¹H-NMR(DMSO-d₆): δ 7.01(1H, d, J=8.6Hz), 7.19(1H, t, J=74.2Hz), 7.20(2H, d, J=8.6Hz), 7.47(1H, dd, J=8.6, 2.6Hz), 7.74(2H, d, J=8.9Hz), 7.94(1H, d, J=2.6Hz), 10.47(1H, s), 11.80(1H, brs).

### Example 498: Preparation of the compound of Compound No. 498.

This compound was obtained by separation from the mixture with the compound of Compound No. 494 described in the aforementioned Example 494.
Yield: 11.6%.
¹H-NMR(DMSO-d₆): δ7.02(1H, d, J=8.6Hz), 7.46(1H, dd, J=8.6, 2.3Hz), 7.83(2H, d, J=8.1Hz), 7.88(1H, d, J=2.3Hz), 7.95(2H, d, J=8.1Hz), 10.71(1H, s).

### Example 499: Preparation of the compound of Compound No. 499.

Using 5-chlorosalicylic acid and 3-(methylsulfanyl)aniline as the raw materials, the same operation as the Example 16 gave the title compound.
Yield: 67.2%.
¹H-NMR(DMSO-d₆): δ 2.49(3H, s), 7.00-7.05(1H, m), 7.01(1H, d, J=8.9Hz), 7.31(1H, t, J=7.9Hz), 7.46(1H, dd, J=8.9, 2.6Hz), 7.44-7.49(1H, m), 7.68(1H, d, J=1.7Hz), 7.93(1H, d, J=2.6Hz), 10.47(1H, s).

### Example 500: Preparation of the compound of Compound No. 500.

Using 5-chlorosalicylic acid and 4-methanesulfonylaniline as the raw materials, the same operation as the Example 16 gave the title compound.
Yield: 28.6%.
¹H-NMR(DMSO-d₆): δ 3.20(3H, s), 7.03(1H, d, J=8.3Hz), 7.48(1H, dd, J=8.3, 2.6Hz), 7.87(1H, d, J=2.6Hz), 7.92(2H, d, J=8.9Hz), 7.98(2H, d, J=8.9Hz), 10.75(1H, s), 11.45(1H, brs).

### Example 501: Preparation of the compound of Compound No. 501.

Using 5-chlorosalicylic acid and 2-amino-4-methylbenzophenone as the raw materials, the same operation as the Example 16 gave the title compound.
Yield: 8.7%.
¹H-NMR(CDCl₃): δ 2.50(3H, s), 6.98(1H, d, J=8.3Hz), 6.99(1H, d, J=7.3Hz), 7.39(1H, dd, J=2.0, 8.6Hz), 7.48-7.64(4H, m), 7.72(2H, d, J=7.6Hz), 7.83(1H, d, J=2.3Hz), 8.57(1H, s), 12.18(1H, s), 12.34(1H, br.s).

### Example 502: Preparation of the compound of Compound No. 502.

Using 5-chlorosalicylic acid and 3-amino-N-butylbenzenesulfonamide as the raw materials, the same operation as the Example 16 gave the title compound.
Yield: 46.7%.
¹H-NMR(DMSO-d₆): δ 0.80(3H, t, J=7.3Hz), 1.17-1.41(4H, m), 2.73-2.80(2H, m), 7.03(1H, d, J=8.9Hz), 7.48(1H, dd, J=8.9, 2.0Hz), 7.53-7.64(2H, m), 7.87-7.92(1H, m), 7.92(1H, d, J=2.0Hz), 8.27(1H, s), 10.62(1H, s), 11.63(1H, s).

### Example 503: Preparation of the compound of Compound No. 503.

Using 5-chlorosalicylic acid and 3-(benzyloxy)aniline as the raw materials, the same operation as the Example 16 gave the title compound.
Yield: 68.5%.
¹H-NMR(DMSO-d₆): δ 5.11(2H, s), 6.79-6.83(1H, m), 7.01(1H, d, J=8.9Hz), 7.27-7.49(9H, m), 7.93(1H, d, J=3.0Hz), 10.40(1H, s), 11.79(1H, brs).

### Example 504: Preparation of the compound of Compound No. 504.

Using 5-chlorosalicylic acid and N-(4-aminophenyl)-4-methylbenzenesulfonamide as the raw materials, the same operation as the Example 16 gave the title compound.
Yield: 40.6%.
¹H-NMR(DMSO-d₆): δ 2.33(3H, s), 6.99(1H, d, J=8.6Hz), 7.07(2H, d, J=8.6Hz), 7.34(2H, d, J=8.3Hz), 7.45(1H, dd, J=8.6, 2.1Hz), 7.53(2H, d, J=8.6Hz), 7.63(2H, d, J=8.3Hz), 7.90(1H, d, J=2.1Hz), 10.14(1H, s), 10.33(1H, s), 11.81(1H, brs).

### Example 505: Preparation of the compound of Compound No. 505.

Using 5-chlorosalicylic acid and 4-(morpholino)aniline as the raw materials, the same operation as the Example 16 gave the title compound.
Yield: 29.8%.
¹H-NMR(DMSO-d₆): δ 3.09(4H, t, J=4.6Hz), 3.74(4H, t, J=4.6Hz), 6.94-7.01(3H, m), 7.46(1H, dd, J=8.9, 2.6Hz), 7.55(2H, d, J=8.9Hz), 8.01(1H, d, J=2.6Hz), 10.29(1H, s), 12.10(1H, brs).

### Example 506: Preparation of the compound of Compound No. 506.

Using 5-chlorosalicylic acid and 3-(tert-butyl)aniline as the raw materials, the same operation as the Example 16 gave the title compound.
Yield: 76.1%.
¹H-NMR(CDCl₃): δ1.35(9H, s), 6.99(1H, d, J=8.9Hz), 7.24-7.28(1H, m), 7.32-7.35(1H, m), 7.40(1H, dd, J=8.9, 2.3Hz), 7.46-7.50(2H, m), 7.51(1H, d, J=2.3Hz), 7.81(1H, brs), 11.94(1H, s).

### Example 507: Preparation of the compound of Compound No. 507.

Using 5-chlorosalicylic acid and 3-(5-methylfuran-2-yl)aniline as the raw materials, the same operation as the Example 16 gave the title compound.
Yield: 61.1%.
¹H-NMR(DMSO-d₆): δ 2.36(3H, s), 6.22-6.23(1H, m), 6.81(1H, d, J=3.0Hz), 7.02(1H, d, J=8.9Hz), 7.36-7.51(3H, m), 7.58-7.61(1H, m), 7.99-8.01(2H, m), 10.49(1H, s), 11.85(1H, brs).

### Example 508: Preparation of the compound of Compound No. 508.

Using 5-chlorosalicylic acid and 3-(1-hydroxyethyl)aniline as the raw materials, the same operation as the Example 16 gave the title compound.
Yield: 37.6%.
¹H-NMR(DMSO-d₆): δ 1.80(3H, d, J=6.6Hz), 5.33(1H, q, J=6.6Hz), 7.01(1H, d, J=8.9Hz), 7.25(1H, d, J=7.9Hz), 7.38(1H, t, J=7.9Hz), 7.47(1H, dd, J=8.9, 2.3Hz), 7.65(1H, d, J=7.9Hz), 7.85(1H, s), 7.96(1H, d, J=2.3Hz), 10.48(1H, s), 11.80(1H, brs).

### Example 509: Preparation of the compound of Compound No. 509.

Using 5-chlorosalicylic acid and 3-aminobenzenesulfonamide as the raw materials, the same operation as the Example 16 gave the title compound.
Yield: 18.7%.
¹H-NMR(DMSO-d₆): δ7.03(1H, d, J=8.9Hz), 7.41(2H, s), 7.48(1H, dd, J=8.9, 2.6Hz), 7.54-7.62(2H, m), 7.84-7.88(1H, m), 7.93(1H, d, J=2.6Hz), 8.30(1H, s), 10.64(1H, s), 11.68(1H, brs).

### Example 510: Preparation of the compound of Compound No. 510.

Using 5-chlorosalicylic acid and 3-(trifluoromethanesulfonyl)aniline as the raw materials, the same operation as the Example 16 gave the title compound.
Yield: 62.6%.
¹H-NMR(DMSO-d₆): δ 7.03(1H, d, J=8.6Hz), 7.48(1H, dd, J=8.6, 2.6Hz), 7.82-7.88(3H, m), 8.23-8.26(1H, m), 8.67(1H, s), 10.88(1H, s), 11.45(1H, brs).

### Example 511: Preparation of the compound of Compound No. 511.

Using 5-chlorosalicylic acid and 2-bromo-4-(trifluoromethoxy)aniline as the raw materials, the same operation as the Example 16 gave the title compound.
Yield: 17.1%.
¹H-NMR(CDCl₃): δ7.02(1H, d, J=8.9Hz), 7.26-7.31(1H, m), 7.44(1H, dd, J=8.9, 2.6Hz), 7.53(2H, d, J=2.6Hz), 8.41(1H, brs, ), 8.42(1H, d, J=8.9Hz), 11.57(1H, s).

### Example 512: Preparation of the compound of Compound No. 512.

Using 5-chlorosalicylic acid and 3,4-(dihexyloxy)aniline as the raw materials, the same operation as the Example 16 gave the title compound.
Yield: 60.5%.
¹H-NMR(CDCl₃): δ0.91(6H, t, J=6.3Hz), 1.34-1.61(12H, m), 1.76-1.89(4H, m), 3.97-4.04(4H, m), 6.88(1H, d, J=8.9Hz), 6.97-7.00(2H, m), 7.22(1H, d, J=2.6Hz), 7.38(1H, dd, J=8.9, 2.6Hz), 7.47(1H, d, J=2.6Hz), 7.73(1H, s), 11.97(1H, s).

### Example 513: Preparation of the compound of Compound No. 513.

Using 5-chlorosalicylic acid and 3,4-dichloroaniline as the raw materials, the same operation as the Example 16 gave the title compound.
Yield: 16.4%.
¹H-NMR(DMSO-d₆): δ 7.03(1H, d, J=8.7Hz), 77.47(1H, dd, J=8.7, 2.7Hz), 7.61-7.70(2H, m), 7.86(1H, d, J=2.7Hz), 8.11(1H, d, J=2.1Hz), 10.56(1H, s), 11.53(1H, s).

### Example 514: Preparation of the compound of Compound No. 514.

Using 5-chlorosalicylic acid and 3-hexyloxyaniline as the raw materials, the same operation as the Example 16 gave the title compound.
Yield: 88.2%.
¹H-NMR(DMSO-d₆): δ 0.89(3H, t, J=7.0Hz), 1.28-1.47(6H, m), 1.67-1.76(2H, m), 3.95(2H, t, J=6.6Hz), 6.69-6.73(1H, m), 7.01(1H, d, J=8.8Hz), 7.21-7.28(2H, m), 7.39-7.40(1H, m), 7.67(1H, dd, J=8.8, 2.6Hz), 7.94(1H, d, J=2.6Hz), 10.34(1H, s), 11.80(1H, s).

### Example 515: Preparation of the compound of Compound No. 515.

Using 5-chlorosalicylic acid and 5-ethoxy-4-fluoro-2-nitroaniline as the raw materials, the same operation as the Example 16 gave the title compound.
Yield: 20.2%.
¹H-NMR(DMSO-d₆): δ1.43(3H, t, J=7.0Hz), 4.27(2H, q, J=7.0Hz), 7.07(1H, d, J=8.8Hz), 7.52(1H, dd, J=8.8, 2.9Hz), 7.95(1H, d, J=2.9Hz), 8.15(1H, d, J=11.4Hz), 8.57(1H, d, J=8.4Hz), 12.16(1H, s), 12.26(1H, s).

### Example 516: Preparation of the compound of Compound No. 516.

Using 5-chlorosalicylic acid and 4-hydroxy-3-methyl-1-naphthylamine as the raw materials, the same operation as the Example 16 gave the title compound.
Yield: 5.9%.
¹H-NMR(DMSO-d₆): δ 2.38(3H, s), 7.03(1H, d, J=9.3Hz), 7.43(2H, s), 7.46(1H, d, J=2.4Hz), 7.50-7.54(2H, m), 7.67(1H, d, J=2.1Hz), 7.78(1H, dd, J=6.0, 2.7Hz), 8.03(1H, brs), 8.18(1H, dd, J=6.0, 3.6Hz), 11.98(1H, brs).

### Example 517: Preparation of the compound of Compound No. 517.

This compound is a known compound.
Reference which describes the preparation method: the pamphlet of International Publication WO99/65449.

### Example 518: Preparation of the compound of Compound No. 518.

This compound is a known compound.
Reference which describes the preparation method: the pamphlet of International Publication WO99/65449.

### Example 519: Preparation of the compound of Compound No. 519.

This compound is a known compound.
Reference which describes the preparation method: the pamphlet of International Publication WO99/65449.

### Example 520: Preparation of the compound of Compound No. 520.

This compound is a known compound.
Reference which describes the preparation method: the pamphlet of International Publication WO99/65449.

### Example 521: Preparation of the compound of Compound No. 521.

This compound is a known compound.
Reference which describes the preparation method: the pamphlet of International Publication WO99/65449.

### Example 522: Preparation of the compound of Compound No. 522.

This compound is a known compound.
Reference which describes the preparation method: the pamphlet of International Publication WO99/65449.

### Example 523: Preparation of the compound of Compound No. 523.

This compound is a known compound.
Reference which describes the preparation method: the pamphlet of International Publication WO99/65449.

### Example 524: Preparation of the compound of Compound No. 524.

Using 5-chlorosalicylic acid and 4-aminobiphenyl as the raw materials, the same operation as the Example 16 gave the title compound.
Yield: 52.4%.
¹H-NMR(DMSO-d₆): δ 7.03(1H, d, J=8.7Hz), 7.33-7.38(1H, m), 7.44-7.51(3H, m), 7.67-7.72(4H, m), 7.82(2H, d, J=8.7Hz), 7.98(1H, d, J=2.4Hz), 10.49(1H, s), 11.84(1H, s).

### Example 525: Preparation of the compound of Compound No. 525.

A mixture of 5-sulfosalicylic acid(218mg, 1mmol), 3,5-bis(trifluoromethyl)aniline(229mg, 1mmol), phosphorus trichloride(88 µ L, 1mmol) and o-xylene(5mL) was refluxed for 3 hours. After the reaction mixture was cooled to room temperature, it was purified by column chromatography on silica gel(n-hexane:ethyl acetate=3:1) to give the title compound(29mg, 9.2%) as a white solid.
¹H-NMR(DMSO-d₆): δ 7.15(1H, d, J=8.8Hz), 7.65(2H, s), 7.73(1H, s), 7.81(1H, s), 7.82(1H, dd, J=8.7, 2.5Hz), 8.23(1H, d, J=2.5Hz), 8.38(2H, s), 10.87(1H, s), 11.15(1H, brs).

### Example 526: Preparation of the compound of Compound No. 526.

Using 5-chlorosalicylic acid and 2,4-bis(trifluoromethyl)aniline as the raw materials, the same operation as the Example 16 gave the title compound.
Yield: 6.9%.
¹H-NMR(CDCl₃): δ 7.03(1H, dd, J=8.7, 0.6Hz), 7.43-7.48(2H, m), 7.91(1H, d, J=9.0Hz), 7.96(1H, s), 8.42(1H, s), 8.49(1H, d, J=8.7Hz), 11.26(1H, s).

### Example 527: Preparation of the compound of Compound No. 527.

Using 3-phenylsalicylic acid and 3,5-bis(trifluoromethyl)aniline as the raw materials, the same operation as the Example 16 gave the title compound.
Yield: 64.6%.
¹H-NMR(DMSO-d₆): δ 7.12(1H, t, J=8.1Hz), 7.37(1H, tt, J=7.5, 1.5Hz), 7.43-7.48(2H, m), 7.56-7.60(3H, m), 7.91(1H, s), 8.07, (1H, dd, J=8.1, 1.5Hz), 8.48(2H, s), 11.00(1H, s), 12.16(1H, s).

### Example 528: Preparation of the compound of Compound No. 528.

Using 4-fluorosalicylic acid and 3,5-bis(trifluoromethyl)aniline as the raw materials, the same operation as the Example 16 gave the title compound.
Yield: 65.7%.
¹H-NMR(DMSO-d₆): δ 6.81-6.90(2H, m), 7.84(1H, s, ), 7.93-7.98(1H, m, ), 8.45(2H, s, ), 10.78(1H, s), 11.81(1H, s, ).

### Example 529: Preparation of the compound of Compound No. 529.

This compound was obtained by separation from the mixture with the compound of Compound No. 471 described in the aforementioned Example 471.
Yield: 9.4%.
¹H-NMR(CD₃OD): δ2.16(3H, s), 2.34(3H, s), 6.69(1H, d, J=8.2Hz), 6.76(1H, brs)6.95(1H, d, J=8.8Hz), 7.02(1H, d, J=8.0Hz), 7.15(1H, d, J=8.2Hz), 7.29(1H, d, J=8.2Hz), 7.37(1H, dd, J=8.8, 2.6Hz), 7.97(1H, d, J=2.6Hz), 7.98(1H, s).

### Example 530: Preparation of the compound of Compound No. 530.

Using 5-chlorosalicylic acid and 4-amino-3-(trifluoromethoxy)benzonitrile as the raw materials, the same operation as the Example 16 gave the title compound.
Yield: 75.2%.
¹H-NMR(DMSO-d₆): δ 7.13(1H, d, J=8.8Hz), 7.54(1H, dd, J=8.8, 2.6Hz), 7.94(1H, dd, J=8.4, 1.6Hz), 7.95(1H, d, J=2.6Hz), 8.15(1H, t, J=1.5Hz), 8.75(1H, d, J=8.8Hz), 11.25(1H, s), 12.45(1H, s).

### Example 531: Preparation of the compound of Compound No. 531.

Using 5-chlorosalicylic acid and 4-[2-amino-4-(trifluromethyl)phenoxy]benzonitrile as the raw materials, the same operation as the Example 16 gave the title compound.
Yield: 11.6%.
¹H-NMR(CD₃OD): δ 6.88(1H, d, J=8.6Hz), 7.19(2H, d, J=8.9Hz), 7.24(1H, d, J=8.6Hz), 7.33(1H, dd, J=8.8, 2.8Hz), 7.46(1H, dd, J=8.9, 1.9Hz), 7.76(2H, d, J=8.9Hz), 7.98(1H, d, J=2.7Hz), 8.96(1H, s).

### Example 532: Preparation of the compound of Compound No. 532.

Using 5-chlorosalicylic acid and 3-amino-4-(4-methoxyphenoxy)benzotrifluoride as the raw materials, the same operation as the Example 16 gave the title compound.
Yield: 88.1%.
¹H-NMR(CDCl₃): δ3.85(3H, s) 6.81(1H, d, J=8.5Hz), 6.97-7.02(3H, m), 7.08(2H, d, J=8.8Hz), 7.30(1H, m), 7.40(1H, dd, J=8.8, 1.9Hz), 7.45(1H, d, J=2.2Hz), 8.70(1H, s), 8.78(1H, d, J=1.6Hz), 11.76(1H, s).

### Example 533: Preparation of the compound of Compound No. 533.

Using salicylic acid and 2,5-bis(trifluoromethyl)aniline as the raw materials, the same operation as the Example 16 gave the title compound.
Yield: 47.8%.
¹H-NMR(CD₃OD): δ 7.00-7.06(2H, m), 7.48(1H, dt, J=1.5, 7.5Hz), 7.74(1H, d, J=8.4Hz), 8.01-8.08(2H, m), 8.79(1H, s), 11.09(1H, s), 12.03(1H, s).

### Example 534: Preparation of the compound of Compound No. 534.

### (1) 2-Amino-4-(2,4-dichlorophenyl)thiazole.

Using 2',4'-dichloroacetophenone and thiourea as the raw materials, the same operation as the Example 395(1) gave the title compound.
Yield: 97.1%.
¹H-NMR(CDCl₃): δ5.01(2H, s), 7.09(1H, s), 7.28(1H, dd, J=8.4, 2.1Hz), 7.45(1H, d, J=2.1Hz), 7.82(1H, d, J=8.4Hz).

### (2) 5-Chloro-2-hydroxy-N-[4-(2,4-dichlorophenyl)thiazol-2-yl]benzamide(Compound No. 534).

Using 5-chlorosalicylic acid and 2-amino-4-(2,4-dichlorophenyl)thiazole as the raw materials, the same operation as the Example 16 gave the title compound.
Yield: 8.0%.
¹H-NMR(DMSO-d₆): δ 7.08(1H, d, J=8.7Hz), 7.50-7.55(2H, m), 7.72-7.76(2H, m), 7.91(1H, d, J=8.4Hz), 7.95(1H, d, J=2.4Hz), 11.87(1H, brs), 12.09(1H, brs).

### Example 535: Preparation of the compound of Compound No. 535.

Using 3-isopropylsalicylic acid and 3,5-bis(trifluoromethyl)aniline as the raw materials, the same operation as the Example 16 gave the title compound.
Yield: 99.2%.
¹H-NMR(CDCl₃): δ 1.26(6H, d, J=6.9Hz), 3.44(1H, Hept, J=6.9Hz), 6.92(1H, t, J=7.8Hz), 7.38(1H, dd, J=8.1, 1.2Hz), 7.44(1H, d, J=7.5Hz), 7.69(1H, s), 8.13(3H, s), 11.88(1H, s).

### Example 536: Preparation of the compound of Compound No. 536.

Bromine(14.4 µL, 0.28mmol) and iron powder(1.7mg, 0.03mmol) were added to a solution of N-[3,5-bis(trifluoromethyl)phenyl]-2-hydroxy-3-isopropylbenzamide (Compound No. 535; 100mg, 0.26mmol) in carbon tetrachloride(5mL) under argon atmosphere, and the mixture was stirred at room temperature for 2 hours. The reaction mixture was diluted with ethyl acetate. The ethyl acetate layer was washed with water and brine, and dried over anhydrous magnesium sulfate. The residue obtained by evaporation of the solvent under reduced pressure was crystallized from n-hexane/ethyl acetate to give the title compound(110mg, 91.5%) as a white solid.
¹H-NMR(CDCl₃): δ1.25(6H, d, J=6.9Hz), 3.39(1H, Hept, J=6.9Hz), 7.49-7.51(2H, m), 7.71(1H, brs), 8.11-8.14(3H, m), 11.81(1H, brs).

### Example 537: Preparation of the compound of Compound No. 537.

N-Bromosuccinimide(88.2mg, 0.50mmol) was added to a solution of N-[3,5-bis(trifluoromethyl)phenyl]-2-hydroxy-3-methylbenzamide(Compound No. 328; 150mg, 0.41mmol) in a mixed solvent of methanol/water(3:1; 5mL), and the mixture was stirred at room temperature for 10 minutes. The reaction mixture was diluted with ethyl acetate. The ethyl acetate layer was washed with 10% aqueous sodium thiosulfate, water and brine, and dried over anhydrous magnesium sulfate. The residue obtained by evaporation under reduced pressure was purified by column chromatography on silica gel(n-hexane:ethyl acetate=5:1) to give the title compound(167mg, 91.5%) as a white powder.
¹H-NMR(CDCl₃): δ 2.28(3H, s), 7.47(1H, s), 7.50(1H, d, J=2.4Hz), 7.71(1H, s), 8.08(1H, brs), 8.13(2H, s), 11.71(1H, s).

### Example 538: Preparation of the compound of Compound No. 538.

### (1) 1-(3-Nitrophenyl)-5-phenyl-3-(trifluoromethyl)pyrazole.

A mixture of 4,4,4-trifluoro-1-phenyl-1,3-butanedione(432.3mg, 2mmol), 3-nitrophenylhydrazine hydrochloride(379.2mg, 2mmol), concentrated hydrochloric acid(0.2mL) and ethanol(8mL) was reflued for 2 hours. After the reaction mixture was cooled to room temperature, it was poured into water and extracted with ethyl acetate. The ethyl acetate layer was washed with water and brine, and dried over anhydrous sodium sulfate. The residue obtained by evaporation under reduced pressure was purified by column chromatography on silica gel(n-hexane:ethyl acetate=4:1→3:1) to give the title compound(631.5mg, 94.7%) as a light yellowish white powder.
¹H-NMR(CDCl₃): δ 6.80(1H, s), 7.23-7.26(2H, m), 7.35-7.45(3H, m), 7.54(1H, t, J=8.4Hz), 7.63(1H, ddd, J=8.1, 1.8, 1.2Hz), 8.19-8.25(2H, m).

### (2) 1-(3-Aminophenyl)-5-phenyl-3-(trifluoromethyl)pyrazole.

Acetic acid(3mL) and ethanol(2mL) were added to 1-(3-nitrophenyl)-5-phenyl-3-(trifluoromethyl)pyrazole(0.59g, 1.77mmol) and 5% palladium on carbon(0.06g), and the mixture was hydrogenated at room temperature for 2 hours under hydrogen atmosphere. After the insoluble matter was filtered off, the residue obtained by evaporation under reduced pressure was purified by column chromatography on silica gel(n-hexane:ethyl acetate=2:1) to give the title compound(491.1mg, 91.4%) as a white solid.
¹H-NMR(CDCl₃): δ 3.78(2H, s), 6.54(1H, ddd, J=7.8, 1.8, 0.6Hz), 6.65(1H, ddd, J=8.4, 2.4, 0.9Hz), 6.73-6.75(2H, m), 7.07(1H, t, J=8.1Hz), 7.24-7.36(5H, m).

### (3) 5-Chloro-2-hydroxy-N-{3-[5-phenyl-3-(trifluoromethyl)pyrazol-1-yl]phenyl}benzamide(Compound No. 538).

Using 5-chlorosalicylic acid and 1-(3-aminophenyl)-5-phenyl-3-(trifluoromethyl)pyrazole as the raw materials, the same operation as the Example 16 gave the title compound.
Yield: 74.4%.
¹H-NMR(CDCl₃): δ 6.77(1H, s), 6.97-7.03(2H, m), 7.27-7.45(8H, m), 7.65(1H, ddd, J=8.4, 2.1, 0.9Hz), 7.74(1H, t, J=2.1Hz), 7.93(1H, s), 11.63(1H, s).

### Example 539: Preparation of the compound of Compound No. 539.

### (1) 5-(tert-Butyl)-1-(4-nitrophenyl)-3-(trifluoromethyl)pyrazole.

Using 1,1,1-trifluoro-5,5-dimethyl-2,4-hexanedione and 4-nitrophenylhydrazine hydrochloride as the raw materials, the same operation as the Example 538(1) gave the title compound.
Yield: 94.7%.
¹H-NMR(CDCl₃): δ1.23(9H, s), 6.51(1H, s), 7.62(2H, d, J=9.0Hz), 8.37(2H, d, J=9.0Hz).

### (2) 1-(4-Aminophenyl)-5-(tert-butyl)-3-(trifluoromethyl)pyrazole.

Using 5-(tert-butyl)-1-(4-nitrophenyl)-3-(trifluoromethyl)pyrazole as the raw material, the same operation as the Example 538(2) gave the title compound.
Yield: 98.9%.
¹H-NMR(CDCl₃): δ 1.20(9H, s), 4.00(2H, br), 6.40(1H, s), 6.69(2H, d, J=8.7Hz), 7.14(2H, d, J=9.0Hz).

### (3) N-{4-[5-(tert-butyl)-3-(trifluoromethyl)pyrazol-1-yl]phenyl}-5-chloro-2-hydroxybenzamide(Compound No. 539).

Using 5-chlorosalicylic acid and 1-(5-aminophenyl)-5-(tert-butyl)-3-(trifluoromethyl)pyrazole as the raw materials, the same operation as the Example 16 gave the title compound.
Yield: 57.6%.
¹H-NMR(CDCl₃): δ 1.23(9H, s), 6.47(1H, s), 7.00(1H, d, J=9.0Hz), 7.40-7.44(3H, m), 7.57(1H, d, J=2.4Hz), 7.72(2H, d, J=8.7Hz), 8.15(1H, s), 11.58(1H, s).

### Example 540: Preparation of the compound of Compound No. 540.

Using N-[3,5-bis(trifluoromethyl)phenyl]-2-hydroxy-3-phenylbenzamide (Compound No. 527), the same operation as the Example 537 gave the title compound.
Yield: 67.5%.
¹H-NMR(DMSO-d₆): δ 7.36-7.50(3H, m), 7.55-7.59(2H, m), 7.71(1H, d, J=2.1Hz), 7.93(1H, brs), 8.28(1H, d, J=2.1Hz), 8.45(2H, s), 11.06(1H, brs), 12.16(1H, brs).

### Example 541: Preparation of the compound of Compound No. 541.

### (1) 2-Amino-4-(3,4-dichlorophenyl)thiazole.

Using 3',4'-dichloroacetophenone and thiourea as the raw materials, the same operation as the Example 395(1) gave the title compound.
Yield: 77.8%.
¹H-NMR(DMSO-d₆): δ7.17(2H, s), 7.24(1H, s), 7.62(1H, d, J=8.4Hz), 7.78(1H, dd, J=8.7, 2.7Hz), 8.22(1H, d, J=2.4Hz).

### (2) 5-Chloro-2-hydroxy-N-[4-(3,4-dichlorophenyl)thiazol-2-yl]benzamide(Compound No. 541).

Using 5-chlorosalicylic acid and 2-amino-4-(3,4-dichlorophenyl)thiazole as the raw materials, the same operation as the Example 16 gave the title compound.
Yield: 15.1%.
¹H-NMR(DMSO-d₆): δ7.08(1H, d, J=8.7Hz), 7.52(1H, dd, J=8.7, 2.7Hz), 7.71(1H, d, J=8.4Hz), 7.91(1H, d, J=1.8Hz), 7.94(1H, s), 8.18(1H, d, J=1.5Hz), 12.09(2H, bs).

### Example 542: Preparation of the compound of Compound No. 542.

### (1) 2-Amino-4-[4-(trifluoromethyl)phenyl]thiazole.

Using 4'-(trifluoromethyl)acetophenone and thiourea as the raw materials, the same operation as the Example 395(1) gave the title compound.
Yield: 77.5%.
¹H-NMR(DMSO-d₆): δ 7.18(2H, s), 7.26(1H, s), 7.72(2H, d, J=8.4Hz), 8.00(2H, d, J=8.1Hz).

### (2) 5-Chloro-2-hydroxy-N-{4-[4-(trifluoromethyl)phenyl]thiazol-2-yl}benzamide (Compound No. 542).

Using 5-chlorosalicylic acid and 2-amino-4-[4-(trifluoromethyl)phenyl]thiazole as the raw materials, the same operation as the Example 16 gave the title compound.
Yield: 16.0%.
¹H-NMR(DMSO-d₆): δ 7.09(1H, d, J=9.0Hz), 7.53(1H, dd, J=8.7, 2.7Hz), 7.81(2H, d, J=8.4Hz), 7.96(1H, d, J=2.4Hz), 7.98(1H, s), 8.16(2H, d, J=8.1Hz), 11.91(1H, bs), 12.13(1H, bs).

### Example 543: Preparation of the compound of Compound No. 543.

### (1) 2-Acetoxy-N-{4-[3,5-bis(trifluoromethyl)pyrazol-1-yl]phenyl}-5-chlorobenzamide.

Using 2-acetoxy-5-chlorobenzoic acid and 1-(4-aminophenyl)-3,5-bis(trifluoromethyl)pyrazole as the raw materials, the same operation as the Example 24 gave the title compound.
Yield: 77.8%.
¹H-NMR(CDCl₃): δ 2.36(3H, s), 7.78(1H, s), 7.14(1H, d, J=8.7Hz), 7.48-7.51(3H, m), 7.77(2H, d, J=9.0Hz), 7.83(1H, d, J=2.7Hz), 8.25(1H, s).

### [1-(4-Aminophenyl)-3,5-bis(trifluoromethyl)pyrazole: Refer to "Journal of Medicinal Chemistry", 2000, Vol.43, No.16, p.2975-2981.]

### (2) N-{4-[3,5-Bis(trifluoromethyl)pyrazol-1-yl]phenyl}-5-chloro-2-hydroxybenzamide (Compound No. 543).

Using 2-acetoxy-N-{4-[3,5-bis(trifluoromethyl)pyrazol-1-yl]phenyl}-5-chlorobenzamide as the raw material, the same operation as the Example 2(2) gave the title compound.
Yield: 73.1%.
¹H-NMR(DMSO-d₆): δ 7.04(1H, d, J=8.7Hz), 7.48(1H, dd, J=8.7, 2.7Hz), 7.63(2H, d, J=8.7Hz), 7.84(1H, s), 7.89(1H, d, J=3.0Hz), 7.94(2H, d, J=9.0Hz), 10.65(1H, s), 11.58(1H, s).

### Example 544: Preparation of the compound of Compound No. 544.

### (1) 3,5-Bis(trifluoromethyl)-1-(3-nitrophenyl)pyrazole.

Using hexafluoroacetylacetone and 3-nitrophenylhydrazine hydrochloride as the raw materials, the same operation as the Example 538(1) gave the title compound.
Yield: 94.0%.
¹H-NMR(CDCl₃): δ7.16(1H, s), 7.77(1H, dd, J=8.7, 8.1Hz), 7.88-7.91(1H, m), 8.42-8.45(2H, m).

### (2) 1-(3-Aminophenyl)-3,5-bis(trifluoromethyl)pyrazole.

Using 3,5-bis(trifluoromethyl)-1-(3-nitrophenyl)pyrazole as the raw material, the same operation as the Example 538(2) gave the title compound.
Yield: 73.1%.
¹H-NMR(CDCl₃): δ3.89(2H, s), 6.77-6.87(3H, m), 7.04(1H, s), 7.26(1H, t, J=8.7Hz).

### (3) 2-Acetoxy-N-{3-[3,5-bis(trifluoromethyl)pyrazol-1-yl]phenyl}-5-chlorobenzamide.

Using 2-acetoxy-5-chlorobenzoic acid and 1-(3-aminophenyl)-3,5-bis(trifluoromethyl)pyrazole as the raw materials, the same operation as the Example 24 gave the title compound.
Yield: 84.4%.
¹H-NMR(CDCl₃): δ 2.33(3H, s), 7.09(1H, s), 7.11(1H, d, J=9.0Hz), 7.30(1H, d, J=7.8Hz), 7.45-7.52(2H, m), 7.67(1H, d, J=8.4Hz), 7.78(1H, d, J=2.4Hz), 7.95(1H, s), 8.29(1H, s).

### (4) N-{3-[3,5-Bis(trifluoromethyl)pyrazol-1-yl]phenyl}-5-chloro-2-hydroxybenzamide (Compound No. 544).

Using 2-acetoxy-N-{3-[3,5-bis(trifluoromethyl)pyrazol-1-yl]phenyl}-5-chlorobenzamide as the raw material, the same operation as the Example 2(2) gave the title compound.
Yield: 69.9%.
¹H-NMR(CDCl₃): δ 7.01(1H, d, J=8.7Hz), 7.10(1H, s), 7.34-7.37(1H, m), 7.42(1H, dd, J=8.7, 2.4Hz), 7.50(1H, d, J=2.4Hz), 7.56(1H, t, J=8.1Hz), 7.69-7.73(1H, m), 7.95-7.98(2H, m), 11.57(1H, s).

### Example 545: Preparation of the compound of Compound No. 545.

### (1) Methyl 2-methoxy-4-phenylbenzoate.

Dichlorobis(triphenylphosphine)palladium(29mg, 0.04mmol) was added to a solution of methyl 4-chloro-2-methoxybenzoate(904mg, 4.5mmol), phenylboronic acid(500mg, 4.1mmol) and cesium carbonate(2.7g, 8.2mmol) in N,N-dimethylformamide(15mL) under argon atmosphere, and the mixture was stirred at 120°C for 8 hours. After the reaction mixture was cooled to room temperature, it was diluted with ethyl acetate. The ethyl acetate layer was washed successively with water and brine, and dried over anhydrous sodium sulfate. The residue obtained by evaporation of the solvent under reduced pressure was purified by column chromatography on silica gel(n-hexane:ethyl acetate=10:1) to give the title compound(410mg, 41.2%) as a colourless oil.
¹H-NMR(CDCl₃): δ3.91(3H, s), 3.98(3H, s), 7.17(1H, d, J=1.5Hz), 7.20(1H, dd, J=8.1, 1.5Hz), 7.31-7.50(3H, m), 7.59-7.63(2H, m), 7.89(1H, d, J=8.1Hz).

### (2) 2-Methoxy-4-phenylbenzoic acid

2N Aqueous sodium hydroxide(5mL) was added to a solution of methyl 2-methoxy-4-phenylbenzoate(410mg, 1.69mmol) in methanol(5mL), and the mixture was refluxed for 1 hour. After the reaction mixture was cooled to room temperature, the solvent was evaporated under reduced pressure. 2N hydrochloric acid was added to the obtained residue and the separated crystal was filtered to give the title compound(371mg, 96.0%) as a crude product.
¹H-NMR(DMSO-d₆): δ 3.93(3H, s), 7.29(1H, dd, J=8.1, 1.5Hz), 7.34(1H, d, J=1.5Hz), 7.40-7.53(3H, m), 7.73-7.77(3H, m), 12.60(1H, s).

### (3) N-[3,5-Bis(trifluoromethyl)phenyl]-2-methoxy-4-phenylbenzamide.

Using 2-methoxy-4-phenylbenzoic acid and 3,5-bis(trifluoromethyl)aniline as the raw materials, the same operation as the Example 16 gave the title compound.
Yield: 97.5%.
¹H-NMR(CDCl₃): δ4.19(3H, s), 7.25(1H, m), 7.38-7.53(4H, m), 7.62-7.65(3H, m), 8.12(2H, s), 8.35(1H, d, J=8.1Hz), 10.15(1H, brs).

### (4) N-[3,5-Bis(trifluoromethyl)phenyl]-2-hydroxy-4-phenylbenzamide(Compound No. 545).

1M Boron tribromide-dichloromethane solution(0.71mL, 0.71mmol) was added to a solution of N-[3,5-bis(trifluoromethyl)phenyl]-2-methoxy-4-phenylbenzamide (100mg, 0.24mmol) in dichloromethane(5mL), and the mixture was stirred at room temperature for 1 hour. The reaction mixture was diluted with ethyl acetate, washed with water and brine, and dried over anhydrous magnesium sulfate. The residue obtained by evaporation of the solvent under reduced pressure was purified by column chromatography on silica gel(n-hexane:ethyl acetate=5:1) to give the title compound(69.3mg, 71.6%) as a white powder.
¹H-NMR(DMSO-d₆): δ 7.20(1H, dd, J=8.4.1.8Hz), 7.30(1H, d, J=1.8Hz), 7.39-7.51(3H, m), 7.60-7.64(3H, m), 7.70(1H, brs), 8.15(2H, s), 8.19(1H, brs), 11.59(1H, s).

### Example 546: Preparation of the compound of Compound No. 546.

### (1) 2-Amino-4-(2,5-difluorophenyl)thiazole.

Using 2',5'-difluoroacetophenone and thiourea as the raw materials, the same operation as the Example 395(1) gave the title compound.
Yield: 77.8%.
¹H-NMR(DMSO-d₆): δ7.45(1H, d, J=2.7Hz), 7.11-7.17(1H, m), 7.19(2H, s), 7.28-7.36(1H, m), 7.65-7.71(1H, m).

### (2) 5-Chloro-2-hydroxy-N-[4-(2,5-difluorophenyl)thiazol-2-yl]benzamide(Compound No. 546).

Using 5-chlorosalicylic acid and 2-amino-4-(2,5-difluorophenyl)thiazole as the raw materials, the same operation as the Example 16 gave the title compound.
Yield: 36.5%.
¹H-NMR(DMSO-d₆): δ 7.09(1H, d, J=8.7Hz), 7.22-7.30(1H, m), 7.37(1H, m), 7.53(1H, dd, J=8.7, 3.0Hz), 7.72(1H, d, J=2.4Hz), 7.77-7.84(1H, m), 7.94(1H, d, J=3.0Hz), 11.89(1H, bs), 12.12(1H, bs).

### Example 547: Preparation of the compound of Compound No. 547.

### (1) 2-Acetoxy-4-chlorobenzoic acid.

Using 4-chlorosalicylic acid, concentrated sulfuric acid and acetic anhydride as the raw materials, the same operation as the Example 34(1) gave the title compound.
Yield: 88.1%.
¹H-NMR(DMSO-d₆): δ 2.25(3H, s), 7.42(1H, d, J=1.8Hz), 7.48(1H, dd, J=8.4, 2.4Hz), 7.94(1H, d, J=8.1Hz), 13.31(1H, s).

### (2) 2-Acetoxy-N-{4-[3,5-bis(trinuoromethyl)pyrazol-1-yl]phenyl}-4-chlorobenzamide.

Using 2-acetoxy-4-chlorobenzoic acid and 1-(4-aminophenyl)-3,5-bis(trifluoromethyl)pyrazole as the raw materials, the same operation as the Example 24 gave the title compound.
Yield: 74.0%.
¹H-NMR(CDCl₃): δ2.37(3H, s), 7.08(1H, s), 7.23(1H, d, J=1.8Hz), 7.37(1H, dd, J=8.1, 2.1Hz), 7.50(2H, d, J=8.7Hz), 7.77(2H, d, J=8.7Hz), 7.82(1H, d, J=8.1Hz), 8.23(1H, s).

### (3) N-{4-[3,5-Bis(trifluoromethyl)pyrazol-1-yl]phenyl}-4-chloro-2-hydroxybenzamide (Compound No. 547).

Using 2-acetoxy-N-{4-[3,5-bis(trifluoromethyl)pyrazol-1-yl]phenyl}-4-chlorobenzamide as the raw material, the same operation as the Example 2(2) gave the title compound.
Yield: 56.6%.
¹H-NMR(DMSO-d₆): δ 7.03-7.06(2H, m), 7.61(2H, d, J=8.7Hz), 7.81(1H, s), 7.89-7.95(3H, m), 10.62(1H, s), 11.82(1H, s).

### Example 548: Preparation of the compound of Compound No. 548.

### (1) 1-(4-Nitrophenyl)-5-phenyl-3-(trifluoromethyl)pyrazole.

Using 4,4,4-trifluoro-1-phenyl-1,3-butanedione and 4-nitrophenylhydrazine hydrochloride as the raw materials, the same operation as the Example 538(1) gave the title compound.
Yield: 95.2%.
¹H-NMR(CDCl₃): δ6.80(1H, s), 7.22-7.26(2H, m), 7.37-7.45(3H, m), 7.51(2H, d, J=9.3Hz), 8.22(2H, d, J=9.0Hz).

### (2) 1-(4-Aminophenyl)-5-phenyl-3-(trifluoromethyl)pyrazole.

Using 1-(4-nitrophenyl)-5-phenyl-3-(trifluoromethyl)pyrazole as the raw material, the same operation as the Example 538(2) gave the title compound.
Yield: 73.0%.
¹H-NMR(CDCl₃): δ3.80(2H, s), 6.62(2H, d, J=8.7Hz), 6.72(1H, s), 7.08(2H, d, J=8.7Hz), 7.22-7.26(2H, m), 7.30-7.33(3H, m).

### (3) 5-Chloro-2-hydroxy-N-{4-[5-phenyl-3-(trifluoromethyl)pyrazol-1-yl]phenyl}benzamide(Compound No. 548).

Using 5-chlorosalicylic acid and 1-(4-aminophenyl)-5-phenyl-3-(trifluoromethyl)pyrazole as the raw materials, the same operation as the Example 16 gave the title compound.
Yield: 73.2%.
¹H-NMR(CDCl₃): δ7.02(1H, d, J=8.7Hz), 7.21(1H, s), 7.30-7.42(7H, m), 7.47(1H, dd, J=8.7, 2.7Hz), 7.79(2H, d, J=8.7Hz), 7.89(1H, d, J=2.7Hz), 10.56(1H, s), 11.61(1H, s).

### Example 549: Preparation of the compound of Compound No. 549.

### (1) 2-Amino-4-(4-methoxyphenyl)thiazole.

Using 4'-methoxyacetophenone and thiourea as the raw materials, the same operation as the Example 395(1) gave the title compound.
Yield: 85.2%.
¹H-NMR(DMSO-d₆): δ 3.76(3H, s), 6.82(1H, s), 6.92(2H, d, J=9.0Hz), 7.01(2H, s), 7.72(2H, d, J=8.7Hz).

### (2) 5-Chloro-2-hydroxy-N-[4-(4-methoxyphenyl)thiazol-2-yl]benzamide(Compound No. 549).

Using 5-chlorosalicylic acid and 2-amino-4-(4-methoxyphenyl)thiazole as the raw materials, the same operation as the Example 16 gave the title compound.
Yield: 16.4%.
¹H-NMR(DMSO-d₆): δ3.80(3H, s), 7.01(2H, d, J=9.0Hz), 7.07(1H, d, J=8.7Hz), 7.50-7.55(2H, m), 7.86(2H, d, J=9.0Hz), 7.96(1H, d, J=2.7Hz), 11.90(1H, bs), 12.04(1H, bs).

### Example 550: Preparation of the compound of Compound No. 550.

### (1) 2-Amino-4-[3-(trifluoromethyl)phenyl]thiazole.

Using 3'-(trifluoromethyl)acetophenone and thiourea as the raw materials, the same operation as the Example 395(1) gave the title compound.
Yield: 94.1%.
¹H-NMR(DMSO-d₆): δ 7.19(2H, s), 7.27(1H, s), 7.61(2H, dd, J=3.9, 1.5Hz), 8.07-8.13(2H, m).

### (2) 5-Chloro-2-hydroxy-N-{4-[3-(trinuoromethyl)phenyl]thiazol-2-yl}benzamide (Compound No. 550).

Using 5-chlorosalicylic acid and 2-amino-4-[3-(trifluoromethyl)phenyl]thiazole as the raw materials, the same operation as the Example 16 gave the title compound.
Yield: 31.0%.
¹H-NMR(DMSO-d₆): δ7.13(1H, d, J=8.7Hz), 7.53(1H, dd, J=9.0, 2.7Hz), 7.70(1H, d, J=2.4Hz), 7.71(1H, d, J=1.2Hz), 7.95(1H, d, J=2.7Hz), 8.00(1H, s), 8.24-8.27(2H, m), 12.16(2H, bs).

### Example 551: Preparation of the compound of Compound No. 551.

### (1) 2-Amino-4-(2,3,4,5,6-pentafluorophenyl)thiazole.

Using 2',3',4',5',6'-pentafluoroacetophenone and thiourea as the raw materials, the same operation as the Example 395(1) gave the title compound.
Yield: 86.7%.
¹H-NMR(CDCl₃): δ 5.19(2H, s), 6.83(1H, s).

### (2) 5-Chloro-2-hydroxy-N-[4-(2,3,4,5,6-pentafluorophenyl)thiazol-2-yl]benzamide (Compound No. 551).

Using 5-chlorosalicylic acid and 2-amino-4-(2,3,4,5,6-pentafluorophenyl)thiazole as the raw materials, the same operation as the Example 16 gave the title compound.
Yield: 23.8%.
¹H-NMR(DMSO-d₆): δ 7.08(1H, d, J=8.7Hz), 7.53(1H, dd, J=8.7, 2.7Hz), 7.73(1H, s), 7.93(1H, d, J=2.7Hz), 11.85(1H, bs), 12.15(1H, bs).

### Example 552: Preparation of the compound of Compound No. 552.

Iron(3mg, 0.05mmol) and bromine(129 µl, 2.5mmol) were added to a solution of 2-hydroxy-N-[2,5-bis(trifluoromethyl)phenyl]benzamide(Compound No. 533; 175mg, 0.5mmol) in carbon tetrachloride(5mL), and the mixture was stirred at 50°C for 12 hours. After the reaction mixture was cooled to room temperature, it was washed with saturated aqueous sodium hydrogen carbonate, water and brine, and dried over anhydrous magnesium sulfate. The residue obtained by evaporation of the solvent under reduced pressure was purified by column chromatography on silica gel(n-hexane:ethyl acetate=2:1) to give the title compound(184.2mg, 72.7%) as a white crystal.
¹H-NMR(DMSO-d₆): δ 7.92-7.98(1H, m), 8.06(1H, d, J=2.1Hz), 8.09(1H, d, J=8.4Hz), 8.22(1H, d, J=2.1Hz), 8.27-8.32(1H, m), 11.31(1H, s).

### Example 553: Preparation of the compound of Compound No. 553.

Using 2,3-dihydroxybenzaldehyde and 3-[3,5-bis(trifluoromethyl)benzyl]thiazolidine-2,4-dione(compound of Example 319(1)) as the raw materials, the same operation as the Example 319(2) gave the title compound.
Yield: 88.5%.
¹H-NMR(DMSO-d₆): δ 5.02(2H, s), 6.88(1H, d, J=7.8Hz), 7.00-7.04(2H, m), 7.79(1H, s), 8.03(2H, s), 8.07(1H, s), 9.49(1H, s), 9.91(1H, s).

### Example 554: Preparation of the compound of Compound No. 554.

A mixture of 5-chlorosalicylaldehyde(157mg, 1mmol), 2-amino-4-tert-amylphenyl phenyl ether(255mg, 1mmol) and ethanol(2mL) was stirred at room temperature for 18 hours. The residue obtained by evaporation of the solvent under reduced pressure was purified by column chromatography on silica gel(n-hexane:ethyl acetate=100:1) to give the title compound(57mg, 14.4%) as a white solid.
¹H-NMR(CDCl₃): δ 0.66(3H, t, J=7.5Hz), 1.26(6H, s), 1.61(2H, q, J=7.5Hz), 6.88-6.94(3H, m), 7.04(1H, dd, J=8.0, 1.6Hz), 7.15-7.32(7H, m), 8.61(1H, s), 13.20(1H, s).

### Example 555: Preparation of the compound of Compound No. 555.

A mixture of 4-chloro-2-({[2-phenoxy-5-(tert-amyl)phenyl]imino}methyl)phenol(Compound No. 554; 13mg, 0.03mmol), sodium borohydride(1.2mg, 0.03mmol) and methanol(1mL) was stirred at room temperature for 5 minutes. The residue obtained by evaporation of the solvent under reduced pressure was purified by thin layer chromatography on silica gel(n-hexane:ethyl acetate=5:1) to give the title compound(13mg, 100%) as a colourless oil.
¹H-NMR(CDCl₃): δ 0.69(3H, t, J=7.6Hz), 1.28(6H, s), 1.63(2H, q, J=7.6Hz), 4.41(2H, s), 6.78(1H, m), 6.93-6.83(5H, m), 7.03(1H, m), 7.15(2H, m), 7.28(3H, m).

### Test Example 1: Measurement of Inhibition of NF-κB Activation by Forced Expression of MEKK-1

Using a transfection reagent(Effectene; QIAGEN), human uterine cancer cell strain HeLa was cotransfected with a plasumid (pNFκB-Luc Reporter Plasmid: STRATAGENE) integrated with an oligonucleotide having five tandem copies of NF-κB binding sequences(TGGGGACTTTCCGC) on an upstream region of the firefly luciferase gene(Luc) and the MEKK-1 gene-contained expression vector(pFC-MEKK: STRATAGENE) according to the QIAGEN's protocol, and the cells were incubated for 24 hours. After incubation in the presence or absence of a test compound for 24 hours, intracellular luciferase activity was measured by using PicaGene LT(TOYO INK MFG Co., Ltd.) and a chemical luminescence measurement apparatus(SPECTRAFluor Plus; TECAN). The inhibitory ratio was measured as a ratio relative to the value of the luciferase activity in the absence of the test compound. The inhibitory ratios of NF-κB activity in the presence of the test compound at 10 µg/ml and µg/ml are shown in the following table.

| Compound Number | Inhibitory Ratio of NF-κB Activation(%) | |
|---|---|---|
| | Drug Concentration 10 µg/ml | Drug Concentration 1 µg/ml |
| 50 | 93.2 | 92.6 |
| 51 | 92.3 | 90.0 |
| 148 | 93.1 | 90.6 |

### Test Example 2: Detection of Phosphorylated IκBα by Western Blot Method

To the culture medium of HepG2 cells, 2 µg/ml of a test compound and 20 µM of proteasome inhibitor MG-132 were added. After 45 minutes, 40 ng/ml of human TNF α was further added. Ten minutes after the addition of the TNF α, the cells were collected, and a cell lysate was prepared by using a tip-type ultrasonic processor (Dr.Hielsher; UP-50H). After the measurement of a protein concentration using a BCA protein assay kit by Pierce (BSA standard), 30 µg of the cell lysate was applied to each lane of 12% SDS slab gel (mini gel) and an electrophoresis was carried out. After the electrophoresis, a detection of phorphorylated IκBα by Western blot method was carried out using anti-phosphorylated IκBα (Ser32) antibody (Cell Signaling) as a primary antibody and rabbit polyclonal anti-IκBα antibody (Santa Cruz Biotechnology) as a secondary antibody.

The results are shown in the following table.

| Compound Number | Drug Concentration | Inhibition Ratio of IκB phosphorylation(%) |
|---|---|---|
| curcumin | 100 µM | 51.6 |
| 50 | 2 µg/ml | 43.0 |
| 51 | 2 µg/ml | 39.7 |
| 56 | 2 µg/ml | 31.3 |
| 63 | 2 µg/ml | 26.5 |
| 67 | 2 µg/ml | 43.8 |
| 71 | 2 µg/ml | 29.5 |
| 73 | 2 µg/ml | 45.6 |
| 98 | 2 µg/ml | 44.9 |
| 114 | 2 µg/ml | 57.6 |
| 122 | 2 µg/ml | 49.5 |
| 163 | 2 µg/ml | 51.0 |
| 195 | 2 µg/ml | 63.5 |
| 196 | 2 µg/ml | 50.6 |
| 199 | 2 µg/ml | 47.9 |
| 201 | 2 µg/ml | 57.4 |

### Industrial Applicability

The medicament of the present invention has an inhibitory activity against IKK-*β and/or MEKK-1 or other protein kinases structurally similar thereto, and can achieve the inhibition of the transcription factor NF-κB activation and the inhibition of the production and release of inflammatory cytokines. Therefore, the medicament of the present invention can be used as a medicament for preventive and/or therapeutic treatment of diseases caused by NF-κB activation and inflammatory cytokine overproduction.

## Claims

1. A medicament having an inhibitory activity against IKK-β and/or MEKK-1 or other protein kinases structurally similar thereto, which comprises as an active ingredient a substance selected from the group consisting of a compound represented by the following general formula (I) and a pharmacologically acceptable salt thereof, and a hydrate thereof and a solvate thereof: 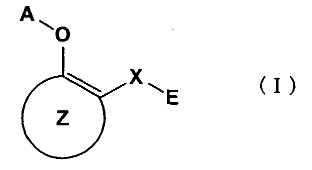 wherein X represents a connecting group whose number of atoms in the main chain is 2 to 5 (said connecting group may be substituted),
A represents hydrogen atom or acetyl group,
E represents an aryl group which may be substituted or a heteroaryl group which may be substituted,
ring Z represents an arene which may have one or more substituents in addition to the group represented by formula -O-A wherein A has the same meaning as that defined above and the group represented by formula -X-E wherein each of X and E has the same meaning as that defined above, or a heteroarene which may have one or more substituents in addition to the group represented by formula -O-A wherein A has the same meaning as that defined above and the group represented by formula -X-E wherein each of X and E has the same meaning as that defined above.

2. The medicament according to claim 1, wherein X is a group selected from the following connecting group α (said group may be substituted):
[Connecting Group α ] The groups of the following formulas: 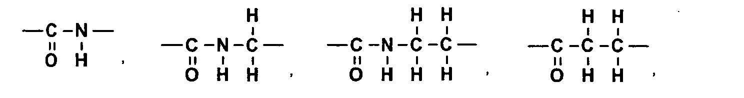 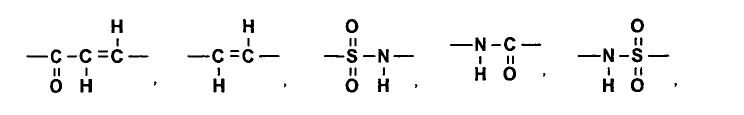 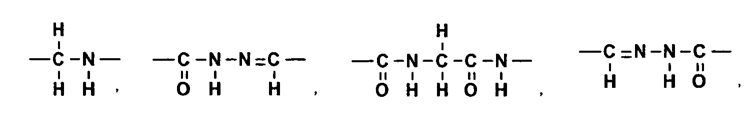 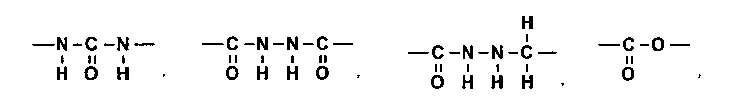 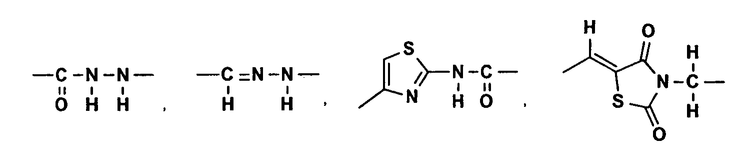 wherein a bond at the left end binds to ring Z and a bond at the right end binds to E.

3. The medicament according to claim 2, wherein X is a group represented by the following formula (said group may be substituted): 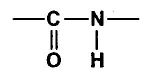 wherein a bond at the left end binds to ring Z and a bond at the right end binds to E.

4. The medicament according to any one of claims 1 to 3, wherein A is a hydrogen atom.

5. The medicament according to any one of claims 1 to 4, wherein ring Z is a C₆ to C₁₀ arene which may have one or more substituents in addition to the group represented by formula -O-A wherein A has the same meaning as that defined in the general formula (I) and the group represented by formula -X-E wherein each of X and E has the same meaning as that defined in the general formula (I), or a 5 to 13-membered heteroarene which may have one or more substituents in addition to the group represented by formula -O-A wherein A has the same meaning as that defined in the general formula (I) and the group represented by formula -X-E wherein each of X and E has the same meaning as that defined in the general formula (I).

6. The medicament according to claim 5, wherein ring Z is a ring selected from the following ring group β:
[Ring Group β] benzene ring, naphthalene ring, thiophene ring, pyridine ring, indole ring, quinoxaline ring, and carbazole ring
wherein said ring may have one or more substituents in addition to the group represented by formula -O-A wherein A has the same meaning as that defined in the general formula (I) and the group represented by formula -X-E wherein each of X and E has the same meaning as that defined in the general formula (I).

7. The medicament according to clam 6, wherein ring Z is a benzene ring which may have one or more substituents in addition to the group represented by formula -O-A wherein A has the same meaning as that defined in the general formula (I) and the group represented by formula -X-E wherein each of X and E has the same meaning as that defined in the general formula (I).

8. The medicament according to claim 7, wherein ring Z is a benzene ring which is substituted with halogen atom(s) in addition to the group represented by formula -O-A wherein A has the same meaning as that defined in the general formula (I) and the group represented by formula -X-E wherein each of X and E has the same meaning as that defined in the general formula (I).

9. The medicament according to claim 6, wherein ring Z is a naphthalene ring which may have one or more substituents in addition to the group represented by formula -O-A wherein A has the same meaning as that defined in the general formula (I) and the group represented by formula -X-E wherein each of X and E has the same meaning as that defined in the general formula (I).

10. The medicament according to any one of claims 1 to 9, wherein E is a C₆ to C₁₀ aryl group which may be substituted or a 5 to 13-membered heteroaryl group which may be substituted.

11. The medicament according to claim 10, wherein E is a phenyl group which may be substituted.

12. The medicament according to claim 11, wherein E is 3,5-bis(trifluoromethyl)phenyl group.

13. The medicament according to claim 10, wherein E is a 5-membered heteroaryl group which may be substituted.
